(19) Europäisches Patentamt
European Patent Office
Office européen des brevets

(11) EP 3 865 152 A1

(12) EUROPEAN PATENT APPLICATION
published in accordance with Art. 153(4) EPC

(43) Date of publication:
**18.08.2021 Bulletin 2021/33**

(21) Application number: **19872052.6**

(22) Date of filing: **09.10.2019**

(51) Int Cl.:
***A61K 47/66*** *(2017.01)* ***A61P 35/00*** *(2006.01)*

(86) International application number:
**PCT/CN2019/110225**

(87) International publication number:
**WO 2020/073930 (16.04.2020 Gazette 2020/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.10.2018 CN 201811174965**

(71) Applicant: **Eubulus Biotherapeutics Inc.**
**Jiaxing, Zhejiang 314000 (CN)**

(72) Inventors:
- **CAO, Sheldon**
  **Jiaxing, Zhejiang 314000 (CN)**
- **WANG, Xiaolei**
  **Jiaxing, Zhejiang 314000 (CN)**
- **HUANG, Chaoran**
  **Jiaxing, Zhejiang 314000 (CN)**

(74) Representative: **Cabinet Laurent & Charras**
**Le Contemporain**
**50 Chemin de la Bruyère**
**69574 Dardilly Cedex (FR)**

# (54) TARGETED PROTEASE DEGRADATION PLATFORM

(57) Disclosed is a targeted protease degradation platform (TED), which in particular is a conjugate of target molecule-linker-E3 ligase ligand as shown in the stmcture of A-L1-B (formula I), wherein the A is the monovalent group of the target molecule, the B is the monovalent group of the E3 ligase ligand, the L1 is the linker linking A and B, and L1 is as shown in -X-L2-Y- (formula II).

EP 3 865 152 A1

## Description

### TECHNICAL FIELD

The present invention belongs to biomedicine, specifically, relates to a Targeted Protease Degradation platform (TED).

### BACKGROUND

Expression level of proteins is regulated on three basic levels according to modern molecular biology. Firstly, at the DNA level, the target protein DNA is inactivated through gene knock-out. Secondly, at the mRNA level, it binds to the mRNA of the target protein through small molecule RNA, thereby inhibiting the translation and expression of mRNA. Thirdly, at the protein level, the amount and activity of the target protein can be regulated by modificating of the target protein after translation, such as methylation, phosphorylation, glycosylation, etc.

In terms of the overall development of drug research and development, both small molecule and macromolecule drug forms have their own advantages and disadvantages. For example, the development of small molecule drugs has been facing crucial challenges such as how to maintain drug concentration in the body and drug resistance. The shapes of some target sites are adverse to design of small molecule drugs, thus becoming non-drugable targets. For these targets, no effective regulatory methods have yet been found. Although, compared to small molecules, monoclonal antibodies have the advantages of high affinity and high selectivity, and easy to develop highly effective and highly selective drugs, but the biggest drawback thereof is that they cannot penetrate cell membranes and therefore cannot act on intracellular targets. Antibody-drug conjugates (ADC) utilize endocytic antibodies to provide targeting and serve as carriers to deliver super toxin drugs to the targeted site. The bottleneck encountered in the development of ADC drugs is that the treatment window is not wide enough. In addition to the side effects caused by the antibody itself, the super toxins will fall off before reaching the targeting site due to the heterogeneity of coupling, and causing serious side effects. In addition, normal physiological function of ubiquitin- proteasome system is responsible for cleaning up denatured, mutated or harmful proteins in cells.

Summary, there is an urgent need in the art to develop a compound that is able to degrade target proteins more efficiently and re-usably so as to treat related diseases.

### SUMMARY OF THE INVENTION

The purpose of the present application is to provide a compound that is able to degrade target proteins more efficiently and re-usably so as to treat related diseases.

In the first aspect of the present invention, a conjugate of formula I is provided:

A-L1-B formula I

wherein

A is a monovalent moiety of a targeting molecule;

B is a monovalent moiety of an E3 ligase ligand;

L1 is a linker connecting A and B; and L1 has a formula II:

-X-L2-Y- formula II

wherein

when X or Y is NH or NR, X or Y and part of L2 chain to which X or Y is attached are combined together to form a 4 - 8 membered aromatic or non-aromatic heterocyclic ring; wherein, R is substituted or unsubstituted C1-C10 alkyl, -(C=O)-R', (C=O)NH-R', -NH(C=O)- R', -$SO_2$-R', -$NHSO_2$-R', - $SO_2$NH-R',-SO-R', -NHSO-R', -SONH-R', -$PO_3$-R', -NHCOO-R', -COO-R', or -NH-CO-NH- R', -NH-CO-O-R', or -X'-L3-Z; wherein L3 is linker, and Z is a polypeptide element or target molecule T; wherein R' is selected from the group consisting of H, substituted or unsubstituted C1-C6 alkyl, substituted or unsubstituted C3-C8 cycloalkyl, amino protecting group, and -X'-L3-Z;

or

each X and Y is independently selected from the group consisting of -O-, -S-, -NH-, -NR-, -(C=O)NH-, -NH(C=O)-, -$SO_2$-, -$NHSO_2$-, - $SO_2$NH-, -SO-, -NHSO-, -SONH-, -$PO_3$-, -NHCOO-, -COO-, - $NHCOCH_2O$-, and -NH-CO-NH-; wherein R is substituted or unsubstituted C1-C10 alkyl, -(C=O)-R', - (C=O)NH-R', -NH(C=O)-R', -$SO_2$-R', -$NHSO_2$-R', - $SO_2$NH-R', -SO-R', -NHSO-R', -SONH-R', -$PO_3$- R', - NHCOO-R', -COO-R' or -NH-CO-NH- R', or -X'-L3-Z;

wherein L3 is linker, and Z is a polypeptide element or target molecule T; wherein R' is selected from the group consisting

of H, substituted or unsubstituted C1-C6 alkyl, substituted or unsubstituted C3-C8 cycloalkyl, protecting group, and -X'-L3-Z;

X' is selected from the group consisting of -O-, -S-, -NH-, -(C=O)NH-, -NH(C=O)-, $-SO_2-$, $-NHSO_2-$,$-SO_2NH-$, -SO-, -NHSO-, -SONH-, $-PO_3-$, -NHCOO-, -COO-, $-COOCH_2-$, and -NH-CO-NH-;

each L2 and L3 is independently linker selected from the group consisted of

(a) substituted or unsubstituted 5-20 membered carbon chain or 5-50 membered carbon chain;
(b) substituted or unsubstituted 5-20 membered heterocarbon chain or 5-50 membered heterocarbon chain containing 1 to 5 heteroatoms selected from N, O and S;
(c) substituted or unsubstituted 5-20 membered carbon chain or 5-50 membered carbon chain; and 1 to 4 chain atoms in the chain are replaced with heterocyclic ring selected from the group consisting of 4-10 membered saturated heterocyclic ring containing 1- 4 heteroatoms selected from N, O and S, and 5-10 membered partially unsaturated or fully unsaturated heterocyclic ring containing 1-4 heteroatoms selected from N, O and S;
(d) substituted or unsubstituted 5-20 membered heterocarbon chain or 5-50 membered heterocarbon chain containing 1 to 5 heteroatoms selected from N, O and S; and 1 to 4 chain atoms in the chain are replaced with heterocyclic ring selected from the group consisting of 4-10 membered saturated heterocyclic ring containing 1- 4 heteroatoms selected from N, O and S, and 5-10 membered partially unsaturated or fully unsaturated heterocyclic rings containing 1-4 heteroatoms selected from N, O and S;

wherein the "substituted" means one or more (preferably, 1, 2, 3 or 4) hydrogen atoms in the group is substituted with substituent selected from the group consisting of C1-C10 alkyl, C3-C8 cycloalkyl, -COOH, -OH, -SH, $-NH_2$, -NHR, $-N(R_1)R_2$, -Z, -X'-L3-Z,

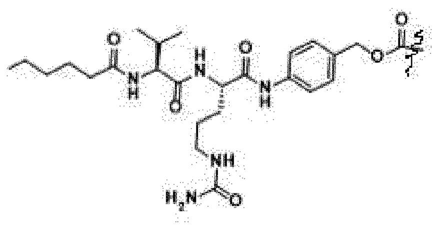

,

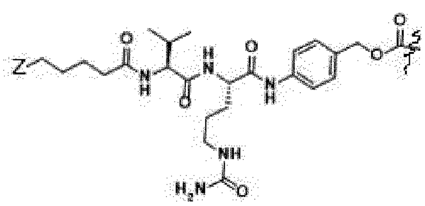

,

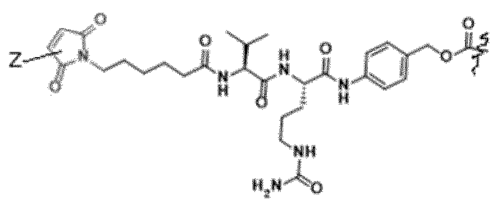

,

and

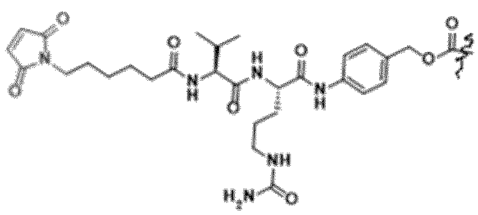

;

wherein Z is a polypeptide element or target molecule T;

wherein when the substituent is -Z, -Z is located only on N atom; wherein each $R_1$ and $R_2$ is indenpedently H, C1-C6 alkyl, C3-C8 cycloalkyl, amino protecting group, -X'-L3-Z,

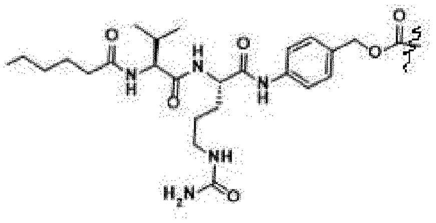,
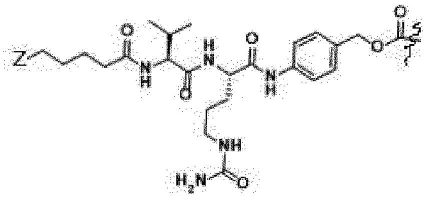,

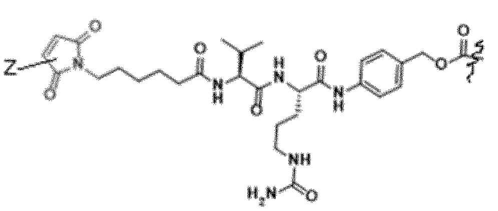, or
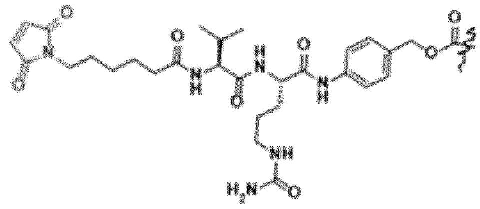;

wherein R is substituted or unsubstituted C1-C10 alkyl, -(C=O)-R', (C=O)NH-R', -NH(C=O)- R', $-SO_2-R'$, $-NHSO_2-R'$,$-SO_2NH-R'$, -SO-R', -NHSO-R', -SONH-R', $-PO_3-R'$, -NHCOO-R', -COO-R', or -NH-CO-NH- R', -NH-CO-O-R', or -X'-L3-Z; wherein L3 is linker, and Z is a polypeptide element or target molecule T; wherein R' is selected from the group consisting of H, substituted or unsubstituted C1-C6 alkyl, substituted or unsubstituted C3-C8 cycloalkyl, protecting group, and X'-L3-Z.

In another preferred embodiment, $R_1$ and $R_2$ are not -X'-L3-Z,

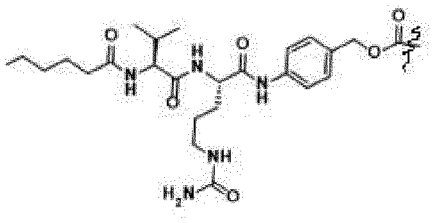,

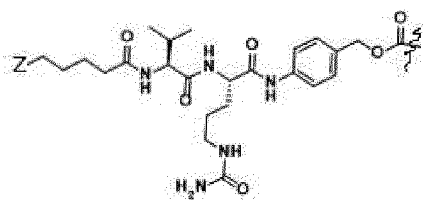,
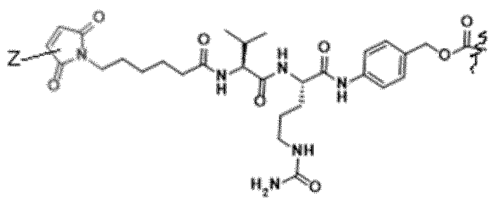,

or

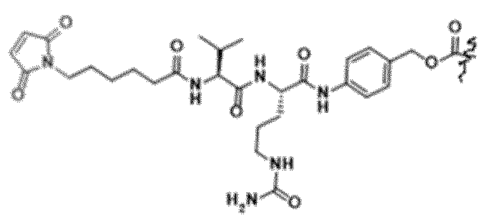

at the same time.

In another preferred embodiment, -X'-L3-Z is a group of fomula I-1, 1-2, 1-3, 1-4, or 1-5:

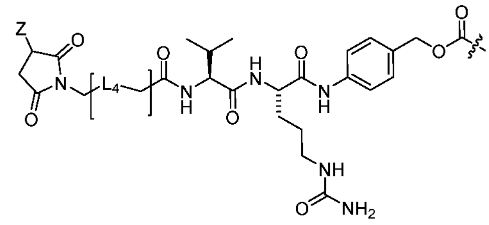 (I-1),

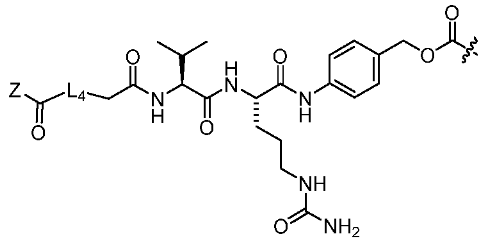 (I-2),

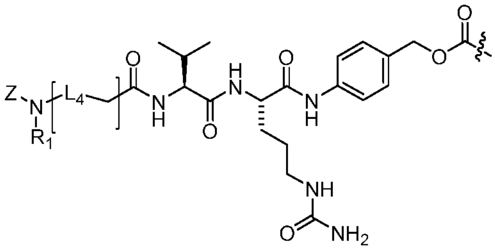 (I-3),

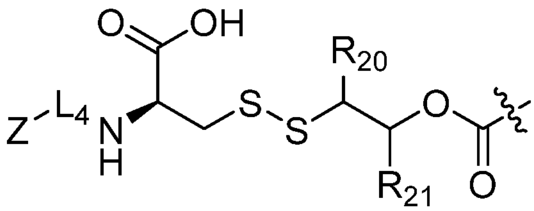 (I-4),

or

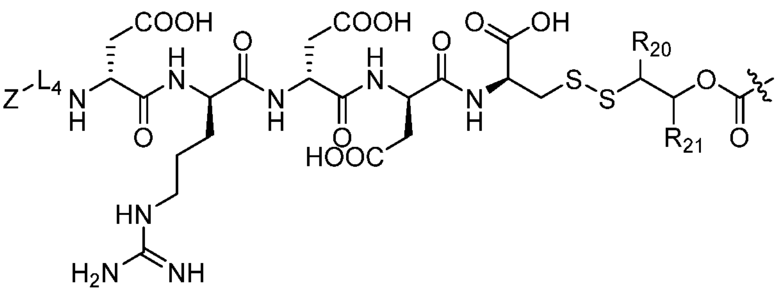 (I-5);

wherein, each $R_{20}$ and $R_{21}$ is indenpendently selected from the group consisting of -H, -Me, -Et, -nPr, iPro, cPro; L4 is defined the same as L3.

In another preferred embodiment, when -X'-L3-Z is a group of I-1, 1-2, or 1-3, Z is a polypeptide element.

In another preferred embodiment, when -X'-L3-Z is a group of I-4, or 1-5, Z is a target molecule T.

In another preferred embodiment, L4 is a linker selected from the group consisted of

(a) substituted or unsubstituted 5-20 membered carbon chains;

(b) substituted or unsubstituted 5-20 membered heterocarbon chains containing 1 to 5 heteroatoms selected from N, O and S;

(c) substituted or unsubstituted 5-20 membered carbon chains, and 1 to 4 chain atoms in the chain are replaced with heterocyclic rings selected from the group consisting of 4-10 membered saturated heterocyclic rings containing 1-4 heteroatoms selected from N, O and S, and 5-10 membered partially unsaturated or fully unsaturated heterocyclic rings containing 1-4 heteroatoms selected from N, O and S;

(d) substituted or unsubstituted 5-20 membered heterocarbon chains containing 1 to 5 heteroatoms selected from N, O and S, and 1 to 4 chain atoms in the chain are replaced with heterocyclic rings selected from the group consisting of 4-10 membered saturated heterocyclic rings containing 1-4 heteroatoms selected from N, O and S, and 5-10 membered partially unsaturated or fully unsaturated heterocyclic rings containing 1-4 heteroatoms selected from N, O and S;

wherein the "substituted" means one or more (preferably, 1, 2, 3 or 4) hydrogen atoms in the group is substituted with substituents selected from the group consisting of C1-C10 alkyl, C3-C8 cycloalkyl,-COOH, -OH, -SH, $-NH_2$, -NHR, $-N(R_1)R_2$; wherein each $R_1$ and $R_2$ is indenpedently H, C1-C6 alkyl, C3-C8 cycloalkyl, amino protecting group; R is substituted or unsubstituted C1-C10 alkyl, -(C=O)-R', (C=O)NH-R', -NH(C=O)- R', $-SO_2$-R', $-NHSO_2$-R', - $SO_2$NH-R', -SO-R', -NHSO-R', -SONH-R', $-PO_3$-R', -NHCOO-R', -COO-R', or -NH-CO-NH- R', or -NH-CO-O-R'; wherein R' is selected from the group consisting of H, substituted or unsubstituted C1-C6 alkyl, substituted or unsubstituted C3-C8

cycloalkyl, and protecting group.

In another preferred embodiment, L4 is linker selected from the group consisted of

(a) substituted or unsubstituted 5-20 membered carbon chain;
(b) substituted or unsubstituted 5-20 membered heterocarbon chain containing 1 to 5 heteroatoms selected from N, O and S.

In another preferred embodiment, the polypeptide element is selected from the group consisting of ligand or active fragment thereof, receptor or active fragment thereof, antibody or active fragment thereof, and a combination thereof.

In another preferred embodiment, Z is selected from the group consisting of ligand, soluble receptor, antibody, or antibody-drug conjugate (ADC), and a combination thereof.

In another preferred embodiment, the target molecule T and the target molecule A are the same or different.

In another preferred embodiment, the target molecule T and the target molecule A are small molecules with targeting functions in the drug conjugates.

In another preferred embodiment, the target molecule T and the target molecule A are each indenpendently selected from the group consisting of folic acid, HSP90 inhibitor, and a combination thereof.

In another preferred embodiment, the ligand comprises full-length ligand or active fragment thereof.

In another preferred embodiment, the antibody comprises full-length antibody or active fragment thereof.

In another preferred embodiment, the antibody comprisessingle-chain antibody or double-chain antibody.

In another preferred embodiment, the 4-10 membered saturated heterocyclic ring containing 1-4 heteroatoms selected from N, O and S can be single ring, double ring or triple ring.

In another preferred embodiment, the 4-10 membered saturated heterocyclic ring containing 1-4 heteroatoms selected from N, O and S can be fused ring or spiro ring.

In another preferred embodiment, the 4-10 membered saturated heterocyclic ring containing 1-4 heteroatoms selected from N, O and S can be a 4 - 6 membered saturated ring containing 1 nitrogen atom or a 4 - 6 membered saturated ring containing 1 nitrogen atom.

In another preferred embodiment, the 5-10 membered partially unsaturated or fully unsaturated heterocyclic ring containing 1-4 heteroatoms selected from N, O and S can be single ring, double ring or triple ring.

In another preferred embodiment, the 5-10 membered partially unsaturated or fully unsaturated heterocyclic ring containing 1-4 heteroatoms selected from N, O and S can be fused ring or spiro ring.

In another preferred embodiment, the 5-10 membered partially unsaturated or fully unsaturated heterocyclic rings containing 1-4 heteroatoms selected from N, O and S can be 5 membered partially unsaturated or fully unsaturated heterocyclic rings containing 1-4 heteroatoms selected from N, O and S.

In another preferred embodiment, L2 and/or L3 are linkers selected from the group consisted of

(a) substituted or unsubstituted $-(CH_2)s-$;
(b) substituted or unsubstituted $-(CH_2)s-$, wherein 1-5 of $-CH_2-$ are replaced with O;
(c) substituted or unsubstituted $-(CH_2)s-$, wherein 1-2 of $-CH_2-$ are replaced with NR and optionally 1 - 2 of $-CH_2-$ are replaced with O; wherein R is H, protecting group or X'-L3-Z;
(d) substituted or unsubstituted $-(CH_2)s-$, wherein 1-4 of $-CH_2-$ are replaced with 5 membered unsaturated heterocyclic ring containing 1-4 heteroatoms selected from N, O, S and P;
(e) substituted or unsubstituted $-(CH_2)s-$, wherein 1-4 of $-CH_2-$ are replaced with 4-6 membered saturated heterocyclic ring containing 1-4 heteroatoms selected from N, O, S and P;
(f) substituted or unsubstituted $-(CH_2)s-$, wherein 1-4 of $-CH_2-$ are replaced with O and 4-6 membered saturated heterocyclic ring containing 1-4 heteroatoms selected from N, O, S and P;
(g) divalent 4-6 membered heterocyclic group containing 1-2 nitrogen atoms;

wherein, s is an integer of 5-20, or an integer of 5-50.

In another preferred embodiment, s is 3, 4, 5, 6, 7, 8, 9, 10, 11, 12, 13, 14, 15, 16, 17, 18, 19, or 20.

In another preferred embodiment, the substituted $-(CH_2)_s-$ means substituted with one or more groups selected from the group consisting of C1-C10 alkyl, C3-C8 cycloalkyl, $-NH_2$, -NHR, $-NR_1R_2$, wherein, each $R_1$ and $R_2$ is independently H, C1-C6 alkyl, C3-C8 cycloalkyl, protecting group, and R, wherein R is defined as above, and $R_1$ and $R_2$ are not the polypeptide element or target molecule T at the same time.

In another preferred embodiment, the L2 and/or L3 are selected from the group consisting of

(d1) substituted or unsubstituted $-(CH_2)s-$, wherein 1-4 of $-CH_2-$ are replaced with 5 membered unsaturated heterocyclic rings containing 1-3 heteroatoms selected from N, O, S and P;

(e1) substituted or unsubstituted $-(CH_2)s-$, wherein 1-4 of $-CH_2-$ are replaced with 4-6 membered saturated heterocyclic rings containing 1-3 heteroatoms selected from N, O, S and P;
(f1) substituted or unsubstituted $-(CH_2)s-$, wherein 1-4 of $-CH_2-$ are replaced with O and 4-6 membered saturated heterocyclic rings containing 1-3 heteroatoms selected from N, O, S and P.

In another preferred embodiment, the L2 and/or L3 are selected from the group consisting of

(d2) substituted or unsubstituted $-(CH_2)s-$, wherein 1-4 of $-CH_2-$ are replaced with 5 membered unsaturated heterocyclic rings containing 1- 2 N atoms;
(e2) substituted or unsubstituted $-(CH_2)s-$, wherein 1-4 of $-CH_2-$ are replaced with 4-6 membered saturated heterocyclic rings containing 1- 2 N atoms;
(f2) substituted or unsubstituted $-(CH_2)s-$, wherein 1-4 of $-CH_2-$ are replaced with O and 4 - 6 membered saturated heterocyclic rings containing 1- 2 N atoms.

In another preferred embodiment, s is 5-15, preferably 6-10.
In another preferred embodiment, whenX is NH or NR, X and part of L2 chainto which X is attached are combined together to form a 4-8 membered heterocyclic ring,

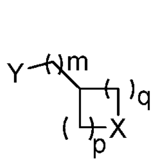 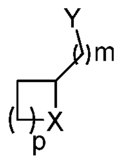

wherein Y and R are defined as above; m is an integer of 1-15; each p and q is indenpendently 0, 1, 2, or 3, and p + q≥2.
In another preferred embodiment, L1 is selected from the group consisting of

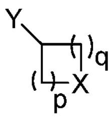 , 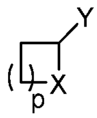 , 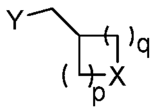 and 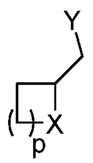

wherein
X and Y are defined as above;
each p and q is indenpendently an integer of 1, 2, or 3.
In another preferred embodiment, L1 is

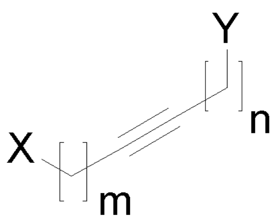

wherein each m and n is independently an integer of 1 - 15.
In another preferred embodiment, L1 is a divalent linker formed from a group selected from the following group by loss of two hydrogen atoms at any position:

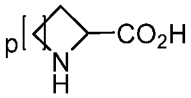 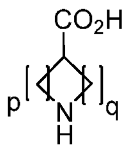 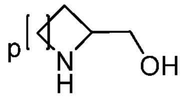 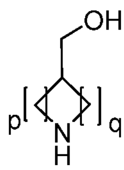 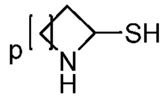 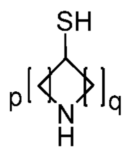

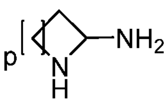 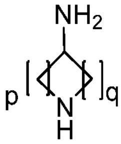 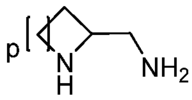 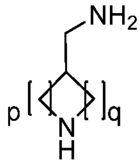 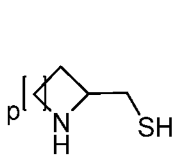 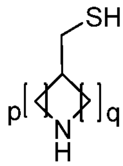

in the formula, p and q are defined as above.

In another preferred embodiment, L1 is derived from

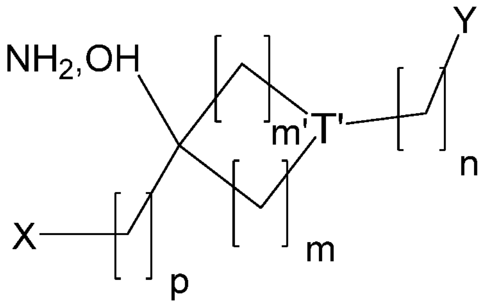

wherein m' and m are each independently 0, 1, 2, 3, 4, or 5(preferably 0, 1, 2, or 3), and m' and m are not 0 at the same time; each p and n is independently an integer of 1 - 15;
in the formula, "$NH_2$,OH" means the terminal is $NH_2$ or OH;
T' is N or $CR_b$; wherein $R_b$ is a group selected from the group consisting of H, and substituted or unsubstituted C1-C6 alkyl.

In another preferred embodiment, L1 is selected from the group consisting of

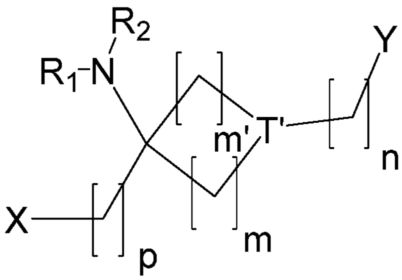 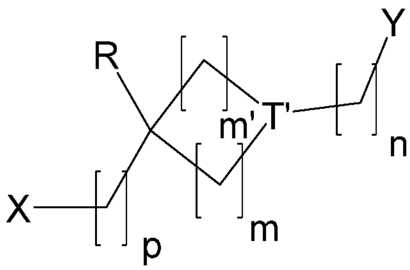 ;

wherein m' and m are each independently 0, 1, 2, 3, 4, or 5(preferably 0, 1, 2, or 3), and m' and m are not 0 at the same time; each p and n is independently an integer of 1 - 15;
the definitions of T', R, $R_1$ and $R_2$ are as above.

In another preferred embodiment, L1 is selected from the group consisting of

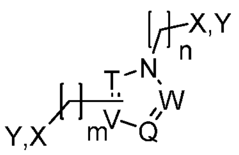 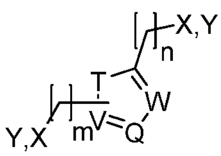 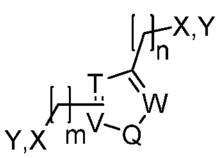 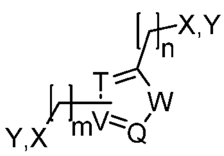

in the formula, "Y, X" means this terminal is X or Y; with the proviso that one end is X, and the other end is Y;
X and Y are defined as above, each m and n is independently an integer of 1 - 15;
T, V, Q and W are each independently CH, C=O, S, O, NH or NR; R is C1-C10 alkyl, -(C=O)NH-,-(C=O)NH-R', -NH(C=O)-R', $-SO_2$-R', $-NHSO_2$-R', - $SO_2NH$-R', -SO-R', -NHSO-R', -SONH-R', $-PO_3$-R', - NHCOO-R', -COO-R', -NH-CO-NH-R', -NH-CO-O-R', or N-'X'-L3-Z (i.e. -X'-L3-Z is a substituent on the N); wherein the definition of L3 is as described above, and Z is a polypeptide element or target molecule T; R' is defined as above.

In another preferred embodiment, at least one of T, V, Q and W is N-'X'-L3-Z.

In another preferred embodiment, m+n≤20.

In another preferred embodiment, m+n≥2.

In another preferred embodiment, L1 is selected from the group consisting of

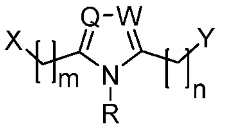 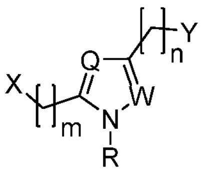 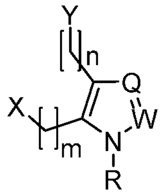

in each formula, X and Y are defined as above, each m and n is indenpendently an integer of 1 - 15;

R is X'-L3-Z, and L3 and Z are defined as above;

Q and W are each independently CH, C=O, S, O, NH or NR; R is -(C=O)-R', (C=O)NH-R', -NH(C=O)-R', $-SO_2$-R', $-NHSO_2$-R', $-SO_2$NH-R', -SO-R', -NHSO-R', -SONH-R', $-PO_3$-R', -NHCOO-R', -COO-R', -NH-CO-NH-R' or -NH-CO-O-R'; R' is defined as above.

In another preferred embodiment, Z is the polypeptide element selected from antibody, receptor, ligand, or active fragment thereof.

In another preferred embodiment, Z is ADC.

In another preferred embodiment, m+n≤20.

In another preferred embodiment, m+n≥2.

In another preferred embodiment, L1 is

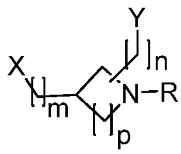

wherein X, Y and R are defined as above;

each m and n is indenpendently an integer of 1-15; p is 1, 2, or 3.

In another preferred embodiment L1 is selected from the group consisting of

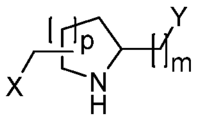 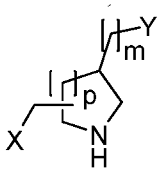

in each formula, X and Y are defined as above, m is an integer of 1 - 15; p is 1, 2 or 3.

In another preferred embodiment, the linker (L1) is selected from the group consisting of

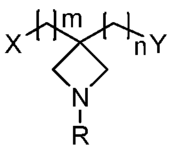 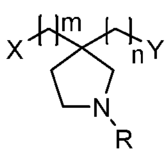 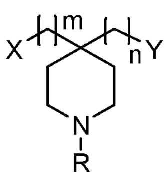 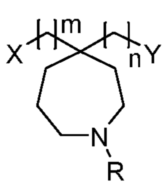 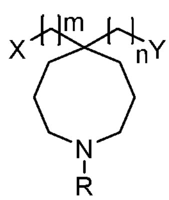

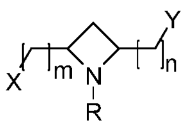 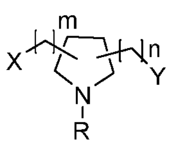 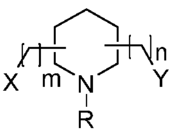 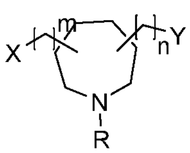 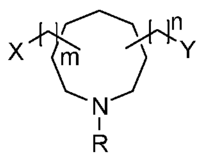

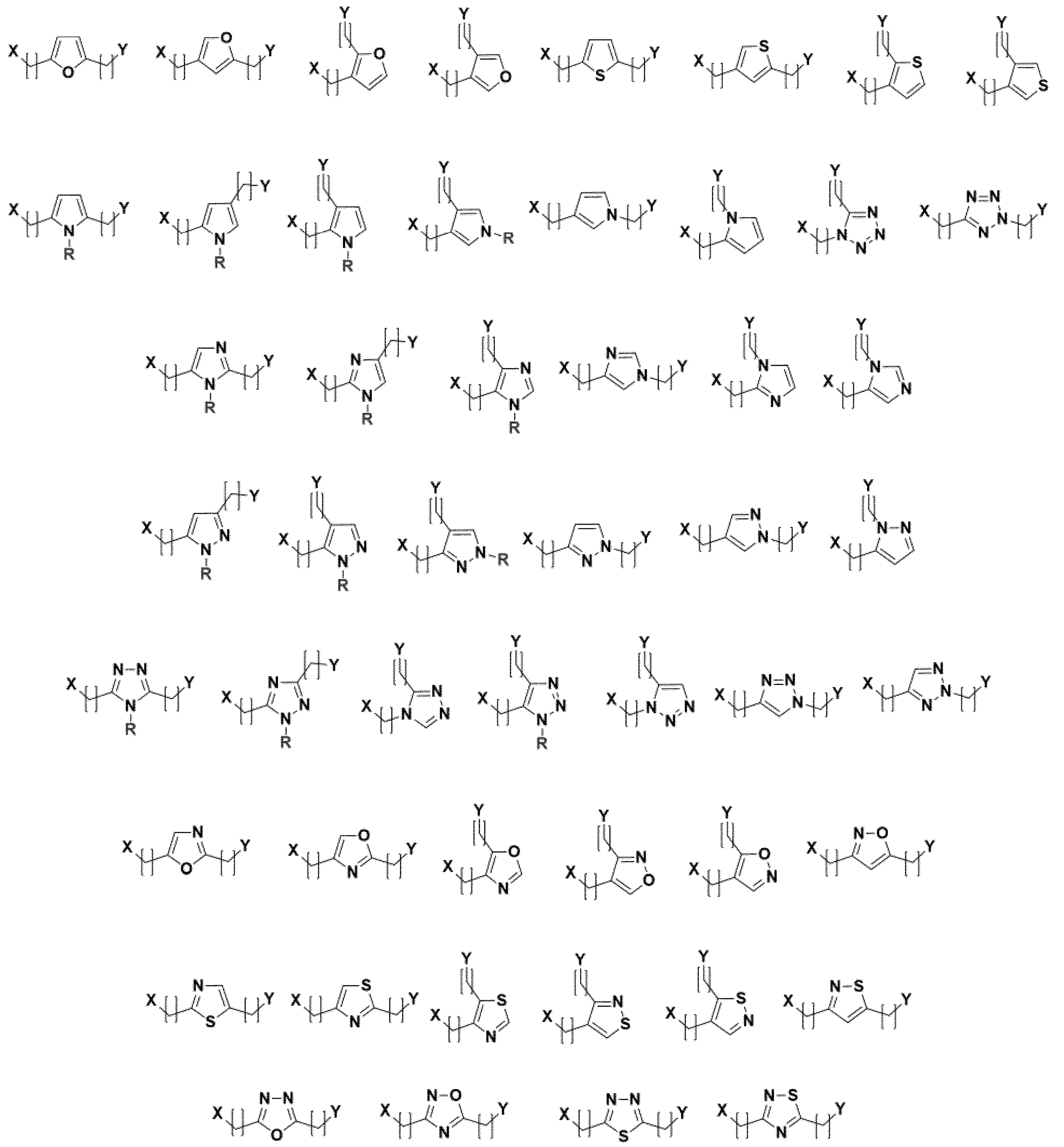

*in the formula, X, Y and R are defined as above, each m and n is indenpendenly an integer of 1 - 15.*

In another preferred embodiment, m+n≤20.

In another preferred embodiment, m+n≥2.

In another preferred embodiment, L1 is selected from the group consisting of

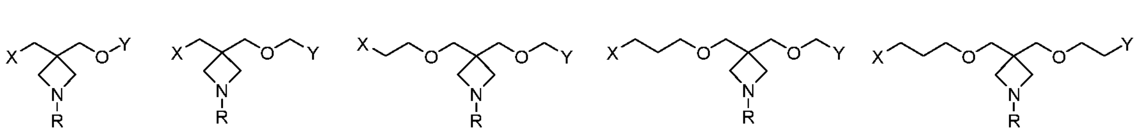

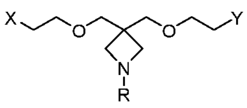 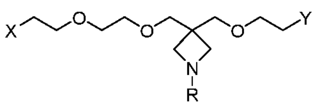 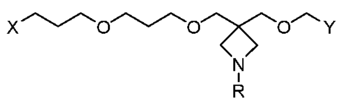 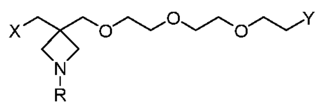

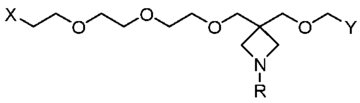 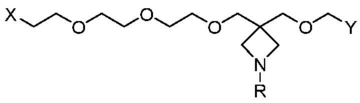 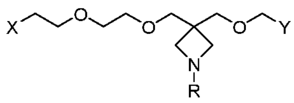

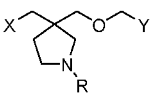 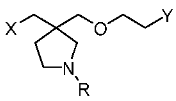 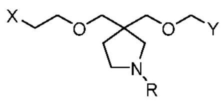 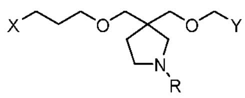 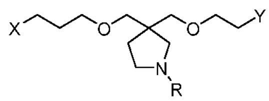

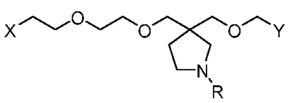 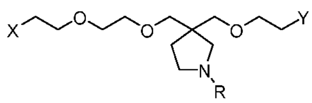 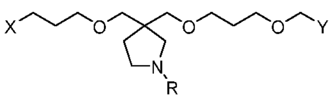 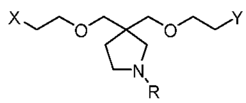

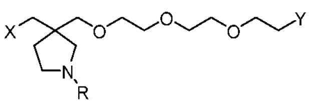 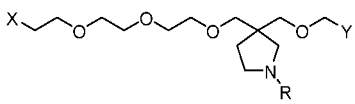 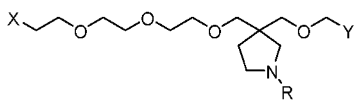

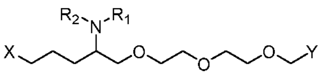 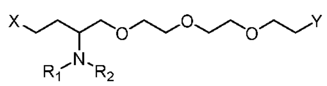 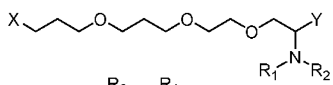

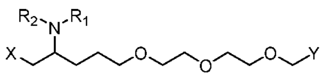 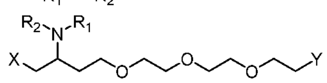 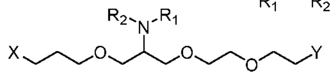

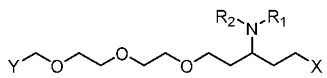 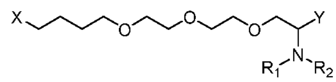 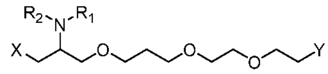

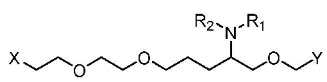 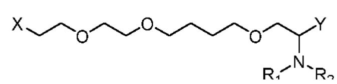 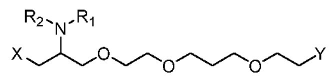

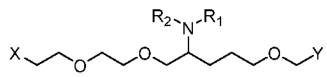 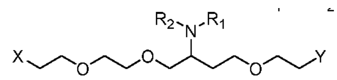 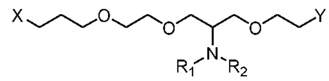

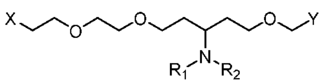 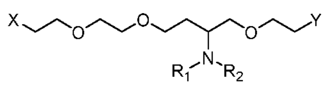 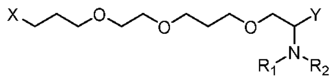

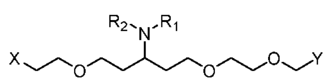 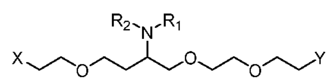 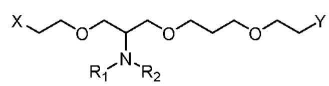

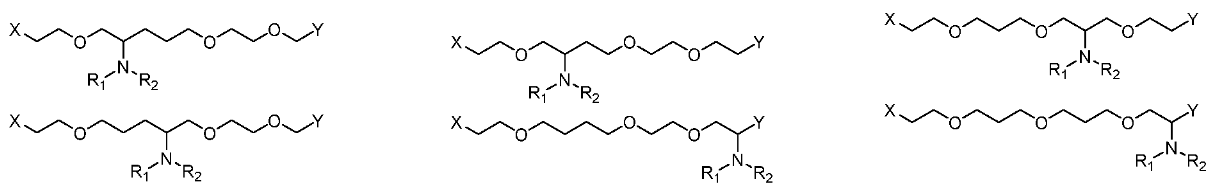

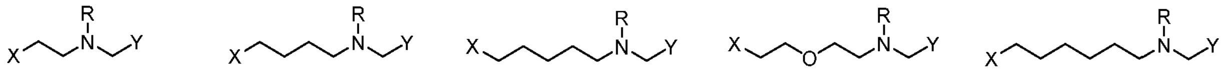

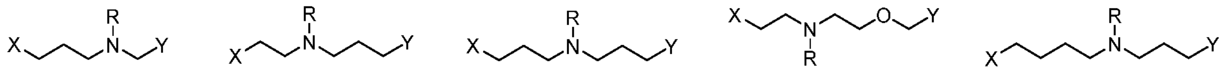

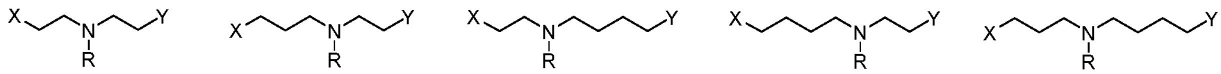

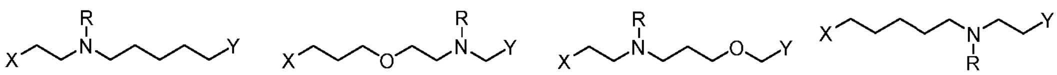

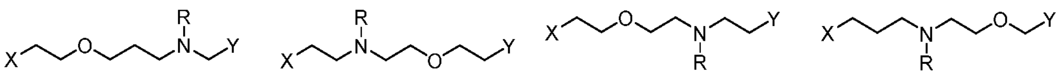

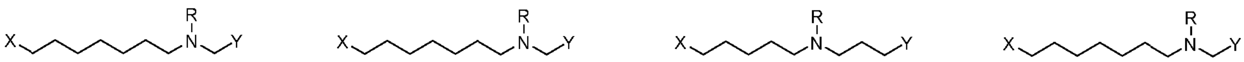

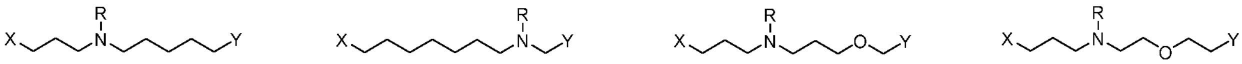

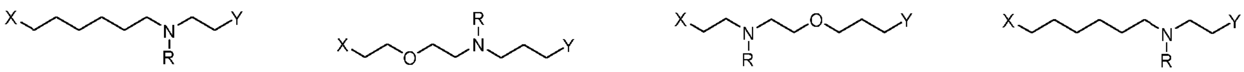

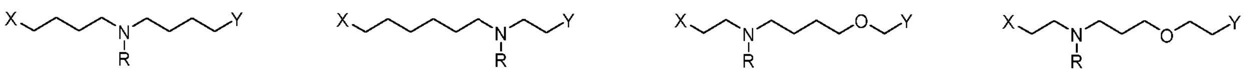

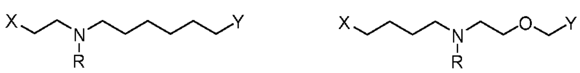

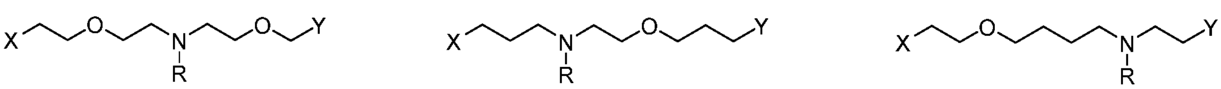

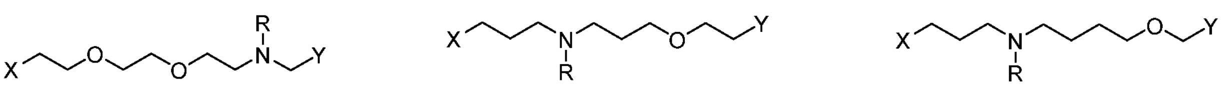

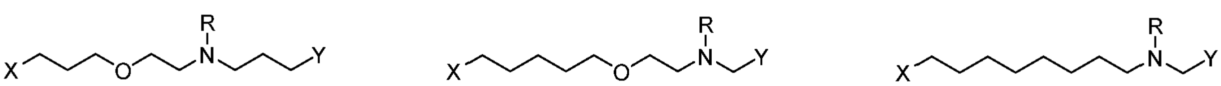

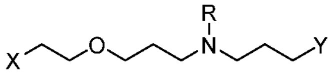 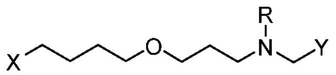 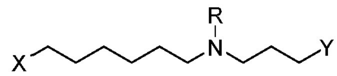

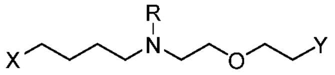 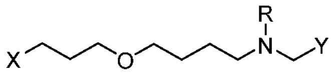 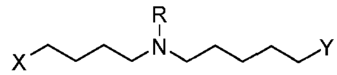

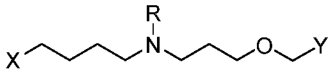 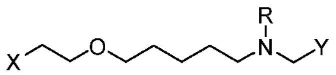 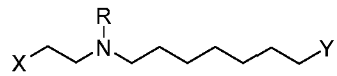

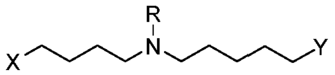 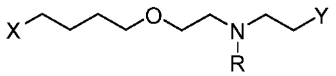 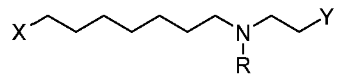

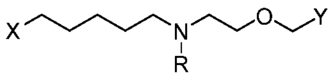 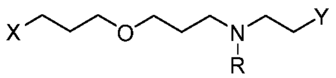 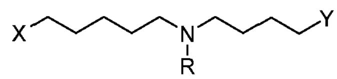

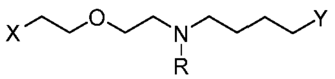 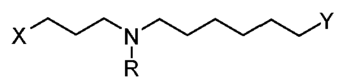

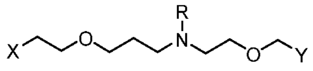 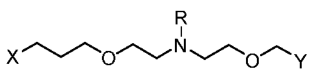 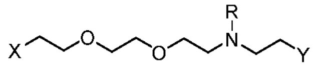

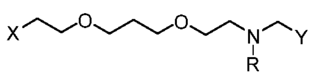 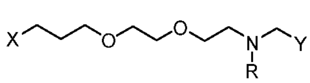 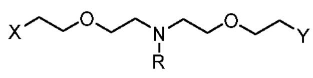

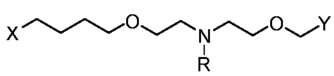 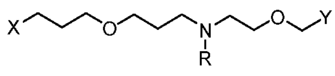 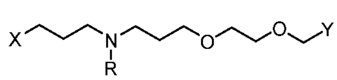

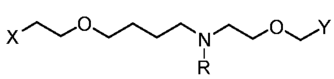 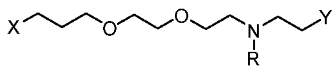 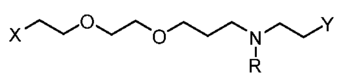

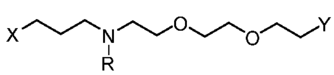 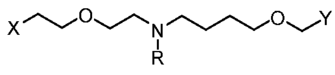 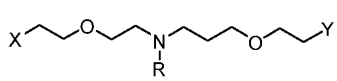

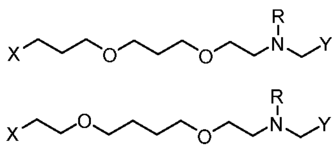 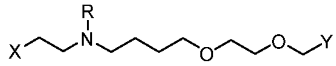 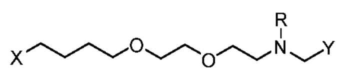

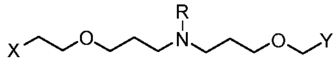 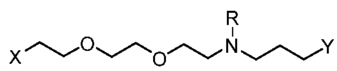

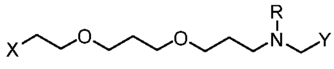 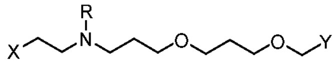 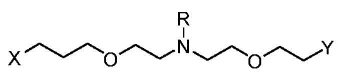

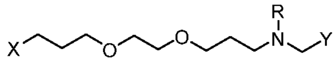 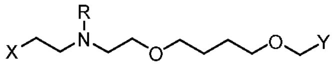 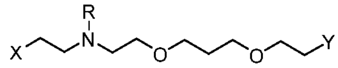

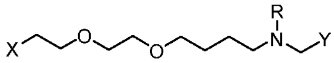 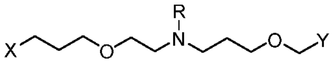 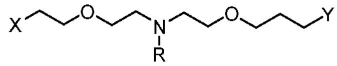

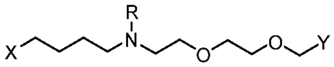 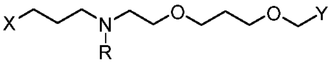 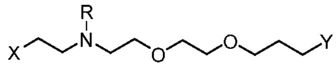

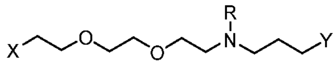 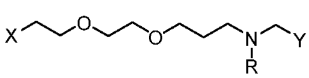 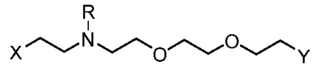

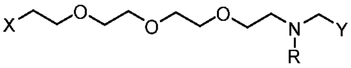 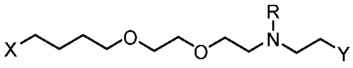 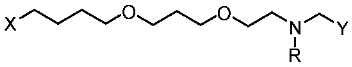

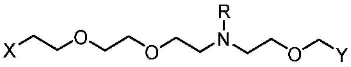 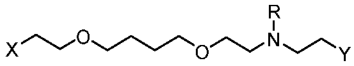 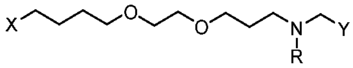

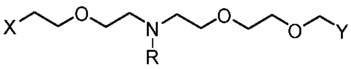 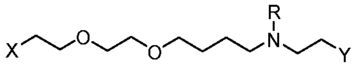 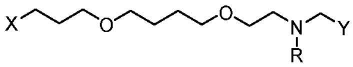

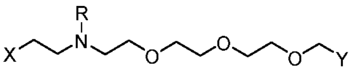 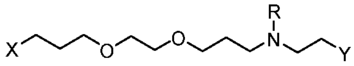 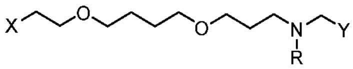

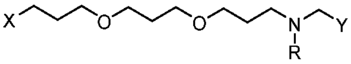 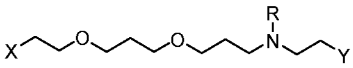 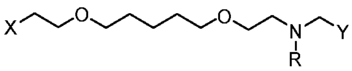

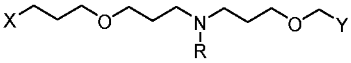 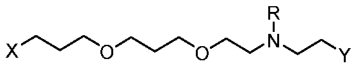 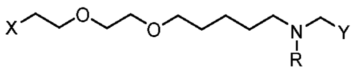

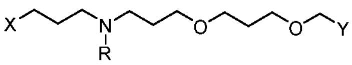 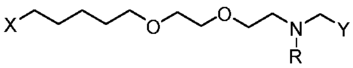

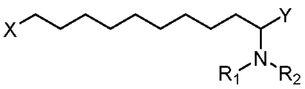 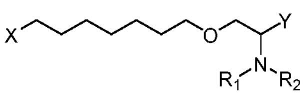 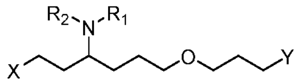

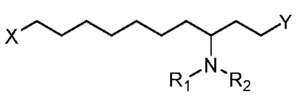
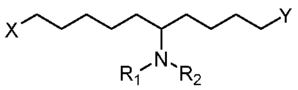
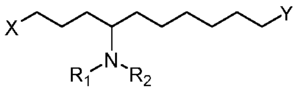
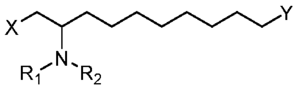
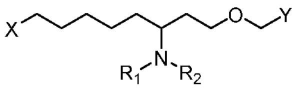
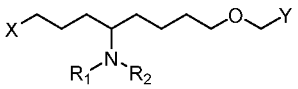
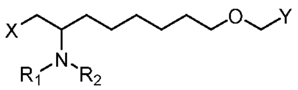
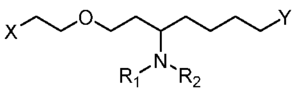
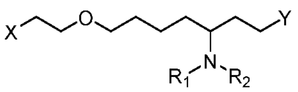
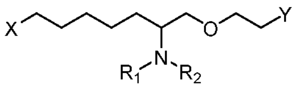
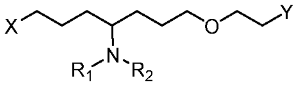
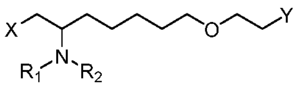
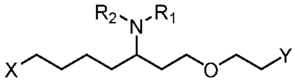
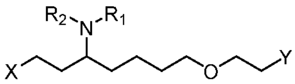
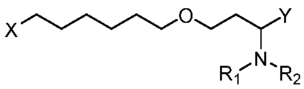
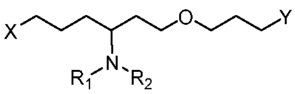
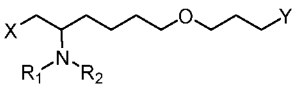
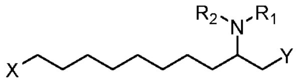
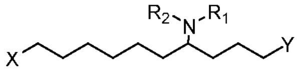
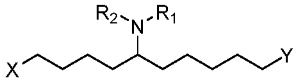
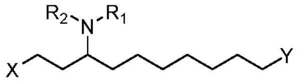
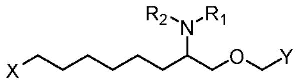
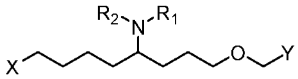
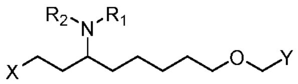
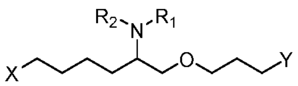
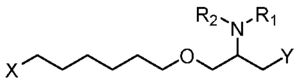

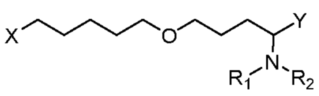
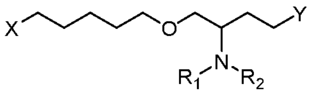
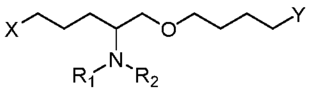
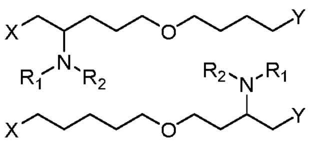
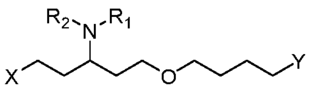
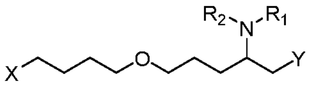
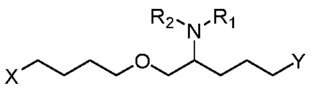
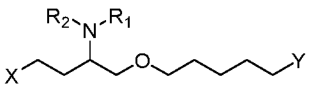

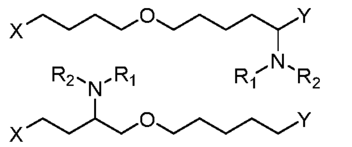
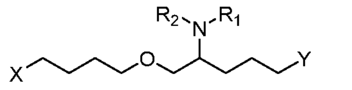
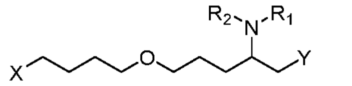
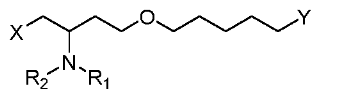
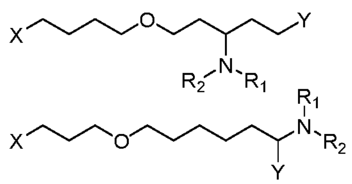
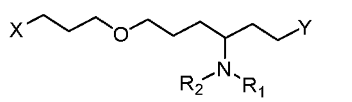
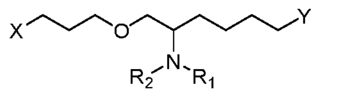

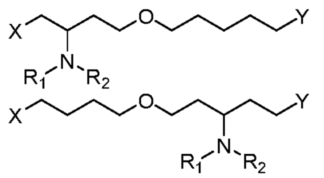
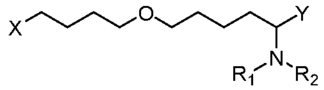
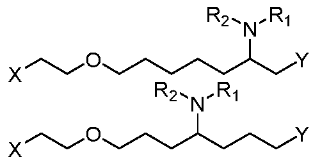
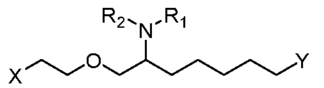
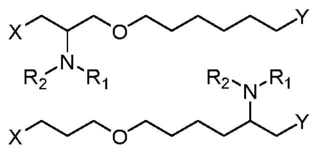
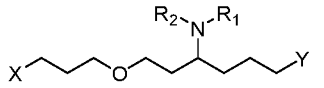
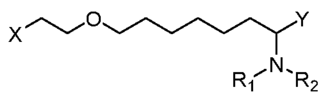

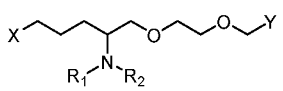
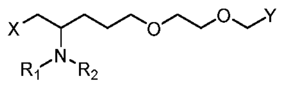
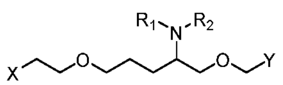
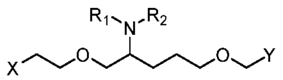
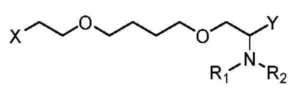
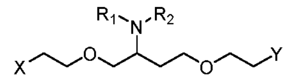

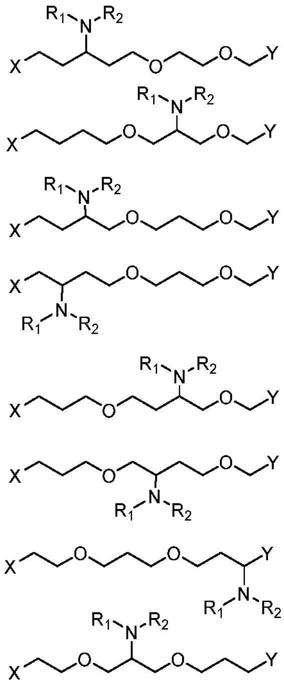
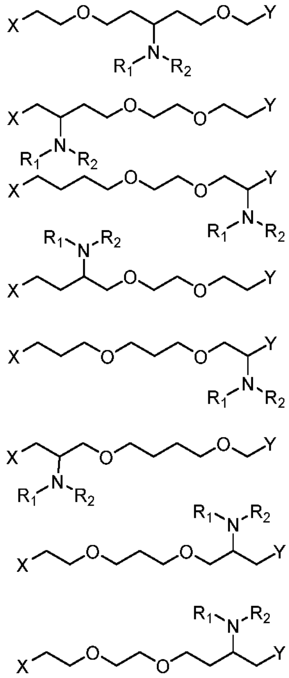
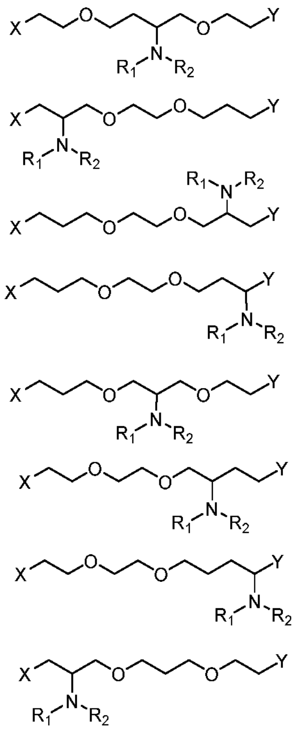

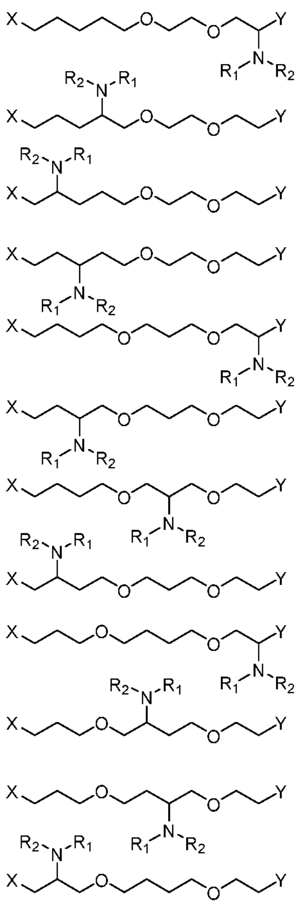
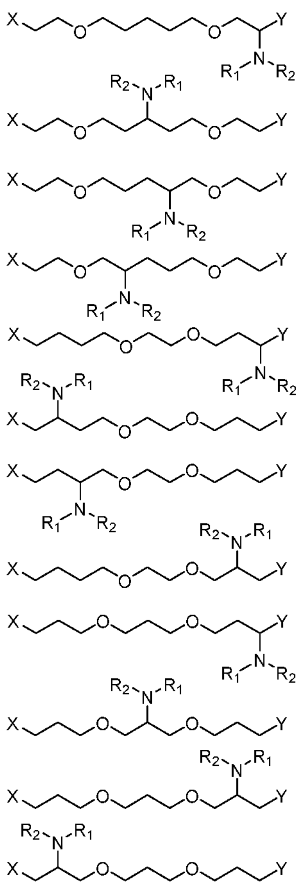
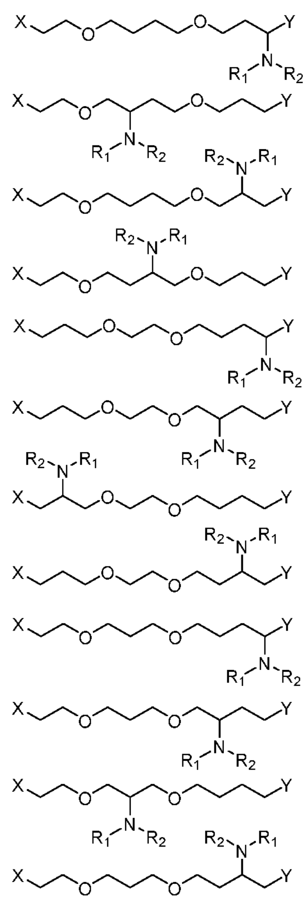

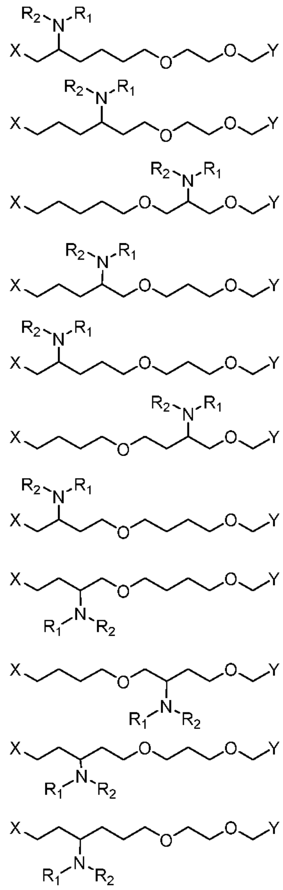

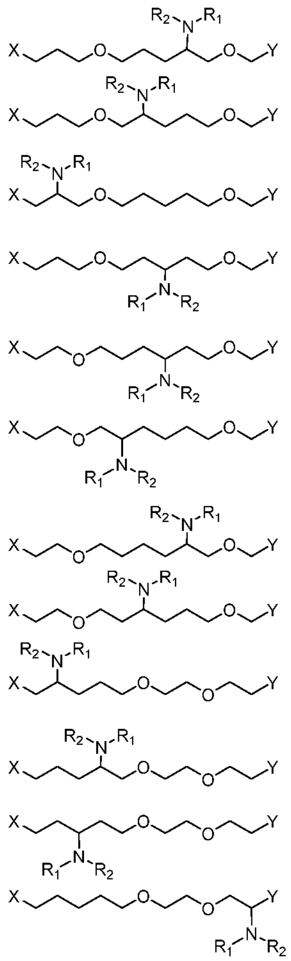

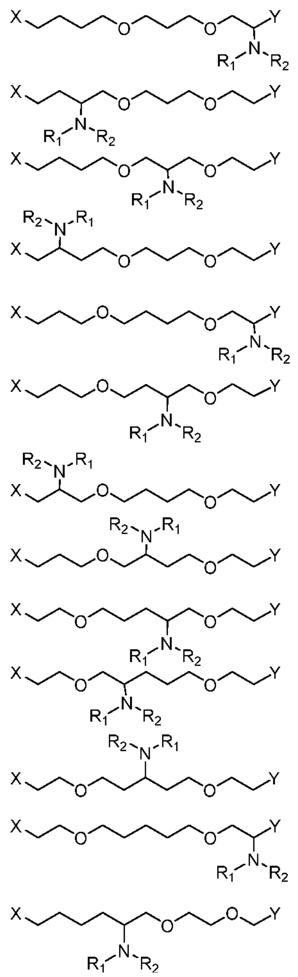

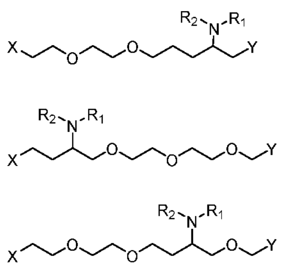

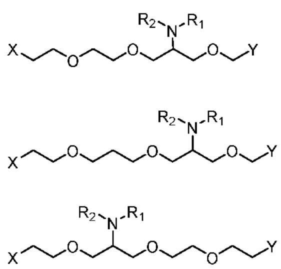

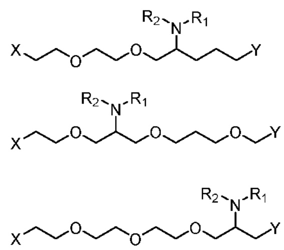

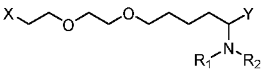
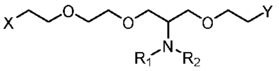
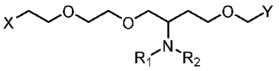
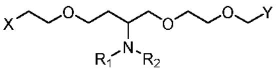
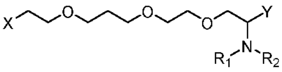
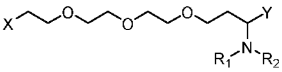
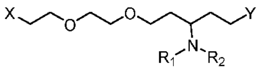
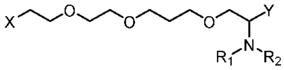
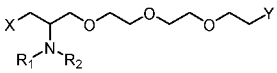
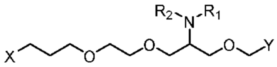
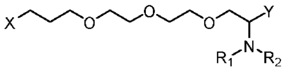
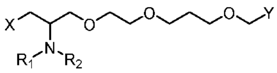
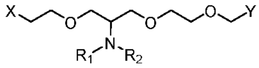
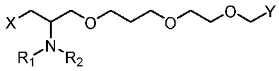
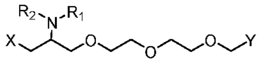
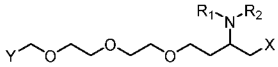
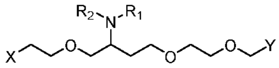
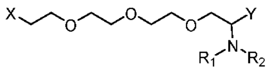
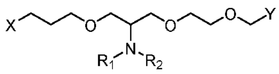
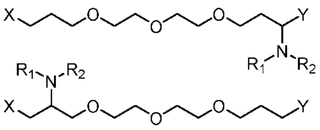
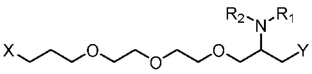
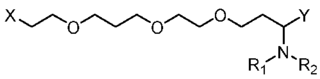
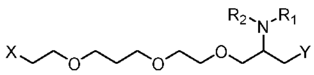
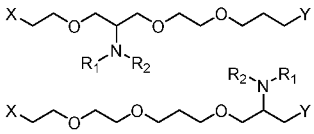
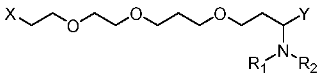
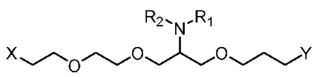
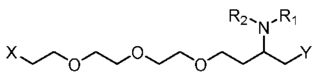
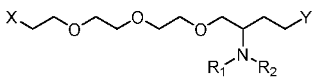
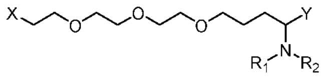

*wherein, each $R_1$ and $R_2$ is indenpendently H, C1-C6 alkyl, C3-C8cycloalkyl, protecting group, or R, wherein R is defined as above, and $R_1$ and $R_2$ are not the polypeptide element at the same time.*

In another preferred embodiment, L1 is a group selected from the group consisting of

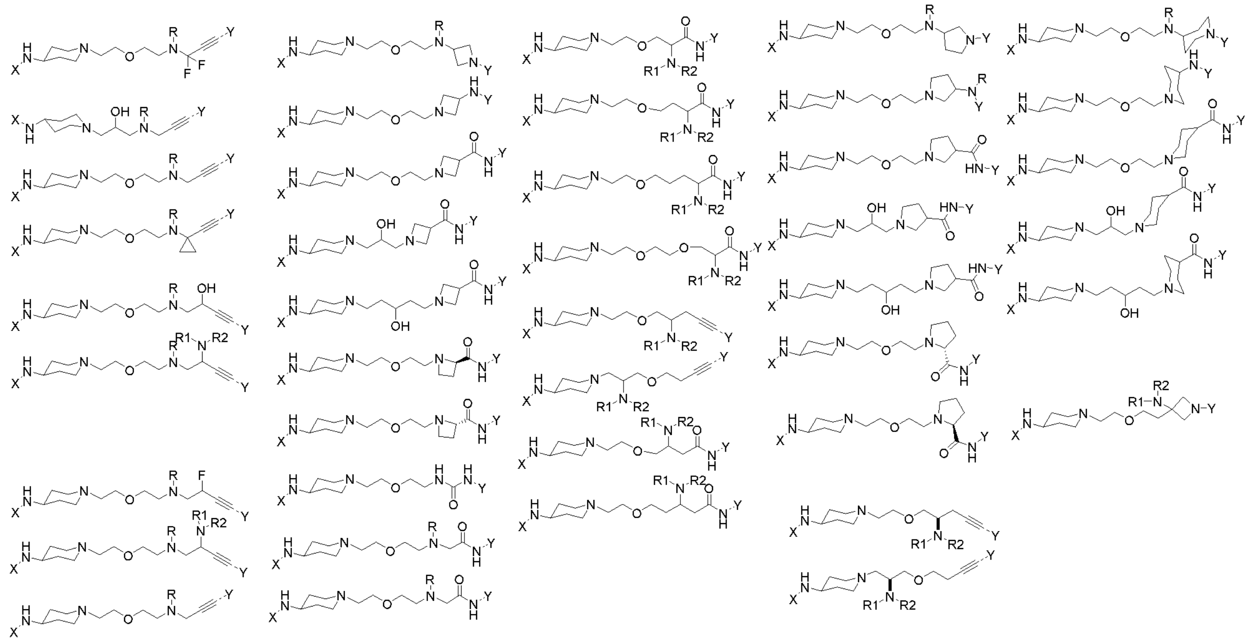

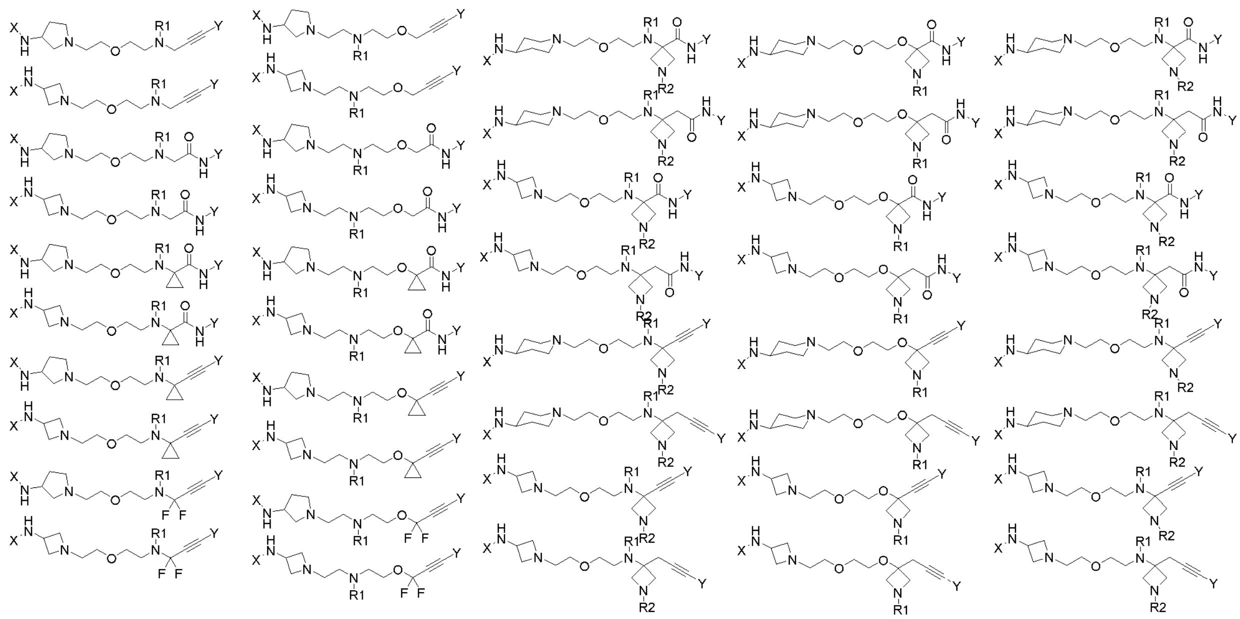

;

wherein, the definitions of R, $R_1$ and $R_2$ are as stated above.

In another preferred example, L1 is a group selected from the group consisting of (active groups such as -NH-, -CH(OH)-, C($NH_2$) on the main chain of L1 from the following group can be coupled with Z by linker moiety thereby forming X'-L3-Z, or directly coupled with Z):

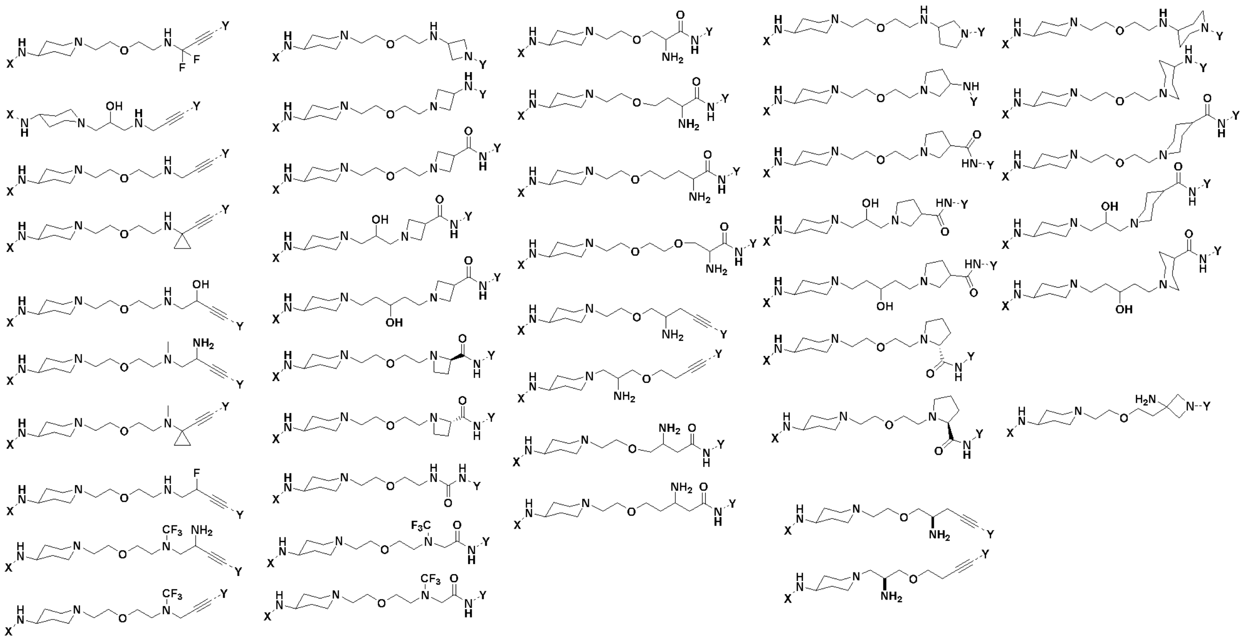

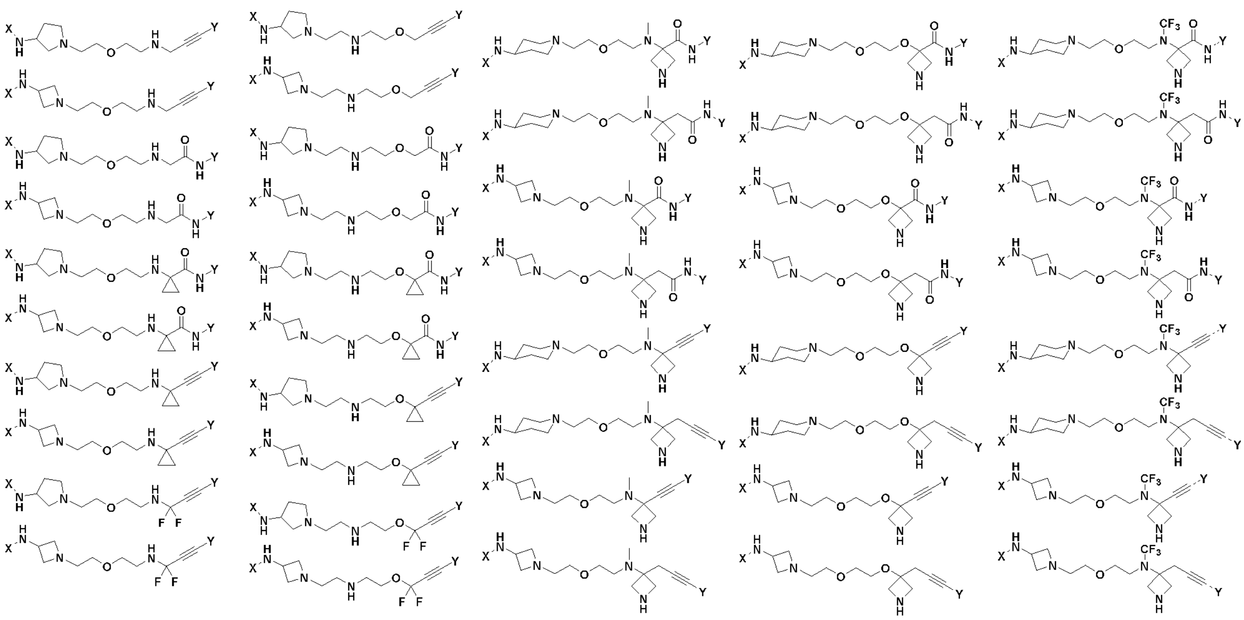

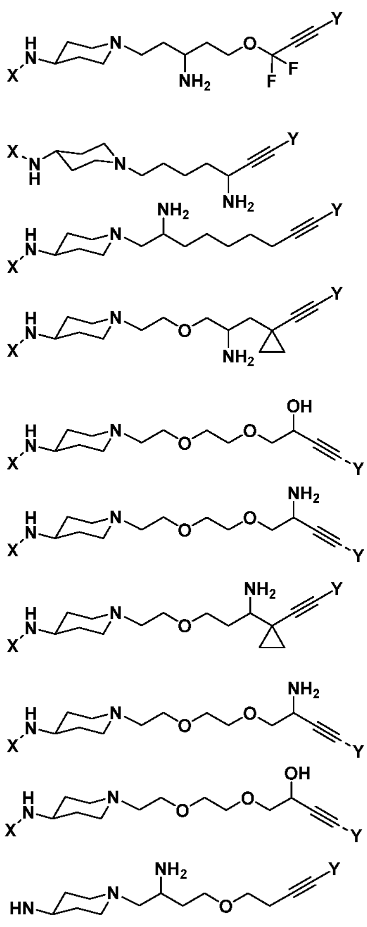

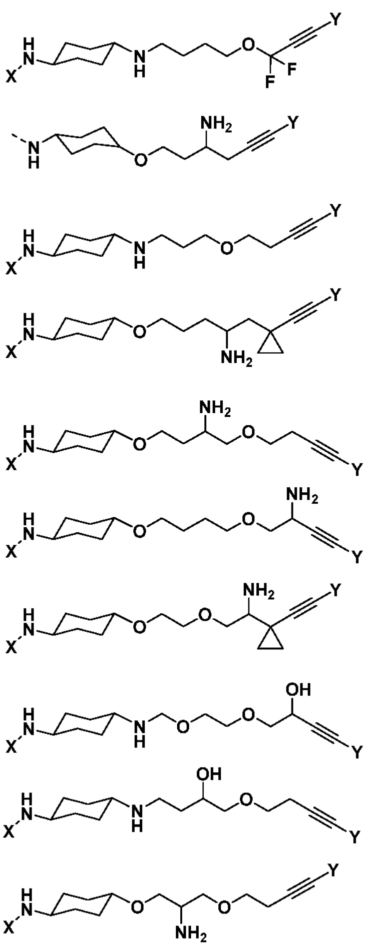

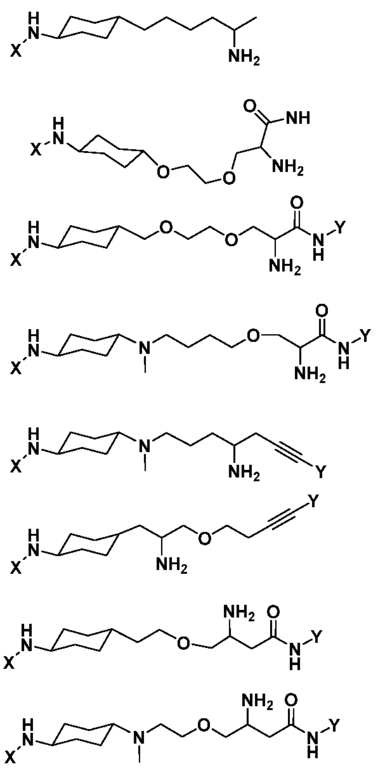

In another preferred embodiment, L1 is a divalent linker derived from a group selected from the following group of (preferably, by loss two of hydrogen atom, hydroxyl, protecting group (boc) at any position, and/or azide_cyclization):

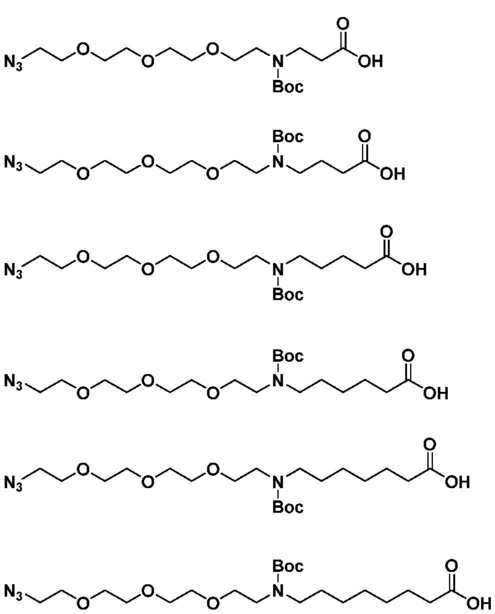

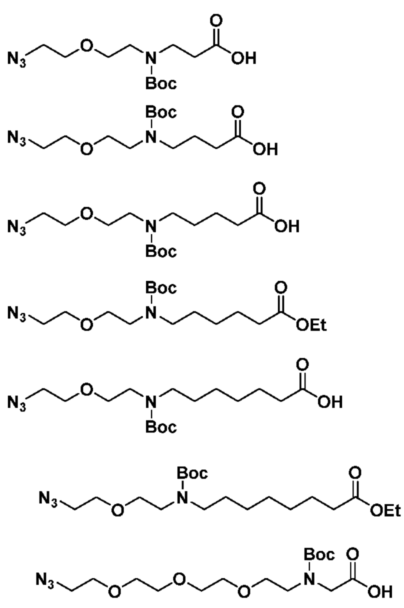

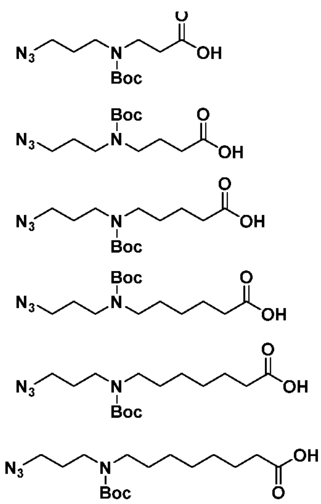

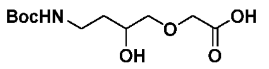
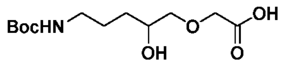
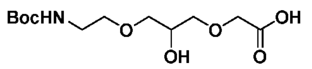
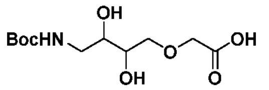
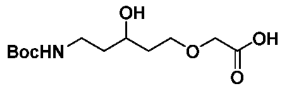
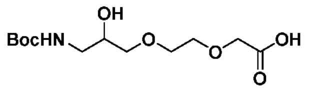
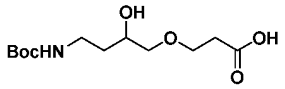
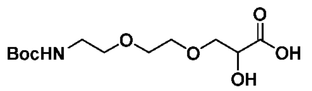
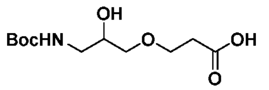
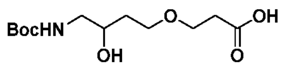
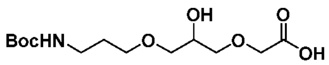
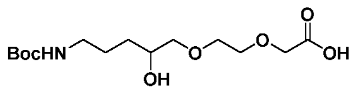
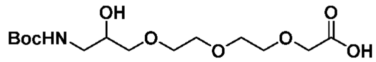
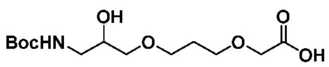
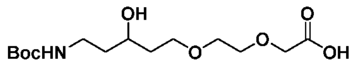
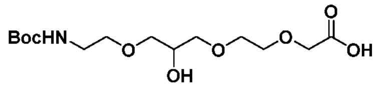
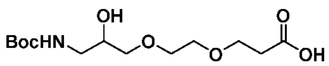
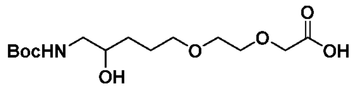
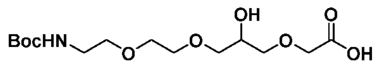
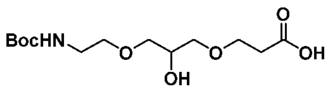
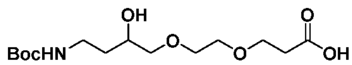
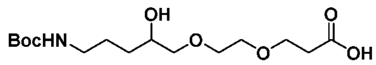
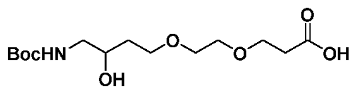
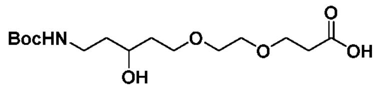
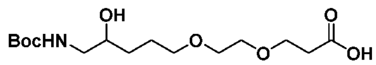
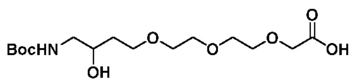
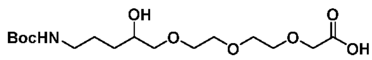
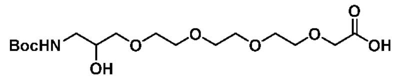
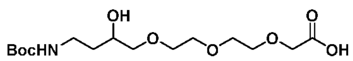
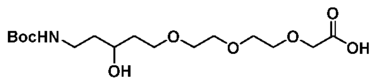
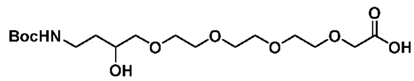
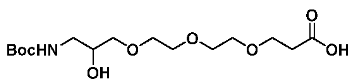
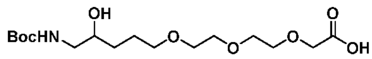
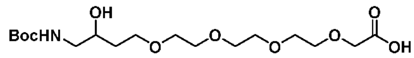
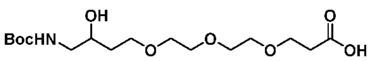
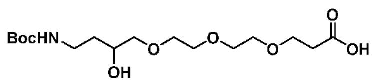
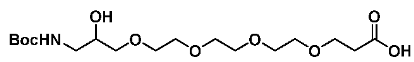
.

In another preferred embodiment, the target molecule A and T are each independently selected from the group consisted of folic acid, HSP90, TINFRm, TNFR2, NADPH oxidase, BcllBax, C5a receptor, HMG-CoA reductase, PDE I-V, Squalene cyclase inhibitors, CXCR1, CXCR2, Nitric oxide (NO)synthase, cyclo-oxygenase 1-2, 5HT receptors, dopamine receptors, G-proteins, Gq, Histamine receptors, Lipoxygenases, Tryptase serine protease, Thymidylate synthase, Purine nucleotide phosphorylase, GAPDH trypanosomal, Glycogen phosphorylase, Carbonic anhydrase, Chemokine receptors, JAW STAT, RXR and the analogues thereof, HIV 1 protease, HIV 1 integrase, Influenza, hepatitis B reverse transcriptase, neuraminidase, Sodium channel, MDR, protein P-glycoprotein, Tyrosine kinases, CD23, CD124, TK p56 lck, CD4, CD5, IL-1 receptor, IL-2 receptor, TNF-aR, ICAM1, Ca+ channels, VCAM, VLA-4 integrin, VLA-4 integrin, Selectins, CD40/40L, Newokinins and receptors, Inosine monophosphate dehydrogenase, p38 MAP kinase, Interleukin-1 converting enzyme, Caspase, HCV NS3 protease, HCV-NS3 RNA helicase, Glycinamide ribonucleotide formyl transferase, rhinovirus 3C protease, HSV-I, CMV, ADP-polymerae, CDK, VEGF, oxytoxin receptor, msomalmsomal transfer protein inhibitor, Bile acid transfer protein inhibitor, 5-a reductase, Angiotensin 11, Glycine receptors, noradrenaline reuptake receptor, Endothelin receptors, Neuropeptide Y and receptors, Estrogen receptors, AMP, AMP deaminase, ACC, EGFR, Farnesyltransferase.

In another preferred embodiment, the polypeptide element is antibody; preferably, the antibody comprises nanobody and/or small molecule antibody (minibody).

In another preferred embodiment, the antibody can bind to an antigen or receptor selected from the group consisting of DLL3, EDAR, CLL1, BMPR1B, E16, STEAP1, 0772P, MPF, 5T4, NaPi2b, Sema 5b, PSCA hlg, ETBR,

MSG783, STEAP2, TrpM4, CRIPTO, CD21, CD22, CD79b, CD19, CD37, CD138, FcRH2, B7-H4, HER2, NCA, MDP, IL20Rα, Brevican, EphB2R, ASLG659, PSCA, GEDA, BAFF-R, CD79a, CXCR5, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, Tyrosinase, TMEM118, GPR172A, MUC1, CD70, CD71, MUC16, methothelin, FOLR1, Trop-2, gpNMB, EGFR, ENPP3, PSMA, CA6, GPC-3, PTK7, CD44, CD56, TIM-1, Cadherin-6, ASG-15ME, ASG-22ME, CanAg, AXL, CEACAM5, EphA4, cMet, FGFR2, FGFR3, CD123, Her3, LAMP1, LRRC15, TDGF1, CD66, CD25, BCMA, GCC, Noch3 and CD33.

In another preferred embodiment, the E3 ligase ligand is selected from the group consisting of the $A^1$ groups in WO2017/176957 A1 (such as, A-10, A-11, A-15, A-28, A- 48, A-69, A-85, A-93, A-98, A-99 or A-101):

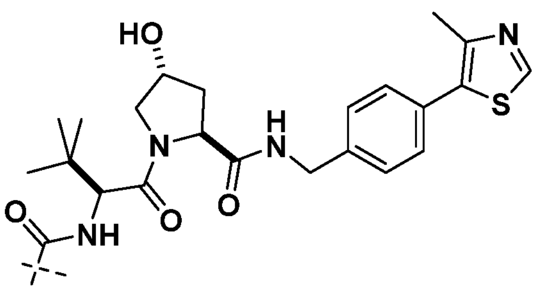

**VHL Ligand**

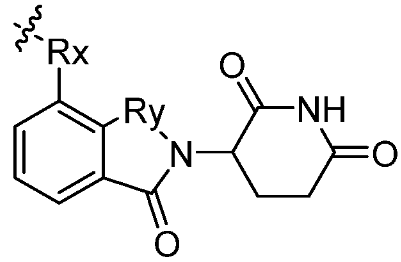

**CRBN Ligand**

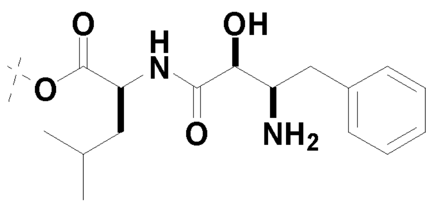

**cIIAP1 Ligand**

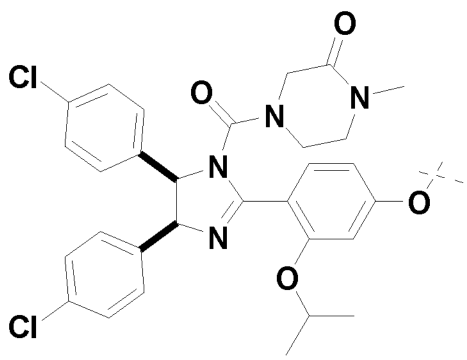

**MDM2 Ligand**

in each formula, a dotted line indicates the position connected with other parts (i.e., the position connected with -L1-A); wherein Rx is selected from the group consisting of none, NH, NH-CO, O, S, SO, $SO_2$, $SO_2(NH_2)NH$, C1~C4 alkylene, C2~C5 alkenylene, C2~C5 alkynylene; $R_y$ is C=O, C=S or $CH_2$.

In another preferred embodiment, the conjugate includes monomer or polymer of the conjugate. Preferably, the polymer of the conjugate shares one or more Z.

In another preferred embodiment, the conjugate of formula I is a conjugate of formula III

$$Z\text{-}(L3\text{-}R_a)_t \qquad \text{(III)}$$

wherein, t=1 to 15 (preferably, 1 to 8; more preferably, 2 to 7);

Z is described as above, preferably Z is a polypeptide element, more preferably, is an antibody;

$R_a$-L3 is the remaining part of the conjugate of formula I after the loss of Z.

Preferably, $R_a$ is a monovalent group derived from the specific compounds in Tables B1 to B11 (wherein, said derived means a monovalent group formed by the specific compounds shown in Tables B1 to B11 losing a hydrogen from NH or $NH_2$ on the main chain or the branch chain of the linker group).

In another preferred embodiment, Ab is connected with L3 of formula III (preferably,

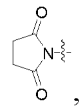

-NH-, -NHR or -$NH_2$ group in L3), through amino acid at N-terminal or C-terminal, or a side chain of amino acid (preferably, a side chain of amino acid selected from the group consisting of Lys, and Cys), or a sulfhydryl group formed by reducing and opening disulfide bond.

In another preferred embodiment, the definitions of each group in the conjugate of formula I are the same as the corresponding group of the specific compound in Tables A1 and A2, Table B1 to Table B11, Table C and Table D.

In the second aspect of the present invention, a pharmaceutical composition is provided, wherein the pharmaceutical composition comprises the conjugate described in the first aspect and pharmaceutically acceptable carrier.

In the third aspect of the present invention, a preparation method of the conjugate as described in the first aspect is provided, wherein the reaction route of the method is shown as follows:

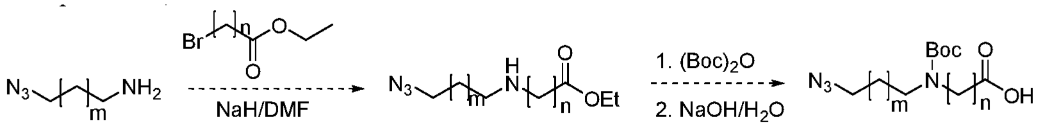


;

or

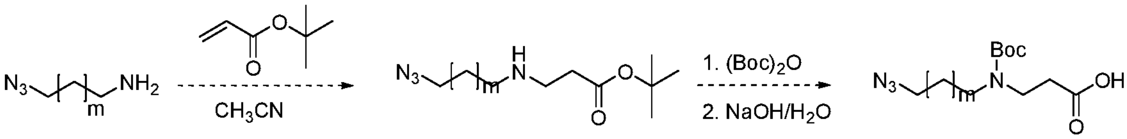


wherein m, and n are defined as above.

In the fourth aspect of the present invention, a use of the conjugate as described in the first aspect in the preparation of pharmaceutical compositions for the treatment of diseases associated with excess of the target protein is provided.

In the fifth aspect of the present invention, a method for reducing the content of target proteins in a cell is provided, wherein the cell is contacted with the conjugate as described in the first aspect, thereby reducing the content of the target proteins in the cell.

In another preferred embodiment, the method is a method *in vitro.*

In another preferred embodiment, the method is non-diagnostic and non-therapeutic.

In the sixth aspect of the present invention, a method for reducing the content of target proteins in a subject or treating diseases associated with excess of target proteins is provided, comprising step ofadministering the conjugate as described in the first aspect to a subject in need thereof.

It should be understood that within the scope of the present invention, the above technical features of the present invention and the technical features specifically described in the following (e.g/, examples) can be combined with each other, thereby forming a new or preferred technical solution. Due to space limitations, it will not be repeated herein.

## DESCRIPTION OF THE DRAWINGS

Figure 1 is a schematic diagram showing the action principle of degrading the target protein by the Targeted Enzyme Degradation platform (TED).
Figure 2 shows the result of biological test of the conjugate in the examples.
Figure 3 shows the changes of tumor volume of nude mice in animal experiments.
Figure 4 shows the changes of weight of nude mice in animal experiments.

## DETAILED DESCRIPTION OF THE INVENTION

After extensively and deeply researching, the inventors developed a TED conjugate with novel structure for the first time, the conjugate of the present invention has a structure of formula I. In addition, the conjugate of the present invention is very suitable for further connected with polypeptide element (especially antibody, protein ligand) or after further connecting with polypeptide element and the like, making the conjugate of the present invention possess excellent dual targeting properties, improve drug selectivity, implement more precise degradation of pathogenic proteins, reduce the possible systemic toxicity induced by non-specific degradation, and is possible to overcome the difficulties encountered in drug absorption and metabolism, and eliminate the possibility for producing drug resistance. The present invention was completed on this basis.

## TERMS

As used herein, the term "compound of the present invention", "conjugate of the present invention", and "TED conjugate of the present invention" are used interchangeably and refer to the compound or the conjugate of formula I described in the first aspect of the present invention.

As used herein, the term "alkyl", by itself or as part of another substituent, means, unless otherwise stated, a straight or branched chain hydrocarbon radical, having the number of carbon atoms designated (i.e. $C_{1-8}$ means 1 - 8 carbons).Examples of alkyl include methyl, ethyl, *n*-propyl, isopropyl, *n*-butyl, *t*-butyl, *iso*butyl, *sec*-butyl, *n*-pentyl, *n*-hexyl, *n*-heptyl, *n*-octyl, and the like. The term "alkenyl" refers to an unsaturated alkyl group having one or more double bonds. Similarly, the term "alkynyl" refers to an unsaturated alkyl having one or more triple bonds. Examples of such unsaturated alkyl include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "cycloalkyl" refers to hydrocarbon ring having the indicated number of ring atoms (e.g., $C_{3-6}$ cycloalkyl) and being fully saturated or having no more than one double bond between ring vertices. "Cycloalkyl" is also meant to refer to bicyclic and polycyclic hydrocarbon rings such as, bicyclo[2.2.1]heptane, bicyclo[2.2.2]octane, etc. The term "heterocycloalkyl" refers to a cycloalkyl that contain from one to five heteroatoms selected from N, O, and S, wherein the nitrogen and sulfur atoms are optionally oxidized, and the nitrogen atom(s) are optionally quaternized. The heterocycloalkyl may be a monocyclic, a bicyclic or a polycylic ring system. Non limiting examples of heterocycloalkyl include pyrrolidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrhydrothiophene, quinuclidine, and the like. The heterocycloalkyl can be attached to the rest of the molecule via a ring carbon or a heteroatom. For terms such as cycloalkylalkyl and heterocycloalkylalkyl, it is meant that a cycloalkyl or a heterocycloalkyl is attached through an alkyl or alkylene linker to the remainder of the molecule. For example, cyclobutylmethyl - is a cyclobutyl ring that is attached to a methylene linker to the remainder of the molecule.

The term "alkylene" by itself or as part of another substituent means a divalent radical derived from an alkane, for example $-CH_2CH_2CH_2CH_2-$.Typically, an alkyl (or alkylene) will have from 1 to 24 carbon atoms, with those groups having 10 or fewer carbon atoms being preferred in the present disclosure. A "lower alkyl" or "lower alkylene" is a shorter chain alkyl or alkylene, generally having four or fewer carbon atoms. Similarly, "alkenylene" and "alkynylene" refer to the unsaturated forms of "alkylene" having double or triple bond, respectively.

Unless otherwise specified, the term "heteroalkyl" by itself or in combination with other terms refers to a stable linear or branched or cyclic hydrocarbon group or a combination thereof, consisting of a specified number of carbon atoms and 1 to 3 heteroatoms selected from O, N, Si and S, and wherein nitrogen and sulfur atoms are optionally oxidized, and nitrogen heteroatoms can be optionally quaternized. The heteroatoms O, N and S can be placed at any internal position of the heteroalkyl. The heteroatom Si can be placed at any position of the heteroalkyl, including the position at which the alkyl is attached to the remainder of the molecule. Examples include $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-Si(CH_3)_3$, $-CH_2-CH=N-OCH_3$, and$-CH=CH-N(CH_3)-CH_3$. Up to two heteroatoms may be consecutive, such as, $-CH_2-NH-OCH_3$ and $-CH_2-O-Si(CH_3)_3$. Similarly, unless otherwise specified, terms "heteroalkenyl" and "heteroalkynyl" by themselves or in combination with another term refer to alkenyl or alkynyl, respectively, that contain the stated number of carbons and 1 to 3 heteroatoms selected from O, N, Si and S, and wherein the nitrogen and sulfur atoms may optionally be oxidized and the nitrogen heteroatom may optionally be quaternized. The heteroatoms O, N and S can be placed at any internal position of the heteroalkyl.

The term "heteroalkylene" by itself or as part of another substituent means a divalent group, saturated or unsaturated or polyunsaturated, derived from heteroalkyl, as exemplified by $-CH_2-CH_2-S-CH_2CH_2-$ and$-CH_2-S-CH_2-CH_2-NH-CH_2-$, $-O-CH_2-CH=CH-$, $-CH_2-CH=C(H)CH_2-O-CH_2-$ and $-S-CH_2-C{\equiv}C-$. For heteroalkylene, heteroatoms can also occupy either or both of the chain termini (e.g., alkyleneoxy, alkylenedioxy, alkyleneamino, alkylenediamino, and the like).

The terms "alkoxy", "alkylamino" and "alkylthio" (or thioalkoxy) are used in their conventional meanings, refer to those alkyl attached to the rest of the molecule via oxygen atom, amino, or sulfur atom, respectively. Additionally, for dialkylamino, the alkyl portions can be the same or different and can also be combined to form a 3-7 membered ring with the nitrogen atom to which each is attached. Accordingly, a group represented as $-NR^aR^b$ is meant to include piperidinyl, pyrrolidinyl, morpholinyl, azetidinyl and the like.

The terms "halo" or "halogen," by itself or as part of another substituent, mean, unless otherwise stated, fluorine, chlorine, bromine, or iodine atom. Additionally, terms such as "haloalkyl," is meant to include monohaloalkyl or polyhaloalkyl. For example, the term "$C_{1-4}$ haloalkyl" is mean to include trifluoromethyl, 2,2,2-trifluoroethyl, 4-chlorobutyl, 3-bromopropyl, and the like.

The term "aryl" means, unless otherwise stated, polyunsaturated, typically aromatic, hydrocarbon group which can be a single ring or multiple rings (up to three rings) which are fused together or linked covalently. The term "heteroaryl" refers to aryl (or ring) that contain from one to five heteroatoms selected from N, O, and S, wherein the nitrogen and sulfur atoms are optionally oxidized, and the nitrogen atom(s) are optionally quaternized. A heteroaryl can be attached to the remainder of the molecule through a heteroatom. Non-limiting examples of aryl include phenyl, naphthyl and biphenyl, while non-limiting examples of heteroaryl include pyridyl, pyridazinyl, pyrazinyl, pyrimindinyl, triazinyl, quinolinyl, quinoxalinyl, quinazolinyl, cinnolinyl, phthalaziniyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl, thienyl and the like. Substituents for above-stated aryl and heteroaryl ring systems are selected from the group of acceptable substituents described below.

For brevity, the term "aryl" when used in combination with other terms (e.g., aryloxy, arylthioxy, arylalkyl) includes both aryl and heteroaryl rings as defined above. Thus, the term "arylalkyl" is meant to include those groups in which an aryl is attached to an alkyl that is attached to the remainder of the molecule (e.g., benzyl, phenethyl, pyridylmethyl and the like).

The above terms (e.g., "alkyl," "aryl" and "heteroaryl"), in some embodiments, will include both substituted and unsubstituted forms of the indicated group. The preferred substituents for each type of group are provided below. For brevity, the terms aryl and heteroaryl will refer to substituted or unsubstituted versions as provided below, while the term "alkyl" and related aliphatic groups are meant to refer to unsubstituted form, unless indicated to be substituted.

Substituents for the alkyl (including those groups often referred to as alkylene, alkenyl, alkynyl and cycloalkyl) can be a variety of groups selected from: -halogen, -OR', -NR'R", -SR', -SiR'R"R"', -OC(O)R', -C(O)R', $-CO_2R'$, -CONR'R", -OC(O)NR'R", -NR"C(O)R', -NR'-C(O)NR"R"', $-NR"C(O)_2R'$, $-NH-C(NH_2)=NH$, $-NR'C(NH_2)=NH$, $-NH-C(NH_2)=NR'$, -S(O)R', $-S(O)_2R'$, $-S(O)_2NR'R"$, $-NR'S(O)_2R"$, -CN and $-NO_2$ in a number ranging from zero to (2m'+1), wherein m' is the total number of carbon atoms in such radical. R', R" and R'" each independently refers to hydrogen, unsubstituted $C_{1-8}$ alkyl, unsubstituted heteroalkyl, unsubstituted aryl, aryl substituted with 1-3 halogens, unsubstituted $C_{1-8}$ alkyl, $C_{1-8}$ alkoxy or $C_{1-8}$ thioalkoxy, or unsubstituted aryl-$C_{1-4}$ alkyl. When R' and R" are attached to the same nitrogen atom, they can be combined with the nitrogen atom to form a 3-, 4-, 5-, 6-, or 7-membered ring. For example, -NR'R" is meant to include 1-pyrrolidinyl and 4-morpholinyl. The term "acyl" as used by itself or as part of another group refers to groups wherein two substitutents on the carbon that is closest to the point of attachment for the radical is replaced with the substitutent =O (e.g., $C(O)CH_3$, $-C(O)CH_2CH_2OR'$ and the like).

Similarly, substituents for the aryl and heteroaryl are varied and are generally selected from: -halogen, - OR', -OC(O)R', -NR'R", -SR', -R', -CN, $-NO_2$, $-CO_2R'$, -CONR'R", -C(O)R', -OC(O)NR'R", -NR"C(O)R', $-NR"C(O)_2R'$, -NR'-C(O)NR"R"', $-NH-C(NH_2)=NH$, $-NR'C(NH_2)=NH$, $-NH-C(NH_2)=NR'$, -S(O)R',$-S(O)_2R'$, $-S(O)_2NR'R"$, $-NR'S(O)_2R"$, $-N_3$, perfluoro($C_1$-$C_4$)alkoxy, and perfluoro($C_1$-$C_4$)alkyl, in a number ranging from zero to the total number of open valences on the aromatic ring system; and wherein R', R" and R'" are independently selected from hydrogen, $C_{1-8}$ alkyl, $C_{3-6}$ cycloalkyl, $C_{2-8}$ alkenyl, $C_{2-8}$ alkynyl, unsubstituted aryl and heteroaryl, (unsubstituted aryl)-$C_{1-4}$ alkyl, and unsubstituted aryloxy-$C_{1-4}$ alkyl. Other suitable substituents include each of the above aryl substituents attached to a ring atom by an alkylene tether of from 1-4 carbon atoms.

Two of the substituents on adjacent atoms of the aryl or heteroaryl ring may optionally be replaced with a substituent of the formula -T-C(O)-(CH2)q-U-, wherein T and U are independently -NH-, -O-, $-CH_2-$ or a single bond, and q is an integer of from 0 to 2. Alternatively, two of the substituents on adjacent atoms of the aryl or heteroaryl ring may optionally be replaced with formula $-A-(CH_2)_r-B-$, wherein A and B are independently $-CH_2-$, -O-, -NH-, -S-, -S(O)-, $-S(O)_2-$, $-S(O)_2NR'-$ or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula $-(CH_2)_s-X-(CH_2)_t-$, where s and t are independently integers of from 0 to 3, and X is -O-, -NR'-, -S-, -S(O)-, $-S(O)_2-$ or $-S(O)_2NR'-$. The substituent R' in -NR'- and $-S(O)_2NR-$ is selected from hydrogen or unsubstituted $C_{1-6}$ alkyl.

In the present invention, when one chain atom (such as a C atom) is replaced with a heterocyclic ring, the

heterocyclic ring may lose two hydrogens on the same ring atom (on ring carbon atom) thereby connect with other chain atoms on the chain (forming a structure similar to a spirocyclic ring), or may lose two hydrogens on different ring atoms thereby connect with other chain atoms on the chain (such as - cyclopentylidene-).

As used herein, the term "heteroatom" is meant to include oxygen (O), nitrogen (N), sulfur (S) and silicon (Si).

For the compounds provided herein, a bond that is drawn from a substituent (typically an R group) to the center of an aromatic ring (e.g., benzene, pyridine, and the like) will be understood to refer to a bond providing a connection at any of the available vertices of the aromatic ring. In some embodiments, the depiction will also include connection at a ring which is fused to the aromatic ring. For example, a bond drawn to the center of the benzene portion of an indole, will indicate a bond to any available vertex of the six- or five-membered ring portions of the indole.

The term "pharmaceutically acceptable salts" is meant to include salts of the active compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of salts derived from pharmaceutically acceptable inorganic bases include aluminum, ammonium, calcium, copper, iron, ferrous, lithium, magnesium, manganese, manganous, potassium, sodium, zinc, and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, *p*-tolylsulfonic, citric, tartaric, methane sulfonic, and the like. Also included are salts of amino acids such as arginate and the like, and salts of organic acids like glucuronic or galactunoric acids and the like (see, for example, Berge, S.M., et al, "Pharmaceutical Salts", Journal of Pharmaceutical Science, 1977, 66, 1-19). Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms thereof in certain physical properties, such as solubility in polar solvents, but in addition to the above, those salts are equivalent to the parent form of the compound for the purposes of the present invention.

In addition to salt forms, the present disclosure provides compounds which are in a prodrug form. Prodrugs of the compounds described herein are those compounds that readily undergo chemical changes under physiological conditions to provide the compounds of the present disclosure. Additionally, prodrugs can be converted to the compounds of the present disclosure by chemical or biochemical methods in an ex vivo environment. For example, when placed in a transdermal patch reservoir containing suitable enzymes or chemical reagents, the prodrug can be slowly converted to the compound of the invention.

Certain compounds of the present disclosure can exist in unsolvated forms as well as solvated forms, including hydrated forms. The solvated forms are generally equivalent to the non-solvated forms and should be included in the scope of the present invention. Certain compounds of the present disclosure may exist in multiple crystallie or amorphous forms. Generally, as for then application considered in the present invention, all physical forms are equivalent and should be included in the scope of the present invention.

Certain compounds of the present disclosure possess asymmetric carbon atoms (optical centers) or double bonds; the racemates, diastereomers, geometric isomers, regioisomers and individual isomers (e.g., separate enantiomers) are all intended to be encompassed within the scope of the present disclosure. When compounds are provided herein with an identified stereochemistry (indicated as R or S, or with dashed or wedge bond designations), those compounds will be understood by one of skill in the art to be substantially free of other isomers (e.g., at least 80%, 90%, 95%, 98%, 99%, and up to 100% free of the other isomer).

The compounds of the present disclosure may also contain unnatural proportions of isotope atomic isotopes at one or more of isotopic atoms that constitute such compounds. The unnatural proportions of certain isotope can be defined as the amount from the naturally found amount of the atom discussed to 100% of that atom. For example, the compounds may incorporate radioactive isotopes, such as tritium ($^{3}H$), iodine-125 ($_{125}I$) or carbon-14 ($^{14}C$), or non-radioactive isotopes, such as deuterium ($^{2}H$) or carbon-13 ($^{13}C$). Such isotopic variants may provide additional uses in

addition to those described in this application. For instance, isotopic variants of the compounds of the disclosure may find additional utility, including but not limited to, as diagnostic and/or imaging reagents, or as cytotoxic/radiotoxic therapeutic agents. Additionally, isotopic variants of the compounds of the disclosure can have altered pharmacokinetic and pharmacodynamic characteristics which can contribute to enhanced safety, tolerability or efficacy during treatment. All isotopic variations of the compounds of the present disclosure, whether radioactive or not, should be encompassed within the scope of the present disclosure.

**Targeted Enzyme Degradation platform TED**

The present invention provides a Targeted Enzyme Degradation platform (TED) on basis of the conjugate of the present invention, which utilizes the intracellular "cleaner" - ubiquitin proteasome system.

Typically, according to the TED technology of the present invention, which can utilize cell’s intrinsic protein destruction mechanism to remove specific oncogenic and pathogenic proteins from the cell, therefore it is an alternative method of targeted therapy.

Different from action mechanism of conventional protein inhibitors, TED technology of the present invention relates to a bifunctional hybrid compound, one side of which is used to bind target proteins, and another side is used to bind E3 ligases, enabling the target proteins binding the E3 ligases, and the target proteins being ubiquitinated, thereby being degraded by the proteome. Theoretically, TED technology only provides binding activity without functional activity that directly inhibiting the target protein, and can be reused. Therefore, TED technology has excellent application prospects.

**Polypeptide element**

As used herein, the term "polypeptide element" includes peptide fragment (such as oligopeptide comprising 3 - 20 aa) or protein. In addition, this term also includes intact protein or fragment thereof. Preferred polypeptide element includes antibody (such as intact antibody, single-chain antibody, nanobody, Fab), especially those antibodies against tumor cell markers (such as tumor markers located on the surface of tumor cells, such as receptors on the cell surface) or inflammatory factors (such as inflammatory factors associated with autoimmune diseases).

As used herein, term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain by a covalent disulfide bond, and the number of disulfide bonds between the heavy chains of different immunoglobulin isotypes are different. Each heavy and light chain also has regularly spaced intrachain disulfide bonds. Each heavy chain has a variable region (VH) at one end, followed by multiple constant regions. There are a variable region (VL) at one end of each light chain and a constant region at the other end. The constant region of the light chain is relative to the first constant region of the heavy chain, and the variable region of the light chain is relative to the variable region of the heavy chain. Special amino acid residues form an interface between the variable regions of the light chain and the heavy chain.

As used herein, terms "single-domain antibody" and "nanobody" have the same meaning, and refer to cloneing the variable region of the heavy chain of an antibody, and constructing a single-domain antibody consisting of only one heavy chain variable region, which is the smallest antigen-binding fragment that having complete functions. Usually, after obtaining an antibody naturally missing constant region 1 (CH1) of light chain and heavy chain, the variable region of the antibody heavy chain is cloned to construct a single domain antibody consisting of only one heavy chain variable region.

As used herein, term "variable" means that certain parts of the variable region of the antibody are different in sequence, which forms the binding and specificity to specific antigens of various specific antibodies. However, variabilities are not evenly distributed throughout the variable regions of antibodies. It is concentrated in three fragments that are called complementarity determining regions (CDR) or hypervariable regions in the variable regions of light chain and heavy chain. More conservative parts of the variable region are called the framework region (FR). The variable regions of the natural heavy chain and light chain each contain four FR regions, which are roughly in a β-folded configuration, connected by three CDRs forming a connecting loop, and in some cases can form a partially β-folded structure. The CDRs in each chain are closely placed together through the FR region and form the antigen binding site of the antibody together with the CDRs of the other chain (see Kabat et al., NIH Publ. No. 91-3242, Volume I, pages 647-669 (1991)). Constant regions do not directly participate in the binding of antibodies to antigens, but they exhibit different effector functions, such as participating in antibody-dependent cytotoxicity of antibodies.

The light chains of vertebrate antibodies (immunoglobulins) can be classified into one of two distinct categories (referred to κ and λ) based on the amino acid sequence of their constant regions. According to the amino acid sequence of the constant region of their heavy chains, immunoglobulins can be divided into different types. There are five main classes of immunoglobulins: IgA, IgD, IgE, IgG and IgM, some of which can be further divided into subclasses (isotypes),

such as IgG1, IgG2, IgG3, IgG4, IgA and IgA2. The heavy chain constant regions corresponding to different classes of immunoglobulins are called $\alpha$, $\delta$, $\varepsilon$, $\gamma$ and $\mu$ respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which are divided into 4 framework regions (FR). The amino acid sequence of 4 FR is relatively conservative and does not directly participate in the binding reaction. These CDRs form a circular structure, and the $\beta$-folded configuration formed by the FRs in between are close to each other in space structure, and the CDRs on the heavy chain and the corresponding CDRs on the light chain constitute the antigen binding site of the antibody. The amino acid sequences of antibodies of the same type can be compared to determine which amino acids constitute the FR or CDR regions.

In the present invention, the polypeptide element can include not only intact antibody, but also fragment of antibody with immunological activity (such as Fab or $(Fab')_2$ fragment; heavy chain of antibody; or light chain of antibody) or fusion protein formed by antibody and other sequences. Therefore, the present invention also includes fragment, derivative and analog of the antibody.

## Targeting ligand

Targeting ligand (or target protein portion or target protein ligand or ligand) is a small molecule that can bind to a target protein of interest.

Some embodiments of this application relate to target molecules, and representative target molecules include but are not limited to: folic acid, Hsp90 inhibitors, kinase inhibitors, MDM2 inhibitors, compounds targeting proteins containing human BET bromodomain, compounds targeting cytoplasmic signaling protein FKBP12, HDAC inhibitors, human lysine methyltransferase inhibitors, angiogenesis inhibitors, immunosuppressive compounds and compounds targeting aryl hydrocarbon receptor (AHR).

In certain embodiments, the targeting ligand is capable of binding kinases, BET bromodomain-containing proteins, cytoplasmic signaling proteins (such as FKBP12), nucleoproteins, histone deacetylases, lysine methyltransferase, protein that regulates angiogenesis, protein that regulates immune response, aromatic hydrocarbon receptor (AHR), estrogen receptor, androgen receptor, glucocorticoid receptor, or transcription factor (e.g., SMARCA4, SMARCA2, TRIM24).

In certain embodiments, kinases which targeting ligands are able to bind to, include, but not limited to: tyrosine kinases(for example, AATK, ABL, ABL2, ALK, AXL, BLK, BMX, BTK, CSF1R, CSK, DDR1, DDR2, EGFR, EPHA1, EPHA2, EPHA3, EPHA4, EPHA5, EPHA6, EPHA7, EPHA8, EPHA10, EPHB1, EPHB2, EPHB3, EPHB4, EPHB6, ERBB2, ERBB3, ERBB4, FER, FES, FGFR1, FGFR2, FGFR3, FGFR4, FGR, FLT1, FLT3, FLT4, FRK, FYN, GSG2, HCK, HRAS, HSP90, IGF1R, ILK, INSR, INSRR, IRAK4, ITK, JAK1, JAK2, JAK3, KDR, KIT, KRAS, KSP, KSR1, LCK, LMTK2, LMTK3, LTK, LYN, MATK, MERTK, MET, MLTK, MST1R, MUSK, NPR1, NRAS, NTRK1, NTRK2, NTRK3, PDGFRA, PDGFRB, PLK4, PTK2, PTK2B, PTK6, PTK7, RET, ROR1, ROR2, ROS1, RYK, SGK493, SRC, SRMS, STYK1, SYK, TEC, TEK, TEX14, TIE1, TNK1, TNK2, TNNI3K, TXK, TYK2, TYRO3, YES1 or ZAP70), serine/threonine kinase(such as Casein Kinase 2, protein kinase A, protein kinase B, protein kinase C, Raf kinase, CaM kinase, AKT1, AKT2, AKT3, ALK1, ALK2, ALK3, ALK4, Aurora A, Aurora B, Aurora C, CHK1, CHK2, CLK1, CLK2, CLK3, DAPK1, DAPK2, DAPK3, DMPK, ERK1, ERK2, ERK5, GCK, GSK3, HIPK, KHS1, LKB1, LOK, MAPKAPK2, MAPKAPK, MEK, MNK1, MSSK1, MST1, MST2, MST4, NDR, NEK2, NEK3, NEK6, NEK7, NEK9, NEK11, PAK1, PAK2, PAK3, PAK4, PAK5, PAK6, PIM1, PIM2, PLK1, RIP2, RIP5, RSK1, RSK2, SGK2, SGK3, SIK1, STK33, TAO1, TAO2, TGF-$\beta$, TLK2, TSSK1, TSSK2, MLK1 or MLK2), cyclin-dependent kinases (such as Cdk1-Cdk11) and Leucine-rich repetitive kinase (such as LRRK2).

## Target molecule

The conjugate of formula I in the present application, binds to the target protein through target molecule.

In the present invention, the target molecule can be a target molecule A, a target molecule T, or the combination thereof.

In the present invention, the target molecule can be any inhibitor of the target protein. The target molecule can be a highly effective inhibitor of the target protein, or an inhibitor with relatively poor activity. Specifically, the target molecule of the present invention may be a small molecule inhibitor known in the art against any target protein in the art.

In some embodiments, the target molecule used herein has a radical, such as -O-, -$NR_a$- (wherein $R_a$ is H, or substituents of C1-C6 alkyl etc., -CO-, -COO-, and the like, that is able to connect to a linker (e.g. L1 in the present invention) monovalently to form an ether, an amine, an amide and the like.

The target protein may be a variety of target proteins known in the art, representative examples include, but are not limited to:MDM2, AKT, BCR-ABL, Tau, BET(BRD2, BRD3, BRD4), ERR$\alpha$, FKBP12, RIPK2, ERBB3, androgen

receptor, MetAP2, TACC3, FRS2α, PI3K, DHFR, GST, Halo Tag, CRABPI, CRABPII, RAR, aromatic hydrocarbon receptor, strogen receptor. Different target proteins and some corresponding inhibitors can be obtained commercially or prepared by conventional methods. For example, as for MDM2, the inhibitors thereof can be referred to documents such as WO 2017176957, and WO2017176958A1.

### E3 Ligase Ligand

In the present invention, the E3 ligase ligand is used for binding E3 ligase. Representative E3 ligase ligands have structures shown as below:

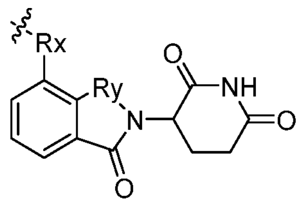

in the above formula, Rx is none, C1-C6 alkyl, C2-C6 alkenyl, C2-C6 alkynyl, O, NH, S, CO or $SO_n$ (n is 1 or 2) and the like; $R_Y$ is $CH_2$, C=S, CO; and the E3 ligase ligand (B in formula I) is able to connect to L1 of the present invention via Rx group in the E3 ligase ligand, for example $-R_x$-L1-A (such as -O-L1-A),

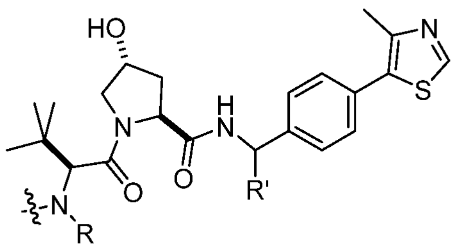

in the above formula, R' is H or Me, and R is H, Me or Et.

In some embodiments, the E3 ligase ligand used herein has a radical, such as -O-, $-NR_a-$ (wherein $R_a$ is H, or substituents of C1-C6 alkyl etc., -CO-, -COO-, and the like, that is able to connect to a linker (e.g. L1 in the present invention and the like) monovalently to form an ether, an amine, an amide and the like.

### Linker molecule (L1 as described herein)

The linker molecule of the present invention is used for connecting the target molecule and the E3 ligase ligand. For example, it can be connected to the target molecule or the E3 ligase ligand through functional groups at both ends (such as -OH, -SH, $-NH_2$, -NHR, -SOOH or -COOH) ; wherein R is selected from: substituted or unsubstituted C1-C10 alkyl, -(C=O)-R', (C=O)NH-R', -NH(C=O)-R', $-SO_2$-R', - $NHSO_2$-R', $-SO_2$NH-R -SO-R', -NHSO-R', -SONH-R', $-PO_3$-R', -NHCOO-R', -COO-R' or -NH-CO-NH-R', -NH-CO-O-R' or -X'-L3-Z; where L3 is linker, and Z is polypeptide element (such as ligand, antibody or peptide fragment, etc.) or a targeting molecule such as targeting function small molecule (such as folic acid, HSP90 inhibitor, etc.).

Preferably, when the linker is -(CH2)s-;wherein, when 1 to 4 $-CH_2-$ are replaced with five-membered unsaturated ring containing 1 to 4 heteroatoms (such as N, O, S, and P), the linker molecule is show as below:

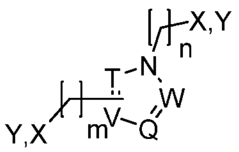
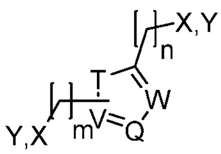
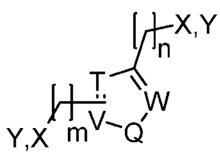
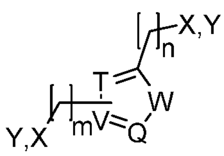

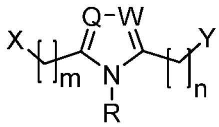

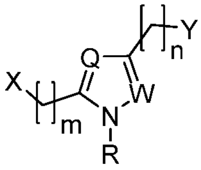

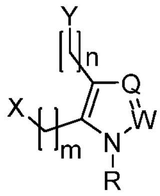

in the formula, X, Y, T, V, Q, W, m, n, s, R are defined as above.

Preferably, when the linker is -(CH2)s-;wherein, when 1 to 4 $-CH_2-$ are replaced with O or the 4-6 membered saturated ring containing one nitrogen atom, the linker molecule of the present invention specifically can have the structure selected from the following:

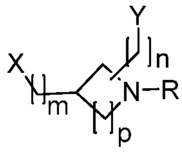

in the formula, X, Y, p, m, n, s, and R are defined as above.

Preferably, the linker molecule of the present invention can have a structure selected from the following:

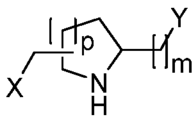

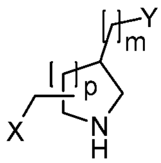

in the formula, X and Y are defined as above, m is an integer of 1-20; p is an integer of 1-3.

Preferably, when the linker is $-(CH_2)_s-$ or the linker is $-(CH_2)_s-$; wherein 1 to 4 $-CH_2-$ are replaced with O. Preferably, the linker molecule of the present invention can further connect with the target molecule or polypeptide element through the linker structure of -X'-L3-, forming a trifunctional Targeted Enzyme Degradation platform (TED) molecule, for example, the Targeted Enzyme Degradation platform (TED) molecules having the following formula:

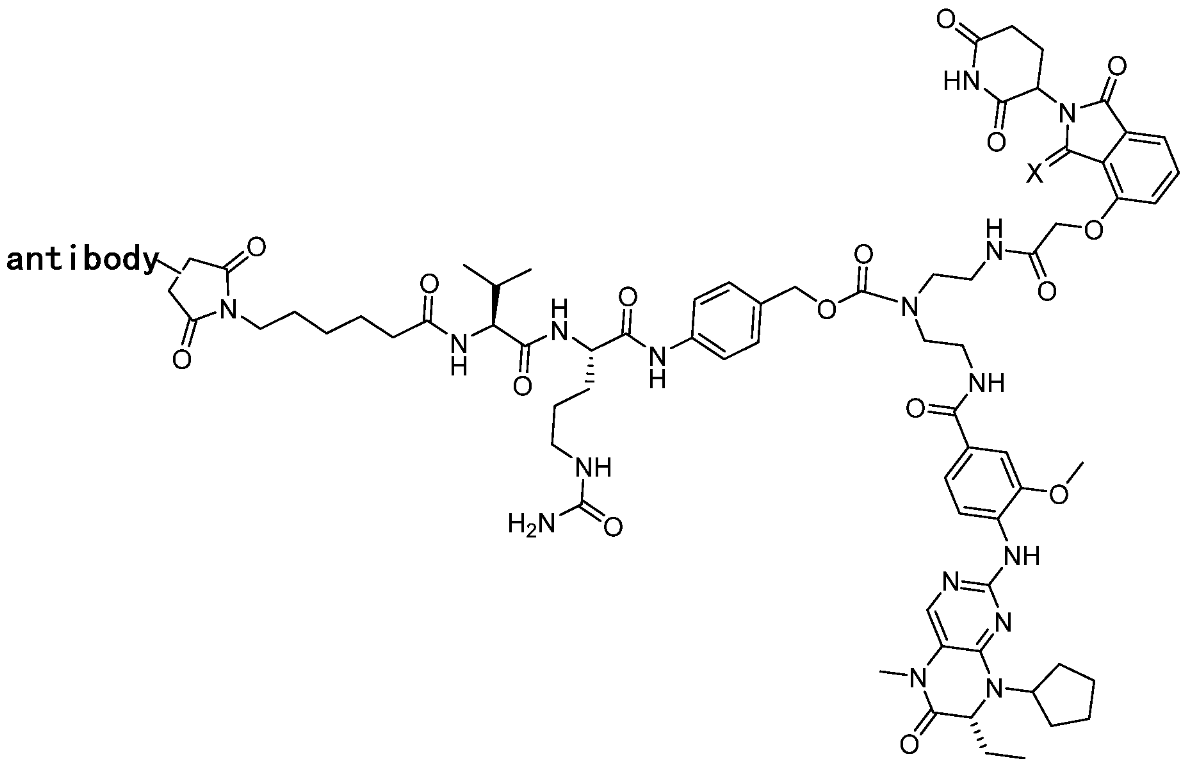
.

**Targeted Enzyme Degradation platform (TED) molecule**

The structure of the Targeted Enzyme Degradation platform (TED) molecule of the present invention is of formula Ia or formula I:

Target molecule-Linker-E3 ligase ligand formula Ia,

A-L1-B formula I

in the formula, the definitions of A, L1 and B are as described in the first aspect of the present invention.

**Linker and coupling method**

The linker of the present invention L1 is used for connecting the target molecule and the E3 ligase ligand.
Preferably, the target molecule or the E3 ligase ligand can be connected through -O-, -S-, -NH-, -NR-, -(C=O)-, -(C=O)O-, $-SO_2-$ and other groups.
The linker of the present invention may further contain a varity of other functional groups, such as - OH, -NHR, -SH and the like.
Typically, the linker of the present invention L1, can be represented by the following general formula II:

-X-L2-Y- formula II

in the formula, the definitions of X, L2, and Y are as described in the first aspect of the present invention.
In another preferred embodiment, X and Y are each independently divalent groups formed by the loss of one hydrogen atom forming bivalence from the following monovalent groups: -OH, $-NH_2$, -SH, -COOH, $-SO_2H$.
Specifically, the connection mode of the linker and the target molecule can be connected through the linker group shown as below:

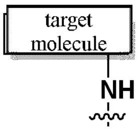
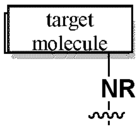
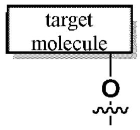
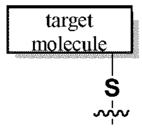
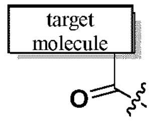
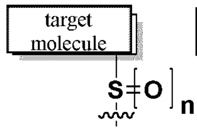
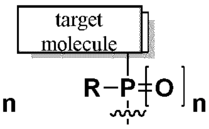

wherein, R in the each of above formulas is defined as above; n is 1 or 2 or 3.

**ACTED**

In the present invention, when the target molecule is an antibody, the conjugate of the present invention also can be referred to as ACTED or ACTED molecule or ACTED compound for short.

In the present invention, the structure of exemplary ACTED compounds (i.e, a TED molecule with Ab attached) is as follows:

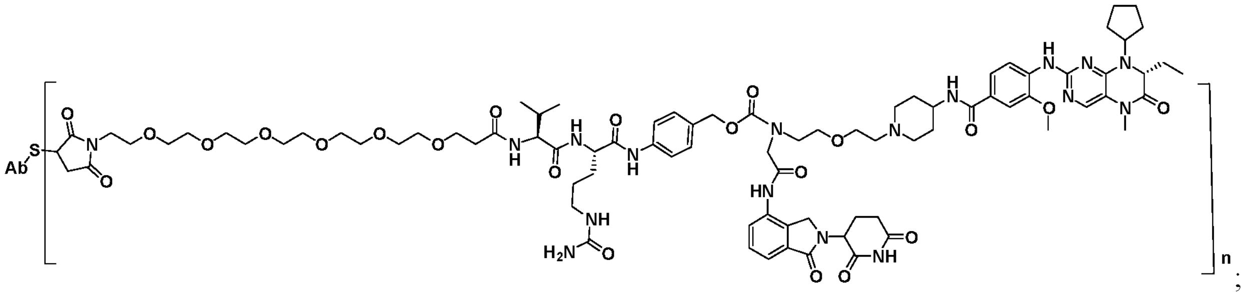

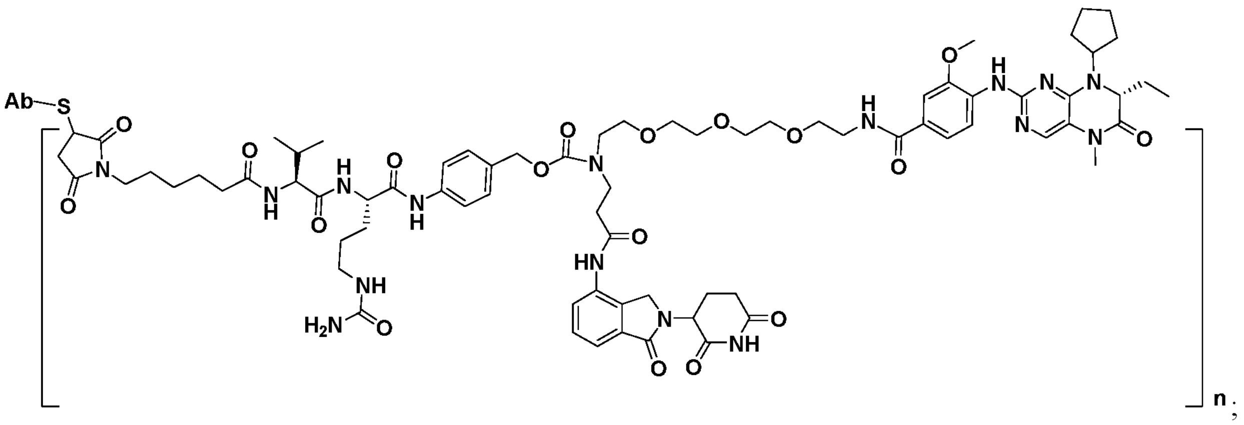

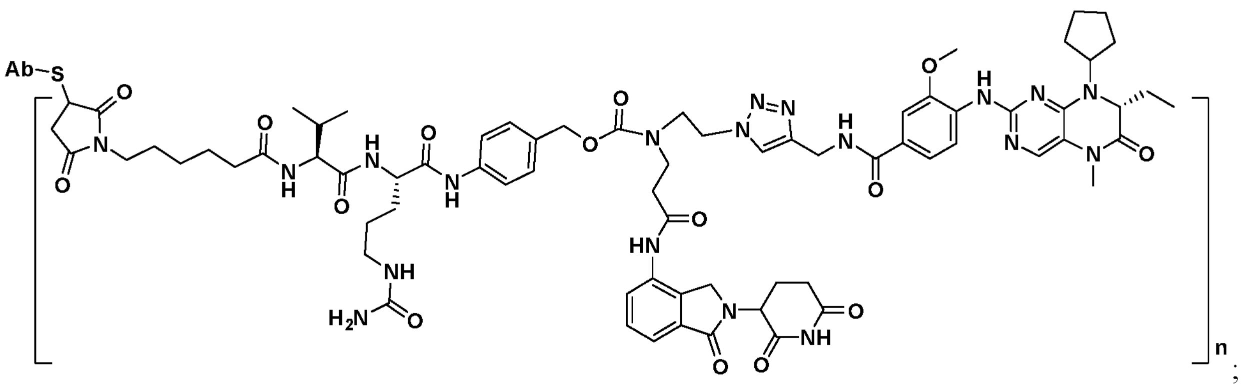

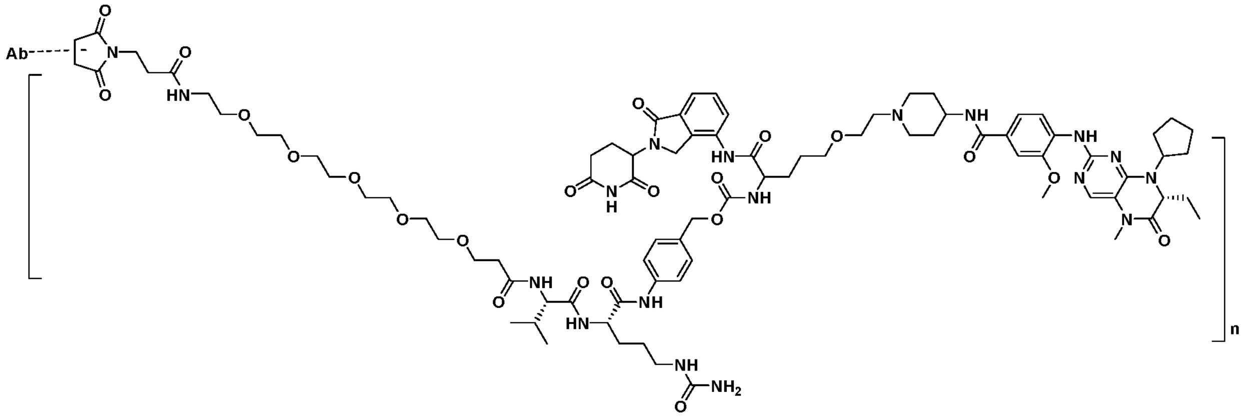

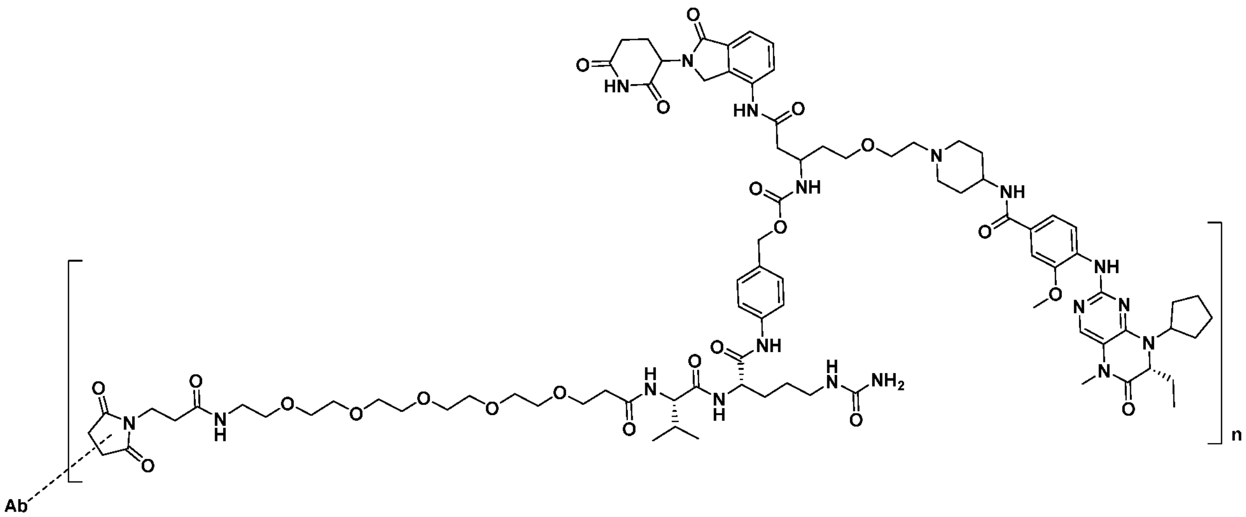

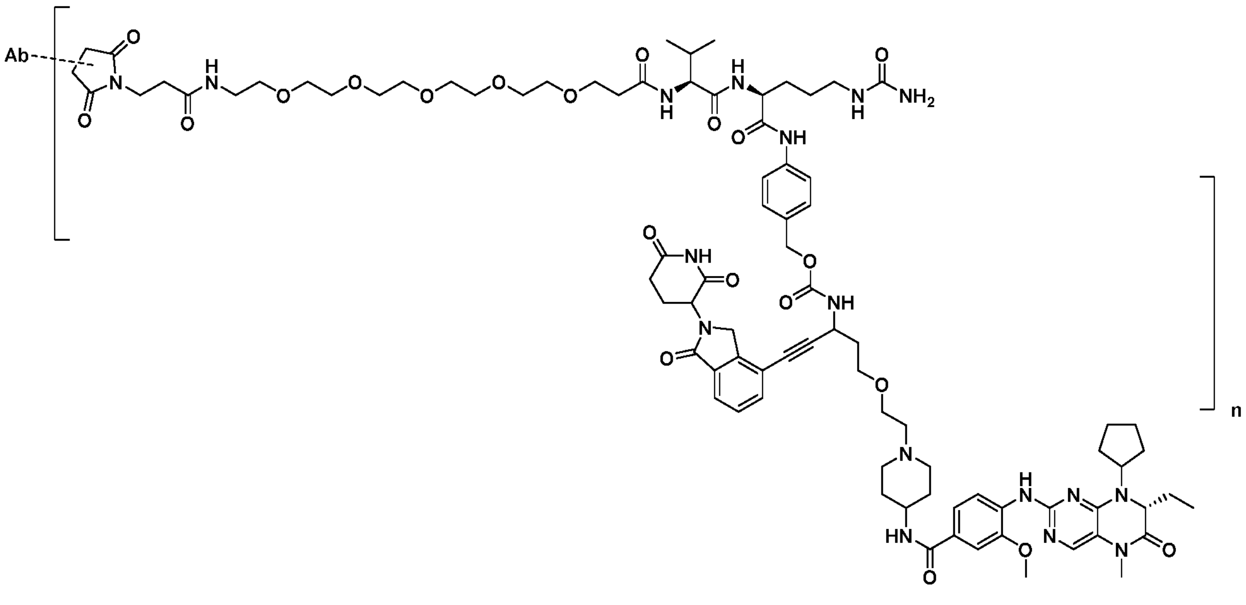

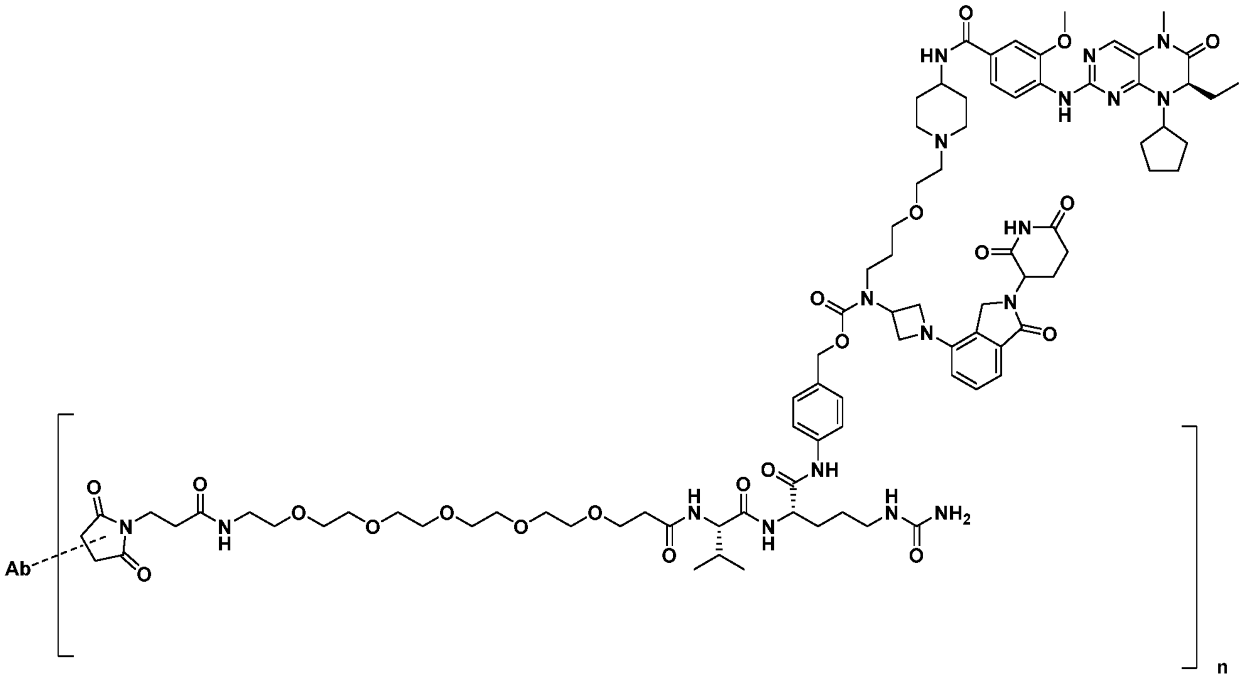

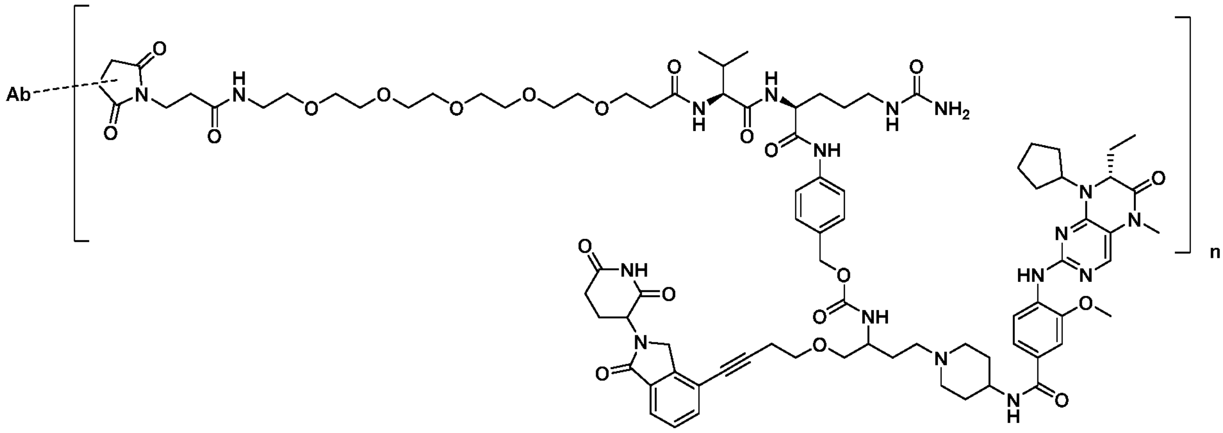

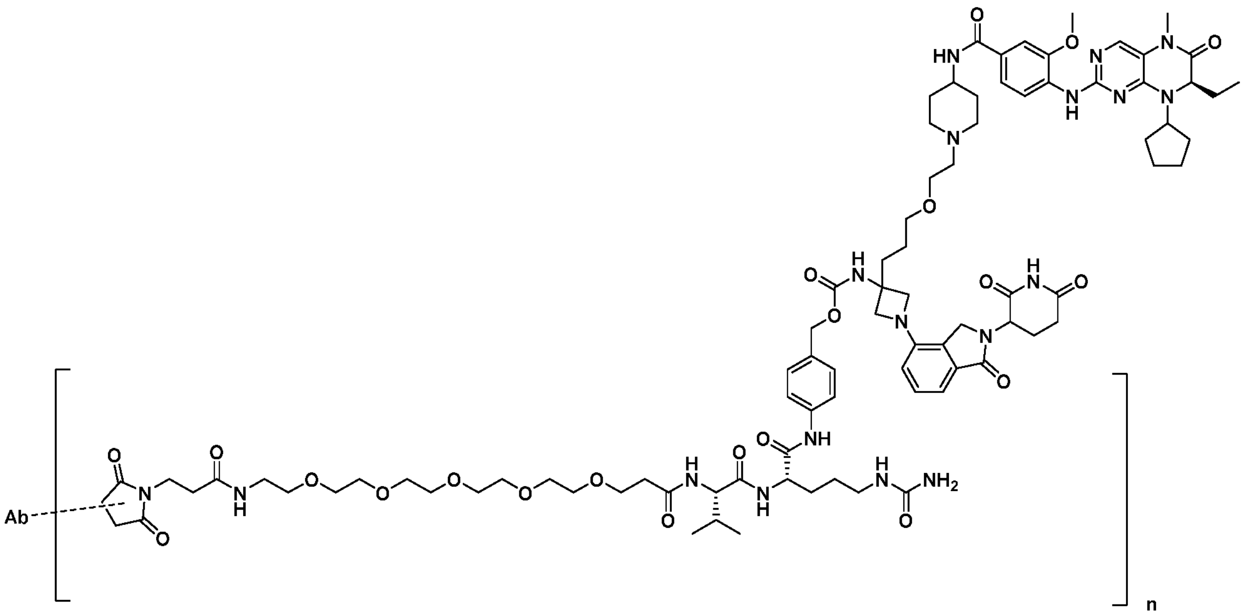

wherein n=1 to 15 (preferably, n=1 to 8, more preferably, n=2 to 7), Ab means an antibody; --- means Ab is connected with L3 of formula III (such as

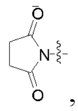

,

-NH-, -NHR or $-NH_2$ group in L3), through amino acid at N-terminal or C-terminal, or a side chain of amino acid (such as a side chain of amino acid selected from the group consisting of Lys, and Cys), or a sulfhydryl formed by reducing and opening disulfide bond.

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are percentages by weight and parts by weight. Unless otherwise specified, the experimental materials and reagents used in the following examples are commercially available.

**Example 1 Synthesis of L1-B moiety**

**Table A1 the structure of linker in TED (i.e. the structure of $NH_2$-L2-Y-B)**

R= 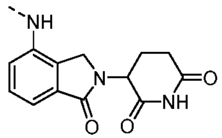 B(A1) 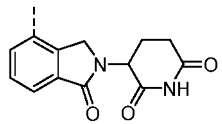 B(A2) 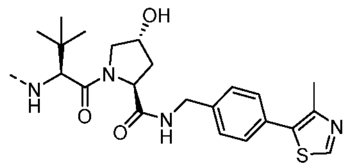 B(B1)

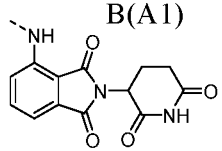 B(A3) 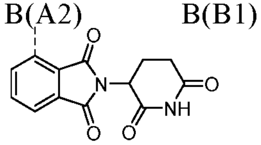 B(A4)

| Cmpd.No. | Structural formula | Cmpd.No. | Structural formula |
|---|---|---|---|
| L1 | 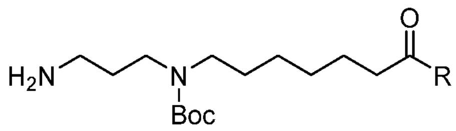 | L44 | 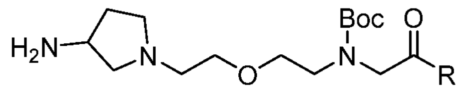 |
| L2 | 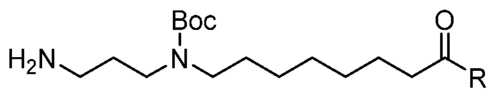 | L45 | 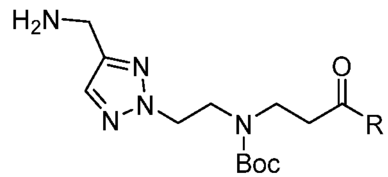 |
| L3 | 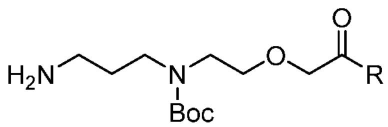 | L46 | 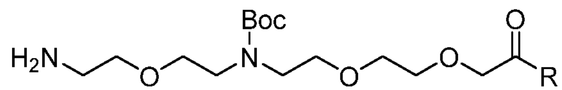 |
| L4 | 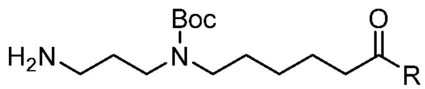 | L47 | 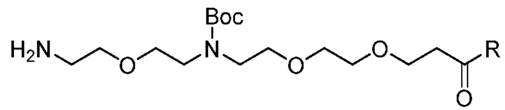 |

(continued)

| Cmpd.No. | Structural formula | Cmpd.No. | Structural formula |
|---|---|---|---|
| L5 | 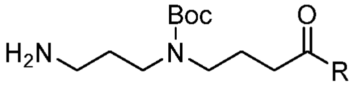 | L48 | 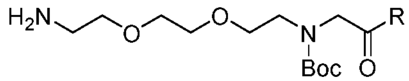 |
| L6 | 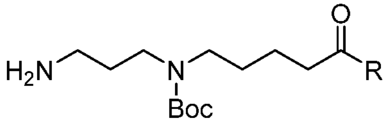 | L49 | 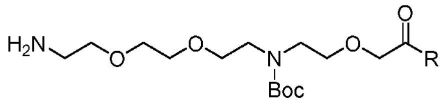 |
| L7 | 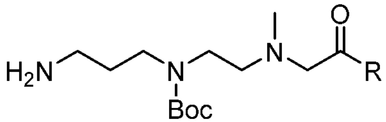 | L50 | 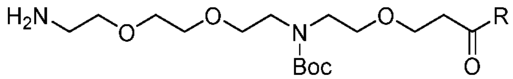 |
| L8 | 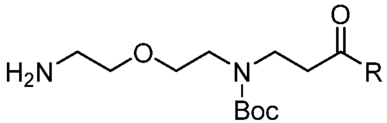 | L51 | 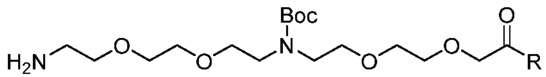 |
| L9 | 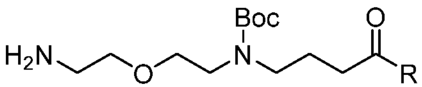 | L52 | 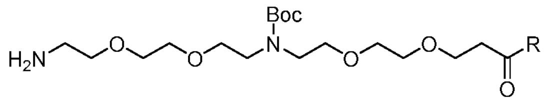 |
| L10 | 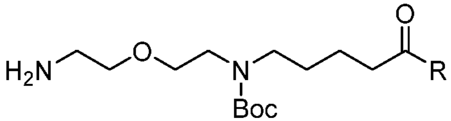 | L53 | 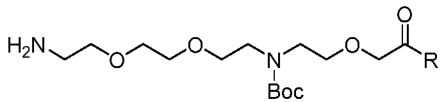 |
| L11 | 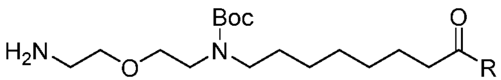 | L54 | 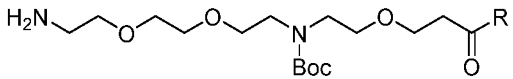 |
| L12 | 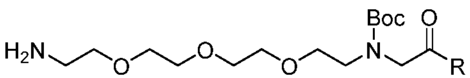 | L55 | 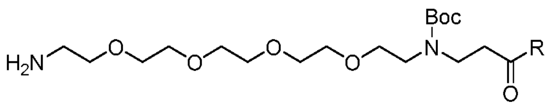 |

(continued)

| Cmpd.No. | Structural formula | Cmpd.No. | Structural formula |
|---|---|---|---|
| L13 | 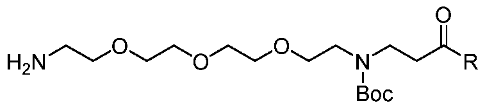 | L56 | 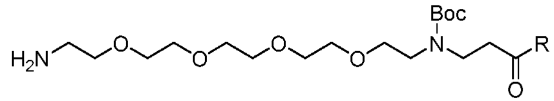 |
| L14 | 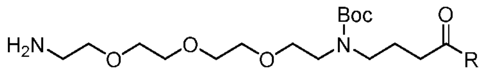 | L57 | 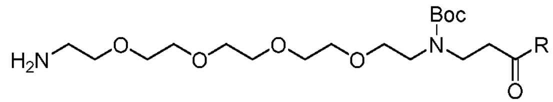 |
| L15 | 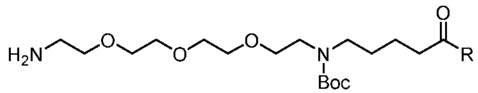 | L58 | 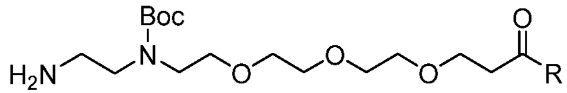 |
| L16 | 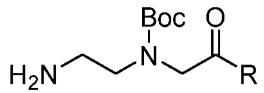 | L59 | 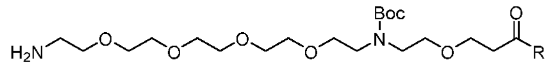 |
| L17 | 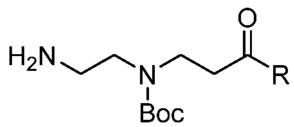 | L60 | 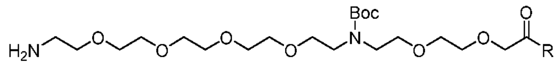 |
| L18 | 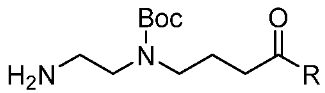 | L61 | 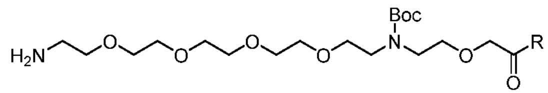 |
| L19 | 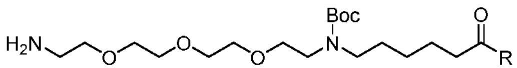 | L62 | 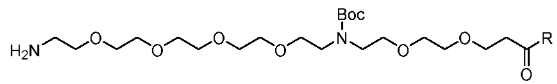 |
| L20 | 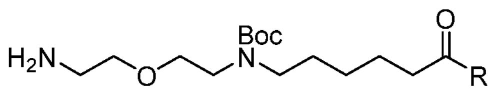 | L63 | 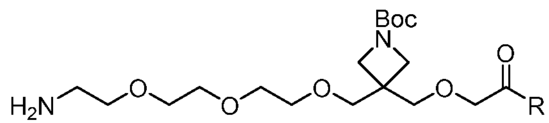 |

(continued)

| Cmpd.No. | Structural formula | Cmpd.No. | Structural formula |
|---|---|---|---|
| L22 | 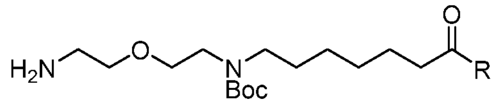 | L64 | 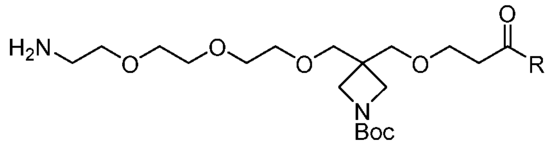 |
| L23 | 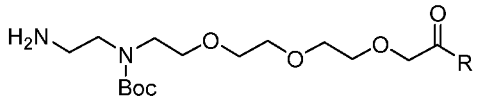 | L65 | 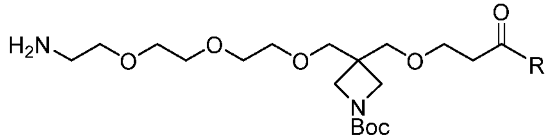 |
| L24 | 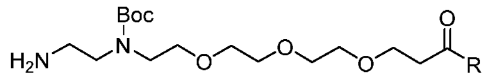 | L66 | 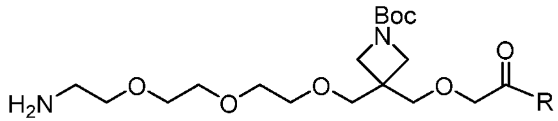 |
| L25 | 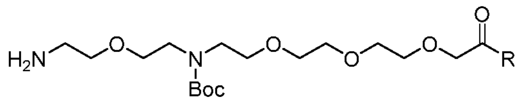 | L67 | 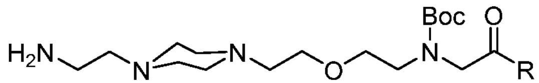 |
| L26 | 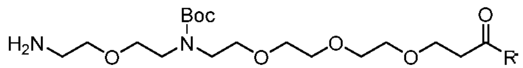 | L68 | 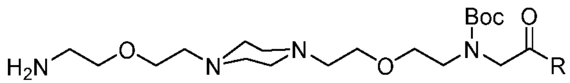 |
| L27 | 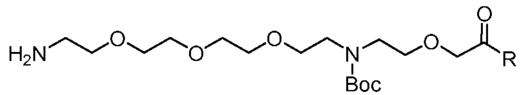 | L69 | 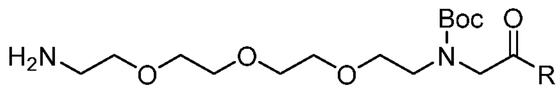 |
| L28 | 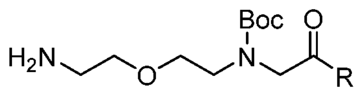 | L70 | 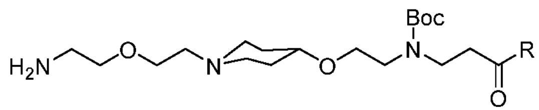 |
| L29 | 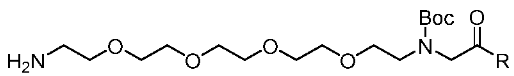 | L71 | 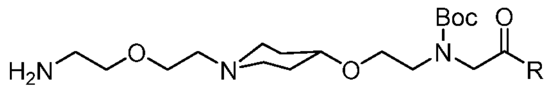 |

(continued)

| Cmpd.No. | Structural formula | Cmpd.No. | Structural formula |
|---|---|---|---|
| L30 | 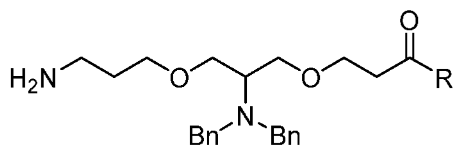 | L72 | 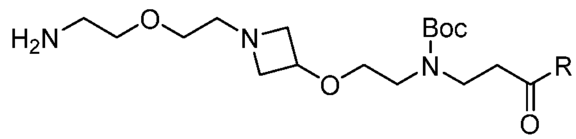 |
| L31 | 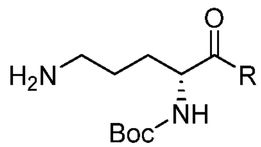 | L73 | 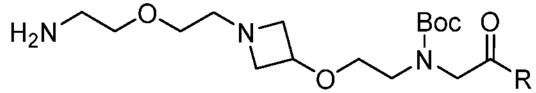 |
| L32 | 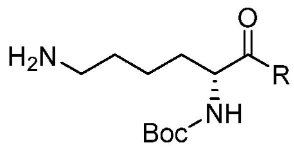 | L74 | 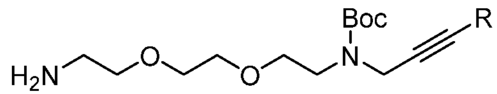 |
| L33 | 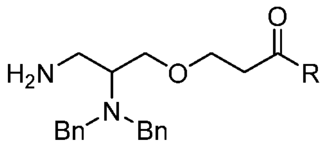 | L75 | 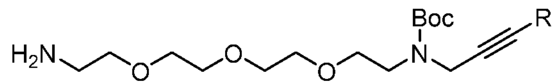 |
| L34 | 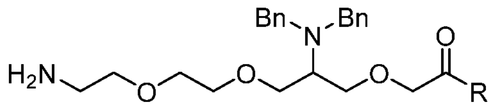 | L76 | 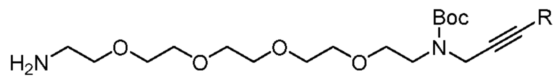 |
| L35 | 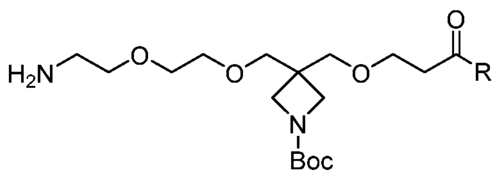 | L77 | 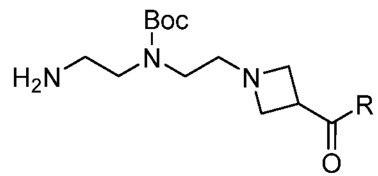 |

(continued)

| Cmpd.No. | Structural formula | Cmpd.No. | Structural formula |
|---|---|---|---|
| L36 | 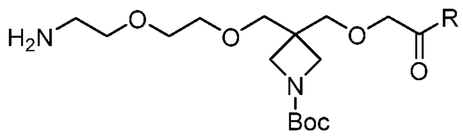 | L78 | 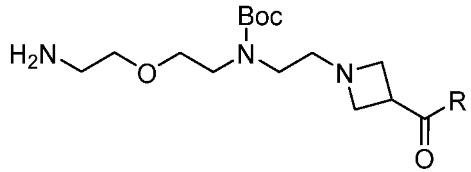 |
| L37 | 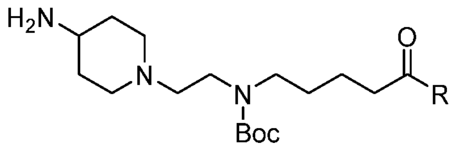 | L79 | 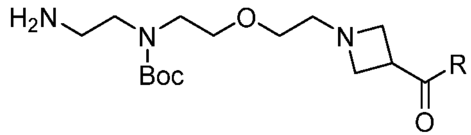 |
| L38 | 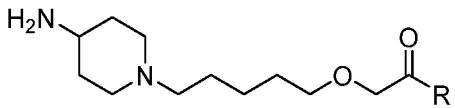 | L80 | 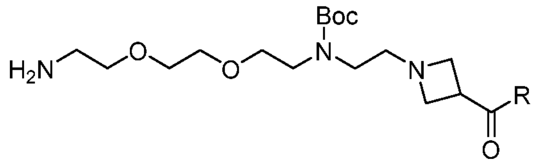 |
| L39 | 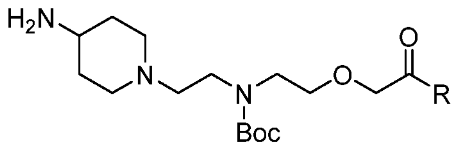 | L81 | 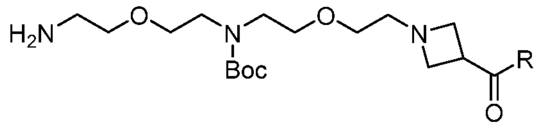 |
| L40 | 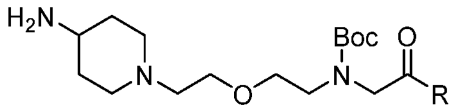 | L82 | 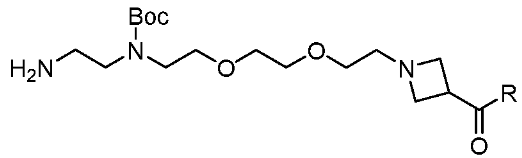 |
| L41 | 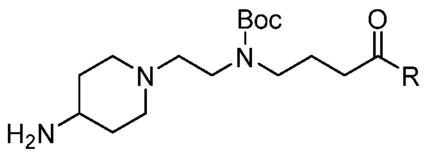 | L83 | 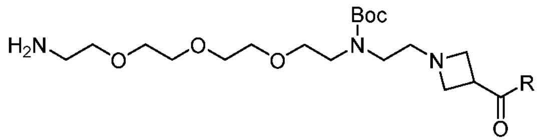 |

(continued)

| Cmpd.No. | Structural formula | Cmpd.No. | Structural formula |
|---|---|---|---|
| L42 | 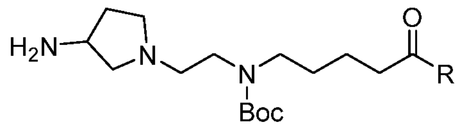 | L84 | 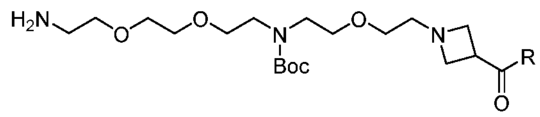 |
| L43 | 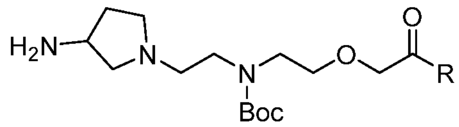 | | |

**Synthesis method of Compounds Linker b-Ligand B(A)**

**Synthesis method of UBI-1097 ($NH_2$-11b-B(A1)):**

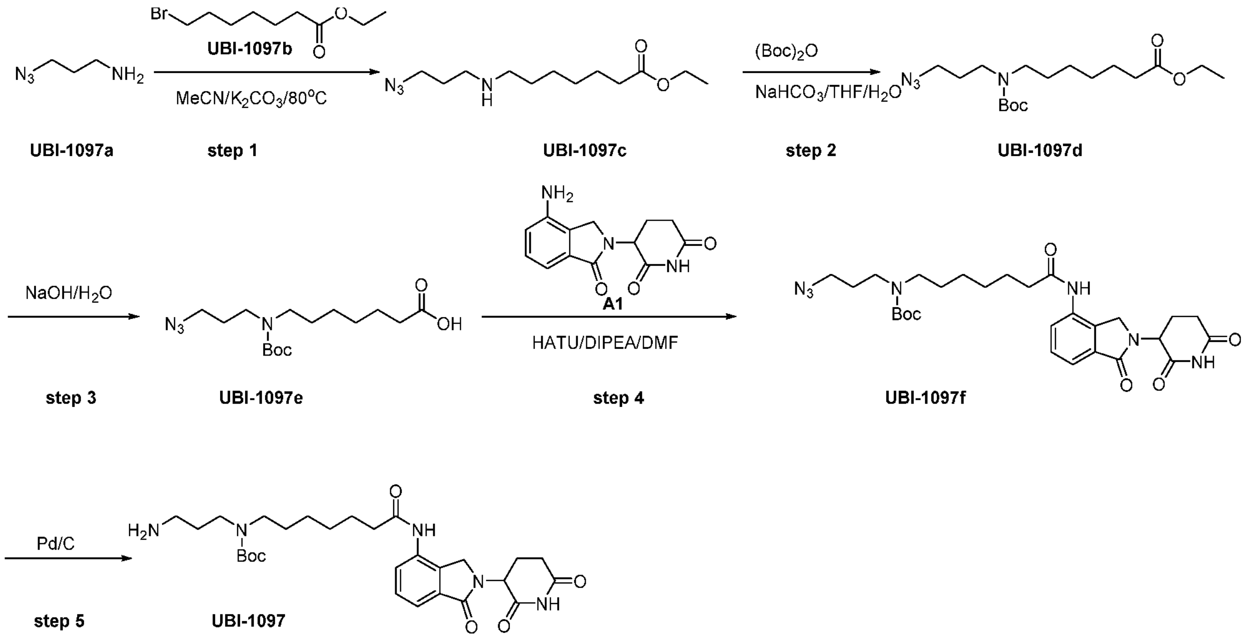


**Step 1: UBI-1097c**

Compound **UBI-1097a** (1.0 g, 10 mmol), compound **UBI-1097b** (2.36 g, 10 mmol) and $K_2CO_3$ (2.76 g, 20 mmol) were disolved in $CH_3CN$ (100 mL), then reacting at room temperature for 18 hours. After the completion of reaction, the reaction was filtrated. The filtrate was concentrated to obtain the crude product. The crude product was isolated by silica gel column chromatography (petroleum ether/ethyl acetate = 50% to 80%, then dichloromethane/methanol = 0% to 10%) to obtain the target product **UBI-1097c** (450mg, yield 18%) as colorless transparent oil. $^1$H NMR (400 MHz, chloroform-*d*) δ 4.12 (q, *J* = 7.1 Hz, 2H), 3.37 (t, *J* = 6.7 Hz, 2H), 2.70 (t, *J* = 7.0 Hz, 2H), 2.60 (t, *J* = 7.2 Hz, 2H), 2.29 (t, *J* = 7.5 Hz, 2H), 1.77 (p, *J* = 6.8 Hz, 2H), 1.67 - 1.60 (m, 2H), 1.53 - 1.44 (m, 2H), 1.38 - 1.30 (m, 4H), 1.25 (t, *J* = 7.1, 1.1 Hz, 3H).

**Step 2: UBI-1097d**

Compound **UBI-1097c** (450mg, 1.76mmol), di-*tert*-butyl dicarbonate (760mg, 3.52mmol) and $NaHCO_3$ (295mg, 3.52mmol) were dissolved in THF (20mL) and reacted at room temperature for 2 hours. After the completion of reaction, water (10mL) was added, extracted with dichloromethane (10mL *3). The organic phases were combined, then washed with saturated brine, dried over anhydrous $Na_2SO_4$, and concentrated by rotary evaporation under reduced pressure to obtain the crude product **UBI-1097d** (450mg, yield 71.9%), which was directly used in the next step.

**Step 3: UBI-1097e**

Compound **UBI-1097d** (450mg, 1.26mmol) and NaOH (200mg, 5.05 mmol) were dissolved in water (0.5mL), MeOH (3mL) and THF (3mL), and reacted at room temperature for 16 hours. After the completion of the reaction, the reaction was concentrated to obtain a crude product. Water (20 mL) was added, then extracted with ether (10 mL*3). The aqueous phase was acidified to pH=5 using dilute hydrochloric acid (1M), and then extracted with dichloromethane (10 mL*3). The organic phases were combined, then dried over anhydrous $Na_2SO_4$, and concentrated by rotary evaporation under reduced pressure to obtain compound **UBI-1097e** (370 mg, yield 64.4%). $^1$H NMR (400 MHz, chloroform-*d*) δ 3.32 (t, *J* = 6.6 Hz, 2H), 3.22 - 3.14 (m, 2H), 3.10 (t, *J* = 7.3 Hz, 2H), 2.18 (t, *J* = 7.3 Hz, 2H), 1.70 (t, *J* = 7.0 Hz, 2H), 1.54 - 1.42 (m, 4H), 1.39 (s, 9H), 1.25 (m, 4H).

**Step 4: UBI-1097f**

Compound **UBI-1097e** (370 mg, 1.13 mmol), **B(A1)** (292 mg, 1.13 mmol), and HATU (859 mg, 2.26) were dissolved in DMF (3 mL) and DIPEA (0.3 mL), and reacted at room temperature for 16 hours. The reaction solution was directly isolated by reversed-phase column (MeOH/$H_2O$=5% to 95%, 45 minutes, collected at 70%) to obtain the target compound **UBI-1097f** (420mg, yield 65.4%) as a white solid. $^1H$ NMR (400 MHz, DMSO-$d_6$) δ 11.02 (s, 1H), 9.75 (s, 1H), 7.82 (dd, *J* = 7.0, 2.0 Hz, 1H), 7.59 - 7.42 (m, 2H), 5.15 (dd, *J* = 13.3, 5.1 Hz, 1H), 4.51 - 4.27 (m, 2H), 3.23 - 3.08 (m, 4H), 2.92 (m, 1H), 2.61 (m, 1H), 2.41 - 2.29 (m, 3H), 2.02 (m, 1H), 1.71 (t, *J* = 6.9 Hz, 2H), 1.61 (t, *J* = 7.4 Hz, 2H), 1.38 (m, 17H). LC-MS: $[M+H]^+$ =570.2

**Step 5: UBI-1097**

Compound **UBI-1097f** (420mg, 0.74mmol) and Pd/C (42mg) were dissolved in MeOH (1 mL) and DCM (10mL), and reacted for 2 hours under hydrogen atmosphere. After the completion of reaction, the reaction was filtrated. The filtrate was concentrated to obtain the crude product. The crude product was washed with cold anhydrous ether (10 mL*3), and filtered to obtain target compound **UBI-1097** (340 mg, yield 85%) as white solid. $^1H$ NMR: (400 MHz, DMSO-$d_6$) δ 9.87 (d, *J* = 7.6 Hz, 1H), 7.82 (dd, *J* = 6.9, 2.1 Hz, 1H), 7.49 (d, *J* = 7.0 Hz, 2H), 5.15 (m, 1H), 4.37 (m, 2H), 3.21 - 3.15 (m, 3H), 3.11 (t, *J* = 7.4 Hz, 2H), 2.73 (t, *J* = 7.6 Hz, 2H), 2.37 (t, *J* = 7.4 Hz, 3H), 2.03 m, 1H), 1.76 (q, *J* = 7.7 Hz, 2H), 1.60 (q, *J* = 7.4 Hz, 2H), 1.39 (m, 15H). LCMS: $(M+H)^+$ = 543.3

**Synthesis method of Compound UBI-1098 ($NH_2$-12b-B(A1))**

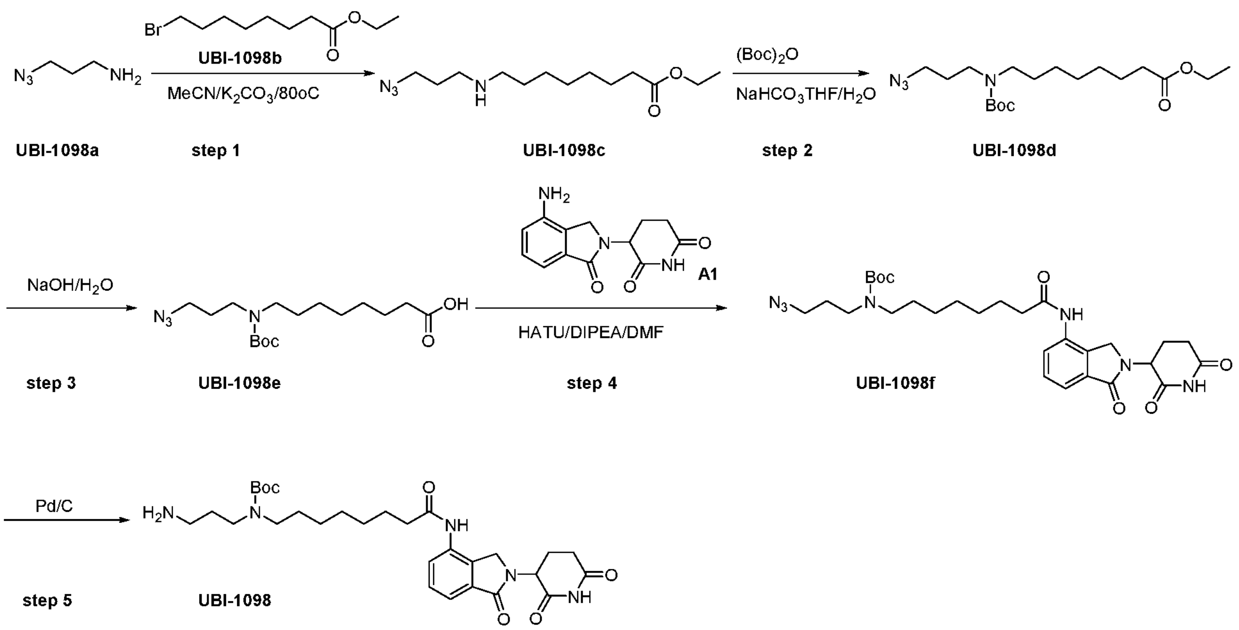


**Step 1: UBI-1098c**

Compound **UBI-1098a** (1.0 g, 10 mmol), **BUI-1098b** (2.5 g, 10 mmol) and $K_2CO_3$ (2.76 g, 20 mmol) were dissolved in $CH_3CN$ (100 mL), reacted at 80°C for 18 hours. The reaction solution was filtered, and the filtrate was concentrated to obtain crude product. The crude product was isolated by column chromatography (petroleum ether/ethyl acetate = 50% to 80%, then DCM/MeOH=0% to 10%) to obtain the target compound **UBI-1098c** (600mg, yield 22%) as colorless oil. $^1H$ NMR (400 MHz, chloroform-*d*) δ 4.12 (q, *J* = 7.1 Hz, 2H), 3.36 (t, *J* = 6.8 Hz, 2H), 2.69 (t, *J* = 7.0 Hz, 2H), 2.59 (t, *J* = 7.2 Hz, 2H), 2.28 (t, *J* = 7.5 Hz, 2H), 1.77 (p, *J* = 6.9 Hz, 2H), 1.66 - 1.58 (m, 2H), 1.52 - 1.44 (m, 2H), 1.33 (m, 6H), 1.25 (t, *J* = 7.1 Hz, 3H).

**Step 2: UBI-1098d**

Compound **UBI-1098c** (450mg, 2.22mmol), di-tert-butyl dicarbonate (960mg, 4.44mmol) and $NaHCO_3$ (373mg,

4.44mmol) were dissolved in THF (20mL) and reacted at room temperature for 2 hours. After the completion of reaction, water (10mL) was added, extracted with dichloromethane (10mL *3). The organic phases were combined, then washed with saturated brine, dried over anhydrous $Na_2SO_4$, and concentrated by rotary evaporation under reduced pressure to obtain the crude product **UBI-1098d** (600mg), which was directly used in the next step.

**Step 3: UBI-1098e**

Compound **UBI-1098d** (600mg, 1.62mmol), NaOH (260mg, 6.48mmol) were dissolved in water (0.5mL), MeOH (3mL) and THF (3mL), and reacted at room temperature for 16 hours. After the completion of the reaction, water (20 mL) was added, then extracted with ether (10 mL*3). The aqueous phase was acidified to pH=5 using dilute hydrochloric acid (1M), and then extracted with dichloromethane (10 mL*3). The organic phases were combined, then dried over anhydrous $Na_2SO_4$, and concentrated by rotary evaporation under reduced pressure to obtain compound **UBI-1098e** (700mg, yield 92%).
$^1$H NMR (400 MHz, chloroform-d) δ 3.31 (t, *J* = 6.6 Hz, 2H), 3.14 (m, *J* = 28.8, 7.2 Hz, 4H), 2.18 (t, *J* = 7.4 Hz, 2H), 1.71 (p, *J* = 6.8 Hz, 2H), 1.39 (s,13H), 1.31 - 1.12 (m, 6H).

**Step 4: UBI-1098f**

Compound **UBI-1098e** (700mg, 2mmol), **B(A1)** (518mg, 2mmol), and HATU (760mg, 4) were dissolved in DMF (3 mL) and DIPEA (0.3 mL), and reacted at room temperature for 16 hours. The reaction solution was directly isolated by reversed-phase column (MeOH/$H_2O$=5% to 95%, 45 minutes, collected at 70%) to obtain the target compound **UBI-1098f** (720mg, yield 62%) as a white solid. $^1$H NMR (400 MHz, DMSO-*d*$_6$) δ 11.02 (s, 1H), 9.75 (s, 1H), 7.82 (dd, *J* = 7.0, 2.0 Hz, 1H), 7.59 - 7.42 (m, 2H), 5.15 (dd, *J* = 13.3, 5.1 Hz, 1H), 4.51 - 4.27 (m, 2H), 3.23 - 3.08 (m, 4H), 2.92 (m, 1H), 2.61 (m, 1H), 2.41 - 2.29 (m, 3H), 2.02 (m, 1H), 1.71 (t, *J* = 6.9 Hz, 2H), 1.61 (t, *J* = 7.4 Hz, 2H), 1.38 (m, 19H). LC-MS : [M+H]+ =584.2

**Step 4: UBI-1098**

Compound **UBI-1098f** (700mg, 1.2mmol) and Pd/C (70mg) were dissolved in MeOH (1 mL) and DCM (10mL), and reacted for 2 hours under hydrogen atmosphere. After the completion of reaction, the reaction was filtrated. The filtrate was concentrated to obtain the crude product. The crude product was washed with cold anhydrous ether (10 mL*3), and filtered to obtain target compound UBI-1098 (400mg, yield 60%) as white solid. $^1$H NMR: (400 MHz, DMSO-*d*$_6$) δ 9.84 (s, 1H), 7.82 (dd, J = 7.0, 2.0 Hz, 1H), 7.56 - 7.39 (m, 2H), 5.15 (dd, J = 13.3, 5.1 Hz, 1H), 4.37 (d, J = 12.9 Hz, 2H), 3.33 (s, 2H), 3.17 (q, J = 5.4, 4.3 Hz, 2H), 3.10 (t, J = 7.4 Hz, 2H), 2.72 (t, J = 7.6 Hz, 2H), 2.36 (q, J = 8.7, 8.2 Hz, 3H), 2.08 - 1.98 (m, 1H), 1.74 (t, J = 7.5 Hz, 2H), 1.60 (t, J = 7.2 Hz, 2H), 1.48 - 1.30 (m, 15H), 1.23 (s, 2H).

**Synthesis method of Compound UBI-1158 ($NH_2$-9b-B(A1))**

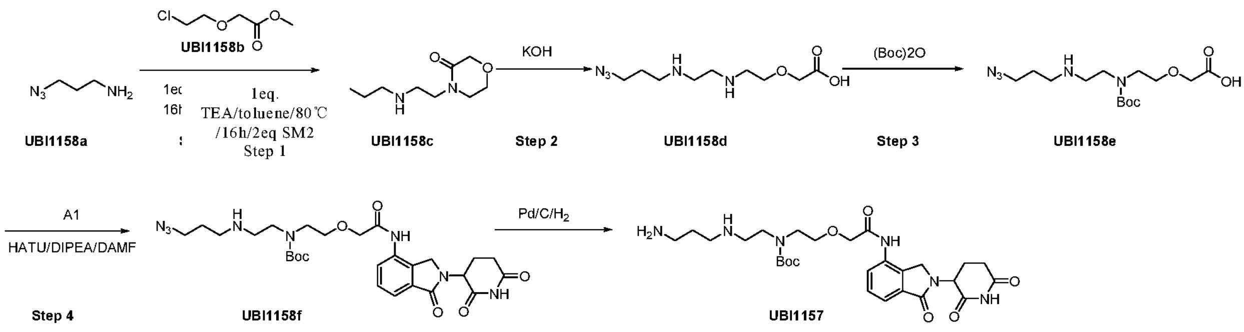


**Step 1: UBI-1158c**

Compound **UBI-1158a** (2g, 20mmol), **UBI-1158b** (3.65g, 24mmol) were dissolved in triethylamine (2.02g, 20mmol) and toluene (100mL), reacted at 80°C for 18 hours. The reaction solution was concentrated and isolated by silica gel column chromatography (petroleum ether/ethyl acetate = 50% to 100%, then MeOH/DCM=0% to 10%) to obtain the target compound **UBI-1158c** (1.4g) as colorless oil. $^1$H NMR (400 MHz, chloroform-d) δ 4.16 (s, 1H), 4.02 (s,

1H), 3.95 - 3.86 (m, 1H), 3.80 (dd, *J* = 6.0, 4.3 Hz, 1H), 3.70 (dd, *J*= 6.0, 4.3 Hz, 1H), 3.52 - 3.46 (m, 1H), 3.45 - 3.33 (m, 4H), 1.93 - 1.77 (m, 2H).

**Step 2: UBI-1158d**

Compound **UBI-1158c** (1.3g, 7.06mmol), KOH (1.19g, 21.2mmol) were dissolved in water (5mL), and refluxed at 100°C for 2 hours. The reaction liquid was directly used in the next step after cooling.

**Step 3: UBI-1158e**

To the aqueous solution of compound **UBI-1158d** (1.3g, 5.4mmol), di-tert-butyl dicarbonate (2.3g, 10.7mmol) and dioxane (30mL) were added, and reacted at room temperature for 2 hours. The reaction solution was concentrated, added water (20 mL), and extracted with ethyl acetate (10 mL*3). The aqueous phase was concentrated to obtain the crude product, which was dissolved in MeOH/DCM=10/1 (30 mL), filtered, and the filtrate was concentrated to obtain the target compound **UBI-1158e** (1.7g).

**Step 4: UBI-1158f**

Compound **UBI-1158e** (600mg, 1.99mmol), **B(A1)** (514mg, 1.99mmol), and HATU (1.51g, 3.98mmol) were dissolved in DMF (3 mL) and DIPEA (0.3 mL), and reacted at room temperature for 16 hours. The reaction solution was directly isolated by reversed-phase column (MeOH/$H_2O$=5% to 95%, 45 minutes, collected at 60%) to obtain the target compound **UBI-11158f** (500mg) as a white solid. LC-MS: $[M+H]^+$ =587.3

**Step 5: UBI-1158**

Compound **UBI-1157**(100mg, 0.17mmol) and Pd/C (20mg) were dissolved in DCM (10mL), and reacted for 16 hours under hydrogen atmosphere. After the completion of reaction, the reaction was filtrated. The filtrate was concentrated to obtain the target compound which was directly used in the next step. LC-MS: $(M+H)^+$= 561.3

**Synthesis method of Compound UBI-1089 ($NH_2$-11b-B(A1))**

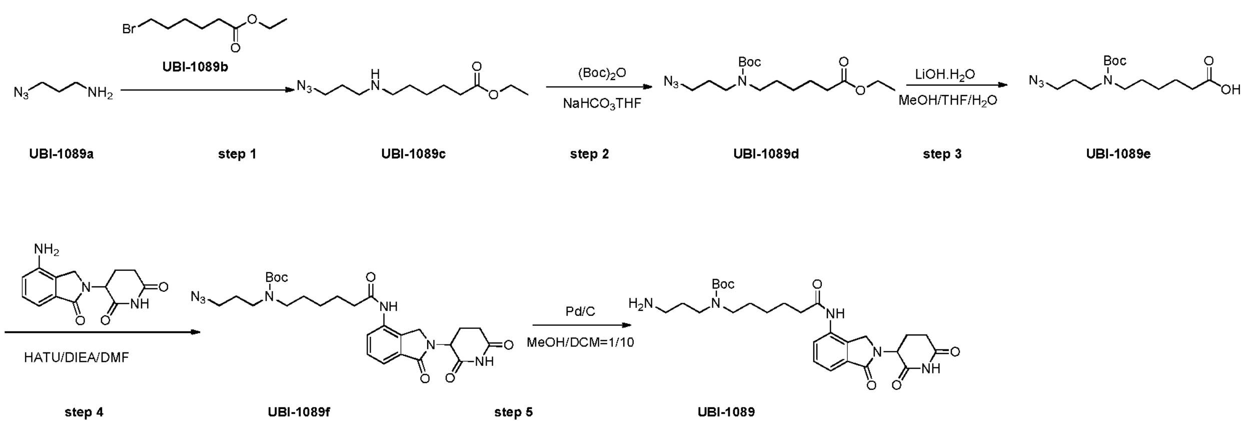


**Step 1: UBI-1089c**

Compound **UBI-1097a** (2.0 g, 20 mmol), **BUI-1097b** (3.57 g, 16 mmol) and $K_2CO_3$ (5.52 g, 40 mmol) were dissolved in acetonirile (15 mL), then reacted at 80°C for 18 hours. The reaction solution was filtered and then concentrated to obtain crude product. The crude product was passed through silica gel column chromatography (ethyl acetate/petroleum ether==50% to 100%, 20 minutes, methanol/dichloromethane=0% to 10%, 20 minutes) to obtain product **UBI-1089c** (460mg, yield 9.5%) as light yellow oil.
$^1$H NMR (400 MHz, chloroform-d) δ 4.13 (q, *J* = 7.1 Hz, 2H), 3.38 (dd, *J* = 7.6, 5.9 Hz, 2H), 2.73 (td, *J* = 7.0, 3.1 Hz, 2H), 2.64 (d, *J* = 6.9 Hz, 2H), 2.30 (t, *J* = 7.4 Hz, 2H), 1.88 - 1.76 (m, 2H), 1.65 (p, *J* = 7.5 Hz, 2H), 1.53 (m, 2H), 1.38 (dt, *J* = 10.2, 6.4 Hz, 2H), 1.25 (t, *J* = 7.1 Hz, 3H).

**Step 2: UBI-1089d**

The compound **UBI-1097c** (460 mg, 2 mmol), $(Boc)_2O$ (460 mg, **2** mmol) and $NaHCO_3$ (320 mg, 4 mmol) were dissolved in tetrahydrofuran (10 mL), and then reacted at room temperature for 2 hours. The reaction solution was added water (10 mL), and extracted with dichloromethane (10 mL*3). Organic phases were combined, dried over $Na_2SO_4$, and concentrated. After the concentration, the crude product **UBI-1089d** (770mg, 100% yield) as yellow oil was obtained, which was directly used in the next step.

**Step 3: UBI-1089e**

Compound **UBI-1089d** (770mg, 2mmol), LiOH (140mg, 3mmol) were dissolved in water (3mL), methanol (3mL) and tetrahydrofuran (6mL), and then reacted at room temperature for 18 hours. The reaction solution was concentrated and extracted with ethyl acetate (10mL*3), the aqueous phase was acidified to pH=5 using (1M) HCl. Then the aqueous phase was extracted with ethyl acetate (10mL*3). The organic phases were combined, then dried over anhydrous sodium sulfate and concentrated to obtain product **UBI-1089e** (450mg, yield 63.6%) as yellow oil.

**Step 4: UBI-1089f**

Compound **UBI-1089e** (450mg, 1.43mmol), **B(A1)** (371mg, 1.43mmol), and HATU (1.09g, 2.87) were dissolved in DMF (3 mL) and DIPEA (0.3 mL), and reacted at room temperature for 18 hours. The reaction was quenched with water (10 mL), then extracted with ethyl acetate (8 mL*3). The organic phases were combined, then dried over anhydrous sodium sulfate and concentrated to obtain crude product. The crude product was purified by reversed-phase column (methanol/water=5% to 95%, collected at 70%) to obtain product **UBI-1089f** (290mg, yield 36.5%) as colorless oil.
$^1$H NMR (400 MHz, DMSO-$d_6$) δ 11.02 (s, 1H), 9.76 (s, 1H), 7.81 (d, *J* = 6.8 Hz, 1H), 7.49 (d, *J* = 7.2 Hz, 2H), 5.14 (dd, *J* = 13.2, 5.2 Hz, 1H), 4.49 - 4.24 (m, 2H), 3.20 - 3.11 (m, 6H), 2.99 - 2.87 (m, 1H), 2.69 - 2.51 (m, 4H), 2.35 (q, *J* = 5.7, 4.0 Hz, 3H), 1.80 - 1.56 (m, 4H), 1.37 (s, 9H), 1.28 (m, 2H).

**Step 5: UBI-1089**

Under hydrogen atmosphere, compound **UBI-1089f** (290mg, 0.52mmol), Pd/C (29mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water=5% to 95%, collected at 15%) to obtain the product **UBI-1089** (180mg, yield 65%) as yellow oil.

**Synthesis method of Compound UBI-1087 ($NH_2$-8b-B(A1))**

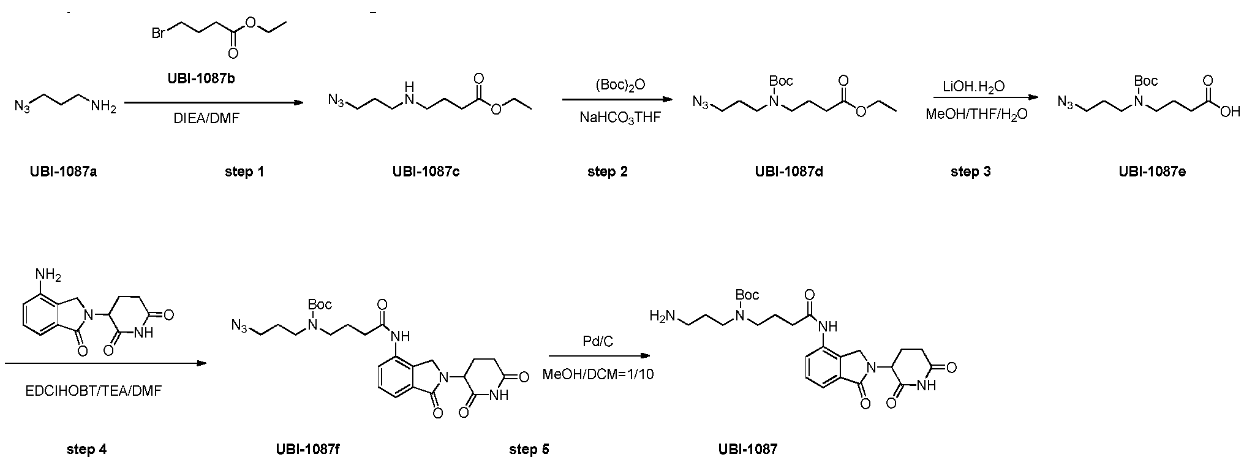


**Step 1: UBI-1087c**

Compound **UBI-1087a** (1.5 g, 15 mmol), **BUI-1087b** (1.46 g, 8 mmol) and DIEA (5.81 g, 45 mmol) were dissolved in DMF (15 mL), and reacted at room temperature for 18 hours. The reaction solution was added water (15 mL), and

extracted with ethyl acetate (15 mL*3). The crude product was obtained after concentration of the organic phase. The crude product was passed through silica gel column chromatography (ethyl acetate/petroleum ether=50% to 80%, 20 minutes, methanol/dichloromethane = 0% to 10%, 20 minutes) to obtain product **UBI-1087c** (480mg, yield 15%) as colorless oil.
$^1$H NMR (400 MHz, chloroform-d) δ 4.26 - 3.95 (m, 2H), 3.42 - 3.25 (m, 2H), 2.67 (dtd, *J*= 22.1, 7.0, 1.2 Hz, 4H), 2.36 (tt, *J* = 7.3, 1.8 Hz, 2H), 1.86 - 1.67 (m, 4H), 1.51 (s, 1H), 1.40 - 1.16 (m, 3H).

**Step 2: UBI-1087d**

The compound **UBI-1087c** (480mg, 2mmol), $(Boc)_2O$ (540mg, **2.2** mmol) and $NaHCO_3$ (380mg, 4 mmol) were dissolved in tetrahydrofuran (15 mL), and reacted at room temperature for 2 hours. The reaction solution was added water (10 mL), and extracted with ethyl acetate (10 mL*3). The organic phases were combined, dried over anhydrous sodium sulfate and then concentrated to obtain crude product **UBI-1087d** (600 mg, yield 85%) as yellow oil, the crude product was directly used in the next step.

**Step 3: UBI-1087e**

Compound **UBI-1087d** (600 mg, 1.9 mmol), LiOH water (88 mg, 2.1 mmol) were dissolved in water (2 mL), methanol (2 mL) and tetrahydrofuran (6 mL), reacted at room temperature for 18 hours.The reaction solution was concentrated and extracted with ethyl acetate (10 mL*3). The aqueous phase was acidified to pH=5 using (1M) HCl, and extracted with ethyl acetate (10 mL*3). The organic phases were combined and dried over anhydrous sodium sulfate, then concentrated to obtain product **UBI-1087e** (480mg, yield 77%) as yellow oil.
$^1$H NMR (400 MHz, DMSO-$d_6$) δ 12.03 (s, 1H), 3.68 - 3.55 (m, 2H), 3.22 - 3.09 (m, 4H), 2.17 (t, *J*= 7.2 Hz, 2H), 1.75 - 1.62 (m, 4H), 1.39 (s, 9H).

**Step 4: UBI-1087f**

Compound **UBI-1087e** (420 mg, 1.47 mmol), **B(A1)** (380 mg, 1.47 mmol), EDCI (564 mg, 2.94 mmol), HOBT (595 mg, 4.41 mmol) and TEA (445 mg, 4.41 mmol) were dissolved in DMF (12 mL) and reacted at room temperature for 18 hours. The reaction solution was quenched by the addition of water (10 mL), then extracted with ethyl acetate (15 mL*3). The organic phases were combined, then washed with saturated brine, dried over anhydrous sodium sulfate and purified by reversed-phase column to obtain product **UBI-1087f** (280 mg, yield 36%) as yellow solid.
$^1$H NMR (400 MHz, DMSO-$d_6$) δ 12.06 (s, 1H), 11.02 (s, 1H), 9.81 (s, 1H), 7.80 (d, *J* = 7.0 Hz, 1H), 7.64 - 7.28 (m, 2H), 5.15 (dd, *J* = 13.3, 5.1 Hz, 1H), 4.49 - 4.21 (m, 2H), 2.93 (ddd, *J* = 18.6, 13.5, 5.3 Hz, 1H), 2.61 (d, *J* = 18.3 Hz, 2H), 2.38 - 2.32 (m, 2H), 2.17 (t, *J* = 7.3 Hz, 2H), 2.06 - 1.95 (m, 1H), 1.88 - 1.59 (m, 8H), 1.39 (d, *J* = 1.8 Hz, 9H).

**Step 5: UBI-1087**

Under hydrogen atmosphere, compound **UBI-1087f** (280mg, 0.53mmol), Pd/C (28mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water = 5% to 95%, collected at 20%) to obtain the product **UBI-1087** (80mg, yield 30%) as yellow oil.

**Synthesis method of Compound UBI-1088 ($NH_2$-9b-B(A1))**

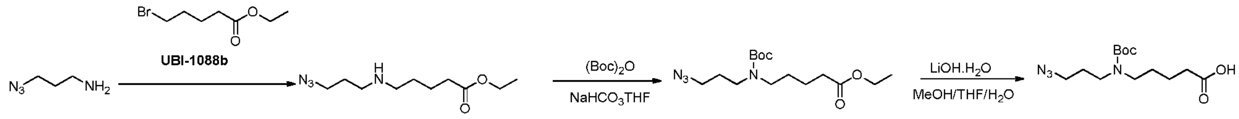


UBI-1088a step 1 UBI-1088c step 2 UBI-1088d step 3 UBI-1088e

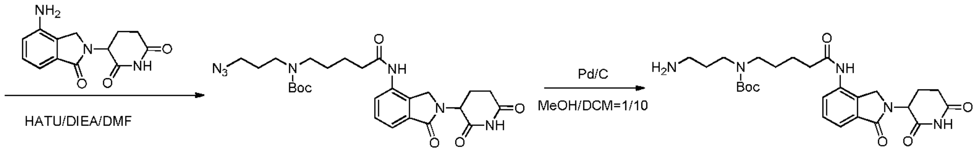


step 4 UBI-1088f step 5 UBI-1088

**Step 1: UBI-1088c**

Compound **UBI-1188a** (2g, 20mmol), **BUI-1188b** (3.34g, 16mmol) and $K_2CO_3$ (5.52g, 40mmol) were dissolved in acetonirile (15 mL), then the reaction was reacted at room temperature for 18 hours. The reaction solution was filtered. The filtrate was concentrated and passed through silica gel column chromatography (ethyl acetate/petroleum ether=50% to 100%, 20 minutes, methanol/dichloromethane = 0% to 10%, 30 minutes) to obtain product **UBI-1188c** (700 mg, yield 15%) as light yellow oil.

**Step 2: UBI-1088d**

Compound **UBI-1088c** (700mg, 3.1mmol), $(Boc)_2O$ (740mg, 3.4mmol) and $NaHCO_3$ (520mg, 6.2mmol) were dissolved in tetrahydrofuran (15mL) and reacted at room temperature for 2 hours. The reaction solution was added water (10 mL), then extracted with ethyl acetate (10 mL*3). The organic phases were combined, dried over anhydrous sodium sulfate and then concentrated to obtain crude product **UBI-1088d** (1 g, yield 100%) as yellow oil, the crude product was directly used in the next step.

**Step 3: UBI-1088e**

Compound **UBI-1088d** (1g, 3 mmol), LiOH.water (190 mg, 5 mmol) were dissolved in water (2 mL), methanol (2 mL) and tetrahydrofuran (6 mL), reacted at room temperature over night.The reaction solution was concentrated and then extracted with ethyl acetate (10 mL*3). The aqueous phase was acidified to pH=5 using (1M) HCl, and extracted with ethyl acetate (10 mL*3). The organic phases were combined and dried over anhydrous sodium sulfate to obtain crude product **UBI-1088e** (600mg, yield 66%) as yellow oil.

**Step 4: UBI-1088f**

Compound **UBI-1088e** (640mg, 2.13mmol), **B(A1)** (497mg, 1.92mmol), and HATU (1.62g, 4.27) were dissolved in DMF (8 mL) and DIPEA (0.5 mL), and reacted at room temperature for 18 hours. The reaction solution was directly purified via reversed-phase column (methanol/water = 5 % to 95 %, collected at 70%) to obtain product **UBI-1088f** (380mg, yield 33%) as light yellow oil.
$^1H$ NMR (400 MHz, DMSO-$d_6$) $\delta$ 11.02 (s, 1H), 9.77 (s, 1H), 7.81 (dd, *J* = 7.0, 2.0 Hz, 1H), 7.57-7.43 (m, 2H), 5.15 (dd, *J* = 13.3, 5.1 Hz, 1H), 4.45 - 4.26 (m, 2H), 3.23 - 3.06 (m, 6H), 3.00 - 2.85 (m, 1H), 2.69 - 2.56 (m, 2H), 2.38 (t, *J*= 7.0 Hz, 2H), 2.10 - 1.98 (m, 1H), 1.77 - 1.66 (m, 2H), 1.54 (m, 4H), 1.39 (s, 9H).

**Step 5: UBI-1088**

Under hydrogen atmosphere, compound **UBI-1188f** (390mg, 0.72mmol), Pd/C (39mg) were dissolved in methanol (1 mL) and dichloromethane (10mL), and reacted at room temperature for 1 hour. The reaction solution was filtered on Celite. The filtrate was concentrated to obtain crude product **UBI-1088** (350 mg, yield 95%) as yellow oil, the crude product was directly used in the next step.

**Synthesis method of Compound UBI-1141 ($NH_2$-9b-B(A1))**

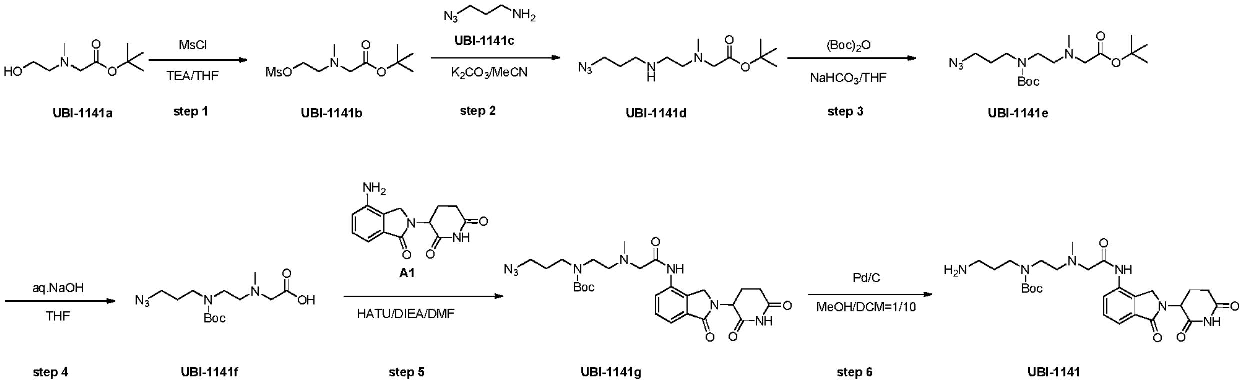


**Step 1: UBI-1141b**

After Compound **UBI-1141a** (2.0 g, 10.6 mmol) was dissolved in tetrahydrofuran (25 mL), TEA (1.6 g, 15.9 mmol) was added, and then MsCl (1.5 g, 12.7 mmol) was added at 0°C. The reaction was reacted at room temperature for 18 hours. The reaction solution was quenched by adding water (30 mL), then extracted with ethyl acetate (20 mL*3). The organic phases were combined, dried over anhydrous sodium sulfate and concentrated to obtain crude product **UBI-1141b** (2.5 g, yield 89%) as yellow oil. The crude product was directly used in the next step.

**Step 2: UBI-1141d**

Compound **UBI-1141c** (1.3 g, 13mmol), **BUI-1141b** (2.78g, 10mmol) and $K_2CO_3$ (2.59 g, 26mmol) were dissolved in acetonirile (15 mL), then reacted at RT for 18 hours. The reaction solution was filtered. The filtrate was concentrated and passed through silica gel column chromatography (ethyl acetate/petroleum ether=50% to 100%, 20 minutes, methanol/dichloromethane = 0% to 10%, 30 minutes) to obtain product **UBI-1141d** (450 mg, yield 16%) as light yellow oil.

**Step 3: UBI-1141e**

Compound **UBI-1141d** (450mg, 1.7mmol), **$(Boc)_2O$** (543mg, 2.5mmol) and **$NaHCO_3$** (279mg, 3.3mmol) were dissolved in tetrahydrofuran (15mL) and reacted at room temperature for 2 hours. The reaction solution was quenched with water (10 mL), and extracted with ethyl acetate (10 mL*3). The organic phases were combined, dried over anhydrous sodium sulfate and concentrated to obtain crude product **UBI-1141e** (550 mg, yield 89%) as light yellow oil. The crude product was directly used in the next step. $^1$H NMR (400 MHz, chloroform-d) δ 3.30 (q, $J$ = 7.1 Hz, 6H), 3.19 (s, 2H), 2.66 (s, 2H), 2.41 (s, 3H), 1.81 (p, $J$ = 6.9 Hz, 2H), 1.46 (d, $J$ = 2.7 Hz, 18H).

**Step 4: UBI-1141f**

Compound **UBI-1141e** (550 mg, 1.5 mmol) was dissolved in 50% aq. NaOH (10 mL) and tetrahydrofuran (10 mL), and reacted at room temperature for 18 hours. The reaction solution was concentrated and then extracted with ethyl acetate (10 mL) once. The aqueous phase was acidified to pH=5 using (1M) HCl, and extracted with ethyl acetate (10 mL*3). The organic phases were combined, dried over anhydrous sodium sulfate and concentrated to obtain crude product **UBI-1141f** (400mg, yield 86%) as light yellow oil. $^1$H NMR (400 MHz, chloroform-d) δ 3.39 - 3.14 (m, 8H), 2.83 (s, 2H), 2.53 (s, 3H), 1.85 - 1.68 (m, 2H), 1.45 (s, 9H).

**Step 5: UBI-1141g**

Compound **UBI-1141f** (200mg, 0.63mmol), **B(A1)** (148mg, 0.57mmol), and HATU (483mg, 4.27) were dissolved in DMF (8 mL) and DIPEA (0.5 mL), and reacted at room temperature for 16 hours. The reaction solution was directly purified via reversed-phase column (methanol/water = 5 % to 95 %, collected at 70%) to obtain product **UBI-11141g** (30mg, yield 9%) as yellow oil.

**Step 6: UBI-1141**

Under hydrogen atmosphere, compound **UBI-1141g** (50mg, 0.09mmol), Pd/C (5mg) were dissolved in methanol (1 mL) and dichloromethane (10mL), and the reaction was reacted at room temperature for 1 hour. The reaction solution was filtered on Celite. The filtrate was concentrated to obtain crude product **UBI-1141** (50 mg, yield 100%). The crude product was directly used in the next step.

**Synthesis method of Compound UBI-1075 ($NH_2$-9b-B(A1))**

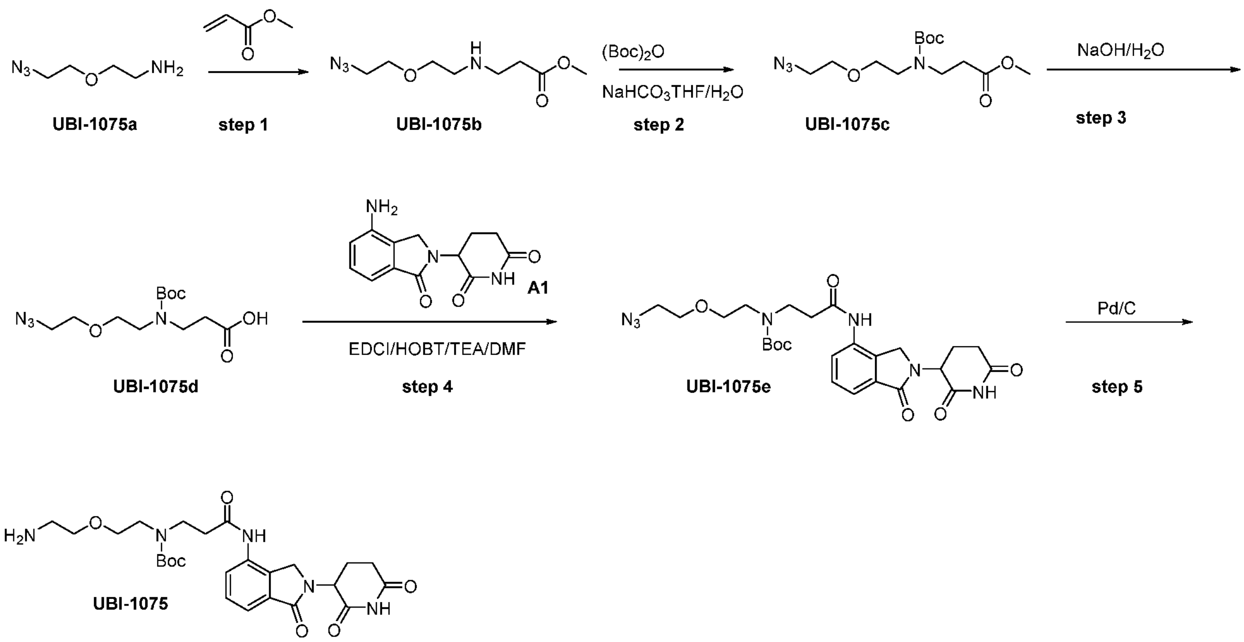

UBI-1075a UBI-1075b UBI-1075c UBI-1075d UBI-1075e UBI-1075

**Step 1: UBI-1075b**

Compound **UBI-1075a** (1.0 g, 7.69 mmol) and methyl acrylate (984 mg, 7.69 mmol) were dissolved in acetonitrile (20 mL), and reacted at 80 ° C for 18 hours. After the completion of reaction, the reaction solution was poured into 5 mL water and extracted with ethyl acetate (5 mL*3). The organic phases were combined, then washed with saturated brine, dried over anhydrous $Na_2SO_4$, concentrated by rotary evaporation under reduced pressure to obtain crude product. The crude product was isolated by silica gel column chromatography (DCM/MeOH = 10/1) to obtain compound as colorless oil (660 mg, yield 40%).

$^1$H NMR (400 MHz, d6-DMSO) δ3.67 - 3.62 (m, 1H), 3.60 - 3.57 (m, 5H), 3.48- 3.43 (m, 2H), 3.39 - 3.36 (m, 2H), 2.77-2.74 (m, 2H), 2.70-2.63(m, 2H),2.43-2.40(m, 2H).

**Step 2: UBI-1075c**

Compound **UBI-1075b** (660 mg, 3.06 mmol), di-tert-butyl dicarbonate (975 mg, 4.58 mmol) and sodium bicarbonate (771 mg, 9.18 mmol) were added to tetrahydrofuran (20mL) and reacted at room temperature for 2 hours. After the completion of reaction, it was poured into 10 mL of water and extracted with dichloromethane (5 mL*3). The organic phases were combined and washed with saturated brine, dried over anhydrous $Na_2SO_4$, and concentrated by rotary evaporation under reduced pressure to obtain compound **UBI-1075c** (490 mg, yield 64%). $^1$H NMR (400 MHz, d6-DMSO) δ3.60 - 3.56 (m, 5H), 3.51 - 3.48 (m, 2H), 3.43-3.38 (m, 4H), 1.43 (s, 9H), 1.38 (s, 4H).

**Step 3: UBI-1075d**

Compound **UBI-1075c** (316 mg, 1.0 mmol) and sodium hydroxide (200 mg, 5.0 mmol) were added to a system of water/methanol/tetrahydrofuran (0.5mL/3mL/3mL), and reacted at room temperature for 16 hours. After the completion of reaction, the organic solvent was removed by concentration, and the aqueous phase was acidified to pH=5 using

hydrochloric acid (1M). Then it was extracted with dichloromethane (10ml*3), the combined organic phase was dried over anhydrous $Na_2SO_4$, and concentrated to obtain the desired compound **UBI-1075d** (170mg, yield 56.3%). $^1H$ NMR (400 MHz, d6-DMSO) δ 12.16 (s, 1H), 3.60-3.57 (m, 2H), 3.54-3.49(m,2H),3.46(dt, *J* = 11.1, 5.1 Hz, 4H), 3.47 - 3.38 (m, 2H), 2.48- 2.40 (m, 2H),1.39 (s, 9H).

**Step 4: UBI-1075e**

Compound **UBI-1075d** (268 mg, 0.88 mmol), **B(A1)** (230 mg, 0.88mmol), HATU (337mg, 0.97) and DIPEA (0.3mL) were added to anhydrous DMF (3mL). The mixture was reacted at room temperature for 16 hours. After the completion of reaction, the organic solvent was removed by concentration. The crude product was isolated by reverse chromatography on silica gel column chromatography (MeOH/$H_2O$ = 5% to 95%, 45mins, collected at 60%) to obtain compound **UBI-1075e** (350 mg, yield 72.7%) as colorless oil.

**Step 5: UBI-1075**

Compound **UBI-1075e** (200 mg, 0.37 mmol), 10% palladium on carbon (42 mg) was added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound UBI-1075 (169 mg, yield 88.9%) as colorless oil.

**Synthesis method of Compound UBI-1093 ($NH_2$-10b-B(A1))**

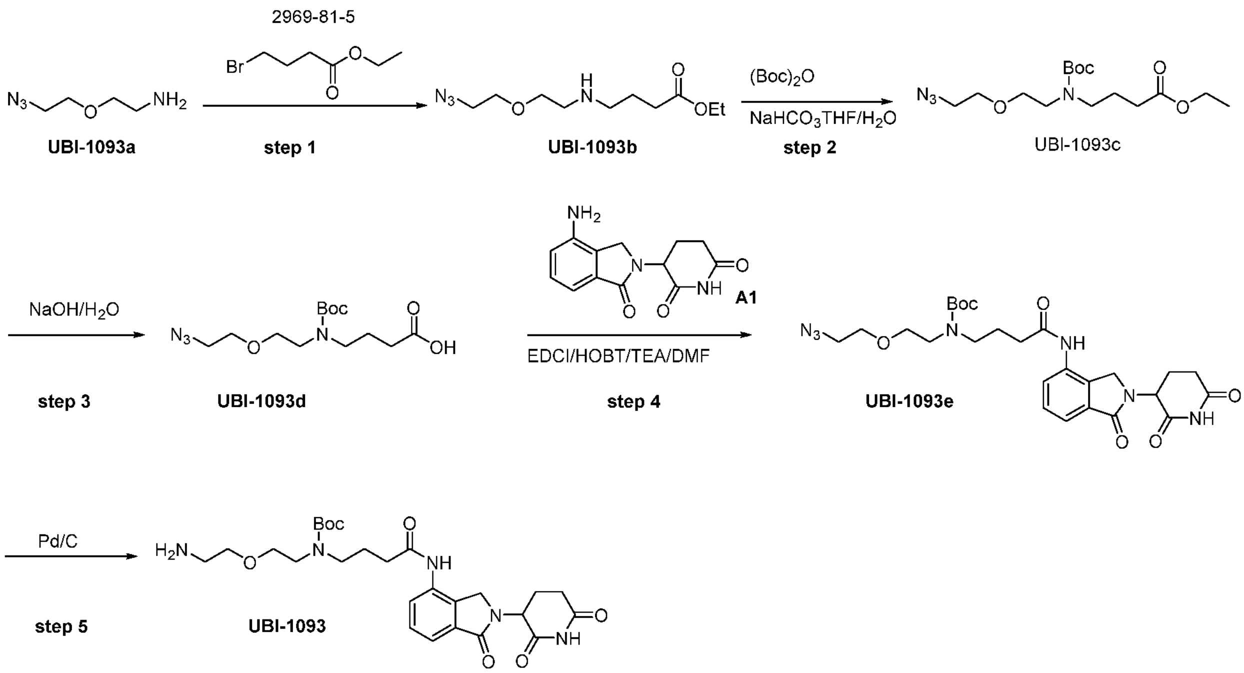


**Step 1: UBI-1093b**

Compound **UBI-1093a** (970 mg, 7.46 mmol), ethyl 4-bromobutyrate (1.45 g, 7.46 mmol) and $K_2CO_3$ (2.06 g, 14.9 mmol) were dissolved in acetonitrile (20 mL), and reacted at 80° C for 18 hours, filtered after the completion of reaction, the filtrate was concentrated by rotary evaporation under reduced pressure, and isolated by silica gel column chromatography (DCM/MeOH = 10/1) to obtain compound **UBI-1093b** (710 mg, yield 22 %) as colorless oil.
$^1H$ NMR (400 MHz, d6-DMSO) δ 4.09 - 4.02 (m, 2H), 3.62 - 3.58 (m, 2H), 3.52 - 3.48 (m, 2H), 3.41 - 3.37 (m, 2H), 2.68 - 2.64(m, 2H), 2.58 - 2.54 (m, 2H), 2.37 - 2.35 (m, 2H), 1.63 (ddd, *J*= 13.8, 10.9, 7.0 Hz, 2H), 1.19 - 1.16 (m, 3H).

**Step 2: UBI-1093c**

Compound **UBI-1093b** (350 mg, 1.43 mmol), di-tert-butyl dicarbonate (469 mg, 2.15 mmol) and sodium bicarbonate (360 mg, 4.29mmol) were added to tetrahydrofuran (20mL) successively and reacted at room temperature for 2 hours. After the completion of reaction, the reaction was poured into 10 mL of water and extracted with dichloromethane (5 mL*3). The organic phases were combined, then washed with saturated brine, dried over anhydrous $Na_2SO_4$, and concentrated by rotary evaporation under reduced pressure to obtain compound **UBI-1093c** (320 mg, yield 71%) as colorless oil.

**Step 3: UBI-1093d**

Compound **UBI-1093c** (300 mg, 0.87 mmol) and sodium hydroxide (69.6 mg, 1.74mmol) were added to a system of water/methanol/tetrahydrofuran (0.5mL/3mL/3mL), and reacted at room temperature for 16 hours. After the completion of reaction, the organic solvent was removed by concentration, and the aqueous phase was acidified to pH=5 using hydrochloric acid (1M). Then it was extracted with dichloromethane (10ml*3), the combined organic layers was dried over anhydrous $Na_2SO_4$, and concentrated to obtain desired compound **UBI-1093d** (250 mg, yield 91%) as colorless oil.

$^1$H NMR (400 MHz, d6-DMSO) $\delta$ 12.03 (s, 1H), 3.67 - 3.51 (m, 4H), 3.51 - 3.30 (m, 4H), 3.24 (dt, *J*= 12.6, 6.0 Hz, 2H), 2.16 (t, *J* = 7.2 Hz, 2H), 1.73 - 1.69 (m, 2H), 1.39 (s, 9H).

**Step 4: UBI-1093e**

Compound **UBI-1093d** (254 mg, 0.80 mmol), **B(A1)** (230 mg, 0.88mmol), HATU (337mg, 0.97) and DIPEA (0.3mL) were added to anhydrous DMF (3mL), reacted at room temperature for 16 hours. After the completion of reaction, the reaction was concentrated. The crude was isolated by silica gel column chromatography (MeOH/DCM = 1% to 10%) to obtain compound **UBI-1158c** (220mg, yield 49%) as colorless oil.

**Step 4: UBI-1093**

Compound **UBI-1093e** (200 mg, 0.35 mmol), 10% palladium on carbon (20 mg) was added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain the compound **UBI-1093** (170 mg, yield 89.4%) as colorless oil.

**Synthesis Method of Compound UBI-1094 ($NH_2$-11b-B(A1))**

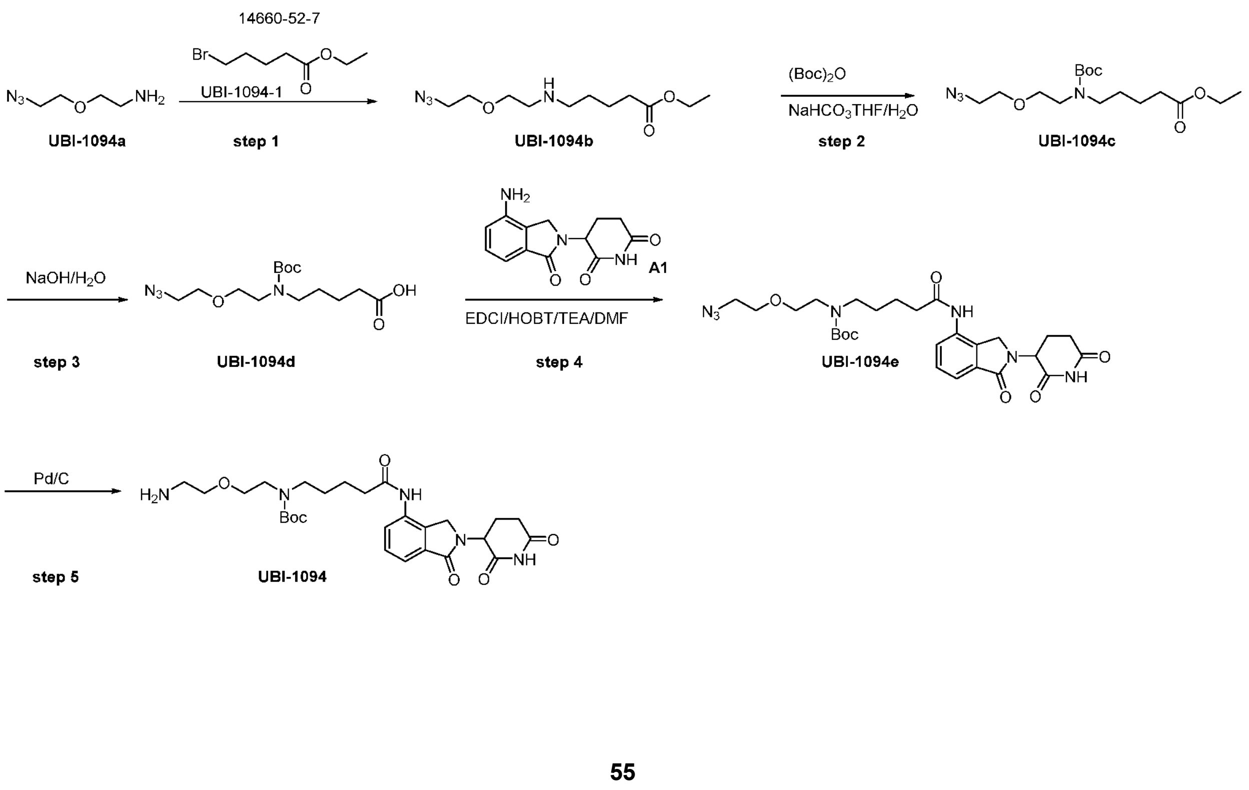

**Step 1: UBI-1094b**

Compound **UBI-1094a** (1.0g, 7.69 mmol), **UBI-1094-1** (1.6g, 7.69 mmol) and triethylamine (2.02g, 20mmol) were added to anhydrous toluene (10 mL). The mixture was reacted at 80 °C for 18 hours After the completion of reaction, the reaction solution was concentrated. The crude was isolated by silica gel column chromatography (DCM/MeOH = 10/1) to obtain compound **UBI-1094b** (740 mg, yield 37 %) as colorless oil.

**Step 2: UBI-1094c**

Compound **UBI-1094b** (350 mg, 1.35 mmol), di-tert-butyl dicarbonate (441 mg, 2.03 mmol) and sodium bicarbonate (360 mg, 4.29 mmol) were added to dioxane (20mL) successively, reacted at room temperature for 2 hours. After the completion of reaction, the reaction solution was poured into 10 mL of water, extracted with ethyl acetate (10 mL*3). The organic phases were combined, then washed with saturated brine, dried over anhydrous $Na_2SO_4$, and concentrated by rotary evaporation under reduced pressure to obtain compound **UBI-1094c** (390 mg, yield 87%) as colorless oil.

**Step 3: UBI-1094d**

Compound **UBI-1094c** (390 mg, 1.09 mmol), sodium hydroxide (1.19g, 21.2mmol) were added to water (25 mL) successively, reacted at 30°C for 12 hours. After the completion of reaction, it was concentrated and the aqueous phase was acidified to pH=5 using hydrochloric acid (1M). Then it was extracted with dichloromethane (10ml*3), the combined organic layers was dried over anhydrous $Na_2SO_4$, and concentrated to obtain target compound **UBI-1094d** (250 mg, yield 91%).
$^1$H NMR (400 MHz, d6-DMSO) $\delta$ 11.99 (s, 1H), 3.69 - 3.48 (m, 4H), 3.39 - 3.27 (m, 6H), 2.22 (t, *J* = 6.9 Hz, 2H), 1.47 (s, 4H), 1.36 (s, 9H).

**Step 4: UBI-1094e**

Compound **UBI-1093d** (210 mg, 0.665 mmol), **B(A1)** (160 mg, 0.618 mmol), HATU (305 mg, 0.803mmol) and DIPEA (0.5mL) were added to anhydrous DMF (5mL), and reacted at room temperature for 16 hours, concentrated after the completion of reaction. The crude product was isolated by reverse chromatography on silica gel column chromatography (DCM/MeOH=10/1) to obtain compound **UBI-1093e** (157 mg, 43% yield) as white solid.
LC-MS: $[M-100+H]^+$ =472.3

**Step 5: UBI-1094**

Compound **UBI-1094e** (100 mg, 0.17mmol), 10% palladium on carbon (20 mg) was added to a mixed solvent of methanol/dichloromethane (1 mL/10 mL), and reacted at room temperature for 2 hours under hydrogen atmosphere. The reaction solution was filtrated, and the filtrate was concentrated to obtain the crude product. The crude product was washed with cold ether (10 mL*3), and dried to obtain target compound **UBI-1094** (81 mg, yield 85%) as colorless oil.
LCMS: $(M-100+H)^+$ = 446.3

**Synthesis Method of Compound UBI-1095 ($NH_2$-14b-B(A1))**

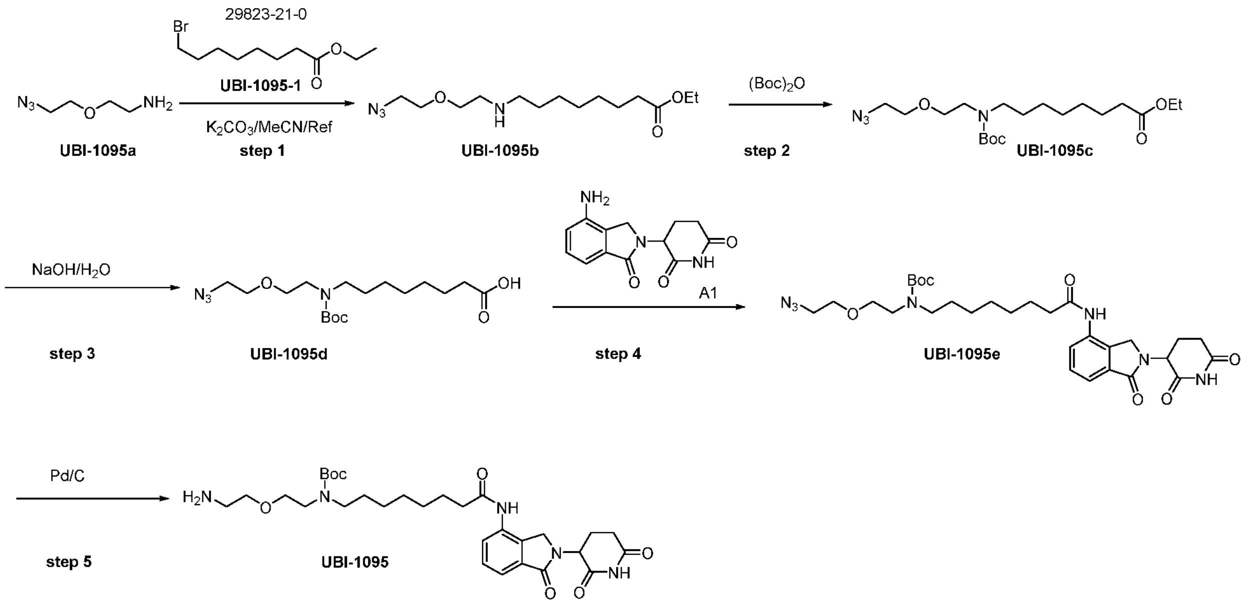


**Step 1: UBI-1095b**

Compound **UBI-1095a** (2g, 15.3mmol), **UBI-1095-1**(3.80g, 15.3mmol) and triethylamine (2.02g, 20mmol) were added to anhydrous toluene (100 mL), and reacted at 80 °C for 18 hours, concentrated after the completion of reaction. The crude product was isolated by silica gel column chromatography (DCM/MeOH = 10/1) to obtain compound **UBI-1095b** (740 mg, yield 16 %) as colorless oil.

**Step 2: UBI-1095c**

Compound **UBI-1095b** (1.3g, 3.7 mmol), di-tert-butyl dicarbonate (2.3g, 10.7mmol) and sodium bicarbonate (360 mg, 4.29mmol) were added to dioxane (30mL) successively and reacted at room temperature for 2 hours. After the completion of the reaction, the reaction solution was poured into 10 mL of water and extracted with ethyl acetate (10 mL*3). The organic phases were combined, then washed with saturated brine, dried over anhydrous $Na_2SO_4$, and concentrated by rotary evaporation under reduced pressure to obtain compound **UBI-1095c** (1.4g, yield 83%).
$^1$H NMR (400 MHz, d6-DMSO) $\delta$ 12.03 (s, 1H), 3.63 - 3.51 (m, 4H), 3.45 - 3.41 (m, 2H),3.32-3.29 (m, 2H), 3.24 (dt, $J$ = 12.6, 6.0 Hz, 2H), 2.16 (t, $J$ = 7.2 Hz, 2H), 1.54 (d, $J$ = 12.2 Hz, 4H), 1.39 (s, 9H), 1.35 - 1.28 (m, 6H).

**Step 3: UBI-1095d**

Compound **UBI-1095c** (1.5g, 6.0 mmol), sodium hydroxide (1.19g, 21.2mmol) were added to water (50 mL) successively, reacted at 30°C for 12 hours. After the completion of reaction, it was concentrated and the aqueous phase was acidified to pH=5 using hydrochloric acid (1M). Then it was extracted with dichloromethane (10ml*3), the combined organic layer was dried over anhydrous $Na_2SO_4$, and concentrated to obtain the desired compound **UBI-1095d** (1.2 g, yield 83%).

**Step 4: UBI-1095e**

Compound **UBI-1095d** (600 mg, 1.57 mmol), **B(A1)** (406 mg, 1.57 mmol), HATU (1.51g, 3.98 mmol) and DIPEA (0.5mL) were added to anhydrous DMF (5 mL),and reacted at room temperature for 16 hours, concentrated after the completion of reaction. The crude product was isolated by reverse chromatography on silica gel column chromatography (DCM/MeOH=10/1) to obtain compound **UBI-1095e** (500 mg, yield 51%) as colorless oil.
LC-MS: $[M-100+H]^+$ =515.3

**Step 5: UBI-1095**

Compound **UBI-1095e** (100 mg, 0.17mmol), 10% palladium on carbon (20 mg) was added to a mixed solvent of methanol/dichloromethane (1 mL/10 mL), and reacted at room temperature for 16 hours under hydrogen atmosphere.

After filtration, the filtrate was concentrated to obtain the crude product. The crude product was washed with cold ether (10 mL*3), and dried to obtain target compound **UBI-1095** (83 mg, yield 86%) as white solid.
LCMS: $(M-100+H)^+$ = 489.3

**Synthesis method of Compound UBI-1100 ($NH_2$-14b-B(A1))**

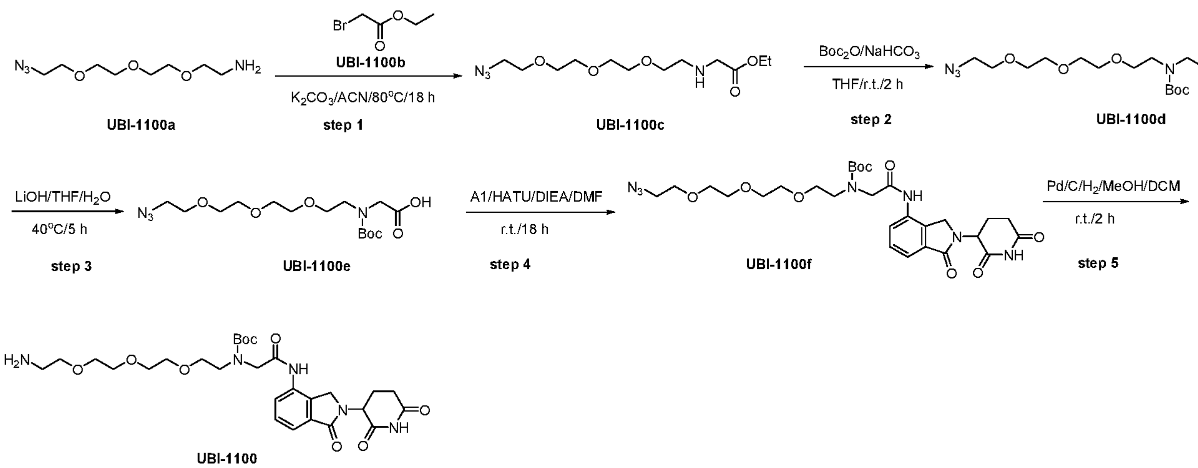


**Step 1: UBI-1100c**

Compound **UBI-1100a** (1.0 g, 4.58 mmol) was dissolved in acetonitrile (30 mL), added anhydrous potassium carbonate (949.8 mg, 6.87 mmol) and **UBI-1160b** (765.2 mg, 4.58 mmol), and then heated to 80 °C reacted for 18 hours. The reaction was cooled to room temperature and filtered. The filtrate was concentrated and subjected to silica gel column chromatography (petroleum ether/ethyl acetate = 50% to 100%, then dichloromethane /methanol = 0% to 10%) to obtain product **UBI-1100c** (0.6 g, yield 43%) as white solid.

**Step 2: UBI-1100d**

Compound **UBI-1100c** (0.6 g, 1.97 mmol) was dissolved in tetrahydrofuran (20 mL), added sodium bicarbonate (331.2 mg, 3.94 mmol) and $Boc_2O$ (0.64 g, 2.92 mmol). The reaction was reacted at room temperature for 2 hours. The reaction was quenched with water (20 mL), then extracted with ethyl acetate (20 mL*3). The organic phases were combined, dried over anhydrous sodium sulfate and concentrated to obtain crude product **UBI-1100d (V1084-016,** 400 mg, yield 50.2%) as yellow oil. LCMS $[M-100]^+$ =305.1

**Step 3: UBI-1100e**

Compound **UBI-1100d** (400 mg, 1 mmol), lithium hydroxide (47 mg, 1.98 mmol) were dissolved in tetrahydrofuran (20 mL) and water (10 mL). The system was reacted at 40°C for 5 hours. After complete hydrolysis, the reaction was added water (15 mL), extracted with ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1100e (V1084-019,** 320 mg, yield 86%) as colorless oil. $^1H$ NMR (400 MHz, chloroform-*d*) $\delta$ 4.00 (s, 2H), 3.72 - 3.63 (m, 10H), 3.52 (s, 2H), 3.42 (d, *J* = 5.2 Hz, 2H), 1.46 (d, *J* = 10.2 Hz, 11H). LCMS $[M-100]^+$ =277.2

**Step 4: UBI-1100f**

Compound UBI-1100e (200 mg, 0.53 mmol), B(A1) (137.7 mg, 0.53 mmol), HATU (303 mg, 0.8 mmol), DIPEA (0.5 mL) were dissolved in DMF (5 mL) and reacted at room temperature overnight. After the completion of reaction, it was purified by reversed-phase column (MeOH/H2O = 5 % to 95 %, 45 minutes, collected at 70%). After purification, product UBI-1100f (V1084-035, 180 mg, yield 54.9%) was obtained as yellow oil. $^1H$ NMR (400 MHz, chloroform-d) $\delta$ 9.09 (s, 1H), 7.99 (s, 1H), 7.72 (s, 2H), 7.48 (t, *J* = 7.7 Hz, 1H), 5.22 (dd, *J* = 13.2, 5.2 Hz, 1H), 4.41 (s, 2H), 3.62 (d, *J* =35.7 Hz, 12H), 3.33 (s, 2H), 2.95 - 2.80 (m, 2H), 2.42 - 2.31 (m, 1H), 2.27 - 2.21 (m, 1H), 1.47 (s, 9H). LCMS [M-100]

$^+$ = 518.1

**Step 5: UBI-1100**

Compound **UBI-1100e** (100 mg, 0.16 mmol) was dissolved in dichloromethane (10 mL) and methanol (1 mL), added Pd/C (20 mg), and reacted out at room temperature for 2 hours under hydrogen atmosphere. The reaction solution was filtered on Celite. The filtrate was concentrated to obtain product **UBI-1100f** (130 mg, yield 100%) as yellow oil. LCMS $[M+H]^+$ = 592.2

**Synthesis method of Compound UBI-1071 ($NH_2$-15b-B(A1))**

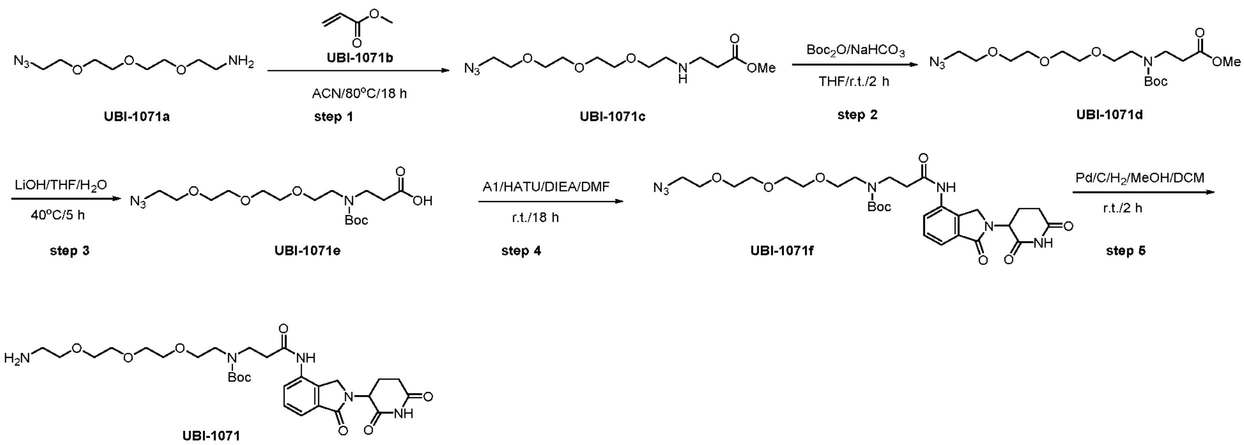


**Step 1: UBI-1071c**

Compound **UBI-1071a** (1.0 g, 4.58 mmol) and **UBI-1071b** (0.39 g, 4.58 mmol) were dissolved in acetonitrile (30 mL), and then heated to 80 °C and reacted for 18 hours. The reaction solution was concentrated and subjected to silica gel column chromatography (petroleum ether/ethyl acetate = 50% to 100%, then dichloromethane/methanol = 0% to 10%) to obtain product **UBI-1071c** (0.4 g, yield 28.7%) as yellow oil.
$^1$H NMR (400 MHz, DMSO-*d6*) $\delta$ 3.63 - 3.47 (m, 13H), 3.44 (t,*J* = 5.6 Hz, 2H), 3.41 - 3.36 (m, 2H),2.76 (t, *J* = 6.8 Hz, 2H), 2.64 (t, *J* = 5.6 Hz, 2H), 2.44 (t, *J* = 6.8 Hz, 2H).

**Step 2: UBI-1071d**

Compound **UBI-1071c** (0.4 g, 1.31 mmol) was dissolved in tetrahydrofuran (20 mL), added sodium bicarbonate (220.8 mg, 2.63 mmol) and $Boc_2O$ (430 mg, 1.97 mmol) and reacted at room temperature for 2 hours. The reaction was quenched with water (20 mL), extracted with ethyl acetate (20 mL*3). The organic phases were combined, dried over anhydrous sodium sulfate and concentrated to obtain crude product **UBI-1071d** (0.42 g, yield 79%) as yellow oil. LCMS $[M-100]^+$ = 305.1

**Step 3: UBI-1071e**

Compound **UBI-1071d** (0.4 g, 0.99 mmol), lithium hydroxide (47.8 mg, 1.98 mmol) were dissolved in tetrahydrofuran (20 mL) and water (10 mL). The system was reacted at 40°C for 5 hours. After complete hydrolysis, the reaction was added water (30 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1071e (V1084-010,** 320 mg, yield 82.9%) as colorless oil. $^1$H NMR (400 MHz, DMSO-d6) $\delta$ 12.15 (s, 1H), 3.63 - 3.58 (m, 2H), 3.58 - 3.49 (m, 8H), 3.46 (s, 2H),3.42 - 3.34 (m, 4H), 3.32 - 3.27 (m, 2H), 2.45 (t, *J* = 7.2 Hz, 2H), 1.39 (s, 9H). LCMS $[M-100]^+$ = 291.2

**Step 4: UBI-1071f**

Compound **UBI-1071e** (200 mg, 0.51 mmol), B(A1) (132.8 mg, 0.51 mmol), HATU (292.2 mg, 0.77 mmol), DIPEA (0.5 mL) were dissolved in DMF (5 mL) and reacted at room temperature overnight. After the completion of reaction, it was purified by reversed-phase column (MeOH/H2O = 5 % to 95 %, 45 minutes, collected at 70%). After purification, product **UBI-1071f** (150 mg, yield 46.7%) was obtained as yellow oil. $^1$H NMR (400 MHz, DMSO-*d6*) δ 11.02 (s, 1H), 9.83 (d,*J* = 14.0 Hz, 1H), 7.90 - 7.75 (m, 1H), 7.57- 7.37 (m, 2H), 5.21 - 5.10 (m, 1H), 4.47 - 4.26 (m, 2H), 3.55 - 3.47 (m, 16H), 3.31 - 3.26 (m, 2H), 3.00- 2.85 (m, 1H), 2.66 - 2.56 (m, 3H), 2.38 - 2.27 (m, 1H), 2.08 - 1.98 (m, 1H), 1.38 (s, 9H). LCMS [M-100] $^+$ = 532.2

**Step 5: UBI-1071**

Compound **UBI-1071e** (100 mg, 0.74 mmol) was dissolved in a mixed solvent of dichloromethane (10 mL) and methanol (1 mL), added Pd/C (20 mg), and reacted out at room temperature for 2 hours under hydrogen atmosphere. The reaction solution was filtered on Celite. The filtrate was concentrated to obtain product **UBI-1071f** (130 mg, yield 86.5%) as yellow oil. LCMS [M+H] $^+$ = 606.2.

**Synthesis method of Compound UBI-1072 ($NH_2$-16b-B(A1))**

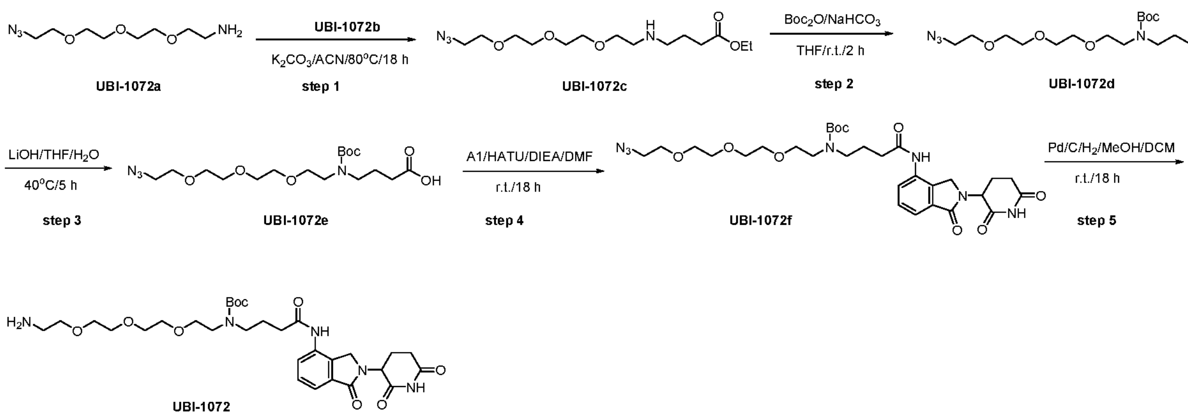


**Step 1: UBI-1072c**

Compound **UBI-1072a** (1.0 g, 4.58 mmol) was dissolved in acetonitrile (30 mL), added anhydrous potassium carbonate (0.95 g, 6.87 mmol) and **UBI-1160b** (0.89 g, 4.58 mmol), and then heated to 80 °C reacted for 18 hours. The reaction was cooled to room temperature and filtered. The filtrate was concentrated and subjected to silica gel column chromatography (petroleum ether/ethyl acetate = 50% to 100%, then dichloromethane /methanol = 0% to 10%) to obtain product **UBI-1072c** (530 mg, yield 34.8%) as yellow oil.
$^1$H NMR (400 MHz, chloroform-*d*) δ 5.83 (s, 1H), 4.14 (d, *J* = 7.1 Hz, 2H), 3.89 - 3.81 (m, 2H), 3.69 (d, *J* = 3.1 Hz, 10H), 3.43 (dd, *J* = 5.6, 4.4Hz, 2H), 3.16 - 3.09 (m, 2H), 3.03 (s, 2H), 2.47 (t, *J* = 7.0 Hz, 2H), 2.11 (s, 2H), 1.26 (t, *J* = 7.1 Hz, 3H).

**Step 2: UBI-1072d**

Compound **UBI-1072c** (0.5 g, 1.5 mmol) was dissolved in tetrahydrofuran (20 mL), added sodium bicarbonate (252.7 mg, 3.01 mmol) and $Boc_2O$ (492.4 mg, 2.26 mmol). The reaction was reacted at room temperature for 2 hours. The reaction was quenched with water (20 mL), extracted with ethyl acetate (20 mL*3). The organic phases were combined, dried over anhydrous sodium sulfate and concentrated to obtain crude product **UBI-1072d** (0.52 g, yield 80%) as yellow oil. LCMS [M-100] $^+$ =233.1

**Step 3: UBI-1072e**

Compound **UBI-1072d** (400 mg, 0.93 mmol), lithium hydroxide (44.3 mg, 1.85 mmol) were dissolved in tetrahydrofuran (20 mL) and water (10 mL). The system was reacted at 40°C for 5 hours. After complete hydrolysis, the

reaction was added water (15 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1072e** (300 mg, yield 80.2%) as colorless oil. $^1$H NMR (400 MHz, chloroform-*d*) δ 3.67 (d, *J* = 5.0 Hz, 8H), 3.64 - 3.60 (m, 4H), 3.40 (d, *J*= 5.2 Hz, 2H), 3.35 - 3.30 (m, 2H), 2.34 (t, *J*= 7.2 Hz, 2H),1.85 (q, *J* = 7.2 Hz, 2H), 1.45 (s, 11H). LCMS [M-100]$^+$ = 305.2

**Step 4: UBI-1072f**

Compound **UBI-1072e** (200 mg, 0.49 mmol), **B(A1)** (128.2 mg, 0.49 mmol), HATU (282 mg, 0.74 mmol), and DIPEA (0.5 mL) were dissolved in DMF (5 mL), reacted at room temperature overnight. After the completion of reaction, it was purified by reversed-phase column (MeOH/$H_2O$ = 5 % to 95 %, 45 minutes, collected at 70%). After purification, product **UBI-1072f** (150 mg, yield 47%) was obtained as yellow oil. LCMS [M-100]$^+$ = 546.1

**Step5: UBI-1072**

Compound **UBI-1072e** (100 mg, 0.15 mmol) was dissolved in a mixed solvent of dichloromethane (10 mL) and methanol (1 mL), added Pd/C (20 mg), and reacted at room temperature for 2 hours under hydrogen atmosphere. The reaction solution was filtered on Celite. The filtrate was concentrated to obtain product **UBI-1072** (80 mg, yield 55.6%) as yellow oil. LCMS [M+H] $^+$ = 620.2

**Synthesis method of Compound UBI-1102 ($NH_2$-17b-B(A1))**

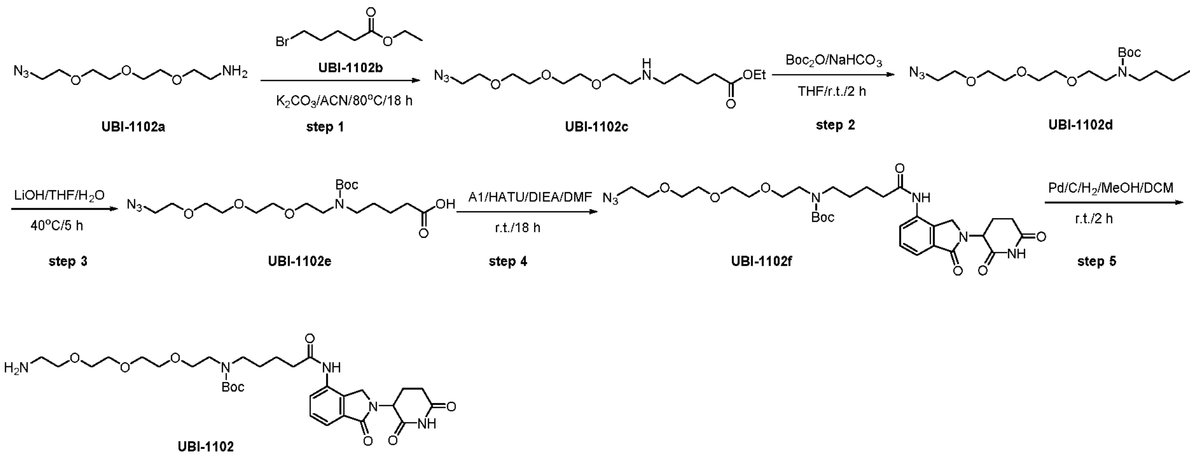


**Step 1: UBI-1102c**

Compound **UBI-1102a** (1.0 g, 4.58 mmol) was dissolved in acetonitrile (30 mL), added anhydrous potassium carbonate (950 mg, 6.87 mmol) and **UBI-1102b** (0.88 g, 1.92 mmol), and then heated to 80 °C reacted for 18 hours. The reaction was cooled to room temperature and filtered. The filtrate was concentrated and subjected to silica gel column chromatography (petroleum ether/ethyl acetate = 50% to 100%, then dichloromethane /methanol = 0% to 10%) to obtain product **UBI-1102c** (0.5 g, yield 25%) as yellow oil.
$^1$H NMR (400 MHz, chloroform-*d*) δ 4.12 (q, *J* = 7.1 Hz, 2H), 3.71 - 3.60 (m, 11H), 3.40 (t, *J* = 5.1 Hz, 2H), 2.86 - 2.77 (m, 2H), 2.70 - 2.62 (m, 2H), 2.32 (t, *J*= 7.4 Hz, 2H), 2.17 (s, 2H), 1.73 - 1.50 (m, 4H), 1.25 (t, *J* = 7.1 Hz, 3H).

**Step 2: UBI-1102d**

Compound **UBI-1102c** (0.5 g, 1.44 mmol) was dissolved in tetrahydrofuran (30 mL) and added sodium bicarbonate (121 mg, 2.16 mmol) and $Boc_2O$ (0.47 g, 2.17 mmol). The reaction was reacted at room temperature for 2 hours. The reaction was quenched with water (20 mL), extracted with ethyl acetate (20 mL*3). The organic phases were combined, dried over anhydrous sodium sulfate and concentrated to obtain crude product **UBI-1102d** (0.45 g, yield 60.7%) as yellow oil.

**Step 3: UBI-1102e**

Compound **UBI-1102d** (0.4 g, 0.9 mmol), lithium hydroxide (40 mg, 1.79 mmol) were dissolved in tetrahydrofuran (20 mL) and water (10 mL). The system was reacted at 40°C for 5 hours. After complete hydrolysis, the reaction was added water (15 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1102e** (300 mg, yield 83.6%) as colorless oil.
$^1$H NMR (400 MHz, chloroform-d) $\delta$ 10.30 (s, 1H), 3.71 - 3.55 (m, 12H), 3.38 (q,*J* = 11.2, 8.1 Hz, 4H), 3.26 (s, 2H), 2.41 - 2.32 (m, 2H), 1.65 - 1.56 (m, 4H),1.45 (d, *J*= 1.7 Hz, 9H).

**Step 4: UBI-1102f**

Compound **UBI-1102e** (300 mg, 0.72 mmol), **B(A1)** (150 mg, 0.57 mmol), HATU (408.9 mg, 1.08 mmol), and DIPEA (0.5 mL) were dissolved in DMF (5 mL), reacted at room temperature overnight. After the completion of reaction, it was purified by reversed-phase column (MeOH/$H_2O$ = 5 % to 95 %, 45 minutes, collected at 70%). After purification, product UBI-1102f (135 mg, yield 35.2%) was obtained as yellow oil. LCMS $[M-100]^+$ = 574

**Step 5: UBI-1102**

Dissolving in a mixed solvent of dichloromethane (30 mL) and methanol (3 mL), adding Pd/C (50 mg), and reacting at room temperature for 2 hours under hydrogen atmosphere. The reaction solution was filtered on Celite. The filtrate was concentrated to obtain product **UBI-1102** (130 mg, yield 89.8%) as yellow oil. LCMS $[M+H]^+$ = 648

**Synthesis method of Compound UBI-1154 ($NH_2$-5b-B(A1))**

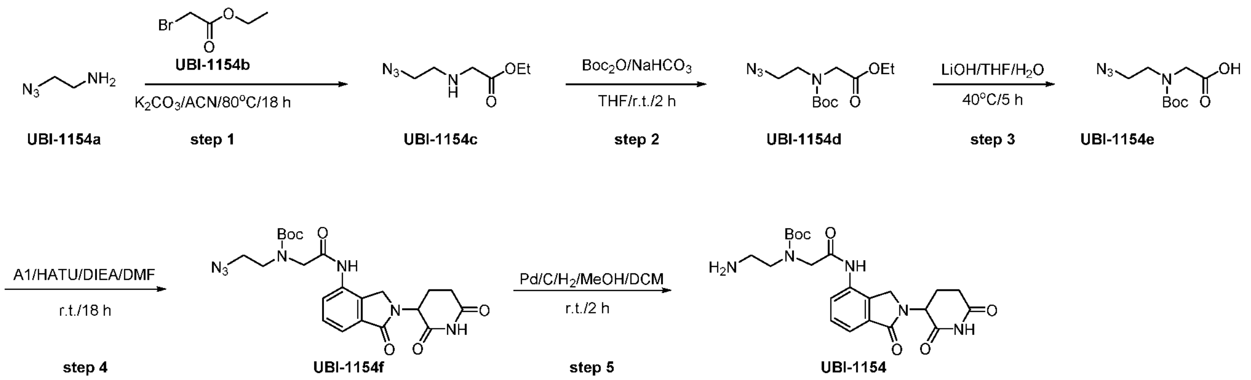


**Step 1: UBI-1154c**

Compound **UBI-1154a** (1.0 g, 11.63 mmol) was dissolved in acetonitrile (30 mL), added anhydrous potassium carbonate (2.41 g, 17.44 mmol) and **UBI-1154b** (1.93 g, 11.63 mmol), and then heated to 80 °C reacted for 18 hours. The reaction was cooled to room temperature and filtered. The filtrate was concentrated and subjected to silica gel column chromatography (petroleum ether/ethyl acetate = 50% to 100%, then dichloromethane /methanol = 0% to 10%) to obtain product **UBI-1154c** (500 mg, yield 25%) as yellow oil.

**Step 2: UBI-1154d**

Compound **UBI-1154c** (500 mg, 2.91 mmol) was dissolved in tetrahydrofuran (20 mL) and added sodium bicarbonate (731.8mg, 8.72 mmol) and $Boc_2O$ (1.27 g, 5.81 mmol). The reaction was reacted at room temperature for 2 hours. The reaction was quenched with water (20 mL), extracted with ethyl acetate (20 mL*3). The organic phases were combined, dried over anhydrous sodium sulfate and concentrated to obtain crude product **UBI-1154d** (0.48 g, yield 60.7%) as yellow oil. LCMS $[M-100]^+$ = 173.1

**Step 3: UBI-1154e**

Compound **UBI-1154d** (0.4 g, 1.47 mmol), lithium hydroxide (70 mg, 2.94 mmol) were dissolved in tetrahydrofuran (20 mL) and water (10 mL). The system was reacted at 40°C for 5 hours. After complete hydrolysis, the reaction was added water (30 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1154e** (230 mg, yield 83.6%) as colorless oil. $^1$H NMR (400 MHz, chloroform-*d*) $\delta$ 4.03 (s, 2H), 3.45 (d, *J*= 4.1 Hz, 4H), 1.44 (s, 9H).

**Step 4: UBI-1154f**

Compound **UBI-1154e** (200 mg, 0.82 mmol), **B(A1)** (212.3 mg, 0.82 mmol), HATU (467 mg, 0.13 mmol), and DIPEA (0.5 mL) were dissolved in DMF (5 mL), reacted at room temperature overnight. After the completion of reaction, it was purified by reversed-phase column (MeOH/$H_2O$ = 5 % to 95 %, 45 minutes, collected at 70%). After purification, product **UBI-1154f** (140 mg, yield 35.2%) was obtained as yellow oil. LCMS [M+H] $^+$ = 486.1

**Step 5: UBI-1154**

Compound **UBI-1154e** (100 mg, 0.21 mmol) was dissolved in a mixed solvent of dichloromethane (10 mL) and methanol (1 mL), added Pd/C (20 mg), and reacted at room temperature for 2 hours under hydrogen atmosphere. The reaction solution was filtered on Celite. The filtrate was concentrated to obtain crude product **UBI-1154f** (85 mg, yield 89.8%) as yellow oil. LCMS [M+H] $^+$ = 460.2

**Synthesis method of Compound UBI-1155 ($NH_2$-6b-B(A1))**

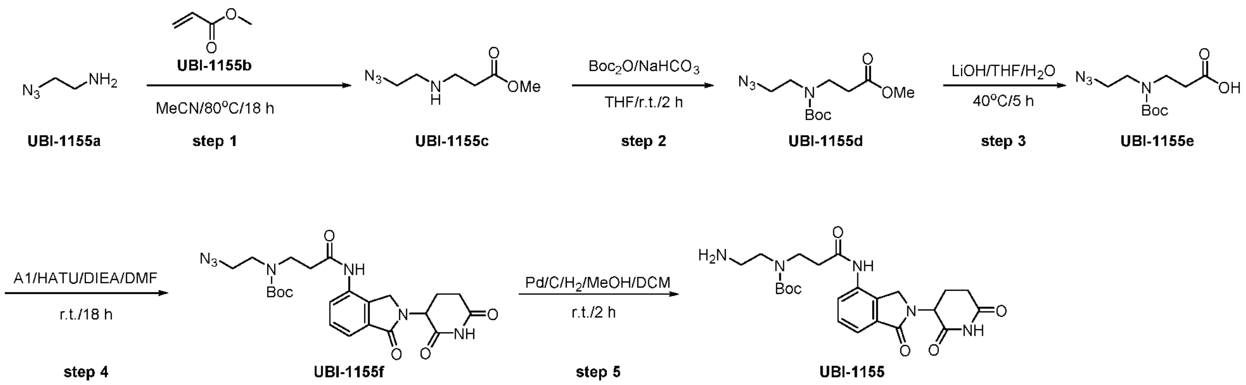


**Step 1: UBI-1155c**

Compound **UBI-1155a** (1.0 g, 11.63 mmol) and **UBI-1155b** (1.0 g, 11.63 mmol) were dissolved in acetonitrile (30 mL), and then heated to 80 °C reacted for 18 hours. The reaction solution was concentrated and subjected to silica gel column chromatography (petroleum ether/ethyl acetate = 50% to 100%, then dichloromethane/methanol = 0% to 10%) to obtain product **UBI-1071c** (0.4 g, yield 30%) as yellow oil.

**Step 2: UBI-1155d**

Compound **UBI-1155c** (500 mg, 2.90 mmol) was dissolved in tetrahydrofuran (20 mL), added sodium bicarbonate (732 mg, 8.71 mmol) and $Boc_2O$ (1.27 g, 5.81 mmol), and reacted at room temperature for 2 hours. The reaction was quenched with water (20 mL), extracted with ethyl acetate (20 mL*3). The organic phases were combined, dried over anhydrous sodium sulfate and concentrated to obtain crude product **UBI-1155d** (500 mg, yield 44.3%) as yellow oil. $^1$H NMR (400 MHz, chloroform-*d*) $\delta$ 3.69 (s, 3H), 3.54 (t, *J*= 6.8 Hz, 2H),3.48 - 3.35 (m, 4H), 2.68 - 2.53 (m, 2H), 1.47 (s, 9H). LCMS [M-100]$^+$ = 173.1

**Step 3: UBI-1155e**

Compound **UBI-1155d** (500 mg, 1.84 mmol), lithium hydroxide (88.0 mg, 3.67 mmol) were dissolved in tetrahydrofuran (20 mL) and water (10 mL). The system was reacted at 40°C for 5 hours. After complete hydrolysis, the reaction was added water (30 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1155e** (350 mg, yield 73.8%) as colorless oil. $^1$H NMR (400 MHz, chloroform-*d*) $\delta$ 7.68 (s, 1H), 3.54 (t, *J* = 6.9 Hz, 2H), 3.49 - 3.30 (m, 4H),2.65 (s, 2H), 1.47 (s, 9H). LCMS [M-100] $^+$ = 159.1

**Step 4: UBI-1155f**

Compound **UBI-1155e** (200 mg, 0.74 mmol), **B(A1)** (183.9 mg, 0.74 mmol), HATU (419 mg, 1.10 mmol), and DIPEA (0.5 mL) were dissolved in DMF (5 mL), reacted at room temperature overnight. After the completion of reaction, it was purified by reversed-phase column (MeOH/$H_2O$ = 5 % to 95 %, 45 minutes, collected at 70%). After purification, product **UBI-1155f (V1084-066,** 200 mg, yield 51.7%) was obtained as yellow oil. $^1$H NMR (400 MHz, DMSO-*d6*) $\delta$ 11.03 (s, 1H), 9.88 (s, 1H), 7.83 (s, 1H), 7.56 - 7.43 (m, 2H), 5.15 (dd, *J*= 13.3, 5.1 Hz, 1H), 4.45 - 4.28 (m, 2H), 3.50(t, *J*= 7.0 Hz, 2H), 3.46 - 3.37 (m, 4H), 3.27 - 2.87 (m, 2H), 2.64 (t, *J*= 6.9 Hz, 2H), 2.39 - 2.00 (m, 2H), 1.40 (s, 9H). LCMS [M+H] $^+$ = 400.2

**Step 5: UBI-1155**

Compound **UBI-1155e** (200 mg, 0.74 mmol) was dissolved in a mixed solvent of dichloromethane (30 mL) and methanol (3 mL), added Pd/C (40 mg), and reacted at room temperature for 2 hours under hydrogen atmosphere. The reaction solution was filtered on Celite. The filtrate was concentrated to obtain crude product **UBI-1155f** (130 mg, yield 68.6%) as yellow oil. LCMS [M+H] $^+$ = 474.2

**Synthesis Method of Compound: UBI-1156 ($NH_2$-7b-B(A1))**

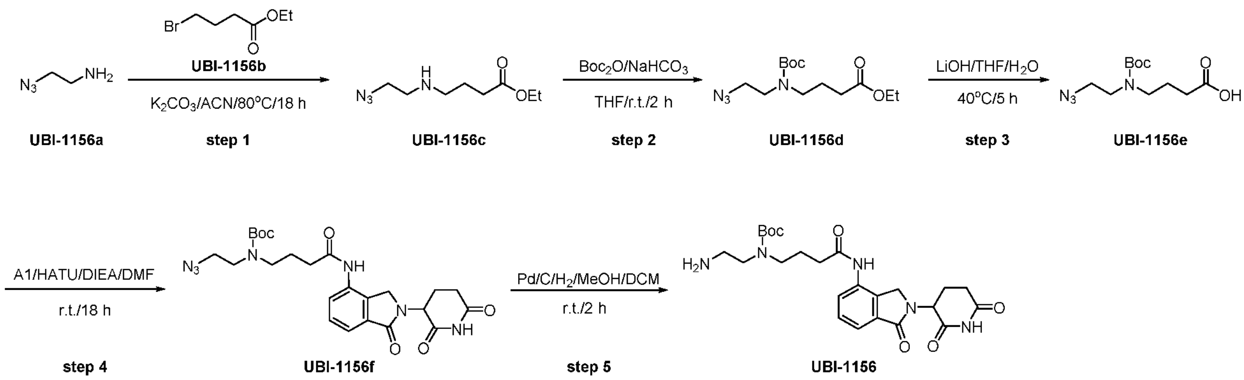


**Step 1: UBI-1156c**

Compound **UBI-1156a** (1.0 g, 11.63 mmol) was dissolved in acetonitrile (30 mL), added anhydrous potassium carbonate (2.41g, 17.44mmol) and **UBI-1156b** (2.26 g, 11.63 mmol), and then heated to 80 °C reacted for 18 hours. The reaction was cooled to room temperature and filtered. The filtrate was concentrated and subjected to silica gel column chromatography (petroleum ether/ethyl acetate = 50% to 100%, then dichloromethane /methanol = 0% to 10%) to obtain product **UBI-1156c** (650 mg, yield 27%) as yellow oil. LCMS [M+H]$^+$ = 201.1

**Step 2: UBI-1156d**

Compound **UBI-1156c** (500 mg, 2.5 mmol) was dissolved in tetrahydrofuran (10 mL) and added sodium bicarbonate (731.8 mg, 7.50 mmol) and $Boc_2O$ (0.47 g, 5.0 mmol). The reaction was reacted at room temperature for 2 hours. The reaction was quenched with water (40 mL), extracted with ethyl acetate (20 mL*3). The organic phases were combined, dried over anhydrous sodium sulfate and concentrated to obtain crude product **UBI-1156d** (500 mg, yield

66.7%) as yellow oil.
$^1$H NMR (400 MHz, chloroform-*d*) δ 4.14 (q, *J* = 7.1 Hz, 2H), 3.48 - 3.32 (m, 4H), 3.29 (t, *J*= 7.3 Hz, 2H), 2.31 (t, *J* = 7.3 Hz, 2H), 1.86 (p, *J* = 7.3 Hz, 2H), 1.47 (s, 9H), 1.26 (t, *J* = 7.1 Hz, 3H).

**Step 3: UBI-1156e**

Compound **UBI-1156d** (500 mg, 1.66 mmol), lithium hydroxide (79.73 mg, 3.33 mmol) were dissolved in tetrahydrofuran (20 mL) and water (10 mL). The system was reacted at 40°C for 5 hours. After complete hydrolysis, the reaction was added water (30 mL), extracted impurities using ethyl acetate (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain product **UBI-1156e** (400 mg, yield 88.2%) as colorless oil. LCMS $[M+H]^+$ = 173.2
$^1$H NMR (400 MHz, DMSO-$d_6$) δ 3.40 (dd, *J* = 6.3, 4.7 Hz, 2H), 3.33 (dd, *J* = 7.1, 5.5 Hz, 2H), 3.18 (t, *J* = 7.3 Hz, 2H), 2.16 (t, *J* = 7.3 Hz, 2H), 1.68 (p, *J* = 7.3 Hz, 2H), 1.40 (s, 9H).

**Step 4: UBI-1156f**

Compound **UBI-1156e** (200 mg, 0.735 mmol), **B(A1)** (191.2mg, 0.735 mmol), HATU (419.12 mg, 1.1 mmol), and DIPEA (0.5 mL) were dissolved in DMF (5 mL), reacted at room temperature overnight. After the completion of reaction, it was purified by reversed-phase column (MeOH/H2O = 5 % to 95 %, 45 minutes, collected at 70%). After purification, product **UBI-1156f** (100 mg, yield 26.5%) was obtained as yellow oil. LCMS $[M-100]^+$ = 414.1

**Step 5: UBI-1156**

Compound **UBI-1156e** (100 mg, 0.20 mmol) was dissolved in a mixed solvent of dichloromethane (20 mL) and methanol (2 mL), added **Pd/C** (20 mg), and reacted at room temperature for 2 hours under hydrogen atmosphere. The reaction solution was filtered on Celite. The filtrate was concentrated to obtain product **UBI-1156f** (100 mg, yield 80.3%) as yellow oil. LCMS $[M+H]^+$ = 488.1

**Synthesis method of Compound UBI-1160 ($NH_2$-18b-B(A1))**

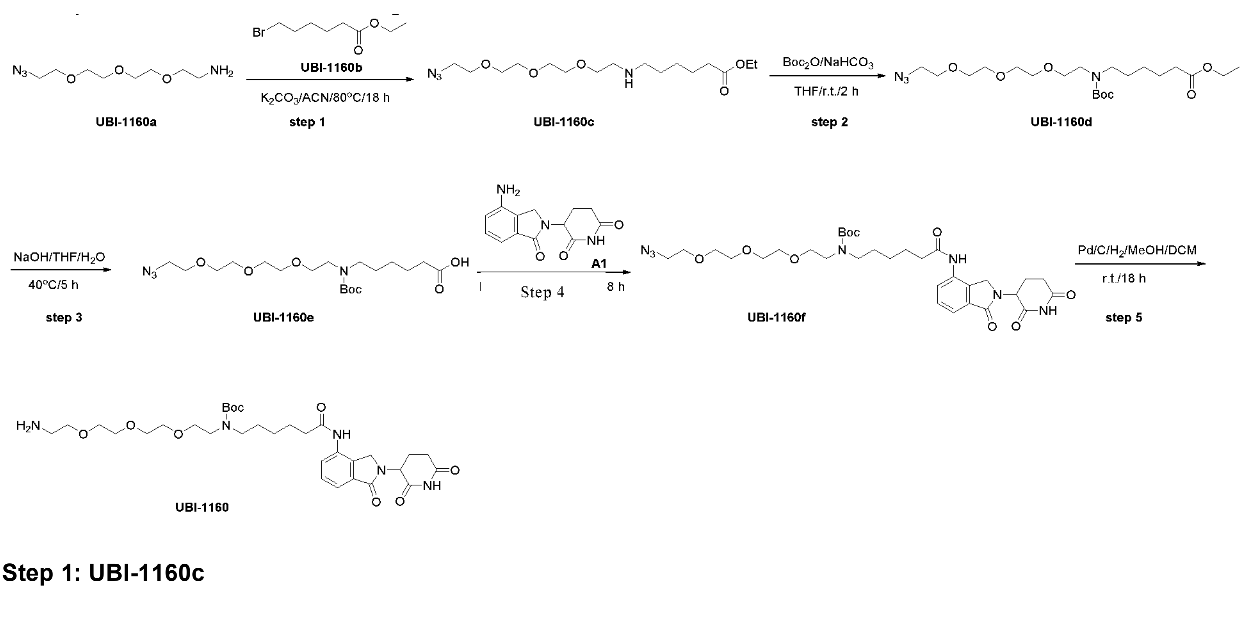


**Step 1: UBI-1160c**

Compound **UBI-1160a** (1.5 g, 6.87 mmol) was dissolved in acetonitrile (30 mL), added anhydrous potassium carbonate (1.9 g, 6.87 mmol) and **UBI-1160b** (1.53 g, 6.87 mmol), reacted at 80 °C overnight. The reaction was cooled to room temperature and filtered. The filtrate was concentrated and isolated by silica gel column chromatography (dichloromethane /methanol = 95/5) to obtain product **UBI-1160c** (1 g, yield 40%) as white solid.

**Step 2: UBI-1160d**

Compound **UBI-1160c** (1 g, 2.77 mmol) was dissolved in tetrahydrofuran (30 mL), added $NaHCO_3$ (700 mg, 8.31 mmol) and $Boc_2O$ (0.72 g, 3.33 mmol), and reacted at room temperature for 2 hours. The reaction was quenched with water (20 mL), extracted with ethyl acetate (20 mL*3). The organic layers were combined, dried over anhydrous sodium sulfate and concentrated. The crude product was isolated by silica gel column chromatography (dichloromethane/methanol = 90/10) to obtain product **UBI-1160d** (2 g, yield 100%) as yellow oil.

**Step 3: UBI-1160e**

Compound **UBI-1160d** (1.27 g, 2.77 mmol) was dissolved in tetrahydrofuran (20 mL) and water (10 mL), added NaOH (332 mg, 8.31 mmol), and reacted at 40°C for 5 hours. The reaction solution was concentrated, and added saturated brine (15 mL) and extracted with ether (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain crude product **UBI-1160e** (500 mg, yield 42%) as colorless oil. $^1$H NMR (400 MHz, DMSO-$d_6$) δ 11.97 (s, 1H), 3.60 (m, 2H), 3.51-3.49 (m, 8H), 3.46 (m, 2H), 3.40 (m, 2H), 3.25 (m, 2H), 3.16-3.12 (m, 2H), 2.17-2.19 (m, 2H), 1.54-1.35 (m, 13H), 1.16-1.21 (m, 2H).

**Step 4: UBI-1160f**

Compound **UBI-1160e** (70 mg, 0.22 mmol), **B(A1)** (50 mg, 0.11 mmol), EDCI (32 mg, 0.165 mmol), HOBT (18 mg, 0.13 mmol), DIEA (0.5 mL) were dissolved in DMF (5 mL) and reacted at room temperature overnight. Further adding HATU (870 mg, 2.3 mmol) and reacting at room temperature overnight. The reaction was quenched with water (50 mL), extracted with ethyl acetate (50 mL*3). The organic layers were combined, dried over anhydrous sodium sulfate and concentrated. The crude product was isolated by silica gel column chromatography (dichloromethane/methanol = 97/3) to obtain crude product **UBI-1160f** (500 mg, yield 76%) as yellow oil. $^1$H NMR (400 MHz, DMSO-$d_6$) δ 11.02 (s, 1H), 9.75 (s, 1H), 7.82 (m, 1H), 7.55 - 7.43 (m, 2H), 5.15 (m, 1H), 4.43 - 4.28 (m, 2H), 3.66 - 3.57 (m, 8H), 3.55 - 3.50 (m, 8H), 3.15 (m, 8H), 2.06 - 1.95 (m, 2H), 1.62 (m, 2H), 1.49 (m, 4H), 1.38 (d, 9H). LCMS: $[M-100]^+ = 574$

**Step 5: UBI-1160**

Compound **UBI-1160e** (500 mg, 0.74 mmol) was dissolved in a mixed solvent of dichloromethane (30 mL) and methanol (3 mL), added Pd/C (500 mg), and reacted for 2 hours under hydrogen atmosphere. It was filtered on Celite. The filtrate was concentrated to obtain product **UBI-1160f** (130 mg, yield 27%) as yellow oil. $^1$H NMR (400 MHz, DMSO-$d_6$) δ 11.02 (s, 1H), 9.76 (s, 1H), 7.54 - 7.46 (m, 1H), 7.25 (t, 1H), 7.20 - 7.11 (m, 1H), 5.15 (dd, 1H), 4.45 - 4.24 (m, 2H), 3.68 - 3.38 (m, 15H), 3.27 (m, 2H), 3.14 (m, 5H), 3.01 - 2.93 (m, 3H), 2.68 - 2.59 (m, 1H), 2.39 - 2.31 (m, 2H), 2.08 - 1.99 (m, 1H), 1.61 (m, 2H), 1.48 (m, 2H), 1.38 (d, 9H). LCMS: $[M+H]^+ = 648$

**Synthesis method of Compound UBI-1069 ($NH_2$-12b-B(A1))**

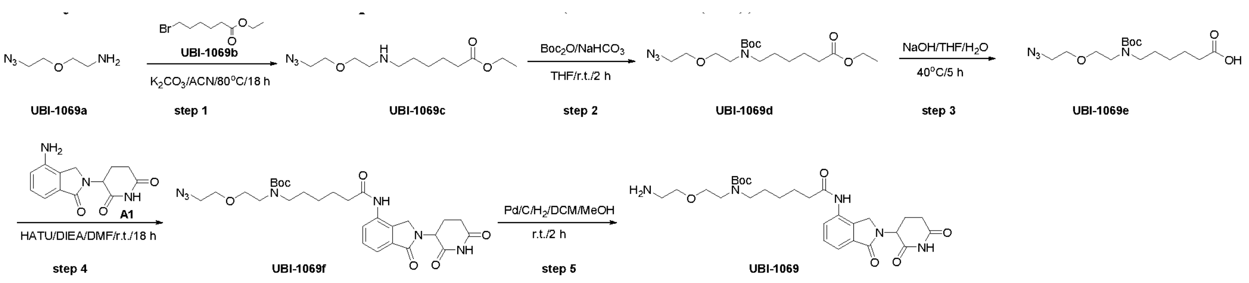


**Step 1: UBI-1169c**

Compound **UBI-1169a** (1.5 g, 11.5 mmol) was dissolved in acetonitrile (30 mL), added anhydrous potassium carbonate (3.18 g, 23 mmol) and **UBI-1169b** (2.56 g, 11.5 mmol), reacted at 80 °C overnight. The reaction was cooled to room temperature and filtered. The filtrate was concentrated and isolated by silica gel column chromatography (dichloromethane /methanol = 95/5) to obtain product **UBI-1169c** (1.3 g, yield 41%) as white solid. $^1$H NMR (400 MHz, chloroform-

d) δ 4.12 (q, *J* = 7.1 Hz, 2H), 3.70 - 3.59 (m, 4H), 3.38 (t, *J* = 5.0 Hz, 2H), 2.86 - 2.79 (m, 2H), 2.70 - 2.60 (m, 2H), 2.30 (t, *J* = 7.5 Hz, 2H), 1.69 - 1.60 (m, 2H), 1.53 (p, *J* = 7.4 Hz, 2H), 1.42 - 1.34 (m, 2H), 1.25 (t, *J* = 7.1 Hz, 3H).

**Step 2: UBI-1169d**

Compound **UBI-1169c** (1.3 g, 4.77 mmol) was dissolved in tetrahydrofuran (30 mL), added $NaHCO_3$ (1.2 g, 14.33 mmol) and $Boc_2O$ (1.24 g, 5.72 mmol), and reacted at room temperature for 2 hours. The reaction was quenched by adding water (20 mL) and extracted with ethyl acetate (20 mL*3). The organic layers were combined, dried over anhydrous sodium sulfate and concentrated to obtain crude product **UBI-1169d** (2.5 g, yield 100%) as yellow oil.

**Step 3: UBI-1169e**

Compound **UBI-1169d** (1.7 g, 4.77 mmol) was dissolved in tetrahydrofuran (20 mL) and water (10 mL), added sodium hydroxide (572 mg, 14.31 mmol), and reacted at 40 ° C for 5 hours under strring. The reaction solution was concentrated, and added saturated brine (15 mL) and extracted with ether (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain crude product **UBI-1169e** (300 mg, yield 18%) as colorless oil.

$^1$H NMR (400 MHz, DMSO-$d_6$) δ 11.96 (s, 1H), 3.62 - 3.56 (m, 2H), 3.51 (t, *J* = 5.9 Hz, 2H), 3.38 (d, *J* = 9.5 Hz, 2H), 3.28 (t, *J* = 5.9 Hz, 2H), 3.16 (q, *J* = 7.4, 6.3 Hz, 2H), 2.19 (t, *J* = 7.3 Hz, 2H), 1.50 (dt, *J* = 15.0, 7.5 Hz, 4H), 1.39 (s, 9H), 1.25 - 1.17 (m, 2H).

**Step 4: UBI-1069f**

Compound **UBI-1169e** (300 mg, 0.87 mmol), **B(A1)** (226 mg, 0.87 mmol), HATU (660 mg, 1.7 mmol), and DIPEA (561 mg, 4.35 mmol) were dissolved in DMF (5 mL), reacted at room temperature overnight. The reaction was quenched with water (20 mL), extracted with ethyl acetate (50 mL*3). The organic layers were combined, dried over anhydrous sodium sulfate and concentrated. The crude product was isolated by silica gel column chromatography (dichloromethane/methanol = 97/3) to obtain crude product **UBI-1069f** (300 mg, yield 71%) as yellow oil. $^1$H NMR (400 MHz, DMSO-$d_6$) δ 11.02 (s, 1H), 9.76 (s, 1H), 7.81 (m, 1H), 7.53 - 7.44 (m, 2H), 5.15 (m, 1H), 4.35 (m, 2H), 3.65 - 3.57 (m, 6H), 3.38 (d, J = 4.8 Hz, 2H), 3.16 (m, 6H), 2.02 (m, 2H), 1.62 (m, 2H), 1.49 (m, 4H), 1.38 (d, 9H). LCMS: $[M-100]^+ = 486$

**Step 5: UBI-1069**

Compound **UBI-1069f** (300 mg, 0.51 mmol) was dissolved in a mixed solvent of dichloromethane (30 mL) and methanol (3 mL), added Pd/C (300 mg), and reacted for 2 hours under hydrogen atmosphere. It was filtered on Celite. The filtrate was concentrated to obtain product **UBI-1069** (200 mg, yield 70%) as yellow oil. LCMS: $[M+H]^+ = 560$

**Synthesis method of Compound UBI-1080 ($NH_2$-13b-B(A1))**

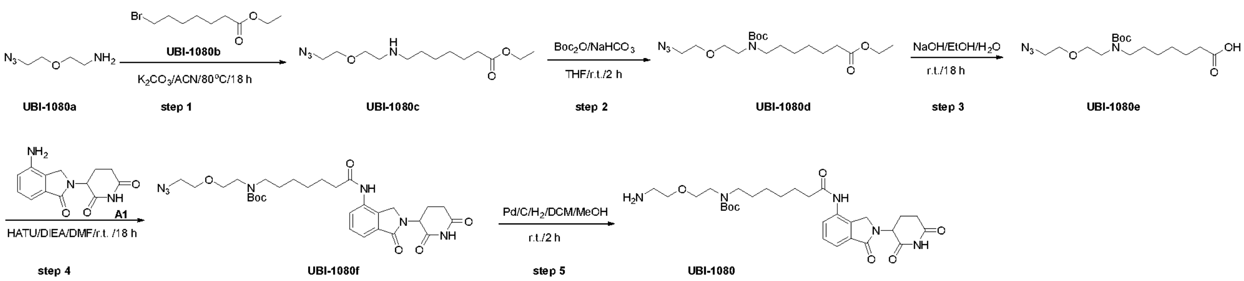


**Step 1: UBI-1180c**

Compound **UBI-1180a** (1.5 g, 11.5 mmol) was dissolved in acetonitrile (30 mL), added anhydrous potassium carbonate (3.17 g, 23 mmol) and **UBI-1180b** (2.73 g, 11.5 mmol), reacted at 80 °C overnight. The reaction was cooled to room temperature and filtered. The filtrate was concentrated and isolated by silica gel column chromatography (dichloromethane /methanol = 95/5) to obtain crude product **UBI-1180c** (1.4 g, yield 42%) as a yellow oil. $^1$H NMR (400 MHz,

chloroform-*d*) δ 4.12 (q, 2H), 3.72 - 3.60 (m, 4H), 3.47 (s, 1H), 3.38 (t, 2H), 2.86 - 2.76 (m, 2H), 2.65 - 2.59 (m, 2H), 2.29 (t, 2H), 1.69 - 1.58 (m, 2H), 1.56 - 1.47 (m, 2H), 1.34 (m, 4H), 1.25 (t, 3H).

**Step 2: UBI-1180d**

Compound **UBI-1180c** (1.4 g, 4.8 mmol) was dissolved in tetrahydrofuran (30 mL), added $NaHCO_3$ (1.2 g, 14.4 mmol) and $Boc_2O$ (1.15 g, 5.28 mmol), and reacted at room temperature for 2 hours. The reaction was quenched with water (20 mL), extracted with ethyl acetate (20 mL*3). The organic layers were combined, dried over anhydrous sodium sulfate and concentrated to obtain crude product **UBI-1180d** (2 g, yield 100%) as yellow oil.

**Step 3: UBI-1180**

Compound **UBI-1180d** (1.85 g, 4.8 mmol) was dissolved in EtOH (20 mL) and water (10 mL), added sodium hydroxide (1.15 g, 28.8 mmol), and reacted at room temperature overnight. The reaction solution was concentrated, and added saturated brine (15 mL) and extracted with ether (20 mL*2). The aqueous phase was acidified to pH=6 using 1M HCl, then extracted with ethyl acetate (40 mL*3). The organic layers were combined, dried over anhydrous sodium sulfate and concentrated. The crude product was isolated by silica gel column chromatography (dichloromethane/methanol = 94/6) to obtain product **UBI-1180** (1.4 g, yield 82%) as colorless oil. $^1$H NMR (400 MHz, DMSO-$d_6$) δ 11.96 (s, 1H), 3.58 (t, *J*= 4.8 Hz, 2H), 3.51 (t, *J* = 5.9 Hz, 2H), 3.38 (t, *J* = 4.9 Hz, 2H), 3.28 (t, *J* = 5.9 Hz, 2H), 3.15 (t, *J* = 7.4 Hz, 2H), 2.18 (t, *J* = 7.4 Hz, 2H), 1.53 - 1.41 (m, 4H), 1.39 (s, 9H), 1.24 (m, 4H).

**Step 4: UBI-1180f**

Compound **UBI-1180e** (500 mg, 1.39 mmol), **B(A1)** (362 mg, 1.39 mmol), HATU (633 mg, 1.67 mmol), and DIPEA (3 mL) were dissolved in DMF (8 mL), reacted at room temperature overnight. The reaction was quenched with water (20 mL), extracted with ethyl acetate (50 mL*3). The organic layers were combined, dried over anhydrous sodium sulfate and concentrated. The crude product was isolated by silica gel column chromatography (dichloromethane /methanol = 93/7) to obtain product **UBI-1180f** (500 mg, yield 60%) as yellow oil. LCMS: $[M+H]^+$ = 600

**Step 5: UBI-1180**

Compound **UBI-1180f** (500 mg, 0.83 mmol) was dissolved in a mixed solvent of dichloromethane (30 mL) and methanol (3 mL), added Pd/C (300 mg), and reacted for 2 hours under hydrogen atmosphere. It was filtered on Celite. The filtrate was concentrated to obtain product as yellow oil. The product was purified by a reversed-phase column (water: methanol = 97:3) to obtain product **UBI-1180** (270 mg, yield 57%) as yellow solid. $^1$H NMR (400 MHz, DMSO-$d_6$) δ 9.77 (s, 1H), 7.82 (m, 1H), 7.53 - 7.46 (m, 2H), 5.15 (m, 1H), 4.42 - 4.27 (m, 2H), 3.56 (m, 3H), 3.50 (m, 3H), 3.19 - 3.11 (m, 4H), 2.95 (m, 4H), 2.37 (m, 2H), 1.61 (m, 2H), 1.46 (m, 4H), 1.38 (m, 13H), 1.26 (m, 5H), 1.14 - 1.09 (m, 3H), 1.07 - 0.98 (m, 2H). LCMS: $[M+H]^+$ = 574

**Synthesis method of Compound UBI-1204 ($N_3$-14b-B(A1))**

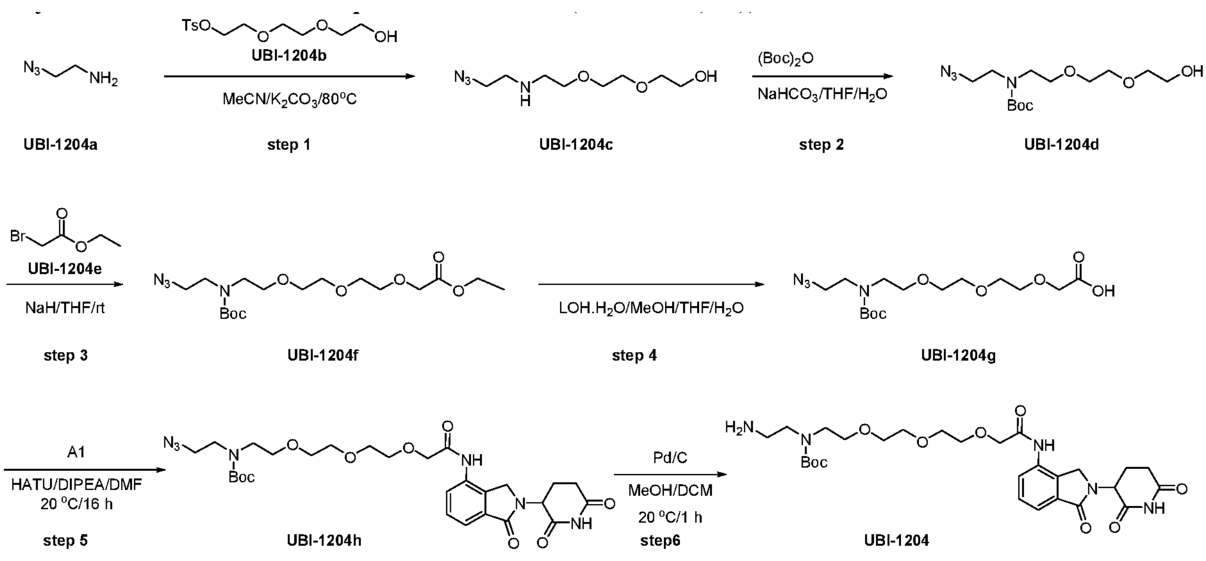


**Step 1: UBI-1204c**

Compound **UBI-1204a** (5.986g, 69.52mmol), **BUI-1204b** (19.05g, 66.05mmol) and $K_2CO_3$ (11.53 g, 83.43 mmol) were dissolved in acetonirile (200 mL), then the reaction was reacted at room temperature for 3 hours. The reaction solution was filtered. The filtrate was concentrated and isolated by silica gel column chromatography (methanol/dichloromethane = 0% to 10%) to obtain product **UBI-1204c** (4.10g, yield 26%) as colorless transparent oil. $^1$H NMR (400 MHz, chloroform-d) δ 3.76 - 3.71 (m, 2H), 3.70 - 3.59 (m, 8H), 3.49 - 3.41 (m, 2H), 2.86 - 2.79 (m, 4H), 2.14 - 1.97 (m, 2H).

**Step 2: UBI-1204d**

Compound **UBI-1204c** (4.100g, 18.76mmol), $(Boc)_2O$ (8.200g, 37.57mmol) and $NaHCO_3$ (3.156g, 37.57mmol) were dissolved in tetrahydrofuran (50 mL) and reacted at room temperature for 16 hours. The reaction was filtered. The filtrate was concentrated and isolated by column chromatography (ethyl acetate/petroleum ether = 0% to 95%) to obtain product **UBI-1204d** (4.85g, yield 71%) as colorless transparent oil. $^1$H NMR (400 MHz, chloroform-d) δ 3.76 - 3.72 (m, 2H), 3.69 - 3.64 (m, 2H), 3.64 - 3.56 (m, 6H), 3.49 - 3.39 (m, 6H), 1.93 (s, 1H), 1.47 (s, 9H).

**Step 3: UBI-1204e**

Compound **UBI-1097d** (1.00g, 3.14mmol) was dissolved in tetrahydrofuran (10 mL) and cooled to 0°C, added NaH (0.25 1g, 6.28mmol), and reacted for ten minutes. Then **UBI-1204e** (0.787g, 4.71mmol) was added, and the reaction was carried out at room temperature for 16 hours. The reaction was filtered. The filtrate was concentrated, added water (50 mL), and extracted with ethyl acetate (50 mL*3). The organic phases were combined, washed with water, saturated brine, dried over anhydrous sodium sulfate, and concentrated under reduced pressure, then isolated by silica gel column chromatography (methanol/ dichloromethane = 0% to 3%)) to obtain product **UBI-1204f** (705mg, 53%) as colorless transparent oil. $^1$H NMR (400 MHz, chloroform-*d*) δ 4.22 (q, *J* = 7.2 Hz, 2H), 4.15 (s, 2H), 3.77 - 3.72 (m, 2H), 3.72 - 3.67 (m, 2H), 3.67 - 3.63 (m, 2H), 3.63 - 3.55 (m, 4H), 3.48 - 3.39 (m, 6H), 1.47 (s, 9H), 1.29 (t, *J*= 7.2 Hz, 3H).

**Step 4: UBI-1204g**

Compound UBI-1204f (698mg, 1.21mmol), LiOH.H2O (253mg, 6.04mmol) were dissolved in a mixed solvent consisting of methanol (3 mL), tetrahydrofuran (9 mL) and water (3 mL), stirred at room temperature overnight. The reaction solution was added water, concentrated and extracted with ethyl acetate. The aqueous phase was acidified to pH=5 using hydrochloric acid (1M), and then lyophilized. The solid was added to a mixed solvent consisting of methanol (2 mL) and dichloromethane (20 mL), filtered after stirring for 10 minutes. The filtrate was concentrated to obtain product UBI-1204g (650 mg, yield 90%) as colorless oil. $^1$H NMR (400 MHz, DMSO-$d_6$) δ 3.86 (s, 2H), 3.58 - 3.55 (m, 2H), 3.54 - 3.46 (m, 8H), 3.43 - 3.38 (m, 4H), 3.36 - 3.32 (m, 2H), 1.40 (s, 9H). LCMS: $[M+Na]^+$ = 399.3.

**Step 5: UBI-1204h**

Compound **UBI-1204g** (120mg, 0.32mmol), **B(A1)** (82.6mg, 0.32mmol), and HATU (181.8 mg, 0.478 mmol) were dissolved in DMF (3 mL) and DIPEA (0.3 mL), and reacted at room temperature for 16 hours. The reaction was quenched with water (10 mL), then extracted with ethyl acetate (8 mL*3). The organic phases were combined, then dried over anhydrous sodium sulfate and concentrated to obtain crude product. The crude product was purified by reversed-phase column (methanol/water = 5% to 95%, collected at 70%) to obtain product **UBI-1204h** (90mg, yield 45.7%) as colorless oil. LCMS: $[M+H-100]^+$ = 518.3.

**Step 6: UBI-1204**

Under hydrogen atmosphere, compound **UBI-1204h** (90mg, 0.15mmol), Pd/C (30mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water = 5% to 95%, collected at 15%) to obtain the product **UBI-1204** (80mg, yield 93%) as yellow oil. LCMS: $[M+H]^+$ = 592.7.

**Synthesis method of Compound UBI-1205 ($N_3$-15b-B(A1))**

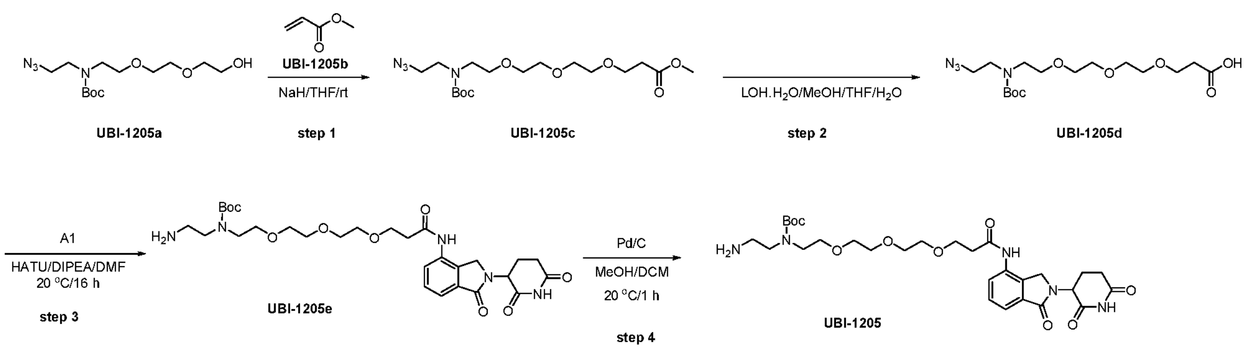


**Step 1: UBI-1205c**

Compound **UBI-1205a** (1.050g, 3.298mmol) was dissolved in tetrahydrofuran (10 mL), added NaH (26.4mg, 0.660mmol) and reacted at room temperature for 10 minutes. Then **UBI-1205b** (0.426 g, 4.95 mmol) was added, and the reaction was carried out at room temperature overnight. The reaction solution was added methanol, then concentrated, and isolated by silica gel column chromatography (ethyl acetate/petroleum ether = 0% to 95%) to obtain product **UBI-1205c** (710mg, yield 51%) as colorless and transparent oil. $^1$H NMR (400 MHz, chloroform-d) δ 3.76 (t, *J*= 6.4 Hz, 2H), 3.69 (s, 3H), 3.66 - 3.53 (m, 10H), 3.48 - 3.37 (m, 6H), 2.61 (t, *J* = 6.4 Hz, 2H), 1.47 (s, 9H).

**Step 2: UBI-1205d**

Compound **UBI-1205c** (700mg, 1.21mmol) and LiOH.H2O (254mg, 6.06mmol) were dissolved in a mixed solvent consisting of methanol (3 mL), tetrahydrofuran (9 mL) and water (3 mL). The reaction was stirred at room temperature overnight. The reaction solution was added water, concentrated and then extracted with ethyl acetate. The aqueous phase was acidified to pH=5 using hydrochloric acid (1M), and then lyophilized. The solid was poured into a mixed solvent of methanol (2 mL) and dichloromethane (20 mL), stirred for 10 minutes, filtered. The filtrate was concentrated to obtain product **UBI-1205d** (650mg, yield 87%) as colorless transparent oil.
$^1$H NMR (400 MHz, DMSO-$d_6$) δ 11.98 (s, 1H), 3.59 (t, *J* = 6.4 Hz, 2H), 3.55 - 3.44 (m, 10H), 3.43 - 3.38 (m, 4H), 3.35 - 3.32 (m, 2H), 2.43 (t, *J* = 6.4 Hz, 2H), 1.40 (s, 9H). LCMS: $[M+Na]^+$ = 413.3.

**Step 3: UBI-1205e**

Compound **UBI-1205d** (150mg, 0.38mmol), **B(A1)** (100mg, 0.38mmol), and HATU (0.22g, 0.58 mmol) were dissolved in DMF (3 mL) and DIPEA (0.3 mL), and reacted at room temperature for 16 hours. The reaction was quenched with water (10 mL), then extracted with ethyl acetate (8 mL*3). The organic phases were combined, then dried over

anhydrous sodium sulfate and concentrated to obtain crude product. The crude product was purified by reversed-phase column (methanol/water = 5% to 95%, collected at 70%) to obtain product **UBI-1205e** (95mg, yield 40%) as colorless oil. LCMS: $[M+H-100]^+$ = 532.3.

**Step 4: UBI-1205**

Under hydrogen atmosphere, compound **UBI-1205e** (95mg, 0.15mmol), Pd/C (29mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water = 5% to 95%, collected at 15%) to obtain the product **UBI-1205** (70mg, yield 77%) as yellow oil. LCMS: $[M+H]^+$ = 606.3.

**Synthesis method of Compound UBI-1206 ($N_3$-17b-B(A1))**

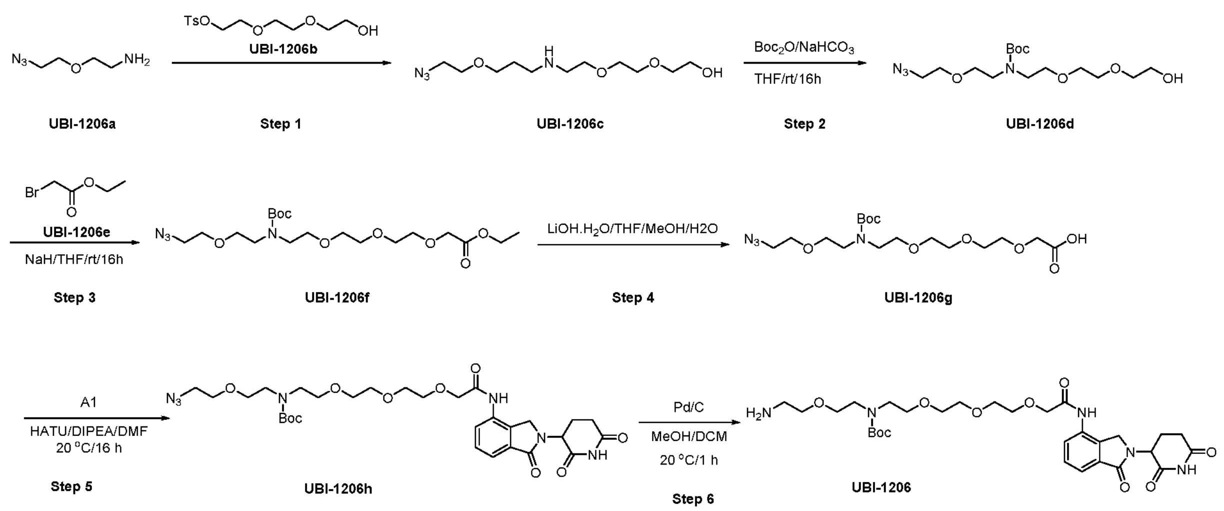


**Step 1: UBI-1206c**

Compound **UBI-1206a** (5.000g, 38.42mmol), **UBI-1206b** (11.69g, 38.42mmol), $K_2CO_3$ (6.371g, 46.10mmol) were added to acetonitrile (200 mL) and reacted at room temperature overnight under stiring. The reaction was filtered. The filtrate was concentrated and isolated by silica gel column chromatography (methanol/dichloromethane = 0% to 6%) to obtain product **UBI-1206c** (4.54g, yield 43%) as colorless transparent oil. $^1H$ NMR (400 MHz, chloroform-d) $\delta$ 3.76 - 3.70 (m, 2H), 3.68 - 3.60 (m, 12H), 3.40 - 3.37 (m, 2H), 2.87 - 2.80 (m, 4H), 2.18 (s, 2H). LCMS: $[M+H]^+$ = 263.2.

**Step 2: UBI-1206d**

Compound **UBI-1206c** (4.524g, 17.25mmol), $(Boc)_2O$ (7.528g, 34.49mmol), $NaHCO_3$ (2.898g, 34.49mmol) were added into tetrahydrofuran (50 mL), and reacted at room temperature overnight under srtiring. The reaction solution was filtered. The filtrate was concentrated and isolated by silica gel column chromatography (ethyl acetate/petroleum ether = 0% to 95%) to obtain product **UBI-1206d** (6.04g, yield 90%) as colorless transparent oil. $^1H$ NMR (400 MHz, chloroform-*d*) $\delta$ 3.77 - 3.70 (m, 2H), 3.70 - 3.54 (m, 12H), 3.47 (s, 4H), 3.37 (t, *J* = 5.2 Hz, 2H), 2.10 (s, 1H), 1.46 (s, 9H).

**Step 3: UBI-1206e**

Compound **UBI-1206d** (2.90 g, 8.00 mmol) was dissolved in tetrahydrofuran (20 mL), added NaH (640 mg, 16.0 mmol) under ice bath, and reacted for ten minutes. Then **UBI-1206e** (2.004 g, 12.00 mol) was added, and reacted at room temperature for 16 hours. the reaction was filtered. The filtrate was concentrated, added (50 mL) and extracted with ethyl acetate (50 mL*3). The organic phases were combined, washed with water (50 mL*3), saturated brine (50 mL*3) successively, dried over anhydrous sodium sulfate, and concentrated, then isolated by silica gel column chromatography (methanol/ dichloromethane = 0% to 10%)) to obtain product **UBI-1206f** (910 mg, 25%) as yellow oil. LCMS: $[M+H-100]^+$ = 349.3.

**Step 4: UBI-1206g**

Compound **UBI-1206f** (900mg, 1.41mmol) and LiOH.$H_2O$ (295mg, 7.02mmol) were dissolved in a mixed solvent consisting of methanol (3 mL), water (3 mL) and tetrahydrofuran (9 mL), and reacted at room temperature overnight under stirring. The reaction solution was added water, concentrated and then extracted with ethyl acetate. The aqueous phase was acidified to pH=5 using hydrochloric acid (1M), then extracted with ethyl acetate. The organic phase was combined, washed with water and saturated brine, then dried over anhydrous sodium sulfate, and concentrated to obtain product **UBI-1206g** (740mg, yield 83%) as colorless oil.
$^1$H NMR (400 MHz, DMSO-$d_6$) $\delta$ 12.42 (s, 1H), 4.01 (s, 2H), 3.64 - 3.56 (m, 4H), 3.55 - 3.43 (m, 10H), 3.39 (d, $J$ = 4.2 Hz, 2H), 3.37 - 3.33 (m, 4H), 1.39 (s, 9H). LCMS: [M+H-100] $^+$ = 321.2.

**Step 5: UBI-1206h**

Compound **UBI-1206g** (120mg, 0.28 mmol), **B(A1)** (74mg, 0.28mmol), and HATU (163 mg, 0.43 mmol) were dissolved in DMF (3 mL) and DIPEA (0.3 mL), and reacted at room temperature for 16 hours. The reaction was quenched with water (10 mL), then extracted with ethyl acetate (8 mL*3). The organic phases were combined, then dried over anhydrous sodium sulfate and concentrated to obtain crude product. The crude product was purified by reversed-phase column (methanol/water = 5% to 95%, collected at 70%) to obtain product **UBI-1206h** (90mg, yield 51%) as colorless oil. LCMS:[M+H-100]$^+$ = 562.3.

**Step 6: UBI-1206**

Under hydrogen atmosphere, compound **UBI-1206g** (90mg, 0.15mmol), Pd/C (29mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water=5% to 95%, collected at 15%) to obtain the product **UBI-1206** (65mg, yield 75%) as yellow oil. LCMS: [M+H] $^+$ = 636.5.

**Synthesis method of UBI-1209 ($N_3$-18b-B(A1))**

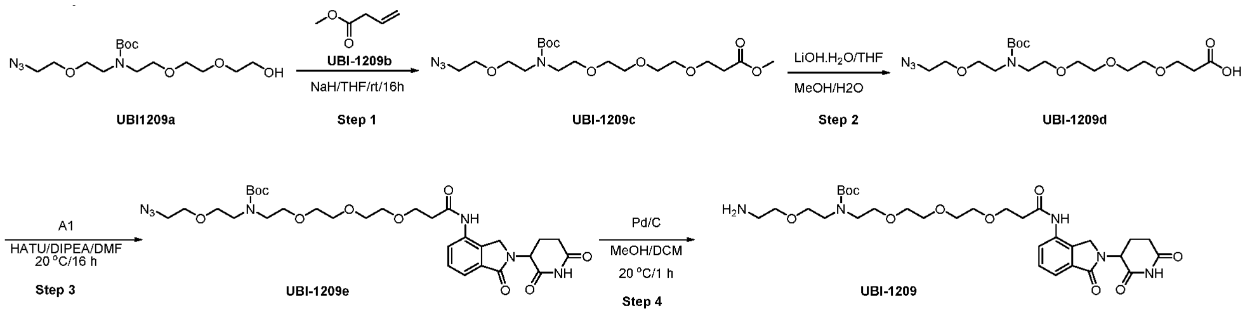


**Step 1: UBI-1209c**

Compound **UBI-1209a** (2.10g, 5.79mmol) was dissolved in tetrahydrofuran THF (10 mL), added NaH (46mg, 1.2mmol) and reacted at room temperature for 10 minutes. Then **UBI-1209b** (0.748 g, 8.69 mmol) was added, and the reaction was sirred at room temperature overnight. The reaction was quenched, concentrated and isolated by silica gel column chromatography (ethyl acetate/petroleum ether = 5% to 95%) to obtain product **UBI-1209c** (645mg, yield 25%) as yellow oil.
$^1$H NMR (400 MHz, chloroform-d) $\delta$ 3.81 - 3.72 (m, 2H), 3.71 - 3.66 (m, 3H), 3.65 - 3.54 (m, 12H), 3.46 (d, $J$ = 5.2 Hz, 4H), 3.38 - 3.34 (m, 2H), 2.68 - 2.53 (m, 2H), 1.58 - 1.57 (m, 2H), 1.45 (s, 9H).

**Step 2: UBI-1209d**

Compound **UBI-1209c** (600mg, 0.936mmol) and LiOH.$H_2O$ (0.196g, 4.68mmol) were dissolved in methanol (3 mL), tetrahydrofuran (3 mL) and water (9 mL), and reacted at room temperature overnight under stirring. The reaction solution was added water, concentrated and then extracted with ethyl acetate. The aqueous phase was acidified to

pH=5 using hydrochloric acid (1M), then extracted with ethyl acetate. The organic phases were combined, then washed with water, saturated brine, and dried over anhydrous sodium sulfate. After concentration, product **UBI-1209d** (305 mg, yield 47%) as yellow oil was obtained.

$^1$H NMR (400 MHz, DMSO-$d_6$) δ 12.19 (s, 1H), 3.65 - 3.55 (m, 2.9 Hz, 4H), 3.55 - 3.43 (m, 12H), 3.42 - 3.37 (m, 2H), 3.36 - 3.32 (m, 4H), 2.43 (t, *J*= 6.4 Hz, 2H), 1.39 (s, 9H). LCMS: [M+H-100] $^+$ = 335.3.

**Step3: UBI-1209e**

Compound **UBI-1209d** (120mg, 0.27mmol), **B(A1)** (71.6mg, 0.27mmol), and HATU (157 mg, 0.41 mmol) were dissolved in DMF (3 mL) and DIPEA (0.3 mL), and reacted at room temperature for 16 hours. The reaction was quenched with water (10 mL), then extracted with ethyl acetate (8 mL*3). The organic phases were combined, then dried over anhydrous sodium sulfate and concentrated to obtain crude product. The crude product was purified by reversed-phase column (methanol/water = 5% to 95%, collected at 70%) to obtain product **UBI-1209e** (90mg, yield 52.7%) as colorless oil. LCMS: [M+H-100] $^+$ = 676.3

**Step 4: UBI-1209**

Under hydrogen atmosphere, compound **UBI-1209e** (90mg, 0.15mmol), Pd/C (29mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water=5% to 95%, collected at 15%) to obtain the product **UBI-1209** (60mg, yield 70%) as yellow oil. LCMS: [M+H] $^+$ = 650.3

**Synthesis method of Compound UBI-1215 ($NH_2$-17b-B(A1))**

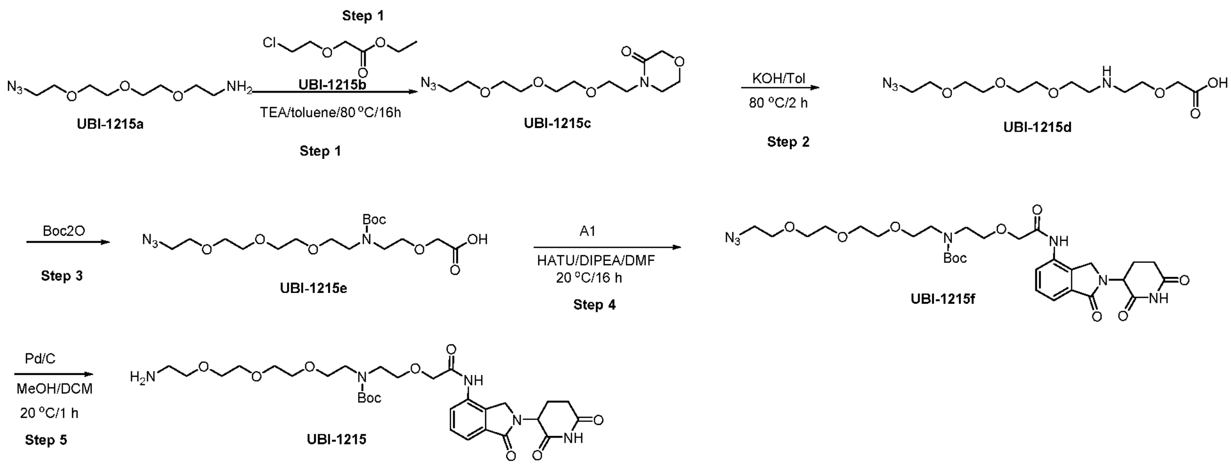


**Step 1: UBI-1215c**

Compound **UBI-1215a** (2.0 g, 9.17mmol), **UBI-1215b** (1.39g, 9.17mmol) were dissolved in TEA (1.39 g, 13.76mmol) and toluene (25mL), reacted at 80°C for 18 hours. The reaction solution was concentrated and isolated by silica gel column chromatography (petroleum ether/ethyl acetate = 50% to 100%, then MeOH/DCM=0% to 10%) to obtain target compound **UBI-1215** (500 mg, yield 18%) as colorless oil. $^1$H NMR (400 MHz, chloroform-*d*) δ 4.17 (s, 2H), 3.89 - 3.83 (m, 2H), 3.69 (ddd, *J* = 4.7, 1.9, 0.7 Hz, 4H), 3.66 (d, *J* = 1.3 Hz, 4H), 3.63 (dtt, *J* = 4.6, 2.9, 1.4 Hz, 4H), 3.61 - 3.58 (m, 2H), 3.53 (dd, *J* = 6.0, 4.2 Hz, 2H), 3.39 (t, *J* = 5.0 Hz, 2H).

**Step 2: UBI-1215d**

Compound **UBI-1215c** (400mg, 1.32mmol), KOH (371mg, 6.62mmol) were dissolved in water (10mL), and refluxed at 100°C for 2 hours. The reaction liquid was directly used in the next step after cooling. LCMS: [M+H] $^+$ = 321.1.

**Step 3: UBI-1215e**

To the aqueous solution of compound **UBI-1215d** (400mg, 1.25mmol), di-tert-butyl dicarbonate (545mg, 2.5 mmol) and dioxane (4mL) were added, and reacted at room temperature for 2 hours. The reaction solution was concentrated, added water (20 mL), and extracted with ethyl acetate (20 mL*3). The aqueous phase was concentrated to obtain the crude product, which was dissolved in MeOH/DCM=10/1 (30 mL), filtered, and the filtrate was concentrated to obtain the target compound **UBI-1215e** (300 mg). LCMS: $[M+H-100]^+ = 335.3$.

**Step 4: UBI-1215f**

Compound **UBI-1215e** (300mg, 0.71mmol), **B(A1)** (184mg, 0.71mmol), and HATU (406.9 mg, 1.07 mmol) were dissolved in DMF (10 mL) and DIPEA (0.3 mL), and reacted at room temperature for 16 hours. The reaction solution was directly isolated by reversed-phase column (MeOH/$H_2O$=5% to 95%, 45 minutes, collected at 60%) to obtain the target compound **UBI-1215f** (150mg, yield 32%) as white solid. LCMS: $[M+H-100]^+ = 562.3$.

**Step 5: UBI-1215**

Compound **UBI-1215f** (150mg, 0.08mmol) and Pd/C (20mg) were dissolved in DCM (5mL), and reacted under hydrogen atmosphere for 1 hours. After the completion of reaction, the reaction was filtrated. The filtrate was concentrated to obtain the target compound **UBI-1215** (100mg, yield 69%), which was directly used in the next step. LCMS: $[M+H]^+ = 636.3$.

**Synthesis method of Compound UBI-1171 ($N_3$-8b-B(A1))**

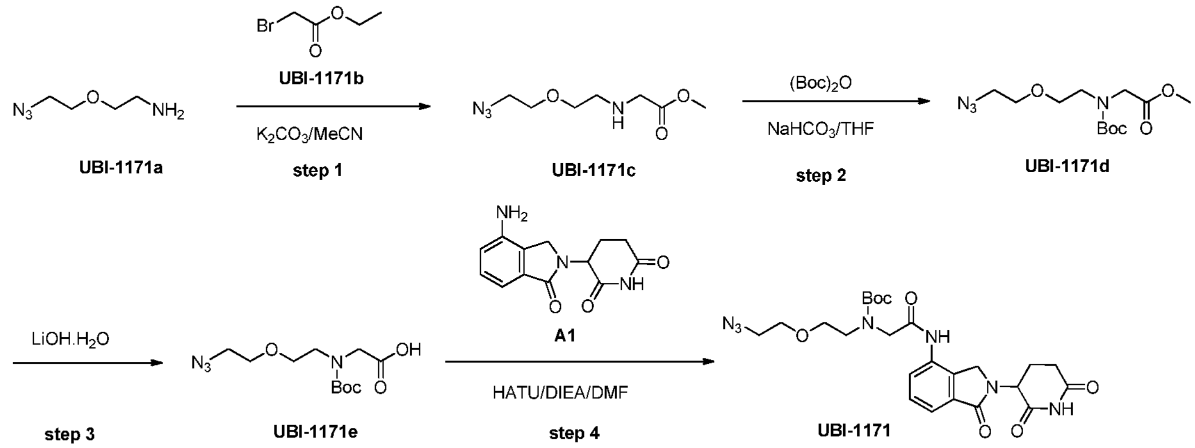


**Step 1: UBI-1171c**

Compound **UBI-1171a** (3 g, 23 mmol) was dissolved in acetonitrile (50 mL), added **UBI-1171b** (3.9 g, 23 mmol) and $K_2CO_3$ (4.8 g, 35 mmol), and reacted at room temperature for 16 hours. The reaction solution was filtered. Then the filtrate was concentrated and isolated by silica gel column chromatography (ethyl acetate/petroleum ether = 1/1 to methanol/dichloromethane = 1/10) to obtain target product **UBI-1171c** (2.6 g, yield 57 %) as colorless transparent oil. $^1H$ NMR (400 MHz, chloroform-*d*) $\delta$ 3.73 (s, 3H), 3.70 - 3.57 (m, 4H), 3.48 (dd, *J*= 5.6, 3.0 Hz, 2H), 3.39 (td, *J* = 5.1, 1.7 Hz, 2H), 2.83 (td, *J* = 5.0, 2.6 Hz, 2H), 1.79 (s, 1H).

**Step 2: UBI-1171d**

Compound **UBI-1171c** (2.6 g, 13 mmol) was dissolved in tetrahydrofuran (50 mL), then added $(Boc)_2O$ (4.2 g, 19 mmol) and $NaHCO_3$ (2.2 g, 26 mmol), and then reacted at room temperature for 16 hours. The reaction solution was added with water (10 mL) and extracted with ethyl acetate (15 mL*2). The organic phase was dried over $Na_2SO_4$ and then concentrated to obtain crude product **UBI-1171d** (3.6 g, yield 93%) as light yellow oil.

**Step 3: UBI-1171e**

Compound **UBI-1171d** (3.6 g, 12 mmol) was dissolved in methanol (10 mL), tetrahydrofuran (30 mL) and water (10 mL), and then added $LiOH.H_2O$ (751 mg, 18 mmol). The reaction was allowed to react at room temperature for 16 hours. The reaction solution was washed with ethyl acetate (20 mL) once, the aqueous phase was adjusted to pH=6 with 3N HCl, and then extracted with ethyl acetate (25 mL*2). After concentration of the organic phase, crude product **UBI-1171e** (2.9g, 85% yield), as colorless oil, was obtained. The crude product was directly used in the next step. $^1H$ NMR (400 MHz, chloroform-*d*) $\delta$ 10.52 (s, 1H), 4.06 (d, *J* = 14.6 Hz, 2H), 3.62 (dt, *J* = 15.8, 5.1 Hz, 4H), 3.55 - 3.42 (m, 2H), 3.35 (q, *J* = 4.5 Hz, 2H), 1.45 (d, *J* = 15.0 Hz, 9H).

**Step 4: UBI-1171**

At 0°C, compound **UBI-1171e** (350 mg, 1.2 mmol) was dissolved in N,N-dimethylformamide (8 mL) and then added HATU (924 mg, 2.4 mmol) and DIEA (313 mg, 2.4 mmol), followed by **B(A1)** (315 mg, 1.2 mmol). The system was reacted at room temperature for 16 h. The reaction solution was quenched with water (10 mL), extracted with ethyl acetate (10 mL*2). The organic phase was dried over $Na_2SO_4$ and then concentrated to obtain crude product. The crude product was purified by a reversed-phase column (methanol/water=5% to 95%, 45 minutes, collected at 80%) to obtain target product **UBI-1171** (350 mg, yield 54%) as yellow solid. LCMS $[M+H]^+$ =530.0

**Synthesis method of Compound UBI-1217 ($NH_2$-17b-B(A1))**

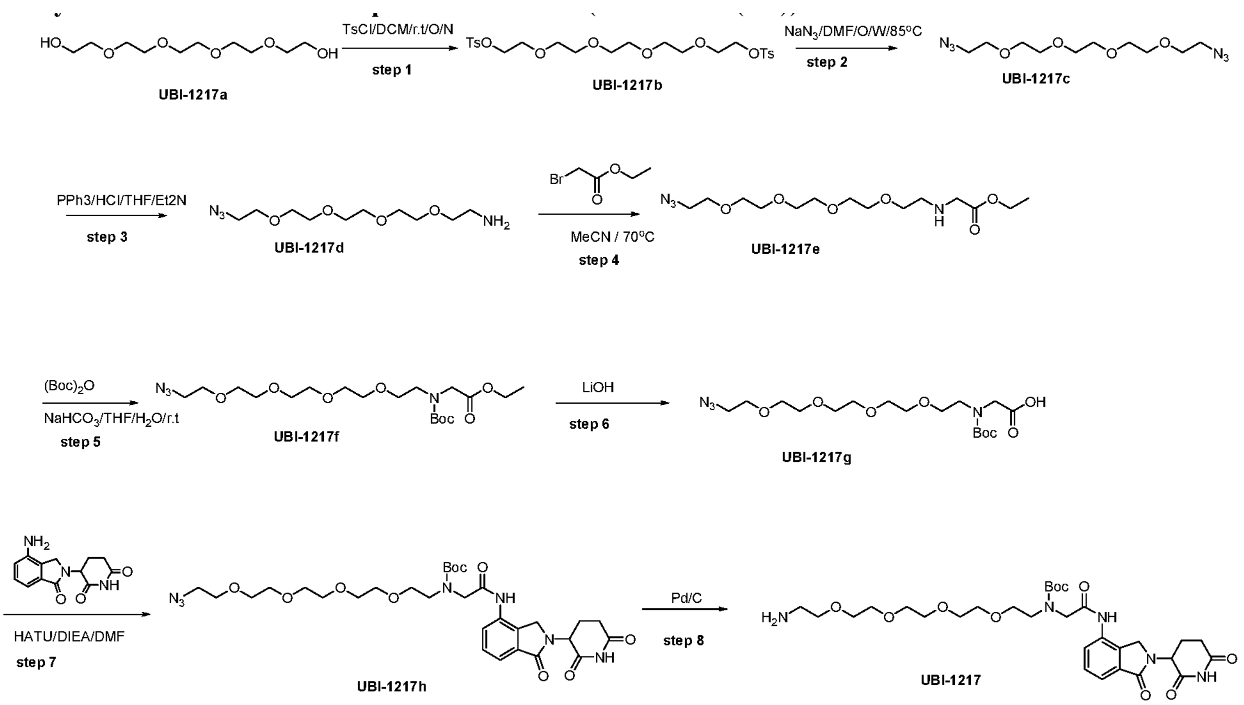


**Step 1: UBI-1217b**

UBI-1217a **(8.0** g, 34 mmol) was dissolved in dichloromethane (100 ml), added *p*-toluenesulfonyl chloride (14.7 g, 7.7 mmol), added triethylamine (10.2 g, 101 mmol) at 25 °C, and stirred overnight. After completion of reaction, to the reaction solution was added water (10ml) and extracted with dichloromethane (10ml*3). The organic phase was dried over $Na_2SO_4$ and concentrated to obtain crude product, which was purified by silica gel column chromatography (dichloromethane/methanol=30/1) to obtain colorless oil (18 g, yield 99%). LC-MS :$(M+H)^+$ =547.1

**Step 2: UBI-1217c**

**UBI-1217b** (18 g, 33 mmol) was dissolved in DMF (100 ml), added $NaN_3$ (6.43 g, 99 mmol), and stirred overnight

at 85 °C. After the completion of the reaction, the reaction solution was filtered and concentrated *in vacuo* to obtain crude product, which was purified with FFC (dichloromethane/methanol=30/1) to obtain colorless oil (8.7g, yield 92%).

**Step 3: UBI-1217d**

Compound **UBI-1217c** (8.7 g, 30 mmol) and triphenylphosphine (7.9 g, 30 mmol) were mixed and then dissolved in $ET_2O$/THF/1M HCl (100 ml), and stirred at 25 □ for 12 h. After the completion of the reaction, the reaction was added water, and extracted with ethyl acetate (50 ml*2), followed by the organic layer was dried over anhydrous magnesium sulfate and concentrated, the crude product was purified by silica gel column chromatography (EtOAc/PE=1/1) to obtain oil (3.9 g, yield 45%). $^1$H NMR (400 MHz, $CDCl_3$) δ3.69 - 3.62 (m, 14H), 3.52 - 3.40 (m, 2H), 3.39 - 3.37 (m, 2H), 2.87-2.80 (m, 2H), 1.40 (s, 2H).

**Step 4: UBI-1217e**

Compound **UBI-1217d** (2 g, 7.6 mmol), ethyl bromoacetate (1.27 g, 7.6 mmol) and potassium carbonate (2.06 g, 14.9 mmol) were mixed and dissolved in acetonitrile (20 mL), reacted at 80 °C for 18 hours. After the completion of reaction, the filtrate was concentrated by rotary evaporation under reduced pressure, and isolated by silica gel column chromatography (dichloromethane/methanol = 10/1) to obtain **UBI-1217e** (1.4g, yield 30%) as colorless oil.

**Step 5: UBI-1217f**

Compound **UBI-1217e** (1.3g, 3.7 mmol), di-tert-butyl dicarbonate (2.3g, 10.7mmol) and sodium bicarbonate (318 mg, 3.79 mmol) were added to dioxane (30mL) successively and reacted at room temperature for 2 hours. After the completion of the reaction, the reaction solution was poured into 10 mL of water and extracted with ethyl acetate (10 mL*3). The organic phases were combined, then washed with saturated brine, dried over anhydrous $Na_2SO_4$, and concentrated under reduced pressure to obtain compound **UBI-1217f** (1.4g, yield 83%).

**Step 6: UBI-1217g**

Compound **UBI-1217f** (150 mg, 0.335 mmol), sodium hydroxide (26.8 mg, 0.669 mmol) were added to water (5 mL) successively, reacted at RT for 12 hours. After the completion of reaction, it was concentrated and the aqueous phase was acidified to pH=5 using hydrochloric acid (1M). Then it was extracted with dichloromethane (10ml*3), the combined organic layer was dried over anhydrous $Na_2SO_4$, and concentrated to obtain the desired compound **UBI-1217g** (131 mg, yield 93%).

**Step 7: UBI-1217h**

**CompoundUBI-1217d** (101 mg, 0.24 mmol), **B(A1)** (75 mg, 0.29 mmol), HATU (137 mg, 0.36 mmol) and DIPEA (0.5mL) were added to anhydrous DMF (5 mL). The mixture was reacted at room temperature for 16 hours, concentrated after the completion of reaction. The crude product was isolated by silica gel column chromatography (dichloromethane/methanol = 10/1) to obtain compound **UBI-1217h** (81 mg, 51% yield) as white solid. LC-MS: $[M-100+H]^+$ =562.3

**Step 8: UBI-1217**

Compound **UBI-1217h** (81 mg, 0.12 mmol), 10% palladium on carbon (20 mg) was added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain target compound **UBI-1217** (63 mg, yield 82%) as white solid. LCMS: $(M-100+H)^+$= 536.3

**Synthesis method of Compound Linker c- Ligand B(A1)**

**Synthesis method of Compound UBI-1167 ($NH_2$-12c-B(A1))**

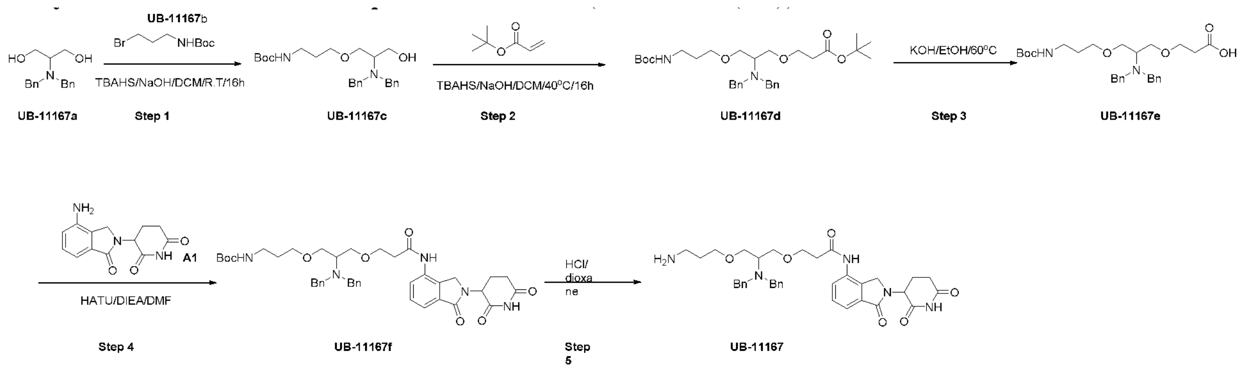


**Step 1: UBI-1167c**

A solution of Compound **UBI-1167a** (5g, 4.8mmol) in DCM (20mL) was cooled to 0°C, added NaOH (50% aq. 20mL), followed by tetrabutylammonium bisulfate (12.5g, 16.9mmol) and compound **UBI-1167b** (4.8g, 20.3mmol), reacted at 40°C for 16 hours. After cooling of reaction solution, the upper organic phase was collected, and the aqueous phase was extracted with dichloromethane (10 mL * 3). All organic phases were combined, then dried over anhydrous $Na_2SO_4$, concentrated by rotary evaporation under reduced pressure, and isolated by y silica gel column chromatography (petroleum ether/ethyl acetate = 10%) to obtain target compound **UBI-1167c** (2.25g) as colorless oil. $^1$H NMR (400 MHz, chloroform-*d*) δ 7.39-7.15 (m, 11H), 4.80 (s, 1H), 3.88 (d, *J* = 13.3 Hz, 2H), 3.71 - 3.43 (m, 8H), 3.31 - 3.20 (m, 2H), 3.17 - 3.02 (m, 1H), 1.77 (p, *J* = 12.8, 9.5 Hz, 2H), 1.43 (s, 9H). LC-MS: [M+H] $^+$ =429.1

**Step 2: UBI-1167d**

A solution of Compound **UBI-1167c** (1.44g, 3.36mmol) in DCM (20mL) was cooled to 0°C, added NaOH (50% aq. 20mL), followed by tetrabutylammonium bisulfate (12.28g , 6.72mmol) and tert-butyl acrylate (861mg, 6.72mmol), then reacted at 40°C for 16 hours. The reaction solution was extracted with dichloromethane (10 mL*3). Organic phases were combined, then dried over anhydrous $Na_2SO_4$, concentrated by rotary evaporation under reduced pressure, and isolated by silica gel column chromatography (petroleum ether/ethyl acetate = 10%) to obtain target compound **UBI-1167d** (480mg) as colorless oil. $^1$H NMR (400 MHz, DMSO-*d*$_6$) δ 7.40 - 7.25 (m, 8H), 7.22 - 7.14 (m, 2H), 6.75 (t, *J* = 5.8 Hz, 1H), 3.70 (s, 4H), 3.61 - 3.41 (m, 6H), 3.30 (d, *J* = 1.4 Hz, 2H), 3.03 - 2.95 (m, 2H), 2.89 (q, *J* = 6.0 Hz, 1H), 2.42 (t, *J* = 6.0 Hz, 2H), 1.61 (p, *J* = 6.5 Hz, 2H), 1.38 (d, *J* = 6.5 Hz, 18H).

**Step 3: UBI-1167e**

Compound **UBI-1167d** (2.45g, 4.4mmol) was dissolved in EtOH (20mL) and $H_2O$ (5mL), then added KOH (2.46g, 44mmol), and reacted at 60°C for 3 hours. After the completion of reaction, it was concentrated and the aqueous phase was acidified to pH=6 using diluted hydrochloric acid (1M). Then it was extracted with dichloromethane (20 mL*3). Organic phases were combined, then dried over anhydrous $Na_2SO_4$, concentrated by rotary evaporation under reduced pressure to obtain target compound UBI-1167e (2g). LC-MS: [M+H] $^+$ =500.7

**Step 4: UBI-1167f**

Compound **UBI-1167e** (1g, 2mmol), **B(A1)** (518mg, 2mmol), HATU (1.52g, 4mmol) were dissolved in DIPEA (1mL) and DMF (10mL), and reacted at room temperature for 16 hours. The reaction solution was directly isolated by reversed-phase column (MeOH/H2O = 5% to 95%, collected at 48%) to obtain target compound **UBI-1167f** (440mg) as white solid.

$^1$H NMR (400 MHz, DMSO-*d*$_6$) δ 11.01 (d, *J* = 4.4 Hz, 1H), 9.87 (d, *J* = 8.4 Hz, 1H), 7.94 - 7.79 (m, 1H), 7.53 - 7.43 (m, 2H), 7.40 - 7.11 (m, 10H), 6.75 (t, *J* = 5.5 Hz, 1H), 5.20 - 5.01 (m, 1H), 4.41 - 4.19 (m, 2H), 3.74 - 3.62 (m, 7H), 3.56 - 3.51 (m, 2H), 3.47 - 3.41 (m, 1H), 3.32 - 3.27 (m, 1H), 3.02 - 2.87 (m, 4H), 2.70 - 2.54 (m, 4H), 2.27 - 2.12 (m, 1H), 1.98 - 1.83 (m, 1H), 1.64 - 1.54 (m, 2H), 1.37 (s, 9H). LC-MS: [M+H] $^+$ =742.6.

**Step 5: UBI-1167**

Compound **UBI-1167f** (100mg, 0.135mmol) was dissolved in HCl/dioxane (10 mL), reacted at room temperature for 16 hours. The reaction solution was concentrated to obtain the target compound crude **UBI-1167g** (100mg), which

was directly used in the next step. LC-MS: $(M+H)^+$=641.7

**Synthesis method of Compound UBI-1050 ($NH_2$-5c-B(A1))**

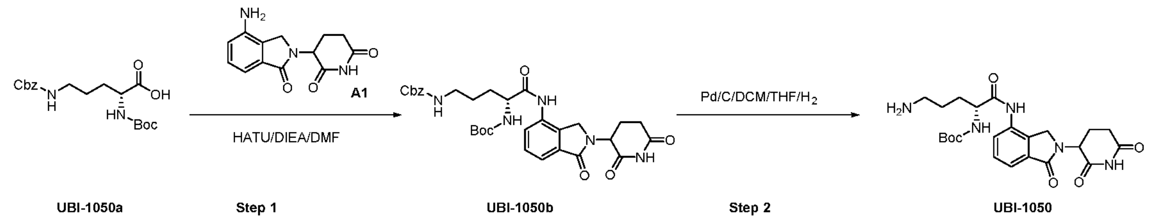


**Step 1: UBI-1050b**

**Compound UBI-1050a** (2g, 5.4mmol), **B(A1)** (1.4g, 5.4mmol), and HATU (4.1g, 10.8mmol) were dissolved in DMF (3 mL) and DIPEA (0.3 mL), and reacted at room temperature for 16 hours. The reaction solution was directly isolated by reversed-phase column ($MeOH/H_2O$ = 5% to 95%, 40 minutes, collected at 65%) to obtain target compound **UBI-1050b** (2g) as white solid. LCMS: $(M+H)^+$= 608.3.

**Step 2: UBI-1050**

Compound **UBI-1050b** (1g, 1.64mmol) and Pd/C (200 mg) were dissolved in DCM (200mL) and THF (100mL), and reacted under hydrogen atmosphere for 48 hours. After completion of the reaction, the reaction solution was concentrated, added 300 mL of MeOH/DCM=10:1 and stirred at room temperature for 3 hours, then filtered. The filtrate was concentrated to obtain the crude of target compound. The crude product was isolated and purified by reversed-phase column chromatography ($MeOH/H_2O$ = 5% to 95%, collected at 45%) to obtain target compound **UBI-1050** (200mg) as white solid.
$^1$H NMR (400 MHz, DMSO-$d_6$) $\delta$ 10.06 (s, 1H), 7.81- 7.73 (m, 1H), 7.57 - 7.44 (m, 3H), 7.21 - 7.10 (m, 1H), 5.26 - 5.12 (m, 1H), 4.44 - 4.27 (m, 2H), 4.13 (d, $J$ = 29.1 Hz, 1H), 2.97 - 2.88 (m, 1H), 2.83-2.72 (m, 2H), 2.65 - 2.56 (m, 1H), 2.36 - 2.27 (m, 1H), 2.10 - 2.01 (m, 1H), 1.64 (s, 4H), 1.39 (s, 9H). LCMS: $(M+H)^+$= 474.2.

**Synthesis method of Compound UBI-1051 ($NH_2$-6c-B(A1))**

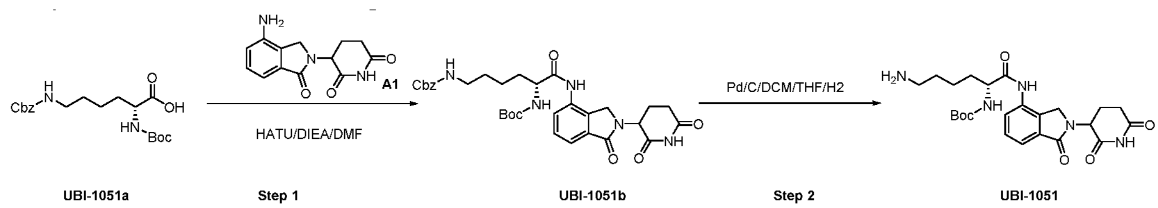


**Step 1: UBI-1051b**

Compound **UBI-1051a** (2g, 5.26mmol), **B(A1)** (1.36g, 5.26mmol), and HATU (4g, 10.52mmol) were dissolved in DMF (3 mL) and DIPEA (0.3 mL), and reacted at room temperature for 16 hours. The reaction solution was directly isolated by reversed-phase column ($MeOH/H_2O$ = 5% to 95%, 40 minutes, collected at 65%) to obtain target compound **UBI-1051b** (1.8g) as white solid. LCMS: $(M+H)^+$= 622.3.

**Step 2: UBI-1051**

Compound **UBI-1051b** (1g, 1.64mmol) and Pd/C (200 mg) were dissolved in DCM (200mL) and THF (100mL), and reacted under hydrogen atmosphere for 48 hours. After completion of the reaction, the reaction solution was concentrated, added 300 mL solvent of MeOH/DCM=10:1 and stirred at room temperature for 3 hours, then filtered. The filtrate was concentrated to obtain the crude of target compound. The crude product was washed with methanol (3mL*3)

and filtered to obtain target compound **UBI-1051** (800mg) as a white solid. $^1$H NMR (400 MHz, DMSO-$d_6$) $\delta$ 11.03 (s, 1H), 10.03 (s, 1H), 7.96 - 7.71 (m, 3H), 7.60 - 7.43 (m, 2H), 7.08 (dd, *J*= 7.7, 4.1 Hz, 1H), 5.16 (dt, *J*= 13.3, 5.0 Hz, 1H), 4.34 (q, *J*= 18.4, 17.9 Hz, 2H), 4.15 - 4.05 (m, 1H), 3.03 - 2.88 (m, 1H), 2.78 (t, *J*= 7.5 Hz, 2H), 2.67 - 2.56 (m, 1H), 2.37 - 2.21 (m, 1H), 2.09 - 1.99 (m, 1H), 1.79 - 1.54 (m, 4H), 1.39 (s, 11H). LCMS: (M+H) $^+$= 488.4.

**Synthesis method of Compound UBI-1040 ($NH_2$-7c-B(A1))**

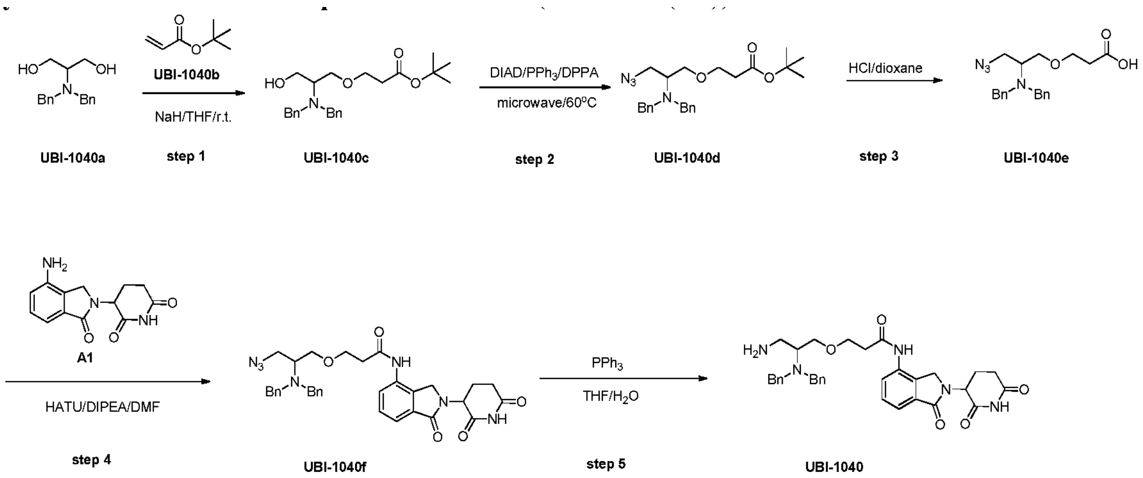


**Step** 1: **UBI-1040c**

Compound **UBI-1040a** (5g, 18.5 mmol), **UBI-1040b** (2.4g, 18.5 mmol) were dissolved in tetrahydrofuran (100 mL), then cooled to 0°C under ice bath, and then added catalytic amount of NaH. The reaction was carried out at room temperature for 18 hours. The reaction solution was added water (30 mL), extracted with ethyl acetate (40 mL*3). The organic phases were combined, concentrated and subjected to silica gel column chromatography (ethyl acetate/petroleum ether = 1/5) to obtain product. **UBI-1040c** (3.6 g, yield 48%) as colorless oil.

**Step 2: UBI-1040d**

Compound **UBI-1040c** (800mg, 2.0 mmol), $PPH_3$ (1.3 g, 5.0 mmol) and DIAD (1.0 g, 5.0 mmol) were dissolved in dry tetrahydrofuran (15 mL) and cooled to 0 °C under ice bath, then added DPPA (1.4 g, 5.0 mmol) slowly. The reaction solution was heated to 60 ° C by microwave and reacted for 2 hours. The reaction solution was concentrated and subjected to silica gel column chromatography (dichloromethane/petroleum ether = 1/5) to obtain product **UBI-1040d** (300 mg, yield 36%) as colorless oil.

**Step 3: UBI-1040e**

Compound **UBI-1040e** (860 mg, 2.0 mmol) was dissolved in a solution of HCl in dioxane (15 mL), reacted at room temperature for 2 hours. The reaction solution was concentrated to obtain crude product **UBI-1040e** (750 mg, yield 100%) as light pink oil. The crude product was directly used in the next step.

**Step 4: UBI-1040f**

Compound **UBI-1140e** (750mg, 2.0mmol), **B(A1)** (528mg, 2.0mmol), and HATU (929mg, 2.4mmol) were dissolved in DMF (8 mL) and DIPEA (0.5 mL), and the reaction was reacted at room temperature for 18 hours. The reaction solution was directly subjected to silica gel column chromatography (methanol/dichloromethane = 1/3) to obtain product **UBI-1040f** (750mg, yield 60%) as colorless oil.

**Step 5: UBI-1040**

Compound **UBI-1040f** (750 mg, 1.2 mmol) and $PPh_3$ (387 mg, 1.5 mmol) were dissolved in tetrahydrofuran

(30 mL), reacted at room temperature for 18 hours, then added water (10 mL), and continued to react for 1 hour. The reaction solution was added water (10 mL), extracted with ethyl acetate (20 mL*3). The organic phases were combined and concentrated, purified by a reversed-phase column (methanol/water = 5% to 95%, 45 minutes, collected at 40%) to obtain product **UBI-1040** (54mg, yield 8%) as white solid. $^1$H NMR (400 MHz, DMSO-$d_6$) δ 9.90 (s, 1H), 7.87 (dd, *J* = 7.7, 4.5 Hz, 2H), 7.63 - 7.06 (m, 11H), 5.13 (dt, *J* = 13.4, 4.3 Hz, 1H), 4.34 (d, *J* = 5.9 Hz, 2H), 3.85 - 3.48 (m, 6H), 3.30 (s, 2H), 3.05 - 2.78 (m, 3H), 2.67 *(d, J*= 4.6 Hz, 2H), 2.29 - 1.81 (m, 6H).

**Synthesis method of Compound UBI-1054 ($N_3$-12c-B(A1))**

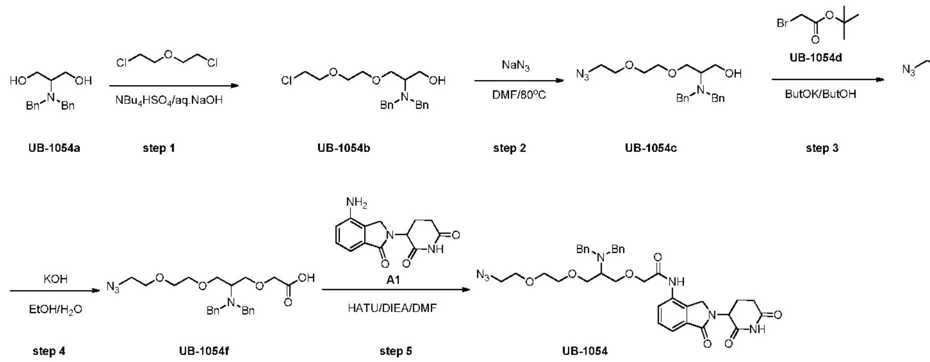


**Step 1: UBI-1054b**

Compound **UBI-1054a** (10 g, 37 mmol) was dissolved in dichloroether (50 mL), and then added $N(Bu)_4HSO_4$ (12.5 g, 37 mmol). After the reaction was cooled to 0°C under ice bath, 50% NaOH (100 mL) was slowly added dropwise to the reaction, with dropping time of over 15 minutes. At the final reaction was carried out at 40°C for 18 hours. The reaction solution was added water (50 mL), extracted with dichloromethane (80 mL*3). The organic phases were combined and then concentrated and subjected to silica gel column chromatography (ethyl acetate/petroleum ether = 1/5) to obtain light yellow product **UBI-1054b** (8.8 g, yield 63%).
$^1$H NMR (400 MHz, chloroform-d) δ 7.43 - 7.14 (m, 10H), 3.86 (d, *J* = 13.4 Hz, 2H), 3.81 - 3.75 (m, 3H), 3.72 - 3.52 (m, 11H), 3.29 - 3.00 (m, 1H), 2.89 (s, 1H).

**Step 2: UBI-1054c**

Compound **UBI-1054b** (7.5 g, 20 mmol) was dissolved in DMF (50 mL), then added $NaN_3$ (1.6 g, 24 mmol). The reaction was heated to 80°C reated for 4 hours. The reaction solution was added iced water (30 mL), then extracted with dichloromethane (50 mL*3). The organic phases were combined, then dried over anhydrous sodium sulfate, and then concentrated to obtain crude product as yellow oil. The crude product was subjected to silica gel column chromatography (ethyl acetate/petroleum ether = 1/4) to obtain product **UBI-1054c** (10 g, yield 100%) as light yellow oil. LCMS [M+1]+ = 384.0

**Step 3: UBI-1054e**

Compound **UBI-1054c** (4.0 g, 10.42 mmol) was dissolved in ButOH (150 mL), then added ButOK (1.4 g, 12.50 mmol) and **UBI-1054d** (2.0 g, 10.42 mmol) at 0 °C. The reaction was reacted at room temperature for 18 hours. The reaction solution was concentrated, then added water (50 mL), and extracted with ethyl acetate (80 mL*3). The organic phases were combined, then dried over anhydrous sodium sulfate, and concentrated to obtain crude product. The crude product was subjected to silica gel column chromatography (ethyl acetate/petroleum ether = 1/4) to obtain product **UBI-1054e** (520mg, yield 10%) as white solid.
$^1$H NMR (400 MHz, chloroform-d) δ 7.43 - 7.15 (m, 10H), 3.96 - 3.89 (m, 2H), 3.81 (s, 4H), 3.77 - 3.54 (m, 10H), 3.35 (t, J = 5.1 Hz, 2H), 3.13 (p, J = 6.0 Hz, 1H), 1.48 (s, 9H).

**Step 4: UBI-1054f**

Compound **UBI-1054e** (520 mg, 1.04 mmol) was dissolved in EtOH (10 mL) and water (1 mL), and added KOH

(234 mg, 4.18 mmol) at 0 °C, and reacted at room temperature for 1 hour. The reaction solution was concentrated to obtain crude product. The crude product was added water (10 mL), acidified to pH=3 with 3N HCl, and then lyophilized to obtain 800 mg crude product. The crude product was eluted with methanol/dichloromethane=1/10 (20 mL) and concentrated to obtain product **UBI-1054f** (500 mg, yield 100%) as white solid.

**Step 5: UBI-1054**

Compound **UBI-1054f** (250mg, 0.57mmol), **B(A1)** (146 mg, 0.57mmol), and HATU (258mg, 4.27) were dissolved in DMF (8 mL) and DIPEA (0.5 mL), and reacted at room temperature for 18 hours. The reaction solution was directly purified via reversed-phase column (methanol/water = 5 % to 95 %, collected at 80%) to obtain product **UBI-1054** (50mg, yield 13%) as yellow solid.
$^1$H NMR (400 MHz, DMSO-$d_6$) δ 11.00 (s, 1H), 9.63 (d, *J* = 5.2 Hz, 1H), 7.77 (ddd, *J* = 7.6, 3.8, 1.3 Hz, 1H), 7.62 - 7.46 (m, 2H), 7.38 (d, *J*= 8.1 Hz, 4H), 7.28 (t, *J*= 7.4 Hz, 4H), 7.23 - 7.12 (m, 2H), 5.12 (dd, *J*= 13.2, 5.2 Hz, 1H), 4.41 - 4.24 (m, 2H), 4.09 (d, *J*= 1.5 Hz, 2H), 3.89 - 3.45 (m, 16H), 3.08 - 2.82 (m, 2H), 2.58 (s, 1H), 2.31 - 2.11 (m, 1H), 2.03 - 1.90 (m, 1H).
**Step 6:** compound UBI-1054 was reduced by the method in accordence with the preparation of compound UBI-1040.

**Synthesis method of Compound Linker d-Ligand B(A1)**

**Synthesis method of Compound UBI-1105 ($N_3$-13d-B(A1)):**

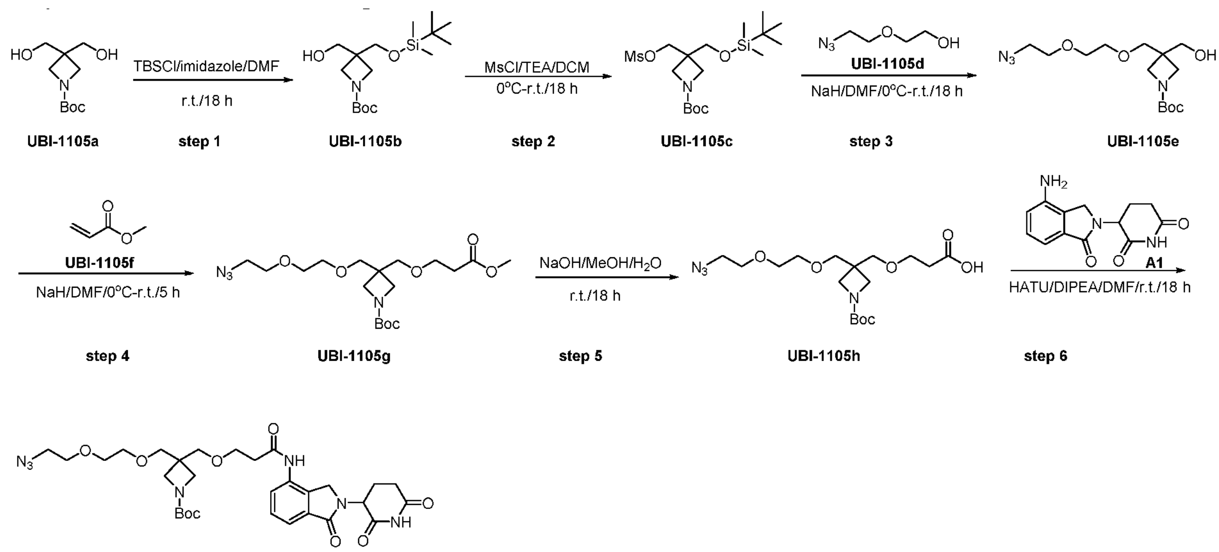


UBI-1105

**Step 1: UBI-1105b**

**Compound UBI-1105a** (1 g, 4.6 mmol) was dissolved in anhydrous DMF (20 mL), added imidazole (1.56 g, 23 mmol) and TBSCl (829 mg, 5.5 mmol) after cooling to 0°C, and reacted at 0°C for 1 hour, then reacted at room temperature overnight. The reaction solution was concentrated and isolated by silica gel column chromatography (dichloromethane /methanol = 20/1, Rf=0.5) to obtain product **UBI-1105b** (500 mg, yield 32%) as white solid. $^1$H NMR (400 MHz, chloroform-*d*) δ 3.73 (d, 4H), 3.58 - 3.60 (d, 2H), 3.51 - 3.53 (d, 2H), 1.35 (s, 9H), 0.82 (s, 9H), 0.0 (s, 6H). LCMS: $[M+H]^+$ = 332

**Step 2: UBI-1105c**

Compound **UBI-1105b** (800 mg, 2.4 mmol) was dissolved in anhydrous dichloromethane (30 mL), and then added triethylamine (48 mg, 4.8 mmol) and MsCl (304 mg, 2.64 mmol) at 0°C, and reacted at 0°C for 1 hour, then reacted at room temperature overnight. The reaction was quenched with water (50 mL), then extracted with dichloromethane

(50 mL*3). The organic layers were combined, dried over anhydrous sodium sulfate and concentrated. The crude product was isolated by silica gel column chromatography (dichloromethane/methanol = 20/1) to obtain product **UBI-1105c** (1 g, yield 100%) as white solid. $^1$H NMR (400 MHz, chloroform-*d*) δ 4.27(s, 2H), 3.65 (s, 2H), 3.62 (s, 4H), 2.97 (s, 3H), 1.38 (s, 9H), 0.82 (s, 9H), 0.0 (s, 6H). LCMS: [M+H] $^+$= 310

**Step 3: UBI-1105e**

Compound **UBI-1105d** (445 mg 3.4 mmol) was dissolved in anhydrous DMF (10 mL), added 60% NaH (272 mg, 6.8 mmol) in batches at 0°C, reacted for 1 hour at 0°C, then added **UBI-1105c** (1 g crude, 1.7 mmol) to dissolve, and reacted overnight. The reaction was quenched with water (20 mL), then extracted with dichloromethane (50 mL*3). The organic layers were combined, dried over anhydrous sodium sulfate and concentrated. The crude product was isolated by silica gel column chromatography (dichloromethane /methanol = 94/6, Rf = 0.7) to obtain product **UBI-1105e** (450 mg, yield 32%) as yellow oil. $^1$H NMR (400 MHz, DMSO-$d_6$) δ 4.62 (s, 2H), 3.98 (s, 2H), 3.61 (m, 2H), 3.58 (m, 2H), 3.54 - 3.44 (m, 6H), 3.39 (m, 2H), 1.36 (s, 9H).

**Step 4: UBI-1105g**

Compound **UBI-1105e** (400 mg, 1.21 mmol), **UBI-1105f** (104 mg, 1.21 mmol) were dissolved in anhydrous DMF (5 mL), added 60% NaH (5 mg, 0.12 mmol) in batches at 0 °C. After that, reacted for 4 hours, the reaction was quenched with water (20 mL), then extracted with ethyl acetate (20 mL*3). The organic layers were combined, dried over anhydrous sodium sulfate and concentrated. The crude product was isolated by silica gel column chromatography (dichloromethane /methanol = 94/6, Rf = 0.7) to obtain product **UBI-1105g** (300 mg, yield 59%) as yellow oil. $^1$H NMR (400 MHz, DMSO-$d_6$) δ 4.62 (s, 2H), 3.98 (s, 2H), 3.64 (m, 3H), 3.62 - 3.56 (m, 5H), 3.56 - 3.43 (m, 6H), 3.39 (d, J = 5.0 Hz, 2H), 3.24 - 3.20 (m, 1H), 2.54 (t, J = 6.2 Hz, 2H), 1.36 (d, J = 1.1 Hz, 9H).

**Step 5: UBI-1105h**

Compound **UBI-1105g** (260 mg, 0.62 mmol) was dissolved in methanol (5 mL) and added 2M NaOH (1.6 mL, 3.1 mmol), reacted at room temperature overnight. The reaction solution was concentrated and added saturated brine (15 mL), extracted with ether (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain crude product **UBI-1105h** (120 mg, yield 46%) as yellow oil, which is directly used in the next step.

**Step 6: UBI-1105**

Compound **UBI-1105h** (120 mg, 0.3 mmol), **B(A1)** (93 mg, 0.36 mmol), HATU (137 mg, 0.36 mmol), DIPEA (0.3 mL) were dissolved in DMF (3 mL), reacted at room temperature overnight. The reaction was quenched with water (20 mL) and extracted with ethyl acetate (50 mL*3). The organic layer was combined, dried over anhydrous sodium sulfate and concentrated. The crude product was isolated by silica gel column chromatography (dichloromethane/methanol=90/10) and reversed phase (water/methanol=20/80) to obtain crude product **UBI-1105** (12 mg, yield 6%) as white solid. $^1$H NMR (400 MHz, DMSO-$d_6$) δ 11.01 (s, 1H), 9.86 (s, 1H), 7.80 (m, 1H), 7.54 - 7.45 (m, 2H), 5.15 (dd, J = 13.3, 5.1 Hz, 1H), 4.39 - 4.29 (m, 2H), 3.75 (m, 2H), 3.65 - 3.56 (m, 8H), 3.55 - 3.47 (m, 8H), 3.37 (m, 2H), 2.97 - 2.88 (m, 1H), 2.63 (m, 2H), 2.36 - 2.27 (m, 2H), 2.02 (m, 1H), 1.34 (d, J = 15.4 Hz, 9H). LCMS: [M+H] $^+$= 644

**Synthesis method of Compound UBI-1104 ($N_3$-12d-COOH):**

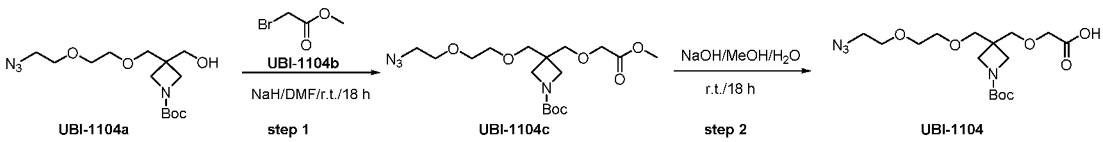


UBI-1104a step 1 UBI-1104c step 2 UBI-1104

**Step 1: UBI-1104c**

Compound **UBI-1104a** (700 mg 2.1 mmol) was dissolved in anhydrous DMF (5 mL), added 60% NaH (252 mg, 6.3 mmol) in batches at 0°C, reacted for 1 hour at 0°C. Compound **UBI-1104b** (324 mg, 2.1 mmol) was added at 0°C,

and then reacted overnight. The reaction was quenched by adding water (20 mL), then extracted with dichloromethane (50 mL*3). The organic layers were combined, dried over anhydrous sodium sulfate and concentrated. The crude product was isolated by silica gel column chromatography (dichloromethane/methanol = 90/10) to obtain crude product **UBI-1104c** (300 mg, yield 35%) as yellow oil. $^1$H NMR (400 MHz, DMSO-$d_6$) δ 4.63 (s, 2H), 3.98 (s, 2H), 3.72 - 3.46 (m, 11H), 3.39 (m, 1H), 2.90 (m, 3H), 2.59 - 2.53 (m, 2H), 1.36 (s, 9H). LCMS: [M+H] $^+$ = 403.

**Step 2: UBI-1104**

Compound **UBI-1104c** (200 mg, 0.49 mmol) was dissolved in methanol (5 mL) and added 2M NaOH (1.24 mL, 2.48 mmol). Reacted at room temperature overnight. The reaction solution was concentrated, added saturated brine (15 mL), extracted with ether twice (20 mL*2). The aqueous phase was acidified to pH=6 using 1M HCl, and then extracted with ethyl acetate (40 mL*3). The organic phases were combined, dried over anhydrous sodium sulfate, filtered and concentrated to obtain crude product **UBI-1104** (80 mg, yield 42%) as yellow oil.

**Step 3:** compound UBI-1104 was reduced by the method in accordence with the preparation of compound UBI-1040.

**Synthesis method of Compound Linkere- Ligand B(A1)/B(B1)**

**Synthesis method** of **Compound UBI-1143 ($NH_2$-12e-B(A1))**

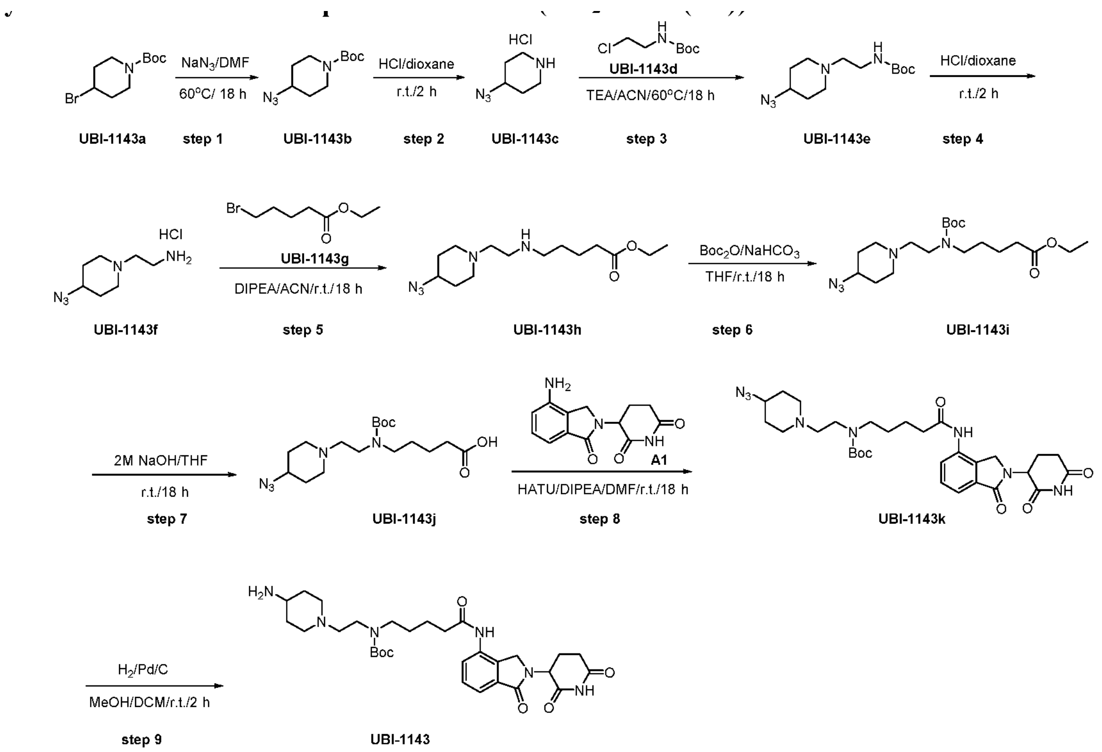


**Step 1: UBI-1143b**

Compound **UBI-1143a** (10 g, 37.8 mmol) was dissolved in anhydrous DMF (50 mL), added sodium azide (2.95 g, 45.4 mmol), reacted 60°C overnight. The reaction was quenched by adding water (20 mL) and extracted with ethyl acetate (80 mL*3). The organic layers were combined, dried over anhydrous sodium sulfate and concentrated. The crude product was isolated by silica gel column chromatography (petroleum ether/ethyl acetate = 9/1) to obtain product **UBI-1143b** (8.6 g, yield 100%) as colorless oil. $^1$H NMR (400 MHz, chloroform-d) δ 3.82 (m, 2H), 3.61 - 3.52 (m, 1H), 3.09 (m, 2H), 1.87 (m, 2H), 1.62 - 1.53 (m, 2H), 1.46 (s, 9H). LCMS: [M-56] $^+$ = 171

**Step 2: UBI-1143c**

Compound **UBI-1143b** (8.6 g, 38 mmol) was added to a solution of 4M hydrochloric acid in dioxane (47.5 mL) in ice bath, and reacted at room temperature for 2 hours. The reaction solution was concentrated to obtain crude product **UBI-1143c** (6.3 g, yield 100%) as yellow solid, which was directly used in the next step.

**Step 3: UBI-1143e**

Compound **UBI-1143c** (5.6 g, 34.4 mmol), **UBI-1143d** (8.8 g, 49 mmol), triethylamine (11 mL, 78 mmol) were dissolved in acetonitrile (100 mL), then reacted 60 °C overnight. The reaction was quenched with water (50 mL), extracted with ethyl acetate (50 mL*3). The organic layers were combined, dried over anhydrous sodium sulfate and concentrated. The crude product was isolated by silica gel column chromatography (petroleum ether/ethyl acetate = 70/30, Rf = 0.3) to obtain product **UBI-1143e** (3.1 g, yield 33%) as yellow oil.

**Step 4: UBI-1143f**

Compound **UBI-1143e** (3.1 g, 11.5 mmol) was added to a solution of 4M hydrochloric acid in dioxane (35 mL) under ice bath, and reacted at room temperature for 2 hours. The reaction solution was concentrated to obtain product **UBI-1143f** (2.36 g, yield 100%) as yellow oil, which was directly used in the nextstep.

**Step 5: UBI-1143h**

Compound **UBI-1143f** (1.52 g, 7.3 mmol) was dissolved in acetonitrile (60 mL), added DIPEA (6 mL, 36.8 mmol), **UBI-1143g** (1.54 g, 7.3 mmol), and reacted at room temperature overnight. The reaction was quenched with water (50 mL), extracted with ethyl acetate (50 mL*3). The organic layers were combined, dried over anhydrous sodium sulfate and concentrated. The crude product was isolated by silica gel column chromatography (dichloromethane /methanol = 40/60, Rf = 0.7) to obtain **UBI-1143g** (400 mg, yield 18%) as yellow solid.
$^1$H NMR (400 MHz, DMSO-$d_6$) δ 4.06 (q, J = 7.1 Hz, 2H), 3.56 (m, 1H), 3.28 (m, 1H), 2.98 (m, 2H), 2.89 (t, J = 7.5 Hz, 2H), 2.73 (m, 2H), 2.55 (m, 2H), 2.34 (t, J = 7.1 Hz, 2H), 2.18 (m, 2H), 1.85 (m, 2H), 1.68 - 1.49 (m, 6H), 1.31 - 1.23 (m, 3H), 1.19 (t, J = 7.1 Hz, 3H). LCMS: $[M+H]^+$ = 298

**Step 6: UBI-1143i**

Compound **UBI-1143h** (400 mg, 1.34 mmol) was dissolved in tetrahydrofuran (10 mL), added $NaHCO_3$ (338 mg, 1.61 mmol) and $Boc_2O$ (352 mg, 1.61 mmol), and reacted at room temperature overnight. After being quenched by adding water (20 mL), the reaction solution was extracted with ethyl acetate (20 mL*3). The organic layers were combined, dried over anhydrous sodium sulfate and concentrated. The crude product was isolated by silica gel column chromatography (dichloromethane/methanol = 90/10) to obtain product **UBI-1143i** (400 mg, yield 75%) as yellow oil. LCMS: $[M+H]^+$ = 398

**Step 7: UBI-1143j**

Compound **UBI-1143i** (400 mg, 1 mmol) was dissolved in tetrahydrofuran (10 mL), added 2M NaOH (5 mL, 10 mmol), and reacted at room temperature overnight. The reaction was added saturated brine (15 mL) and extracted with ether twice (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain crude product **UBI-1143j** (380 mg, yield 100%) as colorless oil. LCMS: $[MS+H]^+$=370

**Step 8: UBI-1143k**

Compound **UBI-1143j** (370 mg, 1 mmol), **B(A1)** (260 mg, 1 mmol), HATU (570 mg, 1.5 mmol), and DIPEA (1 mL) were dissolved in DMF (5 mL), reacted at room temperature overnight. The reaction was quenched with water (50 mL), extracted with ethyl acetate (50 mL*3). The organic layers were combined, dried over anhydrous sodium sulfate and concentrated. The crude product was isolated by silica gel column chromatography (dichloromethane/methanol = 93/7) to obtain crude product **UBI-1143k** (360 mg, yield 59%) as colorless oil. LCMS: $[M+H]^+$= 611 $^1$H NMR (400 MHz, DMSO-$d_6$) δ 11.03 (s, 1H), 9.78 (s, 1H), 7.82 (dd, J = 7.1, 1.9 Hz, 1H), 7.50 (m, 2H), 5.16 (m, 1H), 4.44 - 4.27 (m, 2H), 3.61 (m, 1H), 3.48 (m, 1H), 3.17 (m, 4H), 2.98 - 2.87 (m, 1H), 2.78 - 2.57 (m, 2H), 2.43 - 2.22 (m, 4H), 2.21 - 2.00 (m, 3H), 1.81 (m, 2H), 1.47 (m, 13H), 1.28 - 1.22 (m, 2H).

**Step 9: UBI-1143**

Compound **UBI-1143k** (360 mg, 0.58 mmol) was dissolved in dichloromethane (20 mL) and methanol (2 mL), added Pd/C (250 mg), and reacted for 2 hours under hydrogen atmosphere. Filtered through Celite, the filtrate was concentrated to obtain product **UBI-1143** (300 mg, yield 88%) as yellow solid, which was directly used in the next step. LCMS: [M+H]+ = 585

**Synthesis method of Compound UBI-1159 ($NH_2$-12e-B(B1)):**

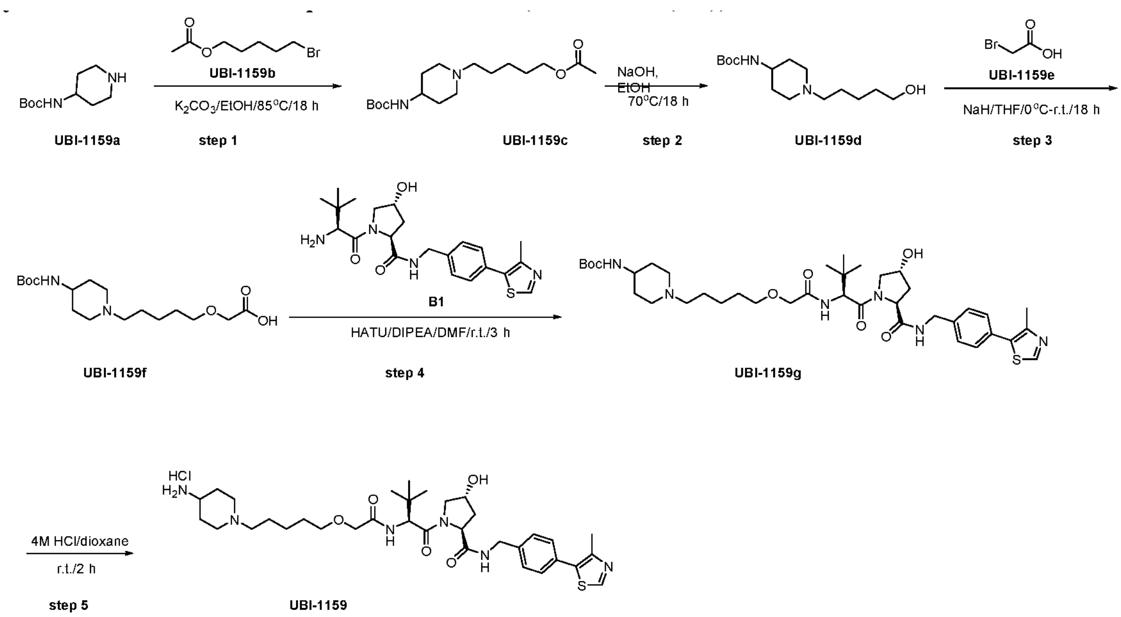


**Step 1: UBI-1159c**

Compound **UBI-1159a** (2 g, 10 mmol), **UBI-1159b** (2 mL, 12 mmol), anhydrous potassium carbonate (11 g, 80 mmol) were dissolved in acetonitrile (40 mL), reacted at 85 °C overnight. The reaction was cooled to room temperature, poured into water and then extracted with ethyl acetate (40 mL*2). The organic layers were combined, dried over anhydrous sodium sulfate and concentrated. The crude product was isolated by silica gel column chromatography (dichloromethane/methanol = 10/1) to obtain product **UBI-1159c** (1.9 g, yield 58%) as white solid. LCMS: $[M+H]^+$ = 329

**Step 2: UBI-1159d**

Compound **UBI-1159c** (1.9 g, 5.8 mmol), 2N NaOH (8.7 mL, 17.4 mmol) were dissolved in ethanol (30 mL), and reacted at 70°C overnight. The reaction was cooled to room temperature, poured into water and then extracted with ethyl acetate (40 mL*2). The organic layers were combined, dried over anhydrous sodium sulfate and concentrated. The crude product was isolated by silica gel column chromatography (dichloromethane/methanol = 10/1) to obtain product **UBI-1159c** (1.3 g, yield 78%) as white solid. LCMS: [M+H]+ = 287

**Step 3: UBI-1159**

Compound **UBI-1159d** (1 g, 3.5 mmol) was dissolved in anhydrous tetrahydrofuran (10 mL) and cooled to 0°C, added 60% NaH (276 mg, 6.9 mmol) in batches, reacted at 0 °C for half an hour. Then the compound **UBI-1159e** (324 mg, 2.3 mmol) was dissolved in anhydrous tetrahydrofuran (2 mL) and added to the above reaction solution, and reacted at room temperature overnight. The reaction was poured into water and then extracted with ethyl acetate (40 mL*2). The aqueous phase was acidified to pH=6 using 1M HCl. The crude product was concentrated and isolated by silica gel column chromatography (dichloromethane /methanol = 90/10) to obtain product as white solid (290 mg, yield 36%) . $^1H$ NMR (400 MHz, DMSO-$d_6$) δ 6.93 (d, 1H), 3.89 (s, 2H), 3.44 (m, 3H), 3.36 (m, 2H), 3.10 (m, 2H), 2.64 (m, 2H), 1.85 - 1.75 (m, 2H), 1.53 (m, 6H), 1.38 (s, 9H), 1.29 (m, 2H). LCMS: $[M+H]^+$ = 345

**Step 4: UBI-1159g**

Compound **UBI-1159f** (190 mg, 0.55 mmol), **B(B1)** (237 mg, 0.55 mmol), HATU (250 mg, 0.66 mmol), and DIPEA (1 mL) were dissolved in DMF (5 mL), reacted at room temperature for 3 hours. The reaction was poured into water (30 ml) and then extracted with ethyl acetate (40 mL*3). The combined organic phases were with 1M HCl, saturated $NaHCO_3$. The organic layers were combined, dried over anhydrous sodium sulfate and concentrated. The crude product was isolated by silica gel column chromatography (dichloromethane /methanol = 10/1, Rf = 0.3) to obtain **UBI-1159g** (180 mg, yield 43%) as white solid. $^1$H NMR (400 MHz, chloroform-*d*) $\delta$ 8.68 (s, 1H), 7.36 (s, 4H), 5.35 (t, 1H), 4.73 (s, 1H), 4.67 - 4.58 (m, 1H), 4.53 (s, 1H), 4.45 (d, 1H), 4.00 (s, 1H), 3.65 (m, 5H), 3.03 (m, 3H), 2.52 (m, 2H), 2.30 (s, 3H), 2.27 - 2.20 (m, 1H), 2.09 (m, 1H), 2.01 (m, 5H), 1.86 (m, 3H), 1.59 (m, 3H), 1.41 (m, 5H), 1.25 (s, 9H), 1.01 (s, 9H), 0.97 - 0.84 (m, 5H). LCMS: [M+H] $^+$ = 757

**Step 5: UBI-1159**

Compound **UBI-1159g** (50 mg, 0.066 mmol) was added to 4M hydrochloride in dioxane (1 mL) under ice bath, and reacted at room temperature for 2 hours. The reaction solution was concentrated to obtain crude product **UBI-1159h** (50 mg, 100% yield) as yellow oil. LCMS: [M+H]$^+$ = 657

**Synthesis method of Compound UBI-1124 ($NH_2$-12e-B(A1)):**

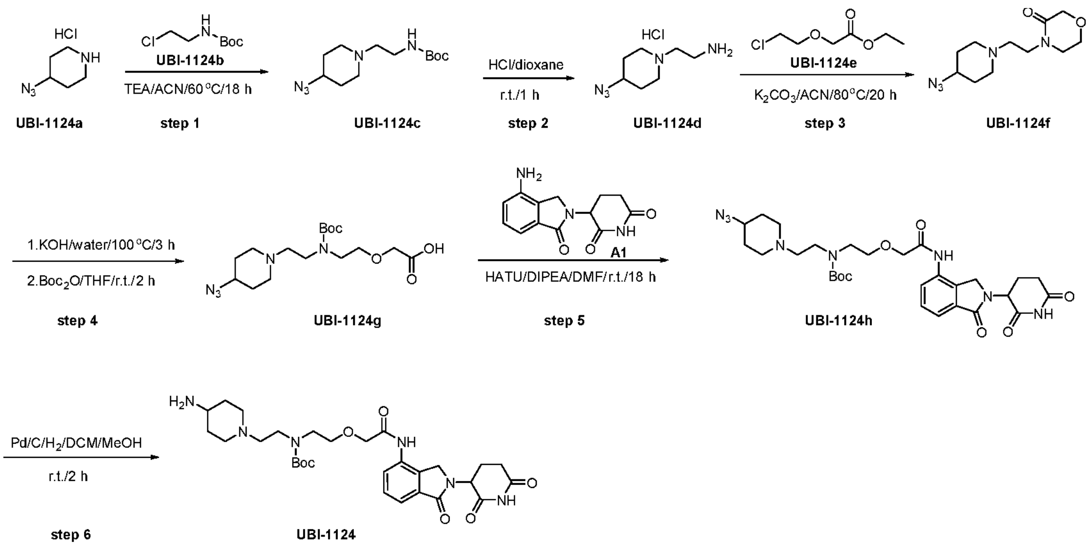


**Step 1: UBI-1124c**

Compound **UBI-1124a** (5.6 g, 34.4 mmol), **UBI-1124b** (8.8 g, 49 mmol), and triethylamine (11 mL, 78 mmol) were dissolved in acetonitrile (100 mL), reacted at 60°C overnight. The reaction was cooled to room temperature, poured into water and then extracted with ethyl acetate (50 mL*3). The organic layers were combined, dried over anhydrous sodium sulfate and concentrated. The crude product was isolated by silica gel column chromatography (petroleum ether/ethyl acetate = 70/30, Rf = 0.3) to obtain product **UBI-1143e** (3.1 g, yield 33%) as yellow oil.

**Step 2: UBI-1124d**

Compound **UBI-1124c** (3.1 g, 11.5 mmol) was added to a solution of 4M hydrochloric acid in dioxane (35 mL) under ice bath, and reacted at room temperature for 2 hours. The reaction solution was concentrated to obtain product **UBI-1124d** (2.36 g, yield 100%) as yellow oil.

**Step 3: UBI-1124f**

Compound **UBI-1124d** (1.5g, 8.8 mmol) was dissolved in acetonitrile (150 mL), added anhydrous potassium

carbonate (3.64 g, 26.4 mmol) and **UBI-1124e** (1.7 g, 10.2 mmol), reacted at 80 □overnight. The reaction was cooled to room temperature and filtered. The filtrate was concentrated and isolated by silica gel column chromatography (dichloromethane /methanol = 97/3, Rf = 0.2) to obtain product **UBI-1124f** (260 mg, yield 11%) as a yellow oil. LCMS: $[M+H]^+$ = 254

**Step 4: UBI-1124g**

Compound **UBI-1124f** (800 mg, 3.15 mmol) was dissolved in water (5 mL), added potassium hydroxide (530 mg, 9.47 mmol), and reacted at 100°C for 3 hours. After completion of reaction, the reaction was cooled to room temperature, added $Boc_2O$ (1.37 g, 6.3 mmol) and tetrahydrofuran (5 mL), and reacted at room temperature for 2 hours. The reaction added saturated brine (15 mL), extracted with ether twice (20 mL*2). The aqueous phase was acidified to pH=5 using 1M HCl, and then extracted with a mixed solvent of ethyl acetate and acetone (40 mL*3). The organic phases were combined, dried over anhydrous sodium sulfate, filtered and concentrated to obtain product **UBI-1124g** (1.1 g, yield 100%) as colorless oil. $^1$H NMR (400 MHz, DMSO-$d_6$) $\delta$ 12.58 (s, 1H), 3.61 - 3.52 (m, 6H), 3.35 (m, 4H), 3.24 (m, 2H), 3.06 (m, 2H), 2.11 (m, 2H), 1.77 (m, 2H), 1.41 (d, *J* = 5.0 Hz, 9H). LCMS: $[MS+H]^+$=372.2

**Step 5: UBI-1124h**

Compound **UBI-1124g** (500 mg, 1.34 mmol), **B(A1)** (347 mg, 1.34 mmol), HATU (763 mg, 2 mmol), and DIPEA (1 mL) were dissolved in DMF (5 mL), reacted at room temperature overnight. The reaction was quenched with water (50 mL) and extracted with ethyl acetate (50 mL*3). The organic layers were combined, dried over anhydrous sodium sulfate and concentrated. The crude product was isolated by silica gel column chromatography (dichloromethane/methanol = 93/7) to obtain product **UBI-1124h** (370 mg, yield 45%) as yellow solid. $^1$H NMR (400 MHz, DMSO-$d_6$) $\delta$ 11.03 (s, 1H), 9.85 (m, 1H), 7.78 (m, 1H), 7.62-7.45 (m, 2H), 5.16 (m, 1H), 4.45-4.30 (m, 2H), 4.16 (m, 3H), 3.69-3.56 (m, 4H), 3.41 (m, 2H), 3.30 (m, 2H), 3.15 (m, 2H), 3.04-2.84 (m, 2H), 2.69 - 2.55 (m, 2H), 2.33 (m, 2H), 2.16 (m, 1H), 2.08 - 1.89 (m, 3H), 1.39 (s, 9H). LCMS: $[M+H]^+$ = 613

**Step 6: UBI-1124**

Compound **UBI-1124h** (370 mg, 0.6 mmol) was dissolved in a mixed solvent of dichloromethane (10 mL) and methanol (2 mL), added Pd/C (300 mg), and reacted for 2 hours under hydrogen atmosphere. Filtered through Celite, the filtrate was concentrated to obtain product **UBI-1124** (370 mg, yield 100%) as yellow solid, which was directly used in the next step. LCMS: $[M+H]^+$ = 587.1

**Synthesis method of Compound UBI-1144 ($NH_2$-12e-B(A1)):**

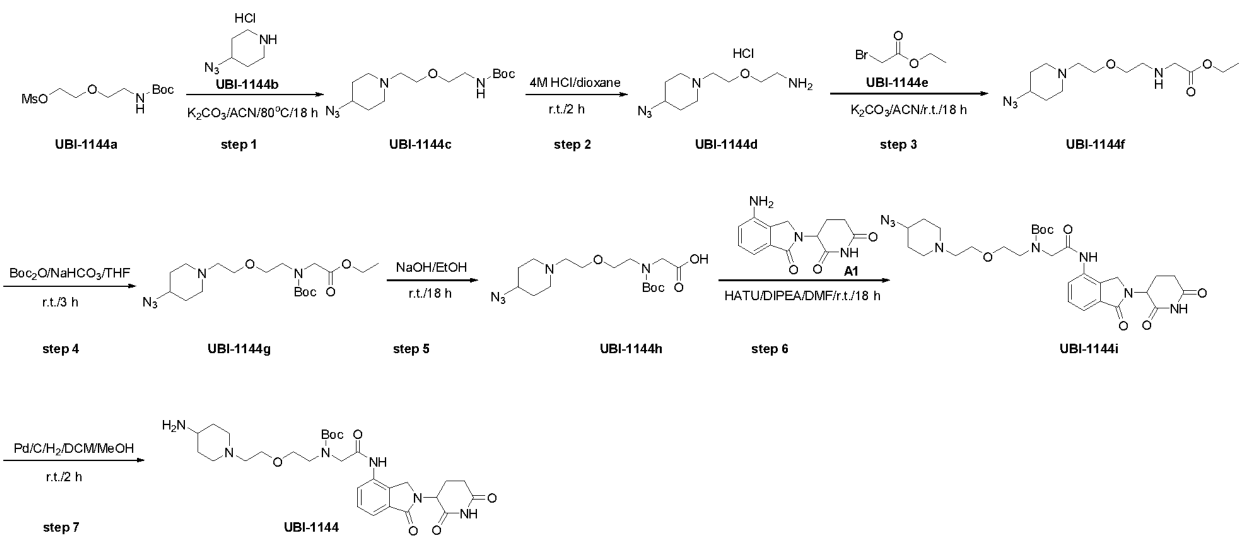


**Step 1: UBI-1144c**

Compound **UBI-1144a** (1.5 g, 6.8 mmol) was dissolved in acetonitrile (30 mL), added anhydrous potassium

carbonate (2.82 g, 20.4 mmol) and **UBI-1144b** (1.14 g, 6.8 mmol), reacted at 80 □ overnight. The reaction was cooled to room temperature and filtered. The filtrate was concentrated and isolated by silica gel column chromatography (dichloromethane /methanol = 95/5) to obtain product **UBI-1144c** (1.1 g, yield 51%) as a yellow oil. $^1H$ NMR (400 MHz, chloroform-*d*) δ 5.22 (s, 1H), 3.54 (m, 4H), 3.42 (m, 1H), 3.30 (m, 2H), 2.81 (m, 2H), 2.57 (m, 2H), 2.23 (m, 2H), 1.91 (m, 2H), 1.71 (m, 2H), 1.45 (s, 9H). LCMS: $[M+H]^+$ = 314

**Step 2: UBI-1144d**

Compound **UBI-1144c** (1.1 g, 3.5 mmol) was added to a solution of 4M hydrochloric acid in dioxane (5 mL) under ice bath, and reacted at room temperature for 2 hours. The reaction solution was concentrated to obtain product **UBI-1144d** (874 mg, yield 100%) as yellow oil, which was directly used in the next step.

**Step 3: UBI-1144f**

Compound **UBI-1144d** (1.5 g, 6.8 mmol) was dissolved in acetonitrile (30 mL), added anhydrous potassium carbonate (2.82 g, 20.4 mmol) and **UBI-1144e** (1.14 g, 6.8 mmol), reacted at RT overnight. The reaction was filtered. The filtrate was concentrated and isolated by silica gel column chromatography (dichloromethane /methanol = 95/5) to obtain product **UBI-1144f** (670 mg, yield 33%) as a yellow oil. LCMS: $[M+H]^+$ = 300

**Step 4: UBI-1144g**

Compound **UBI-1144c** (670 mg, 2.2 mmol) was dissolved in tetrahydrofuran (10 mL) and added sodium bicarbonate (554 mg, 6.6 mmol) and $Boc_2O$ (590 mg, 2.7 mmol). Reacted at room temperature for 3 hours, the reaction was quenched with water (20 mL), extracted with ethyl acetate (20 mL*3). The organic layers were combined, dried over anhydrous sodium sulfate and concentrated. The crude product was isolated by silica gel column chromatography (dichloromethane/methanol = 90/10) to obtain product **UBI-1144g** (800 mg, yield 91%) as yellow oil. LCMS: $[M+H]^+$ = 400

**Step 5: UBI-1144h**

Compound **UBI-1144g** (800 mg, 2 mmol) was dissolved in ethanol(10 mL), added 2M NaOH (4 mL, 8 mmol), and reacted at room temperature overnight. The reaction solution was concentrated, and added saturated brine (15 mL) and extracted with ether (20 mL*2). The aqueous phase was acidified to pH=6 using 1M HCl, and then extracted with ethyl acetate (40 mL*3). The organic phases were combined, dried over anhydrous sodium sulfate, filtered and concentrated to obtain crude product **UBI-1144h** (650 mg, yield 87%) as colorless oil. $^1H$ NMR (400 MHz, DMSO-$d_6$) δ 11.62 (s, 1H), 3.87 (m, 3H), 3.71 - 3.64 (m, 2H), 3.50 (m, 2H), 3.36 (m, 2H), 3.17 (m, 2H), 3.04 (m, 2H), 2.89 (m, 2H), 2.01 (m, 2H), 1.74 (m, 2H), 1.37 (d, J = 19.6 Hz, 9H). LCMS: $[MS+H]^+$=372.2

**Step 6: UBI-1144i**

Compound **UBI-1144h** (300 mg, 0.8 mmol), **B(A1)** (251 mg, 0.96 mmol), HATU (456 mg, 1.2 mmol), and DIPEA (1 mL) were dissolved in DMF (5 mL), reacted at room temperature overnight. The reaction was quenched with water (50 mL) and extracted with ethyl acetate (50 mL*3). The organic layers were combined, dried over anhydrous sodium sulfate and concentrated. The crude product was isolated by silica gel column chromatography (dichloromethane/methanol = 93/7) to obtain product **UBI-1144i** (250 mg, yield 51%) as yellow solid. $^1H$ NMR (400 MHz, DMSO-$d_6$) δ 11.04 (s, 1H), 9.88 (s, 1H), 9.26 (s, 1H), 7.88-7.72 (m, 1H), 7.59-7.46 (m, 2H), 5.23-5.13 (m, 1H), 4.43-4.27 (m, 2H), 4.08-3.98 (m, 3H), 3.80-3.39 (m, 8H), 3.15-2.88 (m, 3H), 2.70-2.58 (m, 2H), 2.35-2.18 (m, 2H), 2.08 (m, 2H), 1.90 (m, 1H), 1.68 (m, 1H), 1.35 (s, 9H). LCMS: $[M+H]^+$ = 613

**Step 7: UBI-1144**

Compound **UBI-1144i** (250 mg, 0.4 mmol) was dissolved in a mixed solvent of dichloromethane (10 mL) and methanol (2 mL), added Pd/C (150 mg), and reacted for 2 hours under hydrogen atmosphere. Filtered through Celite, the filtrate was concentrated to obtain product **UBI-1144** (210 mg, yield 89%) as yellow solid, which was directly used in the next step. LCMS: $[M+H]^+$ = 587.1

**Synthesis method of Compound UBI-1113 ($NH_2$-11e-B(A1))**

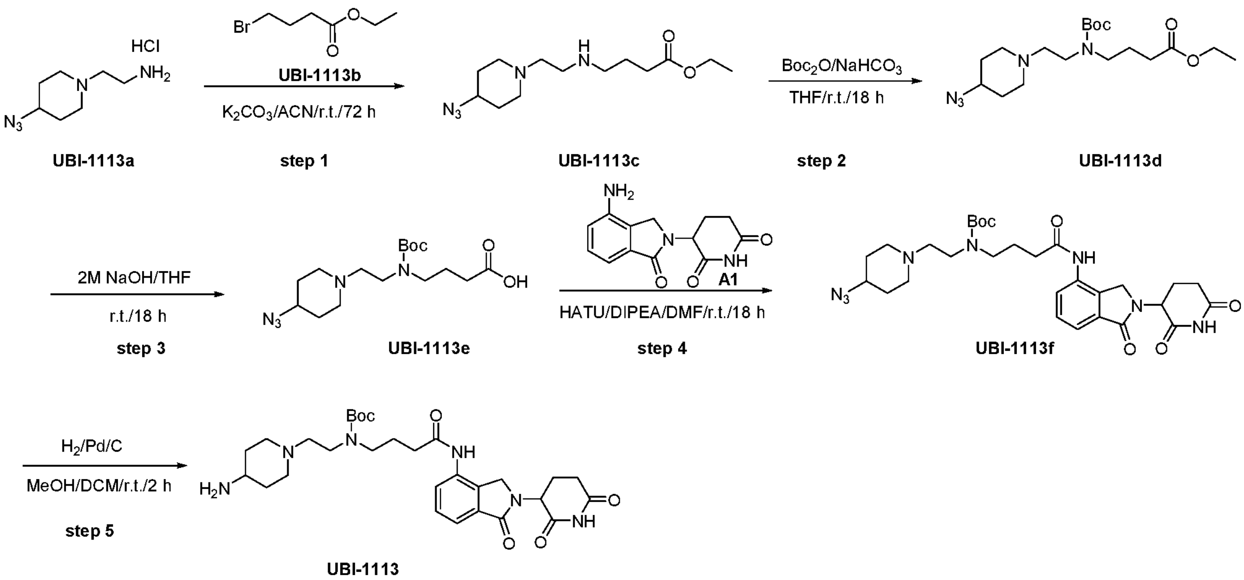


### Step 1: UBI-1113c

**Compound UBI-1113a** (1 g, 3.7 mmol) was dissolved in acetonitrile (30 mL), anhydrous potassium carbonate (1.5 g, 11.1 mmol) and **UBI-1113b** (770 mg, 3.7 mmol) were added, and reacted at room temperature for 72 hours. The reaction was filtered at room temperature, the filtrate was concentrated and isolated by silica gel column chromatography (dichloromethane/methanol = 95/5) to obtainproduct **UBI-1113c** (600 mg, yield 57%) as yellow oil. LCMS: $[M+H]^+$ = 284

### Step2: UBI-1113d

Com pound **UBI-1113c** (600 mg, 2.1 mmol) was dissolved in tetrahydrofuran (10 mL), added sodium bicarbonate (530 mg, 6.3 mmol) and $Boc_2O$ (545 mg, 2.5 mmol), and reacted at room temperature overnight. The reaction was quenched with water (20 mL), extracted with ethyl acetate (20 mL*3). The organic layers were combined, dried over anhydrous sodium sulfate and concentrated. The crude product was isolated by silica gel column chromatography (dichloromethane/methanol = 90/10) to obtain product **UBI-1113d** (600 mg, yield 74%) as yellow oil. LCMS: $[M+H]^+$= 384

### Step 3: UBI-1113e

Compound **UBI-1113d** (400 mg, 1 mmol) was dissolved in tetrahydrofuran (10 mL), added 2M NaOH (5 mL, 10 mmol), and reacted at room temperature overnight. The reaction solution was concentrated, added saturated brine (15 mL), extracted with ether twice (20 mL*2). The aqueous phase was acidified to pH=6 using 1M HCl, then extracted with ethyl acetate (40 mL*3). The organic phases were combined, dried over anhydrous sodium sulfate, filtered and concentrated to obtain crude product **UBI-1113e** (530 mg, yield 100%) as colorless oil, which was directly used in the next step. $^1H$ NMR (400 MHz, DMSO-$d_6$) $\delta$ 12.00 (s, 1H), 3.54 (m, 2H), 3.17 (m, 3H), 2.49 - 2.41 (m, 2H), 2.18 (m, 2H), 1.91 (m, 5H), 1.76 - 1.63 (m, 3H), 1.40 (s, 9H). LCMS: $[M+H]^+$=356.2

### Step 4: UBI-1113f

Compound **UBI-1113e** (530 mg, 1.5 mmol), **B(A1)** (460 mg, 1.8 mmol), HATU (855 mg, 2.2 mmol), and DIPEA (1.2 mL) were dissolved in DMF (5 mL), reacted at room temperature overnight. The reaction was quenched with water (50 mL), extracted with ethyl acetate (50 mL*3). The organic layers were combined, dried over anhydrous sodium sulfate and concentrated. The crude product was isolated by silica gel column chromatography (dichloromethane/methanol = 93/7) to obtain product **UBI-1113f** (350 mg, yield 39%) as white solid. LCMS: $[M+H]^+$= 597

### Step 5: UBI-1113

Compound **UBI-1113f** (350 mg, 0.58 mmol) was dissolved in a mixed solvent of dichloromethane (10 mL) and methanol (2 mL), added Pd/C (250 mg), and reacted for 2 hours under hydrogen atmosphere. Filtered through Celite, the filtrate was concentrated to obtain product **UBI-1113** (300 mg, yield 91%) as white solid, which was directly used in

the next step. LCMS: [M+H] $^+$= 571

**Synthesis method of Compound Linker f - Ligand B(A1)**

**Synthesis method of Compound UBI-1157 ($NH_2$-11f-B(A1))**

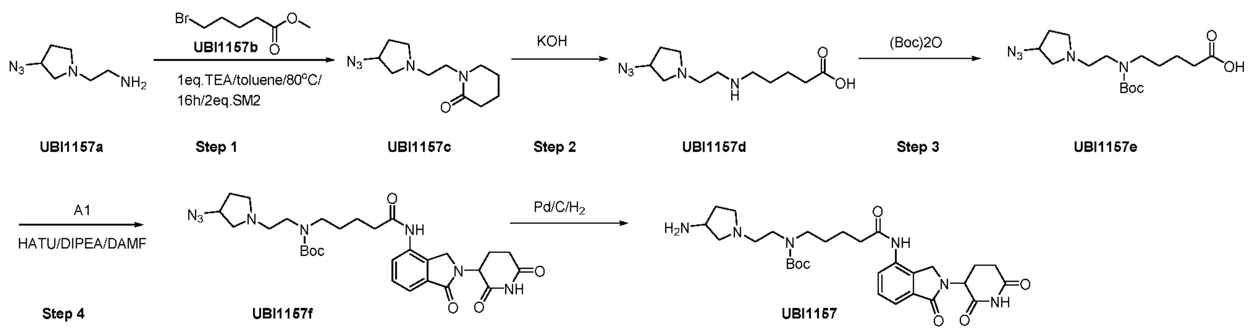


**Step 1: UBI-1157c**

Compound **UBI-1157a** (700mg, 4.48mmol), and **UBI-1157b** (1.74 g, 8.98mmol) were dissolved in triethylamine (452mg, 4.48mmol) and toluene (10mL), reacted at 80°C for 18 hours. The reaction solution was concentrated and isolated by silica gel column chromatography (petroleum ether/ethyl acetate = 50% to 100%, then MeOH/DCM=0% to 10%) to obtain the target compound **UBI-1158c** (1g) as colorless oil. LC-MS: [M+H] $^+$ =238.5

**Step 2: UBI-1157d**

**Compound UBI-11157c** (600mg, 2.5mmol), KOH (708mg, 12.5mmol) were dissolved in water (3mL), and refluxed at 100°C for 1 hours. After cooling, the reaction solution was extracted with ethyl acetate (10 mL * 3), and the aqueous phase was directly used in the next step. LC-MS: [M+H] $^+$ =257.5

**Step 3: UBI-1157e**

To the aqueous solution of compound UBI-1157d (600mg, 2.35mmol), di-tert-butyl dicarbonate (1.01g, 4.7mmol) and dioxane (20mL) were added, and reacted at room temperature for 2 hours. The reaction solution was concentrated, added water (20 mL), and extrated with ethyl acetate (10 mL*3). The aqueous phase was concentrated to obtain the crude product, which was dissolved in MeOH/DCM=10/1 (30 mL), filtered, and the filtrate was concentrated to obtain the target compound **UBI-1157e** (600mg). LC-MS: [M+H] $^+$ =357.2

**Step 4: UBI-1157f**

Compound **UBI-1157e** (600mg, 1.69mmol), **B(A1)** (437mg, 1.69mmol), HATU (1.28g, 3.38mmol) were dissolved in DMF (3 mL) and DIPEA (0.3 mL), and reacted at room temperature for 16 hours. The reaction solution was directly isolated by reversed-phase column (MeOH/$H_2O$ = 5% to 95%, 45 minutes, collected at 40%) to obtain target compound **UBI-11157f** (400mg) as white solid. LC-MS: [M+H] $^+$ =597.5

**Step 5: UBI-1157**

Compound **UBI-1157f** (100mg, 0.17mmol) and Pd/C (20mg) were dissolved in MeOH (1 mL) and DCM (10mL), and reacted for 16 hours under hydrogen atmosphere. The reaction was filtered after completion, and the filtrate was concentrated to obtain crude product. The crude product was washed with anhydrous ether (3mL*3), and filtered to obtain target compound **UBI-1157** (50mg) as a white solid. LCMS: (M+H) $^+$= 543.3

**Synthesis method of Compound UBI-1139 ($NH_2$-11f-B(A1))**

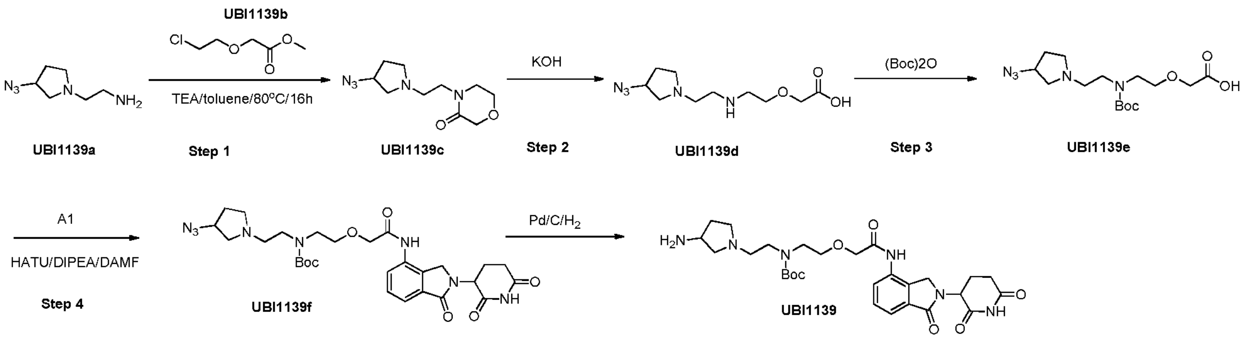


**Step 1: UBI-1139c**

Compound **UBI-1157a** (1.5 g, 10mmol), **UBI-1139b** (4.4 g, 29mmol) and TEA (980mg, 10mmol) were dissolved in toluene (25mL) and reacted at 80°C for 18 hours. The reaction solution was concentrated and subjected to silica gel column chromatography (petroleum ether/ethyl acetate = 50% to 100%, 20 minutes, methanol/dichloromethane = 0% to 10%, 30 minutes) to obtain crude product **UBI-1139c** (500mg, yield 22%) as light yellow oil.
$^1$H NMR (400 MHz, chloroform-d) δ 4.03 (s, 2H), 3.92 (s, 1H), 3.79 (dd, *J* = 5.8, 4.2 Hz, 2H), 3.68 (dd, *J* = 6.2, 4.5 Hz, 2H), 3.43 (d, *J* = 6.4 Hz, 2H), 2.87 (s, 2H), 2.76 (d, *J* = 9.7 Hz, 2H), 2.65 (s, 2H), 2.45 (s, 1H), 2.32 - 2.15 (m, 1H), 1.92 (s, 1H).

**Step 2: UBI-1139d**

Compound **UBI-1139c** (200mg, 0.8mmol), KOH (141mg, 2.5mmol) was dissolved in water (2mL), and the reaction was reacted at 100°C for 3 hours. The reaction liquid was directly used in the next step.

**Step 3: UBI-1139e**

Compound **UBI-1139d** (200mg, 0.8mmol) and $(Boc)_2O$ (340mg, 1.6mmol) were dissolved in dioxane (3mL) and water (2 mL), and reacted at room temperature for 2 hours. The reaction solution was concentrated and washed with ethyl acetate(10mL*3). The aqueous phase was acidified to pH=6 using (1M) HCl, and lyophilized to obtain crude product. The crude product was soaked in methanol/dichloromethane=1/10 (30mL) and filtered. The mother solution was dried by rotary evaporation to obtain crude product **UBI-1139e** (300mg, yield 100%) as colorless oil.

**Step 4: UBI-1139f**

Compound **UBI-1139e** (300mg, 0.84mmol), **B(A1)** (196mg, 0.76mmol), and HATU (639mg, 1.68mmol) were dissolved in DMF (10 mL) and DIPEA (0.3 mL), and reacted at room temperature for 16 hours. The reaction solution was directly purified via reversed-phase column (methanol/water = 5 % to 95 %, 45 minutes, collected at 40%) to obtain product **UBI-1139f** (100mg, yield 20%) as light yellow oil.

**Step 5: UBI-1039**

Under hydrogen atmosphere, compound **UBI-1139f** (50mg, 0.08 mmol), Pd/C (5 mg) were dissolved in methanol (0.5 mL) and dichloromethane (5mL), and reacted at room temperature for 1 hour. The reaction solution was filtered on Celite. The filtrate was concentrated to obtain crude product **UBI-1139** (50mg, yield 100%) as yellow oil.

**Synthesis method of Compound UB-1163 ($NH_2$-11f-B(A1))**

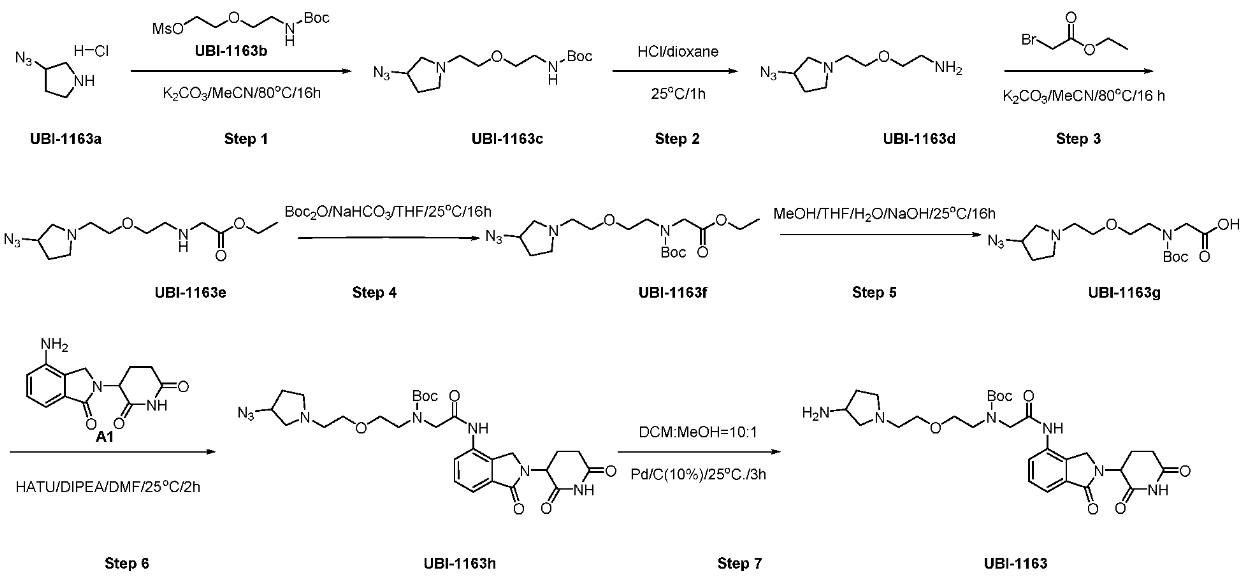


UBI-1163a Step 1 UBI-1163c Step 2 UBI-1163d Step 3

UBI-1163e Step 4 UBI-1163f Step 5 UBI-1163g

Step 6 UBI-1163h Step 7 UBI-1163

### Step 1: UBI-1163c

Compound **UBI-1163a** (1.40 g, 8.92 mmol), **BUI-1163b** (3.032 g, 10.70 mmol) and $K_2CO_3$ (2.456 g, 17.83 mmol) were dissolved in $CH_3CN$ (40 mL), then heated to 80°C and reacted for 16 hours. After completion of reaction, the reaction was concentrated under reduced pressure, added water (50 mL), and then extracted with ethyl acetate (50 mL*3). The organic phases were combined, washed with water (50 mL*3), saturated brine (50 mL*3) successively, then dried over anhydrous sodium sulfate, and concentrated under reduced pressure to obtain crude product. The crude product was isolated by silica gel column chromatography (methanol/ dichloromethane = 0% to 10%) to obtain target compound **UBI-1163c** (1.32g, yield 47%) as yellow solid. $^1$H NMR (400 MHz, chloroform-*d*) δ 5.29 - 4.94 (m, 1H), 4.15 - 3.89 (m, 1H), 3.58 (t, *J* = 5.6 Hz, 2H), 3.52 (t, *J* = 5.2 Hz, 2H), 3.36 - 3.25 (m, 2H), 2.93 - 2.79 (m, 1H), 2.79 - 2.67 (m, 2H), 2.67 - 2.56 (m, 2H), 2.30 - 2.13 (m, 1H), 1.96 - 1.79 (m, 1H), 1.45 (s, 9H).

### Step 2: UBI-1163d

Compound **UBI-1163c** (1.300g, 4.342mmol) was dissolved in a solution of HCl in dioxane (40 mL), reacted at 25 °C for 1 hour. The reaction solution was concentrated to obtain product **UBI-1163d** (1.05g, yield 100%) as yellow oil. The crude product was directly used in the next step.

### Step 3: UBI-1163e

Compound **UBI-1163d** (1.05 g, 4.34 mmol), ethyl 2-bromoacetate (1.09 g, 6.51 mmol) and $K_2CO_3$ (1.8 g, 13.03 mmol) were dissolved in $CH_3CN$ (50 mL) and heated to 80°C and reacted for 16 hours. After completion of reaction, the reaction was concentrated, added water (50 mL), and then extracted with ethyl acetate (50 mL*3). The organic phases were combined, washed with water (50 mL*3), saturated brine (50 mL*3) successively, then dried over anhydrous sodium sulfate, and concentrated under reduced pressure to obtain crude product. The crude product was isolated by silica gel column chromatography (methanol/ dichloromethane = 0% to 10%) to obtain target compound **UBI-1163e** (810 mg, yield 62%) as yellow oil. $^1$H NMR (400 MHz, chloroform-d) δ 4.19 (q, *J* = 7.2 Hz, 2H), 4.10 = 3.96 (m, 1H), 3.67 - 3.52 (m, 4H), 3.51 - 3.41 (m, 2H), 3.01 - 2.54 (m, 8H), 2.29 - 2.14 (m, 2H), 1.95 - 1.81 (m, 1H), 1.28 (t, *J* = 7.2 Hz, 3H).

### Step 4: UBI-1163f

Compound **UBI-1163e** (790mg, 2.22mmol), di-*tert*-butyl dicarbonate (1.208g, 5.537mmol) and $NaHCO_3$ (465mg, 5.54mmol) were dissolved in THF (30mL) and reacted at 25 °C for 1 hours. After the completion of reaction, it was concentrated, added water (50mL), then extracted with ethyl acetate (50 mL * 3). The organic phases were combined, then washed with saturated brine, dried over anhydrous $Na_2SO_4$, concentrated by rotary evaporation under reduced pressure to obtain crude product. The crude product was isolated by silica gel column chromatography (ethyl acetate/petroleum ether = 0% to 20%) to obtain target product **UBI-1163f** (900mg, yield 83%) as colorless transparent oil. $^1$H NMR (400 MHz, chloroform-*d*) δ 4.25 - 4.13 (m, 2H), 4.10 - 3.91 (m, 3H), 3.64 - 3.40 (m, 6H), 2.97 - 2.41 (m, 6H), 2.27 - 2.12

(m, 1H), 1.95 - 1.79 (m, 1H), 1.47 (s, 4H), 1.42 (s, 5H), 1.28 (t, *J*= 7.2 Hz, 3H).

**Step 5: UBI-1163g**

Compound **UBI-11163f** (900mg, 2.34mmol), NaOH (280mg, 7.00mmol) were dissolved in water (3mL), THF(9mL) and MeOH(3mL), and reacted at 25°C for 16 hours. After completion of reaction, the reaction was concentrated, added water (20 mL), and then extracted with ethyl acetate (50 mL*3). The aqueous phase was acidified to pH=5 using diluted hydrochloric acid (1M), and then extracted with dichloromethane (50 mL*3). The organic phases were combined, then washed with water (50mL*3), saturated brine (50mL*3) successively, further dried over anhydrous $Na_2SO_4$, and concentrated by rotary evaporation under reduced pressure to obtain colorless transparent oily liquid, which was the target compound **UBI-1163g** (490mg, yield 59%). $^1$H NMR (400 MHz, DMSO-$d_6$) δ 4.09 - 3.97 (m, 1H), 3.85 (d, *J* = 8.8 Hz, 2H), 3.50 - 3.42 (m, 4H), 3.36 - 3.31 (m, 2H), 2.80 - 2.69 (m, 1H), 2.69 - 2.61 (m, 2H), 2.58 - 2.54 (m, 2H), 2.37 - 2.31 (m, 1H), 2.19 - 2.07 (m, 1H), 1.78 - 1.62 (m, 1H), 1.49 - 1.25 (m, 9H).

**Step 6: UBI-1163h**

Compound **UBI-1098e** (455mg, 1.27mmol), **B(A1)** (330mg, 1.27mmol), HATU (968mg, 2.55mmol), DIPEA (823mg, 6.36mmol) were dissolved in DMF (4 mL) and reacted at 25 °C for 2 hours. After completion of reaction, the reaction was added water (30 mL), and then extracted with ethyl acetate (50 mL*3). The organic phases were combined, washed with water (50 mL*3), saturated brine (50 mL*3) successively, then dried over anhydrous sodium sulfate, and concentrated by rotary evaporation under reduced pressure to obtain crude product. Then the crude product was isolated by silica gel column chromatography (methanol/ dichloromethane = 0% to 20%) to obtain yellow solid, which was the target compound **UBI-1163h** (384mg, yield 50%). $^1$H NMR (400 MHz, DMSO-$d_6$) δ 11.04 (s, 1H), 9.87 (s, 1H), 7.80 (s, 1H), 7.62 - 7.44 (m, 2H), 5.27 - 5.09 (m, 1H), 4.45 - 4.26 (m, 2H), 4.05 (d, *J*= 11.6 Hz, 2H), 3.71 - 3.52 (m, 4H), 3.44 (d, *J* = 5.4 Hz, 2H), 3.32 (s, 7H), 3.17 (d, *J* = 3.6 Hz, 1H), 2.99 - 2.89 (m, 1H), 2.66 - 2.58 (m, 1H), 2.27 (d, *J* = 13.2 Hz, 1H), 2.09 - 2.00 (m, 1H), 1.91 (s, 1H), 1.38 (d, *J* = 26.4 Hz, 9H). LCMS [M+H]+: 599.1.

**Step 7: UBI-1163**

Compound **UBI-1163h** (173mg, 0.290mmol) and Pd/C (156mg) were dissolved in MeOH (3 mL) and DCM (30mL), and reacted for 3 hours under hydrogen atmosphere. After completion of reaction, the reaction was filtered and washed with methanol (4 mL) and dichloromethane (40 mL). The filtrate was concentrated by rotary evaporation under reduced pressure to obtain the target compound **UBI-11163** (140 mg, yield 82%) as yellow solid. $^1$H NMR (400 MHz, DMSO-$d_6$) δ 11.03 (s, 1H), 9.94 (s, 1H), 8.34 (s, 2H), 7.89 - 7.74 (m, 1H), 7.65 - 7.40 (m, 2H), 5.26 - 5.08 (m, 1H), 4.51 - 4.24 (m, 2H), 4.14 - 4.03 (m, 2H), 3.98 - 3.80 (m, 1H), 3.80 - 3.50 (m, 5H), 3.50 - 3.36 (m, 4H), 3.17 (s, 2H), 2.99 - 2.89 (m, 1H), 2.67 - 2.56 (m, 3H), 2.49 - 2.43 (m, 2H), 2.31 - 2.22 (m, 1H), 2.09 - 2.02 (m, 1H), 1.38 (d, *J* = 27.6 Hz, 9H). LCMS $[M+H]^+$: 573.1.

**Synthesis method of Compound Linker g-Ligand B(A1)**

**Synthesis method of Compound UBI-1216 ($NH_2$-9g-B(A1))**

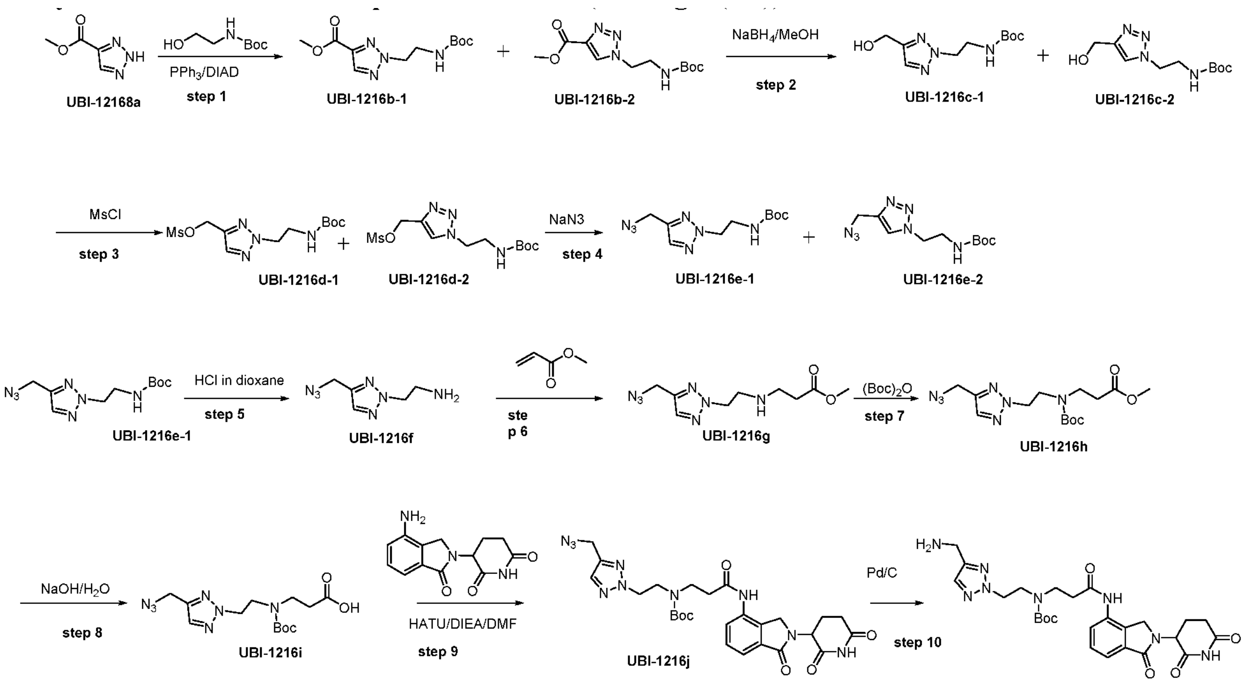


### Step 1: UBI-1216b

Methyl -1H-1,2,4-triazole-3-carboxylate (200 mg, 1.57 mmol) and N-Boc-ethanolamine (304 mg, 1.89 mmol) were added to THF (10 ml). Triphenylphosphine (495 mg, 1.89 mmol) and diisopropyl azodicarboxylate (381 mg, 1.89 mmol) e were added to the above solution, and stirred overnight at room temperature. The reaction solution was purified by a reversed-phase column (methanol/$H_2O$ = 5% to 95%, 45mins) and was collected at 60% to obtain white solid (230 mg, yield 54%). LC-MS: $(M+H)^+$=271.1

### Step 2: UBI-1216c

At 0°C, to a solution of UBI-1216b (1 g, 33.3 mmol) in THF (10 ml) was added lithium aluminum hydride (1.9 g, 50 mmol) for 30 min. The reaction was stirred for 3 hours, added water (1 ml) and 10% NaOH solution (2 ml), filtered, and then the filtrate was dried overh $Na_2SO_4$, concentrated under reduced pressure to obtain the product (8.01 g, yield 51%). LC-MS: $(M+H)^+$=243.1

### Step 3: UBI-1216d

To a solution of UBI-1216c (1.08 g, 4 mmol) and triethylamine (0.68 g, 7 mmol) in dichloromethane (10 ml) was added methanesulfonyl chloride (0.76 g, 7 mmol) dropwise, and stirred overnight at room temperature. After completion of reaction, to the mixture was added water (10ml) and extracted with dichloromethane (10ml*3). The organic layer was dried over $Na_2SO_4$ and concentrated to obtain white solid (1.15 g, crude product). LC-MS: $(M+H)^+$=321.1

### Step 4: UBI-1216e

UBI-1216d (1.7 g, 5 mmol) and $NaN_3$ (520 mg, 8 mmol) were mixed and dissolved in DMF (10 ml), stirred overnight at room temperature. After completion of reaction, the reaction was diluted with $H_2O$ (300 ml) and extracted with ether (2x150 ml). The organic phase was washed with $H_2O$ (3×100 ml) and brine (1×100 ml), dried over $MgSO_4$, filtered, and the solvent was removed under low pressure to obtain light yellow oil (1.1 g, yield 79%).
$^1H$ NMR (400 MHz, $CDCl_3$) δ 7.61 (d, *J* = 8.5 Hz, 1H), 4.94 (s, 1H),4.51 (dd, *J* = 17.7, 12.2 Hz, 2H), 4.43 (s, 2H), 3.70 (dd, *J* = 10.7, 5.6 Hz, 2H), 1.44 (s, 9H). LC-MS: $(M+H)^+$=268.3

### Step 5: UBI-1216f

**UBI-1216e** (500 mg, 1.416 mmol) was dissolved in a solution of hydrochloric acid/dioxane (4N) (10 ml), and stirred overnight at room temperature. After the completion of reaction, the solvent was removed and used directly in

the next step. (430mg, crude product). LC-MS: (M+H) $^+$=168.3

**Step 6: UBI-1216g**

Compound **UBI-1216f** (500 mg, 2.99 mmol) and methyl acrylate (257 mg, 2.99 mmol) were dissolved in acetonitrile (20 mL), and reacted at 80 ° C for 18 hours. After completion of reaction, the reaction solution was poured into 5 mL water and extracted with ethyl acetate (5 mL*3). The organic phases were combined, then washed with saturated brine, dried over anhydrous $Na_2SO_4$, concentrated under reduced pressure. Obtained crude product was isolated by silica gel column chromatography (dichloromethane/methanol = 10/1) to obtain compound as colorless oil (170 mg, yield 23%). LC-MS: (M+H) $^+$=254.1

**Step 7: UBI-1216h**

Compound **UBI-1216g** (650 mg, 2.57 mmol), di-tert-butyl dicarbonate (840 mg, 3.85 mmol) and sodium bicarbonate (318 mg, 3.79 mmol) were added to dioxane (30mL) successively and reacted at room temperature for 2 hours. After the completion of the reaction, the reaction solution was poured into 10 mL of water and extracted with ethyl acetate (10 mL*3). The organic phases were combined, then washed with saturated brine, dried over anhydrous $Na_2SO_4$, and concentrated under reduced pressure to obtain compound **UBI-1216h** (625 mg, yield 69%). LC-MS: (M+H) $^+$=354.1

**Step 8: UBI-1216i**

Compound **UBI-1216h** (500 mg, 1.416 mmol), aqueous solution of sodium hydroxide (4N, 10 mL) were sequentially added into water (50 mL), and reacted at 30 ° C for 12 hours. After the completion of reaction, it was concentrated and the aqueous phase was acidified to pH=5 using hydrochloric acid (1M). Then it was extracted with dichloromethane (10ml*3), the combined organic layers was dried over anhydrous $Na_2SO_4$, and concentrated to obtain compound **UBI-1216i** (1.2 g, yield 83%). LC-MS:(M+H) $^+$ =340.1

**Step 9: UBI-1216j**

**CompoundUBI-1216i** (20 mg, 0.059 mmol), **B(A1)** (15 mg, 0.059 mmol), HATU (33 mg, 0.088 mmol) and DIPEA (0.5mL) were added to anhydrous DMF (5 mL). The mixture was reacted at room temperature for 16 hours, concentrated after the completion of reaction. The crude product was isolated by silica gel column chromatography (dichloromethane/methanol = 10/1) to obtain compound **UBI-1216j** (16 mg, 49% yield) as white solid. LC-MS (M+H) $^+$=581.1

**Step 10: UBI-1216**

Compound **UBI-1216j** (100 mg, 0.17mmol), 10% palladium on carbon (20 mg) were added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain target compound UBI-1095 (83 mg, yield 88%) as white solid. LCMS: (M-100+H) $^+$= 455.3

**Synthesis method of Compound UB-180844e**

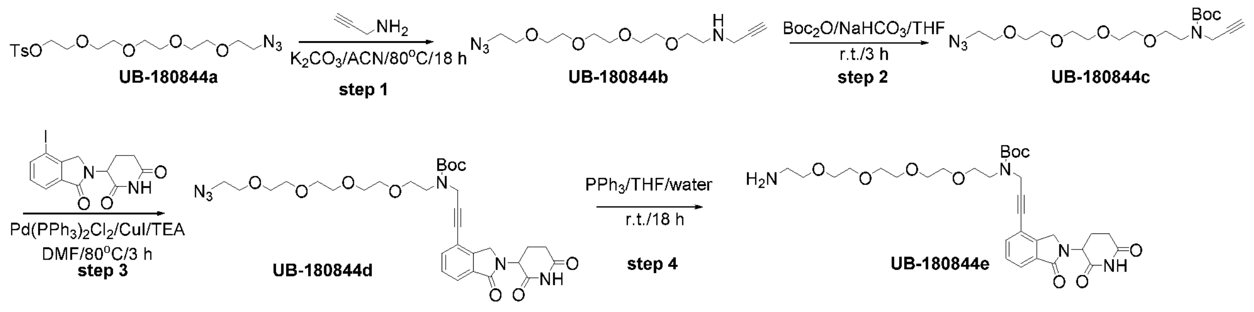

UB-180844a UB-180844b UB-180844c UB-180844d UB-180844e

**Step 1: UB-180844b**

Compound **UB-180844a** (600 mg, 1.4 mmol) was dissolved in acetonitrile (20 mL), added propargylamine (400 mg, 7.2 mmol), potassium carbonate (386 mg, 2.8 mmol). reacted at 80°C for 18 hours. The reaction solution was cooled, filtered, concentrated and was directly used in the next reaction. LCMS $[M+H]^+$ = 301

**Step 2: UB-180844c**

Compound **UB-180844b** (459 mg, 1.53 mmol) was dissolved in tetrahydrofuran (10 mL). Di-*tert*-butyl dicarbonate (1.5 g, 6.88 mmol) and sodium bicarbonate (257 mg, 3.06 mmol) were added. Reacted at room temperature for 3 hours. After completion of the reaction, the filtrate was concentrated to obtain the crude product, which was isolated by silica gel column chromatography (petroleum ether/ethyl acetate = 0% to 30%) to obtain target product **UB-180844c** (340 mg, yield 60%) as a yellow transparent oil. LCMS $[M+H]^+$ = 401

**Step 3: UB-180844d**

Compound **UB-180844c** (346 mg, 0.86 mmol), iodine-substituted lenalidomide (150 mg, 0.55 mmol), $Pd(PPh_3)_2Cl_2$ (35 mg, 0.05 mmol), CuI (21 mg, 0.11 mmol), triethylamine (55 mg, 0.55 mmol) were dissolved in anhydrous DMF (6 mL), reacted at 80°C for 3 hours under nitrogen protection. After completion of reaction, the filtrate was concentrated to obtain the crude product, which was isolated by silica gel column chromatography (ethyl acetate-dichloromethane/methanol=100% - 0% to 10%) to obtain target product **UB-180844d** (200 mg, yield 42%) as brown oil. LCMS $[M-100]^+$ = 543

$^1H$ NMR (400 MHz, chloroform-d) $\delta$ 7.84 (d, *J* = 7.6 Hz, 1H), 7.71 - 7.64 (m, 1H), 7.63 - 7.51 (m, 1H), 7.46 (t, *J* = 7.8 Hz, 1H), 5.24 (dd, *J* = 13.3, 5.2 Hz, 1H), 4.50 (d, *J* = 16.8 Hz, 1H), 4.37 (m, 2H), 3.70 - 3.60 (m, 14H), 3.55 (m, 2H), 3.42 - 3.36 (m, 2H), 2.91 (m, 1H), 2.30 - 2.20 (m, 1H), 2.03 (d, *J* = 14.4 Hz, 1H), 1.49 (s, 9H), 1.31 (m, 2H), 0.88 (t, *J* = 6.2 Hz, 2H).

**Step 4: UB-180844e**

Compound **UB-180844d** (240 mg, 0.37 mmol) was dissolved in tetrahydrofuran (20 mL) and added triphenylphosphine (262 mg, 1 mmol), reacted at room temperature for 3 hours. The reaction was added water (0.3 mL) and reacted at room temperature for 18 hours. After the completion of reaction, the crude product was obtained by concentration, and isolated by silica gel column chromatography (dichloromethane/methanol=0% to 10%) to obtain crude product **UB-180844e** (60 mg) as yellow oil. LCMS $[M+H]^+$ = 617

**Synthetic method of Compound UB-180847b ($N_3$-13b-A1)**

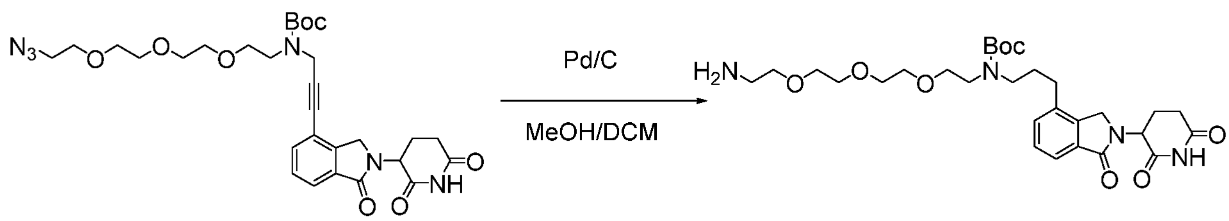


**UB-180847a** **step 1** **UB-180847b**

**Step 1: UB-180847b**

Compound **UB-180847a** (100 mg, 2.3 mmol) and Pd/C (10 mg) were dissolved in methanol (1 mL) and dichloromethane (10 mL), and then reacted at room temperature for 1 hour under hydrogen. After the reaction solution was filtered, the filtrate was concentrated to obtain crude product. The crude product was purified by a reversed-phase column (methanol/water=5% to 95%, collected at 20%) to obtain target product **UB180847b** (80mg, yield 84 %) as yellow oil.

**Synthesis method of Compound UBI-1235 ($NH_2$-16e-A1)**

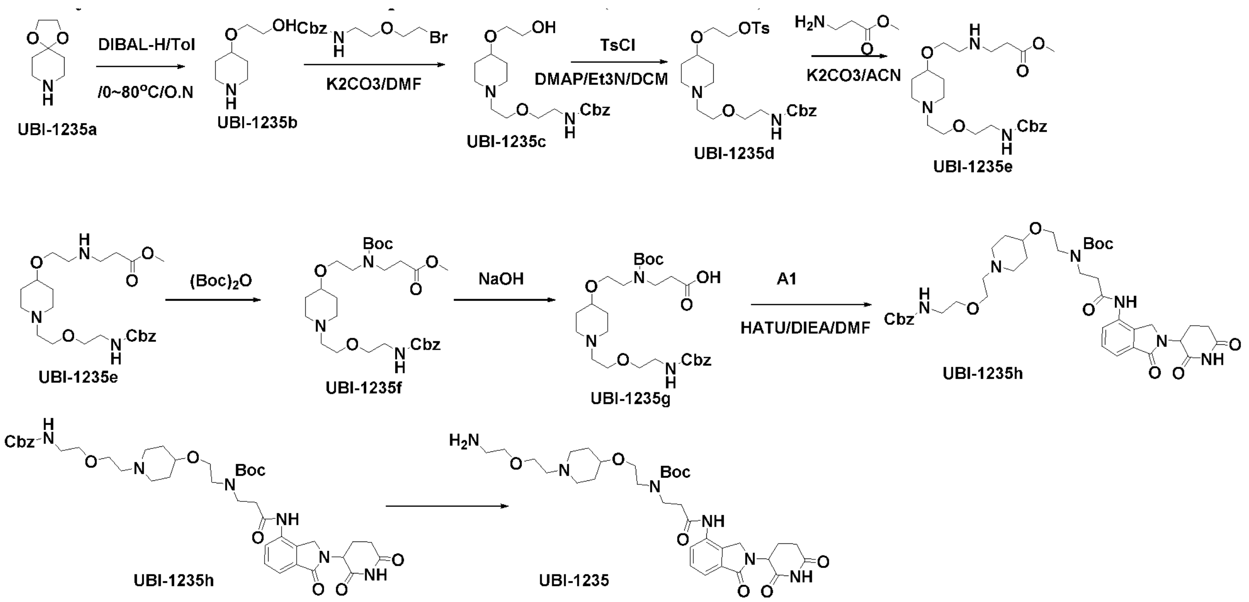


**Step 1: UBI-1235b**

**UBI-1235a** (1.0 g, 7.0 mmol, 1.0 equiv) was dosolved in toluene (20 mL), added 1.0 M diisobutylaluminum hydride in hexane (20 mL, 20 mmol, 2.9 equiv) at 0° C. The solution was heated to 80°C and reacted for 12 hours. added methanol (20 mL), sodium tartrate (20 mL) and chloroform (100 mL). The solution was stirred for 30 minutes. The chloroform was separated, and the aqueous solution was extracted 5 times. The mixture was dried (Na2SO4), filtered and concentrated to obtain 980 mg of crude **UBI-1235b** as white solid. LC-MS: $[M+H]^+$ =146.3

**Step 2: UBI-1235c**

UBI-1235b (0.28 g, 1.93 mmol) was added to DMF (10 ml), and added **UBI-1235b** (581 mg, 1.93 mmol) and $K_2CO_3$ (748 mg, 5.42 mmol). The above solution was stirred at 80°C overnight. TLC showed that the reaction was completed. The reaction mixture was added to a mixed solvent of DCM/MeOH (V/V=20 ml), stirred for 10 minutes, filtered, and concentrated *in vacuo* to obtain the crude product, which was isolated by column chromatography (DCM/MeOH=10/ 1) and purified to obtain product UBI-1235C (625 mg, yield: 89%) as a yellow oil. LC-MS: $[M+H]^+$ =367.5

**Step 3: UBI-1235d**

Compound UBI-1235C (500 mg, 1.366 mmol) was dissolved in DCM (10 ml), added TsCl (313 mg, 1.639 mmol) and triethylamine (275 mg, 2.732 mmol), and stirred overnight at room temperature. After completion of reaction, the reaction solution was filtered, concentrated, and purified by column chromatography (DCM/MeOH=10/1) to obtain the product **UBI-1235d** (510 mg, yield: 72%) as a colorless oil. LC-MS: $[M+H]^+$ =521.6

**Step 4: UBI-1235e**

**UBI-1235d** (450 mg, 0.865 mmol) and methyl 3-aminopropionate (200 mg, 1.30 mmol) were added to MeCN (30 ml), potassium carbonate (239 mg, 1.731 mmol) was added. The mixture was stirred overnight at room temperature. After completion of reaction, the reaction was filtered and concentrated, and isolated by column chromatography (DCM/MeOH=10/1) and purified to obtain the product **UBI-1235e** (190 mg, yield 49%) as a colorless oil. LC-MS: $[M+H]^+$ = 452.5

**Step 5: UBI-1235f**

UBI-1235e (100mg, 0.323mmol) was added to tetrahydrofuran/water (30ml), then di-*tert*-butyl dicarbonate (105mg, 0.484mmol) and sodium bicarbonate (54mg, 0.643mmol) were added, and stirred overnight at room temperature. After completion of reaction, the reaction was filtered and the filtrate was concentrated to obtain crude product, which was isolated by column chromatography (DCM/MeOH=10/1) and purified to obtain the product UBI-1235f (99 mg, yield 81%) as a yellow oil. LC-MS: $[M+H]^+$ = 552.3

**Step 6: UBI-1235g**

**UBI-1235f** (100 mg, 0.181 mmol) was dissolved in 4N NaOH (10 ml) and stirred overnight at room temperature. After completion of reaction, the mixture was adjusted to pH = 5, and added brine and ethyl acetate for stratification. The aqueous layer was extracted with ethyl acetate three times. The combined extract was dried over sodium sulfate, filtered and concentrated to obtain product **UBI-1235g** (56 mg).

**Step 7: UBI-1235h**

**UBI-1235g** (50 mg, 0.093 mmol) and lenalidomide (24 mg, 0.093 mmol) were added to DMF (10 ml), HATU (53 mg, 0.139 mmol) and DIEA (36 mg, 0.279 mmol) were added, and stirred at room temperature overnight. After completion of reaction, the crude product was concentrated to obtain crude product. The crude product was purified by Pre-TLC (DCM/MeOH=10/1) to obtain the product **UBI-1235h** (65 mg, yield 90%) as a white solid. LC-MS: $[M+H]^+$ =779.1

**Step 8: UBI-1235i**

**UBI-1235h** (85mg, 0.109mmol) was added to THF (10ml), and palladium on carbon (10%, 10mg) was added, and the mixture was stirred overnight at room temperature under $H_2$ atmosphere. After completion of reaction, the reaction solution was added to a mixture of DCM/MeOH (v/v=20 ml), stirred for 10 min, filtered, and concentrated to obtain crude product, which was purified by Pre-TLC (DCM/MeOH=10/1) to obtain UBI-1235i (56 mg, 80% yield) as white solid. LC-MS: $[M+H]^+$ = 645.3

L1-B conjugates, shown in Preparation Table A, in which H in structural formula of the conjugate being replaced with structures of E3 ligase ligand B, i.e. the corresponding linker L1 molecule.

Table A

| Compound No. | Structural formula of L1-B |
|---|---|
| L1-B(A1) | |
| 1 | 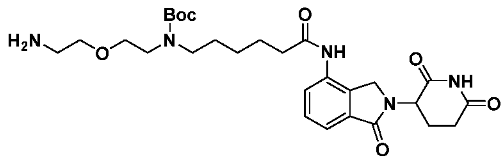 |
| 2 | 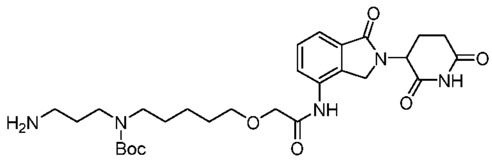 |
| 3 | 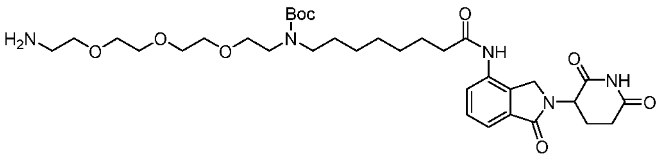 |
| 4 | 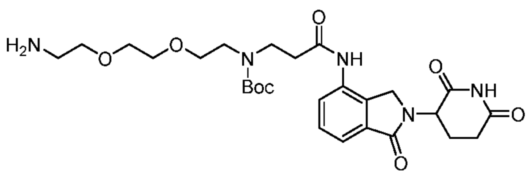 |

(continued)

| Compound No. | Structural formula of L1-B |
| --- | --- |
| 5 | 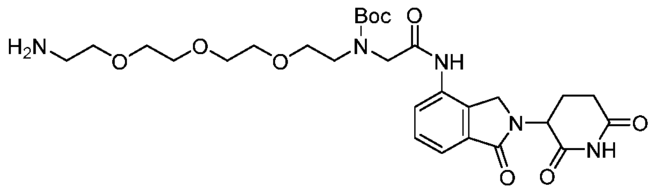 |
| 6 | 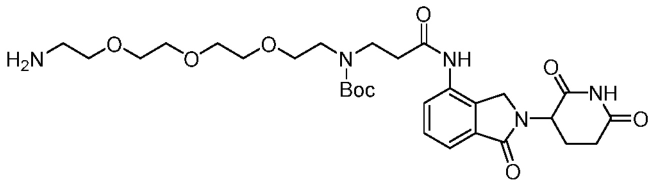 |
| 7 | 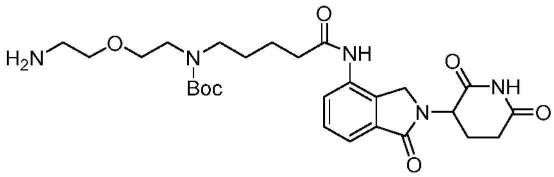 |
| 8 | 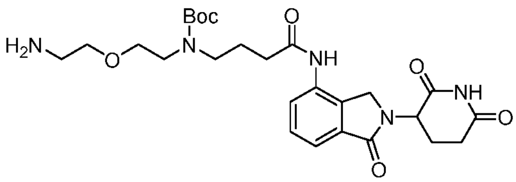 |
| 9 | 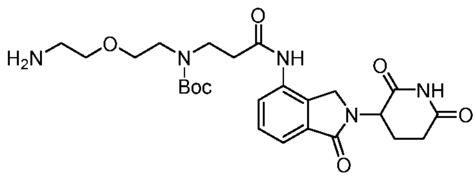 |
| 10 | 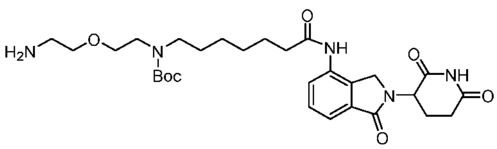 |
| 11 | 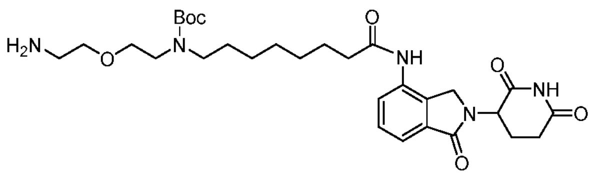 |
| 12 | 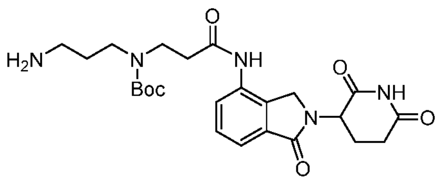 |

(continued)

| Compound No. | Structural formula of L1-B |
|---|---|
| 14 | 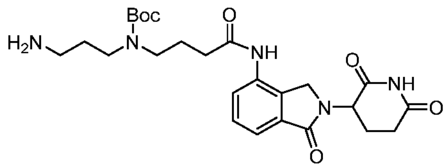 |
| 15 | 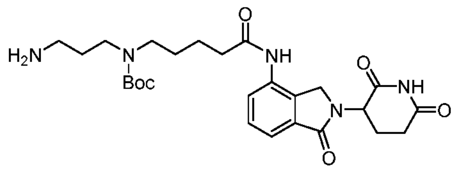 |
| 16 | 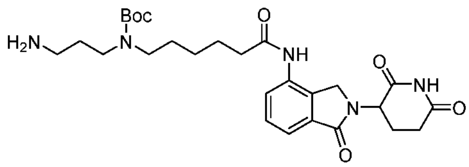 |
| 17 | 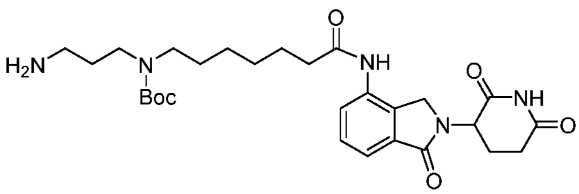 |
| 18 | 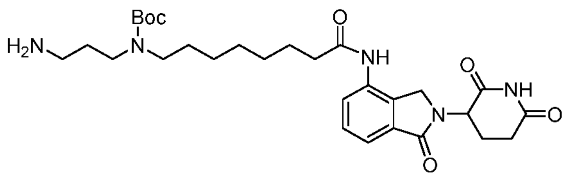 |
| 19 | 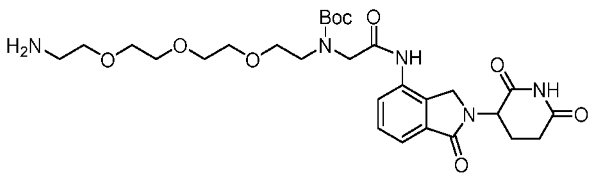 |
| 20 | 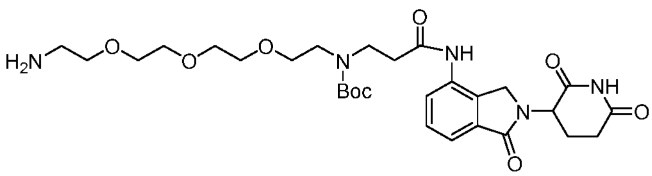 |
| 22 | 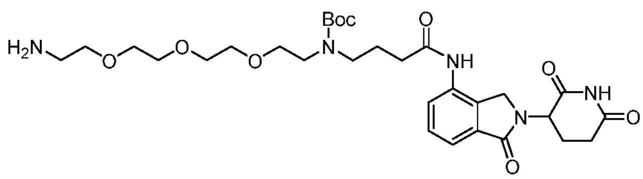 |

(continued)

| Compound No. | Structural formula of L1-B |
|---|---|
| 23 | 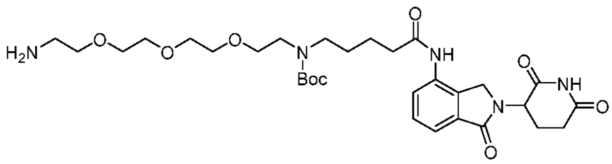 |
| 24 | 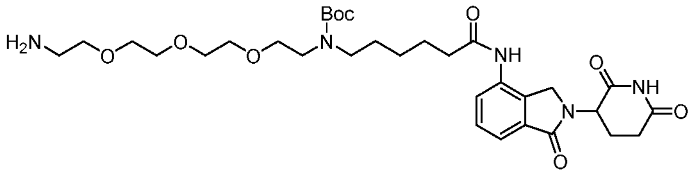 |
| 25 | 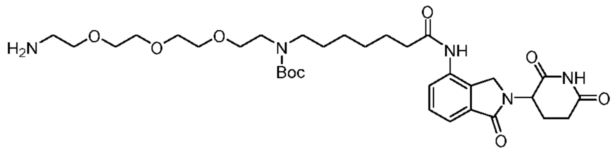 |
| 50 | 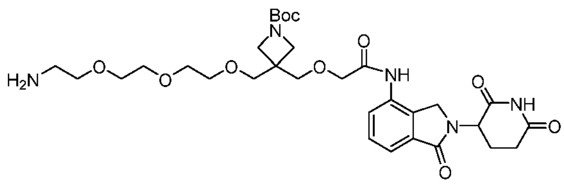 |
| 51 | 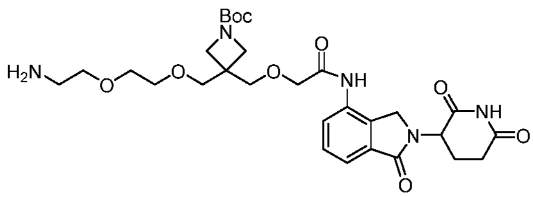 |
| 52 | 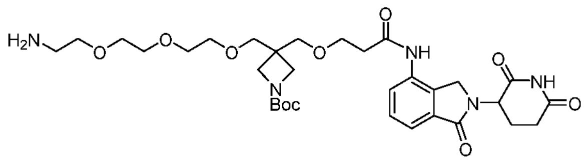 |
| 53 | 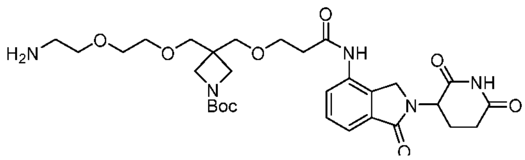 |
| L1-B(B1) | |
| 26 | 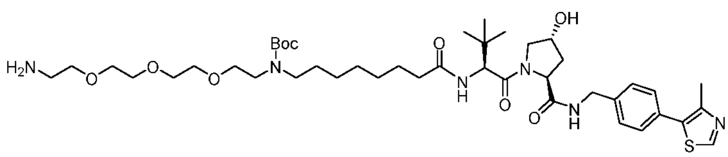 |

(continued)

| Compound No. | Structural formula of L1-B |
|---|---|
| 27 | 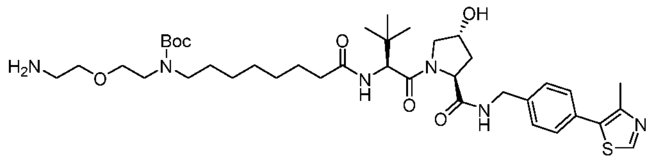 |
| 28 | 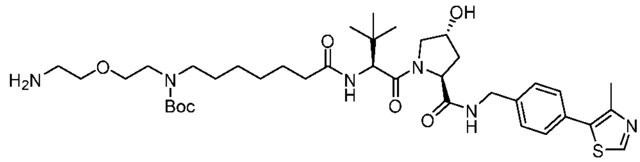 |
| 29 | 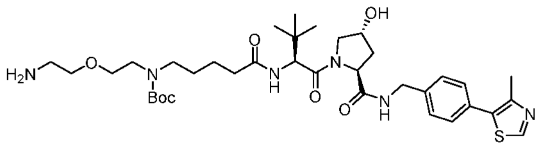 |
| 30 | 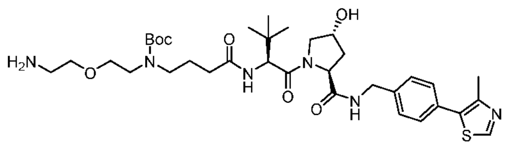 |
| 31 | 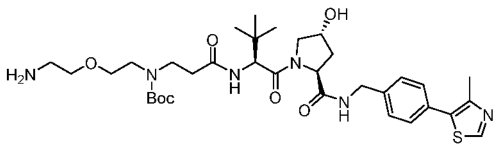 |
| 32 | 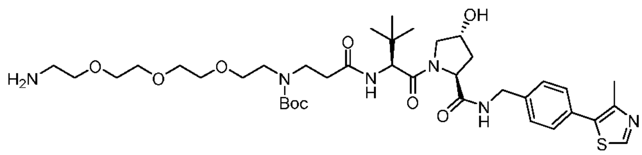 |
| 33 | 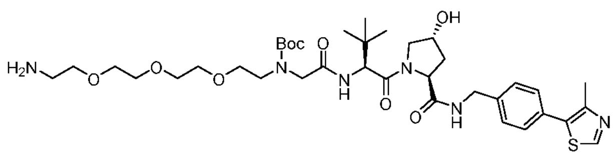 |
| 34 | 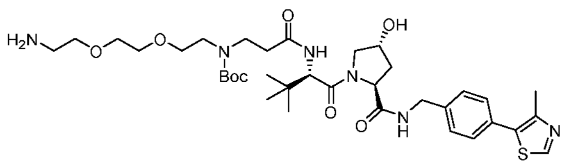 |
| 35 | 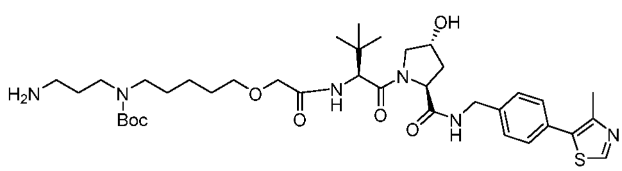 |

(continued)

| Compound No. | Structural formula of L1-B |
|---|---|
| 36 | 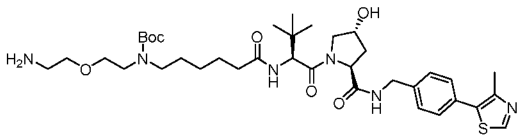 |
| 37 | 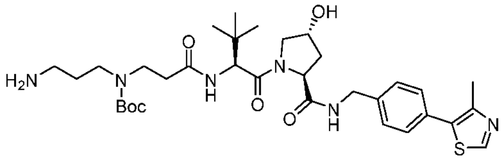 |
| 38 | 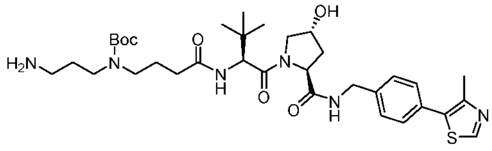 |
| 39 | 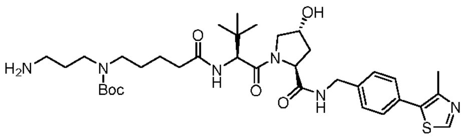 |
| 40 | 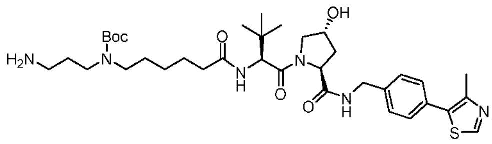 |
| 41 | 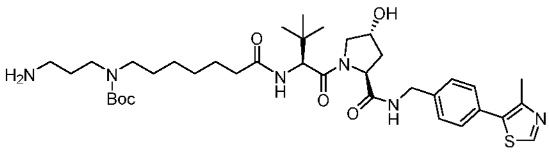 |
| 42 | 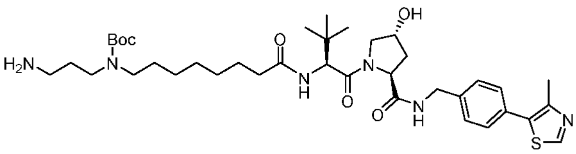 |
| 43 | 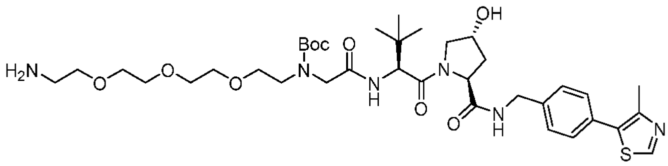 |
| 44 | 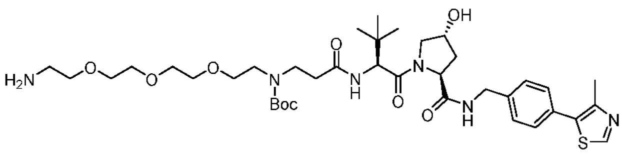 |

(continued)

| Compound No. | Structural formula of L1-B |
|---|---|
| 45 | 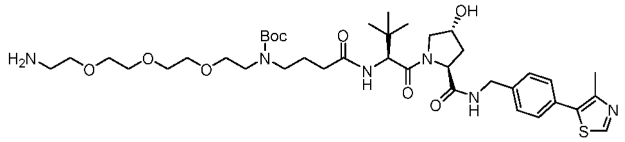 |
| 46 | 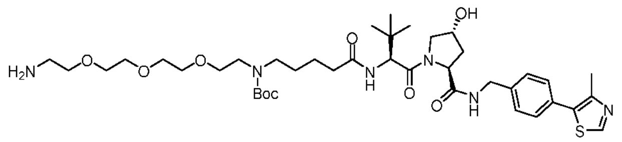 |
| 47 | 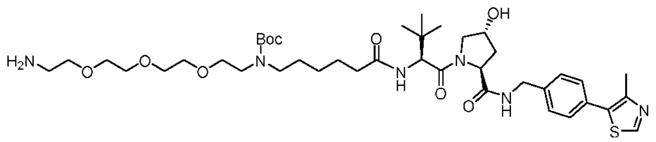 |
| 48 | 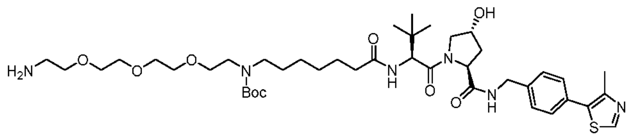 |
| 49 | 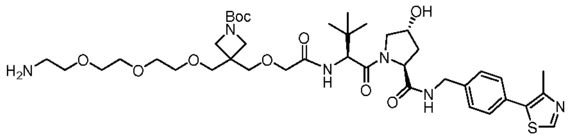 |
| 54 | 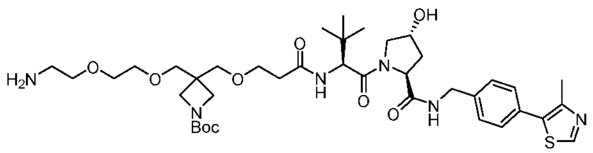 |
| 55 | 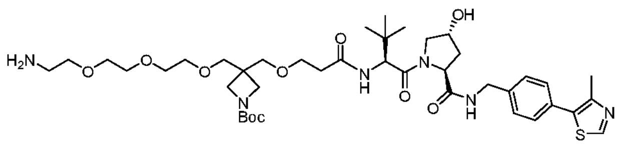 |
| 56 | 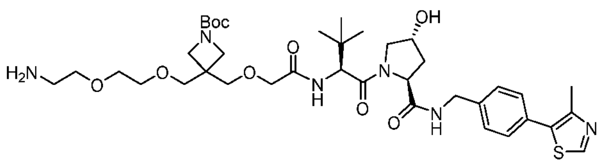 |

**Example Preparation of 2TED**

(2.1) Preparation procedure 1 of TED molecule:

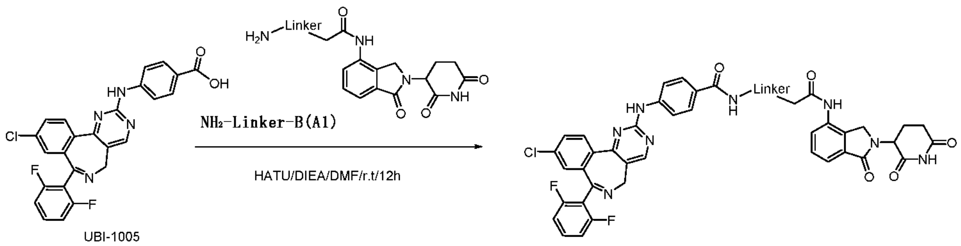


UBI-1005

in the formula,

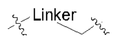

refers to L2.

Compound **UBI-1005** (20 mg, 0.042 mmol) was weighed and dissolved in DMF (2 mL), added $NH_2$-linker-B(Al) (1 eq.), HATU (2eq. 32mg, 0.084mmol) and DIEA (3eq. 16.2mg, 0.126mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL∗3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the corresponding TED compound.

The conjugate of A-L1-B as shown in Table B1 was prepared through similar method.

Table B1

| Examples | Compound structure | Analyze data |
|---|---|---|
| **UB-180501** | 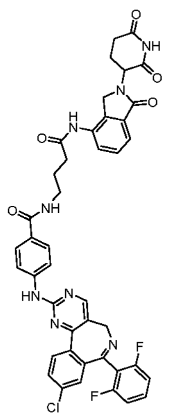 | $^1$H NMR (400 MHz, DMSO) δ 11.01 (s, 1H), 10.16 (s, 1H), 9.84 (s, 1H), 8.73 (s, 1H), 8.49 - 8.26 (m, 2H), 7.98 - 7.76 (m, 6H), 7.76 - 7.39 (m, 3H), 7.34 (s, 1H), 7.31-7.18 (m, 2H), 5.14 (dd, *J* = 13.3, 5.1 Hz, 1H), 4.52 - 4.26 (m, 2H), 3.53 - 3.39 (m, 2H), 3.13 - 2.74 (m, 1H), 2.95 - 2.74 (m, 1H), 2.95 - 2.56 (m, 3H), 2.45 - 2.30 (m, 2H), 2.00-581.98 (m, 1H), 1.88 (dd, *J* = 13.9, 6.9 Hz, 2H), 1.39 - 1.15 (m, 2H). LCMS [M+ H]$^+$ =803.2 |
| **UB-180502** | 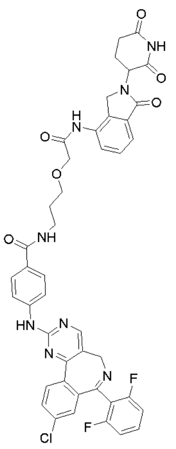 | $^1$H NMR (400 MHz, DMSO) 11.01 (s, 1H), 10.16 (s, 1H), 9.84 (s, 1H), 8.73 (s, 1H),8.33-8.26 (m, 2H), 7.93 - 7.71 (m, 6H), 7.61 -7.43 (m, 3H), 7.29 (s, 1H) 7.21-7.16 (m, 2H), 4.40 (q, J = 17.4 Hz, 1H), 5.14 (dd, J = 13.3, 5.1 Hz, 1H), 4.52 - 4.26 (m, 2H), 4.12 (s, 2H), 3.61 (t, J = 6.2 Hz, 2H), 2.67 (s, 1H), 2.59 (s, 1H), 2.43 - 2.08 (m, 3H), 2.03 - 1.81 (m, 2H).LCMS [M+ H]$^+$ =832.8 |

(continued)

| Examples | Compound structure | Analyze data |
|---|---|---|
| **UB-180503** | 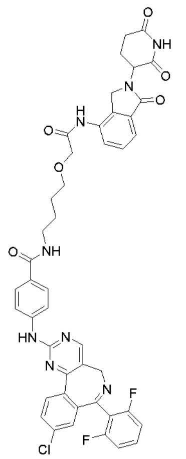 | $^1$H NMR (400 MHz, DMSO) δ 11.00 (s, 1H), 10.15 (s, 1H), 9.74 (s, 1H), 8.73 (s, 1H), 8.30 (dd, *J* = 9.9, 4.7 Hz, 2H), 7.95 - 7.77 (m, 5H), 7.77 - 7.69 (m, 1H), 7.63 - 7.45 (m, 3H), 7.34 (d, *J* = 2.1 Hz, 1H), 7.16 (d, *J* = 8.2 Hz, 2H), 5.13 (dd, *J* = 13.3, 5.0 Hz, 1H), 4.38 (q, *J* = 17.4 Hz, 2H), 4.10 (s, 2H), 3.75 - 3.41 (m, 2H), 2.89 (dd, *J* = 21.4, 9.8 Hz, 1H), 2.67 - 2.57 (m, 2H), 2.13 - 1.97 (m, 1H), 1.64 (d, *J* = 2.9 Hz, 4H). LCMS $[M+H]^+$ =846.8 |
| **UB-180504** | 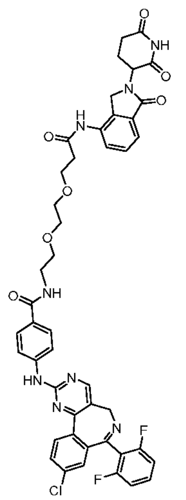 | $^1$H NMR (400 MHz, DMSO-d6) δ 11.02 (s, 1H), 10.16 (s, 1H), 9.85 (s, 1H), 8.72 (s, 1H), 8.40 - 8.24 (m, 1H), 7.95 - 7.78 (m, 6H), 7.60 - 7.43 (m, 2H), 7.34 (d, J = 2.2 Hz, 1H), 7.17 (t, J = 8.6 Hz, 2H), 5.14 (m, 1H), 4.36 (d, J = 8.8 Hz, 2H), 3.72 (t, J = 6.4 Hz, 2H), 3.54 (d, J = 10.5 Hz, 6H), 3.40 (q, J = 5.7 Hz, 2H), 2.99 - 2.84 (m, 1H), 2.68 - 2.55 (m, 3H), 2.32 (m, 1H), 2.09 - 1.96 (m, 1H), 1.31 - 1.21 (m, 2H). LCMS $[M+H]^+$ =877.2 |
| **UB-180509** | 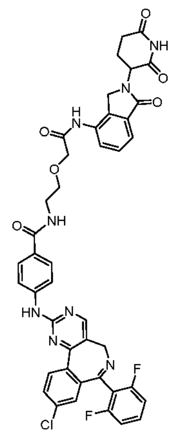 | $^1$H NMR (400 MHz, DMSO-$d_6$) δ 11.00 (s, 1H), 10.16 (s, 1H), 9.78 (s, 1H), 8.73 (s, 1H), 8.45 (t, *J* = 5.6 Hz, 1H), 8.30 (d, *J* = 8.6 Hz, 1H), 7.99 - 7.79 (m, 5H), 7.73 (dd, *J* = 7.8, 1.2 Hz, 1H), 7.61 - 7.43 (m, 3H), 7.34 (d, *J* = 2.1 Hz, 1H), 7.17 (t, *J* = 8.8 Hz, 2H), 5.13 (dd, *J* = 13.2, 5.2 Hz, 1H), 4.49 - 4.29 (m, 2H), 4.17 (s, 2H), 3.70 (t, *J* = 5.8 Hz, 2H), 3.53 (t, *J* = 5.6 Hz, 2H), 3.45 - 3.34 (m, 2H), 2.90 (M, 1H), 2.74 - 2.59 (m, 2H), 2.37 (m, 1H), 2.00 (m, 1H). LCMS $[M+H]^+$=819.2 |

(continued)

| Examples | Compound structure | Analyze data |
|---|---|---|
| **UB-180520** | 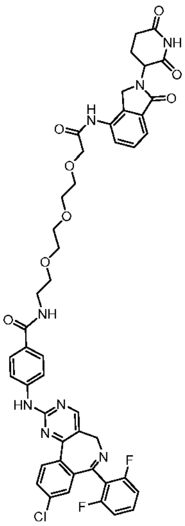 | $^1H$ NMR (400 MHz, DMSO-$d_6$) δ 11.01 (s, 1H), 10.16 (s, 1H), 9.70 (s, 1H), 8.72 (s, 1H), 8.40 - 8.24 (m, 2H), 7.97 - 7.80 (m, 5H), 7.74 (d, *J* = 7.7 Hz, 1H), 7.52 (m, 3H), 7.34 (d, *J* = 2.2 Hz, 1H), 7.17 (t, *J* = 8.4 Hz, 2H), 5.14 (m, 1H), 4.46-4.28 (m, 2H), 4.14 (s, 2H), 3.76-3.36 (m, 14H), 2.98 - 2.80 (m, 1H), 2.59 (d, *J* = 18.3 Hz, 1H), 2.37 (dd, *J* = 13.3, 4.3 Hz, 1H), 2.05 - 1.96 (m, 1H). LCMS $[M+H]^+$=1362.1 |
| **UB-180530** | 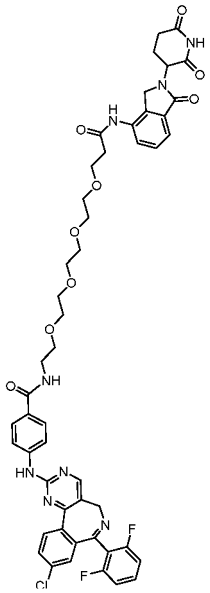 | $^1H$ NMR (400 MHz, DMSO-$d_6$) δ 11.02 (s, 1H), 10.16 (s, 1H), 9.85 (s, 1H), 8.73 (s, 1H), 8.42 - 8.18 (m, 2H), 7.97 -7.74 (m, 6H), 7.60 - 7.45 (m, 3H), 7.34 (d, *J* = 2.1 Hz, 1H), 7.17 (t, *J* = 8.7 Hz, 3H), 5.14 (m, 1H), 4.47 - 4.24 (m, 2H), 3.70 (t, *J*= 6.3 Hz, 2H), 3.58 - 3.45 (m, 15H), 3.39 (m, 2H), 3.01 - 2.84 (m, 1H), 2.71 - 2.51 (m, 5H), 2.33 (m, 1H), 2.10 - 1.96 (m, 1H). LCMS $[M+H]^+$ =1449.3 |

(continued)

| Examples | Compound structure | Analyze data |
|---|---|---|
| **UB-180534** | 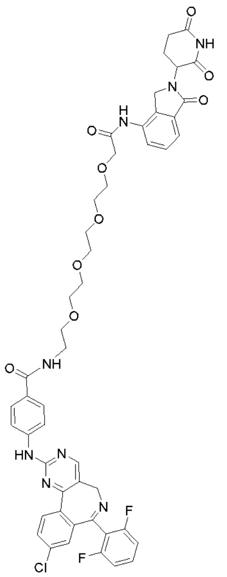 | $^1$H NMR (400 MHz, DMSO-$d_6$) δ 10.16 (s, 1H), 9.69 (s, 1H), 8.72 (s, 1H), 8.40 - 8.24 (m, 2H), 7.86 (m, 6H), 7.74 (d, *J* = 7.7 Hz, 1H), 7.53 (m, 3H), 7.34 (d, *J* = 2.1 Hz, 1H), 7.17 (t, *J* = 8.7 Hz, 2H), 5.14 (dd, *J* = 13.3, 5.1 Hz, 1H), 4.49 - 4.30 (m, 2H), 4.13 (s, 2H), 3.64 (m, 4H), 3.57 - 3.45 (m, 10H), 3.40 (m, 2H), 2.91 (m, 1H), 2.59 (m, 2H), 2.36 (m, 2H), 2.08 - 1.92 (m, 1H). LCMS $[M+H]^+$=1428.2 |
| **UB-180535** | 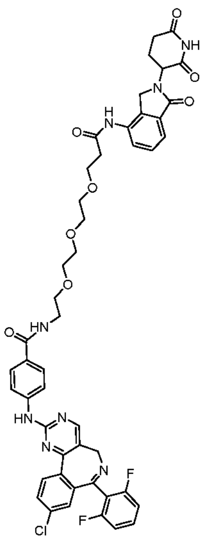 | $^1$H NMR (400 MHz, DMSO-$d_6$) δ 11.02 (s, 1H), 10.16 (s, 1H), 9.81 (d, *J* = 24.7 Hz, 1H), 8.72 (s, 1H), 8.43 - 8.24 (m, 2H), 7.98 - 7.78 (m, 6H), 7.62 - 7.41 (m, 3H), 7.34 (d, *J* = 2.2 Hz, 1H), 7.18 (m, 2H), 5.14 (dd, *J* = 13.3, 5.1 Hz, 1H), 4.44 - 4.27 (m, 2H), 3.70 (t, *J* = 6.3 Hz, 2H), 3.51 (m, 12H), 3.39 (m, 2H), 2.97 - 2.86 (m, 1H), 2.67 - 2.58 (m, 3H), 2.35 - 2.23 (m, 1H), 2.09 - 2.00 (m, 1H). LCMS $[M+H]^+$ =1382.9 |
| **UB-180559** | 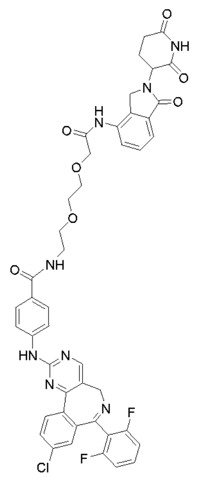 | $^1$H NMR (400 MHz, DMSO-$d_6$) δ 11.00 (s, 1H), 10.16 (s, 1H), 9.72 (s, 1H), 8.73 (s, 1H), 8.47 - 8.21 (m, 2H), 7.95 - 7.79 (m, 4H), 7.73 (m, 1H), 7.62 - 7.44 (m, 3H), 7.34 (d, *J* = 2.2 Hz, 1H), 7.17 (t, *J* = 8.7 Hz, 2H), 5.13 (m, 1H), 4.48 - 4.30 (m, 2H), 4.15 (s, 2H), 3.79 - 3.54 (m, 6H), 3.43 (m, 2H), 3.30 (m, 1H), 2.90 (m, 1H), 2.59 (m, 1H), 2.44 - 2.32 (m, 1H), 2.08 - 1.97 (m, 1H). LCMS $[M+H]^+$ = 863.5 |

(2.2) Preparation procedure 2 of TED molecule:

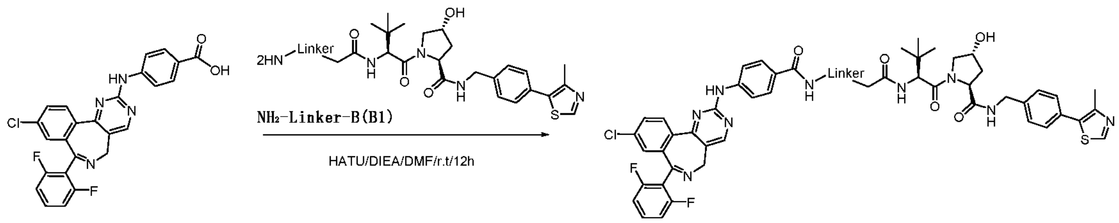


UBI-1005

TED

Compound **UBI-1005** (20 mg, 0.042 mmol) was weighed and dissolved in DMF (2 mL), added **$NH_2$-linker-B(BI)** (1 eq. ), HATU (2eq. 32mg, 0.084mmol) and DIEA (3eq. 16.2mg, 0.126mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL∗3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the corresponding TED compound.

The conjugate of A-L1-B as shown in Table B2 was prepared through similar method.

Table B2

| Examples | Compound structure | Analyze data |
|---|---|---|
| **UB-180505** | 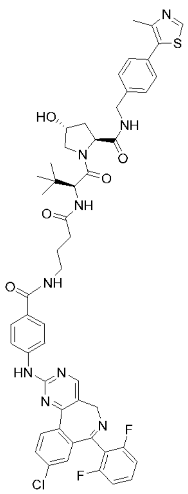 | $^1$H NMR (400 MHz, DMSO-$d_6$) δ 10.15 (s, 1H), 8.97 (s, 1H), 8.73 (s, 1H), 8.57 (t, *J* = 6.1 Hz, 1H), 8.30 (d, *J* = 8.4 Hz, 2H), 8.00-7.78 (m, 6H), 7.59 - 7.51 (m, 1H), 7.40 (q, *J* = 8.3 Hz, 4H), 7.17 (t, *J* = 8.5 Hz, 3H), 5.13 (d, *J* = 3.6Hz, 1H), 4.56 (d, *J* = 9.3 Hz, 1H), 4.50 - 4.32 (m, 3H), 4.22 (m, 1H), 3.75 - 3.62 (m, 2H), 3.26 (p, *J* = 6.6, 6.0 Hz, 2H), 2.44 (s, 3H), 2.27 (m, 2H), 2.09 - 1.87 (m, 2H), 1.75 (s, 2H), 1.24 (d, *J* = 5.8 Hz, 2H), 0.95 (s, 9H). LCMS $[M+H]^+$ =974.3 |

(continued)

| Examples | Compound structure | Analyze data |
|---|---|---|
| **UB-180506** | 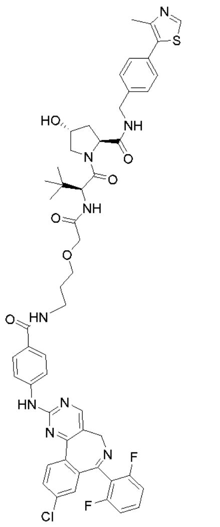 | $^1$H NMR (400 MHz, DMSO) δ 10.16 (s, 1H), 8.96 (d, *J* = 4.7 Hz, 1H), 8.73 (s, 1H), 8.61 (t, *J* = 5.9 Hz, 1H), 8.50 - 8.25 (m, 2H), 7.86 (dt, *J* = 19.7, 8.9 Hz, 6H), 7.65 - 7.34 (m, 7H), 7.34 - 7.00 (m, 3H), 4.58 (d, *J* = 9.6 Hz, 4H), 5.23-5.18 (m, 1H), 4.55 - 4.32 (m, 6H), 3.91 (s, 2H), 4.32 - 3.51 (m, 3H), 2.46 (s, 3H), 2.08 (d, *J* = 8.2 Hz, 2H), 2.07 - 1.85 (m, 1H), 1.85 - 1.59 (m, 2H), 0.93 (d, *J* = 6.2 Hz, 9H). LCMS $[M+H]^+$ =1003.8 |
| **UB-180514** | 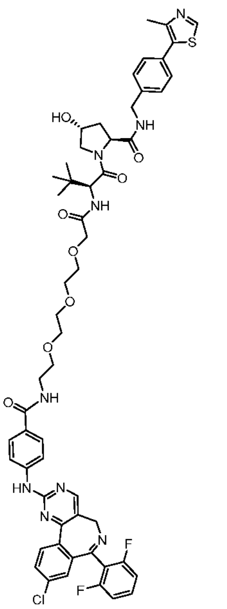 | $^1$H NMR (400 MHz, DMSO-$d_6$) δ 10.16 (s, 1H), 8.97 (s, 1H), 8.72 (s, 1H), 8.60 (s, 1H), 8.44 - 8.22 (m, 2H), 7.96 - 7.73 (m, 5H), 7.54 (s, 1H), 7.40 (d, *J* = 12.1 Hz, 3H), 7.32 (d, *J* = 13.5 Hz, 2H), 7.17 (m, 2H), 7.05 (m, 2H), 5.16 (s, 1H), 4.56 (d, *J* = 9.4 Hz, 1H), 4.49 - 4.18 (m, 3H), 3.97 (s, 2H), 3.74 - 3.45 (m, 12H), 3.40 (d, *J* = 5.5 Hz, 2H), 2.43 (s, 3H), 1.98 (m, 2H), 0.94 (s, 9H). LCMS $[M+H]^+$ =1078.4 |

(continued)

| Examples | Compound structure | Analyze data |
|---|---|---|
| **UB-180527** | 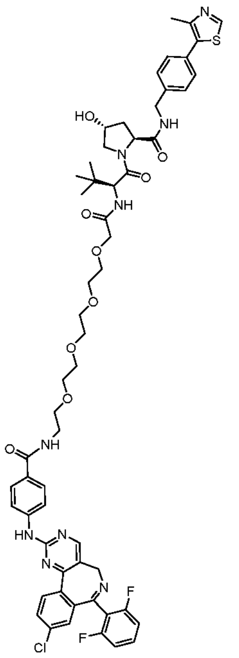 | $^1$H NMR (400 MHz, DMSO-$d_6$) δ 10.16 (s, 1H), 8.97 (s, 1H), 8.72 (s, 1H), 8.60 (t, *J* = 6.0 Hz, 1H), 8.44 - 8.24 (m, 2H), 7.94 - 7.80 (m, 5H), 7.54 (m,, 1H), 7.47 - 7.30 (m, 6H), 7.17 (t, *J* = 8.7 Hz, 2H), 5.15 (d, *J* = 3.6 Hz, 1H), 4.56 (d, *J* = 9.5 Hz, 1H), 4.49 - 4.31 (m, 2H), 4.25 (m, 1H), 3.96 (s, 2H), 3.73 - 3.45 (m, 18H), 3.40 (q, *J* = 7.4, 6.5 Hz, 2H), 2.43 (s, 3H), 2.06 (t, *J* = 10.4 Hz, 1H), 1.90 (m, 1H), 0.94 (s, 9H). LCMS $[M+H]^+$ =1122.5 |
| **UB-180531** | 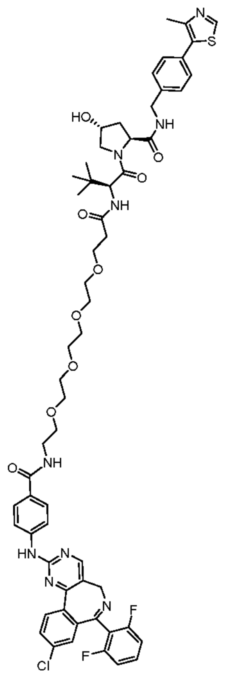 | $^1$H NMR (400 MHz, DMSO-$d_6$) δ 10.16 (s, 1H), 8.97 (s, 1H), 8.72 (s, 1H), 8.57 (t, *J* = 6.2 Hz, 1H), 8.41 - 8.23 (m, 2H), 7.99 - 7.81 (m, 5H), 7.54 (m, 1H), 7.45 - 7.32 (m, 5H), 7.17 (t, *J* = 8.9 Hz, 3H), 5.13 (d, *J* = 3.5 Hz, 1H), 4.55 (d, *J* = 9.4 Hz, 1H), 4.50 - 4.29 (m, 3H), 4.21 (m, 1H), 3.73 - 3.35 (m, 23H), 2.44 (d, *J* = 1.2 Hz, 3H), 2.14 - 1.78 (m, 2H), 0.93 (s, 9H). LCMS $[M+H]^+$=1136.5 |

(continued)

| Examples | Compound structure | Analyze data |
|---|---|---|
| UB-180532 | 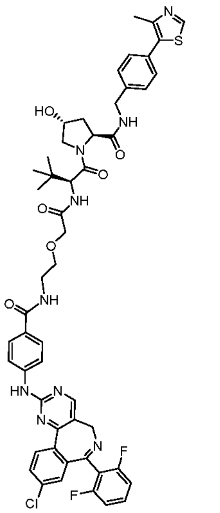 | $^1$H NMR (400 MHz, DMSO-$d_6$) δ 10.15 (s, 1H), 8.96 (s, 1H), 8.72 (s, 1H), 8.60 (d, *J* = 6.1 Hz, 1H), 8.46 (d, *J* = 5.7 Hz, 1H), 8.29 (d, *J* = 8.7 Hz, 1H), 7.87 (q, *J* = 8.5 Hz, 4H), 7.53 (d, *J* = 9.3 Hz, 2H), 7.36 (d, *J* = 19.2 Hz, 5H), 7.16 (s, 2H), 7.03 (s, 1H), 5.16 (d, *J* = 3.5 Hz, 1H), 4.57 (d, *J* = 9.5 Hz, 1H), 4.50 - 4.21 (m, 4H), 3.99 (d, *J* = 16.4 Hz, 2H), 3.55 (m, 9H), 2.42 (s, 3H), 2.06 (t, *J* = 10.4 Hz, 1H), 1.91 (d, *J* = 8.4 Hz, 1H), 0.93 (s, 9H). LCMS [M+H]$^+$ =1486.3 |
| UB-180533 | 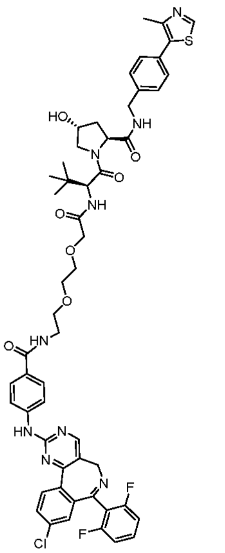 | $^1$H NMR (400 MHz, DMSO-$d_6$) δ 10.16 (s, 1H), 8.95 (s, 1H), 8.72 (s, 1H), 8.59 (t, *J* = 6.1 Hz, 1H), 8.43 - 8.26 (m, 2H), 7.94 - 7.78 (m, 4H), 7.54 (t, *J* = 7.5 Hz, 1H), 7.45 (d, *J* = 9.5 Hz, 1H), 7.35 (m, 5H), 7.18 (m, 3H), 5.18 (d, *J* = 3.5 Hz, 1H), 4.58 (d, *J* = 9.5 Hz, 1H), 4.53 - 4.18 (m, 4H), 3.98 (s, 2H), 3.66 - 3.41 (m, 11H), 2.45 - 2.39 (m, 3H), 2.07 - 1.87 (m, 2H), 0.94 (s, 9H). LCMS [M+H]$^+$ =1034.5 |
| UB-180536 | 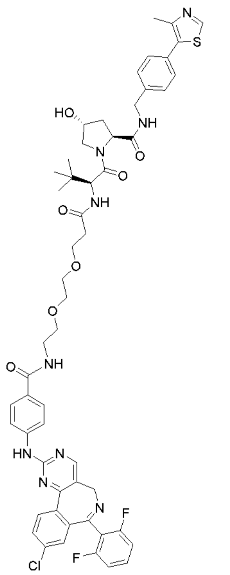 | $^1$H NMR (400 MHz, DMSO-$d_6$) δ 10.16 (s, 1H), 8.97 (s, 1H), 8.72 (s, 1H), 8.57 (t, *J* = 6.0 Hz, 1H), 8.39 - 8.20 (m, 2H), 7.99 - 7.77 (m, 6H), 7.61 - 7.48 (m, 1H), 7.45 - 7.30 (m, 5H), 7.17 (t, *J* = 8.7 Hz, 2H), 5.13 (d, *J* = 3.5 Hz, 1H), 4.55 (d, *J* = 9.4 Hz, 1H), 4.51 - 4.32 (m, 3H), 4.22 (dd, *J* = 15.8, 5.4 Hz, 1H), 3.64 (m, 4H), 3.51 (m, 6H), 3.44 - 3.35 (m, 2H), 2.55 (m, 1H), 2.44 (s, 3H), 2.39 - 2.32 (m, 1H), 2.08 - 1.81 (m, 2H), 0.93 (s, 9H). LCMS [M+H]$^+$ =1048.5 |

(continued)

| Examples | Compound structure | Analyze data |
| --- | --- | --- |
| **UB-180558** | 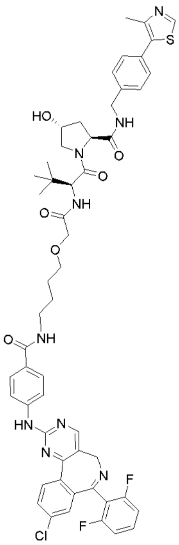 | $^1H$ NMR (400 MHz, DMSO-$d_6$) δ 10.15 (s, 1H), 8.96 (s, 1H), 8.72 (s, 1H), 8.60 (t, *J* = 6.0 Hz, 1H), 8.38 - 8.21 (m, 2H), 7.99 - 7.77 (m, 5H), 7.61 - 7.52 (m, 1H), 7.39 (s, 4H), 7.34 (d, *J* = 2.1 Hz, 1H), 7.17 (t, *J* = 8.7 Hz, 2H), 5.16 (d, *J* = 3.6 Hz, 1H), 4.56 (d, *J* = 9.5 Hz, 1H), 4.51 - 4.21 (m, 4H), 4.01 - 3.88 (m, 2H), 3.70 - 3.58 (m, 2H), 3.50 (d, *J* = 5.9 Hz, 2H), 3.33 - 3.17 (m, 3H), 2.43 (s, 3H), 2.06 (m, 1H), 1.90 (m, 1H), 1.68 - 1.51 (m, 4H), 0.94 (s, 9H). LCMS $[M+H]^+$=1018.7 |

(2.3) Preparation procedure 3 of TED molecule:

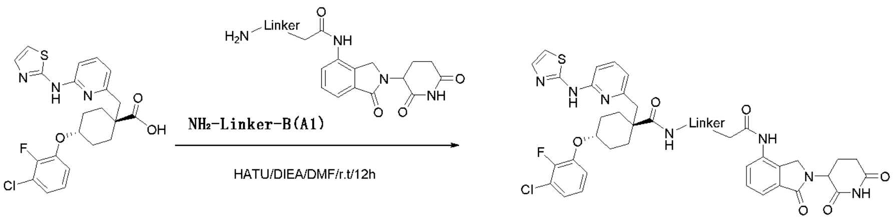


UBI-1001 TED

Compound **UBI-1001**(20 mg, 0.043 mmol)) was weighed and dissolved in DMF (2 mL), added **$NH_2$-linker-B(Al)** (1 eq.), **HOBT** (2eq. 23mg, 0.086mmol), **EDCI** (2eq. 16.4mg, 0.086mmol) and **TEA** (3eq. 13mg, 0.129mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL∗3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound TED.

The conjugate of A-L1-B as shown in Table B3 was prepared through similar method.

Table B3

| Examples | Compound structure | Analyze data |
|---|---|---|
| **UB-180572** | 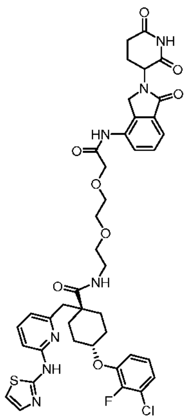 | $^1$H NMR (400 MHz, DMSO-d6) δ 11.12 (s, 1H), 11.01 (s, 1H), 9.71 (s, 1H), 7.73 (d, J = 7.7 Hz, 1H), 7.63 (d, J = 6.0 Hz, 1H), 7.52 (dt, J = 19.2, 7.5 Hz, 3H), 7.35 (d, J = 3.6 Hz, 1H), 7.16 - 7.03 (m, 3H), 6.95 (d, J = 3.7 Hz, 1H), 6.86 (d, J = 8.2 Hz, 1H), 6.62 (d, J = 7.3 Hz, 1H), 5.13 (dd, J = 13.2, 5.1 Hz, 1H), 4.52 (s, 1H), 4.45 - 4.26 (m, 2H), 4.14 (s, 2H), 3.68 (t, J = 4.6 Hz, 2H), 3.59 (t, J = 4.7 Hz, 2H), 3.41 (m, 3H), 3.21 (d, J = 6.1 Hz, 1H), 2.90 (s, 2H), 2.59 (m, 2H), 2.35 (m, 1H), 2.12 - 1.55 (m, 9H). LCMS $[M+1]^+$ =849.3 |
| **UB-180573** | 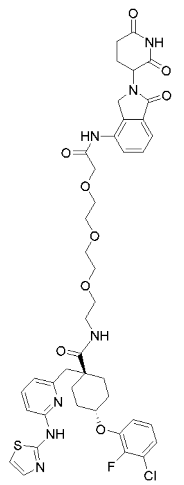 | $^1$H NMR (400 MHz, DMSO-d6) δ 11.12 (s, 1H), 11.01 (s, 1H), 9.72 (s, 1H), 7.73 (d, J = 7.7 Hz, 1H), 7.63 (s, 1H), 7.58 - 7.44 (m, 3H), 7.35 (d, J = 3.6 Hz, 1H), 7.23 - 7.01 (m, 3H), 6.95 (d, J = 3.6 Hz, 1H), 6.86 (d, J = 8.2 Hz, 1H), 6.62 (d, J = 7.4 Hz, 1H), 5.76 (s, 1H), 5.32 (t, J = 4.9 Hz, 1H), 5.14 (dd, J = 13.3, 5.2 Hz, 1H), 4.54 (s, 1H), 4.44 - 4.29 (m, 2H), 4.13 (s, 2H), 3.70 - 3.46 (m, 8H), 3.19 - 3.12 (m, 3H), 2.90 (m, 3H), 2.64 (m, 1H), 2.39 - 2.26 (m, 1H), 1.63 (m, 10H). LCMS $[M+1]^+$ =892.5 |
| **UB-180574** | 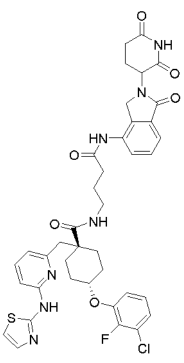 | $^1$H NMR (400 MHz, DMSO-d6) δ 11.12 (s, 1H), 11.02 (s, 1H), 9.83 (s, 1H), 7.82 (dd, J = 6.9, 2.0 Hz, 1H), 7.68 (t, J = 5.5 Hz, 1H), 7.57 - 7.44 (m, 3H), 7.35 (d, J = 3.6 Hz, 1H), 7.17 - 7.04 (m, 3H), 6.95 (d, J = 3.6 Hz, 1H), 6.86 (d, J = 8.2 Hz, 1H), 6.60 (d, J = 7.3 Hz, 1H), 5.15 (dd, J = 13.3, 5.2 Hz, 1H), 4.52 (s, 1H), 4.47 - 4.18 (m, 2H), 3.10 (q, J = 6.7 Hz, 2H), 2.99 - 2.79 (m, 3H), 2.58 (d, J = 18.0 Hz, 1H), 2.33 (p, J = 6.7, 5.9 Hz, 3H), 2.07 - 1.96 (m, 1H), 1.74 (m, 10H). LCMS $[M+1]^+$ =789.2 |

(continued)

| Examples | Compound structure | Analyze data |
|---|---|---|
| UB-180575 | 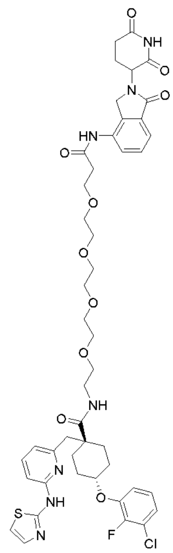 | $^1$H NMR (400 MHz, DMSO-$d_6$) δ 11.07 (d, $J$ = 38.3 Hz, 2H), 9.85 (s, 1H), 7.82 (m, 1H), 7.64 (t, $J$ = 5.7 Hz, 1H), 7.59 - 7.43 (m, 3H), 7.36 (d, $J$ = 3.6 Hz, 1H), 7.21 - 7.07 (m, 3H), 6.95 (d, $J$ = 3.6 Hz, 1H), 6.86 (d, $J$ = 8.2 Hz, 1H), 6.63 (d, $J$ = 7.4 Hz, 1H), 5.15 (m, 1H), 4.54 (s, 1H), 4.47 - 4.26 (m, 2H), 3.70 (t, $J$ = 6.3 Hz, 2H), 3.56 - 3.42 (m, 12H), 3.37 (m, 2H), 3.18 (q, $J$ = 5.9 Hz, 2H), 2.90 (m, 3H), 2.68 - 2.57 (m, 4H), 2.40 - 2.27 (m, 1H), 2.03 (m, 1H), 1.84 (m, 6H), 1.65 (m, 2H), 1.23 (d, $J$ = 4.1 Hz, 2H). LCMS $[M+H]^+$ =950.6 |
| UB-180576 | 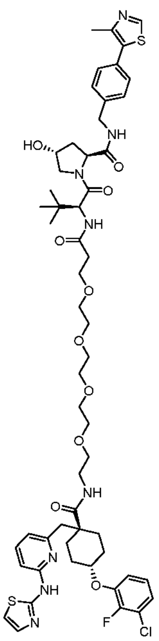 | $^1$H NMR (400 MHz, DMSO-$d_6$) δ 11.12 (s, 1H), 8.98 (s, 1H), 8.57 (t, $J$ = 6.1 Hz, 1H), 7.92 (d, $J$ = 9.3 Hz, 1H), 7.64 (t, $J$ = 5.6 Hz, 1H), 7.54 (t, $J$ = 7.8 Hz, 1H), 7.48 - 7.31 (m, 5H), 7.18 - 7.05 (m, 3H), 6.95 (d, $J$ = 3.6 Hz, 1H), 6.86 (d, $J$ = 8.2 Hz, 1H), 6.63 (d, $J$ = 7.3 Hz, 1H), 5.13 (d, $J$ = 3.5 Hz, 1H), 4.55 (d, $J$ = 9.1 Hz, 2H), 4.43 (m, 2H), 4.35 (s, 1H), 4.22 (m, 1H), 3.71 - 3.55 (m, 5H), 3.47 (d, $J$ = 7.6 Hz, 12H), 3.39 - 3.33 (m, 2H), 3.19 (q, $J$ = 6.2 Hz, 2H), 2.91 (s, 2H), 2.44 (s, 3H), 2.35 (m, 1H), 2.03 (m, 1H), 1.90 (m, 2H), 1.78 (m, 4H), 1.66 (m, 2H), 0.93 (s, 9H). LCMS $[M+H]^+$ =1121.7 |
| UB-180577 | 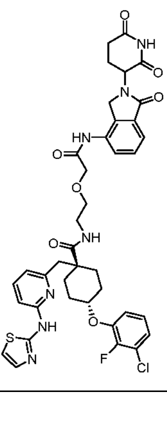 | $^1$H NMR (400 MHz, DMSO-d6) δ 11.11 (s, 1H), 11.00 (s, 1H), 9.74 (s, 1H), 7.76 (t, J = 5.7 Hz, 1H), 7.71 (dd, J = 7.9, 1.2 Hz, 1H), 7.57 - 7.46 (m, 3H), 7.35 (d, J = 3.6 Hz, 1H), 7.14 - 7.04 (m, 3H), 6.95 (d, J = 3.6 Hz, 1H), 6.84 (d, J = 8.2 Hz, 1H), 6.62 (d, J = 7.3 Hz, 1H), 5.13 (dd, J = 13.3, 5.1 Hz, 1H), 4.49 (s, 1H), 4.43 - 4.28 (m, 2H), 4.11 (s, 2H), 3.53 (t, J = 5.9 Hz, 2H), 2.91 (m, 3H), 2.33 (dd, J = 13.0, 4.8 Hz, 1H), 2.01 - 1.53 (m, 10H). LCMS $[M+1]^+$ =804.4 |

(continued)

| Examples | Compound structure | Analyze data |
|---|---|---|
| **UB-180581** | 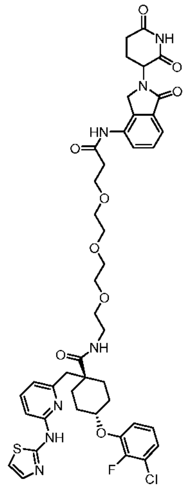 | $^1$H NMR (400 MHz, DMSO-$d_6$) δ 11.12 (s, 1H), 11.02 (s, 1H), 9.85 (s, 1H), 7.82 (d, *J* = 8.4 Hz, 1H), 7.63 (t, *J* = 5.6 Hz, 1H), 7.59 - 7.43 (m, 3H), 7.35 (d, *J* = 3.6 Hz, 1H), 7.21 - 7.05 (m, 3H), 6.95 (d, *J* = 3.6 Hz, 1H), 6.86 (d, *J* = 8.0 Hz, 1H), 6.63 (d, *J* = 7.2 Hz, 1H), 5.15 (dd, *J* = 13.2, 5.2 Hz, 1H), 4.54 (s, 1H), 4.45 - 4.26 (m, 2H), 3.70 (t, *J* = 6.4 Hz, 2H), 3.55 - 3.41 (m, 10H), 3.17 (q, *J* = 5.6 Hz, 2H), 2.90 (s, 2H), 2.65 - 2.55 (m, 4H), 2.33 (d, *J* = 17.5 Hz, 1H), 2.08 - 1.94 (m, 2H), 1.91 - 1.74 (m, 5H), 1.70 - 1.59 (m, 2H). LCMS $[M+H]^+$= 906.3. |
| **UB-180582** | 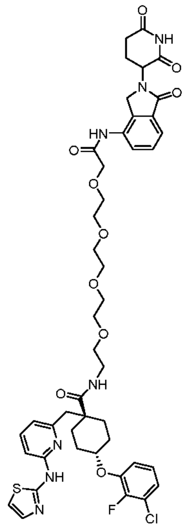 | $^1$H NMR (400 MHz, DMSO-$d_6$) δ 11.12 (s, 1H), 11.01 (s, 1H), 9.70 (s, 1H), 7.74 (d, *J* = 7.6 Hz, 1H), 7.63 (t, *J* = 5.6 Hz, 1H), 7.59 - 7.43 (m, 3H), 7.36 (d, *J* = 3.6 Hz, 1H), 7.23 - 7.04 (m, 3H), 6.95 (d, *J* = 3.6 Hz, 1H), 6.86 (d, *J* = 8.0 Hz, 1H), 6.63 (d, *J* = 7.2 Hz, 1H), 5.14 (dd, *J* = 13.2, 5.2 Hz, 1H), 4.58 - 4.49 (m, 1H), 4.46 - 4.30 (m, 2H), 4.13 (s, 2H), 3.73 - 3.64 (m, 2H), 3.64 - 3.56 (m, 2H), 3.56 - 3.41 (m, 8H), 3.31 - 3.25 (m, 2H), 3.24 - 3.06 (m, 3H), 2.98 - 2.82 (m, 3H), 2.65 - 2.55 (m, 1H), 2.43 - 2.33 (m, 1H), 2.07 - 1.93 (m, 2H), 1.90 - 1.74 (m, 5H), 1.70 - 1.58 (m, 2H). LCMS $[M+ H]^+$= 936.6. |
| **UB-180590** | 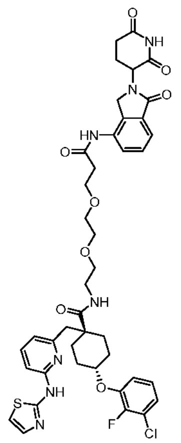 | $^1$H NMR (400 MHz, DMSO-*d*6) δ 11.11 (s, 1H), 11.02 (s, 1H), 9.85 (s, 1H), 7.81 (dd, *J* = 7.2, 1.6 Hz, 1H), 7.63 (t, *J* = 5.6 Hz, 1H), 7.58 - 7.39 (m, 3H), 7.35 (d, *J* = 3.6 Hz, 1H), 7.22 - 7.04 (m, 3H), 6.95 (d, *J* = 3.6 Hz, 1H), 6.86 (d, *J* = 8.4 Hz, 1H), 6.62 (d, *J* = 7.2 Hz, 1H), 5.32 (t, *J* = 4.8 Hz, 1H), 5.14 (dd, *J* = 13.2, 5.2 Hz, 1H), 4.54 (s, 1H), 4.42 - 4.28 (m, 2H), 3.71 (t, *J* = 6.3 Hz, 2H), 3.51 (q, *J* = 5.2 Hz, 3H), 3.17 (d, *J* = 5.9 Hz, 2H), 2.99 - 2.81 (m, 3H), 2.65 - 2.56 (m, 2H), 2.39 - 2.26 (m, 2H), 2.07 - 1.94 (m, 3H), 1.93 - 1.71 (m, 5H), 1.65 (d, *J* = 12.8 Hz, 2H), 1.46 (d, *J* = 7.6 Hz, 1H). LCMS [M+ H]: 862.5. |

(continued)

| Examples | Compound structure | Analyze data |
|---|---|---|
| **UB-180588** | 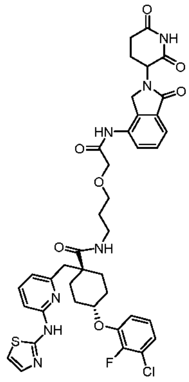 | $^1$H NMR (400 MHz, DMSO-$d_6$) δ 11.12 (s, 1H), 11.00 (s, 1H), 9.74 (s, 1H), 7.75 (dd, *J* = 7.8, 1.1 Hz, 1H), 7.63 (t, *J* = 5.6 Hz, 1H), 7.57 - 7.44 (m, 3H), 7.35 (d, *J* = 3.6 Hz, 1H), 7.10 (d, *J* = 4.0 Hz, 3H), 6.94 (d, *J* = 3.6 Hz, 1H), 6.86 (d, *J* = 8.2 Hz, 1H), 6.58 (d, *J* = 7.2 Hz, 1H), 5.13 (dd, *J* = 13.3, 5.1 Hz, 1H), 4.50 (s, 1H), 4.48 - 4.25 (m, 2H), 4.08 (s, 2H), 3.48 (t, *J* = 6.2 Hz, 2H), 3.17 (d, *J* = 6.3 Hz, 2H), 2.89 (s, 3H), 2.59 (s, 1H), 2.36 (dd, *J* = 13.2, 4.6 Hz, 1H), 2.02 - 1.95 (m, 1H), 1.92 - 1.51 (m, 10H). LCMS [M+H]+ =818.5 |
| **UB-180586** | 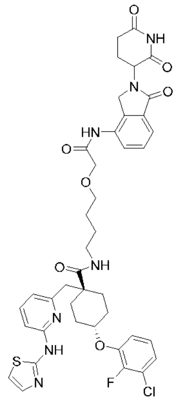 | $^1$H NMR (400 MHz, DMSO-$d_6$) δ 11.12 (s, 1H), 11.00 (s, 1H), 9.75 (s, 1H), 7.74 (dd, *J* = 7.8, 1.2 Hz, 1H), 7.64 - 7.57 (m, 1H), 7.59 - 7.46 (m, 3H), 7.35 (d, *J* = 3.6 Hz, 1H), 7.23 - 7.02 (m, 3H), 6.95 (d, *J* = 3.6 Hz, 1H), 6.86 (d, *J* = 8.2 Hz, 1H), 6.59 (d, *J* = 7.3 Hz, 1H), 5.13 (dd, *J* = 13.3, 5.1 Hz, 1H), 4.53 (s, 1H), 4.47 - 4.21 (m, 2H), 4.09 (s, 2H), 3.52 (t, *J* = 6.2 Hz, 2H), 3.06 (d, *J* = 6.2 Hz, 2H), 2.90 (s, 3H), 2.59 (d, *J* = 17.4 Hz, 1H), 2.37 (dd, *J* = 13.1, 4.5 Hz, 1H), 2.07 - 1.93 (m, 1H), 1.93 - 1.40 (m, 12H). LCMS [M+H]+ =832.3 |

(2.4) Preparation procedure 4 of TED molecule:

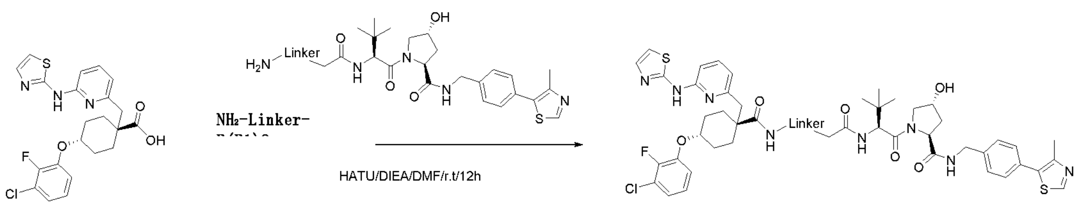


UBI-1001 TED

Compound **UBI-1001**(20 mg, 0.043 mmol)) was weighed and dissolved in DMF(2 mL), added **$NH_2$-linker-B(BI)** (1 eq.), **HOBT** (2eq. 23mg, 0.086mmol), **EDCI** (2eq. 16.4mg, 0.086mmol) and **TEA** (3eq. 13mg, 0.129mmol) in an ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL*3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound TED.

The conjugate of A-L1-B as shown in Table B4 was prepared through similar method.

Table B4

| Examples | Compound structure | Analyze data |
|---|---|---|
| **UB-180583** | 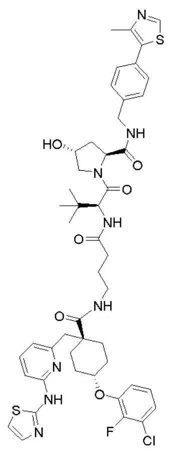 | $^1$H NMR (400 MHz, DMSO-$d_6$) δ 11.11 (s, 1H), 8.98 (s, 1H), 8.57 (t, *J* = 6.1 Hz, 1H), 7.91 (d, *J* = 9.3 Hz, 1H), 7.63 (t, *J* = 5.6 Hz, 1H), 7.53 (dd, *J* = 8.2, 7.4 Hz, 1H), 7.45 - 7.32 (m, 5H), 7.21 - 7.03 (m, 3H), 6.96 (d, *J* = 3.6 Hz, 1H), 6.86 (d, *J* = 8.1 Hz, 1H), 6.59 (d, *J* = 7.3 Hz, 1H), 5.14 (d, *J* = 3.6 Hz, 1H), 4.55 (d, *J* = 8.9 Hz, 2H), 4.50 - 4.40 (m, 2H), 4.36 (s, 1H), 4.22 (dd, *J* = 15.8, 5.4 Hz, 1H), 3.73 - 3.58 (m, 2H), 3.02 (q, *J* = 6.8 Hz, 2H), 2.91 (s, 2H), 2.44 (s, 3H), 2.26 - 2.18 (m, 1H), 2.20 - 1.92 (m, 2H), 1.98 - 1.46 (m, 10H), 0.94 (s, 9H). LCMS $[M/2+H]^+$ =480.3 |
| **UB-180584** | 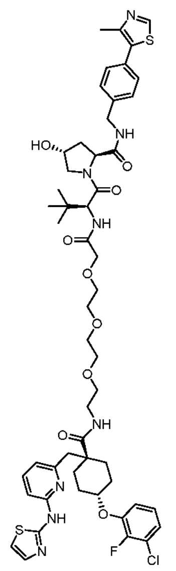 | $^1$H NMR (400 MHz, DMSO-d6) δ 11.12 (s, 1H), 8.98 (s, 1H), 8.60 (t, J = 6.0 Hz, 1H), 7.64 (t, J = 5.6 Hz, 1H), 7.54 (t, J = 7.8 Hz, 1H), 7.45 - 7.35 (m, 6H), 7.16 - 7.08 (m, 3H), 6.95 (d, J = 3.7 Hz, 1H), 6.86 (d, J = 8.2 Hz, 1H), 6.63 (d, J = 7.3 Hz, 1H), 5.17 (d, J = 3.5 Hz, 1H), 4.58 - 4.52 (m, 2H), 4.46 - 4.35 (m, 3H), 4.30 - 4.23 (m, 1H), 3.96 (s, 2H), 3.76 - 3.41 (m, 11H), 3.18 (q, *J* = 6.0 Hz, 2H), 2.91 (s, 2H), 2.44 (s, 3H), 2.11 - 2.03 (m, 1H), 1.95 - 1.58 (m, 9H), 0.94 (s, 9H). LCMS $[M/2+H]^+$ =532.3 |
| **UB-180585** | 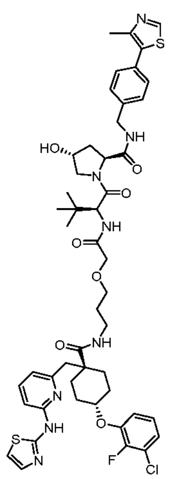 | $^1$H NMR (400 MHz, DMSO-$d_6$) δ 11.11 (s, 1H), 8.96 (s, 1H), 8.60 (t, *J* = 6.0 Hz, 1H), 7.62 (d, *J* = 6.2 Hz, 1H), 7.54 (t, *J* = 7.8 Hz, 1H), 7.47 - 7.27 (m, 6H), 7.21 - 7.04 (m, 3H), 6.96 (d, *J* = 3.6 Hz, 1H), 6.86 (d, *J* = 8.2 Hz, 1H), 6.58 (d, *J* = 7.3 Hz, 1H), 5.17 (d, *J* = 3.5 Hz, 1H), 4.56 (m, 2H), 4.45 (t, *J* = 8.3 Hz, 1H), 4.32 (dd, *J* = 26.7, 5.6 Hz, 3H), 3.91 (m, 2H), 3.71 - 3.53 (m, 2H), 3.40 (m, 2H), 3.11 (m, 2H), 2.90 (s, 2H), 2.43 (s, 3H), 2.06 (s, 1H), 1.96 - 1.53 (m, 10H), 0.94 (s, 9H). LCMS $[M/2+1]^+$=495.3 |

(continued)

| Examples | Compound structure | Analyze data |
|---|---|---|
| **UB-180587** | 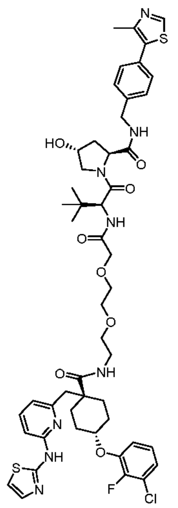 | $^1$H NMR (400 MHz, DMSO-$d_6$) δ 11.11 (s, 1H), 8.97 (s, 1H), 8.58 (t, *J* = 5.9 Hz, 1H), 7.65 (t, *J* = 5.6 Hz, 1H), 7.54 (dd, *J* = 8.2, 7.4 Hz, 1H), 7.46 - 7.32 (m, 6H), 7.18 - 7.05 (m, 3H), 6.95 (d, *J* = 3.6 Hz, 1H), 6.86 (d, *J* = 8.2 Hz, 1H), 6.62 (d, *J* = 7.4 Hz, 1H), 5.17 (d, *J* = 3.5 Hz, 1H), 4.58 (d, *J* = 9.6 Hz, 1H), 4.53 (s, 1H), 4.45 (t, *J* = 8.1 Hz, 1H), 4.39 - 4.22 (m, 3H), 3.97 (s, 2H), 3.67 - 3.50 (m, 5H), 3.40 (t, *J* = 6.4 Hz, 2H), 3.22 (d, *J* = 6.1 Hz, 2H), 2.91 (s, 2H), 2.43 (s, 3H), 2.07 (t, *J* = 10.3 Hz, 1H), 1.98 - 1.57 (m, 10H), 0.94 (s, 9H). LCMS [M+H]+ =1019.6 |
| **UB-180589** | 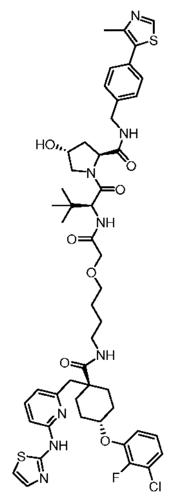 | $^1$H NMR (400 MHz, DMSO-$d_6$) δ 11.11 (s, 1H), 8.97 (s, 1H), 8.61 (t, *J* = 6.0 Hz, 1H), 7.63 - 7.50 (m, 2H), 7.37 (m, 6H), 7.21 - 7.05 (m, 3H), 6.95 (d, *J* = 3.6 Hz, 1H), 6.86 (d, *J* = 8.2 Hz, 1H), 6.59 (d, *J* = 7.4 Hz, 1H), 5.17 (d, *J* = 3.5 Hz, 1H), 4.57 (m, 2H), 4.51 - 4.19 (m, 4H), 3.92 (d, *J* = 1.4 Hz, 2H), 3.75 - 3.54 (m, 2H), 3.45 (t, *J* = 5.2 Hz, 2H), 3.05 (d, *J* = 6.0 Hz, 2H), 2.90 (s, 2H), 2.44 (s, 3H), 2.18 - 2.00 (m, 1H), 1.95 - 1.34 (m, 13H), 0.94 (s, 9H). LCMS [M/2+H]+ =502.9 |
| **UB-180599** (199) | 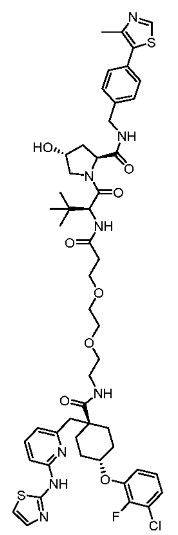 | $^1$H NMR (400 MHz, DMSO-*d*6) δ 11.12 (s, 1H), 8.98 (s, 1H), 8.58 (t, *J* = 6.0 Hz, 1H), 7.93 (d, *J* = 9.3 Hz, 1H), 7.66 (t, *J* = 5.6 Hz, 1H), 7.54 (t, *J* = 7.8 Hz, 1H), 7.47 - 7.30 (m, 4H), 7.22 - 7.05 (m, 3H), 6.95 (d, *J* = 3.6 Hz, 1H), 6.86 (d, *J* = 8.1 Hz, 1H), 6.63 (d, *J* = 7.2 Hz, 1H), 5.39 - 5.27 (m, 1H), 5.14 (d, *J* = 3.5 Hz, 1H), 4.64 - 4.51 (m, 2H), 4.50 - 4.38 (m, 2H), 4.38 - 4.31 (m, 1H), 4.22 (dd, *J* = 15.9, 5.4 Hz, 1H), 3.68 - 3.56 (m, 3H), 3.52 - 3.43 (m, 4H), 3.23 - 3.13 (m, 2H), 2.91 (s, 2H), 2.44 (s, 3H), 2.39 - 2.34 (m, 1H), 2.08 - 1.95 (m, 3H), 1.94 - 1.71 (m, 6H), 1.70 - 1.59 (m, 2H), 1.54 - 1.42 (m, 2H), 1.24 (s, 9H). [M+H]+ =1033.6. |

(continued)

| Examples | Compound structure | Analyze data |
|---|---|---|
| **UB-180576** | 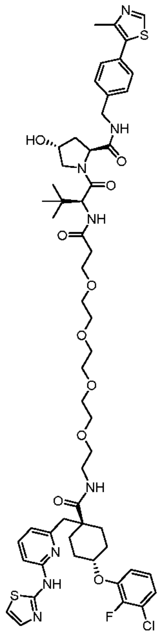 | $^1$H NMR (400 MHz, DMSO-$d_6$) δ 11.12 (s, 1H), 8.98 (s, 1H), 8.57 (t, *J* = 6.1 Hz, 1H), 7.92 (d, *J* = 9.3 Hz, 1H), 7.64 (t, *J* = 5.6 Hz, 1H), 7.54 (t, *J* = 7.8 Hz, 1H), 7.48 - 7.31 (m, 5H), 7.18 - 7.05 (m, 3H), 6.95 (d, *J* = 3.6 Hz, 1H), 6.86 (d, *J* = 8.2 Hz, 1H), 6.63 (d, *J* = 7.3 Hz, 1H), 5.13 (d, *J* = 3.5 Hz, 1H), 4.55 (d, *J* = 9.1 Hz, 2H), 4.43 (m, 2H), 4.35 (s, 1H), 4.22 (m, 1H), 3.71 - 3.55 (m, 5H), 3.47 (d, *J* = 7.6 Hz, 12H), 3.39 - 3.33 (m, 2H), 3.19 (q, *J* = 6.2 Hz, 2H), 2.91 (s, 2H), 2.44 (s, 3H), 2.35 (m, 1H), 2.03 (m, 1H), 1.90 (m, 2H), 1.78 (m, 4H), 1.66 (m, 2H), 0.93 (s, 9H). LCMS $[M+H]^+$ =1121.7 |
| **UB-180578** | 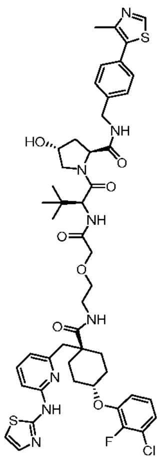 | $^1$H NMR (400 MHz, DMSO-$d_6$) δ 11.11 (s, 1H), 8.96 (d, *J* = 2.8 Hz, 1H), 8.59 (d, *J* = 7.1 Hz, 1H), 7.76 (s, 1H), 7.55 (t, *J* = 7.9 Hz, 1H), 7.47 (d, *J* = 9.6 Hz, 1H), 7.37 (m, 5H), 7.11 (m, 3H), 6.95 (t, *J* = 3.2 Hz, 1H), 6.86 (d, *J* = 8.3 Hz, 1H), 6.63 (d, *J* = 7.3 Hz, 1H), 5.16 (d, *J* = 3.5 Hz, 1H), 4.56 (t, *J* = 9.7 Hz, 2H), 4.48 - 4.23 (m, 4H), 3.94 (t, *J* = 10.2 Hz, 2H), 3.71 - 3.58 (m, 2H), 3.48 (m, 2H), 3.24 (m, 2H), 2.92 (m, 2H), 2.43 (d, *J* = 2.9 Hz, 3H), 2.13 - 1.57 (m, 10H), 0.93 (s, 9H). LCMS $[M+H]^+$ =975.6 |

(continued)

| Examples | Compound structure | Analyze data |
|---|---|---|
| **UB-180580** | 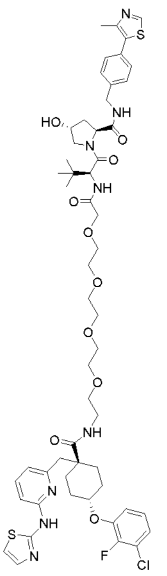 | $^1$H NMR (400 MHz, DMSO-$d_6$) δ 11.12 (s, 1H), 8.98 (s, 1H), 8.60 (t, *J* = 6.0 Hz, 1H), 7.64 (t, *J* = 5.6 Hz, 1H), 7.54 (t, *J* = 7.6 Hz, 1H), 7.47 - 7.32 (m, 6H), 7.19 - 7.07 (m, 3H), 6.95 (d, *J* = 3.6 Hz, 1H), 6.86 (d, *J* = 8.0 Hz, 1H), 6.63 (d, *J* = 7.2 Hz, 1H), 5.15 (d, *J* = 3.6 Hz, 1H), 4.60 - 4.49 (m, 2H), 4.47 - 4.33 (m, 3H), 4.25 (dd, *J* = 15.6, 5.6 Hz, 1H), 3.96 (s, 2H), 3.70 - 3.64 (m, 1H), 3.63 - 3.57 (m, 3H), 3.57 - 3.50 (m, 6H), 3.49 - 3.44 (m, 4H), 3.22 - 3.13 (m, 2H), 2.90 (s, 1H), 2.89 (s, 1H), 2.44 (s, 3H), 2.09 - 2.02 (m, 1H), 1.94 - 1.84 (m, 3H), 1.84 - 1.73 (m, 4H), 1.69 - 1.60 (m, 2H), 0.94 (s, 9H). LCMS $[M+H]^+$=1107.7. |

Compounds in Table B9 were prepared by preparation procedures similar to (2.1), (2.2), (2.3) or (2.4)

Table B9

| Examples | Structural data analysis |
|---|---|
| **UB-180600 (100)** | 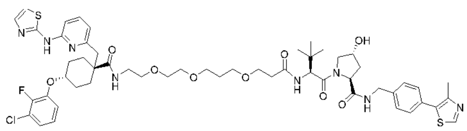 |
| | $^1$H NMR (400 MHz, DMSO-$d_6$) δ 11.12 (s, 1H), 8.98 (s, 1H), 8.57 (t, *J* = 6.1 Hz, 1H), 7.90 (d, *J* = 9.4 Hz, 1H), 7.65 (t, *J* = 5.5 Hz, 1H), 7.54 (dd, *J* = 8.2, 7.3 Hz, 1H), 7.45 - 7.32 (m, 5H), 7.22 - 7.05 (m, 3H), 6.95 (d, *J* = 3.6 Hz, 1H), 6.87 (d, *J* = 8.2 Hz, 1H), 6.64 (d, *J* = 7.3 Hz, 1H), 5.14 (d, *J* = 3.6 Hz, 1H), 4.56 (d, *J* = 9.8 Hz, 2H), 4.49 - 4.38 (m, 2H), 4.35 (s, 1H), 4.22 (dd, *J* = 15.9, 5.5 Hz, 1H), 3.74 - 3.42 (m, 9H), 3.42 - 3.36 (m, 6H), 3.19 (q, *J* = 5.9 Hz, 2H), 2.91 (s, 2H), 2.44 (s, 3H), 2.31 (dt, *J* = 14.5, 5.8 Hz, 1H), 2.02 (d, *J* = 9.1 Hz, 1H), 1.95 - 1.55 (m, 11H), 0.93 (s, 9H). LCMS $[M/2+1]^+$ =546.9 |
| **UB-180609 (109)** | 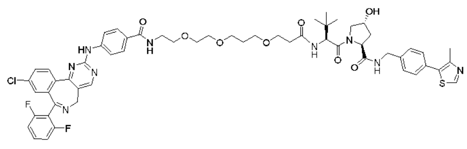 |
| | $^1$H NMR (400 MHz, DMSO-$d_6$) δ 10.16 (s, 1H), 8.98 (s, 1H), 8.72 (s, 1H), 8.56 (t, *J* = 6.1 Hz, 1H), 8.34 (t, *J* = 5.6 Hz, 1H), 8.30 (d, *J* = 8.5 Hz, 1H), 7.92 - 7.83 (m, 6H), 7.55 (ddd, *J* = 15.0, 8.4, 6.6 Hz, 1H), 7.44 - 7.31 (m, 5H), 7.17 (t, *J* = 8.8 Hz, 2H), 5.13 (d, *J* = 3.5 Hz, 1H), 4.55 (d, *J* = 9.4 Hz, 1H), 4.42 (td, *J* = 7.3, 6.5, 3.9 Hz, 2H), 4.35 (s, 1H), 4.22 (dd, *J* = 15.8, 5.4 Hz, 1H), 3.74 - 3.44 (m, 11H), 3.44 - 3.36 (m, 7H), 3.14 (s, 1H), 2.44 (s, 3H), 2.32 (dt, *J* = 14.9, 5.9 Hz, 1H), 2.03 (t, *J* = 10.3 Hz, 1H), 1.90 (ddd, *J* = 12.9, 8.6, 4.6 Hz, 1H), 1.69 (p, *J* = 6.5 Hz, 2H), 0.93 (s, 9H). LCMS $[M+1]^+$=1106.3 |

(continued)

| Examples | Structural data analysis |
| --- | --- |
| **UB-180610 (110)** | 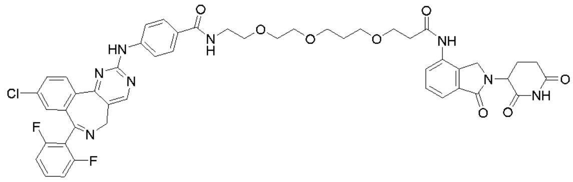 |
| | $^1$H NMR (400 MHz, DMSO-$d_6$) δ 11.02 (s, 1H), 10.16 (s, 1H), 9.85 (s, 1H), 8.72 (s, 1H), 8.34 (t, *J* = 5.6 Hz, 1H), 8.30 (d, *J* = 8.5 Hz, 1H), 7.95 - 7.80 (m, 6H), 7.63 - 7.43 (m, 3H), 7.34 (d, *J* = 2.2 Hz, 1H), 7.17 (t, *J* = 8.8 Hz, 2H), 5.15 (dd, *J* = 13.3, 5.1 Hz, 1H), 4.46 - 4.22 (m, 2H), 3.66 (t, *J* = 6.3 Hz, 2H), 3.51 (dd, *J* = 6.8, 4.1 Hz, 4H), 3.43 (dt, *J* = 13.7, 6.4 Hz, 8H), 3.14 (tt, *J* = 7.4, 3.7 Hz, 1H), 2.92 (ddd, *J* = 18.2, 13.5, 5.4 Hz, 1H), 2.59 (s, 4H), 2.32 (dd, *J* = 12.9, 4.3 Hz, 1H), 2.02 (dd, *J* = 13.0, 6.6 Hz, 1H), 1.70 (t, *J* = 6.4 Hz, 2H). LCMS $[M+H]^+$=935.3 |
| **UB-180611 (111)** | 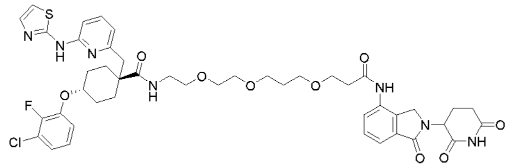 |
| | $^1$H NMR (400 MHz, DMSO-$d_6$) δ 11.12 (s, 1H), 11.02 (s, 1H), 9.86 (s, 1H), 7.81 (dd, *J* = 7.3, 1.7 Hz, 1H), 7.64 (t, *J* = 5.5 Hz, 1H), 7.56 - 7.46 (m, 3H), 7.35 (d, *J* = 3.6 Hz, 1H), 7.20 - 7.05 (m, 3H), 6.95 (d, *J* = 3.6 Hz, 1H), 6.86 (d, *J* = 8.2 Hz, 1H), 6.63 (d, *J* = 7.3 Hz, 1H), 5.15 (dd, *J* = 13.3, 5.2 Hz, 1H), 4.54 (s, 1H), 4.43 - 4.30 (m, 2H), 3.65 (t, *J* = 6.3 Hz, 2H), 3.50 - 3.33 (m, 9H), 3.22 - 3.15 (m, 2H), 2.90 (m, 2H), 2.65 (d, *J* = 18.1 Hz, 1H), 2.61 - 2.55 (m, 3H), 2.34 - 2.25 (m, 1H), 2.06 - 1.97 (m, 1H), 1.92 - 1.61 (m, 9H). LCMS [M+H]+ =922.26. |
| **UB-180612 (112)** | 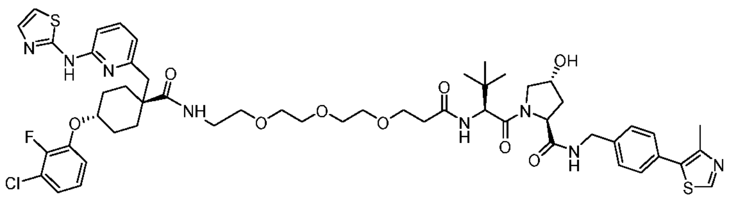 |
| | $^1$H NMR (400 MHz, DMSO-$d_6$) δ 10.16 (s, 1H), 8.97 (s, 1H), 8.72 (s, 1H), 8.56 (t, *J* = 6.0 Hz, 1H), 8.34 (t, *J* = 5.6 Hz, 1H), 8.30 (d, *J* = 8.4 Hz, 1H), 7.97 - 7.79 (m, 6H), 7.58 - 7.51 (m, 1H), 7.44 - 7.36 (m, 4H), 7.34 (d, *J* = 2.0 Hz, 1H), 7.21 - 7.13 (m, 2H), 5.13 (d, *J* = 3.6 Hz, 1H), 4.55 (d, *J* = 9.2 Hz, 1H), 4.47 - 4.39 (m, 2H), 4.37 - 4.32 (m, 1H), 4.21 (dd, *J* = 15.6, 5.6 Hz, 1H), 3.69 - 3.57 (m, 4H), 3.55 - 3.45 (m, 10H), 3.43 - 3.38 (m, 3H), 2.48 - 2.46 (m, 1H), 2.44 (s, 3H), 2.39 - 2.34 (m, 1H), 2.08 - 1.95 (m, 2H), 1.93 - 1.87 (m, 1H), 0.93 (s, 9H). LCMS $[M+H]^+$: 1092.3. |
| **UB-180613 (113)** | 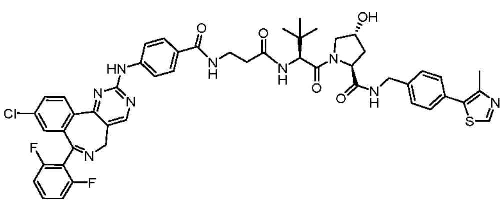 |
| | $^1$H NMR (400 MHz, DMSO-$d_6$) δ 10.15 (s, 1H), 8.97 (s, 1H), 8.72 (s, 1H), 8.57 (t, *J* = 6.1 Hz, 1H), 8.35 - 8.21 (m, 2H), 8.01 (d, *J* = 9.1 Hz, 1H), 7.91 - 7.80 (m, 5H), 7.59 - 7.49 (m, 1H), 7.42 - 7.33 (m, 5H), 7.17 (t, *J* = 8.8 Hz, 2H), 5.15 (d, *J* = 3.6 Hz, 1H), 4.56 (d, *J* = 9.2 Hz, 1H), 4.51 - 4.31 (m, 3H), 4.22 (dd, *J* = 15.9, 5.6 Hz, 1H), 3.68 (s, 2H), 3.47 (dt, *J* = 13.4, 6.9 Hz, 1H), 3.39 (m, 3H), 3.14 (m, 2H), 2.43 (s, 3H), 2.03 (d, J = 9.0 Hz, 1H), 1.91 (ddd, *J* = 13.0, 8.6, 4.6 Hz, 1H), 0.94 (s, 9H). LCMS $[M+H]^+$ =960.36 |

(continued)

| Examples | Structural data analysis |
|---|---|
| **UB-180614 (114)** | 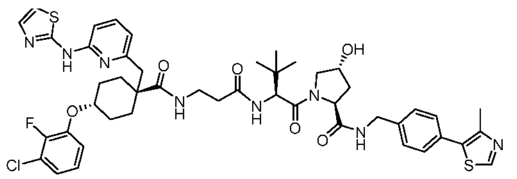 |
| | $^1$H NMR (400 MHz, DMSO-$d_6$) δ 11.12 (s, 1H), 8.98 (s, 1H), 8.56 (t, *J* = 6.1 Hz, 1H), 7.98 (d, *J* = 9.3 Hz, 1H), 7.67 - 7.49 (m, 2H), 7.45 - 7.34 (m, 5H), 7.20 - 7.04 (m, 3H), 6.95 (d, *J* = 3.6 Hz, 1H), 6.87 (d, *J* = 8.2 Hz, 1H), 6.60 (d, *J* = 7.3 Hz, 1H), 5.15 (d, *J* = 3.6 Hz, 1H), 4.54 (d, *J* = 9.0 Hz, 2H), 4.46 - 4.38 (m, 2H), 4.36 (s, 1H), 4.22 (dd, *J* = 15.8, 5.5 Hz, 1H), 3.75 - 3.56 (m, 2H), 3.24 (d, *J* = 7.0 Hz, 2H), 2.90 (s, 2H), 2.44 (s, 3H), 2.37 (t, *J* = 7.3 Hz, 2H), 2.02 (d, *J* = 8.6 Hz, 1H), 1.93 - 1.61 (m, 9H), 0.94 (s, 9H). LCMS $[M+1]^+$ =945.3 |
| **UB-180615 (115)** | 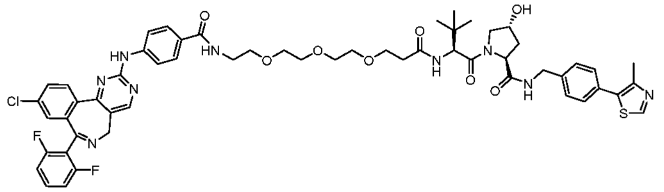 |
| | $^1$H NMR (400 MHz, DMSO-$d_6$) δ 10.16 (s, 1H), 8.97 (s, 1H), 8.72 (s, 1H), 8.56 (t, *J* = 6.0 Hz, 1H), 8.34 (t, *J* = 5.6 Hz, 1H), 8.30 (d, *J* = 8.4 Hz, 1H), 7.97 - 7.79 (m, 6H), 7.58 - 7.51 (m, 1H), 7.44 - 7.36 (m, 4H), 7.34 (d, *J* = 2.0 Hz, 1H), 7.21 - 7.13 (m, 2H), 5.13 (d, *J* = 3.6 Hz, 1H), 4.55 (d, *J* = 9.2 Hz, 1H), 4.47 - 4.39 (m, 2H), 4.37 - 4.32 (m, 1H), 4.21 (dd, *J* = 15.6, 5.6 Hz, 1H), 3.69 - 3.57 (m, 4H), 3.55 - 3.45 (m, 10H), 3.43 - 3.38 (m, 3H), 2.48 - 2.46 (m, 1H), 2.44 (s, 3H), 2.39 - 2.34 (m, 1H), 2.08 - 1.95 (m, 2H), 1.93 - 1.87 (m, 1H), 0.93 (s, 9H). LCMS $[M+H]^+$: 1092.3. |
| **UB-180616 (116)** | 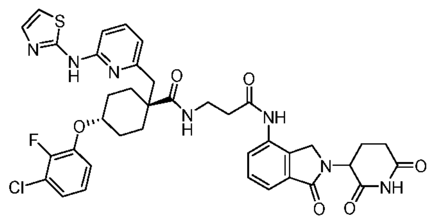 |
| | $^1$H NMR (400 MHz, DMSO-$d_6$) δ 11.12 (s, 1H), 11.02 (s, 1H), 9.86 (s, 1H), 7.85 - 7.75 (m, 2H), 7.57 - 7.44 (m, 3H), 7.36 (d, *J* = 3.6 Hz, 1H), 7.09 (d, *J* = 4.3 Hz, 3H), 6.95 (d, *J* = 3.6 Hz, 1H), 6.85 (d, *J* = 8.2 Hz, 1H), 6.61 (d, *J* = 7.4 Hz, 1H), 5.14 (dd, *J* = 13.3, 5.1 Hz, 1H), 4.47 (s, 1H), 4.35 (s, 2H), 2.91 (m, 3H), 2.61 - 2.54 (m, 3H), 2.37 - 2.23 (m, 2H), 2.08 - 1.57 (m, 10H). LCMS $[M+H]^+$ =774.2 |
| **UB-180617 (117)** | 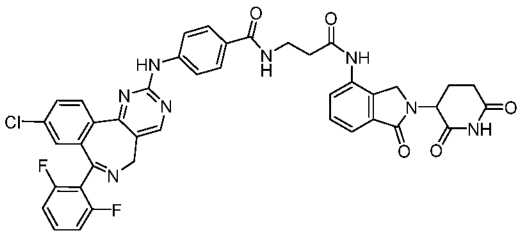 |
| | $^1$H NMR (400 MHz, DMSO-$d_6$) δ 11.01 (s, 1H), 10.16 (s, 1H), 9.93 (s, 1H), 8.73 (s, 1H), 8.48 (t, *J* = 5.7 Hz, 1H), 8.30 (d, *J* = 8.5 Hz, 1H), 7.91 - 7.79 (m, 6H), 7.62 - 7.43 (m, 3H), 7.34 (d, *J* = 2.2 Hz, 1H), 7.17 (t, *J* = 8.7 Hz, 2H), 5.11 (dd, *J* = 13.3, 5.1 Hz, 1H), 4.33 (s, 2H), 4.06 - 3.94 (m, 1H), 3.67 - 3.52 (m, 2H), 2.98 - 2.82 (m, 1H), 2.73 - 2.63 (m, 2H), 2.36 - 2.19 (m, 2H), 1.98 (d, *J* = 5.4 Hz, 2H). LCMS $[M+H]^+$ =789.2 |

(continued)

| Examples | Structural data analysis |
|---|---|
| **UB-180726 (226)** | 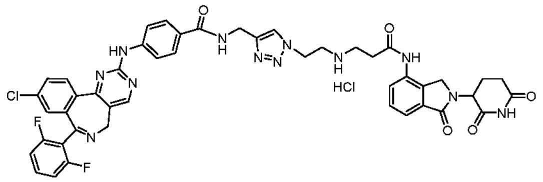 |
| | $^1H$ NMR (400 MHz, DMSO-$d_6$) δ 11.03 (s, 1H), 10.21 (d, J = 12.0 Hz, 2H), 9.17 (s, 2H), 8.92 (t, J = 5.8 Hz, 1H), 8.73 (s, 1H), 8.33 - 8.25 (m, 1H),8.09 (s, 1H), 7.94 - 7.80 (m, 7H), 7.59 - 7.47 (m, 3H), 7.34 (d, J = 2.1 Hz, 1H), 7.17 (t, J = 8.6 Hz, 2H), 5.16 (dd, J = 13.3, 5.2 Hz, 1H), 4.73 (t, J = 6.4Hz, 2H), 4.53 (d, J = 5.6 Hz, 2H), 4.49 - 4.33 (m, 2H), 3.52 (d, J = 6.2 Hz, 2H), 3.32 - 3.21 (m, 2H), 2.98 - 2.86 (m, 3H), 2.67 - 2.58 (m, 1H), 2.32 (dt,J = 13.6, 6.7 Hz, 1H), 2.09 - 1.98 (m, 1H).LCMS $[M+H]^+$ = 913.3 |
| **UB-180727 (227)** | 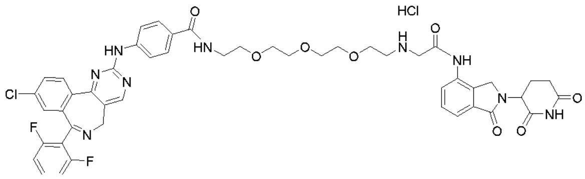 - |
| | $^1H$ NMR (400 MHz, DMSO-*d6*) δ 11.05 (s, 1H), 10.73 (s, 1H), 10.18 (s, 1H), 9.15 (s, 2H), 8.73 (s, 1H), 8.40 (t, J = 5.7 Hz, 1H), 8.30 (d, J = 8.5 Hz, 1H), 7.97 -7.78 (m, 7H), 7.64 - 7.49 (m, 3H), 7.35 (d, J = 2.3 Hz, 1H), 7.17 (t, J = 8.8 Hz, 2H), 4.52 - 4.31 (m, 2H), 4.05 (t, J = 5.5 Hz, 2H), 3.73 (t, J = 5.1 Hz, 2H), 3.55 (t,J = 8.9 Hz, 11H), 3.41 (q, J = 5.8 Hz, 2H), 3.26 - 3.17 (m, 2H), 2.93 (ddd, *J* = 18.8, 11.2, 4.5 Hz, 1H), 2.65 (s, 1H), 2.37 - 2.24 (m, 1H), 2.11 - 2.00 (m, 1H).LCMS $[M+H]^+$ =950.2 |
| **UB-180728 (228)** | 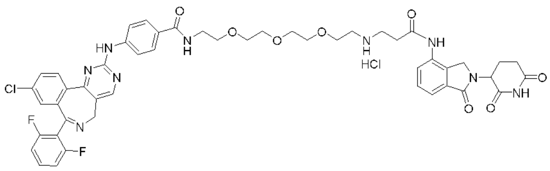 |
| | $^1H$ NMR (400 MHz, DMSO-*d6*) δ 11.03 (s, 1H), 10.20 (d, J = 16.5 Hz, 2H), 8.87 (s, 2H), 8.73 (s, 1H), 8.39 (t, J = 5.7 Hz, 1H), 8.30 (d, J = 8.5 Hz,1H), 7.96 - 7.79 (m, 6H), 7.62 - 7.46 (m, 3H), 7.34 (d, J = 2.2 Hz, 1H), 7.17 (t, J = 8.8 Hz, 2H), 4.51 - 4.32 (m, 2H), 3.70 (t, J = 5.1 Hz, 2H), 3.63 -3.49 (m, 11H), 3.41 (q, J = 6.0 Hz, 2H), 3.24 (d, J = 7.2 Hz, 2H), 3.14 (s, 2H), 2.90 (dt, J = 9.4, 6.4 Hz, 3H), 2.63 (s, 1H), 2.38 - 2.26 (m, 1H), 2.09 -1.99 (m, 1H).LCMS $[M+H]^+$ =964.2 |
| **UB-180729 (229)** | 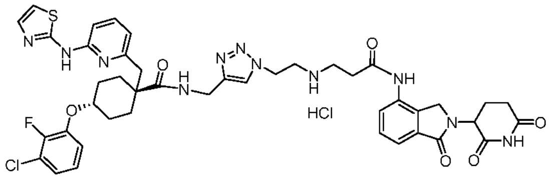 |
| | $^1H$ NMR (400 MHz, DMSO-*d6*) δ 11.77 (s, 1H), 11.03 (s, 1H), 10.25 (s, 1H), 9.29 (s, 2H), 8.29 (t, J = 5.6 Hz, 1H), 7.95 (s, 1H), 7.84 (dd, J = 7.3, 1.7 Hz, 1H), 7.59- 7.42 (m, 4H), 7.18 - 7.04 (m, 4H), 6.96 (d, J = 8.2 Hz, 1H), 6.62 (d, J = 7.4 Hz, 1H), 5.16 (dd, J = 13.2, 5.2 Hz, 1H), 4.76 (t, J = 6.4 Hz, 2H), 4.55 (s, 1H), 4.50 -4.33 (m, 2H), 4.29 (d, J = 5.4 Hz, 2H), 3.50 (s, 2H), 3.27 (s, 2H), 3.00 - 2.88 (m, 5H), 2.62 (d, J = 17.4 Hz, 1H), 2.33 (d, J = 17.6 Hz, 1H), 2.09 - 1.99 (m, 1H),1.92 (d, J = 10.9 Hz, 2H), 1.73 (dd, J = 46.8, 12.0 Hz, 6H). LCMS $[M+H]^+$ =898.2 |

(continued)

| Examples | Structural data analysis |
|---|---|
| UB-180730 (230) | 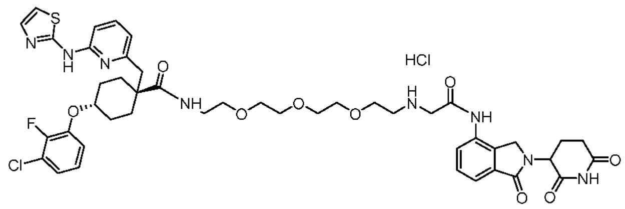 |
| | $^1$H NMR (400 MHz, DMSO-*d6*) δ 11.75 (s, 1H), 11.05 (s, 1H), 10.66 (s, 1H), 9.11 (s, 2H), 7.87 (dd, J = 7.5, 1.6 Hz, 1H), 7.71 (d, J = 5.7 Hz, 1H), 7.65 - 7.41(m, 4H), 7.20 - 7.05 (m, 4H), 6.97 (d, J = 8.2 Hz, 1H), 6.73 (d, J = 7.4 Hz, 1H), 5.18 (dd, J = 13.2, 5.1 Hz, 1H), 4.54 (s, 1H), 4.50 - 4.33 (m, 2H), 4.05 (s, 2H),3.71 (d, J = 5.1 Hz, 2H), 3.51 (dq, J = 13.1, 5.2, 4.4 Hz, 5H), 3.35 (t, J = 6.2 Hz, 2H), 3.19 (d, J = 12.4 Hz, 4H), 3.00 - 2.89 (m, 3H), 2.64 (d, J = 3.7 Hz, 1H),2.34 - 2.25 (m, 1H), 2.13 - 2.00 (m, 1H), 1.96 - 1.56 (m, 8H).LCMS $[M+H]^+$ =935.2 |
| UB-180731 (231) | 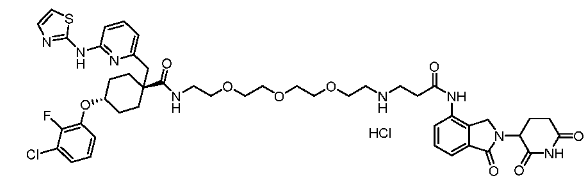 |
| | $^1$H NMR (400 MHz, DMSO-*d6*) δ 11.95 (s, 1H), 11.03 (s, 1H), 10.25 (s, 1H), 8.95 (s, 2H), 7.85 (dd, J = 7.3, 1.7 Hz, 1H), 7.72 (t, J = 5.7 Hz, 1H), 7.65 (t, J =7.8 Hz, 1H), 7.55 - 7.45 (m, 3H), 7.20 - 7.06 (m, 4H), 7.00 (d, J = 8.2 Hz, 1H), 6.76 (d, J = 7.4 Hz, 1H), 5.16 (dd, J = 13.2, 5.1 Hz, 1H), 4.55 (s, 1H), 4.51 -4.36 (m, 2H), 3.70 (t, J = 5.2 Hz, 2H), 3.54 - 3.43 (m, 5H), 3.35 (t, J = 6.2 Hz, 2H), 3.28 - 3.10 (m, 6H), 2.93 (dt, J = 19.0, 6.8 Hz, 5H), 2.63 (d, J = 4.0 Hz, 1H),2.36 - 2.27 (m, 1H), 2.09 - 1.98 (m, 1H), 1.90 (d, J = 12.5 Hz, 2H), 1.85 - 1.57 (m, 6H).LCMS $[M+H]^+$ =949.3 |
| UB-180732 (232) | 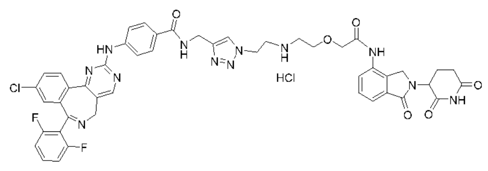 |
| | $^1$H NMR (400 MHz, DMSO-*d6*) δ 11.00 (s, 1H), 10.18 (d, J = 14.5 Hz, 2H), 9.52 (s, 2H), 8.91 (t, J = 5.8 Hz, 1H), 8.74 (d, J = 2.4 Hz, 1H), 8.30 (d, J =8.5 Hz, 1H), 8.10 (s, 1H), 7.94 - 7.85 (m, 5H), 7.75 (dd, J = 7.8, 1.2 Hz, 1H), 7.60 - 7.49 (m, 3H), 7.35 (d, J = 2.1 Hz, 1H), 7.17 (t, J = 8.7 Hz, 2H), 5.14 (dd, J = 13.3, 5.1 Hz, 1H), 4.80 (t, J = 6.6 Hz, 2H), 4.55 - 4.33 (m, 4H), 4.20 (s, 2H), 3.82 (t, J = 4.9 Hz, 2H), 3.59 - 3.49 (m, 2H), 3.22 (s, 2H), 2.91(ddd, J = 17.9, 13.5, 5.4 Hz, 1H), 2.62 (s, 1H), 2.37 (dd, J = 13.4, 4.7 Hz, 1H), 2.07 - 1.94 (m, 1H).LCMS $[M+H]^+$ =943.4 |
| UB-180735 (235) | 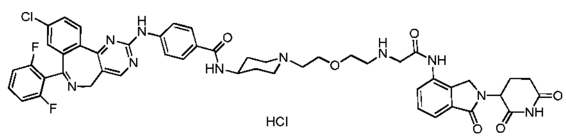 |
| | 1H NMR (400 MHz, DMSO-d6) δ 11.04 (s, 1H), 10.75 (s, 1H), 10.41 (s, 1H), 10.18 (d, J = 4.7 Hz, 1H), 9.40 (s, 2H), 8.73 (s, 1H), 8.41 (d, J = 7.2 Hz, 1H), 8.30 (d, J = 8.5 Hz, 1H), 7.94 - 7.88 (m, 5H), 7.62 - 7.48 (m, 3H), 7.35 (d, J = 2.1 Hz, 1H), 7.17 (t, J = 8.8 Hz, 2H), 5.17 (dd, J = 13.3, 5.1 Hz, 1H), 4.56 - 4.33 (m, 2H), 4.10 (s, 2H), 4.02 (s, 1H), 3.84 (s, 2H), 3.78 (d, J = 4.9 Hz, 2H), 3.66 (d, J = 11.4 Hz, 1H), 3.62 - 3.49 (m, 2H), 3.39 (s, 1H), 3.29 (d, J = 14.6 Hz, 4H), 3.21 - 3.06 (m, 2H), 3.01 - 2.85 (m, 1H), 2.71 - 2.61 (m, 1H), 2.36 - 2.22 (m, 1H), 2.05 (d, J = 23.7 Hz, 5H). LCMS [M+H]+ =945.1 |

(continued)

| Examples | Structural data analysis |
| --- | --- |
| **UB-180736 (236)** | 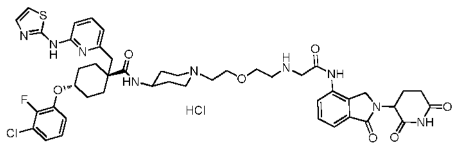 |
| | $^1$H NMR (400 MHz, DMSO-$d_6$) δ 11.62 (s, 1H), 11.05 (s, 1H), 10.77 (s, 1H), 10.41 (s, 1H), 9.39 (s, 2H), 7.89 (dd, *J* = 7.6, 1.5 Hz, 1H), 7.75 (d, *J* = 7.2 Hz, 1H), 7.63 - 7.50 (m, 3H), 7.42 (d, *J* = 3.7 Hz, 1H), 7.18 - 7.09 (m, 2H), 7.05 (d, *J* = 3.8 Hz, 1H), 6.94 (d, *J* = 8.1 Hz, 1H), 6.67 (d, *J* = 7.3 Hz, 1H), 5.18 (dd, *J* = 13.2, 5.1 Hz, 1H), 4.62 - 4.33 (m, 5H), 4.09 (s, 2H), 3.78 (dd, *J* = 23.7, 5.9 Hz, 4H), 3.54 (d, *J* = 12.0 Hz, 2H), 3.26 (s, 4H), 3.15 - 2.87 (m, 4H), 2.72 - 2.58 (m, 1H), 2.38 - 2.23 (m, 1H), 2.12 - 1.88 (m, 5H), 1.79 (d, *J* = 13.8 Hz, 5H), 1.63 (d, *J* = 13.0 Hz, 2H). LCMS [M+H]$^+$ =930.3 |
| **UB-180738 (238)** | 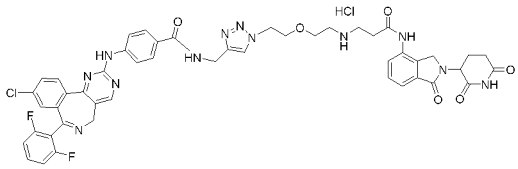 |
| | $^1$H NMR (400 MHz, DMSO-*d6*) δ 11.02 (s, 1H), 10.28 (s, 1H), 10.18 (s, 1H), 9.04 (s, 2H), 8.93 (t, J = 5.7 Hz, 1H), 8.72 (s, 1H), 8.31 - 8.25 (m, 1H), 8.07(s, 1H), 7.93 - 7.81 (m, 7H), 7.62 - 7.44 (m, 3H), 7.35 (d, J = 2.2 Hz, 1H), 7.17 (t, J = 8.7 Hz, 2H), 5.16 (d, J = 5.1 Hz, 1H), 4.56 (t, J = 5.1 Hz, 2H), 4.51 (d, J = 5.5 Hz, 2H), 4.46 - 4.33 (m, 2H), 3.86 (t, J = 5.2 Hz, 2H), 3.72 (t, J = 5.2 Hz, 2H), 3.16 (dt, J = 31.0, 5.8 Hz, 4H), 2.93 - 2.89 (m, 2H), 2.60 (d, J =17.1 Hz, 1H), 2.35 - 2.27 (m, 1H), 2.07 - 1.98 (m, 1H).LCMS [M+H]$^+$ =957.2 |
| **UB-180739 (239)** | 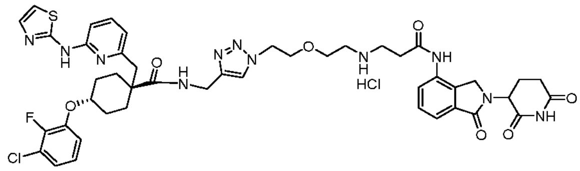 |
| | $^1$H NMR (400 MHz, DMSO-*d6*) δ 12.08 (s, 1H), 10.32 - 10.10 (m, 1H), 9.00 (s, 2H), 8.30 (s, 1H), 7.98 - 7.83 (m, 2H), 7.64 - 7.47 (m, 4H), 7.13 (dtd, J =23.0, 7.9, 7.1, 4.7 Hz, 4H), 7.00 (d, J = 8.2 Hz, 1H), 6.65 (d, J = 7.4 Hz, 1H), 5.15 (dd, J = 13.3, 5.1 Hz, 1H), 4.78 (dd, J = 10.3, 4.8 Hz, 1H), 4.57 (t, J =6.6 Hz, 3H), 4.44 (d, J = 8.0 Hz, 1H), 3.87 (d, J = 5.5 Hz, 2H), 3.71 (d, J = 5.4 Hz, 2H), 3.21 (s, 2H), 3.12 (s, 2H), 3.02 - 2.88 (m, 5H), 2.63 (s, 1H), 2.28(dd, J = 8.0, 5.5 Hz, 2H), 1.92 (s, 2H), 1.85 - 1.59 (m, 7H).LCMS [M+H]$^+$ =942.3 |
| **UB-180740 (240)** | 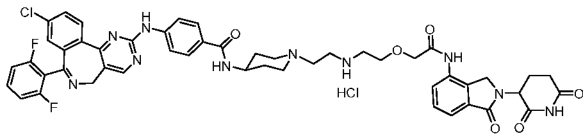 |
| | $^1$H NMR (400 MHz, DMSO-*d6*) δ 11.01 (s, 1H), 10.77 (s, 1H), 10.16 (dd, J = 17.7, 7.1 Hz, 2H), 9.75 (s, 2H), 8.74 (s, 1H), 8.43 (d, J = 7.5 Hz, 1H), 8.30(d, J = 8.5 Hz, 1H), 7.94 - 7.84 (m, 4H), 7.75 (dd, J = 7.9, 1.2 Hz, 1H), 7.65 - 7.46 (m, 3H), 7.34 (d, J = 2.1 Hz, 1H), 7.17 (t, J = 8.8 Hz, 2H), 5.15 (dd, J =13.3, 5.1 Hz, 1H), 4.59 - 4.35 (m, 2H), 4.22 (s, 2H), 4.07 (s, 1H), 3.87 (t, J = 4.8 Hz, 2H), 3.29 (s, 2H), 3.16 (d, J = 11.0 Hz, 2H), 2.92 (ddd, J = 17.9, 13.5,5.4 Hz, 1H), 2.62 (d, J = 3.8 Hz, 1H), 2.42 - 2.33 (m, 1H), 2.02 (s, 4H).LCMS [M+H]$^+$ =945.2 |

(continued)

| Examples | Structural data analysis |
| --- | --- |
| **UB-180741 (241)** | 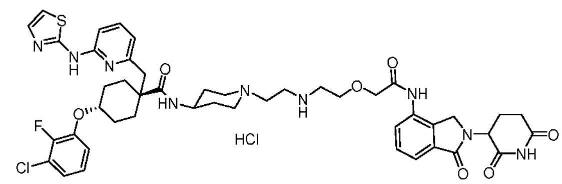 |
| | $^1$H NMR (400 MHz, DMSO-*d6*) δ 11.47 (s, 1H), 11.01 (s, 1H), 10.74 (s, 1H), 10.12 (s, 1H), 9.76 (s, 2H), 7.73 (t, J = 8.8 Hz, 2H), 7.55 (dt, J = 24.3, 7.6 Hz, 3H),7.40 (d, J = 3.8 Hz, 1H), 7.20 - 7.08 (m, 3H), 7.02 (d, J = 3.7 Hz, 1H), 6.93 (d, J = 8.2 Hz, 1H), 6.65 (d, J = 7.3 Hz, 1H), 5.15 (dd, J = 13.2, 5.2 Hz, 1H), 4.58 -4.36 (m, 3H), 4.21 (s, 2H), 3.98 - 3.79 (m, 7H), 3.58 (d, J = 11.7 Hz, 3H), 3.27 (s, 2H), 3.08 (t, J = 12.4 Hz, 2H), 2.91 (d, J = 24.1 Hz, 3H), 2.62 (s, 1H), 2.38 (d,J = 4.5 Hz, 1H), 2.06 - 1.72 (m, 10H), 1.65 (d, J = 13.2 Hz, 2H).LCMS $[M+H]^+$ = 930.1 |
| **UB-180742 (242)** | 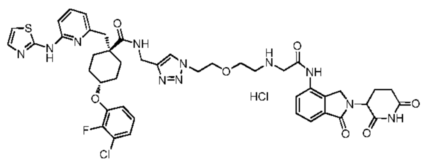 |
| | $^1$H NMR (400 MHz, DMSO-$d_6$) δ 11.82 (s, 1H), 11.04 (s, 1H), 10.73 (d, *J* = 12.4 Hz, 1H), 9.19 (s, 2H), 8.29 (s, 1H), 7.92 (d, *J* = 1.5 Hz, 1H), 7.87 (dd, *J* = 7.7, 1.4 Hz, 1H), 7.63 -7.35 (m, 4H), 7.11 (dtd, *J* = 13.4, 6.5, 3.0 Hz, 4H), 6.95 (d, *J* = 8.6 Hz, 1H), 6.58 (d, *J* = 8.3 Hz, 1H), 5.17 (dd, *J* = 13.2, 5.1 Hz, 1H), 4.58 (t, *J* = 5.3 Hz, 2H), 4.52 - 4.33 (m, 2H), 4.27 (d, *J* = 5.5 Hz, 2H), 4.04 (s, 2H), 3.85 (t, *J* = 5.3 Hz, 2H), 3.72 (t, *J* = 5.0 Hz, 2H), 3.19 (s, 2H), 3.01 - 2.83 (m, 3H), 2.64 (dd, *J* = 16.8, 12.8 Hz, 1H), 2.36 - 2.16 (m, 2H), 2.05 (dd, *J* = 9.7, 4.4 Hz, 1H), 1.95 -1.90 (m, 2H), 1.76 (t, *J* = 11.8 Hz, 4H), 1.65 (d, *J* = 13.3 Hz, 2H). LCMS $[M+H]^+$=928.1 |
| **UB-180743 (243)** | 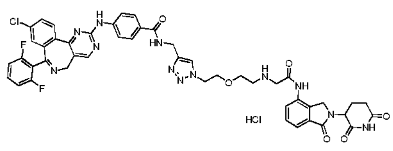 |
| | $^1$H NMR (400 MHz, DMSO-$d_6$) δ 11.04 (s, 1H), 10.66 (s, 1H), 10.18 (s, 1H), 9.14 (s, 2H), 8.92 (t, *J* = 5.8 Hz, 1H), 8.72 (s, 1H), 8.28 (d, *J* = 8.5 Hz, 1H), 8.06 (s, 1H), 7.88 (dd, *J* = 8.5, 2.2 Hz, 6H), 7.67 - 7.45 (m, 3H), 7.34 (d, *J* = 2.2 Hz, 1H), 7.17 (t, *J* = 8.9 Hz, 2H), 5.17 (dd, *J* = 13.3, 5.2 Hz, 1H), 4.57 (t, *J* = 5.2 Hz, 2H), 4.49 (t, *J* = 4.8 Hz, 2H), 4.46 - 4.32 (m, 2H), 4.03 (t, *J* = 5.6 Hz, 2H), 3.86 (t, *J* = 5.2 Hz, 2H), 3.72 (t, *J* = 5.0 Hz, 2H), 3.20 (d, *J* = 7.6 Hz, 2H), 3.04 - 2.83 (m, 1H), 2.75 - 2.55 (m, 2H), 2.36 - 2.17 (m, 2H), 2.11 - 1.95 (m, 1H). LCMS $[M+H]^+$ =943.1 |
| **UB-180744 (244)** | 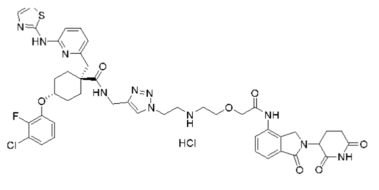 |
| | $^1$H NMR (400 MHz, DMSO-*d6*) δ 11.84 (s, 1H), 11.00 (s, 1H), 10.13 (s, 1H), 9.48 (s, 2H), 8.27 (t, J = 5.7 Hz, 1H), 7.95 (s, 1H), 7.75 (dd, J = 7.8, 1.2 Hz,1H), 7.62 - 7.43 (m, 4H), 7.18 -7.05 (m, 4H), 6.97 (d, J = 8.2 Hz, 1H), 6.63 (d, J = 7.4 Hz, 1H), 5.14 (dd, J = 13.3, 5.1 Hz, 1H), 4.80 (t, J = 6.6 Hz, 2H), 4.54(s, 1H), 4.52 - 4.35 (m, 2H), 4.28 (d, J = 5.4 Hz, 2H), 4.20 (s, 3H), 3.82 (t, J = 4.9 Hz, 2H), 3.53 (s, 2H), 3.23 (s, 2H), 3.03 - 2.83 (m, 3H), 2.61 (d, J = 3.8 Hz,1H), 2.38 - 2.28 (m, 1H), 2.06 - 1.58 (m, 9H)LCMS $[M+H]^+$ = 928.1 |

(continued)

| Examples | Structural data analysis |
|---|---|
| **UB-180748 (248)** | 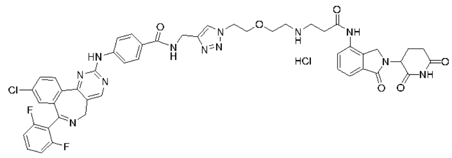 |
| | $^1$H NMR (400 MHz, DMSO-*d6*) δ 11.02 (s, 1H), 10.19 (s, 1H), 10.05 (s, 1H), 9.11 (s, 2H), 9.01 (s, 1H), 8.92 (t, J = 6.1 Hz, 1H), 8.73 (d, J = 1.1 Hz, 1H), 8.29 (d,J = 8.5 Hz, 1H), 8.06 (d, J = 2.6 Hz, 1H), 7.94 - 7.85 (m, 7H), 7.62 - 7.47 (m, 3H), 7.34 (d, J = 2.2 Hz, 1H), 7.17 (t, J = 8.8 Hz, 3H), 5.15 (dd, J = 13.3, 5.1 Hz,1H), 4.70 (td, J = 6.6, 2.6 Hz, 2H), 4.52 (dd, J = 5.8, 3.4 Hz, 2H), 4.40 (q, J = 17.5 Hz, 2H), 3.77 (t, J = 6.2 Hz, 2H), 3.70 (t, J = 5.2 Hz, 2H), 3.67 - 3.63 (m, 2H),3.50 - 3.46 (m, 2H), 3.15 (s, 2H), 2.92 (ddd, J = 17.2, 13.6, 5.3 Hz, 1H), 2.70 (t, J = 6.3 Hz, 2H), 2.61 (d, J = 17.7 Hz, 1H), 2.36 - 2.29 (m, 1H), 2.11 - 1.98 (m,1H).LCMS [M+H]$^+$= 957.1 |
| **UB-180749 (249)** | 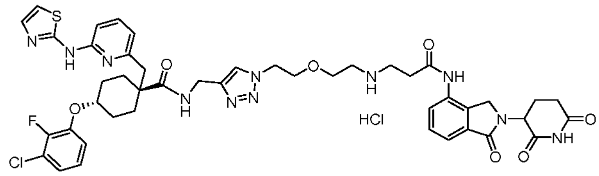 |
| | $^1$H NMR (400 MHz, DMSO-*d6*) δ 12.30 (s, 1H), 11.02 (s, 1H), 10.12 (s, 1H), 9.27 (d, J = 36.0 Hz, 3H), 8.30 (dd, J = 8.3, 3.4 Hz, 1H), 7.93 (d, J = 1.9 Hz, 1H),7.85 (dd, J = 7.2, 1.7 Hz, 1H), 7.60 (t, J = 7.8 Hz, 1H), 7.56 - 7.47 (m, 3H), 7.16 (td, J = 4.1, 2.4 Hz, 2H), 7.13 - 7.04 (m, 4H), 6.71 (d, J = 7.4 Hz, 1H), 5.15(dd, J = 13.3, 5.1 Hz, 1H), 4.76 - 4.73 (m, 2H), 4.56 (d, J = 4.1 Hz, 2H), 4.41 (d, J = 21.4 Hz, 2H), 4.27 (d, J = 5.1 Hz, 2H), 3.77 (t, J = 6.2 Hz, 2H), 3.73 (t, J =5.2 Hz, 2H), 3.15 (s, 2H), 2.61 (d, J = 17.6 Hz, 1H), 2.33 (dd, J = 19.6, 6.2 Hz, 1H), 2.07 - 1.64 (m, 12H).LCMS [M+H]$^+$ = 942.1 |
| **UB-180761 (261)** | 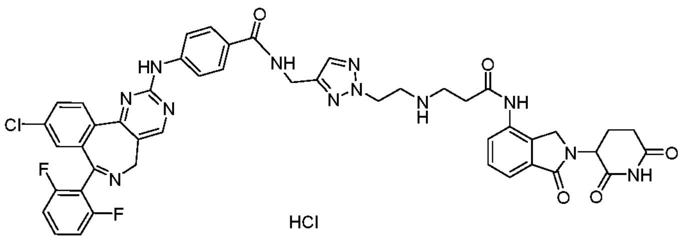 |
| | $^1$H NMR (400 MHz, DMSO-$d_6$) δ 11.03 (s, 1H), 10.30 (s, 1H), 10.20 (s, 1H), 9.41 (s, 2H), 8.94 (t, *J* = 5.5 Hz, 1H), 8.74 (d, *J* = 9.2 Hz, 1H), 8.31 (t, *J* = 8.4 Hz, 1H), 8.13 -7.77 (m, 6H), 7.74 (s, 1H), 7.67 - 7.37 (m, 3H), 7.36 - 7.02 (m, 3H), 5.17 - 5.08 (m, 1H), 4.79 (t, *J* = 6.3 Hz, 2H), 4.55 - 4.29 (m, 4H), 3.53 (dd, *J* = 6.1, 1.6 Hz, 2H), 3.27 (s, 2H), 2.87 (dt, *J* = 8.8, 4.5 Hz, 3H), 2.61 (d, *J* = 16.9 Hz, 1H), 2.56 - 2.47 (m, 1H), 2.47-2.14 (m, 1H), 2.14 - 1.88 (m, 1H).LCMS [M+H]$^+$ =913.2 |
| **UB-180768 (268)** | 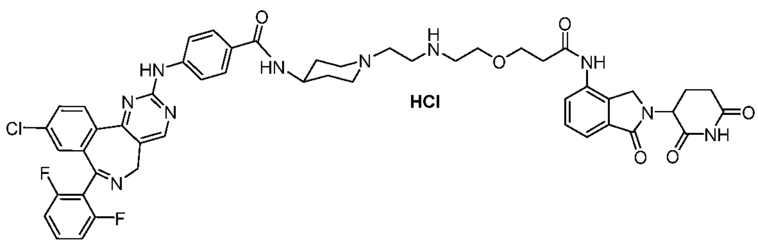 |
| | $^1$H NMR (400 MHz, DMSO-*d6*) δ 11.04 (s, 1H), 10.83 (s, 1H), 10.53 (s, 1H), 9.42 (s, 2H), 8.49 (d, J = 7.1 Hz, 1H), 8.19 (s, 1H), 7.98 - 7.82 (m, 2H),7.62 - 7.46 (m, 5H), 7.22 (t, J = 2.9 Hz, 1H), 5.27 - 5.12 (m, 2H), 4.79 (d, J = 15.4 Hz, 1H), 4.56 - 4.33 (m, 3H), 4.10 (s, 2H), 3.95 (d, J = 5.5 Hz, 3H),3.83 (d, J = 5.3 Hz, 2H), 3.77 (d, J = 6.4 Hz, 2H), 3.61 (d, J = 11.7 Hz, 2H), 3.30 (s, 2H), 3.24 (s, 3H), 3.14 (d, J = 12.0 Hz, 2H), 2.98 - 2.88 (m, 1H),2.64 (s, 1H), 2.35 - 2.27 (m, 1H), 2.05 (q, J = 20.1, 13.6 Hz, 5H), 1.88 (t, J = 6.3 Hz, 2H), 0.70 (t, J = 7.4 Hz, 3H).LCMS [M+H]$^+$ =959.4 |

(continued)

| Examples | Structural data analysis |
|---|---|
| **UB-180769 (269)** | 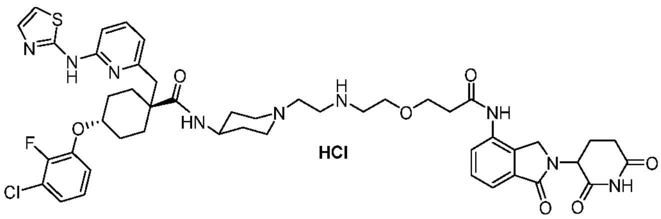 HCl |
| | $^1$H NMR (400 MHz, DMSO-*d6*) δ 11.49 (s, 1H), 11.03 (s, 1H), 10.38 (s, 1H), 10.25 (d, J = 18.6 Hz, 1H), 9.30 (s, 2H), 7.89 - 7.75 (m, 2H), 7.62 - 7.49 (m, 3H),7.43 - 7.38 (m, 1H), 7.18 - 7.09 (m, 4H), 7.02 (d, J = 3.6 Hz, 1H), 6.93 (d, J = 8.4 Hz, 1H), 6.66 (d, J = 7.3 Hz, 1H), 5.15 (dd, J = 13.3, 5.2 Hz, 1H), 4.54 (s,1H), 4.50 - 4.35 (m, 2H), 3.81 (s, 5H), 3.55 (d, J = 4.4 Hz, 2H), 3.22 (d, J = 30.1 Hz, 7H), 3.00 (d, J = 6.1 Hz, 5H), 2.63 (s, 1H), 2.31 (s, 1H), 1.98 (d, J = 21.8Hz, 5H), 1.80 (dd, J = 11.9, 5.5 Hz, 6H), 1.64 (d, J = 12.9 Hz, 2H).LCMS [M+H]$^+$ =944.4 |
| **UB-180770 (270)** | 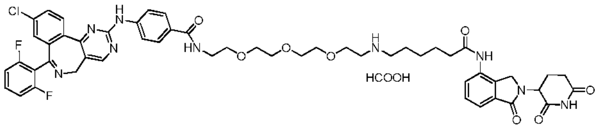 HCOOH |
| | $^1$H NMR (400 MHz, DMSO-$d_6$) δ 10.17 (s, 1H), 9.79 (s, 1H), 8.73 (d, *J* = 1.6 Hz, 1H), 8.46 - 8.19 (m, 4H), 7.96 - 7.76 (m, 8H), 7.58 - 7.45 (m, 3H), 7.34 (d, *J* = 2.1 Hz, 1H), 7.17 (t, *J* = 8.8 Hz, 2H), 5.14 (dd, *J* = 13.2, 5.1 Hz, 1H), 4.49 - 4.23 (m, 2H), 3.05 - 2.95 (m, 2H), 2.96 - 2.83 (m, 2H), 2.82 - 2.70 (m, 4H), 2.60 (tt, *J* = 6.8, 3.6 Hz, 5H), 2.40 - 2.31 (m, 2H), 2.27 - 2.18 (m, 1H), 2.08 - 1.94 (m, 2H), 1.67 - 1.54 (m, 2H), 1.52 - 1.38 (m, 5H), 1.37 - 1.20 (m, 5H), 1.04 - 0.90 (m, 2H).LCMS [M+H]$^+$ =1006.5 |
| **UB-180771 (271)** | 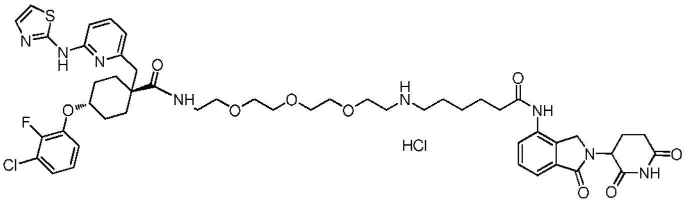 HCl<br>$^1$H NMR (400 MHz, DMSO-$d_6$) δ 11.02 (s, 1H), 9.95 (s, 1H), 8.86 (s, 2H), 7.83 (dd, *J* = 7.2, 1.9 Hz, 1H), 7.79 - 7.67 (m, 2H), 7.61 - 7.41 (m, 3H), 7.28 - 7.03 (m, 6H), 6.84 (d, *J* = 7.5 Hz, 1H), 5.15 (dd, *J* = 13.3, 5.1 Hz, 1H), 4.55 (s, 1H), 4.39 (q, *J* = 17.6 Hz, 2H), 3.68 (t, *J* = 5.3 Hz, 3H), 3.55 (s, 4H), 3.49 (dt, *J* = 8.8, 3.7 Hz, 4H), 3.36 (t, *J* = 6.2 Hz, 2H), 3.18 (d, *J* = 5.8 Hz, 2H), 3.06 (t, *J* = 5.7 Hz, 2H), 2.97 (s, 2H), 2.90 (dd, *J* = 11.5, 4.6 Hz, 3H), 2.69 - 2.58 (m, 1H), 2.40 (t, *J* = 7.4 Hz, 2H), 1.99 - 1.87 (m, 2H), 1.77 (q, *J* = 13.6, 12.5 Hz, 4H), 1.70 - 1.56 (m, 6H), 1.35 (t, *J* = 7.5 Hz, 3H). LCMS [M+H]$^+$=991.5 |
| **UB-180772 (272)** | 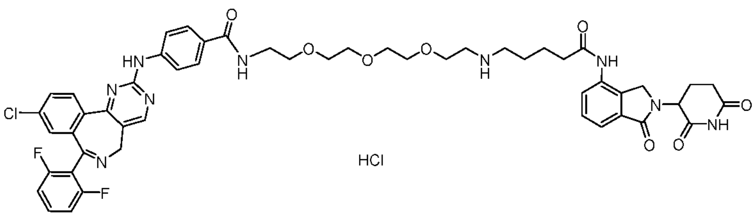 HCl |
| | $^1$H NMR (400 MHz, DMSO-$d_6$) δ 11.03 (s, H), 10.30 (s, 1H), 10.20 (s, 1H), 9.41 (s,2H), 8.94 (t, *J* = 5.5 Hz, 1H), 8.74 (d, *J* = 9.2 Hz, 1H), 8.31 (t, *J* = 8.4 Hz, 1H), 8.13 - 7.77 (m, 6H), 7.67 - 7.37 (m, 3H), 7.36 - 7.02 (m,3H), 5.17 - 5.08 (m, 1H), 4.45 - 4.29 (m, 2H), 3.75 - 3.44 (m, 15H), 3.43-3.27 (m, 3H), 2.87 (dt, *J* = 8.8, 4.5 Hz, 4H), 2.61 (d, *J* = 16.9 Hz, 1H), 2.47 - 2.14 (m, 2H), 2.14 - 1.88 (m, 2H), 1.67 (s, 4H).LCMS [M+ H]$^+$ =992.5 |

(continued)

| Examples | Structural data analysis |
|---|---|
| **UB-180773 (273)** | 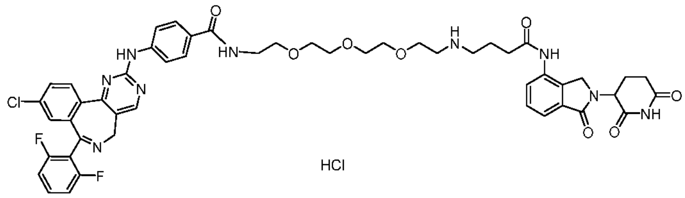 |
| | $^1$H NMR (400 MHz, DMSO-$d_6$) δ11.03 (s, H), 10.30 (s, 1H), 10.20 (s, 1H),8.73 (s, 1H), 8.66 (s, 2H), 8.33 (dd, *J* = 2.6, 7.0 Hz, 2H), 7.98 - 7.77 (m, 6H), 7.63 - 7.43 (m, 3H), 7.17 (s,1H), 5.15 (dd, *J* = 13.3, 5.2 Hz, 1H), 4.38 (dd, *J* = 3.2, 1.8 Hz,2H), 3.94 (s, 3H), 3.94 - 3.29 (m, 20H), 3.29 - 2.87 (m, 5H), 2.89 (s, 2H), 2.77 - 2.61 (m, 1H), 2.32 (dd, *J* = 7.5, 5.7 Hz, 1H), 1.95 (d, *J* = 7.9 Hz, 1H), 1.24 (ddd, *J* = 3.3, 1.9, 7.0 Hz, 2H).LCMS $[M+H]^+$ =978.4 |
| **UB-180774 (274)** | 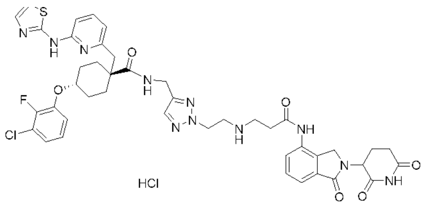 |
| | $^1$H NMR (400 MHz, DMSO-$d_6$) 11.03 (s, 1H), 10.25 (s, 1H), 9.29 (s, 2H), 8.29 (s, 1H), 7.96 (d, *J* = 10.8 Hz, 1H), 7.92 - 7.79 (m,1H), 7.67 - 7.24 (m, 5H), 7.28 - 6.96 (m, 5H), 6.95 (s, 1H), 6.62 (d, *J* = 7.4 Hz, 1H), 5.16 (dd, *J* = 13.3, 5.1 Hz, 1H), 4.76 (t, *J* = 6.4 Hz, 2H), 4.67 - 4.40 (m, 5H), 3.43 (dd, *J* = 8.7, 2.7 Hz, 2H), 3.28 (s, 2H), 3.10 - 2.77 (m, 5H), 2.68 - 2.57 (m,1H), 2.20 - 2.00 (m, 2H), 2.00 - 1.51 (m, 9H). LCMS $[M+H]^+$ =990.2. |
| **UB-180775 (275)** | 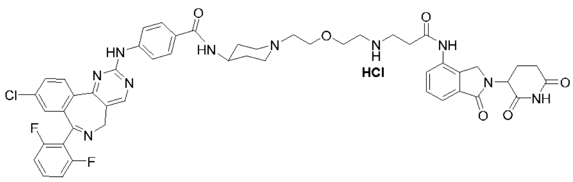 |
| | $^1$H NMR (400 MHz, DMSO-*d6*) δ 11.04 (s, 1H), 10.75 (s, 1H), 10.41 (s, 1H), 10.18 (d, J = 4.7 Hz, 1H), 9.40 (s, 2H), 8.73 (s, 1H), 8.41 (d, J = 7.2 Hz, 1H),8.30 (dd, J = 8.5, 2.8 Hz, 1H), 7.96 - 7.81 (m, 6H), 7.62 - 7.51 (m, 3H), 7.35 (d, J = 2.1 Hz, 1H), 7.17 (t, J = 8.8 Hz, 2H), 5.17 (dd, J = 13.3, 5.2 Hz, 4H), 4.55- 4.34 (m, 3H), 4.06 (d, J = 30.6 Hz, 4H), 3.89 - 3.73 (m, 5H), 3.66 (d, J = 11.4 Hz, 1H), 3.58 (dd, J = 14.2, 8.7 Hz, 2H), 3.39 (s, 1H), 3.29 (d, J = 14.3 Hz,4H), 3.19 - 3.07 (m, 2H), 2.99 - 2.87 (m, 1H), 2.64 (s, 1H), 2.30 (d, J = 4.1 Hz, 2H), 2.05 (q, J = 13.7 Hz, 5H).LCMS $[M+H]^+$ =959.4 |
| **UB-180776 (276)** | 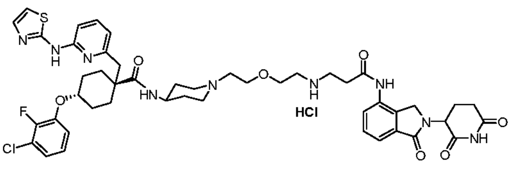<br>$^1$H NMR (400 MHz, DMSO-$d_6$) δ 11.01 (s, 1H), 10.77 (s, 1H), 10.16 (dd, *J* = 17.7, 7.1 Hz, 2H), 9.75 (s, 2H), 8.74 (s, 1H), 8.43 (d, J = 7.5 Hz, 1H), 8.30 (d, J =8.5 Hz, 1H), 7.98 - 7.83 (m, 5H), 7.75 (dd, J = 7.9, 1.2 Hz, 1H), 7.62 - 7.48 (m, 3H), 7.34 (d, J = 2.1 Hz, 1H), 7.17 (t, J = 8.8 Hz, 2H), 5.15 (dd, J = 13.3, 5.1 Hz,1H), 4.61 - 4.32 (m, 3H), 4.22 (s, 2H), 4.07 (s, 1H), 3.87 (t, J = 4.8 Hz, 3H), 3.29 (s, 3H), 3.16 (d, J = 11.0 Hz, 2H), 2.92 (ddd, J = 17.9, 13.5, 5.4 Hz, 1H), 2.62 (d,J = 3.8 Hz, 1H), 2.42 - 2.33 (m, 1H), 2.05 (d, J = 25.8 Hz, 5H), 1.45 - 1.18 (m, 1H). LCMS $[M+H]^+$=944.5 |

(continued)

| Examples | Structural data analysis |
|---|---|
| **UB-180777 (277)** | 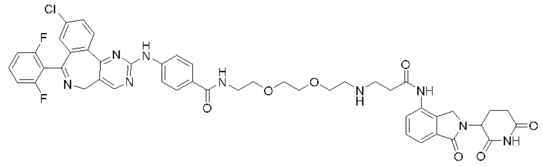 |
| | $^1$H NMR (400 MHz, DMSO-$d_6$) δ 11.03 (s, 1H), 10.25 (s, 1H), 10.17 (s, 1H), 8.72 (s, 1H), 8.40 (t, *J* = 5.6 Hz, 1H), 8.29 (d, *J* = 8.5 Hz, 1H), 7.96 - 7.81 (m, 5H), 7.63 - 7.44 (m, 3H), 7.34 (d, *J* = 2.2 Hz, 1H), 7.17 (t, *J* = 9.0 Hz, 2H), 5.32 (t, *J* = 4.8 Hz, 1H), 5.15 (dd, *J* = 13.3, 5.2 Hz, 1H), 4.47 - 4.30 (m, 3H), 3.66 (s, 2H), 3.54 (t, *J* = 6.2 Hz, 5H), 3.42 (d, *J* = 5.9 Hz, 3H), 3.16 (s, 2H), 3.04 (s, 2H), 2.81 (s, 2H), 2.67 (p, *J* = 1.9 Hz, 2H), 2.35 - 2.27 (m, 2H), 2.00 (dq, *J* = 13.3, 5.9 Hz, 3H). LCMS $[M+H]^+$ =920 |
| **UB-180778 (278)** | 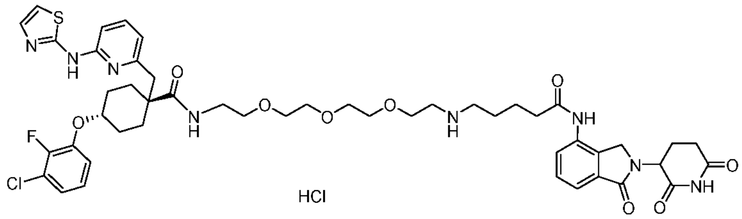 HCl |
| | $^1$H NMR (400 MHz, DMSO-$d_6$) δ12.67 (s, 1H), 11.02 (s, 1H), 10.03 (s, 7H), 8.93 (s, 2H), 7.90 - 7.63 (m, 3H), 7.58 - 7.28 (m, 3H), 7.09 (dddd, *J* = 11.5, 10.4, 1.8, 9.0 Hz, 6H), 5.35 (s, 1H), 4.63 - 4.34 (m, 5H), 3.68 (dd, *J* = 13.5, 8.3 Hz, 2H), 3.59 - 3.09 (m, 10H), 3.05 - 2.85 (m, 5H), 2.64 (dd, *J* = 2.5, 9.3 Hz, 2H), 2.46 - 2.31 (m, 4H), 2.31 - 1.99 (m, 2H), 1.93-1.49 (m, 13H).LCMS $[M+H]^+$ =977.6 |
| UB-180779 (279) | 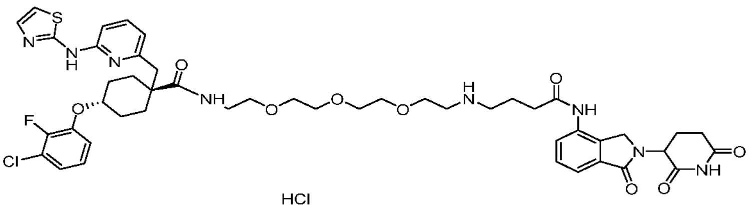 HCl |
| | $^1$H NMR (400 MHz, DMSO-$d_6$) δ11.03 (s, 1H), 10.04 (s, 1H), 8.85 (s, 1H), 7.84 (dd, *J* = 7.2, 1.5 Hz, 1H), 7.80 - 7.59 (m, 2H), 7.49 (ddd, *J* = 22.8, 11.4, 2.7 Hz, 3H), 7.29 - 7.05 (m, 4H), 6.96 (d, *J* = 8.2 Hz, 1H), 6.73 (d, *J* = 7.3 Hz, 1H), 5.15 (dd, *J* = 13.3, 5.1 Hz, 1H), 4.66 - 4.29 (m, 5H), 3.76 - 3.42 (m, 12H), 2.89 (d, *J* = 5.2 Hz, 2H), 2.72 - 2.57 (m, 3H), 2.40 - 2.25 (m, 2H), 2.16 - 1.71 (m, 12H) 1.60 (t, *J* = 3.6 Hz, 2H).LCMS $[M+H]^+$ =963.6 |
| **UB-180780 (280)** | 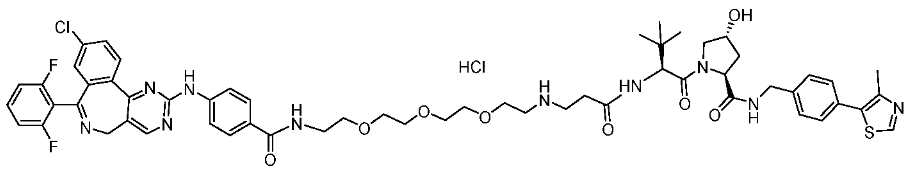 |
| | $^1$H NMR (400 MHz, DMSO-$d_6$) δ $^1$H NMR (400 MHz, DMSO) δ 10.18 (s, 1H), 9.16 - 8.66 (m, 5H), 8.43 - 8.27 (m, 3H), 7.91 - 7.79 (m, 5H), 7.47 - 7.33 (m, 6H), 7.12 (dt, *J* = 42.1, 8.5 Hz, 2H), 4.99 - 3.95 (m, 6H), 3.85 - 3.36 (m, 15H), 3.23 - 2.91 (m, 4H), 2.70 (dd, *J* = 11.4, 7.3 Hz, 3H), 2.55 - 2.42 (m, 3H), 2.13 - 2.01 (m, 2H), 0.93 (d, *J*= 10.1 Hz, 9H).LCMS $[M+H]^+$ =1135.7 |
| **UB-180781 (281)** | 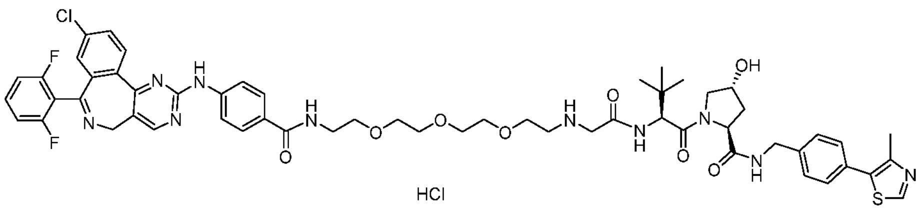 HCl |

(continued)

| Examples | Structural data analysis |
|---|---|
| | $^1$H NMR (400 MHz, DMSO-$d_6$) δ10.19 (s, 1H), 9.01 (d, *J* = 6.0 Hz, 3H), 8.67 (dd, *J* = 2.2, 1.7 Hz, 3H), 8.36 (dd, *J* = 4.7, 7.0 Hz, 2H), 7.98 - 7.75 (m,5H), 7.75 - 7.31 (m, 6H), 7.17 (t, *J* = 8.2 Hz, 2H), 4.53 (dd, *J* = 4.0, 8.1 Hz, 1H), 4.40 (dt, *J* = 6.8, 2.7 Hz, 3H), 4.25 (s, 3H), 4.28 - 3.76 (m, 5H), 3.75 - 3.19 (m, 13H), 2.45 (s, 3H), 2.14 - 2.02 (m, 2H), 0.95 (d, *J* = 9.1 Hz, 9H). LCMS [M+H]$^+$ =1121.5 |
| **UB-180782 (282)** | 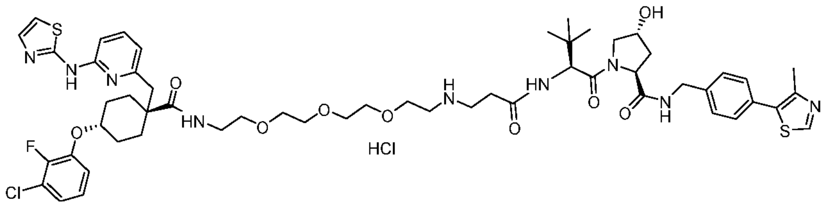 |
| | $^1$H NMR (400 MHz, DMSO-$d_6$) δ9.07 (s, 1H),8.97(s,2H),8.76-8.71(m, 1H), 8.46-8.31(m, 1H),7.75 (dd, *J* = 24.2, 16.5 Hz, 2H), 7.49 (dt, *J* = 16.3, 6.1 Hz, 5H), 7.38 - 7.09 (m, 5H), 6.85-6.77 (m, 1H), 4.55 - 4.22 (m, 2H), 4.16 - 3.90 (m,10H), 4.16 - 3.38 (m, 10H), 3.09 - 3.04 (m, 2H), 3.09 - 2.72 (m, 3H), 3.59 - 2.14 (m, 3H), 2.31 - 1.86 (m, 4H), 1.72 (d, *J* = 12.7 Hz, 6H), 1.72 (d, *J* = 12.7 Hz, 6H), 1.06 - 0.72 (m, 9H).LCMS [M+H]$^+$ =1120.6 |
| **UB-180783 (283)** | 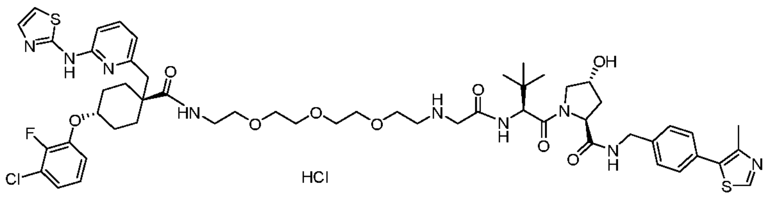 |
| | $^1$H NMR (400 MHz, DMSO-$d_6$) δ9.04 (s, 1H), 8.93 (s, 2H), 8.65 (dd, *J* = 10.5, 5.0 Hz, 2H), 7.90 - 7.64 (m, 2H), 7.54 (d, *J* = 4.0 Hz, 1H), 7.42 (dq, *J* = 16.5, 8.3 Hz,4H), 7.33 - 7.03 (m, 5H), 6.86 (d, *J* = 7.4 Hz, 1H), 5.62 (s, 1H), 4.76 - 4.44 (m, 4H), 4.44 - 4.10 (m, 2H), 4.02 - 3.56 (m, 15H), 3.56 - 3.12 (m, 6H), 3.02 (s, 2H), 2.45 (s, 3H), 2.07 (dd, *J* = 13.2, 6.1 Hz, 3H), 1.97 - 1.25 (m, 9H), 0.95 (d, *J* = 9.6 Hz, 9H).LCMS [M+H]$^+$ =1106.6 |
| **UB-180784 (284)** | 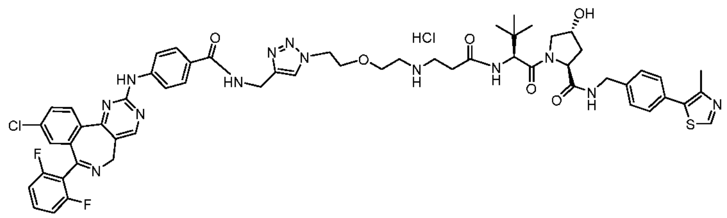 |
| | $^1$H NMR (400 MHz, DMSO-*d6*) δ 10.19 (s, 1H), 9.01 (s, 2H), 8.94 (t, J = 5.7 Hz, 1H), 8.73 (s, 1H), 8.62 (t, J = 6.1 Hz, 1H), 8.30 (t, J = 8.3 Hz, 2H), 8.05 (s,1H), 7.94 - 7.85 (m, 5H), 7.55 (tt, J = 8.3, 6.5 Hz, 1H), 7.43 - 7.33 (m, 5H), 7.17 (t, J = 8.8 Hz, 2H), 4.94 (s, 1H), 4.59 - 4.49 (m, 5H), 4.43 (q, J = 7.4 Hz, 3H),4.35 (s, 1H), 4.21 (dd, J = 15.9, 5.4 Hz, 2H), 3.84 (s, 2H), 3.68 (dt, J = 10.5, 4.7 Hz, 4H), 3.07 (d, J = 6.6 Hz, 4H), 2.72 (t, J = 7.4 Hz, 2H), 2.44 (s, 3H), 2.05 (t,J = 10.4 Hz, 1H), 1.90 (ddd, J = 13.0, 8.6, 4.7 Hz, 1H), 0.94 (s, 9H). LCMS [M+H]$^+$=1128.6 |

(continued)

| Examples | Structural data analysis |
|---|---|
| **UB-180785 (285)** | 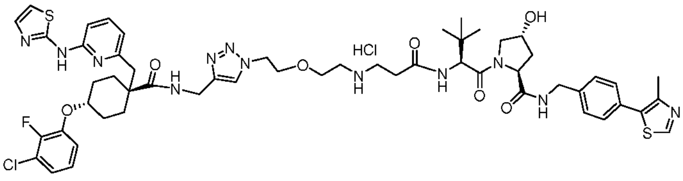 |
| | $^1$H NMR (400 MHz, DMSO-*d6*) δ 12.24 (s, 1H), 9.16 (d, J = 25.5 Hz, 2H), 9.02 (s, 1H), 8.63 (t, J = 6.1 Hz, 1H), 8.40 - 8.25 (m, 2H), 7.92 (s, 1H), 7.56 (t, J =7.8 Hz, 1H), 7.48 (s, 1H), 7.40 (q, J = 8.4 Hz, 4H), 7.21 - 7.08 (m, 4H), 7.03 (d, J = 8.2 Hz, 1H), 6.67 (d, J = 7.4 Hz, 1H), 4.61 - 4.53 (m, 4H), 4.45 (t, J = 7.8Hz, 3H), 4.28 (d, J = 5.6 Hz, 4H), 4.23 (d, J = 5.5 Hz, 2H), 4.19 (d, J = 5.4 Hz, 2H), 3.83 (t, J = 5.2 Hz, 2H), 3.68 (dt, J = 19.9, 6.8 Hz, 4H), 3.14 - 3.01 (m, 4H),2.73 (t, J = 7.5 Hz, 2H), 2.15 - 1.58 (m, 11H), 0.94 (s, 9H).LCMS $[M+H]^+$=1113.6 |
| **UB-180786 (286)** | 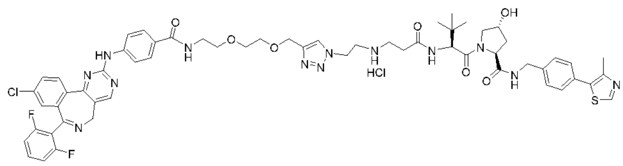 |
| | $^1$H NMR (400 MHz, DMSO-$d_6$) δ 10.18 (s, 1H), 9.13 (s, 1H), 9.02 (s, 2H), 8.73 (s, 1H), 8.60 (t, J = 6.1 Hz, 1H), 8.39 (t, J = 5.6 Hz, 1H), 8.30 (dd, J = 8.9, 3.8Hz, 2H), 8.18 (d, J = 2.3 Hz, 1H), 7.93 - 7.80 (m, 6H), 7.55 (tt, J = 8.3, 6.5 Hz, 1H), 7.44 - 7.33 (m, 5H), 7.17 (t, J = 8.8 Hz, 2H), 4.95 (s, 1H), 4.73 (t, J = 6.1Hz, 2H), 4.55 (d, J = 5.1 Hz, 4H), 4.40 - 4.34 (m, 4H), 4.21 (dd, J = 15.9, 5.4 Hz, 1H), 3.68 (dd, J = 10.5, 4.1 Hz, 1H), 3.63 - 3.56 (m, 5H), 3.53 (t, J = 6.1 Hz,2H), 3.47 (t, J = 6.2 Hz, 2H), 3.41 (d, J = 5.7 Hz, 2H), 3.15 (d, J = 8.3 Hz, 2H), 2.70 (d, J = 3.4 Hz, 2H), 2.44 (s, 2H), 2.09 - 1.87 (m, 2H), 0.94 (s, 9H).LCMS $[M+H]^+$ =1172.7 |
| **UB-180787 (287)** | 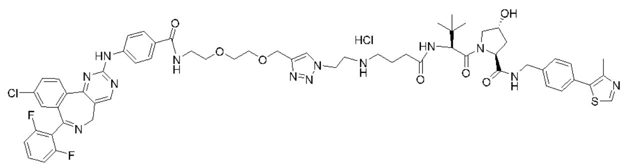 |
| | $^1$H NMR (400 MHz, DMSO-*d6*) δ 10.17 (s, 1H), 9.00 (d, J = 13.9 Hz, 3H), 8.73 (s, 1H), 8.58 (t, J = 6.0 Hz, 1H), 8.38 (t, J = 5.6 Hz, 1H), 8.30 (d, J = 8.5 Hz,1H), 8.18 (s, 1H), 8.05 (d, J = 9.3 Hz, 1H), 7.94 - 7.80 (m, 5H), 7.55 (ddd, J = 14.9, 8.4, 6.4 Hz, 1H), 7.46 - 7.32 (m, 5H), 7.17 (t, J = 9.0 Hz, 2H), 5.16 (s,1H), 4.71 (t, J = 6.3 Hz, 2H), 4.54 (d, J = 11.7 Hz, 3H), 4.47 - 4.38 (m, 2H), 4.36 (s, 1H), 4.22 (dd, J = 15.8, 5.4 Hz, 1H), 3.72 - 3.50 (m, 9H), 2.92 (s, 2H),2.44 (s, 2H), 2.39 - 2.24 (m, 2H), 2.05 (t, J = 10.6 Hz, 1H), 1.94 - 1.79 (m, 3H), 0.94 (s, 9H).LCMS $[M+H]^+$ =1186.7 |
| **UB-180788 (288)** | 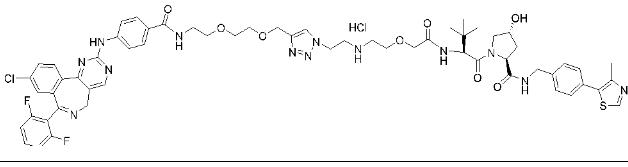 |
| | $^1$H NMR (400 MHz, DMSO-*d6*) δ 10.17 (s, 1H), 9.16 (s, 2H), 9.01 (s, 1H), 8.73 (s, 1H), 8.61 (t, J = 6.1 Hz, 1H), 8.39 (t, J = 5.6 Hz, 1H), 8.30 (d, J = 8.5Hz, 1H), 8.17 (s, 1H), 7.92 - 7.79 (m, 6H), 7.55 (tt, J = 8.4, 6.5 Hz, 1H), 7.39 (d, J = 1.6 Hz, 4H), 7.34 (d, J = 2.2 Hz, 1H), 7.17 (t, J = 8.8 Hz, 2H), 4.74(t, J = 6.4 Hz, 2H), 4.57 (d, J = 17.5 Hz, 3H), 4.43 (q, J = 8.9 Hz, 2H), 4.37 (d, J = 5.8 Hz, 1H), 4.24 (dd, J = 15.8, 5.5 Hz, 2H), 4.08 (s, 2H), 3.76 (d, J =6.3 Hz, 2H), 3.59 (qd, J = 4.8, 2.4 Hz, 5H), 3.53 (t, J = 6.2 Hz, 4H), 3.42 (t, J = 5.8 Hz, 2H), 3.18 (s, 2H), 2.44 (s, 2H), 2.07 (dd, J = 14.9, 5.7 Hz, 1H),1.91 (m, 1H), 0.95 (s, 9H).LCMS $[M+H]^+$ =1202.7 |

(continued)

| Examples | Structural data analysis |
|---|---|
| **UB-180789 (289)** | 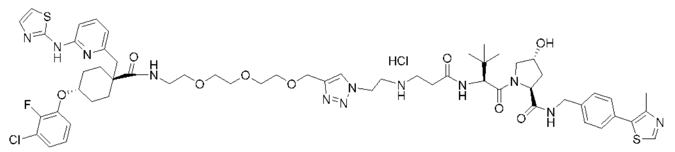 |
| | $^1$H NMR (400 MHz, DMSO-*d6*) δ 12.23 (s, 1H), 9.33 - 8.98 (m, 3H), 8.61 (t, J = 6.2 Hz, 1H), 8.31 (d, J = 9.2 Hz, 1H), 8.18 (s, 1H), 7.69 (dd, J = 17.6, 9.8Hz, 2H), 7.53 (s, 1H), 7.40 (q, J = 8.3 Hz, 5H), 7.21 - 7.02 (m, 6H), 6.80 (d, J = 7.5 Hz, 1H), 4.73 (d, J = 6.3 Hz, 2H), 4.58 - 4.50 (m, 5H), 4.48 - 4.40 (m, 3H), 4.37 (d, J = 5.2 Hz, 1H), 4.26 - 4.15 (m, 2H), 3.71 - 3.60 (m, 2H), 3.57 - 3.46 (m, 10H), 3.36 (t, J = 6.1 Hz, 2H), 3.18 (d, J = 5.9 Hz, 4H), 2.96 (s, 2H),2.75 - 2.62 (m, 2H), 2.05 (t, J = 10.4 Hz, 1H), 1.94 - 1.89 (m, 2H), 1.83 - 1.63 (m, 6H), 0.95 (s, 9H). LCMS $[M+H]^+$ =1201.7 |
| **UB-180790 (290)** | 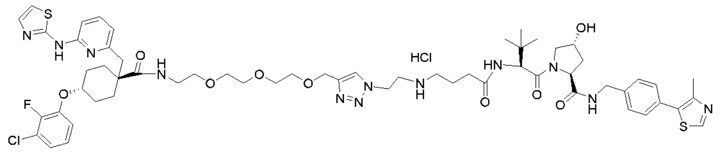 |
| | $^1$H NMR (400 MHz, DMSO-*d6*) δ 12.33 (s, 1H), 9.41 (s, 2H), 9.02 (s, 1H), 8.61 (t, J = 6.0 Hz, 1H), 8.20 (s, 1H), 8.05 (d, J = 9.2 Hz, 1H), 7.82 - 7.61 (m, 2H),7.56 - 7.32 (m, 5H), 7.22 - 7.00 (m, 5H), 6.81 (d, J = 7.5 Hz, 1H), 4.76 (t, J = 6.4 Hz, 2H), 4.53 (d, J = 8.3 Hz, 4H), 4.43 (q, J = 7.4 Hz, 2H), 4.35 (s, 1H), 4.27 - 4.20 (m, 2H), 3.66 (dd, J = 11.6, 7.8 Hz, 2H), 3.59 - 3.39 (m, 11H), 3.36 (t, J = 6.1 Hz, 2H), 3.21 - 3.14 (m, 2H), 2.93 (d, J = 22.6 Hz, 4H), 2.33 (dt, J = 26.5, 7.4Hz, 2H), 2.05 (t, J = 10.5 Hz, 1H), 1.95 - 1.61 (m, 11H), 0.94 (s, 9H). LCMS $[M+H]^+$ = 1215.7 |
| **UB-180791 (291)** | 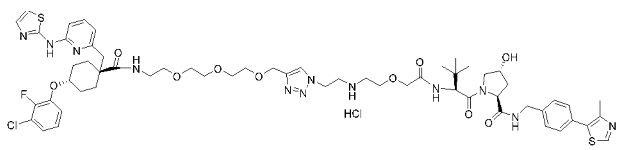<br>$^1$H NMR (400 MHz, DMSO-*d6*) δ 11.45 (s, 1H), 9.21 (s, 2H), 9.00 (s, 1H), 8.60 (d, J = 6.0 Hz, 1H), 8.17 (s, 1H), 7.83 (d, J = 9.4 Hz, 1H), 7.68 (s, 1H), 7.58 (t, J= 7.8 Hz, 1H), 7.45 - 7.35 (m, 5H), 7.19 - 7.06 (m, 3H), 7.02 (s, 1H), 6.92 (d, J = 8.3 Hz, 1H), 6.68 (d, J = 7.4 Hz, 1H), 4.74 (t, J = 6.3 Hz, 2H), 4.56 (d, J = 27.7Hz, 4H), 4.41 (dt, J = 22.4, 7.5 Hz, 3H), 4.24 (dd, J = 16.2, 5.6 Hz, 1H), 4.08 (s, 2H), 3.77 (s, 2H), 3.35 (d, J = 6.0 Hz, 6H), 3.18 (d, J = 5.7 Hz, 4H), 2.92 (s, 1H),2.44 (s, 4H), 2.21 (s, 1H), 2.07 (t, J = 10.0 Hz, 1H), 1.77 (m, 10H), 0.95 (s, 9H)..LCMS $[M+H]^+$ =1231.7 |
| **UB-180792 (292)** | 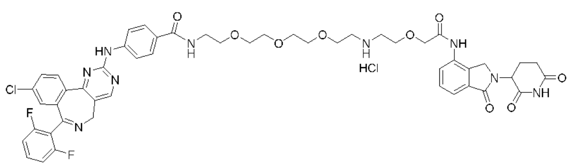 |
| | $^1$H NMR (400 MHz, DMSO-*d6*) δ 11.01 (s, 1H), 10.17 (s, 2H), 9.15 (s, 2H), 8.73 (s, 1H), 8.40 (t, J = 5.6 Hz, 1H), 8.30 (d, J = 8.5 Hz, 1H), 7.93 - 7.81 (m,5H), 7.74 (dd, J = 7.9, 1.2 Hz, 1H), 7.61 - 7.47 (m, 3H), 7.34 (d, J = 2.1 Hz, 1H), 7.17 (t, J = 8.7 Hz, 2H), 5.14 (dd, J = 13.2, 5.1 Hz, 1H), 4.52 - 4.36 (m, 2H), 4.19 (s, 3H), 3.82 (t, J = 5.0 Hz, 2H), 3.75 (t, J = 5.2 Hz, 2H), 3.55 (s, 4H), 3.45 - 3.34 (m, 3H), 3.27 - 3.09 (m, 5H), 2.92 (td, J = 13.0, 12.4, 6.7 Hz,1H), 2.60 (d, J = 17.4 Hz, 1H), 2.36 (d, J = 4.5 Hz, 1H), 2.06 - 1.96 (m, 1H), 1.35 - 1.24 (m, 4H). LCMS $[M+H]^+$ =994.45 |

(continued)

| Examples | Structural data analysis |
|---|---|
| **UB-180793 (293)** | 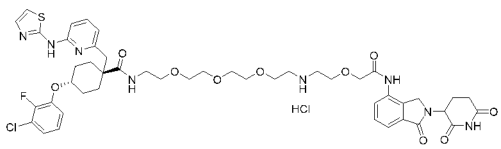 |
| | $^1$H NMR (400 MHz, DMSO-*d6*) δ 11.72 (s, 1H), 11.00 (s, 1H), 10.30 (s, 1H), 9.35 (s, 2H), 7.74 (dd, J = 7.1, 4.3 Hz, 2H), 7.65 - 7.47 (m, 3H), 7.42 (d, J =3.8 Hz, 1H), 7.19 - 7.03 (m, 4H), 6.97 (d, J = 8.2 Hz, 1H), 6.72 (d, J = 7.3 Hz, 1H), 5.14 (dd, J = 13.4, 5.1 Hz, 1H), 4.44 (dd, J = 64.8, 17.4 Hz, 3H), 4.19 (s, 2H), 3.82 (s, 2H), 3.76 (d, J = 5.4 Hz, 2H), 3.48 (q, J = 2.2 Hz, 4H), 3.35 (t, J = 6.3 Hz, 2H), 3.26 - 3.09 (m, 7H), 2.98 - 2.85 (m, 3H), 2.62 (s, 1H), 2.38- 2.30 (m, 1H), 2.10 - 1.58 (m, 10H).LCMS [M+H]$^+$ = 979.5 |
| **UB-180794 (294)** | 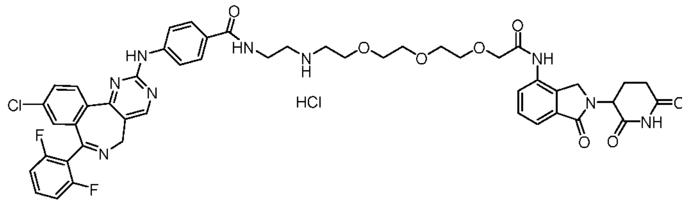 |
| | $^1$H NMR (400 MHz, DMSO-*d6*) δ 11.01 (s, 1H), 10.21 (s, 1H), 9.87 (s, 1H), 9.16 (s, 2H), 8.76 (d, J = 18.6 Hz, 2H), 8.29 (d, J = 8.5 Hz, 1H), 7.96 - 7.84(m, 5H), 7.75 (dd, J = 7.6, 1.3 Hz, 1H), 7.59 - 7.47 (m, 3H), 7.34 (d, J = 2.2 Hz, 1H), 7.17 (t, J = 8.6 Hz, 2H), 5.14 (dd, J = 13.3, 5.1 Hz, 1H), 4.39 (q, J =17.5 Hz, 3H), 4.17 (s, 2H), 3.77 - 3.67 (m, 8H), 3.64 (s, 3H), 3.14 (t, J = 5.8 Hz, 4H), 2.91 (ddd, J = 18.1, 13.3, 5.4 Hz, 1H), 2.60 (d, J = 17.1 Hz, 1H),2.39 - 2.31 (m, 1H), 2.08 - 1.95 (m, 1H), 1.29 (ddd, J = 16.8, 8.0, 5.7 Hz, 2H).LCMS [M+H]$^+$ =950.5 |
| **UB-180795 (295)** | 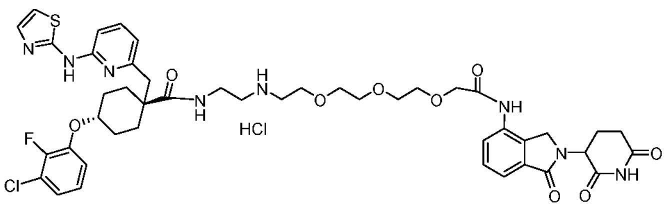 |
| | $^1$H NMR (400 MHz, DMSO-*d6*) δ 11.57 (s, 1H), 11.01 (s, 1H), 9.87 (s, 1H), 9.23 (s, 2H), 8.13 (s, 1H), 7.76 (d, J = 7.5 Hz, 1H), 7.56 (ddt, J = 22.0, 14.9,7.5 Hz, 3H), 7.41 (d, J = 3.6 Hz, 1H), 7.12 (td, J = 9.4, 7.4, 4.2 Hz, 3H), 7.03 (d, J = 3.6 Hz, 1H), 6.95 (d, J = 8.1 Hz, 1H), 6.69 (d, J = 7.3 Hz, 1H), 5.14 (dd, J = 13.4, 5.0 Hz, 1H), 4.53 (s, 1H), 4.39 (q, J = 17.5 Hz, 2H), 4.17 (s, 2H), 3.62 (d, J = 5.6 Hz, 10H), 3.38 (d, J = 5.9 Hz, 2H), 3.06 (s, 2H), 3.02 -2.87 (m, 5H), 2.62 (s, 1H), 2.38 (s, 1H), 1.81 (td, J = 61.1, 59.4, 14.2 Hz, 10H).LCMS [M+H]$^+$=935.5 |
| **UB-180796 (296)** | 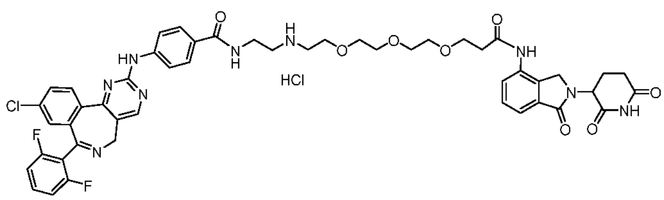 |
| | $^1$H NMR (400 MHz, DMSO-*d6*) δ 11.03 (s, 1H), 10.21 (s, 1H), 9.98 (s, 1H), 8.93 (s, 2H), 8.71 (d, J = 16.9 Hz, 2H), 8.30 (d, J = 8.6 Hz, 1H), 7.93 - 7.77(m, 6H), 7.52 (dq, J = 15.4, 7.8 Hz, 3H), 7.34 (d, J = 2.1 Hz, 1H), 7.16 (q, J = 8.8 Hz, 3H), 5.14 (dd, J = 13.3, 5.1 Hz, 1H), 4.37 (q, J = 17.5 Hz, 2H),3.71 (t, J = 6.1 Hz, 4H), 3.55 (s, 3H), 3.13 (d, J = 4.2 Hz, 2H), 2.64 (d, J = 6.0 Hz, 2H), 2.55 (d, J = 4.9 Hz, 1H), 2.32 (s, 2H), 2.01 (p, J = 8.2 Hz, 2H).LCMS [M+H]$^+$ = 964.5 |

(continued)

| Examples | Structural data analysis |
| --- | --- |
| **UB-180797 (297)** | 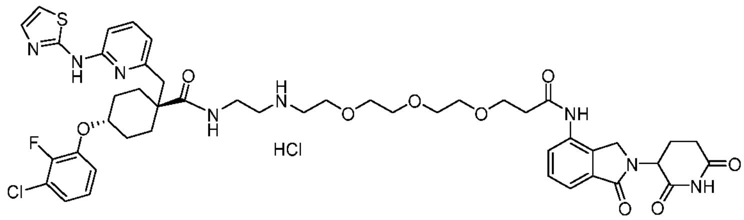 |
| | $^1$H NMR (400 MHz, DMSO-$d_6$) δ 11.72 (s, 1H), 11.00 (s, 1H), 10.30 (s, 1H), 9.35 (s, 2H), 7.74 (dd, *J* = 7.3, 4.3 Hz, 1H), 7.64 - 7.48 (m, 3H), 7.42 (d, *J* = 3.8 Hz, 1H), 7.20 - 7.08 (m, 2H), 7.05 (d, *J* = 3.8 Hz, 1H), 6.97 (d, *J* = 8.2 Hz, 1H), 6.72 (d, *J* = 7.3 Hz, 1H), 5.14 (dd, *J* = 13.4, 5.1 Hz, 1H), 4.44 (dd, *J* = 64.8, 17.4 Hz, 3H), 4.19 (s, 2H), 3.83 (d, *J* = 4.8 Hz, 5H), 3.76 (t, *J* = 5.3 Hz, 4H), 3.48 (dp, *J* = 5.1, 2.5 Hz, 3H), 3.35 (t, *J* = 6.3 Hz, 2H), 3.26 - 3.07 (m, 5H), 3.00 - 2.90 (m, 2H), 2.61 (d, *J* = 3.7 Hz, 1H), 2.36 (d, *J* = 8.7 Hz, 1H), 2.07 - 1.55 (m, 8H).LCMS $[M+H]^+$ = 949.5 |
| **UB-180798 (298)** | 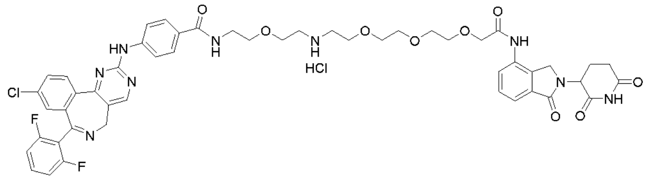 |
| | $^1$H NMR (400 MHz, DMSO-$d_6$) δ 11.01 (s, 1H), 10.19 (s, 1H), 9.80 (s, 1H), 8.85 (s, 2H), 8.73 (s, 1H), 8.54 - 8.47 (m, 1H), 8.29 (d, J = 8.5 Hz, 1H),7.92 - 7.83 (m, 6H), 7.74 (dd, J = 7.7, 1.2 Hz, 1H), 7.57 - 7.46 (m, 3H), 7.35 (d, J = 2.1 Hz, 1H), 7.17 (t, J = 8.8 Hz, 2H), 5.15 (dd, J = 13.3, 5.1 Hz,1H), 4.48 (s, 2H), 4.35 - 4.28 (m, 2H), 4.16 (d, J = 8.6 Hz, 2H), 3.74 - 3.66 (m, 7H), 3.62 - 3.57 (m, 5H), 3.47 (t, J = 5.4 Hz, 3H), 3.12 (dt, J = 11.4,5.8 Hz, 5H), 2.98 - 2.84 (m, 1H), 2.62 (s, 1H), 2.27 (dd, J = 9.2, 6.9 Hz, 1H), 2.14 - 1.95 (m, 1H).LCMS $[M+H]^+$ = 994.6 |
| **UB-180799 (299)** | 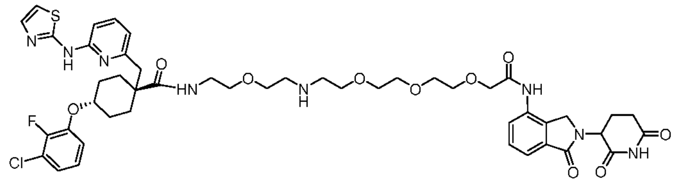 |
| | $^1$H NMR (400 MHz, DMSO-$d_6$) δ 11.63 (s, 1H), 11.02 (s, 1H), 9.80 (d, J = 2.5 Hz, 1H), 8.81 (s, 2H), 7.79 (dd, J = 29.1, 6.7 Hz, 2H), 7.63 (t, J = 7.9 Hz,2H), 7.59 - 7.48 (m, 3H), 7.43 (d, J = 3.8 Hz, 1H), 7.12 (ddt, J = 13.4, 9.5, 5.0 Hz, 4H), 7.06 (d, J = 3.8 Hz, 1H), 6.96 (d, J = 8.2 Hz, 1H), 6.72 (d, J = 7.3Hz, 1H), 5.14 (dd, J = 13.3, 5.1 Hz, 1H), 4.55 (s, 1H), 4.42 - 4.31 (m, 2H), 4.17 (d, J = 8.7 Hz, 3H), 3.68 - 3.66 (m, 4H), 3.13 - 3.10 (m, 2H), 2.96 (s, 2H),2.63 (s, 1H), 2.05 - 1.87 (m, 4H), 1.83 - 1.74 (m, 4H), 1.69 - 1.58 (m, 3H).LCMS $[M+H]^+$ = 979.6 |
| **UB-180801 (301)** | 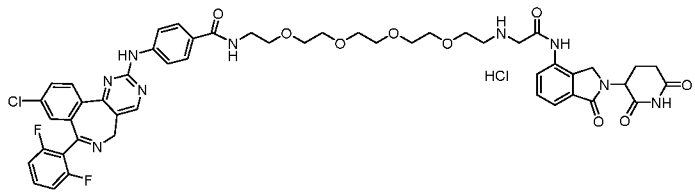 |
| | $^1$H NMR (400 MHz, DMSO-$d_6$) δ 11.05 (s, 1H), 10.56 (s, 1H), 10.17 (s, 1H), 9.05 (s, 2H), 8.74 (d, *J* = 9.6 Hz, 1H), 8.45 - 8.26 (m, 2H), 8.05 - 7.76 (m, 6H), 7.65 - 7.34 (m, 3H), 7.34 (s, 1H), 7.17 (t, *J* = 8.4 Hz, 2H), 5.18 (dd, *J* = 13.3, 5.2 Hz, 1H), 5.15 - 3.94 (m, 5 H), 3.84 - 3.68 (m, 2H), 3.68 - 3.44 (m, 14H), 3.44 - 3.34 (m, 2H), 3.22 (s, 2H), 2.93 (dd, *J* = 2.2, 9.1 Hz, 1H),2.61 - 2.22 (m, 3H), 2.02 (m, 1H). LCMS $[M+H]^+$ =994.3 |

(continued)

| Examples | Structural data analysis |
|---|---|
| **UB-180802 (302)** | 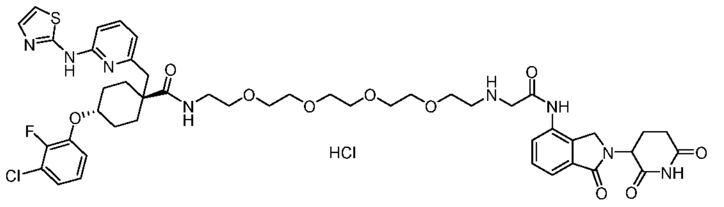 |
| | $^1$H NMR (400 MHz, DMSO-$d_6$) δ11.86 (s, 1H), 11.05 (s, 1H), 10.67 (s, 1H), 9.12 (s, 2H), 7.87 (d, *J* = 6.1 Hz, 1H), 7.81 - 7.38 (m, 5H), 7.45 (s, 1H), 7.45 - 7.05 (m, 4H), 6.98 (d, *J* = 8.2 Hz, 1H), 6.75 (d, *J* = 7.4 Hz, 1H), 5.18 (dd, *J* = 13.3, 5.1 Hz, 1H), 4.55 (s, 1H), 4.46 (d, *J* = 17.6 Hz, 2H), 4.05 (s, 2H), 3.92 - 3.40 (m, 14H), 3.40 - 2.96 (m, 6H), 2.96 - 2.67 (m, 2H), 2.63 (d, *J* = 18.8 Hz, 2H), 2.29 (dd, *J* = 15.7, 11.0 Hz, 1H), 2.25 - 2.02 (m, 2H), 2.13 - 2.02 (m, 1H), 1.91-1.82 (m, 2H), 1.71-1.58 (m, 2H). LCMS $[M+H]^+$ =979.7 |
| **UB-180803 (303)** | 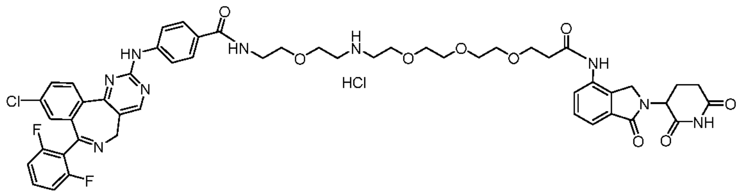 |
| | $^1$H NMR (400 MHz, DMSO-$d_6$) δ 11.03 (s, 1H), 10.18 (d, J = 9.5 Hz, 1H), 9.99 (s, 1H), 9.33 (s, 2H), 8.88 (s, 1H), 8.52 (t, J = 5.6 Hz, 1H), 8.31 (d, J = 2.1Hz, 1H), 7.91 - 7.79 (m, 6H), 7.57 - 7.49 (m, 3H), 7.34 (d, J = 2.1 Hz, 1H), 7.17 (s, 2H), 5.15 (dd, J = 13.3, 5.1 Hz, 1H), 4.44 - 4.29 (m, 2H), 3.72 (d, J =4.8 Hz, 4H), 3.57 (d, J = 2.9 Hz, 4H), 3.47 (d, J = 5.1 Hz, 3H), 3.12 (qd, J = 7.4, 4.2 Hz, 10H), 2.96 - 2.87 (m, 2H), 2.78 (d, J = 4.5 Hz, 1H), 2.63 (t, J = 6.2Hz, 3H), 2.30 (d, J = 4.4 Hz, 1H), 2.03 (dd, J = 9.9, 4.7 Hz, 1H).LCMS $[M+H]^+$ = 1088.4 |
| **UB-180804 (304)** | 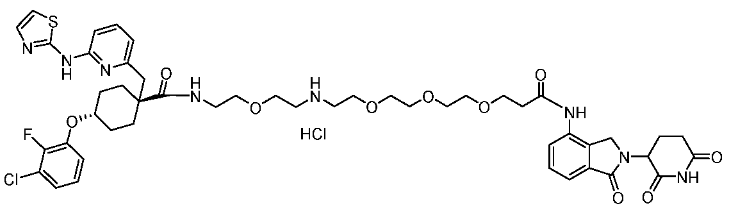 |
| | $^1$H NMR (400 MHz, DMSO-$d_6$) δ 12.29 (s, 1H), 11.03 (s, 1H), 10.02 (s, 1H), 8.97 (s, 2H), 7.92 (d, J = 7.0 Hz, 1H), 7.83 (dd, J = 7.1, 1.9 Hz, 1H), 7.71 (td, J =7.8, 3.7 Hz, 2H), 7.53 - 7.47 (m, 3H), 7.20 - 7.14 (m, 4H), 6.85 (dd, J = 15.7, 7.4 Hz, 2H), 5.15 (dd, J = 13.2, 5.1 Hz, 1H), 4.60 (s, 2H), 4.38 (d, J = 19.7 Hz,3H), 3.73 - 3.67 (m, 4H), 3.58 (s, 2H), 3.52 (d, J = 1.8 Hz, 5H), 3.42 (t, J = 6.0 Hz, 2H), 3.24 (t, J = 5.6 Hz, 2H), 3.13 - 3.06 (m, 4H), 3.01 (s, 3H), 2.67 - 2.61(m, 2H), 2.41 - 2.29 (m, 2H), 1.99 (dd, J = 20.4, 7.6 Hz, 2H), 1.89 - 1.81 (m, 4H), 1.66 (q, J = 12.6, 10.2 Hz, 4H).LCMS $[M+H]^+$ = 993.4 |
| **UB-180807 (307)** | 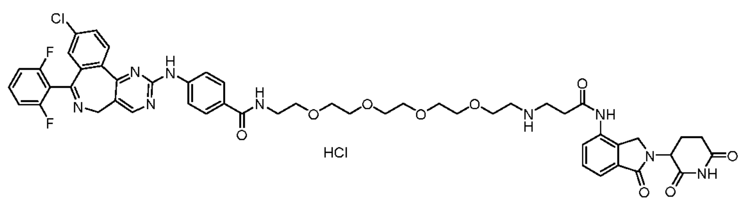 |
| | $^1$H NMR (400 MHz, DMSO-$d_6$) δ11.03 (s, 1H), 10.29 (s, 1H), 10.18 (s, 1H), 9.49 - 8.56 (m, 2H), 8.79 - 8.56 (m, 1H), 8.40 (t, *J* = 5.4 Hz, 1H), 8.31 (t, *J* = 8.1 Hz, 1H), 8.37 - 7.77 (m, 6H), 7.62 - 7.33 (m, 3H), 7.35 (d, *J* = 2.1 Hz, 1H), 7.36 - 6.99 (m, 2H), 5.17-5.21(m,1H),4.51-4.22(m,2H), 3.95 - 2.89 (m, 23H), 2.99 - 2.78 (m, 3H), 2.63 (t, *J* = 16.3 Hz, 2H), 2.40 - 2.14 (m, 1H), 2.04 (dd, *J* = 14.3, 9.0 Hz, 1H).LCMS $[M+H]^+$ =1008.4 |

(continued)

| Examples | Structural data analysis |
|---|---|
| **UB-180808 (308)** | 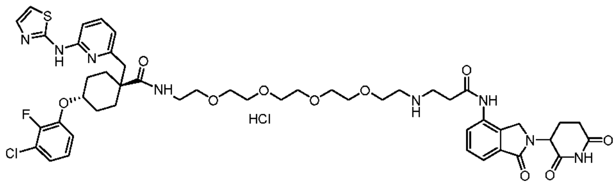 |
| | $^1$H NMR (400 MHz, DMSO-$d_6$) δ11.03 (s, 1H), 10.29 (s, 1H), 10.18 (s, 1H), 8.79 - 8.56 (m, 2H), 7.92 - 7.33 (m, 6H), 7.35 (d, *J* = 2.1 Hz, 1H), 7.36 - 6.67(m, 4H), 5.51- 4.98 (m, 1H), 4.74 - 4.26 (m,4H), , 3.95 - 3.12 (m, 28H), 2.99 - 2.78 (m, 2H), 2.63 (t, *J* = 16.3 Hz, 2H), 2.40 - 2.14 (m, 2H), 2.04 (dd, *J* = 14.3, 9.0 Hz, 1H), 1.93 -1.51 (m, 11H).LCMS $[M+H]^+$=993.4 |

(2.5) Preparation procedure 5 of TED molecule:

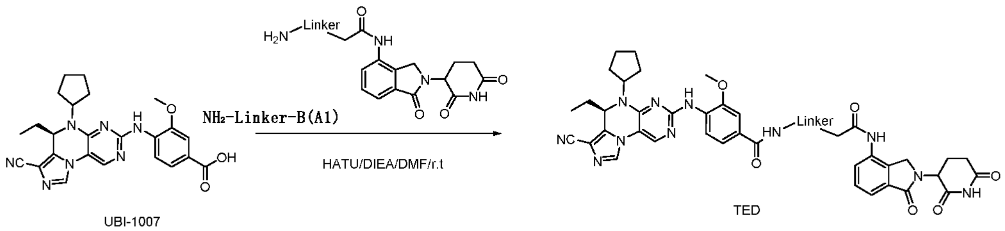


Compound UBI-1007 (20 mg, 0.0436 mmol) was weighed and dissolved in anhydrous DMF (1 mL), added $NH_2$-linker-B(A1) (0.0436 mmol), HATU(33 mg, 0.0872 mmol) and DIEA (0.072mL, 0.436 mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL*3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound.

The conjugate of A-L1-B as shown in Table B5 was prepared through similar method.

Table B5

| Examples | Compound structure | Analyze data |
|---|---|---|
| **UB-180512** | 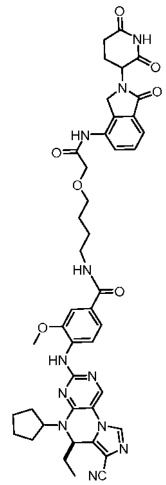 | $^1$H NMR (400 MHz, *d6*- DMSO) δ 11.00 (s, 1H), 9.74 (s, 1H), 8.70 (s, 1H), 8.64 (s, 1H), 8.40 (t, *J* = 5.6 Hz, 1H), 8.23 (d, *J* = 8.2 Hz, 1H), 8.00 (s, 1H), 7.74 (d, *J* = 7.7 Hz, 1H), 7.60 - 7.41 (m, 4H), 5.24 - 5.05 (m, 2H), 4.60 - 4.22 (m, 3H), 4.10 (s, 2H), 3.91 (s, 3H), 3.71 - 3.37 (m, 4H), 2.97 - 2.83 (m, 1H), 2.45 - 2.13 (m, 2H), 2.06 - 1.87 (m, 11H), 0.78 (dt, *J* = 14.7, 7.3 Hz, 3H). LCMS [M+H]+ =830.3 |
| **UB-180518** | 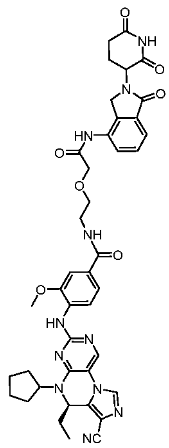 | $^1$H NMR (400 MHz, *d6*-DMSO) δ 10.99 (s, 1H), 9.79 (s, 1H), 8.70 (s, 1H), 8.64 (s, 1H), 8.55 (t, *J* = 5.4 Hz, 1H), 8.24 (d, *J* = 8.9 Hz, 1H), 7.99 (s, 1H), 7.71 (d, *J* = 7.7 Hz, 1H), 7.52 (ddd, *J* = 12.7, 8.6, 4.3 Hz, 3H), 5.24 - 5.05 (m, 2H), 4.58 - 4.28 (m, 3H), 4.18 (s, 2H), 3.87 (s, 3H), 3.70 (t, *J* = 5.7 Hz, 2H), 3.63 - 3.34 (m, 2H), 2.94-2.85 (m,1H), 2.58-2.52 (m,1H), 2.43 - 2.07 (m, 1H), 2.07 - 1.57 (m, 11H), 0.74 (t, *J* = 7.3 Hz, 3H). LCMS $[M+H]^+$ =802.8 |
| **UB-180537** | 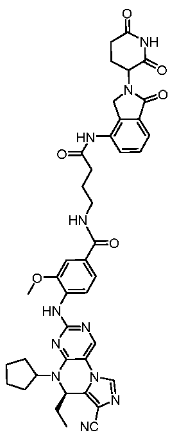 | $^1$H NMR (400 MHz, *d6*-DMSO) δ 11.01 (s, 1H), 9.86 (s, 1H), 8.70 (s, 1H), 8.64 (s, 1H), 8.45 (t, *J* = 5.7 Hz, 1H), 8.25 (d, *J* = 8.2 Hz, 1H), 8.00 (s, 1H), 7.81 (dd, *J* = 7.1, 1.7 Hz, 1H), 7.49 (dd, *J* = 15.4, 8.2 Hz, 4H), 5.27 - 5.07 (m, 2H), 4.55 - 4.28 (m, 3H), 3.92 (s, 3H), 3.30 (s, 2H), 3.12 - 2.86 (m, 1H), 2..41-2.49 (m, 4H), 2.07 - 1.76 (m, 13H), 0.74 (t, *J* = 7.3 Hz, 3H). LCMS $[M+H]^+$ =786.3 |

(continued)

| Examples | Compound structure | Analyze data |
|---|---|---|
| **UB-180538** | 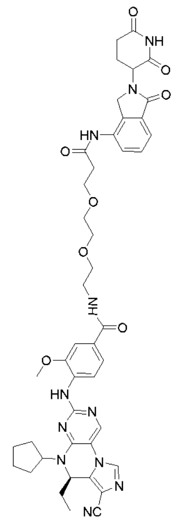 | $^1$H NMR (400 MHz, *d6-DMSO*) δ 11.01 (s, 1H), 9.86 (s, 1H), 8.70 (s, 1H), 8.64 (s, 1H), 8.45 (t, *J* = 5.7 Hz, 1H), 8.25 (d, *J* = 8.2 Hz, 1H), 8.00 (s, 1H), 7.81 (dd, *J* = 7.1, 1.7 Hz, 1H), 7.49 (dd, *J* = 15.4, 8.2 Hz, 4H), 5.27 - 5.07 (m, 2H), 4.55 - 4.28 (m, 3H), 3.92 (s, 3H), 3.53 - 3.32 (m, 8H), 3.12 - 2.86 (m, 1H), 2.61-2.55 (m, 1H), 2.37 - 1.76 (m, 16H), 0.74 (t, *J* = 7.3 Hz, 3H). LCMS $[M+H]^+$ =860.9 |
| **UB-180545** | 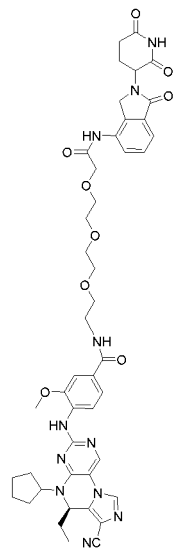 | $^1$H NMR (400 MHz, *d6-DMSO*) δ 11.01 (s, 11H), 9.70 (s, 1H), 8.70 (s, 1H), 8.64 (s, 1H), 8.45 (t, *J* = 5.6 Hz,1H), 8.24 (d, *J*= 8.3 Hz, 1H), 7.99 (s, 1H), 7.77 - 7.70 (m, 1H), 7.52 (ddd, *J* = 11.4, 10.5, 4.6 Hz, 4H), 5.24 - 5.07 (m, 2H), 4.38 (dt, *J* = 27.7, 14.0 Hz, 3H), 4.13 (s, 2H), 3.91 (s, 3H), 3.75 - 3.52 (m, 9H), 3.42 - 3.36 (m, 2H), 3.02 - 2.79 (m, 1H) 2.10 - 1.85 (m, 11H), 1.35 - 1.14 (m, 2H), 0.74 (t, *J* = 7.3 Hz, 3H). LCMS $[M+H]^+$ =890.5 |

(continued)

| Examples | Compound structure | Analyze data |
|---|---|---|
| **UB-180548** | 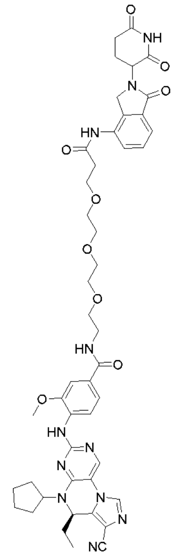 | $^1$H NMR (400 MHz, *d6-DMSO*) δ 11.02 (s, 1H), 9.83 (s, 1H), 8.70 (s, 1H), 8.63 (s, 1H), 8.44 (t, *J* = 5.4 Hz, 1H), 8.25 (d, *J* = 8.3 Hz, 1H), 8.03 - 7.82 (m, 2H), 7.53 - 7.29 (m, 4H), 5.25 - 5.07 (m, 2H), 4.43-4.39 (m, 3H), 3.92 (s, 3H), 3.78 - 3.61 (m, 5H), 3.46 (dt, *J* = 11.0, 4.2 Hz, 10H), 3.41 (d, *J* = 9.6 Hz, 2H), 2.75 - 2.10 (m, 5H), 2.49 - 2.10 (m, 15H), 2.10 - 1.26 (m, 11H), 0.74 (t, *J* = 7.4 Hz, 3H). LCMS $[M+H]^+$ = 904.4 |
| **UB-180551** | 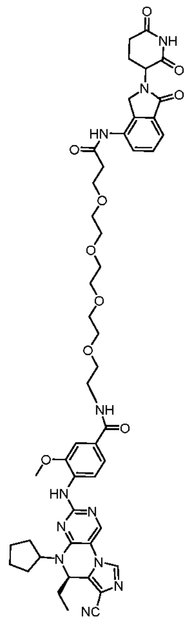 | $^1$H NMR (400 MHz, *d6-DMSO*) δ 11.01 (s, 1H), 9.86 (s, 1H), 8.70 (s, 1H), 8.64 (s, 1H), 8.45 (t, *J* = 5.7 Hz, 1H), 8.25 (d, *J* = 8.2 Hz, 1H), 8.00 (s, 1H), 7.81 (dd, *J* = 7.1, 1.7 Hz, 1H), 7.49 (dd, *J* = 15.4, 8.2 Hz, 4H), 5.27 - 5.07 (m, 2H), 4.55 - 4.28 (m, 3H), 3.92 (s, 3H), 3.53 - 3.32 (m, 7H), 3.12 - 2.86 (m, 3H), 2.41-2.32 (m, 3H), 2.07 - 1.76 (m, 18H), 0.86 -0.79(m,3H), 0.74 (t, *J* = 7.3 Hz, 3H). LCMS $[M+H]^+$ =948.7 |

(continued)

| Examples | Compound structure | Analyze data |
|---|---|---|
| **UB-180557** | 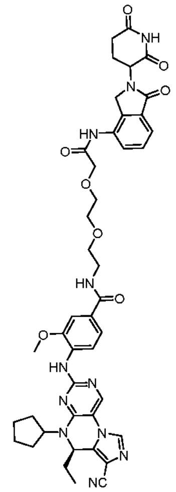 | $^1$H NMR (400 MHz, *d6-DMSO*) $\delta$ 11.00 (s, 1H), 9.70 (s, 1H), 8.70 (s, 1H), 8.64 (s, 1H), 8.45 (t, *J* = 5.4 Hz, 1H), 8.24 (d, *J* = 8.2 Hz, 1H), 7.99 (s, 1H), 7.73 (d, *J* = 7.6 Hz, 1H), 7.62 - 7.40 (m, 4H), 5.27 - 5.04 (m, 2H), 4.65 - 4.23 (m, 3H), 4.14 (s, 2H), 3.91 (s, 3H), 3.80 - 3.60 (m, 5H), 3.58 (d, *J* = 5.8 Hz, 2H), 3.04 - 2.76 (m, 1H), 2.71 - 2.57 (m, 1H), 2.07 - 1.59 (m, 11H), 0.74 (t, *J* = 7.3 Hz, 3H). LCMS $[M+H]^+$ =846.4 |
| **UB-180560** | 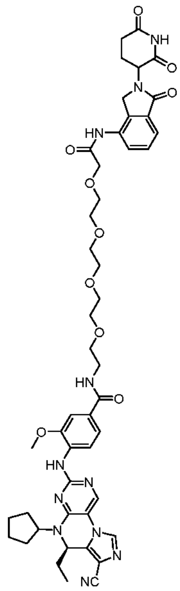 | $^1$H NMR (400 MHz, *d6-DMSO*) $\delta$ 11.00 (s, 1H), 9.68 (s, 1H), 8.70 (s, 1H), 8.63 (s, 1H), 8.44 (s, 1H), 8.25 (d, *J* = 8.3 Hz, 1H), 8.00 (s, 1H), 7.73 (d, *J* = 7.7 Hz, 1H), 7.52 (dt, *J* = 8.5, 5.3 Hz, 4H), 5.23 - 5.04 (m, 2H), 4.49 - 4.22 (m, 3H), 4.13 (s, 2H), 3.92 (s, 3H), 3.68 (dd, *J* = 5.8, 3.2 Hz, 2H), 3.65 - 3.42 (m, 13H), 3.03 - 2.72 (m, 1H), 2.64 (d, *J* = 22.0 Hz, 1H), 2.41 - 2.08 (m, 2H), 2.08 - 1.88 (m, 5H), 1.79 - 1.71 (m, 3H), 1.61 - 1.26 (m, 3H), 1.46 - 1.26 (m, 1H), 1.46 - 1.05 (m, 2H), 0.74 (t, *J* = 7.4 Hz, 3H). LCMS $[M+H]^+$ =934.4 |

(2.6) Preparation procedure 6 of TED molecule:

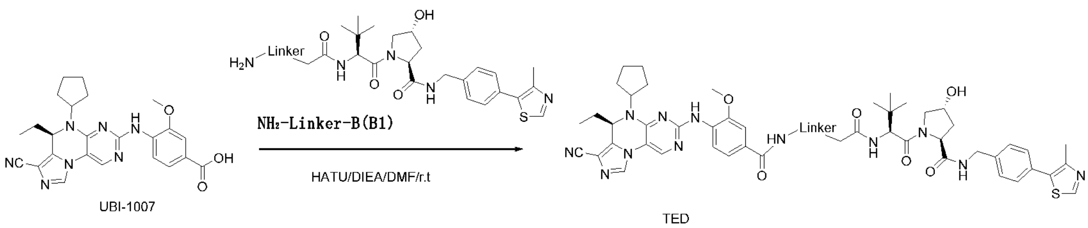


UBI-1007

TED

Compound UBI-1007 (20 mg, 0.0436 mmol) was weighed and dissolved in anhydrous DMF (1 mL), added $NH_2$-linker-B(B1) (0.0436 mmol), HATU(33 mg, 0.0872 mmol) and DIEA (0.072mL, 0.436 mmol) under ice-water bath successively. After completion of addition, the material system was stirred and reacted at room temperature under the

protection of nitrogen for 18 hours. After cpmpletion of reaction, the reaction solution was poured into 5 mL of water and extracted with ethyl acetate (5 mL*3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound.

The conjugate of A-L1-B as shown in Table B6 was prepared through similar method.

Table B6

| Examples | Compound structure | Analyze data |
|---|---|---|
| **UB-180519** | 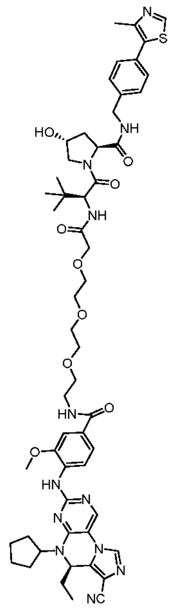 | $^1$H NMR (400 MHz, *d6-DMSO*) δ 8.97 (d, *J* = 3.9 Hz, 1H), 8.70 (s, 1H), 8.66 - 8.53 (m, 2H), 8.46 (t, *J* = 5.4 Hz, 1H), 8.25 (d, *J* = 8.3 Hz, 1H), 7.99 (s, 1H), 7.52 (s, 1H), 7.52 - 7.36 (m, 5H), 5.25 - 5.10 (m, 2H), 4.56 (d, *J* = 9.6 Hz, 1H), 4.39-4.31 (m, 5H), 4.25 (dd, *J* = 15.8, 5.7 Hz, 1H), 3.70 - 3.48 (m, 13H), 3.24 (dd, *J* = 7.9, 8.5 Hz, 2H), 2.33 (s, 3H), 2.01 - 1.59 (m, 12H), 1.00 - 0.73 (m, 9H), 0.74 (t, *J* = 7.4 Hz, 3H). LCMS $[M+H]^+$ =1061.3 |
| **UB-180525** | 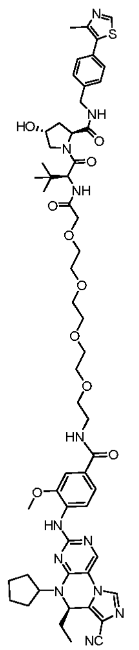 | $^1$H NMR (400 MHz, *d6-DMSO*) δ 8.97 (s, 1H), 8.74 - 8.57 (m, 3H), 8.46 (s, 1H), 8.25 (d, *J* = 8.2 Hz, 1H), 8.01 (s, 1H), 7.58 - 7.35 (m, 2H), 7.35 - 7.32 (m, 5H), 5.27 - 4.84 (m, 2H), 4.62 - 4.38 (m, 3H), 4.38 - 4.17 (m, 3H), 4.06 - 4.00 (m, 3H), 3.94 (d, *J* = 15.3 Hz,3H), 3.70 - 3.39 (m, 11H), 3.41 - 3.13 (m, 2H), 2.44 (s, 3H), 2.04-1.55 (m, 10H), 0.94 (s, 9H), 0.74 (t, *J* = 7.2 Hz, 3H). LCMS $[M+H]^+$ =1105.6 |

(continued)

| Examples | Compound structure | Analyze data |
|---|---|---|
| **UB-180529** | 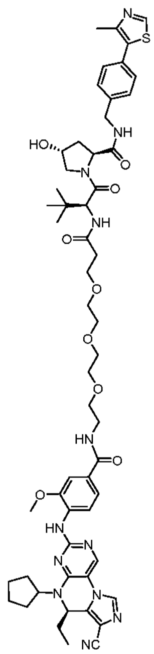 | $^{1}$H NMR (400 MHz, *d6-DMSO*) δ 8.98 (s, 1H), 8.57 (t, *J* = 5.9 Hz, 1H), 8.46 - 8.34 (m, 2H), 8.02 - 7.83 (m, 2H), 7.60 (s, 1H), 7.55 - 7.34 (m, 6H), 5.14 (s, 1H), 4.55 - 4.18 (m, 9H), 3.87(s, 3H), 3.71 - 3.38 (m, 18H), 2.43 (s, 3H), 2.02-1.55 (m, 10H), 0.93 (d, *J* = 5.7 Hz,9H), 0.87 - 0.48 (m, 3H). LCMS [M+H]$^{+}$ =1075.6 |
| **UB-180542** | 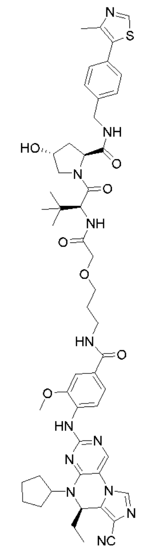 | $^{1}$H NMR (400 MHz, *d6-DMSO*) δ 8.70 (s, 1H), 8.66 - 8.49 (m, 2H), 8.40 (t, *J* = 5.6 Hz, 1H), 8.23 (d, *J* = 8.3 Hz, 1H), 8.00 (s, 1H), 7.58 - 6.95 (m, 7H), 5.27 - 5.07 (m, 2H), 4.57 (d, *J* = 9.6 Hz, 1H), 4.54 - 4.05 (m, 2H), 4.07 - 3.85 (m, 11H), 2.38 (s, 3H), 2.06 - 1.60 (m, 13H), 0.94 (d, *J* = 6.9 Hz, 9H), 0.74 (t, *J* = 7.3 Hz, 3H). LCMS [M+H]$^{+}$ =987.6 |

(continued)

| Examples | Compound structure | Analyze data |
|---|---|---|
| **UB-180543** | 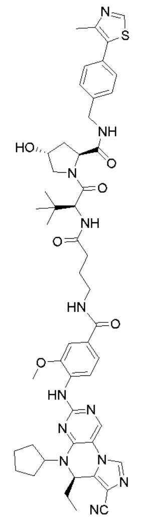 | $^1$H NMR (400 MHz, *d6-DMSO*) δ 8.96 (d, *J* = 12.3 Hz, 1H), 8.70 (s, 1H), 8.64 (s, 1H), 8.57 (t, *J* = 6.0 Hz, 1H), 8.41 (t, *J* = 5.2 Hz, 1H), 8.24 (d, *J* = 8.3 Hz, 1H), 8.03 - 7.87 (m, 2H), 7.50 (dd, *J* = 10.8, 2.3 Hz, 2H), 7.40 (q, *J* = 8.4 Hz, 4H), 5.23 - 5.10 (m, 2H), 4.59 - 4.27 (m, 5H), 4.21 (dd, *J* = 15.8, 5.4 Hz, 1H), 3.92 (d, *J* = 5.5 Hz, 3H), 3.78 - 3.41 (m, 2H), 3.29 - 3.10 (m, 3H), 2.36 (s, 3H), 2.37 - 1.63 (m, 15H), 0.94 (d, *J* = 8.3 Hz, 9H), 0.74 (t, *J* = 7.4 Hz, 3H). LCMS $[M+H]^+$ =957.5 |
| **UB-180544** | 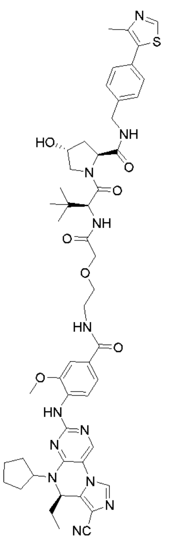 | $^1$H NMR (400 MHz, *d6-DMSO*) δ 8.96 (s, 1H), 8.70 (s, 1H), 8.68 - 8.39 (m, 4H), 8.25 (d, *J* = 8.4 Hz, 1H), 7.99 (s, 1H), 7.60 - 7.51 (m, 3H), 7.51 - 7.36 (m, 4H), 5.17 (dd, *J* = 7.9, 3.3 Hz, 2H), 4.56 (d, *J* = 9.6 Hz, 1H), 4.50 - 4.21 (m, 5H), 4.09 - 3.89 (m, 5H), 3.74 - 3.39 (m, 4H), 3.13 - 3.06 (m, 1H), 2.43 (s,3H), 1.97 - 1.47 (m, 12H), 0.91 (s, 9H), 0.73 (d, *J* = 7.4 Hz, 3H). LCMS $[M+H]^+$ =973.6 |

(continued)

| Examples | Compound structure | Analyze data |
|---|---|---|
| **UB-180552** | 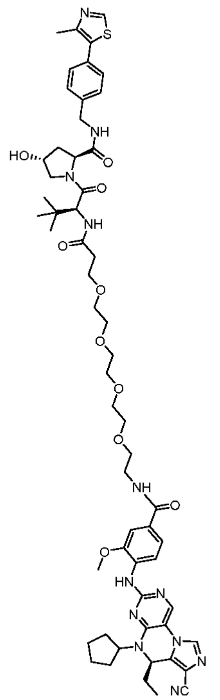 | $^1$H NMR (400 MHz, *d6-DMSO*) δ 11.01 (s, 1H), 9.70 (s, 1H), 8.70 (s, 1H), 8.64 (s,1H), 8.45 (t, *J* = 5.6 Hz, 1H), 8.24 (d, *J* = 8.3 Hz, 1H), 7.99 (s, 1H), 7.77 - 7.70 (m, 1H), 7.52 (ddd, *J* = 11.4, 10.5, 4.6 Hz, 4H), 5.24 - 5.07 (m, 2H), 4.28-4.59 (m, 6H), 3.91 (s, 3H), 3.75 - 3.52 (m, 20H), 2.41 - 2.10 (m, 4H), 2.10 - 1.55 (m, 12H), 0.84 (s, 9H), 0.74 (t, *J* = 7.3 Hz, 3H). LCMS $[M+H]^+$ =1119.8 |
| **UB-180554** | 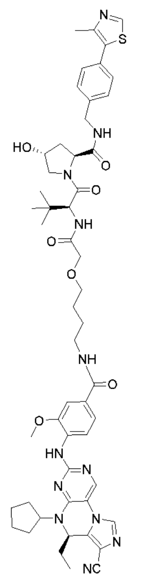 | $^1$H NMR (400 MHz, *d6-DMSO*) δ8.70 (s, 1H), 8.66 - 8.49 (m, 2H), 8.40 (t, *J* = 5.6 Hz, 1H), 8.23 (d, *J* = 8.3 Hz, 1H), 8.00 (s, 1H), 7.58 - 6.95 (m, 6H), 5.27 - 5.07 (m, 2H), 4.28-4.59 (m, 6H), 3.96 - 3.45 (m,14H), 3.36 (s, 6H), 2.46 - 2.26 (m, 3H), 2.26 - 1.60 (m, 16H), 0.94 (d, *J* = 6.9 Hz, 9H), 0.74 (t, *J* = 7.3 Hz, 3H). LCMS $[M+H]^+$ =1119.8 |

(continued)

| Examples | Compound structure | Analyze data |
|---|---|---|
| **UB-180563** | 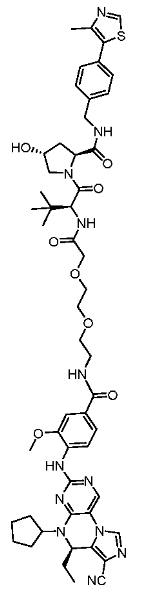 | $^1$H NMR (400 MHz, *d6-DMSO*) δ 8.97 (d, *J* = 9.0 Hz, 1H), 8.73 (s, 1H), 8.70 - 8.53 (m, 3H), 8.24 (d, *J* = 8.4 Hz, 1H), 7.99 (s, 1H), 7.59 - 7.33 (m, 7H), 5.27 - 5.10 (m,2H), 4.57 (d, *J* = 9.6 Hz, 1H), 4.51 - 4.30 (m, 4H), 4.25 (dd, *J* = 15.8, 5.7 Hz, 1H), 3.98 (s,2H), 3.88 (s, 3H), 3.43 - 2.65 (m, 9H), 2.44 (d, *J* = 5.2 Hz, 3H), 2.06-1.55 (m, 12H),, 0.93 (d, *J* = 5.7 Hz,9H), 0.87 - 0.48 (m, 3H). LCMS $[M+H]^+$ =1017.2 |

(2.7) Preparation procedure 7 of TED molecule:

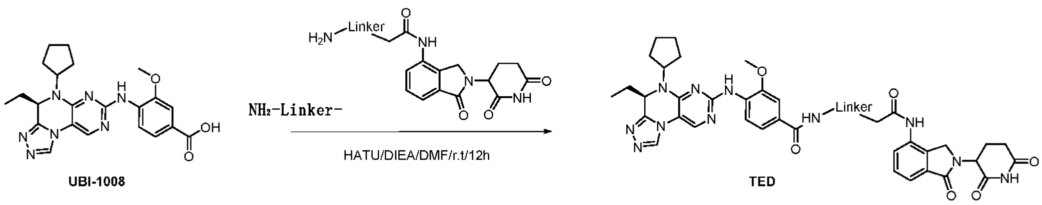


Compound UBI-1008 (20 mg, 0.0436 mmol) was weighed and dissolved in anhydrous DMF (1 mL), added $NH_2$-linker-B(A1) (0.0436 mmol), HATU(33 mg, 0.0872 mmol) and DIEA (0.072mL, 0.436 mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL*3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound.

The conjugate of A-L1-B as shown in Table B7 was prepared through similar method.

Table B7

| Examples | Compound structure | Analyze data |
|---|---|---|
| **UB-180510** | 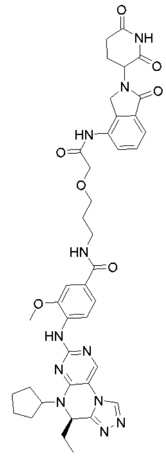 | $^1$H NMR (400 MHz, *d6-DMSO*) δ 10.99 (s, 1H), 9.78 (s, 1H), 9.25 (s, 1H), 8.59 (s, 1H), 8.45 (t, *J* = 5.6 Hz, 1H), 8.25 (d, *J* = 8.7 Hz, 1H), 7.93 (s, 1H), 7.77 (d, *J* = 7.6 Hz, 1H), 7.61 - 7.40 (m, 4H), 5.26 - 5.04 (m, 2H), 4.57 - 4.32 (m, 3H), 4.08 (s, 2H), 3.88 (s, 3H), 3.78 - 3.31 (m, 3H), 3.44 (d, *J* = 10.5 Hz, 2H), 2.92 - 2.83 (m, 3H), 2.70 - 2.43 (m, 2H), 2.14 -1.53 (m, 10H), 0.71 (t, *J* = 7.3 Hz, 3H). LCMS $[M+H]^+$ =792.0 |
| **UB-180511** | 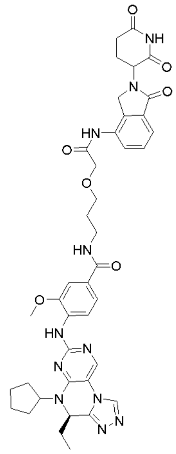 | $^1$H NMR (400 MHz, *d6-DMSO*) δ10.96 (s, 1H), 9.74 (s, 1H), 9.25 (s, 1H), 8.64 (s, 1H), 8.45 (s, 1H), 8.23 (d, *J* = 8.80 Hz, 1H), 7.98 (s, 1H), 7.76 (d, *J*=7.60Hz,1H), 7.52((dt,*J*=15.9,6.0Hz,4H),5.26-5.04 (m, 2H), 412 (s, 2H), 3.88 (s, 3H), 3.67(t,*J*=6.1Hz,2H), 2.97 (m, 1H), 2.70 - 2.33 (m, 2H), 2.01 -1.91(m, 10H), 0.74 (t, *J* = 7.3 Hz, 3H). LCMS $[M+H]^+$ =816.3 |
| **UB-180521** | 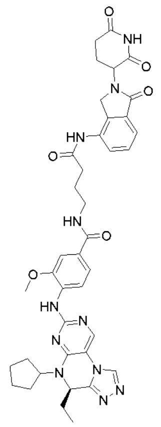 | $^1$H NMR (400 MHz, *d6-DMSO*) δ 11.01 (s, 1H), 9.86 (s, 1H), 9.25 (s, 1H), 8.59 (s, 1H), 8.45 (s, 1H), 8.28 (d, *J* = 8.4 Hz, 1H), 7.94 (s, 1H), 7.81 (d, *J* = 7.0 Hz, 1H), 7.49 (dd, *J* = 15.3, 8.1 Hz, 4H), 5.29 - 4.98 (m, 2H), 4.72 - 3.90 (m, 1H), 3.90 - 3.85 (m, 3H), 2.98 - 2.86 (m, 2H), 2.75 (s, 1H), 2.60 (d, *J* = 17.4 Hz, 2H), 2.57 - 2.31 (m, 3H), 2.05-1.54 (m, 16H), 0.71 (t, *J* = 7.3 Hz, 3H). LCMS $[M+H]^+$ =762.3 |

(continued)

| Examples | Compound structure | Analyze data |
|---|---|---|
| **UB-180524** | 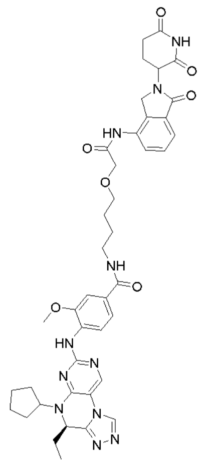 | $^{1}H$ NMR (400 MHz, *d6-DMSO*) δ 11.00 (s, 1H), 9.75 (s, 1H), 9.25 (s, 1H), 8.58 (s, 1H), 8.40 (t, *J* = 5.6 Hz, 1H), 8.26 (d, *J* = 8.2 Hz, 1H), 7.94 (s, 1H), 7.78 - 7.71 (m, 1H), 7.52 (qd, *J* = 7.8, 4.8 Hz, 4H), 5.17-5.08 (m, 2H), 4.69-4.29 (m, 3H), 4.10 (s, 2H), 3.95 (s, 3H), 3.57 (d, *J* = 5.5 Hz, 2H), 3.19 -2.85 (m, 1H), 2.67 (d, *J* = 1.8 Hz, 1H), 2.06- 1.59 (m, 16H), 0.71 (t, *J* = 7.4 Hz, 3H). LCMS $[M+H]^+$ =807.2. |
| **UB-180546** | 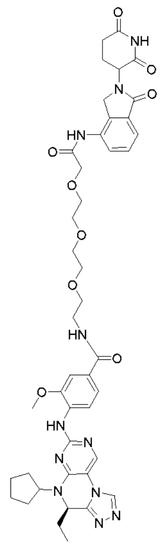 | $^{1}H$ NMR (400 MHz, *d6-DMSO*) δ11.01 (s, 1H), 9.68 (s, 1H), 9.25 (s, 1H), 8.59 (s, 1H), 8.58 (s, 1H), 8.44 (t, *J* = 5.6 Hz, 1H), 8.28 (d, *J* = 8.3 Hz, 1H), 7.94 (s, 1H), 7.61 - 7.43 (m, 4H), 5.17-5.11 (m, 2H),4.50 - 4.29 (m, 3H), 4.29 (s, 2H), 3.97 - 3.48 (m, 13H), 2.41 - 2.27 (m, 2H), 2.07 -1.56 (m, 12H), 0.70 (t, *J* = 7.4 Hz, 3H). LCMS $[M+H]^+$ =866.4 |
| **UB-180547** | 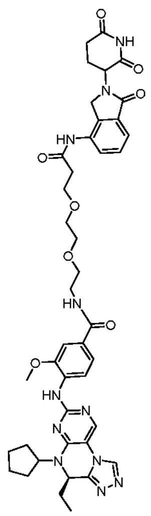 | $^{1}H$ NMR (400 MHz, *d6-DMSO*) δ 8.97 (d, *J* = 9.0 Hz, 1H), 8.73 (s, 1H), 8.70 - 8.53 (m, 3H), 8.24 (d, *J* = 8.4 Hz, 1H), 7.99 (s, 1H), 7.59 - 7.33 (m, 4H), 5.27 - 5.10 (m, 2H), 4.57 (d, *J* = 9.6 Hz,1H), 4.51 - 4.30 (m, 3H), 4.25 (dd, *J* = 15.8, 5.7 Hz, 1H), 3.98 (s, 3H), 3.95 - 3.28 (m, 8H), 2.83 - 2.65 (m, 5H), 2.44 (d, *J* = 5.2 Hz, 2H), 2.06-1.65 (m, 10H), 0.73 (t, *J* = 7.2 Hz, 3H). LCMS $[M+H]^+$ =836.5 |

(continued)

| Examples | Compound structure | Analyze data |
|---|---|---|
| **UB-180549** | 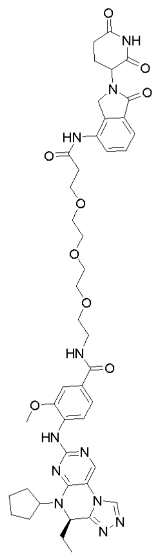 | $^1$H NMR (400 MHz, *d6-DMSO*) δ11.01 (s, 1H), 9.68 (s, 1H), 9.25 (s, 1H), 8.59 (s, 1H), 8.58 (s, 1H), 8.44 (t, *J* = 5.6 Hz, 1H), 8.28 (d, *J* = 8.3 Hz, 1H), 7.94 (s, 1H), 7.57 - 7.33 (m, 4H), 5.17-5.11 (m, 2H), 4.52 - 4.25 (m, 3H), 3.92 (s, 3H), 3.70 (t, *J* = 6.3 Hz, 3H), 3.61 - 3.35 (m, 14H), 2.53 - 2.44 (m, 1H), 2.06-1.60 (m, 11H), 0.92 (s, 9H), 0.70 (t, *J* = 7.4 Hz, 3H). LCMS $[M+H]^+$ =880.4 |
| **UB-180556** | 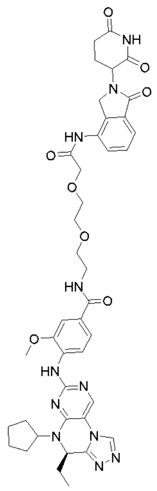 | $^1$H NMR (400 MHz, *d6-DMSO*) δ11.01 (s, 1H), 9.68 (s, 1H), 9.25 (s, 1H), 8.58 (s, 1H), 8.44 (t, *J* = 5.6 Hz, 1H), 8.28 (d, *J* = 8.3 Hz, 1H), 7.95 (s, 1H), 7.74 (d, *J* = 6.9 Hz, 1H), 7.61 - 7.41 (m, 4H), 5.17-5.11 (m, 2H), 4.59 - 4.33 (m, 3H), 4.33 - 4.09 (m, 2H), 3.92 (s, 3H), 3.74 - 3.55 (m, 5H), 2.35 - 1.94 (m, 1H), 2.35 -1.58 (m, 14H), 0.70 (t, *J* = 7.3 Hz, 3H). LCMS $[M+H]^+$ =822.6 |
| **UB-180561** | 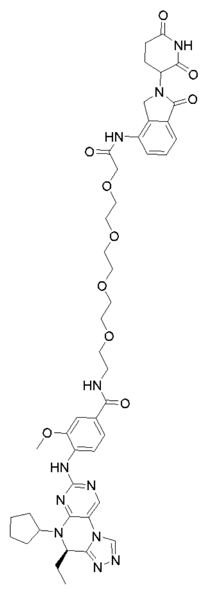 | $^1$H NMR (400 MHz, *d6-DMSO*) δ11.01 (s, 1H), 9.68 (s, 1H), 9.25 (s, 1H), 8.58 (s, 1H), 8.44 (t, *J* = 5.6 Hz, 1H), 8.28 (d, *J* = 8.3 Hz, 1H), 7.94 (s, 1H), 7.57 - 7.33 (m, 4H), 5.17-5.11 (m, 2H),4.52 - 4.25 (m, 3H), 3.92 (s, 3H),,3.77 - 3.45 (m, 15H), 3.45 - 3.21 (m, 3H), 3.01 - 2.78 (m, 1H), 2.07-1.46 (m, 9H), 0.70 (t, *J* = 7.3 Hz, 3H). LCMS $[M+H]^+$ =910.6 |

(continued)

| Examples | Compound structure | Analyze data |
|---|---|---|
| **UB-180562** | 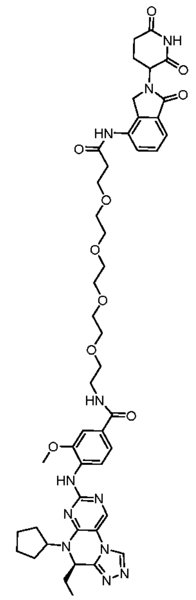 | $^1$H NMR (400 MHz, DMSO) δ 11.02 (s, 1H), 9.84 (s, 1H), 9.25 (s, 1H), 8.59 (s, 1H), 8.45 (t, *J* = 5.5 Hz, 1H), 8.28 (d, *J* = 8.3 Hz, 1H), 7.95 (s, 1H), 7.82 (dd, *J* = 7.1, 1.7 Hz, 1H), 7.56 - 7.31 (m, 4H), 5.18-5.11 (m, 2H), 4.55 - 4.25 (m, 3H), 3.92 (s, 3H), 3.70 (t, *J* = 6.3 Hz, 2H), 3.64 - 2.89 (m, 16H), 3.13 (dd, *J* = 7.3, 4.2 Hz, 1H), 2.92 - 2.85 (m, 1H), 2.60 (t, *J* = 6.3 Hz, 3H), 2.45 - 2.39 (m, 1H), 2.19 - 1.55 (m, 11H), 0.70 (t, *J* = 7.4 Hz, 3H). LCMS $[M+H]^+$ =924.7. |

(2.8) Preparation procedure 8 of TED molecule:

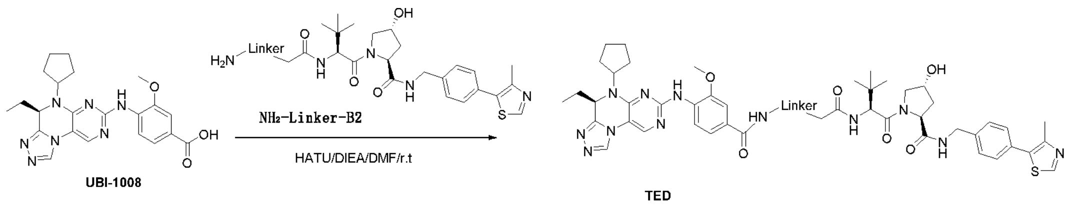


Compound UBI-1008 (20 mg, 0.0436 mmol) was weighed and dissolved in anhydrous DMF (1 mL), added $NH_2$-linker-B(B1) (0.0436 mmol), HATU(33 mg, 0.0872 mmol) and DIEA (0.072mL, 0.436 mmol) under ice-water bath successively. After completion of addition, the material system was stirred and reacted at room temperature under the protection of nitrogen for 18 hours. After cpmpletion of reaction, the reaction solution was poured into 5 mL of water and extracted with ethyl acetate (5 mL*3) three times. The organic phases were combined and washed with saturated brine, dried over anhydrous $Na_2SO_4$ and concentrated by rotary evaporation under reduced pressure to obtain the crude product. After that, the crude product was isolated by thin-layer chromatography on silica gel plate with the polarity of the developing solvent (DCM/MeOH = 10/1) to prepare the target compound.

The conjugate of A-L1-B as shown in Table B8 was prepared through similar method.

Table B8

| Examples | Compound structure | Analyze data |
|---|---|---|
| **UB-180516** | 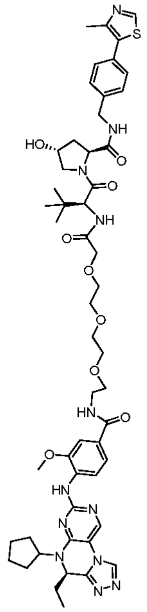 | $^1$H NMR (400 MHz, *d6-DMSO*) $\delta$9.25 (s, 1H), 8.97 (s, 1H), 8.74 - 8.57 (m, 2H), 8.46 (s, 1H), 8.25 (d, *J* = 8.2 Hz, 1H), 8.01 (s, 1H), 7.58 - 7.35 (m, 5H), 7.35 - 7.32 (m, 2H), 5.27 - 5.14 (m, 2H), 4.62 -4.17 (m, 6H), 3.94-3.89 (m, 5H), 3.81 - 3.43 (m, 11H), 2.52 (s, 3H), 2.04 - 1.55 (m, 12H), 0.94 (s, 9H), 0.74 (t, *J* = 7.2 Hz, 3H). LCMS $[M+H]^+$ =1037.5 |
| **UB-180522** | 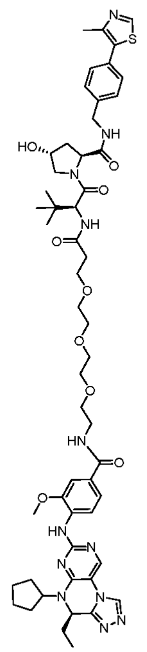 | $^1$H NMR (400 MHz, *d6-DMSO*) $\delta$ 9.25 (s, 1H), 8.98 (s, 1H), 8.61-8.46 (m, 2H), 8.45 (s, 1H), 8.28 (d, *J* = 8.2 Hz, 1H), 7.98 - 7.82 (m, 2H), 7.51 (d, *J* = 10.1 Hz, 2H), 7.40 (q, *J* = 8.3 Hz, 4H), 5.28 - 5.16 (m, 1H), 5.13 (d, *J* = 3.5 Hz, 1H), 4.52 (t, *J* = 19.4 Hz, 2H), 4.42 (dd, *J* = 9.4, 6.2 Hz, 3H), 4.29 (d, *J* = 42.2 Hz, 2H), 4.24 - 4.01 (m, 2H), 3.92 (s, 3H), 3.67 - 2.65 (m, 9H), 2.48 (s, 3H), 2.02-1.57 (m, 11H), 0.92 (s, 9H), 0.70 (t, *J* = 7.3 Hz, 3H). LCMS $[M+H]^+$ =1051.6. |

(continued)

| Examples | Compound structure | Analyze data |
|---|---|---|
| **UB-180523** | 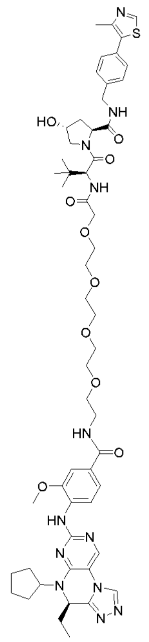 | $^1$H NMR (400 MHz, *d6-DMSO*) δ 9.25 (s, 1H), 8.98 (d, *J* = 2.7 Hz, 1H), 8.67 - 8.56 (m, 2H), 8.45 (t, *J* = 5.6 Hz, 1H), 8.28 (d, *J* = 8.2 Hz, 1H), 7.94 (s, 1H), 7.56 - 7.45 (m, 2H), 7.45 - 7.20 (m, 5H), 5.27 - 5.11 (m, 2H), 4.56 (d, *J* = 9.6 Hz, 1H), 4.45 (d, *J* = 8.3 Hz, 1H), 4.41 (d, *J* = 6.8 Hz, 2H), 4.00-3.88 (m, 5H), 3.69 - 3.46 (m, 9H), 2.41 (s, 3H), 2.18 - 1.56 (m, 11H), 1.48 (s, 9H), 0.69 (d, *J* = 7.4 Hz, 2H). LCMS $[M+H]^+$ =1081.5 |
| **UB-180539** | 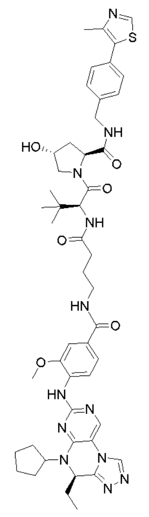 | $^1$H NMR (400 MHz, *d6-DMSO*) δ 9.25 (s, 1H), 8.96 (d, *J* = 12.9 Hz, 1H), 8.61 - 8.49 (m, 2H), 8.40 (t, *J* = 5.5 Hz, 1H), 8.28 (d, *J* = 8.3 Hz, 1H), 8.03 - 7.89 (m, 2H), 7.50 (dd, *J* = 10.2, 1.7 Hz, 2H), 7.40 (q, *J* = 8.4 Hz, 4H), 5.21 (dd, *J* = 7.8, 3.3 Hz, 1H), 5.14 (d, *J* = 3.6 Hz, 1H), 4.61 - 4.25 (m, 5H), 4.22 (dd, *J* = 15.9, 5.4 Hz, 1H), 3.92 (d, *J* = 5.5 Hz, 3H), 3.77 - 3.54 (m, 2H),2.47(s,3H), 2.33 - 2.09 (m, 2H), 2.05-1.39 (m, 12H), 0.94 (d, *J* = 8.4 Hz, 9H), 0.71 (t, *J* = 7.4 Hz, 3H). LCMS $[M+H]^+$ =933.6 |

(continued)

| Examples | Compound structure | Analyze data |
|---|---|---|
| **UB-180540** | 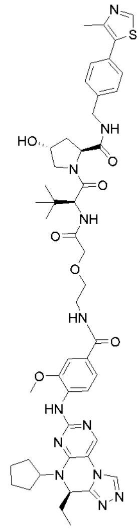 | $^1$H NMR (400 MHz, *d6-DMSO*) δ 9.25 (s, 1H), 8.96 (s, 1H), 8.61 - 8.42 (m, 3H), 8.28 (d, *J* = 8.4 Hz, 1H), 7.93 (s, 1H), 7.53 (dd, *J* = 11.7, 5.8 Hz, 3H), 7.45 (s, 4H), 5.27 - 5.12 (m, 2H), 4.57 (d, *J* = 9.6 Hz, 1H), 4.37-4.26 (m, 5H), 4.23 (d, *J* = 5.6 Hz, 1H), 4.16 - 3.90 (m, 5H), 3.87 - 3.44 (m, 6H), 2.43 (s, 3H), 2.07-1.59 (m, *11* H), 0.70 (t, *J* = 7.4 Hz, 3H). LCMS $[M+H]^+$ =949.5 |
| **UB-180541** | 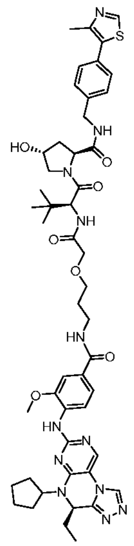 | $^1$H NMR (400 MHz, *d6-DMSO*) δ 9.25 (s, 1H), 8.96 (d, *J* = 6.3 Hz, 1H), 8.59 (d, *J* = 5.5 Hz, 2H), 8.44 (s, 1H), 8.27 (d, *J* = 8.3 Hz, 1H), 7.94 (s, 1H), 7.56 - 7.37 (m, 7H), 5.26 - 5.12 (m, 2H), 4.57 (d, *J* = 9.6 Hz, 1H), 4.51 - 4.27 (m, 5H), 4.24 (d, *J* = 5.5 Hz, 1H), 3.94-3.92 (m, 5H), 3.67 - 3.52 (m, 4H), 2.09 -1.60 (m,13H), 1.34 - 1.11 (m, 2H), 0.93 (d, *J* = 5.9 Hz, 9H), 0.70 (t, *J* = 7.3 Hz, 3H). LCMS $[M+H]^+$ =963.4 |

(continued)

| Examples | Compound structure | Analyze data |
|---|---|---|
| UB-180550 | 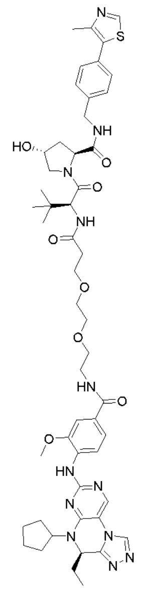 | $^1$H NMR (400 MHz, *d6-DMSO*) δ 9.25 (s, 1H), 8.85 (d, *J* = 10.9 Hz, 1H), 8.61 - 8.51 (m, 2H), 8.46 (t, *J* = 5.5 Hz, 1H), 8.27 (d, *J* = 8.3 Hz, 1H), 8.11 - 7.86 (m, 2H), 7.52 (d, *J* = 2.7 Hz, 2H), 7.43 (dt, *J* = 15.7, 5.1 Hz, 4H), 5.20-5.15 (m, 1H), 5.14 (s, 1H), 4.55 (d, *J* = 9.4 Hz, 1H), 4.50 - 4.31 (m, 4H), 4.31- 4.13 (m, 1H), 3.92 (s, 3H), 3.85 - 3.41 (m, 13H), 3.14 (td, *J* = 7.4, 4.3 Hz, 2H), 2.47 (s, 3H), 2.09 (s, 4H), 2.09 - 1.51 (m, 11H), 0.92 (d, *J* = 7.1 Hz, 9H), 0.70 (t, *J* = 7.4 Hz, 3H). LCMS $[M+H]^+$ =1007.4 |
| UB-180553 | 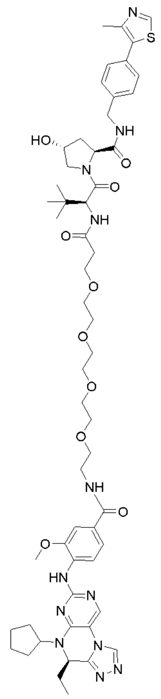 | $^1$H NMR (400 MHz, *d6-DMSO*) δ 9.25 (s, 1H), 8.98 (s, 1H), 8.61 - 8.51 (m, 2H), 8.46 (t, *J* = 5.6 Hz, 1H), 8.28 (d, *J* = 8.3 Hz, 1H), 7.98 - 7.84 (m, 2H), 7.51 (dd, *J* = 10.2, 1.8 Hz, 2H), 7.46 - 7.32 (m, 4H), 5.21 (dd, *J* = 7.8, 3.3 Hz, 1H), 5.13 (d, *J* = 3.5 Hz, 1H), 4.60 - 4.32 (m, 5H), 4.21 (dd, *J* = 15.8, 5.3 Hz, 1H), 3.93 (s, 3H), 3.80 - 3.46 (m, 20H), 2.42 - 2.19 (m, 4H), 2.02-1.60 (m, 12H), 0.92 (d, *J* = 8.1 Hz, 9H), 0.70 (t, *J* = 7.4 Hz, 3H). LCMS $[M+H]^+$ =1095.8 |

(continued)

| Examples | Compound structure | Analyze data |
|---|---|---|
| **UB-180555** | 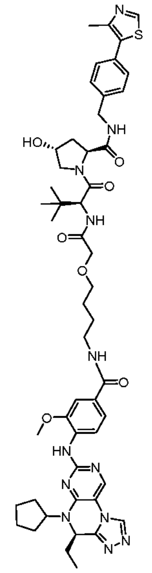 | $^1$H NMR (400 MHz, *d6-DMSO*) δ9.25 (s, 1H), 8.98 (s, 1H), 8.61 - 8.51 (m, 2H),8.43 - 8.30 (m, 1H), 8.26 (d, *J* = 8.2 Hz, 1H), 7.94(s, 1H), 7.57 - 7.38 (m, 7H), 5.29 - 5.12 (m, 4H), 4.58 (t, *J* = 14.8 Hz, 1H), 4.53 - 4.25 (m, 5H), 3.98 - 3.85 (m, 5H), 3.71 - 3.57 (m, 8H), 3.17 (s, 1H), 2.12-1.76 (m, 12H), 0.93 (d, *J* = 7.1 Hz, 9H), 0.70 (t, *J* = 7.4 Hz, 3H). LCMS $[M+H]^+$=977.8 |
| **UB-180564** | 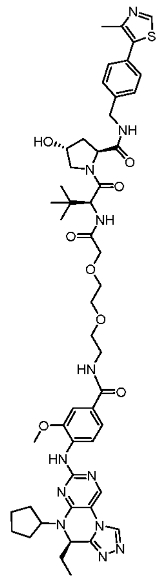 | $^1$H NMR (400 MHz, *d6-DMSO*) δ 9.25 (s, 1H), 8.97 (s, 1H), 8.65 (s, 2H), 8.44 (s, 1H), 8.27 (d, *J* = 8.3 Hz, 1H), 7.94 (s, 1H), 7.46 - 7.13 (m, 7H), 5.28 - 4.59 (m, 2H), 4.57 (d, *J* = 9.6 Hz, 1H), 4.46-4.39 (m, 4H), 4.25 (dd, *J* = 15.7, 5.6 Hz, 1H), 3.98 (s, 2H), 3.91 (s, 3H), 3.86 - 3.69 (m, 10H), 2.93 (s, 3H), 2.01-1.59 (m, 11H), 0.93 (d, *J* = 6.5 Hz, 9H), 0.70 (t, *J* = 7.4 Hz, 3H). LCMS $[M+H]^+$ =994.5 |

Compounds in Table B10 were prepared by preparation procedures similar to (2.5), (2.6), (2.7) or (2.8)

**Table B10**

| Examples | Structure and data analysis |
|---|---|
| **UB-180551 (51)** | 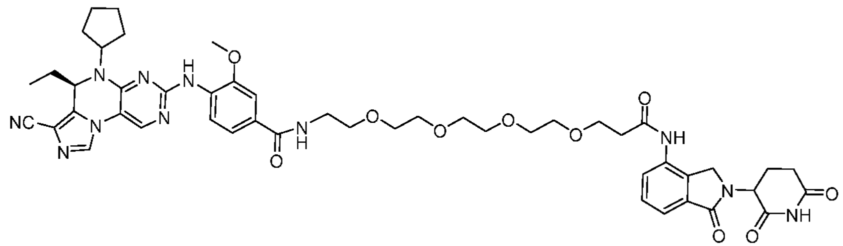 |
| | $^1H$ NMR (400 MHz, DMSO-$d_6$) δ 11.01 (s, 1H), 9.86 (s, 1H), 8.70 (s, 1H), 8.64 (s, 1H), 8.45 (t, *J* = 5.7 Hz, 1H), 8.25 (d, *J* = 8.2 Hz, 1H), 8.00 (s, 1H), 7.81 (dd, *J* = 7.1, 1.7 Hz, 1H), 7.49 (dd, *J* = 15.4, 8.2 Hz, 4H), 5.27 - 5.07 (m, 2H), 4.55 - 4.28 (m, 3H), 3.92 (s, 3H), 3.53 - 3.32 (m, 7H), 3.12 - 2.86 (m, 3H), 2.41-2.32 (m, 3H), 2.07 - 1.76 (m, 18H), 0.86 - 0.79(m,3H), 0.74 (t, *J* = 7.3 Hz, 3H). LCMS $[M+H]^+$ =948.7 |
| **UB-180552 (52)** | 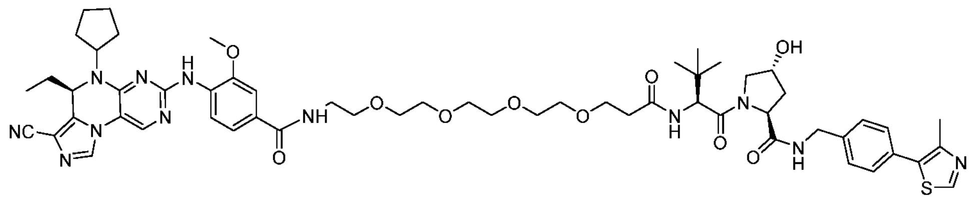 |
| | $^1H$ NMR (400 MHz, DMSO-$d_6$) δ 11.01 (s, 1H), 9.70 (s, 1H), 8.70 (s, 1H), 8.64 (s,1H), 8.45 (t, *J* = 5.6 Hz, 1H), 8.24 (d, *J* = 8.3 Hz, 1H), 7.99 (s, 1H), 7.77 - 7.70 (m, 1H), 7.52 (ddd, *J* = 11.4, 10.5, 4.6 Hz, 4H), 5.24 - 5.07 (m, 2H), 4.28-4.59 (m, 6H), 3.91 (s, 3H), 3.75 - 3.52 (m, 20H), 2.41 - 2.10 (m, 4H), 2.10 - 1.55 (m, 12H), 0.84 (s, 9H), 0.74 (t, *J* = 7.3 Hz, 3H). LCMS $[M+H]^+$ =1119.8 |
| **UB-180554 (54)** | 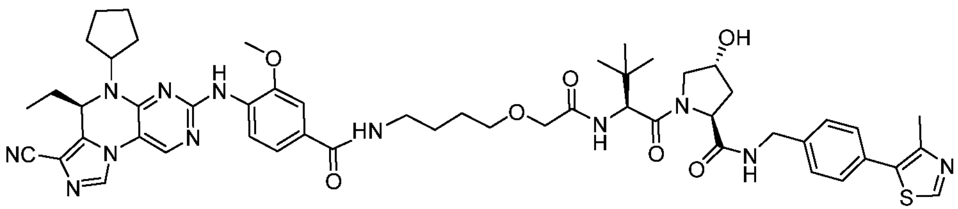 |
| | $^1H$ NMR (400 MHz, DMSO-$d_6$) δ8.70 (s, 1H), 8.66 - 8.49 (m, 2H), 8.40 (t, *J* = 5.6 Hz, 1H), 8.23 (d, *J* = 8.3 Hz, 1H), 8.00 (s, 1H), 7.58 - 6.95 (m, 6H), 5.27 - 5.07 (m, 2H), 4.28-4.59 (m, 6H), 3.96 - 3.45 (m,14H), 3.36 (s, 6H), 2.46 - 2.26 (m, 3H), 2.26 - 1.60 (m, 16H), 0.94 (d, *J* = 6.9 Hz, 9H), 0.74 (t, *J* = 7.3 Hz, 3H). LCMS $[M+H]^+$=1119.8 |

(continued)

| Examples | Structure and data analysis |
|---|---|
| **UB-180557 (57)** | 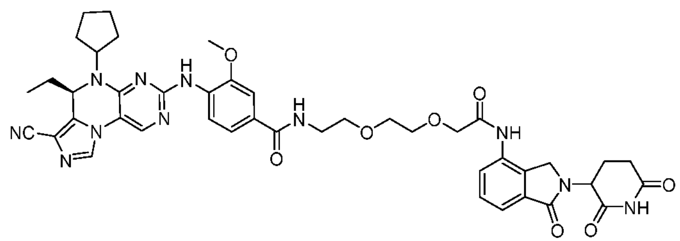 |
| | $^1$H NMR (400 MHz, DMSO-$d_6$) δ 11.00 (s, 1H), 9.70 (s, 1H), 8.70 (s, 1H), 8.64 (s, 1H), 8.45 (t, $J$ = 5.4 Hz, 1H), 8.24 (d, $J$ = 8.2 Hz, 1H), 7.99 (s, 1H), 7.73 (d, $J$ = 7.6 Hz, 1H), 7.62 - 7.40 (m, 4H), 5.27 - 5.04 (m, 2H), 4.65 - 4.23 (m, 3H), 4.14 (s, 2H), 3.91 (s, 3H), 3.80 - 3.60 (m, 5H), 3.58 (d, $J$ = 5.8 Hz, 2H), 3.04 - 2.76 (m, 1H), 2.71 - 2.57 (m, 1H), 2.07 - 1.59 (m, 11H), 0.74 (t, $J$ = 7.3 Hz, 3H). LCMS $[M+H]^+$ =846.4 |
| **UB-180560 (60)** | 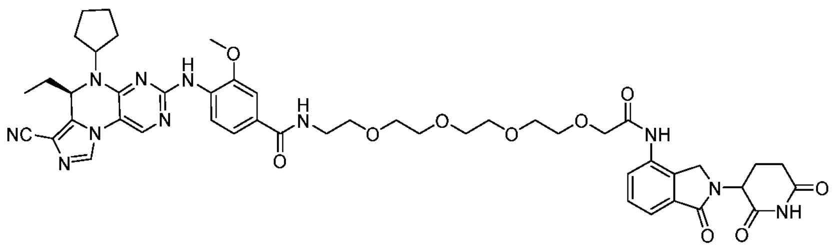 |
| | $^1$H NMR (400 MHz, DMSO-$d_6$) δ 11.00 (s, 1H), 9.68 (s, 1H), 8.70 (s, 1H), 8.63 (s, 1H), 8.44 (s, 1H), 8.25 (d, $J$ = 8.3 Hz, 1H), 8.00 (s, 1H), 7.73 (d, $J$ = 7.7 Hz, 1H), 7.52 (dt, $J$ = 8.5, 5.3 Hz, 4H), 5.23 - 5.04 (m, 2H), 4.49 - 4.22 (m, 3H), 4.13 (s, 2H), 3.92 (s, 3H), 3.68 (dd, $J$ = 5.8, 3.2 Hz, 2H), 3.65 - 3.42 (m, 13H), 3.03 - 2.72 (m, 1H), 2.64 (d, $J$ = 22.0 Hz, 1H), 2.41 - 2.08 (m, 2H), 2.08 - 1.88 (m, 5H), 1.79 - 1.71 (m, 3H), 1.61 - 1.26 (m, 3H), 1.46 - 1.26 (m, 1H), 1.46 - 1.05 (m, 2H), 0.74 (t, $J$ = 7.4 Hz, 3H). LCMS $[M+H]^+$ =934.4 |
| **UB-180566 (66)** | 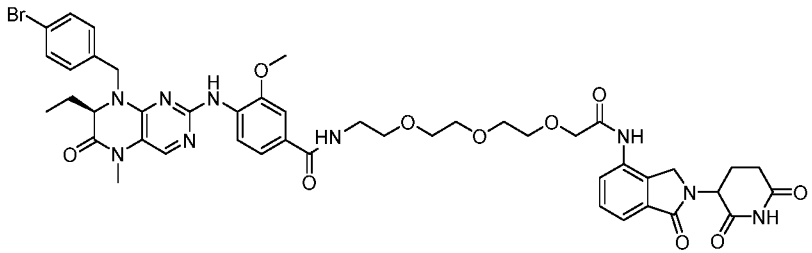 |

(continued)

| Examples | Structure and data analysis |
| --- | --- |
| | $^1H$ NMR (400 MHz, DMSO-$d_6$) δ11.01 (s, 1H), 9.71(s, 1H), 8.37-8.32(m,1H),8.25 (d, *J* = 8.2 Hz, 1H), 7.85 (s, 1H), 7.62 - 7.41(m, 5H), 7.38 (dt, *J* = 28.2, 20.4 Hz, 1H), 5.25 (dd, *J* = 12.8, 5.4 Hz, 1H), 5.21-5.14 (m, 1H),4.41 - 4.26 (m, 3H), 4.23 - 4.00 (m, 3H), 3.84 (m, 1H), 3.77 -3.59 (m, 11H), 2.89-2.67 (m, 1H), 2.06 - 1.97 (m, 2H), 1.75 - 1.66 (m, 2H), 0.82 - 0.78 (m, 2H), 0.76 (t, *J* = 7.4 Hz, 3H). LCMS $[M+H]^+$ =956.3 |
| **UB-180567 (67)** | 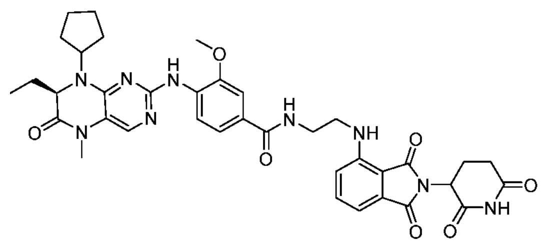 |
| | $^1H$ NMR (400 MHz, DMSO-$d_6$) δ 11.09 (s, 1H), 8.59 (s, 1H), 8.42 (d, *J* = 8.2 Hz, 1H), 7.85 (s, 1H), 7.64 - 7.53 (m, 2H), 7.47 (d, *J* = 8.9 Hz, 2H), 7.31 (t, *J* = 11.1 Hz, 1H), 7.03 (d, *J* = 7.0 Hz, 1H), 6.85 (d, *J* = 6.1 Hz, 1H), 5.05 (dd, *J* = 12.8, 5.4 Hz, 1H), 4.59 - 4.19 (m, 2H), 3.93 (s, 3H), 3.70 - 3.34 (m, 4H), 3.31 - 3.21 (m, 5H), 2.89-2.75 (m, 1H), 2.64 - 2.54 (m, 1H), 2.05 - 1.51 (m, 11H), 0.76 (t, *J* = 7.4 Hz, 3H). LCMS $[M+H]^+$ =725.3 |
| UB-180568 (68) | 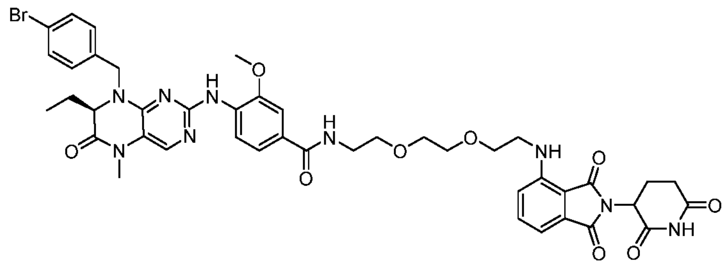 |
| | $^1H$ NMR (400 MHz, chloroform-d) δ 8.55 (s, 1H), 8.29 (s, 1H), 7.71 (s, 1H), 7.45 (m, 3H), 7.20 (m, 2H), 7.07 (d, 1H), 6.83 (d, 1H), 6.52 (m, 1H), 5.50 (d, 1H), 4.79 (m, 1H), 4.10 (m, 2H), 3.93 (s, 3H), 3.70 (m, 8H), 3.33 (m, 4H), 2.70 (m, 3H), 2.03 (d, 2H), 1.92 (m, 1H), 1.80 (m, 1H), 1.25 (m, 7H), 0.80 (m, 3H).LCMS $[M+H]^+$ =912.6 |

(continued)

| Examples | Structure and data analysis |
|---|---|
| **UB-180569 (69)** | 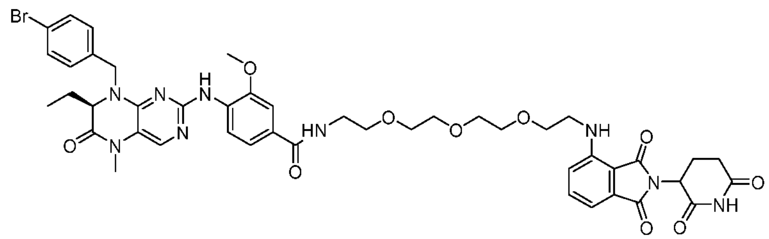 |
| | $^1$H NMR (400 MHz, DMSO-$d_6$) δ 8.68 (d, $J$ = 11.3 Hz, 1H), 8.38 (d, $J$ = 8.4 Hz, 1H), 7.70 (d, $J$ = 21.3 Hz, 2H), 7.40-7.53 (m, 3H), 7.19 (s, 2H), 7.08 (d, $J$ = 7.1 Hz, 1H), 6.86 (d, $J$ = 8.5 Hz, 1H), 6.73 (s, 1H), 6.44 (d, $J$ = 5.0 Hz, 1H), 5.50 (d, $J$ = 15.3 Hz, 1H), 4.90 (m, 1H), 4.10-4.22 (m, 2H), 3.93 (d, $J$ = 1.5 Hz, 2H), 3.59-3.78 (m, 12H), 3.26-3.46 (m, 5H), 2.61-2.98 (m, 3H), 2.03-2.19 (m, 1H), 1.88-1.97 (m, 1H), 1.74-1.86 (m, 1H), 0.84 (t, $J$ = 7.4 Hz, 3H). LCMS $[M+H]^+$ =956.5. |
| **UB-180570 (70)** | 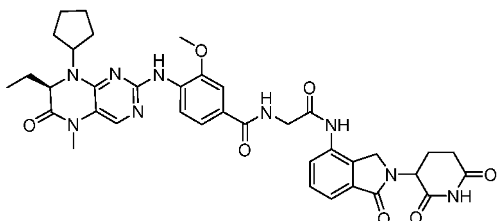 |
| | $^1$H NMR (400 MHz, DMSO-$d_6$) δ 11.03 (s, 1H), 9.97 (s, 1H), 8.76 (t, $J$ = 5.8 Hz, 1H), 8.47 (d, $J$ = 8.9 Hz, 1H), 7.91 - 7.79 (m, 3H), 7.63 (s, 1H), 7.62 - 7.20 (m, 6H), 5.16 (dd, $J$ = 13.3, 5.1 Hz, 1H), 4.51 - 4.29 (m, 4H), 4.29 - 4.14 (m, 2H), 3.95 (s, 3H), 3.25 (s, 3H), 3.00 - 2.86 (m, 1H), 2.04 - 1.58 (m, 11H), 0.77 (t, $J$ = 7.4 Hz, 3H). LCMS $[M+H]^+$ =725.3 |
| **UB-180571 (71)** | 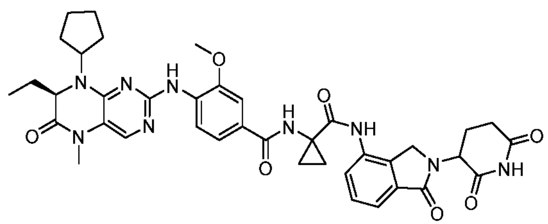 |
| | $^1$H NMR (400 MHz, DMSO-$d_6$) δ 11.09 (s, 1H), , 9.81 (s, 1H), 8.96 (s, 1H), 8.47 (d, $J$ = 8.9 Hz, 1H), 8.42 (d, $J$ = 8.2 Hz, 1H), 7.85 (s, 1H), 7.64 - 7.53 (m, 6H), 5.05 (dd, $J$ = 12.8, 5.4 Hz, 1H), 4.59 - 4.19 (m, 4H), 3.93 (s, 3H), 3.24 (s, 3H), 2.89-2.75 (m, 1H), 2.64 - 2.54 (m, 1H), 2.54 - 2.46 (m, 52H), 2.33 (s, 2H), 2.05 - 1.51 (m, 13H), 1.26 (dd, $J$ = 14.4, 10.8 Hz, 3H), 0.76 (t, $J$ = 7.4 Hz, 3H).LCMS $[M+H]^+$ =750.58 |

(continued)

| Examples | Structure and data analysis |
|---|---|
| **UB-180579 (79)** | 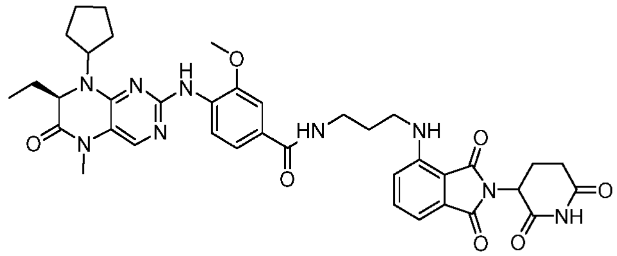 |
| | $^1$H NMR (400 MHz, DMSO-$d_6$) δ 11.09 (s, 1H), 8.49-8.40 (m,2H), 7.85 (s, 1H), 7.64 - 7.53 (m, 2H), 7.47 (d, *J* = 8.9 Hz, 2H), 7.31 (t, *J* = 11.1 Hz, 1H), 7.03 (d, *J* = 7.0 Hz, 1H), 6.85 (d, *J* = 6.1 Hz, 1H), 5.05 (dd, *J* = 12.8, 5.4 Hz, 1H), 4.59 - 4.19 (m, 2H), 3.93 (s, 3H), 3.47 - 3.44 (m, 3H), 3.27 - 3.24 (m, 3H),3.21 (s, 3H), 2.89-2.81 (m, 1H), 2.05 - 1.51 (m, 13H), 0.76 (t, *J* = 7.4 Hz, 3H). LCMS [M+H]$^+$ =739.5 |
| **UB-180591 (91)** | 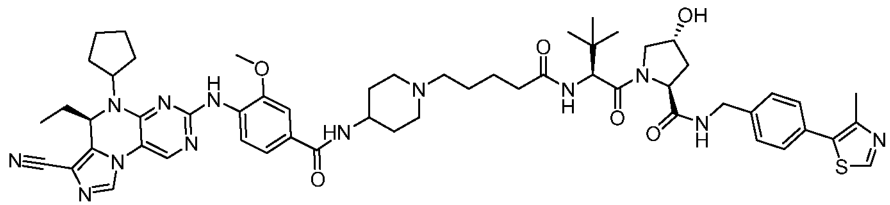 |
| | $^1$H NMR (400 MHz, DMSO-$d_6$) δ 8.98 (s, 1H), 8.70 (s, 1H), 8.64 (s, 1H), 8.57 (m, 1H), 8.24 (d, *J* = 8.0 Hz, 1H), 8.14 (m, 1H), 7.99 (m, 1H), 7.86 (m, 1H), 7.50 (m, 1H), 7.40 (m, 3H), 7.21 (m, 2H), 6.66 (m, 1H), 5.32 (m, 2H), 5.15 (m, 1H), 4.55 (m, 1H), 4.47 (m, 1H), 4.47 (m, 2H),4.36 (m, 1H), 3.93 (s, 3H), 2.89 (m, 2H), 2.44 (s, 3H), 2.31 (m, 2H), 2.08-1.87 (m, 8H), 1.77 (m, 4H), 1.46 (m, 10H), 0.94 (s, 9H), 0.85 (t, *J* = 8.0 Hz, 3H), 0.74 (t, *J* = 8.0 Hz, 3H). LCMS [M+H]$^+$ = 1054.7 |
| **UB-180592 (92)** | 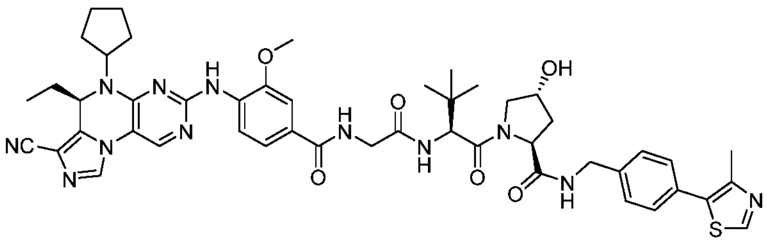 |
| | $^1$H NMR (400 MHz, DMSO-$d_6$) δ 8.96 (d, *J* = 6.3 Hz, 1H), 8.71 (d, *J* = 6.2 Hz, 2H), 8.67 - 8.44 (m, 2H), 8.27 (d, *J* = 8.1 Hz, 1H), 8.02 (s, 1H), 7.86 (d, *J* = 9.3 Hz, 1H), 7.60 - 7.37 (m, 6H), 5.29 - 5.10 (m, 2H), 4.56 (d, *J* = 9.3 Hz, 1H), 4.56-4.09 (m, 6H), 4.09 - 3.97 (m, 2H),3.93(s,3H), 3.73 - 3.60 (m, 2H), 2.43 (s, 3H), 2.18 -1.67 (m, 12H), 0.94 (s, 9H), 0.74 (t, *J* = 7.3 Hz, 3H). LCMS [M+H]$^+$ =950.3 |

(continued)

| Examples | Structure and data analysis |
|---|---|
| **UB-180593 (93)** | 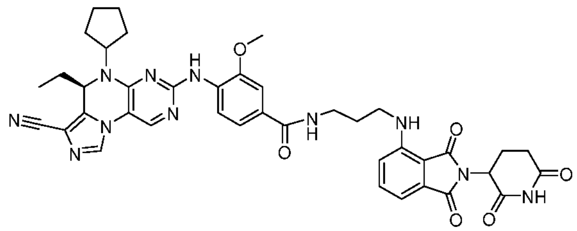 |
| | $^1$H NMR (400 MHz, DMSO-$d_6$) δ 11.10 (s, 1H), 8.70 (s, 1H), 8.64 (s, 1H), 8.49 (s, 1H), 8.24 (d, *J* = 8.1 Hz, 1H), 8.01 (s, 1H), 7.71 - 7.44 (m, 3H), 7.12 (d, *J* = 8.7 Hz, 1H), 7.03 (d, *J* = 7.1 Hz, 1H), 6.77 (s, 1H), 5.16 (s, 2H), 5.05 (dd, *J* = 12.1, 4.9 Hz, 1H), 4.45 - 4.36 (m, 1H), 3.93(s,3H), 2.87 (d, *J* = 14.0 Hz, 2H), 2.44 - 2.36 (m, 1H), 2.03- 1.55 (m, 15H), 0.74 (t, *J* = 6.8 Hz, 3H). LCMS $[M+H]^+$ =773.3 |
| **UB-180594 (94)** | 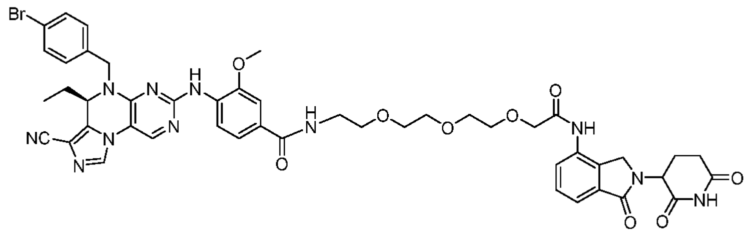 |
| | $^1$H NMR (400 MHz, DMSO-$d_6$) δ11.01 (s, 1H), 9.70 (s, 1H),8.96 (d, *J* = 6.3 Hz, 1H), 8.71 (d, *J* = 6.2 Hz, 1H), 8.67 - 8.44 (m, 2H), 8.27 (d, *J* = 8.1 Hz, 1H), 8.02 (s, 1H), 7.86 (d, *J* = 9.3 Hz, 1H), 7.60 - 7.37 (m, 5H), 7.36 (s, 1H), 5.29 - 5.10 (m, 3H), 4.56 (d, *J* = 9.3 Hz, 1H), 4.41 - 4.29 (m, 2H), 4.21(s,3H),3.93(S, 3H), 3.73 - 3.40 (m, 13H), 2.87-2.81 (m, 1H), 2.43-2.33 (s, 2H), 2.05-1.95 (m, 1H), 1.92 (d, *J* = 8.5 Hz, 2H), 0.74 (t, *J* = 7.3 Hz, 3H). LCMS $[M+H]^+$ =992.5 |
| **UB-180595 (95)** | 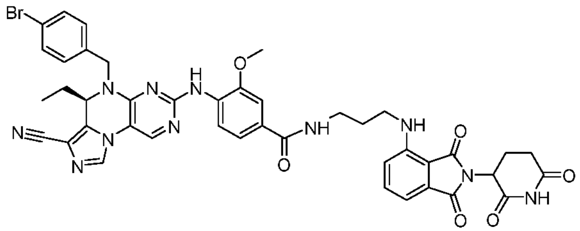 |
| | $^1$H NMR (400 MHz, DMSO-$d_6$) δ 11.09 (s, 1H), 8.75 (s, 1H), 8.70 (s, 1H), 8.46 (t, *J* = 5.6 Hz,1H), 8.17 - 7.99 (m, 2H), 7.68 - 7.50 (m,4H), 7.50 - 7.40 (m, 1H), 7.36 (t, *J* = 14.5 Hz, 2H), 7.12 (d, *J* = 8.7 Hz, 1H), 7.03 (d, *J* = 7.1 Hz, 1H), 6.76 (t, *J* = 6.0 Hz, 1H), 5.32 - 5.11 (m, 3H), 5.05 (dd, *J* = 12.9, 5.3 Hz, 1H), 3.91 (s, 3H),2.89 - 2.76 (m, 1H), 2.29 - 1.96 (m, 1H), 1.83 (dt, *J* = 13.8, 6.9 Hz, 5H), 0.72 (t, *J* = 7.3 Hz, 3H).LCMS $[M+H]^+$ =872.2 |

(continued)

| Examples | Structure and data analysis |
|---|---|
| **UB-180596 (96)** | 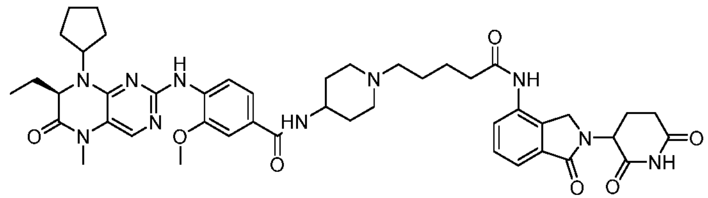 |
| | $^1$H NMR (400 MHz, DMSO-$d_6$) δ 11.03 (s, 1H), 9.88 (s, 1H), 8.43-7.40 (d, $J$ = 12 Hz, 1H), 8.16 (m, 1H), 7.85-7.81 (m, 2H), 7.60 (s, 1H), 7.51-7.48 (m, 2H), 5.17-5.13 (m, 1H), 4.40-4.32 (m, 2H), 4.25-4.22 (m, 1H), 3.94 (s, 3H), 3.83 (m, 2H), 3.25 (s, 3H), 3..02-2.96 (m, 5H), 2.45-2.32 (m, 6H), 2.01-1.73 (m, 11H), 1.67-1.62 (m, 8H), 0.78 (t, $J$ = 14.7, 8.0 Hz, 3H). LCMS $[M+H]^+$ =850.4 |
| **UB-180597 (97)** | 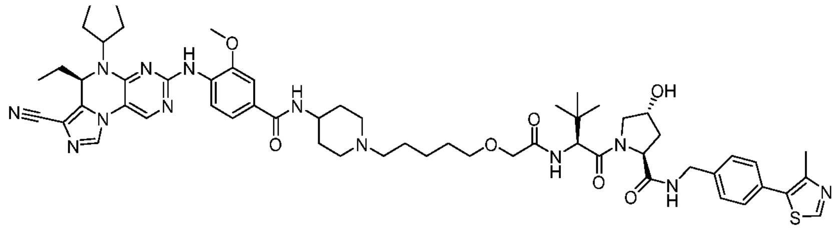 |
| | $^1$H NMR (400 MHz, DMSO-$d_6$) δ 8.98 (s, 1H), 8.70 (s, 1H), 8.64 (m, 2H), 8.25 (m, 2H), 8.00 (s, 1H), 7.56-7.46 (m, 2H), 7.40 (m, 4H), 7.21 (m, 1H), 6.66 (m, 1H), 6.53 (m, 1H), 5.32 (m, 3H), 5.18 (m, 1H), 4.56 (m, 1H), 4.47-4.41 (m, 2H), 4.36 (m, 2H), 4.29 (m, 1H), 3.93 (s, 3H), 3.64 (m, 3H), 2.44 (s, 3H), 1.99 (m, 10H), 1.76 (m, 4H), 1.64-1.50 (m, 4H), 1.45 (m, 4H), 0.95 (s, 9H), 0.90-0.83 (m, 4H), 0.74 (t, $J$ = 8.0 Hz, 3H). LCMS $[M+H]^+$ =1098.8 |
| **UB-180598 (98)** | 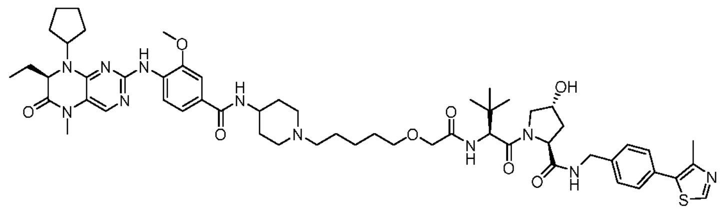 |
| | $^1$H NMR (400 MHz, DMSO-$d_6$) δ 8.98 (s, 1H), 8.65 (m, 1H), 8.42 (m, 1H), 8.30 (m, 1H), 7.92 (m, 1H), 7.85 (s, 1H), 7.65 (m, 1H), 7.61 (s, 1H), 7.52 (m, 1H), 7.39 (m, 4H), 5.32 (m, 1H), 5.19 (m, 1H), 4.56 (m, 1H), 4.47-4.22 (m, 7H), 3.94 (m, 4H), 3.64 (m, 2H), 3.48 (m, 4H), 3.25 (s, 3H), 2.44 (s, 3H), 2.09-1.95 (m, 4H), 1.93-1.86 (m, 6H), 1.78 (m, 5H), 1.61 (m, 8H), 0.95 (s, 9H), 0.84 (m, 3H), 0.76 (t, $J$ = 8.0 Hz, 3H). LCMS $[M+H]^+$ =1064.8 |

(continued)

| Examples | Structure and data analysis |
|---|---|
| **UB-180601 (101)** | 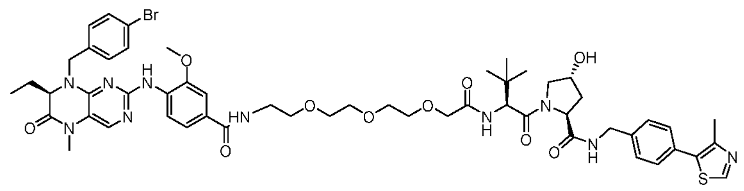 |
| | $^1$H NMR (400 MHz, DMSO-$d_6$) δ 8.97 (s, 1H), 8.61 (t, $J$ = 6.0 Hz, 1H), 8.39 (t, $J$ = 5.6 Hz, 1H), 8.27 (d, $J$ = 8.5 Hz, 1H), 7.87 (s, 1H), 7.65 (s, 1H), 7.54 (d, $J$ = 8.3 Hz, 2H), 7.48 (d, $J$ = 1.9 Hz, 1H), 7.45 - 7.37 (m, 6H), 7.34 (d, $J$ = 8.2 Hz, 2H), 5.25 (d, $J$ = 15.6 Hz, 1H), 5.17 (d, $J$ = 3.6 Hz, 1H), 4.56 (d, $J$ = 9.6 Hz, 1H), 4.48 - 4.33 (m, 4H), 4.30 - 4.15 (m, 2H), 3.96 (s, 2H), 3.90 (s, 3H), 3.69 - 3.48 (m, 12H), 3.40 (t, $J$ = 5.5 Hz, 2H), 3.27 (s, 3H), 2.44 (s, 3H), 2.06 (t, $J$ = 10.4 Hz, 1H), 1.95 - 1.86 (m, 1H), 1.76 (dq, $J$ = 14.2, 6.8 Hz, 2H), 0.94 (s, 10H), 0.73 (t, $J$ = 7.4 Hz, 3H). LC-MS : V879-129-F LCMS [M+H]+ =1127.1 |
| **UB-180602 (102)** | 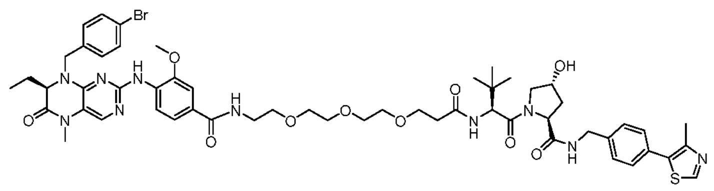 |
| | $^1$H NMR (400 MHz, DMSO-$d_6$) δ 8.98 (s, 1H), 8.57 (t, $J$ = 6.1 Hz, 1H), 8.38 (t, $J$ = 5.7 Hz, 1H), 8.27 (d, $J$ = 8.5 Hz, 1H), 7.89 (q, $J$ = 9.6 Hz, 2H), 7.65 (s, 1H), 7.56 - 7.51 (m, 2H), 7.48 (d, $J$ = 1.9 Hz, 1H), 7.44 - 7.33 (m, 7H), 5.25 (d, $J$ = 15.6 Hz, 1H), 5.13 (d, $J$ = 3.5 Hz, 1H), 4.55 (d, $J$ = 9.4 Hz, 1H), 4.49 - 4.39 (m, 3H), 4.35 (s, 1H), 4.28 - 4.13 (m, 2H), 3.91 (s, 3H), 3.66-3.50 (m, 18H), 3.27 (s, 3H), 2.44 (s, 3H), 2.08 - 1.99 (m, 1H), 1.90 (M, 1H), 1.77 (dt, $J$ = 14.4, 7.0 Hz, 2H), 0.93 (s, 9H), 0.73 (t, $J$ = 7.4 Hz, 3H).LC-MS : V879-130-F LCMS [M+H]+ =1143.4 |
| **UB-180605 (105)** | 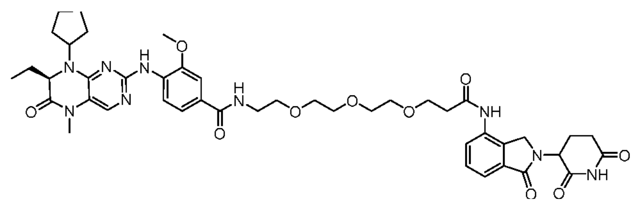 |
| | $^1$H NMR (400 MHz, DMSO-$d_6$) δ 11.02 (s, 1H), 9.83 (s, 1H), 8.50 - 8.32 (m, 2H), 7.92 - 7.73 (m, 2H), 7.60 (s, 1H), 7.55 - 7.36 (m, 4H), 5.14 (dd, $J$ = 13.2, 5.2 Hz, 1H), 4.47 - 4.28 (m, 3H), 4.27 - 4.19 (m, 1H), 3.93 (s, 3H), 3.70 (t, $J$ = 6.4 Hz, 2H), 3.63 - 3.44 (m, 9H), 3.44 - 3.37 (m, 2H), 3.24 (s, 3H), 2.97 - 2.85 (m, 1H), 2.62 - 2.57 (m, 2H), 2.46 - 2.42 (m, 1H), 2.38 - 2.29 (m, 1H), 2.09 - 1.96 (m, 2H), 1.96 - 1.70 (m, 6H), 1.69 - 1.52 (m, 3H), 0.94 (s, 1H), 0.76 (t, $J$ = 7.6 Hz, 3H). LCMS $[M+H]^+$: 870.5. |

(continued)

| Examples | Structure and data analysis |
| --- | --- |
| **UB-180606 (106)** | 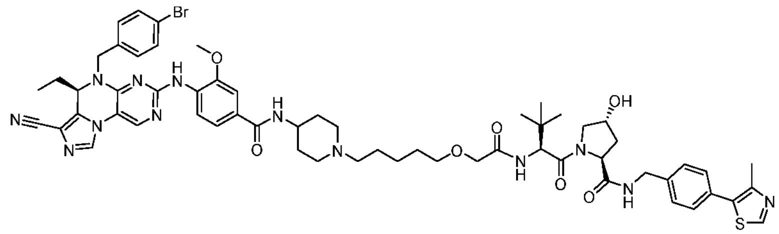 |
| | $^1$H NMR (400 MHz, DMSO-$d_6$) δ 8.98 (s, 1H), 8.75 (d, 1H), 8.70 (s, 1H), 8.62 (m, 1H), 8.09 (m, 1H), 8.04 (m, 1H), 7.63-7.48 (m, 4H), 7.36-7.31 (m, 4H), 7.35 (m, 3H), 7.20 (m, 1H), 6.65 (s, 1H), 5.32 (t, 1H), 5.27-5.06 (m, 4H), 4.69-4.53 (m, 2H), 4.45 (t, 1H), 4.35 (m, 1H), 4.28 (m, 1H), 3.96-3.87 (m, 5H), 3.66 (m, 2H), 3.50 (m, 4H), 2.44 (s, 3H), 2.09-1.93 (m, 6H), 1.91-1.82 (m, 6H), 1.58 (m, 2H), 1.45 (m, 2H), 0.95 (s, 9H), 0.88-0.83 (m, 3H), 0.72 (t, *J*= 8.0 Hz, 3H). LCMS $[M+H]^+$ =1198.3 |
| **UB-180607 (107)** | 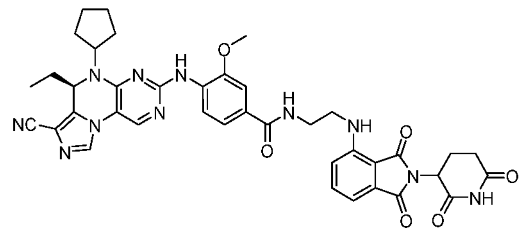 |
| | $^1$H NMR (400 MHz, DMSO-$d_6$) δ11.09 (s, 1H),8.70 (s, 1H), 8.71 (d, *J* = 6.2 Hz, 2H), 8.27 - 8.24 (m, 1H), 8.02 (s, 1H), 7.86 (d, *J* = 9.3 Hz, 3H), 7.60 - 7.37 (m, 17H), 7.36 (s, 2H), 5.29 - 5.10 (m,2H), 4.56 (d, *J* = 9.3 Hz, 1H), 4.09 - 3.73 (m, 16H), 3.91 (s, 3H),3.73 - 3.40 (m, 4H), 2.67 -2.43 (m, 4H), 2.03 -1.52 (m, 13H), 0.74 (t, *J* = 7.3 Hz, 3H).LCMS $[M+H]^+$ =758.3 |
| **UB-180608 (108)** | 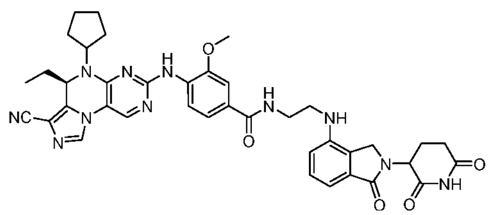 |
| | $^1$H NMR (400 MHz, DMSO-$d_6$) δ11.01 (s, 1H), 8.72 (d, *J* = 8.6Hz, 1H), 8.69-8.46 (m, 2H), 8.27 (d, *J* = 8.3 Hz, 1H), 8.01 (s, 1H), 7.57 - 7.45 (m, 2H), 7.32 (t, *J* = 7.7 Hz, 1H), 6.94 (t, *J* = 8.0 Hz, 2H), 5.83 (dd, *J* = 24.0, 18.3 Hz, 1H), 5.15 (ddd, *J* = 18.3, 10.6, 4.3 Hz, 2H), 4.48 - 4.23 (m, 3H), 3.93 (s, 3H), 3.46 - 3.26 (m, 2H), 3.05 - 2.83 (m, 1H), 2.38 - 2.21 (m, 1H), 2.02- 1.50 (m, 11H), 1.34 (s, 2H), 0.75 (t, *J* = 7.4 Hz, 3H).LCMS $[M+H]^+$ =744.2 |

(continued)

| Examples | Structure and data analysis |
|---|---|
| **UB-180618 (118)** | 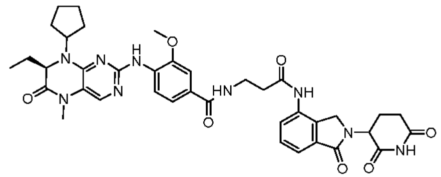<br>$^1$H NMR (400 MHz, DMSO-$d_6$) δ 11.00 (s, 1H), 9.90(s, 1H), 8.53 (t, $J$ = 5.7 Hz, 1H), 8.39 (d, $J$ = 8.3 Hz, 1H), 7.84 (s, 1H), 7.81 (dd, $J$ = 7.0, 2.1 Hz, 1H), 7.64 (s, 1H), 7.54 - 7.45 (m, 4H), 5.09 (dd, $J$ = 13.3, 5.2 Hz, 1H), 4.42 - 4.18 (m, 4H), 3.91 (s, 3H), 3.58 (q, $J$ = 6.5 Hz, 2H), 3.25 (s, 3H), 2.97 - 2.81 (m, 1H), 2.67 (t, $J$ = 6.7 Hz, 2H), 2.05 - 1.51 (m, 12H), 0.76 (t, $J$ = 7.4 Hz, 3H). LCMS [M+H]+ =738.3 |
| **UB-180619 (119)** | 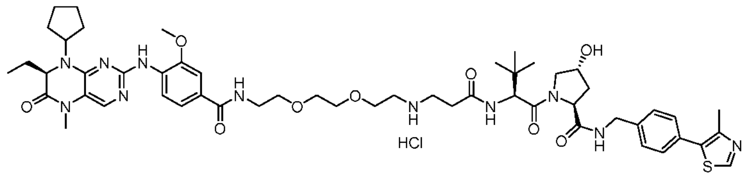 |
| | $^1$H NMR (400 MHz, DMSO-$d_6$) δ 8.98 (s, 1H), 8.60 (s, 1H), 8.49 (s, 1H), 8.42 (d, J = 8.3 Hz, 1H), 8.33 (d, J = 9.2 Hz, 1H), 7.84 (s, 1H), 7.60 (s, 1H),7.53 (t, J = 2.7 Hz, 2H), 7.40 (q, J = 8.4 Hz, 4H), 7.21 (s, 1H), 6.65 (s, 1H), 5.32 (dd, J = 5.6, 4.1 Hz, 1H), 5.18 (d, J = 3.4 Hz, 1H), 4.54 (d, J = 9.3 Hz,1H), 4.43 (s, 2H), 4.35 (d, J = 6.8 Hz, 2H), 4.27 - 4.18 (m, 2H), 3.94 (s, 3H), 3.70 - 3.60 (m, 4H), 3.58 - 3.56 (m, 3H), 3.53 - 3.49 (m, 2H), 3.45 - 3.40(m, 2H), 3.24 (s, 3H), 3.07 - 2.91 (m, 4H), 2.59 (d, J = 7.3 Hz, 1H), 2.44 (s, 3H), 1.99 (dd, J = 8.6, 6.5 Hz, 4H), 1.89 (q, J = 4.3 Hz, 2H), 1.80 - 1.76 (m,2H), 1.65 - 1.59 (m, 2H), 1.46 (d, J = 7.3 Hz, 2H), 0.94 (s, 9H), 0.76 (t, 3H). LCMS [M+H]+ =1040.2 |
| **UB-180620 (120)** | 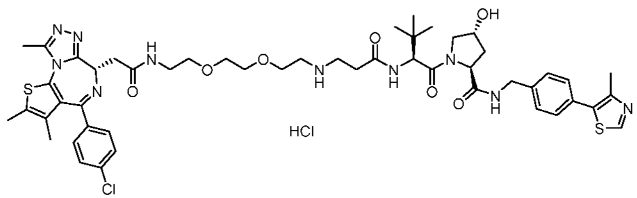 |
| | $^1$H NMR (400 MHz, DMSO-$d_6$) δ 8.96 (s, 1H), 8.60 (t, J = 6.1 Hz, 1H), 8.34 - 8.25 (m, 2H), 7.48 - 7.36 (m, 8H), 7.29 (s, 1H), 6.70 - 6.63 (m, 1H), 5.32- 5.30 (m, 1H), 5.26 (d, J = 3.5 Hz, 1H), 4.54 - 4.47 (m, 2H), 4.45 - 4.39 (m, 2H), 4.36 (s, 1H), 4.26 (q, J = 5.3 Hz, 8H), 4.19 (d, J = 5.4 Hz, 1H), 3.26(d, J = 4.7 Hz, 2H), 3.08 (d, J = 7.2 Hz, 2H), 2.70 - 2.62 (m, 2H), 2.59 (s, 3H), 2.43 (s, 3H), 2.39 (s, 3H), 2.00 - 1.93 (m, 4H), 1.61 (s, 3H), 1.45 (t, J =7.1 Hz, 2H), 0.93 (s, 9H). LCMS [M+H]+ =1015.2 |

(continued)

| Examples | Structure and data analysis |
|---|---|
| **UB-180621 (121)** | 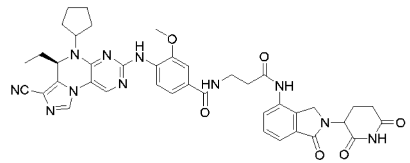 |
| | $^1H$ NMR (400 MHz, DMSO-$d_6$) δ 11.00 (s, 1H), 9.90 (s, 1H), 8.70 (s, 1H), 8.64 (d, *J* = 1.0 Hz, 1H), 8.58 (d, *J* = 5.8 Hz, 1H), 8.23 (dd, *J* = 8.3, 2.0 Hz, 1H), 7.99 (s, 1H), 7.81 (dd, *J* = 6.9, 2.0 Hz, 1H), 7.53 - 7.45 (m, 4H), 5.17 (dd, *J* = 8.1, 3.7 Hz, 1H), 5.10 (dd, *J* = 13.2, 5.2 Hz, 1H), 4.39 (dd, *J* = 10.7, 6.7 Hz, 1H), 4.31 (s, 1H), 3.90 (s, 3H), 3.59 (q, *J* = 6.7 Hz, 2H), 2.71 - 2.62 (m, 2H), 2.23 (tt, *J* = 15.7, 7.0 Hz, 2H), 2.01 - 1.51 (m, 12H), 0.74 (t, J = 7.3 Hz, 3H). LCMS [M+H]+ =772.3 |
| **UB-180622 (122)** | 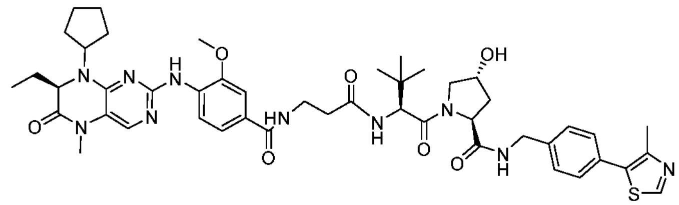 |
| | $^1H$ NMR (400 MHz, DMSO-$d_6$) δ 8.98 (s, 1H), 8.56 (t, *J* = 6.1 Hz, 1H), 8.42 - 8.29 (m, 2H), 7.99 (d, *J* = 9.2 Hz, 1H), 7.84 (s, 1H), 7.53 - 7.43 (m, 2H), 7.40 (q, *J* = 8.4 Hz, 4H), 5.15 (s, 1H), 4.56 (d, *J* = 9.3 Hz, 1H), 4.50 - 4.38 (m, 2H), 4.34 (d, *J* = 17.3 Hz, 2H), 4.29 - 4.15 (m, 2H), 3.92 (s, 3H), 3.72 - 3.55 (m, 3H), 3.51 - 3.36 (m, 2H), 3.24 (s, 3H), 3.14 (qd, *J* = 7.3, 4.2 Hz, 1H), 2.44 (s, 3H), 2.10 - 1.52 (m, 12H), 0.93 (s, 9H), 0.76 (t, *J* = 7.4 Hz, 3H). LCMS [M+H]+ =909.5 |
| **UB-180623 (123)** | 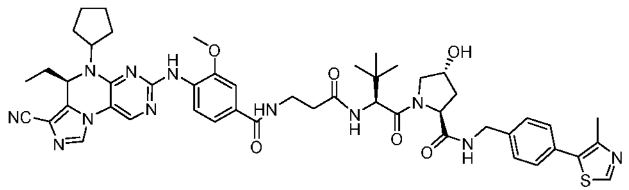 |
| | $^1H$ NMR (400 MHz, DMSO-$d_6$) δ 8.97 (s, 1H), 8.70 (s, 1H), 8.64 (s, 1H), 8.57 (t, *J* = 6.1 Hz, 1H), 8.37 (t, *J* = 5.5 Hz, 1H), 8.24 (d, *J* = 8.3 Hz, 1H), 8.05 - 7.96 (m, 2H), 7.60 - 7.45 (m, 2H), 7.40 (q, *J* = 8.3 Hz, 4H), 5.26 - 5.08 (m, 2H), 4.56 (d, *J* = 9.3 Hz, 1H), 4.49 - 4.32 (m, 4H), 4.22 (dd, *J* = 15.8, 5.5 Hz, 1H), 3.92 (s, 3H), 3.68 (d, *J* = 5.0 Hz, 2H), 3.52 - 3.36 (m, 2H), 2.44 (s, 3H), 2.08 - 1.53 (m, 13H), 0.93 (s, 9H), 0.74 (t, *J* = 7.4 Hz, 3H). LCMS [M+H]+ =943.4 |

(continued)

| Examples | Structure and data analysis |
|---|---|
| **UB-180624 (124)** | 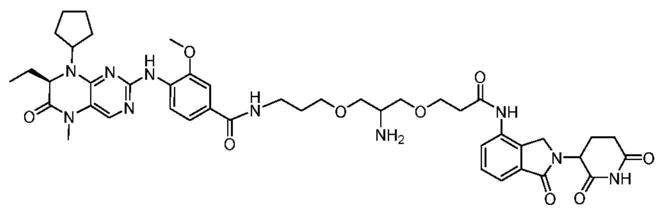 |
| | $^1$H NMR (400 MHz, DMSO-$d_6$) δ 11.02 (s, 1H), 10.21 (s, 1H), 8.58 (t, *J* = 5.8 Hz, 1H), 8.41 (d, *J* = 8.4 Hz, 1H), 7.88 - 7.78 (m, 2H), 7.67 - 7.42 (m, 5H), 5.14 (dd, *J* = 13.3, 5.1 Hz, 1H), 4.52 - 4.30 (m, 3H), 4.24 (dd, *J* = 7.6, 3.6 Hz, 1H), 3.94 (s, 3H), 3.77 (q, *J* = 6.0 Hz, 2H), 3.61( m, 4H), 3.47 (m, 4H), 3.25 (s, 3H), 2.98 - 2.85 (m, 2H), 2.71 (m, 1H), 2.66 - 2.56 (m, 2H), 2.09 - 1.99 (m, 2H), 1.88 (m, 2H), 1.80 - 1.75 (m, 7H), 1.64 (dt, *J* = 14.4, 7.4 Hz, 2H), 1.28 - 1.20 (m, 2H), 0.76 (t, *J* = 7.4 Hz, 3H). LCMS: $(M+H)^+$= 869.4 |
| **UB-180625 (125)** | 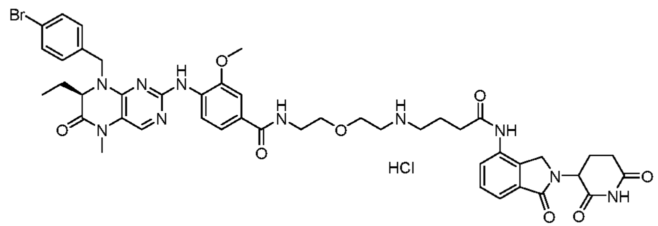 |
| | $^1$H NMR (400 MHz, DMSO-$d_6$) δ 11.09 (s, 1H), 8.75 (s, 1H), 8.70 (s, 1H), 8.46 (t, *J* = 5.6 Hz, 1H), 8.17 - 7.99 (m, 2H), 7.68 - 7.50 (m, 3H), 7.50 - 7.40 (m, 2H), 7.36 (t, *J* = 14.5 Hz, 3H), 7.12 (d, *J* = 8.7 Hz, 2H), 7.03 (d, *J* = 7.1 Hz, 1H), 6.76 (t, *J* = 6.0 Hz, 1H), 5.32 - 5.11 (m, 2H), 5.05 (dd, *J* = 12.9, 5.3 Hz, 1H), 4.60 (d, *J* = 15.7 Hz, 6H), 3.91 (s, 3H), 3.87 - 3.39 (m, 12H), 3.19 - 2.94 (m, 4H), 3.19 - 2.85 (m, 4H), 2.09 - 1.96 (m, 2H), 0.72 (t, *J* = 7.3 Hz, 3H).LCMS $[M+H]^+$ =927.3 |
| **UB-180626 (126)** | 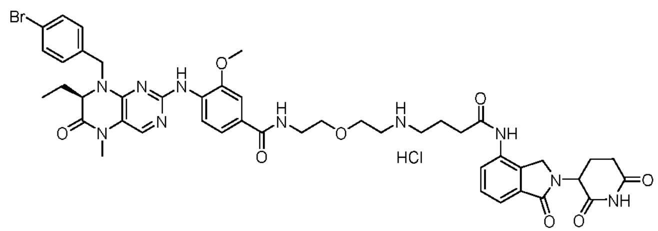 |
| | $^1$H NMR (400 MHz, DMSO-$d_6$) δ 11.02 (s, 1H), 10.01 (s, 7H), 9.03-8.69(m, 3H),7.93 (s, 1H), 7.81 (t, *J* = 16.4 Hz, 2H), 7.67 (s, 1H), 7.58 - 7.40 (m, 6H), 7.34 (d, *J* = 8.4 Hz, 2H), 5.15 (dd, *J* = 13.0, 5.2 Hz, 2H), 4.77 - 4.46 (m, 1H), 4.37 (t, *J* = 17.5 Hz, 3H), 3.93 (s, 3H), 3.93 (s, 46H), 3.98 - 3.62 (m, 8H), 3.24 (s, 3H), 3.22 - 2.86 (m, 2H), 2.70 - 2.06 (m, 4H), 2.28 - 2.16 (m, 1H), 2.06-1.89 (m, 6H), 0.74 (t, *J* = 7.3, Hz, 3H).LCMS $[M+H]^+$ =939.3 |

(continued)

| Examples | Structure and data analysis |
|---|---|
| **UB-180627 (127)** | 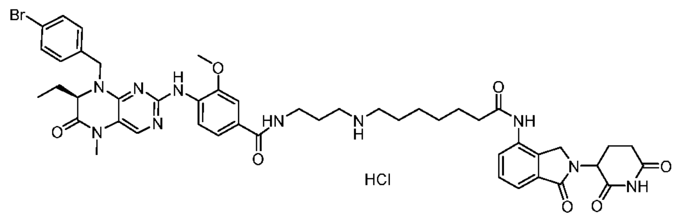 |
| | $^1$H NMR (400 MHz, DMSO-$d_6$) δ 11.02 (s, 1H), 9.86 (s, 1H), 8.77 (s, 4H), 7.93 (s, 1H), 7.88 - 7.78 (m, 2H), 7.60 - 7.44 (m, 5H), 7.42 - 7.31 (m, 3H), 5.15 (dd, J = 14.3, 4.1 Hz, 2H), 4.61 (d, J = 15.6 Hz, 1H), 4.46 - 4.30 (m, 4H), 3.92 (s, 3H), 3.56 (d, J = 2.6 Hz, 3H), 3.24 (s, 3H), 2.90 (d, J = 18.4 Hz, 6H), 2.61 (d, J = 18.1 Hz, 2H), 2.41 - 2.28 (m, 3H), 2.03 (d, J = 6.1 Hz, 1H), 1.88 (s, 4H), 1.62 (s, 4H), 1.35 (s, 4H), 0.74 (d, J = 7.5 Hz, 3H).LC-MS : [M+H]+ =953.3 |
| **UB-180628 (128)** | 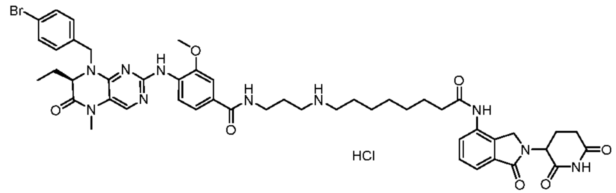 |
| | $^1$H NMR (400 MHz, DMSO-$d_6$) δ 11.02 (s, 1H), 9.85 (s, 1H), 8.76 (s, 3H), 8.08 - 7.74 (m, 3H), 7.61 - 7.45 (m, 5H), 7.46 - 7.31 (m, 3H), 5.22 - 5.10 (m, 2H), 4.59 (d, J = 15.5 Hz, 1H), 4.46 - 4.27 (m, 3H), 3.92 (s, 3H), 3.25 (s, 3H), 2.99 - 2.79 (m, 6H), 2.70 - 2.57 (m, 2H), 2.43 - 2.24 (m, 3H), 2.08 - 1.80 (m, 6H), 1.61 (s, 4H), 1.32 (s, 6H), 0.75 (t, J = 7.4 Hz, 3H).LC-MS : [M+H]+ =967.3 |
| **UB-180629 (129)** | 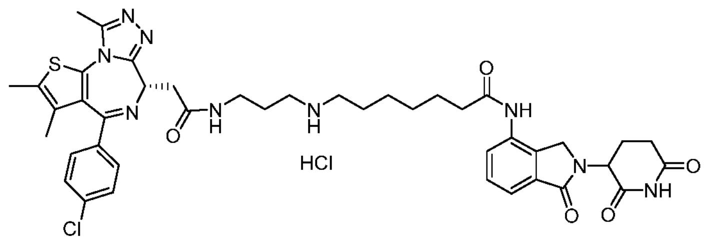 |
| | $^1$H NMR (400 MHz, DMSO-$d_6$) δ 11.02 (s, 1H), 9.89 (s, 1H), 8.77 (s, 2H), 8.47 (t, *J* = 5.9 Hz, 1H), 7.82 (dd, *J* = 7.0, 2.0 Hz, 1H), 7.56 - 7.38 (m, 5H), 5.15 (dd, *J* = 13.3, 5.1 Hz, 1H), 4.56 - 4.50 (m, 1H), 4.43 - 4.30 (m, 2H), 3.30 - 3.14 (m, 4H), 2.95 - 2.77 (m, 5H), 2.61 (s, 4H), 2.44 - 2.32 (m, 6H), 2.07 - 1.99 (m, 1H), 1.84 - 1.78 (m, 2H), 1.64 - 1.56 (m, 7H), 1.37 - 1.30 (m, 4H). LC-MS : [M+H]+ =826.3 |

(continued)

| Examples | Structure and data analysis |
|---|---|
| **UB-180630 (130)** | 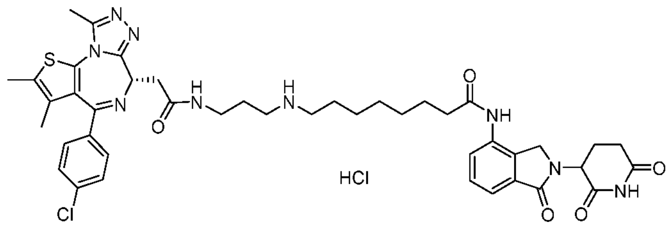 HCl<br>$^1$H NMR (400 MHz, DMSO-$d_6$) δ 11.03 (s, 1H), 9.88 (s, 1H), 8.76 (s, 2H), 8.48 (t, *J* = 5.9 Hz, 1H), 7.82 (d, *J* = 6.9 Hz, 1H), 7.57 - 7.39 (m, 6H), 5.14 (dd, *J* = 13.3, 5.2 Hz, 1H), 4.56 (t, *J* = 7.2 Hz, 2H), 4.38 (q, *J* = 17.5 Hz, 2H), 3.24 (m, 4H), 2.97 - 2.76 (m, 6H), 2.63 (s, 5H), 2.39 (m, 6H), 2.12 - 1.99 (m, 1H), 1.83 (d, *J* = 8.6 Hz,2H), 1.61 (mz, 6H), 1.30 (s, 6H).LC-MS : [M+H]+ =840.3 |
| **UB-180631 (131)** | 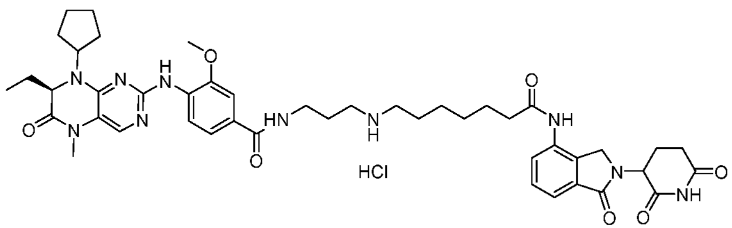 HCl<br>$^1$H NMR (400 MHz, DMSO-$d_6$) δ 11.02 (s, 1H), 9.88 (s, 1H), 8.90 - 8.79 (m, 3H), 7.97 - 7.77 (m, 3H), 7.65 (d, *J* = 1.9 Hz, 1H), 7.61 - 7.41 (m, 3H), 5.15 (dd, *J* = 13.3, 5.1 Hz, 1H), 4.54 - 4.28 (m, 3H), 4.17 (t, *J* = 8.8 Hz, 1H), 3.92 (s, 3H), 3.22 (s, 3H), 3.03 - 2.82 (m, 6H), 2.68 - 2.58 (m, 2H), 2.45 - 2.27 (m, 3H), 2.09 - 1.76 (m, 9H), 1.69 - 1.40 (m, 8H), 1.35 (d, *J* = 5.9 Hz, 4H), 0.75 (t, *J* = 7.4 Hz, 3H). LC-MS : [M+H]+ =851.4 |
| **UB-180632 (132)** | 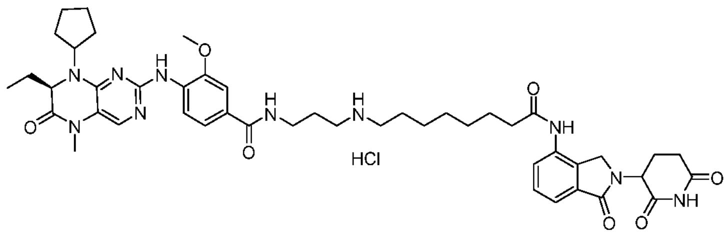 HCl<br>$^1$H NMR (400 MHz, DMSO-$d_6$) δ 11.02 (s, 1H), 9.86 (s, 1H), 9.65 (s, 1H), 8.84 (d, J = 18.1 Hz, 3H), 7.93 - 7.80 (m, 3H), 7.73 - 7.42 (m, 4H), 5.15 (dd, J = 13.3, 5.1 Hz, 1H), 4.60 - 4.26 (m, 3H), 4.16 (t, J = 8.8 Hz, 1H), 3.92 (s, 3H),3.33-3.36(m, 2H), 3.22 (s, 3H), 2.92 (m, 5H), 2.63 (m, 2H), 2.43 - 2.25 (m, 3H), 2.09 - 1.75 (m, 8H), 1.69 - 1.44 (m, 8H), 1.32 (s, 6H), 0.75 (t, J = 7.4 Hz, 3H). LC-MS : [M+H]+ =865.3 |

(continued)

| Examples | Structure and data analysis |
|---|---|
| **UB-180633 (133)** | 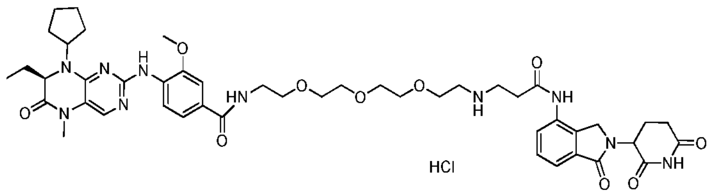 HCl |
| | $^1$H NMR (400 MHz, DMSO-$d_6$) δ 11.02 (s, 1H), 10.12 (s, 1H), 9.77 (s, 1H), 9.00 (s, 2H), 8.69 (s, 1H), 7.85 (d, J = 4.8 Hz, 3H), 7.64 (d, J = 1.8 Hz, 1H),7.51 (d, J = 1.8 Hz, 3H), 5.15 (dd, J = 13.3, 5.2 Hz, 1H), 4.53 - 4.33 (m, 3H), 4.15 (p, J = 8.8 Hz, 1H), 3.90 (s, 3H), 3.58 - 3.50 (m, 12H), 3.44 (t, J = 5.8Hz, 2H), 3.21 (s, 3H), 3.10 (d, J = 5.6 Hz, 2H), 3.02 - 2.88 (m, 4H), 2.09 - 1.75 (m, 10H), 1.57 - 1.39 (m, 4H), 0.76 (t, J = 7.4 Hz, 3H). LCMS $[M+H]^+$=913.58 |
| **UB-180634 (134)** | 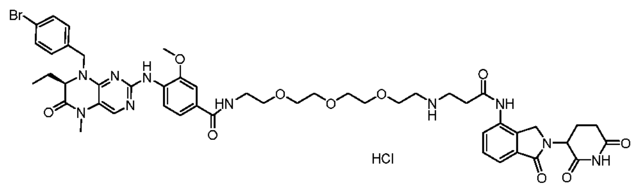 HCl |
| | $^1$H NMR (400 MHz, DMSO-$d_6$) δ 11.03 (s, 1H), 10.28 (s, 1H), 9.60 (s, 1H), 9.00 (s, 2H), 8.62 (s, 1H), 7.96 (s, 1H), 7.85 (dd, J = 7.3, 1.7 Hz, 1H), 7.78(d, J = 8.4 Hz, 1H), 7.59 (d, J = 1.9 Hz, 1H), 7.53 (dd, J = 7.0, 5.1 Hz, 4H), 7.34 (d, J = 8.5 Hz, 3H), 5.21 - 5.07 (m, 2H), 4.63 (d, J = 15.5 Hz, 1H), 4.51- 4.32 (m, 3H), 3.91 (s, 3H), 3.72 - 3.69 (m, 2H), 3.56 (s, 10H), 3.24 (s, 3H), 3.13 (t, J = 5.5 Hz, 3H), 2.93 (hept, J = 5.3, 4.6 Hz, 3H), 2.65 (d, J = 15.0Hz, 2H), 2.32 (qd, J = 13.4, 5.0 Hz, 2H), 2.16 - 1.97 (m, 2H), 1.96 - 1.85 (m, 2H), 0.75 (t, 3H). LCMS $[M-100]^+$ =913.6 |
| **UB-180635 (135)** | 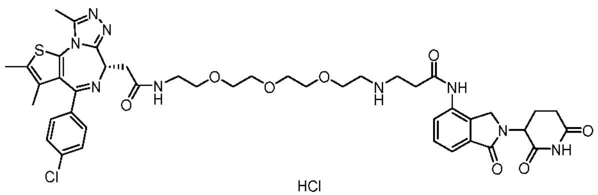 HCl |
| | $^1$H NMR (400 MHz, DMSO-$d_6$) δ 11.03 (s, 1H), 10.28 (d, J = 3.7 Hz, 1H), 8.99 (s, 2H), 8.33 (t, J = 5.7 Hz, 1H), 7.85 (dd, J = 7.2, 1.8 Hz, 1H), 7.55 -7.47 (m, 4H), 7.43 (d, J = 8.4 Hz, 2H), 5.15 (dd, J = 13.3, 5.2 Hz, 1H), 4.55 (t, J = 7.0 Hz, 1H), 4.52 - 4.32 (m, 2H), 3.57 (d, J = 14.9 Hz, 11H), 3.46 (t,J = 5.9 Hz, 3H), 3.34 - 3.19 (m, 7H), 3.14 (p, J = 5.9, 5.4 Hz, 3H), 2.93 (qd, J = 11.6, 10.9, 5.1 Hz, 3H), 2.63 (s, 3H), 2.42 (s, 3H), 2.38 - 2.13 (m, 2H),2.02 (dtd, J = 16.8, 9.0, 8.1, 5.2 Hz, 2H). $[M-100]^+$ =888.4 |

(continued)

| Examples | Structure and data analysis |
|---|---|
| **UB-180636 (136)** | 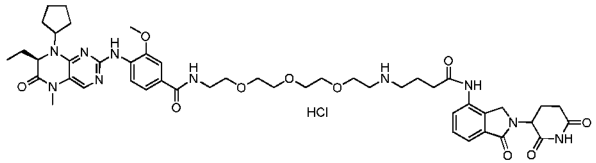 HCl |
| | $^1$H NMR (400 MHz, DMSO-$d_6$) δ 11.03 (s, 1H), 10.23 (s, 1H), 8.92 (s, 2H), 8.64 (s, 1H), 7.84 (d, J = 4.5 Hz, 3H), 7.61 (d, J = 1.8 Hz, 1H), 7.57 - 7.48 (m, 3H),5.16 (dd, J = 13.2, 5.1 Hz, 1H), 4.53 - 4.45 (m, 2H), 4.40 (d, J = 20.7 Hz, 2H), 4.25 - 4.12 (m, 1H), 3.91 (s, 3H), 3.71 (td, J = 6.4, 5.5, 2.9 Hz, 4H), 3.57 (s, 8H),3.22 (s, 3H), 3.14 (s, 3H), 2.91 (q, J = 6.9 Hz, 3H), 2.38 - 2.25 (m, 2H), 2.10 - 1.75 (m, 8H), 1.54 (s, 4H), 0.75 (t, J = 7.4 Hz, 3H). LCMS $[M+H]^+$ =888.43 |
| **UB-180637 (137)** | 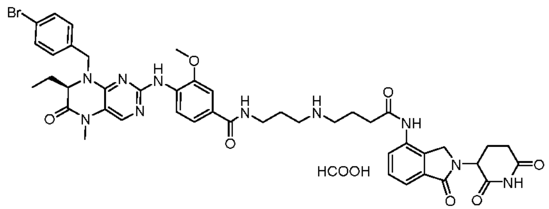 HCOOH |
| | $^1$H NMR (400 MHz, DMSO-$d_6$) δ 11.02 (s, 1H), 10.08 (s, 1H), 9.23 (s, 2H), 9.01 (s, 2H), 8.81 (t, J = 5.8 Hz, 1H), 7.95 (s, 1H), 7.84 (dd, J = 7.3, 1.8 Hz, 2H), 7.61 (d, J = 1.8 Hz, 1H), 7.58 - 7.46 (m, 4H), 7.42 (dd, J = 8.5, 1.8 Hz, 1H), 7.34 (d, J = 8.3 Hz, 2H), 5.26 - 5.05 (m, 2H), 4.60 (d, J = 15.6 Hz, 1H), 4.46 - 4.36 (m, 2H), 3.92 (s, 3H), 3.61 (m, 2H), 3.25 (s, 3H), 3.12 (qd, J = 7.3, 4.2 Hz, 4H), 3.00 - 2.91 (m, 4H), 1.99 (m, 4H), 1.95 - 1.81 (m, 4H), 0.75 (t, J = 7.4 Hz, 3H). LCMS $[M+H]^+$ =909.2 |
| **UB-180638 (138)** | 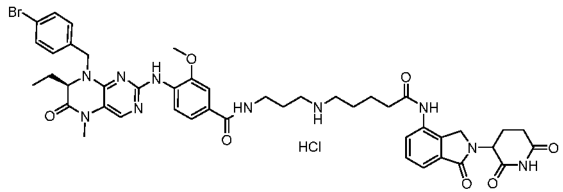 HCl |
| | $^1$H NMR (400 MHz, DMSO-$d_6$) δ 11.02 (s, 1H), 9.94 (s, 1H), 8.73 (m, 4H), 8.02 (s, 1H), 7.91 (d, J = 2.8 Hz, 1H), 7.83 (dd, J = 7.1, 1.9 Hz, 1H), 7.59 - 7.39 (m, 6H), 7.38 - 7.29 (m, 2H), 5.28 - 5.04 (m, 2H), 4.65 - 4.22 (m, 4H), 3.92 (s, 3H), 3.64 - 3.55 (m, 2H), 3.26 (s, 3H), 3.13 (qt, J = 7.3, 3.8 Hz, 2H), 3.01 - 2.90 (m, 5H), 2.43 (m, 2H), 2.09 - 1.98 (m, 1H), 1.88 (m, 4H), 1.68 (s, 4H), 0.74 (t, J = 7.4 Hz, 3H). LCMS $[M+H]^+$ =926.2 |

(continued)

| Examples | Structure and data analysis |
|---|---|
| **UB-180639 (139)** | 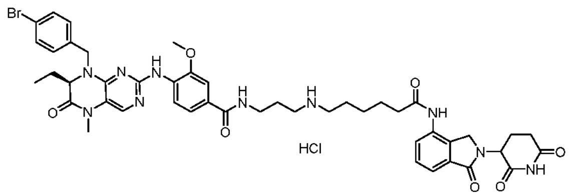 |
| | $^1$H NMR (400 MHz, DMSO-$d_6$) δ 11.02 (s, 1H), 9.86 (s, 1H), 8.70 (d, J = 28.6 Hz, 4H), 7.91 (s, 1H), 7.82 (dd, J = 7.2,1.8 Hz, 2H), 7.59 (s, 1H), 7.58 - 7.51 (m, 2H), 7.53 - 7.43 (m, 2H), 7.41 (dd, J = 8.6, 1.8 Hz, 1H), 7.34 (d, J = 8.5 Hz, 2H), 5.25 - 5.07 (m, 2H), 4.61 (d, J = 15.6 Hz, 1H), 4.48 - 4.25 (m, 3H), 3.92 (s, 3H), 3.40 - 3.30 (m, 2H), 3.25 (s, 3H), 2.90 (m, 5H), 2.42 - 2.24 (m, 4H), 2.03 (m, 1H), 1.88 (m, 4H), 1.73 - 1.55 (m, 4H), 1.45 - 1.26 (m, 2H), 0.75 (t, J = 7.4 Hz, 3H). LCMS [M+H]+ =938.4 |
| **UB-180640 (140)** | 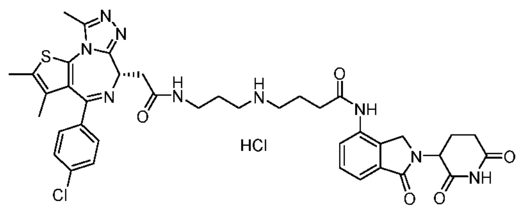<br>$^1$H NMR (400 MHz, DMSO-$d_6$) δ 11.02 (s, 1H), 10.07 (s, 1H), 8.93 (s, 2H), 8.46 (t, J = 5.8 Hz, 1H), 7.85 (dd, J = 7.2, 1.8 Hz, 1H), 7.64 - 7.32 (m, 6H), 5.15 (dd, J = 13.3, 5.1 Hz, 1H), 4.55 (t, J = 7.2 Hz, 1H), 4.50 - 4.28 (m, 2H), 3.67 - 3.57 (m, 2H), 3.27 (d, J = 7.3 Hz, 2H), 3.20 (q, J = 6.6 Hz, 2H), 3.11 (td, J = 7.4, 4.3 Hz, 1H), 2.90 (dd, J = 18.0, 6.2 Hz, 5H), 2.62 (s, 3H), 2.40 (s, 3H), 2.32 (t, J = 13.9 Hz, 1H), 1.99 (dt, J = 32.7, 7.1 Hz, 3H), 1.83 (t, J = 8.2 Hz, 2H), 1.62 (s, 3H). LCMS [M+H]+ =784.5 |
| **UB-180641 (141)** | 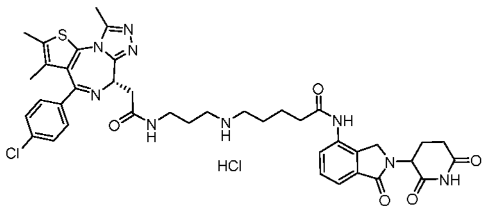 |
| | $^1$H NMR (400 MHz, DMSO-$d_6$) δ 11.02 (s, 1H), 9.95 (s, 1H), 8.76 (s, 2H), 8.46 (t, J = 5.8 Hz, 1H), 7.84 (dd, J = 7.0, 2.0 Hz, 1H), 7.61 - 7.31 (m, 6H), 5.15 (dd, J = 13.3, 5.1 Hz, 1H), 4.54 (t, J = 7.3 Hz, 1H), 4.40 (q, J = 17.5 Hz, 2H), 3.63 - 3.57 (m, 1H), 3.35 - 3.05 (m, 5H), 2.98 - 2.78 (m, 5H), 2.61 (s, 3H), 2.43 (m, 2H), 2.40 (s, 3H), 2.35 (dd, J = 13.3, 4.2 Hz, 1H), 2.10 - 1.96 (m, 1H), 1.82 (d, J = 10.3 Hz, 2H), 1.72 - 1.62 (m, 3H), 1.62 (s, 3H). LCMS $[M+H]^+$ =798.5 |

(continued)

| Examples | Structure and data analysis |
|---|---|
| UB-180642 (142) | 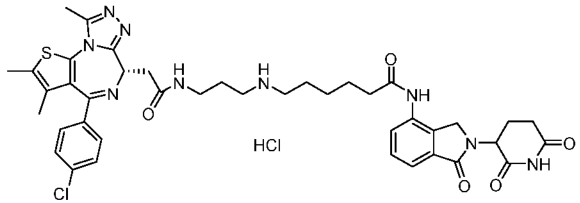 HCl |
| | $^1$H NMR (400 MHz, DMSO-$d_6$) δ 11.02 (s, 1H), 9.89 (s, 1H), 8.72 (s, 2H), 8.46 (t, J = 5.9 Hz, 1H), 7.83 (dd, J = 7.0, 2.0 Hz, 1H), 7.56 - 7.36 (m, 6H), 5.15 (dd, J = 13.3, 5.1 Hz, 1H), 4.53 (t, J = 7.2 Hz, 1H), 4.43 - 4.30 (m, 2H), 3.30 - 3.24 (m, 2H), 3.25 - 3.17 (m, 1H), 3.17 (s, 2H), 3.12 (dd, J = 7.4, 4.2 Hz, 1H), 2.90 (qd, J = 15.3, 14.0, 8.9 Hz, 4H), 2.61 (d, J = 1.1 Hz, 3H), 2.40 (s, 3H), 2.38 (d, J = 7.6 Hz, 2H), 2.09 - 1.94 (m, 1H), 1.89 - 1.73 (m, 2H), 1.62 (m, 6H), 1.35 (d, J = 7.2 Hz, 2H). LCMS [M+H]+ =812.0 |
| UB-180643 (143) | 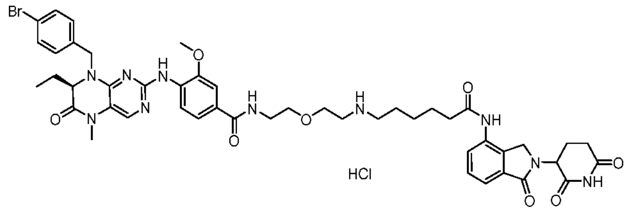 HCl |
| | $^1$H NMR (400 MHz, DMSO-$d_6$) δ 11.02 (s, 1H), 9.85 (s, 1H), 8.76 (m, 3H), 7.92 (m, 1H), 7.84-7.80 (m, 1H), 7.65 (d, *J* = 1.8 Hz, 1H), 7.57-7.51 (m, 2H), 7.50-7.43 (m, 2H), 7.35 (m, 2H), 5.15 (m, 2H), 4.62 (m, 1H), 4.44-4.34 (m, 2H), 3.93 (s, 3H), 3.72 (d, *J* = 5.7 Hz, 2H), 3.61-3.58 (m, 3H), 3.49 (m, 2H), 3.24 (d, *J* = 4.6 Hz, 3H), 3.10 (m, 2H), 2.97 (m, 1H), 2.89 (s, 3H), 2.68-2.59 (m, 1H), 2.35 (m, 2H), 2.22 (m, 1H), 1.89 (m, 2H), 1.62 (m, 2H), 1.60 (m, 2H), 1.57 (m, 1H), 1.46 (m, 1H), 1.31 (t, *J* = 6.4 Hz, 1H), 1.24 (m, 1H), 0.96 (t, *J* = 7.1 Hz, 1H), 0.75 (q, *J* = 7.0 Hz, 3H).LCMS $[M+H]^+$ =967.3 |
| UB-180644 (144) | 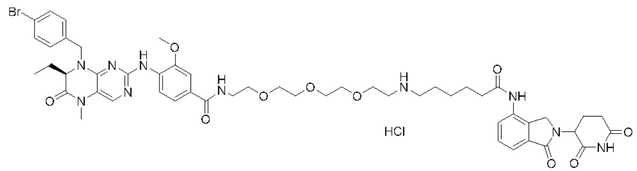 HCl<br>$^1$H NMR (400 MHz, DMSO-$d_6$) δ 11.02 (s, 1H), 9.93 (s, 1H), 9.69 (s, 1H), 8.82 (s, 2H), 8.62 (s, 1H), 7.96 (s, 1H), 7.83 (m, 1H), 7.75 (d, *J* = 8.4 Hz, 1H), 7.59 (d, *J* = 1.8 Hz, 1H), 7.56-7.52 (m, 2H), 7.49 (s, 1H), 7.40 -7.32 (m, 3H), 5.20-5.10 (m, 2H), 4.65 (m, 1H), 4.47-4.32 (m, 3H), 3.91 (m, 3H), 3.55 (m, 5H), 3.43 (m, 2H), 3.24 (s, 3H), 3.06 (t, *J* = 5.6 Hz, 4H), 3.01-2.86 (m, 5H), 2.68-2.58 (m, 2H), 2.39 (m, 2H), 2.33-2.24 (m, 1H), 2.08-1.87 (m, 4H), 1.71-1.58 (m, 4H), 1.49 (m, 1H), 1.36 (m, 2H), 0.97 (m, 1H), 0.75 (t, *J* = 7.2 Hz, 3H). LCMS $[M+H]^+$ =1057.1 |

(continued)

| Examples | Structure and data analysis |
|---|---|
| **UB-180645 (145)** | 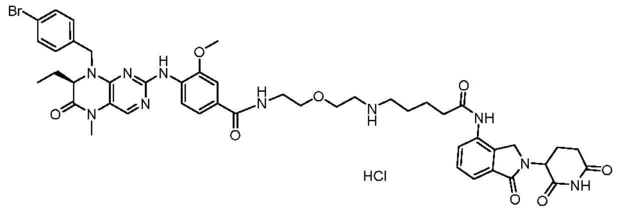 |
| | $^1$H NMR (400 MHz, DMSO-$d_6$) δ 11.02 (s, 1H), 9.95 (s, 1H), 8.90-8.71 (m, 3H), 7.93 (s, 1H), 7.91 - 7.67 (m, 2H), 7.67 (d, *J* = 1.5 Hz, 1H), 7.49 (ddd, *J* = 25.5, 18.0, 8.5 Hz, 5H), 7.35 (d, *J* = 8.4 Hz, 2H), 5.27 - 5.06 (m, 2H), 4.63 (d, *J* = 15.7 Hz, 1H), 4.41 (dt, *J* = 35.1, 12.2 Hz, 3H), 3.97 (s, 1H), 4.12 - 3.48 (m, 9H), 3.24 (s, 3H), 2.90 (dd, *J* = 17.1, 5.4 Hz, 2H), 2.77 - 2.57 (m, 1H), 2.36 (dt, *J* = 22.0, 10.0 Hz, 3H), 2.03 (d, *J* = 5.4 Hz, 1H), 1.87 -1.62 (m, 6H), 1.35 - 1.17 (m, 2H), 0.88 - 0.55 (m, 3H).LCMS $[M+H]^+$ =954.3 |
| **UB-180647 (147)** | 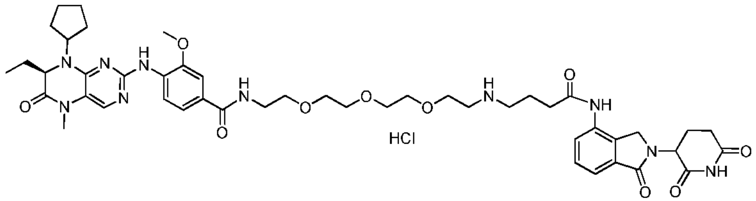 |
| | $^1$H NMR (400 MHz, DMSO-$d_6$) δ 11.02 (s, 1H), 10.06 (s, 1H), 8.89 (s, 2H), 8.59 (t, J = 5.6 Hz, 1H), 7.94 (s, 1H), 7.84 (dd, J = 7.3, 1.8 Hz, 2H), 7.59 -7.45 (m, 5H), 7.39 (dd, J = 8.5, 1.9 Hz, 1H), 7.34 (d, J = 8.2 Hz, 2H), 5.20 - 5.10 (m, 2H), 4.61 (d, J = 15.6 Hz, 1H), 4.49 - 4.32 (m, 3H), 3.91 (s, 3H),3.69 (t, J = 5.2 Hz, 3H), 3.57 (s, 11H), 3.24 (s, 3H), 3.15 - 3.05 (m, 3H), 3.01 - 2.88 (m, 4H), 2.09 - 1.79 (m, 6H), 0.75 (t, J = 7.4 Hz, 3H). LCMS [M+H]+ =927.5 |
| **UB-180648 (148)** | 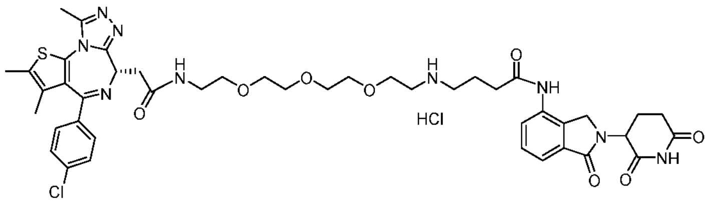 |
| | $^1$H NMR (400 MHz, DMSO-$d_6$) δ 11.00 (s, 1H), 10.25 (d, J = 3.6 Hz, 1H), 8.97 (s, 2H), 8.30 (s, 1H), 7.82 (dd, J = 7.2, 1.8 Hz, 1H), 7.52 - 7.45 (m, 4H), 7.41 (d, J = 8.4 Hz, 2H), 5.13 (d, J = 8.1 Hz,1H), 4.53 (d, J = 6.9 Hz, 1H), 4.49 - 4.30 (m, 2H), 3.54 (d, J = 14.8 Hz, 12H), 3.43 (t, J = 5.9 Hz, 3H), 3.31 - 3.16 (m, 7H), 3.12 (q, J = 5.4 Hz, 3H), 2.90 (q, J = 7.4, 6.8 Hz, 3H), 2.61 (s, 3H), 2.39 (s,3H), 2.36 - 2.10 (m, 2H), 1.99 (t, J = 16.8, 9.0, 8.1, 5.2 Hz, 2H). LCMS $[M+H]^+$ =902.3 |

(continued)

| Examples | Structure and data analysis |
| --- | --- |
| **UB-180649 (149)** | 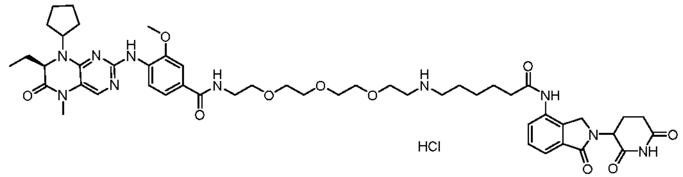 HCl |
| | $^1$H NMR (400 MHz, DMSO-$d_6$) $\delta$11.02 (s, 1H), 9.88 (s, 1H), 8.76-8.57 (m, 3H), 7.90 (m, 1H), 7.82 (s, 1H), 7.61 (m, 1H), 7.54 (m, 1H), 7.52-7.46 (m, 1H), 5.15 (m, 1H), 4.47 (m, 1H), 4.44-4.30 (m, 2H), 4.19 (m, 1H), 3.91 (m, 3H), 3.68 (m, 4H), 3.55 (m, 10H), 3.43 (m, 4H), 3.22 (s, 3H), 3.08 (m, 2H), 2.91 (m, 3H), 2.39 (m, 2H), 1.93 (m, 4H), 1.87-1.78 (m, 3H), 1.62 (m, 4H), 1.56-1.44 (m, 4H), 1.36 (m, 2H), 0.97 (m, 1H), 0.75 (t, $J$ = 8.0 Hz, 3H). LCMS $[M+H]^+$ =955.7 |
| **UB-180650 (150)** | 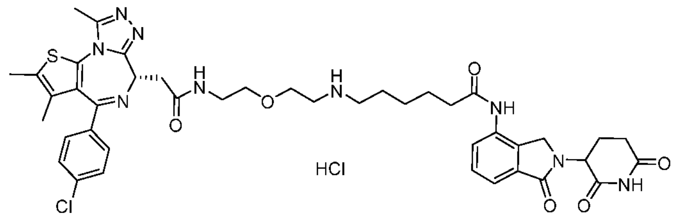 HCl |
| | $^1$H NMR (400 MHz, DMSO-$d_6$) $\delta$ 9.80 (s, 1H), 8.32 (d, 2H), 7.81 (m, 1H), 7.48 (m, 3H), 7.42 (m, 2H), 5.14 (m, 1H), 4.51 (m, 1H), 4.46-4.27 (m, 2H), 3.56-3.54 (m, 2H), 3.45 (m, 4H), 3.27 (m, 4H), 2.97 (m, 3H), 2.82 (m, 3H), 2.67 (m, 3H), 2.59 (m, 3H), 2.41 (m, 3H), 2.34 (m, 2H), 2.24 (m, 1H), 2.07- 1.96 (m, 1H), 1.62 (m, 4H), 1.54-1.41 (m, 3H), 1.32 (m, 2H), 0.96 (m, 1H). LCMS $[M+H]^+$ =842.3 |
| **UB-180651 (151)** | 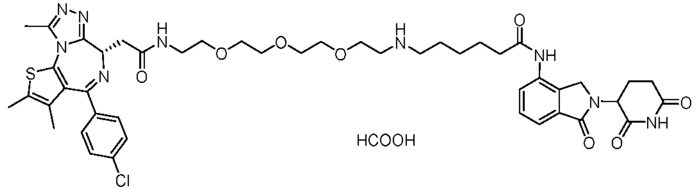 HCOOH |
| | $^1$H NMR (400 MHz, DMSO-$d_6$) $\delta$ 9.80 (s, 1H), 8.30 (m, 2H), 7.81 (m, 1H), 7.45 (m, 6H), 5.14 (m, 1H), 4.50 (m, 1H), 4.43-4.27 (m, 2H), 3.51 (m, 4H), 3.46 (m, 4H), 3.25 (m, 2H), 2.92 (m, 2H), 2.78 (d, J = 5.8 Hz, 2H), 2.66 - 2.61 (m, 4H), 2.59 (s, 3H), 2.41 (s, 3H), 2.38-2.31 (m, 3H), 2.06-1.93 (m, 2H), 1.62 (m, 5H), 1.49 (m, 2H), 1.37-1.30 (m, 2H).LCMS [M+H]+ =930.4 |

(continued)

| Examples | Structure and data analysis |
|---|---|
| **UB-180652 (152)** | 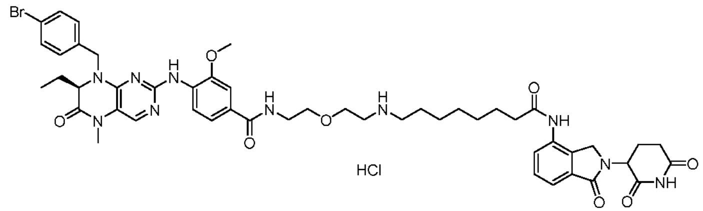 HCl |
| | $^1$H NMR (400 MHz, DMSO-$d_6$) δ 11.02 (s, 1H), 9.89 (d, *J* = 10.9 Hz, 1H), 9.21 - 8.73 (m, 5H), 8.15 - 7.84 (m, 3H), 7.82 (d, *J* = 6.9 Hz, 1H), 7.66 (s, 1H), 7.61 - 7.41 (m, 5H), 7.35 (d, *J* = 8.2 Hz, 2H), 5.28 - 5.05 (m, 2H), 4.54- 4.10 (m, 4H), 3.93 (s, 3H), 3.93 - 3.37 (m, 10H), 3.24 (s, 3H), 3.18 - 2.79 (m, 3H), 2.78 - 2.56 (m, 4H), 2.38 - 2.29 (m, 38H), 2.11 (s, 1H), 2.29 - 1.50 (m, 3H), 1.54 - 1.50 (m, 1H), 1.42 (d, *J* = 7.1 Hz, 2H), 0.88 - 0.19 (m, 3H). LCMS $[M+H]^+$ =996.3 |
| **UB-180653 (153)** | 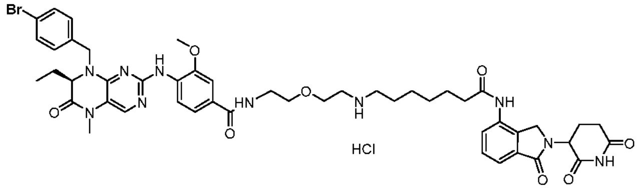 HCl |
| | $^1$H NMR (400 MHz, DMSO-$d_6$) δ 11.02 (s, 1H), 9.84 (s, 1H), 8.73 (m, 3H), 7.91 (m, 1H), 7.82 (m, 2H), 7.64 (m, 1H), 7.54 (m, 2H), 7.52-7.42 (m, 3H), 7.35 (m, 2H), 5.18-5.11 (m, 2H), 4.62 (m, 1H), 4.44-4.31 (m, 3H), 3.93 (s, 3H), 3.71 (m, 2H), 3.62-3.58 (m, 3H), 3.48 (m, 4H), 3.23 (m, 3H), 3.11 (m, 3H), 2.88 (m, 3H), 2.63 (m, 2H), 2.35 (t, 3H), 2.05-2.00 (m, 1H), 1.88 (m, 2H), 1.60 (m, 4H), 0.74 (t, 3H).LCMS $[M+H]^+$ =980.8 |
| **UB-180654 (154)** | 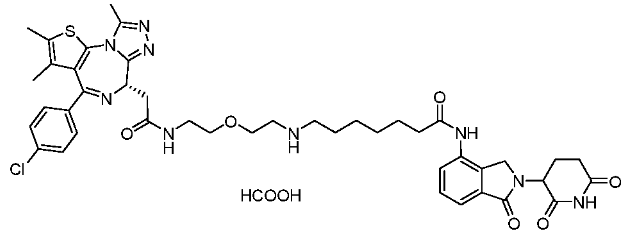 HCOOH<br>$^1$H NMR (400 MHz, DMSO-$d_6$) δ 11.02 (s, 1H), 9.86 (s, 1H), 8.80 (m, 2H), 8.44 (m, 1H), 7.82 (m, 1H), 7.54-7.41 (m, 6H), 5.15 (m, 1H), 4.55 (m, 1H), 4.45-4.31 (m, 2H), 3.70 (m, 2H), 3.51 (m, 2H), 3.36- 3.28 (m, 4H), 3.16-3.10 (m, 2H), 2.97-2.86 (m, 3H), 2.62 (m, 4H), 2.41 (s, 3H), 2.34 (m, 3H), 2.02 (m, 1H), 1.62 (m, 5H), 1.59 (m, 2H), 1.30 (m, 4H). LCMS [M+H]+ =856.4 |

(continued)

| Examples | Structure and data analysis |
|---|---|
| **UB-180655 (155)** | 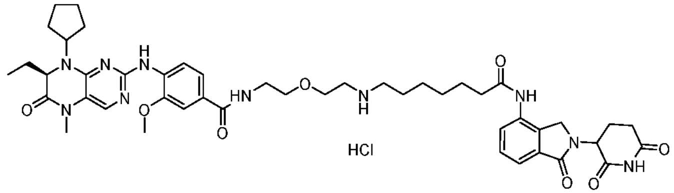 |
| | $^1$H NMR (400 MHz, DMSO-$d_6$) $\delta$11.02 (s, 1H), 9.81 (s, 1H), 8.69 (m, 2H), 7.97 (m, 1H), 7.85-7.78 (m, 2H), 7.65 (m, 1H), 7.58 (m, 1H), 7.52-7.43 (m, 2H), 5.19-5.12 (m, 1H), 4.44 (m, 1H), 4.41-4.29 (m, 2H), 4.20 (m 1H), 3.93 (s, 3H), 3.71 (m, 2H), 3.61 (m, 2H), 3.50 (m, 3H), 3.21 (s, 3H), 3.12 (m, 3H), 2.90 (m, 3H), 2.68-2.59 (m, 2H), 2.36 (m, 3H), 2.00 (m, 4H), 1.82 (m, 3H), 1.65-1.57 (m, 5H), 1.49 (m, 2H), 1.29 (m, 4H), 0.75 (t, $J$ = 7.4 Hz, 3H). LCMS $[M+H]^+$ =881.6. |
| **UB-180656 (156)** | 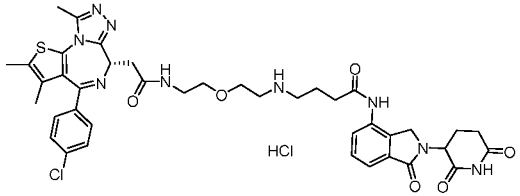 |
| | $^1$H NMR (400 MHz, DMSO-$d_6$) $\delta$ 11.02 (s, 1H), 10.08 (s, 1H), 9.06 (s, 2H), 8.49 (t, $J$ = 5.4 Hz, 1H), 7.80 (t, $J$ = 29.2 Hz, 1H), 7.61 - 7.36 (m, 6H), 5.25-5.10 (m,1H), 4.41 (d, $J$ = 21.5 Hz, 3H), 3.83 - 3.57 (m, 4H), 3.57 - 3.29 (m, 4H), 3.29 - 3.22 (m, 2H), 3.07- 2.98 (m, 3H), 3.01 - 2.69 (m, 11H), 3.01 - 2.26 (m, 137H), 2.04 -1.96(m, 3H), 1.62 (s, 3H), 1.37 - 1.17 (m, 4H).LCMS $[M+H]^+$ =814.3 |
| **UB-180657 (157)** | 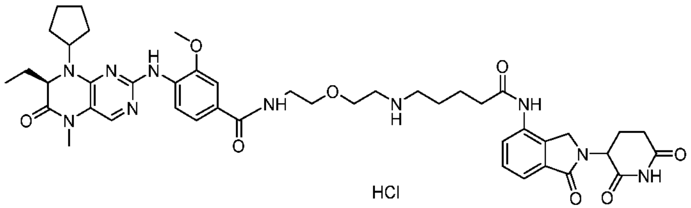 |
| | $^1$H NMR (400 MHz, DMSO-$d_6$) $\delta$ 11.02 (s, 1H), 9.93 (s, 1H), 8.90 (s, 1H), 8.77 (s, 1H), 7.97 - 7.82 (m, 3H), 7.69 (s, 2H), 7.69 (s, 1H), 7.69 - 7.39 (m, 4H), 5.15 (dd, $J$ = 13.2, 5.1 Hz, 1H), 4.39 -4.16(m, 4H),3.92 (m, 3H), 3.72 - 3.57 (m, 4H), 3.24 (s 3H), 3.13- 2.76 (m, 5H), 2.09 - 1.49 (m, 16H), 0.88 - 0.09 (m, 3H).LCMS $[M+H]^+$ =854.4 |

(continued)

| Examples | Structure and data analysis |
|---|---|
| **UB-180658 (158)** | 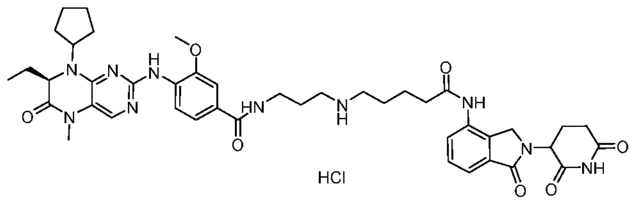 HCl |
| | $^1$H NMR (400 MHz, DMSO-$d_6$) δ 11.02 (s, 1H), 9.94 (s, 1H), 9.39 (s, 1H), 8.80 (d, J = 8.0 Hz, 3H), 7.92 (d, J = 8.1 Hz, 1H), 7.90 - 7.76 (m, 2H), 7.64 (d, J = 1.9 Hz, 1H), 7.60 - 7.44 (m, 3H), 5.15 (dd, J = 13.2, 5.2 Hz, 1H), 4.56 - 4.29 (m, 3H), 3.92 (s, 3H), 3.22 (s, 3H), 3.06 - 2.78 (m, 5H), 2.70 - 2.56 (m, 2H), 2.44 (d, J = 6.7 Hz, 2H), 2.39 - 2.26 (m, 2H), 1.91 (m, 10H), 1.75 - 1.63 (m, 4H), 1.58 - 1.39 (m, 4H), 0.75 (t, J = 7.4 Hz, 3H). LCMS [M+H]+ =823.4 |
| **UB-180659 (159)** | 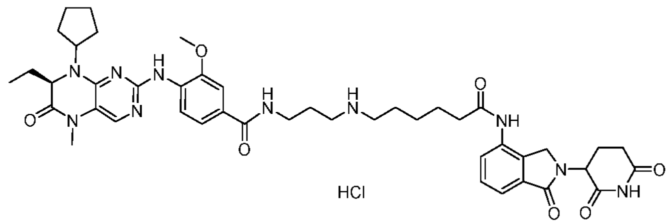 HCl |
| | $^1$H NMR (400 MHz, DMSO-$d_6$) δ 9.83 (s, 1H), 8.52 (s, 1H), 8.42 (d, J = 8.3 Hz, 1H), 8.34 (s, 1H), 7.95 (t, J = 4.5 Hz, 1H), 7.84 (s, 1H), 7.60 (s, 1H), 7.54 - 7.42 (m, 4H), 7.22 (s, 1H), 5.14 (dd, J = 13.3, 5.1 Hz, 1H), 4.46 - 4.27 (m, 3H), 4.24 (dd, J = 7.6, 3.5 Hz, 1H), 3.93 (s, 3H), 3.24 (s, 3H), 3.05 - 2.83 (m, 2H), 2.72 (td, J = 15.4, 7.6 Hz, 5H), 2.44 - 2.29 (m, 3H), 2.08 - 1.48 (m, 20H), 1.36 (d, J = 7.3 Hz, 2H), 0.76 (t, J = 7.4 Hz, 3H).LCMS [M+H]+ =837.4 |
| **UB-180660 (160)** | 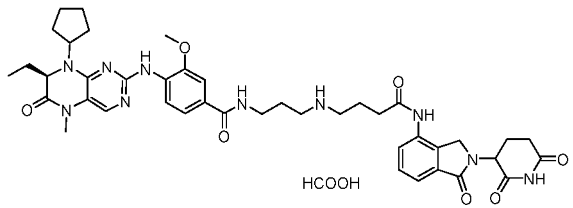 HCOOH |
| | $^1$H NMR (400 MHz, DMSO-$d_6$) δ 11.02 (s, 1H), 9.86 (s, 1H), 8.53 - 8.35 (m, 2H), 8.29 (s, 1H), 7.83 (d, J = 8.0 Hz, 2H), 7.60 (s, 1H), 7.48 (td, J = 7.7, 7.3, 1.8 Hz, 4H), 5.32 (t, J = 4.8 Hz, 1H), 5.14 (dd, J = 13.3, 5.1 Hz, 1H), 4.45 - 4.28 (m, 3H), 4.24 (dd, J = 7.6, 3.5 Hz, 1H), 3.93 (s, 3H), 3.25 (s, 3H), 2.79 - 2.64 (m, 5H), 2.33 (dd, J = 3.7, 1.8 Hz, 1H), 2.00 (q, J = 6.9, 6.4 Hz, 4H), 1.91 - 1.55 (m, 15H), 0.76 (t, J = 7.4 Hz, 3H). LCMS [M+H]+ =809.5 |

(continued)

| Examples | Structure and data analysis |
|---|---|
| **UB-180661 (161)** | 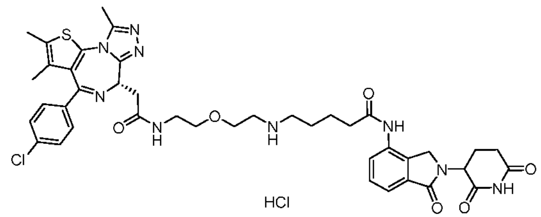 |
| | $^1$H NMR (400 MHz, DMSO-$d_6$) δ 11.02 (s, 1H), 9.98 (s, 1H), 8.96 (s, 2H), 8.49 (s, 1H), 7.83 (d, $J$ = 6.7 Hz, 2H), 7.59 - 7.28 (m, 4H), 5.63 - 4.52 (m, 1H), 4.07-4.21 (m, 4H),3.93(s,3H), 3.87 - 3.57 (m, 4H), , 3.63 - 3.24 (m, 4H), 3.21 - 2.88 (m, 9H), 2.86 - 2.74 (m, 4H), 2.03 -1.78(m,9H), 1.54 - 1.15 (m, 5H), 1.38 - 0.99 (m, 3H).LCMS [M+H]$^+$ =828.3 |
| **UB-180662 (162)** | 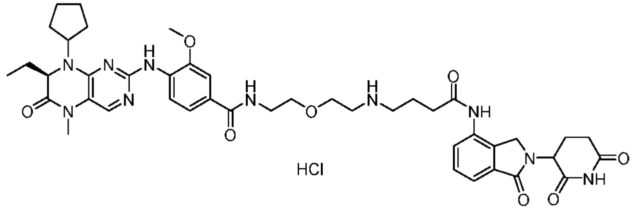 |
| | $^1$H NMR (400 MHz, DMSO-$d_6$) δ 11.02 (s, 1H), 10.03 (d, $J$ = 3.1 Hz, 1H), 9.65 (s, 1H), 9.05 (s, 2H), 8.84 (t, $J$ = 5.3 Hz, 1H), 7.85 (dd, $J$ = 8.6, 4.0 Hz, 3H), 7.72 (d, $J$ = 1.4 Hz, 1H), 7.62 (d, $J$ = 8.3 Hz, 1H), 7.61 - 7.28 (m, 2H), 5.14 (dd, $J$ = 13.2, 5.1 Hz, 1H), 4.64 - 4.30 (m, 3H), 4.37 (t, $J$ = 16.4 Hz, 1H), 3.93(s, 3H), 3.89-3.41 (m, 7H), 3.16 - 2.92 (m, 4H), 2.27 - 1.48(m, 16H), 0.78 (t, $J$ = 7.2 Hz, 3H).LCMS [M+H]$^+$ =839.4 |
| **UB-180663 (163)** | 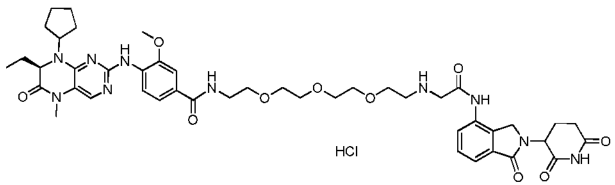<br>$^1$H NMR (400 MHz, DMSO-$d_6$) δ 11.02 (s, 1H), 10.04 (d, J = 15.1 Hz, 1H), 9.76 (s, 1H), 8.91 (s, 3H), 8.69 (t, J = 5.6 Hz, 1H), 7.85 (dt, J = 8.0, 4.6Hz, 3H), 7.64 (d, J = 1.9 Hz, 1H), 7.57 - 7.47 (m, 3H), 5.15 (dd, J = 13.3, 5.1 Hz, 1H), 4.50 (dd, J = 6.4, 3.3 Hz, 1H), 4.48 - 4.38 (m, 2H), 3.90 (s,3H), 3.70 (h, J = 4.7 Hz, 4H), 3.43 (q, J = 5.9 Hz, 2H), 3.21 (s, 3H), 3.07 (d, J = 7.6 Hz, 2H), 2.97 - 2.89 (m, 3H), 2.61 (d, J = 18.2 Hz, 3H), 2.46 - 2.40 (m, 2H), 2.05 - 2.00 (m, 1H), 1.97 - 1.90 (m, 2H), 1.82 (q, J = 7.1 Hz, 3H), 1.71 - 1.63 (m, 4H), 1.49 (d, J = 16.1 Hz, 3H), 0.75 (t, J = 7.4 Hz,3H)..LCMS [M+H]$^+$ =899.4 |

(continued)

| Examples | Structure and data analysis |
|---|---|
| **UB-180664 (164)** | 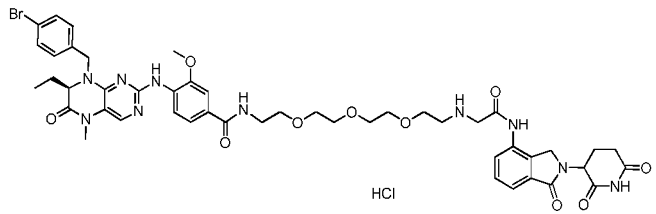 HCl |
| | $^1$H NMR (400 MHz, DMSO-$d_6$) δ 11.02 (s, 1H), 10.00 (d, J = 10.0 Hz, 1H), 9.61 (s, 1H), 8.84 (s, 2H), 8.61 (t, J = 5.5 Hz, 1H), 7.95 (d, J = 3.6 Hz, 1H), 7.89 -7.74 (m, 2H), 7.58 (d, J = 1.9 Hz, 1H), 7.55 - 7.44 (m, 4H), 7.40 - 7.31 (m, 3H), 5.22 - 5.10 (m, 2H), 4.63 (d, J = 15.4 Hz, 1H), 4.51 - 4.31 (m, 3H), 3.90 (s, 3H), 3.70 (d, J = 6.3 Hz, 2H), 3.24 (s, 4H), 3.13 - 3.00 (m, 3H), 2.99 - 2.83 (m, 4H), 2.63 (s, 1H), 2.38 (d, J = 15.0 Hz, 1H), 2.14 - 1.97 (m, 2H), 1.95 - 1.85 (m, 2H), 1.67 (s, 5H), 0.74 (t, J = 7.4 Hz, 3H).LCMS $[M+H]^+$ = 999.31 |
| **UB-180665 (165)** | 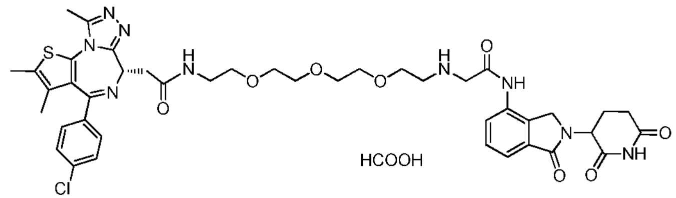 HCOOH |
| | $^1$H NMR (400 MHz, DMSO-$d_6$) δ 11.02 (s, 1H), 8.26 (t, J = 5.7 Hz, 1H), 7.87 (dd, J = 7.1, 1.9 Hz, 1H), 7.56 - 7.40 (m, 7H), 5.15 (dd, J = 13.3, 5.1 Hz, 1H), 4.50 (dd, J = 7.9, 6.2 Hz, 1H), 4.45 - 4.31 (m, 2H), 3.53 (d, J = 9.7 Hz, 10H), 3.44 (d, J = 11.8 Hz, 2H), 3.27 - 3.17 (m, 4H), 3.03 - 2.81 (m, 4H), 2.59 (s,4H), 2.46 (dd, J = 4.6, 2.8 Hz, 1H), 2.41 (s, 3H), 2.37 - 2.27 (m, 2H), 2.03 (d, J = 12.6 Hz, 1H), 1.62 (s, 3H).LCMS $[M+H]^+$ = 874.20 |
| **UB-180666 (166)** | 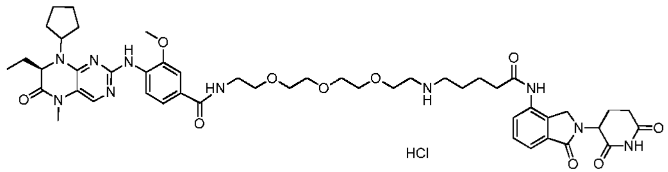 HCl |
| | $^1$H NMR (400 MHz, DMSO-$d_6$) δ 11.02 (s, 1H), 10.04 (d, J = 15.1 Hz, 1H), 9.76 (s, 1H), 8.91 (s, 3H), 8.69 (t, J = 5.6 Hz, 1H), 7.85 (dt, J = 8.0, 4.6 Hz, 3H), 7.64(d, J = 1.9 Hz, 1H), 7.58 - 7.43 (m, 3H), 5.15 (dd, J = 13.3, 5.1 Hz, 1H), 4.52 - 4.47 (m, 1H), 4.44 - 4.32 (m, 2H), 3.90 (s, 3H), 3.70 (dt, J = 10.6, 5.0 Hz, 3H),3.48 - 3.38 (m, 2H), 3.21 (s, 3H), 3.07 (d, J = 7.6 Hz, 3H), 3.00 - 2.89 (m, 3H), 2.70 - 2.55 (m, 4H), 2.45 - 2.36 (m, 2H), 2.14 - 1.98 (m, 2H), 1.98 - 1.75 (m,6H), 1.72 - 1.60 (m, 4H), 1.48 (d, J = 22.8 Hz, 4H), 0.75 (t, J = 7.4 Hz, 3H). LCMS $[M+H]^+$ = 941.30 |

(continued)

| Examples | Structure and data analysis |
|---|---|
| **UB-180667 (167)** | 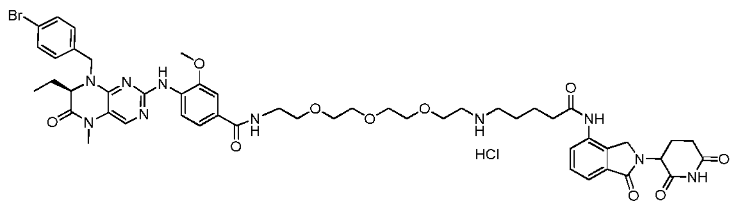<br>$^{1}H$ NMR (400 MHz, DMSO-$d_6$) δ 11.02 (s, 1H), 10.00 (d, J = 10.0 Hz, 1H), 9.61 (s, 1H), 8.84 (s, 2H), 8.61 (t, J = 5.5 Hz, 1H), 7.96 (d, J = 3.6 Hz, 1H), 7.87 -7.74 (m, 2H), 7.58 (d, J = 1.9 Hz, 1H), 7.56 - 7.46 (m, 4H), 7.40 - 7.32 (m, 3H), 5.23 - 5.06 (m, 2H), 4.64 (d, J = 15.4 Hz, 1H), 4.51 - 4.31 (m, 3H), 3.91 (s, 3H), 3.68 (t, J = 5.3 Hz, 2H), 3.57 (d, J = 6.8 Hz, 10H), 3.24 (s, 3H), 3.12 - 3.01 (m, 3H), 3.00 - 2.85 (m, 4H), 2.61 (d, J = 16.8 Hz, 2H), 2.44 - 2.35 (m, 2H), 2.16 - 1.97 (m, 2H), 1.96 - 1.85 (m, 2H), 1.67 (s, 4H), 0.75 (t, J = 7.4 Hz, 3H). LCMS $[M+H]^+$ = 1040.96 |
| **UB-180668 (168)** | 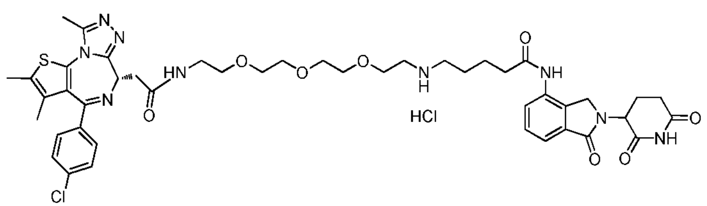<br>$^{1}H$ NMR (400 MHz, DMSO-$d_6$) δ 11.02 (s, 1H), 10.03 (s, 1H), 8.88 (d, J = 33.7 Hz, 2H), 8.34 (t, J = 5.7 Hz, 1H), 7.83 (dd, J = 7.0, 2.0 Hz, 1H), 7.59 - 7.37 (m, 7H), 5.15 (dd, J = 13.3, 5.1 Hz, 1H), 4.56 (dd, J = 7.8, 6.3 Hz, 1H), 4.51 - 4.31 (m, 2H), 3.70 (q, J = 5.3 Hz, 3H), 3.60 - 3.52 (m, 10H), 3.46 (t, J = 5.9Hz, 2H), 3.31 - 3.20 (m, 4H), 3.10 - 3.05 (m, 2H), 2.98 - 2.88 (m, 3H), 2.65 (d, J = 1.4 Hz, 3H), 2.42 (d, J = 1.9 Hz, 5H), 2.07 - 1.98 (m, 1H), 1.62 (s, 3H). LCMS $[M+H]^+$ = 915.99 |
| **UB-180670 (170)** | 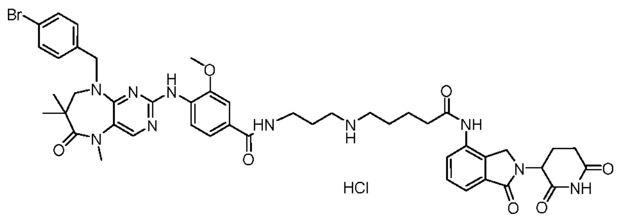<br>$^{1}H$ NMR (400 MHz, DMSO-$d_6$) δ 11.02 (s, 1H), 9.98 (s, 1H), 8.66 - 8.40 (m, 4H), 8.10 (s, 1H), 7.83 (d, *J* = 7.0 Hz, 1H), 7.64 - 7.48 (m, 5H),7.46-7.35(m, 3H), 5.15 (dd, *J* = 13.3, 5.2 Hz, 1H), 4.95 (s, 2H), 4.39 (dd, *J* = 15.8, 8.7 Hz, 2H), 3.92 (s, 3H), 3.67 - 3.56 (m, 4H), 3.25-3.19 (m, 4H), 2.93 - 2.78 (m, 4H), 2.56 - 2.45 (m, 1H), 1.88 (s, 1H), 1.05 (s, 6H). LCMS $[M+H]^+$ =937.3 |

(continued)

| Examples | Structure and data analysis |
|---|---|
| **UB-180671 (171)** | 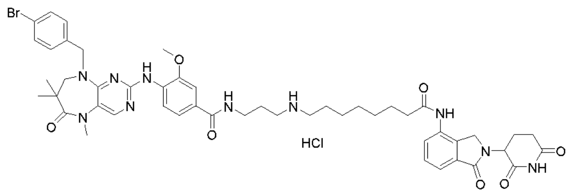 |
| | $^1$H NMR (400 MHz, DMSO-$d_6$) δ 11.02 (s, 1H), 9.98 (s, 1H), 9.65 (s, 1H), 9.05 (s, 2H), 8.84 (d, *J* = 5.3 Hz, 2H), 8.15(s,1H),7.85 (dd, *J* = 8.6, 4.0 Hz, 1H), 7.72 (d, *J* = 1.4 Hz, 1H), 7.62 (d, *J* = 8.3 Hz, 1H), 7.61 - 7.28 (m, 2H), 5.14 (dd, *J* = 13.2, 5.1 Hz, 1H), 4.98(s,2H),4.45 - 4.30 (m, 2H), 3.98 (s, 3H), 3.63 - 3.47 (m,2H), 3.39 (s, 2H), 3.21 (s, 3H), 3.16 - 2.92 (m, 2H), 2.92 - 2.66 (m, 6H), 2.57 - 2.46 (m, 4H), 2.05-1.58 (m, 7H), 0.78 (s, 6H).LCMS $[M+H]^+$ =982.3 |
| **UB-180672 (172)** | 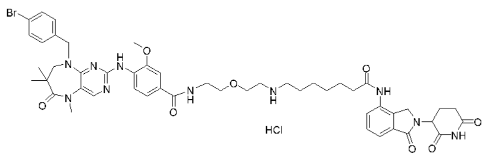 |
| | $^1$H NMR (400 MHz, DMSO-$d_6$) δ 11.02 (s, 1H), 10.03 (d, *J* = 3.1 Hz, 1H), 9.65 (s, 9H), 9.05 (s, 2H), 8.84 (t, *J* = 5.3 Hz, 1H), 8.15((s, 2H),7.85 (dd, *J* = 8.6, 4.0 Hz, 3H), 7.72 (d, *J* = 1.4 Hz, 1H), 7.62 (d, *J* = 8.3 Hz, 1H), 7.61 - 7.28 (m, 2H), 5.14 (dd, *J* = 13.2, 5.1 Hz, 1H), 4.94 - 4.30 (m, 3H), 3.98 (m, 3H), 3.79-3.39 (m, 10H), 3.21 (s, 3H), 3.16 - 2.92 (m, 5H), 2.62 - 2.56 (m, 1H), 2.47 - 2.36 (m, 4H), 2.05- 1.98 (m, 1H), 1.65 - 1.56 (m, 4H), 1.23 (s, 4H), 1.08 (s, 6H). LCMS $[M+H]^+$ =996.3 |
| **UB-180673 (173)** | 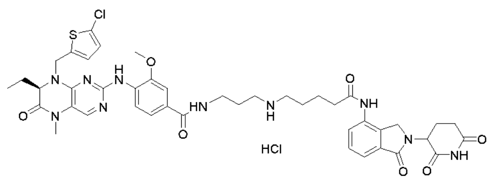 |
| | $^1$H NMR (400 MHz, DMSO-$d_6$) δ 11.02 (s, 1H), 9.95 (s, 1H), 8.78 (m, 3H), 8.08 (d, *J* = 8.1 Hz, 1H), 7.90 (s, 1H), 7.83 (dd, *J* = 7.2, 1.9 Hz, 1H), 7.64 (d, *J* = 1.8 Hz, 1H), 7.56 - 7.42 (m, 3H), 7.11 (d, *J* = 3.8 Hz, 1H), 6.98 (d, *J* = 3.8 Hz, 1H), 5.21 - 5.07 (m, 2H), 4.80 (d, *J* = 15.5 Hz, 1H), 4.54 - 4.48 (m, 1H), 4.39 (q, *J* = 17.6 Hz, 2H), 3.95 (s, 3H), 3.22 (s, 3H), 3.01 - 2.77 (m, 6H), 2.70 - 2.53 (m, 2H), 2.49 - 2.26 (m, 4H), 2.13 - 1.80 (m, 6H), 1.68 (s, 4H), 0.67 (t, *J* = 7.4 Hz, 3H). LCMS [M+H]+ =885.4 |

(continued)

| Examples | Structure and data analysis |
|---|---|
| **UB-180674 (174)** | 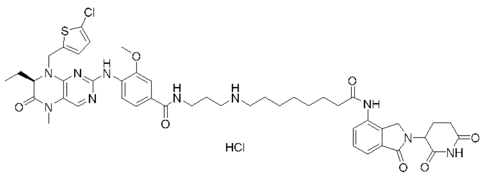 |
| | $^1$H NMR (400 MHz, DMSO-$d_6$) δ 11.02 (s, 1H), 9.87 (s, 1H), 9.65 (s, 1H), 8.82 (d, *J* = 8.3 Hz, 3H), 8.03 (d, *J* = 8.5 Hz, 1H), 7.92 (s, 1H), 7.82 (dd, *J* = 7.0, 2.1 Hz, 1H), 7.66 (d, *J* = 1.9 Hz, 1H), 7.58 - 7.38 (m, 3H), 7.11 (d, *J* = 3.8 Hz, 1H), 6.98 (d, *J* = 3.8 Hz, 1H), 5.23 - 4.99 (m, 2H), 4.82 (d, *J* = 15.4 Hz, 1H), 4.53 (dd, *J* = 5.6, 3.6 Hz, 1H), 4.37 (q, *J* = 17.5 Hz, 2H), 3.95 (s, 3H), 3.34 (d, *J* = 6.3 Hz, 2H), 3.21 (s, 3H), 2.99 - 2.83 (m, 5H), 2.70 - 2.57 (m, 2H), 2.35 (dt, *J* = 12.4, 5.2 Hz, 3H), 2.10 - 1.97 (m, 1H), 1.92 (q, *J* = 7.2 Hz, 4H), 1.61 (m, 4H), 1.39 - 1.22 (m, 9H), 0.67 (t, *J* = 7.4 Hz, 3H).LCMS [M+H]+ =927.3 |
| **UB-180675 (175)** | 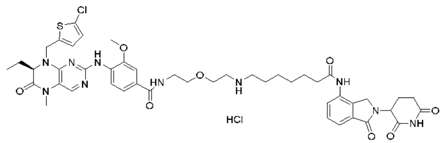 |
| | $^1$H NMR (400 MHz, DMSO-$d_6$) δ 11.02 (s, 1H), 9.83 (s, 1H), 8.71 (d, *J* = 29.1 Hz, 3H), 8.14 (s, 1H), 7.88 (s, 1H), 7.82 (dd, *J* = 7.2, 1.8 Hz, 1H), 7.67 (d, *J* = 1.9 Hz, 1H), 7.58 - 7.41 (m, 3H), 7.11 (d, *J* = 3.8 Hz, 1H), 6.98 (d, *J* = 3.8 Hz, 1H), 5.20 - 5.04 (m, 2H), 4.79 (d, *J* = 15.5 Hz, 1H), 4.49 (d, *J* = 4.9 Hz, 1H), 4.48 - 4.20 (m, 2H), 3.97 (s, 3H), 3.70 (t, *J* = 5.1 Hz, 2H), 3.60 (t, *J* = 5.8 Hz, 2H), 3.21 (s, 3H), 3.10 (s, 2H), 2.99 - 2.79 (m, 3H), 2.63 (s, 2H), 2.35 (q, *J* = 7.5 Hz, 3H), 2.05 - 1.81 (m, 4H), 1.59 (d, *J* = 8.2 Hz, 4H), 1.28 (d, *J* = 5.7 Hz, 4H), 0.66 (t, *J* = 7.4 Hz, 3H).LCMS [M+H]+ =943.2 |
| **UB-180676 (176)** | 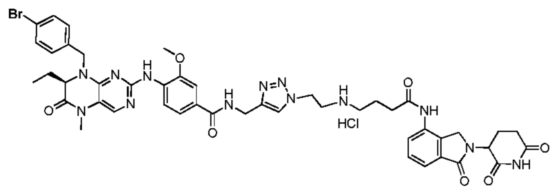 |
| | $^1$H NMR (400 MHz, DMSO-$d_6$) δ 11.02 (s, 1H), 10.01 (s, 1H), 9.10 (d, J = 23.7 Hz, 3H), 8.10 (s, 1H), 7.93 (s, 1H), 7.84 (dd, J = 7.2, 1.9 Hz, 2H), 7.60 (d, J= 1.9 Hz, 1H), 7.57 - 7.47 (m, 4H), 7.43 (dd, J = 8.4, 1.9 Hz, 1H), 7.34 (d, J = 8.3 Hz, 2H), 5.19 - 5.10 (m, 2H), 4.72 (t, J = 6.3 Hz, 2H), 4.60 (d, J = 15.9Hz, 1H), 4.52 (d, J = 5.6 Hz, 2H), 4.47 - 4.31 (m, 3H), 3.91 (s, 3H), 3.24 (s, 3H), 3.06 - 2.87 (m, 3H), 2.61 (d, J = 16.5 Hz, 1H), 2.07 - 1.80 (m, 6H), 0.75 (t,J = 7.4 Hz, 3H).LCMS [M+H]+ = 978.39 |

(continued)

| Examples | Structure and data analysis |
| --- | --- |
| **UB-180677 (177)** | 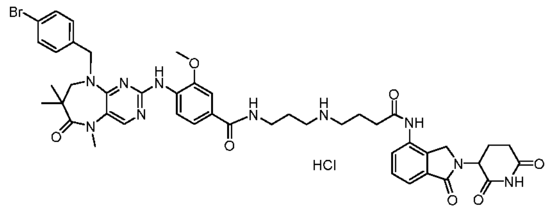 HCl<br>$^1$H NMR (400 MHz, DMSO-$d_6$) δ 11.02 (s, 1H), 10.03 (d, *J* = 3.1 Hz, 1H), 8.65 (s, 3H), 8.11 (s, 1H), 7.93 (t, *J* = 5.3 Hz, 1H), 7.85 (dd, *J* = 8.6, 4.0 Hz, 1H), 7.62 -7.49(m, 6H), 7.42 (d, *J* = 8.3 Hz, 4H), 5.14 (dd, *J* = 13.2, 5.1 Hz, 1H), 4.94(s, 2H), 4.43 - 4.10 (m, 2H), 3.98 (s, 3H),.3.63-3.51 (m, 4H), 3.21 (s, 3H), 2.92 - 2.86 (m, 4H), 2.66 (s, 1H), 2.47 - 2.36 (m, 2H), 2.06 - 1.80 (m, 7H), 1.18 (s, 6H). LCMS $[M+H]^+$ =925.3 |
| **UB-180678 (178)** | 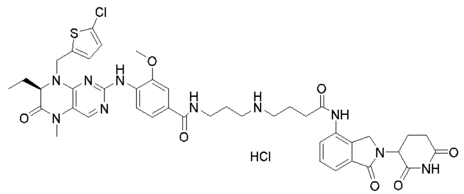 HCl |
| | $^1$H NMR (400 MHz, DMSO-$d_6$) δ 11.02 (s, 1H), 10.02 (s, 1H), 8.11 (d, *J* = 8.2 Hz, 1H), 7.90 (s, 1H), 7.84 (d, *J* = 7.2 Hz, 1H), 7.63 (d, *J* = 1.9 Hz, 1H), 7.51 (td, *J* = 7.4, 6.3, 3.2 Hz, 3H), 7.11 (d, *J* = 3.9 Hz, 1H), 6.98 (d, *J* = 3.8 Hz, 1H), 5.14 (dd, *J* = 14.0, 7.7 Hz, 2H), 4.79 (d, *J* = 15.4 Hz, 1H), 4.50 (t, *J* = 4.7 Hz, 1H), 4.40 (q, *J* = 17.5 Hz, 2H), 3.95 (s, 3H), 3.22 (s, 3H), 3.04 - 2.93 (m, 5H), 2.69 - 2.52 (m, 2H), 2.33 (h, *J* = 10.6, 10.1 Hz, 2H), 2.10 - 1.80 (m, 8H), 1.34 - 1.26 (m, 1H), 0.67 (t, *J* = 7.4 Hz, 3H).LCMS [M+H]+ =871.2 |
| **UB-180679 (179)** | 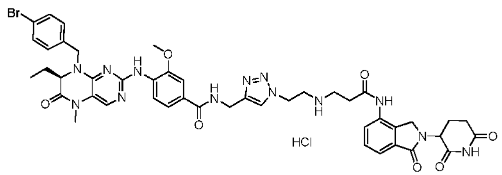 HCl |
| | $^1$H NMR (400 MHz, DMSO-$d_6$) δ 11.03 (s, 1H), 10.21 (s, 1H), 9.10 (d, J = 33.5 Hz, 4H), 8.09 (d, J = 7.4 Hz, 1H), 8.00 - 7.82 (m, 3H), 7.60 (d, J = 1.9 Hz, 1H), 7.53 (h, J = 7.3, 6.9 Hz, 4H), 7.43 (dd, J = 8.5, 1.8 Hz, 1H), 7.34 (d, J = 8.2 Hz, 2H), 5.21 - 5.11 (m, 2H), 4.71 (dt, J = 14.4, 6.3 Hz, 2H), 4.63 - 4.37 (m, 6H), 3.91 (s, 3H), 3.25 (s, 3H), 2.90 (d, J = 6.5 Hz, 2H), 2.67 (d, J = 20.1 Hz, 2H), 2.31 (s, 1H), 2.05 (d, J = 5.8 Hz, 1H), 1.89 (d, J = 7.6 Hz, 2H), 0.75 (t, J =7.4 Hz, 3H). LCMS $[M+H]^+$ = 963.9 |

(continued)

| Examples | Structure and data analysis |
|---|---|
| **UB-180680 (180)** | 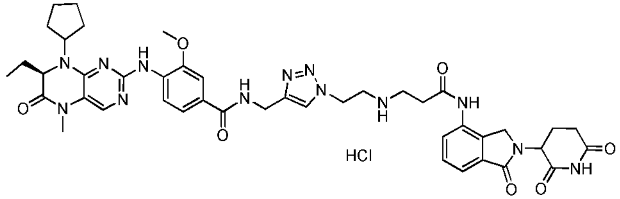 HCl |
| | $^1$H NMR (400 MHz, DMSO-$d_6$) δ 11.02 (s, 1H), 10.30 (s, 1H), 9.76 (s, 1H), 9.41 (s, 2H), 9.17 (t, J = 5.7 Hz, 1H), 8.13 (s, 1H), 7.90 - 7.81 (m, 3H),7.67 (d, J = 1.9 Hz, 1H), 7.60 (dd, J = 8.3, 1.8 Hz, 1H), 7.57 - 7.46 (m, 2H), 5.15 (dd, J = 13.3, 5.1 Hz, 1H), 4.76 (t, J = 6.5 Hz, 2H), 4.54 (d, J = 5.5Hz, 2H), 4.52 - 4.39 (m, 4H), 4.22 - 4.14 (m, 2H), 3.90 (s, 3H), 3.51 (q, J = 6.7, 6.0 Hz, 2H), 3.21 (s, 5H), 3.00 - 2.86 (m, 3H), 2.62 (d, J = 17.5 Hz,1H), 2.40 - 2.26 (m, 1H), 2.08 - 1.75 (m, 7H), 1.58 - 1.41 (m, 4H), 0.75 (t, J = 7.4 Hz, 3H).LCMS $[M+H]^+$ = 862.48 |
| **UB-180681 (181)** | 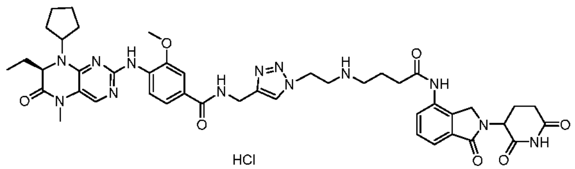 HCl |
| | $^1$H NMR (400 MHz, *DMSO-d6*) δ 11.02 (s, 1H), 10.08 (s, 1H), 9.74 (s, 1H), 9.33 (s, 2H), 9.16 (t, J = 5.6 Hz, 1H), 8.12 (s, 1H), 7.93 - 7.80 (m, 3H), 7.67 (d, J =1.8 Hz, 1H), 7.59 (dd, J = 8.4, 1.8 Hz, 1H), 7.54 - 7.40 (m, 1H), 5.15 (dd, J = 13.2, 5.2 Hz, 1H), 4.75 (t, J = 6.5 Hz, 2H), 4.62 - 4.42 (m, 6H), 4.24 - 4.15 (m,2H), 3.90 (s, 3H), 3.53 - 3.41 (m, 2H), 3.22 (s, 3H), 3.07 - 2.83 (m, 3H), 2.40 - 2.27 (m, 1H), 2.08 - 1.76 (m, 9H), 1.63 - 1.40 (m, 4H), 0.75 (t, J = 7.4 Hz, 3H). LCMS [M+H]+ = 876.54 |
| **UB-180682 (182)** | 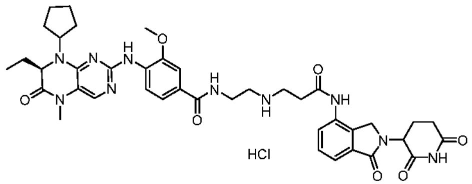 HCl |
| | $^1$H NMR (400 MHz, *DMSO-d6*) δ 11.02 (s, 1H), 10.25 (s, 1H), 9.71 (s, 1H), 9.26 (s, 2H), 9.00 (t, J = 5.5 Hz, 1H), 7.92 - 7.80 (m, 3H), 7.75 (d, J = 1.8 Hz, 1H), 7.63 (dd, J = 8.3, 1.8 Hz, 1H), 7.58 - 7.45 (m, 2H), 5.16 (dd, J = 13.3, 5.1 Hz, 1H), 4.57 - 4.47 (m, 2H), 4.47 - 4.32 (m, 4H), 4.16 (s, 2H), 3.65 (q, J =5.7 Hz, 2H), 3.22 (s, 7H), 3.00 - 2.89 (m, 3H), 2.61 (d, J = 17.6 Hz, 1H), 2.40 - 2.25 (m, 1H), 2.10 - 1.72 (m, 7H), 1.61 - 1.38 (m, 4H), 0.76 (t, J = 7.4 Hz,3H). LCMS $[M+H]^+$ = 781.48 |

(continued)

| Examples | Structure and data analysis |
|---|---|
| **UB-180683 (183)** | 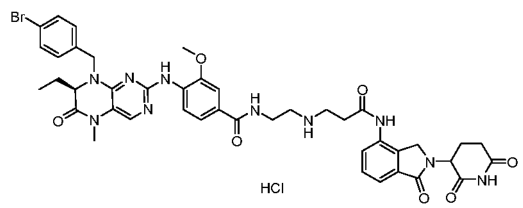 |
| | $^1$H NMR (400 MHz, *DMSO-d6*) δ 11.02 (s, 1H), 10.24 (s, 1H), 9.64 (s, 1H), 9.19 (s, 2H), 8.94 (t, J = 5.6 Hz, 1H), 7.97 (s, 1H), 7.86 - 7.74 (m, 2H), 7.70 (d, J = 1.9 Hz, 1H), 7.59 - 7.41 (m, 5H), 7.35 (d, J = 8.4 Hz, 2H), 5.22 - 5.08 (m, 2H), 4.65 (d, J = 15.7 Hz, 1H), 4.51 - 4.32 (m, 4H), 3.93 (s, 3H), 3.63 (q, J =6.0 Hz, 2H), 3.24 (s, 5H), 3.20 - 3.10 (m, 2H), 2.94 (q, J = 6.8, 6.0 Hz, 3H), 2.61 (d, J = 18.1 Hz, 2H), 2.31 (t, J = 13.4 Hz, 1H), 2.07 - 1.85 (m, 3H), 0.76 (t,J = 7.4 Hz, 3H).LCMS [M+H]$^+$ = 883.38 |
| **UB-180684 (184)** | 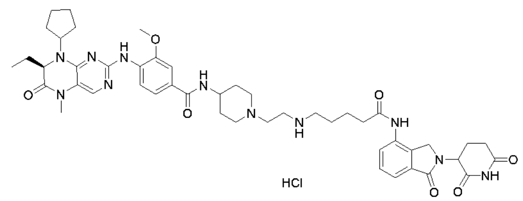 |
| | $^1$H NMR (400 MHz, *d6*-DMSO) δ 11.02 (s, 1H), 10.81 (s, 1H), 9.97 (s, 1H), 9.28 (s, 2H), 8.56 (m, 1H), 7.92 (s, 1H), 7.82 (m, 2H), 7.60 (m, 2H), 7.55-7.47 (m, 2H), 5.16 (m, 1H), 4.50-4.34 (m, 3H), 4.21 (m, 1H), 4.10 (m, 1H), 3.92 (s, 3H), 3.67 (m, 2H), 3.22 (s, 3H), 3.16 (m, 2H), 2.97 (m, 3H), 2.65 (m, 3H), 2.43-2.31 (m, 2H), 2.12-1.64 (m, 17H), 1.51 (m, 4H), 0.76 (t, *J* = 7.4 Hz, 3H). LCMS [M+H]$^+$ =892.4 |
| **UB-180685 (185)** | 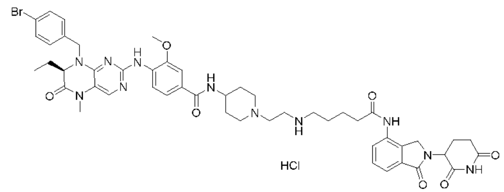 |
| | $^1$H NMR (400 MHz, DMSO-$d_6$) δ 11.02 (s, 1H), 10.74 (s, 1H), 9.94 (s, 1H), 9.21 (m, 2H), 8.49 (m, 1H), 7.92 (s, 1H), 7.82 (m, 1H), 7.58 - 7.49 (m, 4H), 7.47 - 7.40 (m, 1H), 7.35 (m, 2H), 5.16 (m, 2H), 4.58 (m, 1H), 4.47 - 4.33 (m, 3H), 4.08 (m, 1H), 3.93 (s, 3H), 3.66 (m, 2H), 3.25 (s, 3H), 3.16 (m, 2H), 3.05 - 2.89 (m, 4H), 2.69 - 2.58 (m, 2H), 2.47 - 2.41 (m, 2H), 2.33 (m, 1H), 2.10 - 1.94 (m, 6H), 1.88 (m, 2H), 1.71 (m, 4H), 1.46 (m, 1H), 1.27 (m, 2H), 0.86 (m, 1H), 0.74 (t, J = 7.4 Hz, 3H). LCMS [M+H]$^+$ =992.4 |

(continued)

| Examples | Structure and data analysis |
|---|---|
| **UB-180687 (187)** | 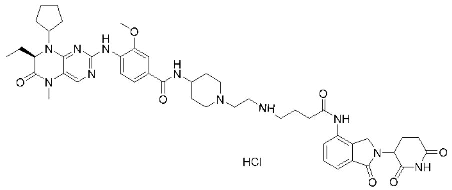 HCl<br>$^1$H NMR (400 MHz, *d6*-DMSO) δ11.03 (s, 1H), 10.73 (s, 1H), 9.99 (s, 1H), 9.23 (m, 2H), 8.53 (s, 1H), 7.88-7.76 (m, 2H), 7.65-7.44 (m, 4H), 5.16 (m, 1H), 4.40 (m, 3H), 4.23 (m, 1H), 4.09 (m, 1H), 3.93 (s, 2H), 3.68 (m, 2H), 3.57 (s, 4H), 3.23 (s, 3H), 3.17 (m, 2H), 3.05 (m, 2H), 2.96-2.87 (m, 1H), 2.70-2.58 (m, 2H), 2.35-2.28 (m, 2H), 2.00 (m, 7H), 1.83 (m, 3H), 1.60 (m, 2H), 1.50 (m, 2H), 0.76 (t, *J* = 7.4 Hz, 3H). LCMS $[M+H]^+$ =879.4 |
| **UB-180688 (188)** | 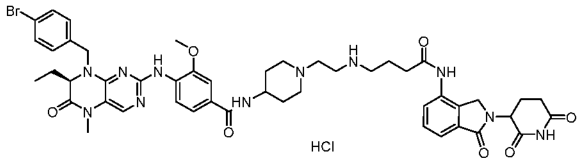 HCl |
| | $^1$H NMR (400 MHz, DMSO-$d_6$) δ 11.03 (s, 1H), 10.71 (s, 1H), 10.00 (s, 1H), 9.24 (m, 2H), 8.49 (d, J = 7.5 Hz, 1H), 7.98 (s, 1H), 7.91 (s, 1H), 7.86-7.80 (m, 1H), 7.59-7.48 (m, 4H), 7.43 (d, J = 8.5 Hz, 1H), 7.35 (m, 2H), 5.24-5.12 (m, 2H), 4.54 (m, 1H), 4.40 (m, 3H), 4.07 (m, 1H), 3.93 (s, 2H), 3.65 (m, 4H), 3.57 (s, 3H), 3.25 (s, 3H), 3.15 (m, 2H), 3.05 (m, 2H), 2.99-2.89 (m, 1H), 2.68-2.62 (m, 2H), 2.38-2.30 (m, 2H), 2.11-1.81 (m, 9H), 1.30-1.24 (m, 1H), 0.74 (t, J = 7.4 Hz, 3H). LCMS $[M+H]^+$ =978.3 |
| **189 (189)** | 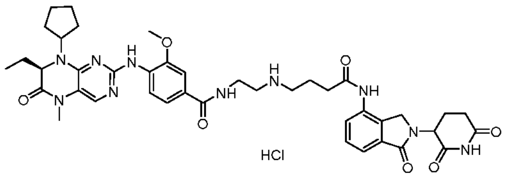 HCl |
| | $^1$H NMR (400 MHz, *DMSO-d6*) δ 11.02 (s, 1H), 10.04 (d, J = 6.0 Hz, 1H), 9.52 (s, 1H), 9.16 (s, 2H), 8.98 (s, 1H), 7.93 (d, J = 7.8 Hz, 1H), 7.87 - 7.80 (m, 2H),7.74 (d, J = 2.2 Hz, 1H), 7.62 (dd, J = 8.3, 1.9 Hz, 1H), 7.54 - 7.45 (m, 2H), 5.15 (dd, J = 13.3, 5.1 Hz, 1H), 4.56 - 4.32 (m, 3H), 4.20 (p, J = 8.9 Hz, 1H), 3.93(s, 3H), 3.65 (dd, J = 10.5, 4.3 Hz, 2H), 3.22 (s, 3H), 3.15 (s, 2H), 3.02 (s, 2H), 2.98 - 2.89 (m, 1H), 2.67 - 2.57 (m, 1H), 2.40 - 2.29 (m, 1H), 2.06 - 1.71 (m,10H), 1.52 (d, J = 32.6 Hz, 4H), 0.76 (t, J = 7.4 Hz, 3H).LCMS $[M+H]^+$ = 795.20 |

(continued)

| Examples | Structure and data analysis |
|---|---|
| **UB-180690 (190)** | 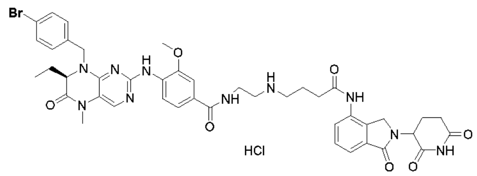 HCl |
| | $^{1}H$ NMR (400 MHz, *DMSO-d6*) δ 11.02 (s, 1H), 10.03 (s, 1H), 9.07 (s, 2H), 8.89 (s, 1H), 7.94 (d, J = 5.5 Hz, 1H), 7.83 (dd, J = 7.5, 1.6 Hz, 1H), 7.67 (s,1H), 7.57 - 7.44 (m, 5H), 7.39 - 7.32 (m, 2H), 5.15 (dd, J = 13.8, 5.8 Hz, 2H), 4.61 (d, J = 16.1 Hz, 1H), 4.48 - 4.31 (m, 3H), 3.93 (s, 3H), 3.66 (s, 4H), 3.25(s, 3H), 3.13 (s, 2H), 3.02 (s, 2H), 2.94 (dd, J = 8.8, 3.9 Hz, 1H), 2.63 (s, 1H), 2.32 (d, J = 30.5 Hz, 1H), 2.10 - 1.81 (m, 6H), 0.75 (t, J = 7.4 Hz, 3H).LCMS $[M+H]^{+}$ = 896.49 |
| **UB-180691 (191)** | 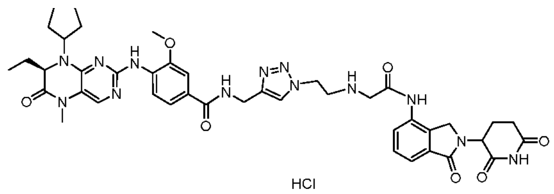 HCl |
| | $^{1}H$ NMR (400 MHz, *DMSO-d6*) δ 11.04 (s, 1H), 10.81 (d, J = 8.5 Hz, 1H), 9.79 (s, 1H), 9.56 (s, 2H), 9.20 (t, J = 5.5 Hz, 1H), 8.12 (s, 1H), 7.91- 7.80 (m, 3H), 7.68 (d, J = 1.8 Hz, 1H), 7.62 - 7.50 (m, 3H), 5.17 (dd, J = 13.2, 5.1 Hz, 1H), 4.77 (t, J = 6.3 Hz, 2H), 4.57 - 4.48 (m, 4H), 4.46(s, 1H), 4.06 (s, 2H), 3.90 (s, 3H), 3.57 (s, 2H), 3.22 (s, 3H), 2.94 (ddd, J = 17.9, 13.5, 5.4 Hz, 1H), 2.63 (d, J = 17.0 Hz, 1H), 2.28 (dd, J = 13.1,4.5 Hz, 1H), 2.06 (dd, J = 9.3, 4.0 Hz, 1H), 2.01 - 1.74 (m, 6H), 1.59 - 1.40 (m, 4H), 0.75 (t, J = 7.4 Hz, 3H).LCMS $[M+H]^{+}$ = 848.35 |
| **UB-180692 (192)** | 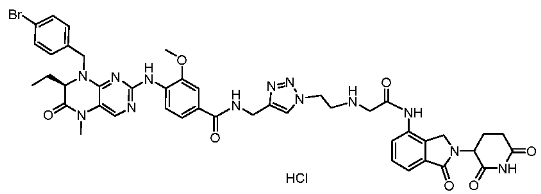 HCl |
| | $^{1}H$ NMR (400 MHz, DMSO-$d_6$) δ 11.04 (s, 1H), 10.70 (d, J = 13.8 Hz, 1H), 9.46 (s, 2H), 9.14 - 9.05 (m, 1H), 8.10 (s, 1H), 7.95 (d, J = 3.7 Hz, 1H), 7.86 (dd, J = 7.5, 1.5 Hz, 2H), 7.63 - 7.56 (m, 2H), 7.54 (d, J = 8.3 Hz, 2H), 7.43 (dd, J = 8.4, 1.9 Hz, 1H), 7.34 (d, J = 8.3 Hz, 2H), 5.16 (dd, J = 13.9, 7.6 Hz, 2H), 4.75 (t, J = 6.4 Hz, 2H), 4.60 (d, J = 15.6 Hz, 1H), 4.53 (d, J = 5.6 Hz, 2H), 4.49 - 4.32 (m, 3H), 4.05 (s, 2H), 3.90 (s, 3H), 3.57 (s, 2H), 3.25 (s, 3H), 2.94 (ddd, J = 17.3, 13.3, 5.3 Hz, 1H), 2.69 - 2.58 (m, 1H), 2.26 (dt, J = 14.2, 7.2 Hz, 2H), 2.10 - 2.00 (m, 1H), 1.89 (d, J = 9.6 Hz, 2H), 0.75 (t, J = 7.4 Hz, 3H).LCMS $[M+H]^{+}$ = 950.32 |

(continued)

| Examples | Structure and data analysis |
|---|---|
| **UB-180693 (193)** | 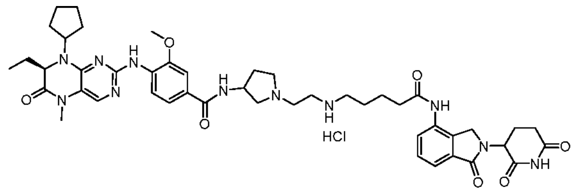 |
| | $^1$H NMR (400 MHz, DMSO-$d_6$) δ 11.43 - 11.24 (m, 1H), 11.02 (s, 1H), 9.95 (s, 1H), 9.25 (s, 2H), 8.04 - 7.93 (m, 1H), 7.88 - 7.79 (m, 2H), 7.72 - 7.56 (m, 2H), 7.53 - 7.43 (m, 2H), 5.16 (m, 1H), 4.80 - 4.69 (m, 1H), 4.52 - 4.30 (m, 3H), 4.29 - 4.16 (m, 1H), 4.00 - 3.90 (m, 3H), 3.72 - 3.43 (m, 6H), 3.22 (s, 3H), 3.07 - 2.81 (m, 4H), 2.70 - 2.54 (m, 4H), 2.11 - 1.39 (m, 14H), 0.76 (t, *J* = 7.4 Hz, 3H). LC-MS: $(M+H)^+$= 878.43 |
| **UB-180694 (194)** | 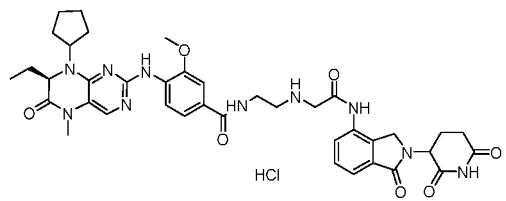 |
| | $^1$H NMR (400 MHz, *DMSO-d6*) δ 11.04 (s, 1H), 10.65 (s, 1H), 9.31 (s, 2H), 8.91 (s, 1H), 7.98 (s, 1H), 7.89 - 7.83 (m, 2H), 7.68 (s, 1H), 7.61 - 7.53 (m,3H), 5.18 (dd, J = 13.3, 5.2 Hz, 1H), 4.56 - 4.32 (m, 3H), 4.28 - 4.03 (m, 3H), 3.93 (s, 3H), 3.65 (d, J = 5.8 Hz, 2H), 2.95 (td, J = 16.6, 15.1, 5.4 Hz, 1H),2.62 (d, J = 17.2 Hz, 1H), 2.27 (dd, J = 13.1, 4.5 Hz, 1H), 2.13 - 2.02 (m, 1H), 2.02 - 1.70 (m, 7H), 1.53 (d, J = 38.8 Hz, 4H), 0.76 (t, J = 7.4 Hz, 3H).LCMS $[M+H]^+$ = 767.41 |
| **UB-180695 (195)** | 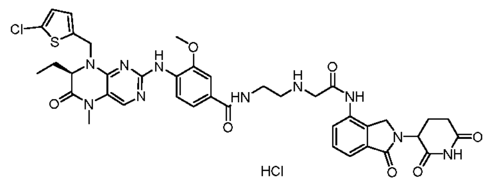 |
| | $^1$H NMR (400 MHz, *DMSO-d6*) δ 11.04 (s, 1H), 10.66 (s, 1H), 9.28 (s, 2H), 8.85 (s, 1H), 8.16 (s, 1H), 7.93 (s, 1H), 7.87 (dd, J = 7.5, 1.6 Hz, 1H), 7.68 (d, J= 1.9 Hz, 1H), 7.63 - 7.50 (m, 3H), 7.12 (d, J = 3.8 Hz, 1H), 6.99 (d, J = 3.8 Hz, 1H), 5.25 - 5.09 (m, 2H), 4.80 (d, J = 15.4 Hz, 1H), 4.54 - 4.32 (m, 3H),4.08 (s, 2H), 3.96 (s, 3H), 3.22 (s, 5H), 3.00 - 2.88 (m, 1H), 2.62 (d, J = 17.1 Hz, 1H), 2.29 (d, J = 4.4 Hz, 1H), 2.10 - 2.01 (m, 1H), 1.96 - 1.82 (m, 2H), 0.68(t, J = 7.4 Hz, 3H).LCMS $[M+H]^+$ = 829.04 |

(continued)

| Examples | Structure and data analysis |
| --- | --- |
| **UB-180696 (196)** | 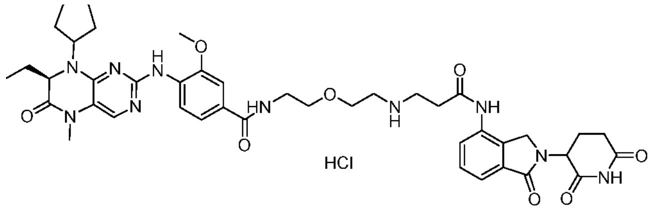 HCl<br>$^1$H NMR (400 MHz, DMSO-$d_6$) δ 11.03 (s, 1H), 10.15 (d, *J* = 2.7 Hz, 1H), 8.87 (s, 2H), 8.70 (s, 1H), 7.93 (s, 1H), 7.87 - 7.76 (m, 2H), 7.65 (s, 1H), 7.61 - 7.43 (m, 3H), 5.16 (dd, *J* = 13.4, 5.2 Hz, 1H), 4.56 - 4.27 (m, 3H), 4.19 (t, *J* = 8.8 Hz, 1H), 3.92 (s, 3H), 3.74 (t, *J* = 5.2 Hz, 2H), 3.61 (d, *J* = 5.8 Hz, 2H), 3.27 (d, *J* = 7.0 Hz, 2H), 3.22 (s, 3H), 3.18 (m, 2H), 3.00 - 2.82 (m, 3H), 2.71 - 2.59 (m, 1H), 2.37 - 2.22 (m, 2H), 2.07 - 1.72 (m, 8H), 1.53 (d, *J* = 34.6 Hz, 4H), 0.75 (t, *J* = 7.4 Hz, 3H). LCMS [M/2+1]+=413.2 |
| **UB-180697 (197)** | 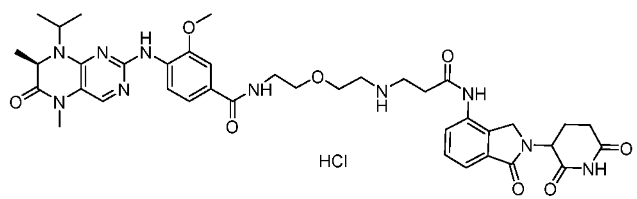 HCl<br>$^1$H NMR (400 MHz, *d6-DMSO*) δ 11.02(s, 1H), 10.24 (s, 1H), 9.13(s,2H), 8.79 (d, *J* = 13.3 Hz, 1H), 7.99 (t, *J* = 2.4 Hz, 1H), 7.90 (s, 1H), 7.88 - 7.78 (m, 1H), 7.71(s 1H), 7.68 - 7.64 (m, 1H), 7.52-7.17 (m, 2H), 5.15 (dd, *J* = 13.2, 5.1 Hz, 1H), 4.58 - 4.36 (m, 4H), 3.92 (s, 3H), 3.77 - 3.49 (m, 6H), 3.39 - 3.30 (m, 7H), 2.96 -2.87(m, 3H), 2.67-2.53(m, 2H), 2.37 - 2.32 (m, 1H), 2.04-1.99 (m, 1H), 1.46 - 1.21 (m, 9H).LCMS $[M+H]^+$ =785.4 |
| **UB-180698 (198)** | 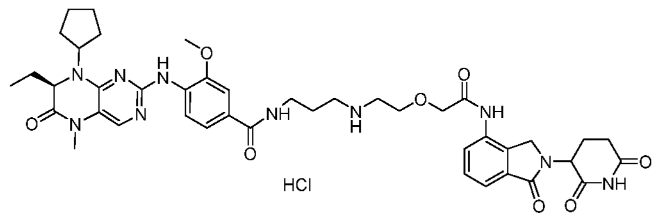 HCl<br>$^1$H NMR (400 MHz, DMSO-$d_6$) δ 11.00 (s, 1H), 10.12 (s, 1H), 9.18 (s, 2H), 8.84 (s, 1H), 7.90 (s, 1H), 7.84 (s, 1H), 7.74 (d, *J* = 7.8 Hz, 1H), 7.64 (s, 1H), 7.60 - 7.44 (m, 3H), 5.14 (dd, *J* = 13.3, 5.1 Hz, 1H), 4.58 - 4.32 (m, 3H), 4.26 - 4.10 (m, 3H), 3.90 (s, 3H), 3.83 (t, *J* = 5.0 Hz, 2H), 3.41 - 3.37 (m, 3H), 3.22 (s, 3H), 3.02 (s, 2H), 2.97 - 2.83 (m, 1H), 2.69 - 2.62 (m, 1H), 2.39 - 2.33 (m, 1H), 2.04 - 1.73 (m, 10H), 1.51 (m, 4H), 0.75 (t, *J* = 7.4 Hz, 3H). LC-MS: $(M+H)^+$= 824.31 |

(continued)

| Examples | Structure and data analysis |
|---|---|
| **UB-180699 (199)** | 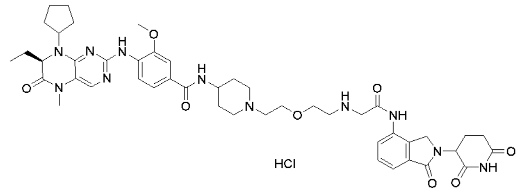 HCl |
| | 1H NMR (400 MHz, DMSO-d6) δ 11.04 (s, 1H), 10.69 (s, 1H), 10.44 (s, 1H), 9.35 (s, 2H), 8.53 (d, J = 7.1 Hz, 1H), 7.87 (t, J = 5.5 Hz, 2H), 7.82 (s, 2H), 7.61 - 7.50 (m, 4H), 5.32 (t, J = 4.8 Hz, 1H), 5.20 - 5.15 (m, 1H), 4.51 - 4.34 (m, 4H), 4.22 (m, 2H), 3.92 (s, 3H), 3.82 (m, 2H), 3.75 (m, 2H), 3.59 (m, 2H), 3.24 (s, 3H), 2.92 (m,2H), 2.65 (m, 2H), 2.33-1.83 (m, 11H), 1.59-1.48 (m, 6H), 0.87-0.84 (t, J = 6.6 Hz, 2H), 0.74-0.78 (t, J = 7.4 Hz, 3H). LCMS $[M+H]^+$ = 956 |
| **UB-180700 (200)** | 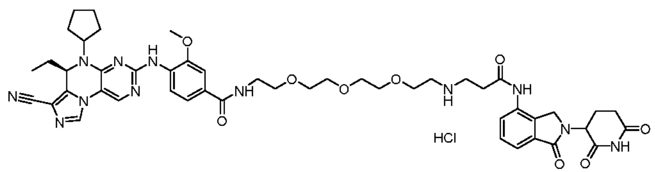 HCl |
| | $^1$H NMR (400 MHz, *DMSO-d6*) δ 11.03 (s, 1H), 10.23 (s, 1H), 8.90 (s, 2H), 8.77 (d, J = 1.7 Hz, 1H), 8.68 (s, 1H), 8.59 (t, J = 5.5 Hz, 1H), 7.98 (dd, J = 19.3, 6.6Hz, 1H), 7.85 (dd, J = 7.2,1.8 Hz, 1H), 7.61 - 7.43 (m, 4H), 5.31 (t, J = 5.8 Hz, 1H), 5.16 (dd, J = 13.3, 5.1 Hz, 1H), 4.53 - 4.39 (m, 1H), 4.39 - 4.28 (m, 2H), 3.91(d, J = 1.9 Hz, 3H), 3.70 (t, J = 5.2 Hz, 4H), 3.43 (q, J = 5.9 Hz, 6H), 3.24 (d, J = 7.3 Hz, 2H), 3.14 (s, 3H), 2.90 (q, J = 7.3, 6.2 Hz, 2H), 2.62 (d, J = 17.1 Hz, 1H),2.28 (dd, J = 11.7, 5.3 Hz, 1H), 2.05 - 1.78 (m, 8H), 1.56 (d, J = 45.4 Hz, 4H), 1.27 (d, J = 20.9 Hz, 2H), 0.73 (t, J = 7.3 Hz, 3H).LCMS $[M+H]^+$ = 947.62 |
| **UB-180701 (201)** | 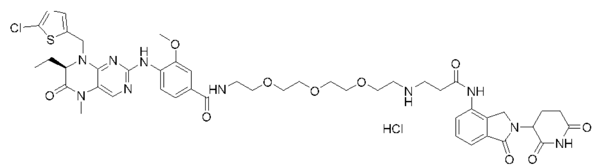 HCl |
| | $^1$H NMR (400 MHz, *DMSO-d6*) δ 11.03 (s, 1H), 10.23 (s, 1H), 9.34 (s, 1H), 8.89 (s, 2H), 8.58 (t, J = 5.7 Hz, 1H), 8.09 (d, J = 8.4 Hz, 1H), 7.91 (s, 1H), 7.85 (dd, J = 7.2, 1.8 Hz, 1H), 7.60 (d, J = 1.9 Hz, 1H), 7.50 (dddd, J = 11.5, 9.7, 6.7, 2.1 Hz, 3H), 7.11 (d, J = 3.8 Hz, 1H), 6.98 (d, J = 3.8 Hz, 1H), 5.23 - 5.08 (m, 2H), 4.80 (d, J = 15.5 Hz, 1H), 4.50 (t, J = 4.6 Hz, 1H), 4.48 - 4.33 (m, 2H), 3.94 (s, 3H), 3.70 (t, J = 5.1 Hz, 2H), 3.56 (s, 5H), 3.22 (s, 5H), 3.17 - 3.12 (m, 3H), 2.91 (dt, J = 14.4, 6.2 Hz, 3H), 2.62 (d, J = 17.2 Hz, 2H), 2.28 (dd, *J* = 11.9, 5.3 Hz, 2H), 2.10 - 1.83 (m, 4H), 1.30 - 1.18 (m, 2H), 0.67 (t, J = 7.4Hz, 3H). LCMS $[M+H]^+$ = 975.47 |

(continued)

| Examples | Structure and data analysis |
| --- | --- |
| **UB-180702 (202)** | 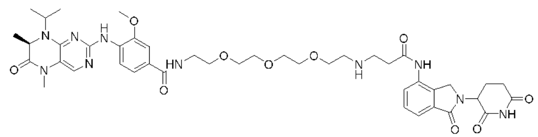 |
| | $^1$H NMR (400 MHz, *DMSO-d6*) δ 11.03 (s, 1H), 10.32 - 10.18 (m, 1H), 9.57 (s, 1H), 8.98 (s, 2H), 8.63 (s, 1H), 8.04 (d, J = 8.3 Hz, 1H), 7.91 (d, J = 3.6 Hz, 1H), 7.85 (dd, J = 7.2, 1.9 Hz, 1H), 7.62 (d, J = 2.0 Hz, 1H), 7.56 (dd, J = 8.4, 1.8 Hz, 1H), 7.53 - 7.46 (m, 2H), 5.15 (dd, J = 13.3, 5.1 Hz, 1H), 4.58 - 4.31 (m,5H), 3.93 (s, 3H), 3.72 (d, J = 5.1 Hz, 2H), 3.56 (d, J = 8.6 Hz, 7H), 3.20 (s, 5H), 3.13 (s, 3H), 2.93 (dp, J = 13.6, 5.5 Hz, 4H), 2.62 (d, J = 17.6 Hz, 1H), 2.31(dd, J = 14.8, 10.5 Hz, 1H), 2.09 - 1.94 (m, 2H), 1.41 (d, J = 6.8 Hz, 3H), 1.35 (t, J = 7.2 Hz, 6H).LCMS $[M+H]^+$ = 873.54 |
| **UB-180703 (203)** | 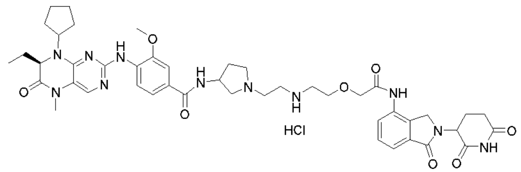 |
| | $^1$H NMR (400 MHz, DMSO-$d_6$) δ 11.44 (s, 1H), 11.00 (s, 1H), 10.13 (d, *J* = 10.0 Hz, 1H), 9.68 (s, 2H), 8.87 (d, *J* = 6.8 Hz, 1H), 7.95 - 7.83 (m, 2H), 7.79 - 7.67 (m, 2H), 7.62 (d, *J* = 8.3 Hz, 1H), 7.57 (d, *J* = 7.4 Hz, 1H), 7.51 (d, *J* = 7.6 Hz, 1H), 5.14 (dd, *J* = 13.3, 5.1 Hz, 1H), 4.59 - 4.31 (m, 3H), 4.22 (s, 2H), 4.18 (d, *J* = 8.9 Hz, 1H), 3.93 (d, *J* = 4.3 Hz, 3H), 3.86 (t, *J* = 4.8 Hz, 3H), 3.74 (s, 2H), 3.48 (s, 6H), 3.29 (s, 3H), 3.22 (s, 3H), 2.91 (ddd, *J* = 18.0, 13.5, 5.3 Hz, 1H), 2.67 - 2.56 (m, 1H), 2.43 - 2.29 (m, 1H), 2.07 - 1.70 (m, 7H), 1.59 - 1.40 (m, 4H), 0.76 (t, *J* = 7.4 Hz, 3H). LCMS [M+H]+ =879.99 |
| **UB-180705 (205)** | 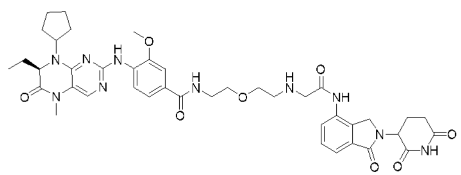 |
| | $^1$H NMR (400 MHz, DMSO-d6) δ 11.01 (s, 1H), 8.44 (t, J = 5.7 Hz, 1H), 8.40 (d, J = 8.3 Hz, 1H), 7.87 (dd, J = 7.1, 1.9 Hz, 1H), 7.83 (s, 1H), 7.58 (s, 1H), 7.54 - 7.46 (m, 4H), 5.14 (dd, J = 13.3, 5.1 Hz, 1H), 4.48 - 4.28 (m, 3H), 4.24 (dd, J = 7.6, 3.6 Hz, 1H), 3.92 (s, 3H), 3.57 (dt, J = 12.5, 5.5 Hz, 4H), 3.51 (s, 2H), 3.44 (q, J = 5.8 Hz, 2H), 3.25 (s, 3H), 2.97 - 2.81 (m, 3H), 2.66 - 2.57 (m, 1H), 2.01 (dd, J = 7.2, 4.7 Hz, 2H), 1.93 - 1.52 (m, 10H), 0.76 (t, J = 7.4 Hz, 3H). LCMS [M+H]+ =811.0 |

(continued)

| Examples | Structure and data analysis |
| --- | --- |
| **UB-180706 (206)** | 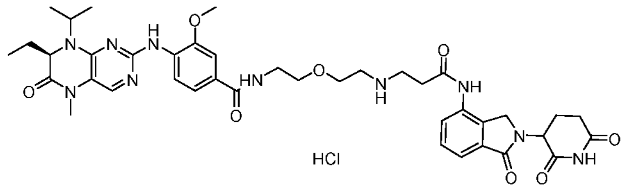<br>$^1$HNMR (400 MHz, *d6-DMSO*) δ 11.02 (s, 1H), 10.18 (s, 1H), 8.98 (s, 2H), 8.73(s,1H),7.99 (t, *J* = 2.4 Hz, 1H), 7.84 (s, 2H), 7.82(s, 1H), 7.60 (d, *J* = 3.3 Hz, 1H), 7.51 - 7.47 (m, 2H), 5.15 (dd, *J* = 13.2, 5.1 Hz, 1H), 4.52 - 4.39 (m, 4H), 3.94 (s, 3H), 3.75 - 3.73 (m, 6H), 3.39 - 3.30 (m, 7H), 2.96 - 2.81 (m, 3H), 2.67 - 2.58 (m, 2H), 2.39-2.20 (m, 1H), 2.04-1.78 (m, 3H),1.36-1.35(m,6H),0.77-0.74(m,3H). LCMS $[M+H]^+$ =800.4 |
| **UB-180707 (207)** | 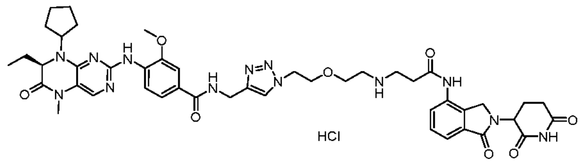<br>$^1$H NMR (400 MHz, *DMSO-d6*) δ 11.02 (s, 1H), 10.28 (s, 1H), 9.73 (s, 1H), 9.16 (d, J = 5.5 Hz, 1H), 9.06 (s, 2H), 8.09 (d, J = 2.3 Hz, 1H), 7.91 - 7.81 (m, 3H),7.67 (d, J = 2.0 Hz, 1H), 7.59 (dd, J = 8.3, 1.8 Hz, 1H), 7.50 (d, J = 7.4 Hz, 2H), 5.15 (dd, J = 13.3, 5.2 Hz, 1H), 4.67 - 4.47 (m, 6H), 4.47 - 4.33 (m, 2H), 4.17 (p,J = 8.9 Hz, 2H), 3.86 (s, 1H), 3.74 (s, 1H), 3.21 (s, 5H), 3.12 (t, J = 5.4 Hz, 2H), 2.92 (ddt, J = 13.2, 9.5, 5.1 Hz, 3H), 2.63 (d, J = 3.9 Hz, 1H), 2.35 - 2.25 (m,1H), 2.13 - 1.68 (m, 8H), 1.59 - 1.40 (m, 4H), 0.75 (t, J = 7.4 Hz, 3H).LCMS $[M+H]^+$ = 906.27 |
| **UB-180708 (208)** | 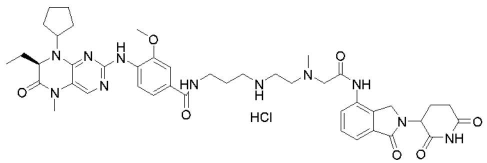<br>$^1$H NMR (400 MHz, Methanol-$d_4$) δ 7.83 (dd, *J* = 8.4, 2.9 Hz, 1H), 7.78 (d, *J* = 8.0 Hz, 1H), 7.67 - 7.58 (m, 3H), 7.58 - 7.45 (m, 2H), 5.16 (dd, *J* = 13.3, 5.2 Hz, 1H), 4.58 - 4.43 (m, 3H), 4.39 - 4.20 (m, 3H), 3.95 (s, 3H), 3.61 (d, *J* = 33.8 Hz, 6H), 3.23 (q, *J* = 7.5, 7.1 Hz, 3H), 3.06 (s, 3H), 2.89 (ddd, *J* = 18.5, 13.5, 5.5 Hz, 1H), 2.75 (d, *J* = 17.1 Hz, 1H), 2.49 (dd, *J* = 13.1, 4.7 Hz, 1H), 2.20 - 1.81 (m, 10H), 1.74 - 1.50 (m, 4H), 1.37 (dd, *J* = 6.7, 3.8 Hz, 1H), 0.86 (t, *J* = 7.5 Hz, 3H).LCMS [M+H]+ =839.4 |

(continued)

| Examples | Structure and data analysis |
|---|---|
| UB-180709 (209) | 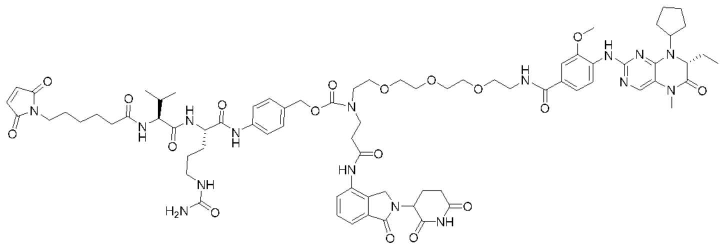 |
| | $^1$H NMR (400 MHz, DMSO-$d_6$) δ 11.02 (s, 1H), 9.98 (s, 1H), 9.87 (s, 1H), 8.53 (d, *J* = 6.0 Hz, 1H), 8.07 (d, *J* = 7.4 Hz, 1H), 7.90 (d, *J* = 8.5 Hz, 1H), 7.83 - 7.71 (m, 3H), 7.64 - 7.44 (m, 6H), 7.28 (d, *J* = 8.3 Hz, 2H), 7.00 (s, 2H), 6.05 - 5.92 (m, 1H), 5.14 (dd, *J* = 13.3, 5.1 Hz, 1H), 4.99 (s, 2H), 4.51 - 4.41 (m, 1H), 4.40 - 4.30 (m, 4H), 4.23 - 4.11 (m, 4H), 3.90 (s, 3H), 3.51 - 3.41 (m, 9H), 3.41- 3.30 (m, 6H), 3.08 - 2.85 (m, 3H), 2.72 - 2.58 (m, 4H), 2.38 - 2.28 (m, 4H), 2.21 - 2.07 (m, 3H), 1.95 (dt, *J* = 13.2, 6.8 Hz, 4H), 1.79 (dd, *J* = 14.8, 7.4 Hz, 4H), 1.57 - 1.39 (m, 10H), 1.19 (q, *J* = 7.6 Hz, 2H), 0.87 - 0.79 (m, 6H), 0.75 (t, *J* = 7.4 Hz, 3H). LCMS [M+H]+=1512 |
| UB-180710 (210) | 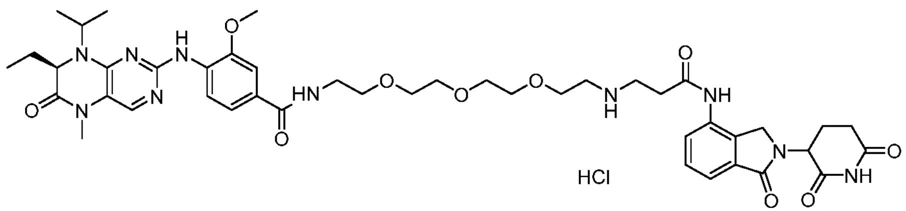 HCl |
| | $^1$H NMR (400 MHz, *d6-DMSO*) δ 11.02 (s, 1H), 10.37 (s, 1H), 9.85(s,1H),9.19(s,2H),8.72 (d, *J* = 13.3 Hz, 1H), 7.99 (t, *J* = 2.4 Hz, 1H), 7.90 (s, 1H), 7.88 - 7.78 (m, 1H), 7.66 (d, *J* = 3.3 Hz, 2H), 7.61 - 7.47 (m, 4H), 5.15 (dd, *J* = 13.2, 5.1 Hz, 1H), 4.68 - 4.36 (m, 4H), 3.93 (s, 3H), 3.92-3.73(m,2H), 3.66 - 3.51 (m, 9H), 3.49 - 3.40 (m, 2H), 3.30 - 3.11 (m, 7H), 2.96 - 2.81 (m, 43H), 2.53-2.51(m,1H)2.47 - 2.36 (m, 1H), 2.19 - 1.71(m, 3H), 1.46 - 1.21 (m, 6H), 0.77-0.74 (m,3H). LCMS $[M+H]^+$ =887.3 |

(continued)

| Examples | Structure and data analysis |
|---|---|
| **UB-180711 (211)** | 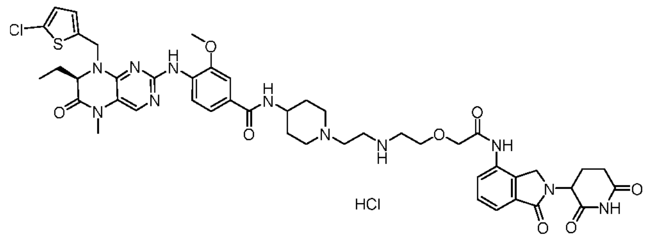 HCl |
| | $^1$H NMR (400 MHz, DMSO-$d_6$) δ 11.01 (s, 1H), 10.66 (s, 1H), 10.01 (s, 1H), 9.49 (m, 2H), 8.51 (m, 1H), 8.16 (m, 1H), 7.90 (s, 1H), 7.74 (d, J = 7.7 Hz, 1H), 7.61 - 7.50 (m, 4H), 7.12 (d, J = 3.9 Hz, 1H), 6.99 (d, J = 3.8 Hz, 1H), 5.19 - 5.10 (m, 2H), 4.79 (m, 1H), 4.53 - 4.33 (m, 4H), 4.23 (s, 2H), 4.07 (m, 1H), 3.96 (s, 3H), 3.86 (m, 2H), 3.66 (m, 3H), 3.23 (m, 4H), 3.15 (m, 3H), 2.98 - 2.87 (m, 2H), 2.10 - 1.83 (m, 10H), 0.67 (t, J = 7.4 Hz, 3H).LCMS $[M+H]^+$ =956.3 |
| **UB-180712 (212)** | 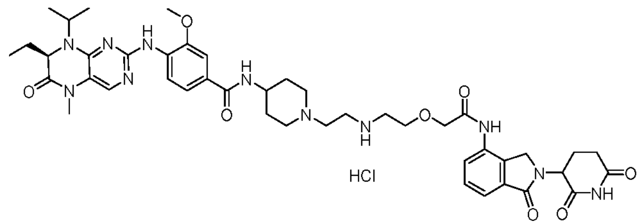 HCl |
| | $^1$H NMR (400 MHz, DMSO-$d_6$) δ 11.01 (s, 1H), 10.74 (s, 1H), 10.04 (s, 1H), 9.56 (m, 2H), 8.55 (d, *J* = 7.5 Hz, 1H), 8.08 (d, *J* = 8.4 Hz, 1H), 7.87 (s, 1H), 7.75 (dd, *J* = 7.9, 1.2 Hz, 1H), 7.63 - 7.49 (m, 4H), 5.15 (dd, *J* = 13.3, 5.1 Hz, 1H), 4.55 - 4.48 (m, 2H), 4.46 - 4.34 (m, 2H), 4.23 (s, 2H), 4.09 (s, 1H), 3.94 (s, 3H), 3.87 (m, 2H), 3.67 (m, 2H), 3.51 (m, 6H), 3.22 (s, 3H), 3.15 (s, 2H), 2.68 - 2.61 (m, 1H), 2.39 - 2.32 (m, 1H), 2.01 (m, 7H), 1.81 (m, 1H), 1.37 (t, *J* = 7.2 Hz, 6H), 0.77 (t, *J* = 7.4 Hz, 3H).LCMS $[M+H]^+$ =868.3 |
| **UB-180713 (213)** | 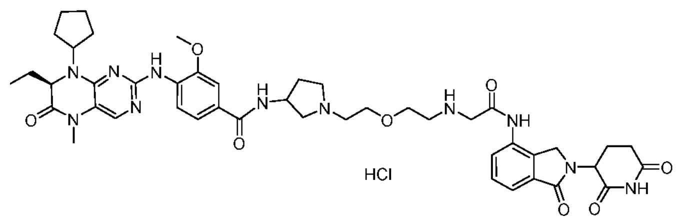 HCl |
| | $^1$H NMR (400 MHz, DMSO-$d_6$) δ 11.04 (s, 1H), 10.83 (d, *J* = 12.4 Hz, 1H), 10.77 - 10.58 (m, 1H), 9.54 - 9.27 (m, 2H), 9.21 - 8.86 (m, 1H), 8.22 - 8.00 (m, 1H), 7.96 - 7.78 (m, 2H), 7.76 - 7.47 (m, 4H), 5.24 - 5.09 (m, 1H), 4.83 - 4.63 (m, 1H), 4.55 - 4.46 (m, 1H), 4.44 - 4.34 (m, 2H), 4.28 - 4.19 (m, 1H), 4.16 - 4.07 (m, 2H), 3.97 - 3.89 (m, 3H), 3.84 - 3.76 (m, 4H), 3.62 - 3.54 (m, 6H), 3.53 - 3.44 (m, 6H), 3.23 (s, 3H), 2.99 - 2.90 (m, 1H), 2.66 - 2.60 (m, 1H), 2.32 - 2.20 (m, 1H), 2.10 - 2.02 (m, 1H), 1.98 - 1.92 (m, 1H), 1.90 - 1.77 (m, 3H), 1.68 - 1.58 (m, 2H), 1.57 - 1.45 (m, 2H), 0.82 - 0.69 (m, 3H). LCMS $[M+H]^+$ =880.35. |

(continued)

| Examples | Structure and data analysis |
|---|---|
| **UB-180714 (214)** | 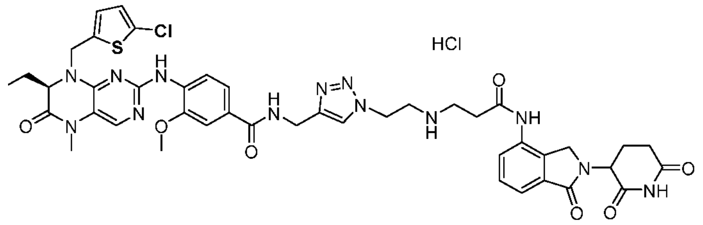 HCl |
| | $^1$H NMR (400 MHz, DMSO-$d_6$) δ 11.03 (s, 1H), 10.25 (s, 1H), 9.25 (s, 2H), 9.09 (t, J = 5.7 Hz, 1H), 8.09 (d, J = 9.3 Hz, 2H), 7.92 (s, 1H), 7.84 (dd, J =7.3, 1.7 Hz, 1H), 7.65 (d, J = 1.9 Hz, 1H), 7.59 - 7.47 (m, 3H), 7.11 (d, J = 3.8 Hz, 1H), 6.98 (d, J = 3.8 Hz, 1H), 5.22 - 5.08 (m, 2H), 4.88 - 4.67 (m,3H), 4.55 - 4.32 (m, 5H), 3.94 (s, 3H), 3.52 - 3.48 (m, 2H), 3.22 (s, 5H), 2.93 - 2.86 (m, 2H), 2.62 (d, J = 14.8 Hz, 1H), 2.39 - 2.27 (m, 1H), 2.04 (dd, J= 9.1, 4.1 Hz, 1H), 1.98 - 1.83 (m, 2H), 0.67 (t, J = 7.4 Hz, 3H).LCMS $[M+H]^+$ =924.2 |
| **UB-180715 (215)** | 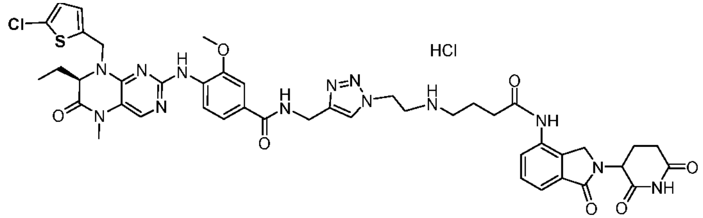 HCl |
| | $^1$H NMR (400 MHz, DMSO-$d_6$) δ 11.02 (s, 1H), 10.02 (s, 1H), 9.07 (d, J = 53.3 Hz, 3H), 8.55 (d, J = 8.3 Hz, 1H), 8.34 (s, 1H), 8.13 (s, 1H), 7.84 (d, J = 6.7 Hz,1H), 7.63 - 7.44 (m, 4H), 7.10 (d, J = 3.6 Hz, 1H), 6.98 (d, J = 3.5 Hz, 1H), 5.22 - 5.06 (m, 2H), 4.80 - 4.66 (m, 3H), 4.61 - 4.32 (m, 5H), 3.96 (s, 3H), 3.59 (d,J = 7.8 Hz, 2H), 3.25 (s, 3H), 2.96 (d, J = 26.1 Hz, 4H), 2.66 (d, J = 18.4 Hz, 1H), 2.34 (s, 1H), 1.95 (s, 2H), 1.83 (s, 2H), 0.66 (t, J = 7.2 Hz, 3H). LCMS $[M+H]^+$ =938.2 |
| **UB-180716 (216)** | 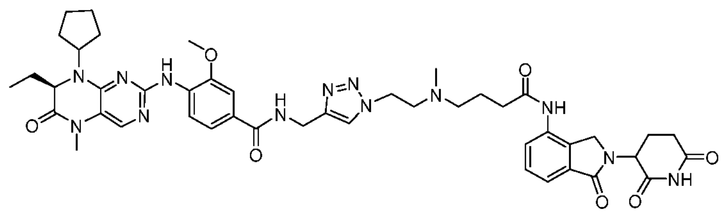 |
| | $^1$H NMR (400 MHz, DMSO-$d_6$) δ 11.03 (s, 1H), 9.93 (s, 1H), 9.73 (s, 1H), 9.07 (t, J = 5.8 Hz, 1H), 8.83 (s, 1H), 8.08 (d, J = 17.8 Hz, 2H), 7.88 - 7.76 (m,2H), 7.64 - 7.44 (m, 4H), 5.16 (dd, J = 13.3, 5.1 Hz, 1H), 4.80 (t, J = 6.7 Hz, 2H), 4.53 (d, J = 5.7 Hz, 2H), 4.46 - 4.29 (m, 4H), 4.22 (p, J = 8.9 Hz, 2H),3.91 (s, 4H), 3.72 (s, 4H), 3.23 (s, 5H), 2.93 (ddd, J = 17.8, 13.5, 5.4 Hz, 1H), 2.84 (s, 3H), 2.61 (d, J = 17.6 Hz, 1H), 2.37 - 2.25 (m, 1H), 2.11 - 1.68 (m,9H), 1.68 - 1.42 (m, 4H), 0.75 (t, J = 7.4 Hz, 3H). LCMS $[M+H]^+$ =890.4 |

(continued)

| Examples | Structure and data analysis |
|---|---|
| **UB-180717 (217)** | 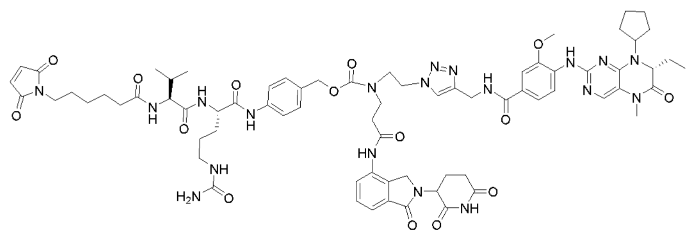 |
| | $^1$H NMR (400 MHz, DMSO-$d_6$) δ 11.02 (s, 1H), 9.98 (s, 1H), 9.87 (s, 1H), 8.53 (d, *J* = 6.0 Hz, 1H), 8.07 (d, *J* = 7.4 Hz, 1H), 7.90 (d, *J* = 8.5 Hz, 1H), 7.83 - 7.71 (m, 3H), 7.64 - 7.44 (m, 6H), 7.28 (d, *J* = 8.3 Hz, 2H), 7.00 (s, 2H), 6.05 - 5.92 (m, 1H), 5.14 (dd, *J* = 13.3, 5.1 Hz, 1H), 4.99 (s, 2H), 4.51 - 4.41 (m, 1H), 4.40 - 4.30 (m, 4H), 4.23 - 4.11 (m, 4H), 3.90 (s, 3H), 3.51 - 3.41 (m, 9H), 3.41- 3.30 (m, 6H), 3.08 - 2.85 (m, 3H), 2.72 - 2.58 (m, 4H), 2.38 - 2.28 (m, 4H), 2.21 - 2.07 (m, 3H), 1.95 (dt, *J* = 13.2, 6.8 Hz, 4H), 1.79 (dd, *J* = 14.8, 7.4 Hz, 4H), 1.57 - 1.39 (m, 10H), 1.19 (q, *J* = 7.6 Hz, 2H), 0.87 - 0.79 (m, 6H), 0.75 (t, *J* = 7.4 Hz, 3H). LCMS $[M+H]^+$ =1512 |
| **UB-180718 (218)** | 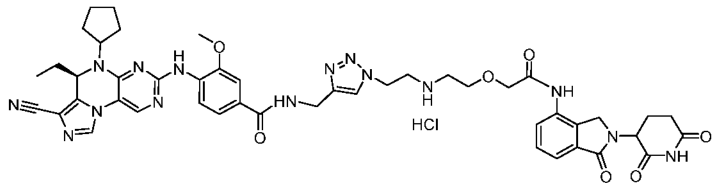 |
| | $^1$H NMR (400 MHz, DMSO-$d_6$) δ 11.00 (s, 1H), 10.11 (s, 1H), 9.43 (s, 2H), 9.09 (t, J = 5.8 Hz, 2H), 8.78 (s, 1H), 8.68 (s, 1H), 8.11 (s, 1H), 7.99 (d, J =8.4 Hz, 1H), 7.75 (d, J = 7.7 Hz, 1H), 7.57 (ddt, J = 26.2, 15.2, 4.7 Hz, 4H), 5.33 (t, J = 5.4 Hz, 1H), 5.14 (dd, J = 13.3, 5.1 Hz, 1H), 4.79 (t, J = 6.6 Hz,2H), 4.57 - 4.30 (m, 6H), 4.20 (s, 2H), 3.90 (s, 3H), 3.83 (d, J = 4.9 Hz, 2H), 3.53 (q, J = 6.4 Hz, 3H), 3.23 (s, 2H), 2.94 - 2.84 (m, 1H), 2.62 (d, J = 3.8Hz, 1H), 2.36 (dd, J = 13.3, 4.6 Hz, 1H), 2.12 - 1.77 (m, 8H), 1.55 (d, J = 43.4 Hz, 5H), 0.74 (t, J = 7.3 Hz, 3H). LCMS $[M+H]^+$ =926.5 |
| **UB-180719 (219)** | 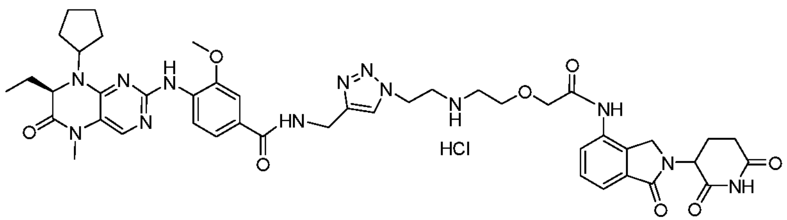 |

(continued)

| Examples | Structure and data analysis |
|---|---|
| | $^1$H NMR (400 MHz, DMSO-$d_6$) δ 11.00 (s, 1H), 10.10 (d, J = 2.3 Hz, 1H), 9.59 (s, 1H), 9.42 (s, 2H), 9.12 (t, J = 5.6 Hz, 1H), 8.11 (s, 1H), 7.88 (d, J = 8.4Hz, 1H), 7.84 (s, 1H), 7.75 (dd, J = 7.8, 1.2 Hz, 1H), 7.65 (d, J = 1.8 Hz, 1H), 7.58 (dt, J = 8.1, 1.7 Hz, 2H), 7.52 (t, J = 7.6 Hz, 1H), 5.14 (dd, J = 13.3, 5.2Hz, 1H), 4.79 (t, J = 6.5 Hz, 2H), 4.52 (dd, J = 11.2, 5.0 Hz, 3H), 4.47 - 4.33 (m, 2H), 4.20 (s, 3H), 3.90 (s, 3H), 3.81 (d, J = 5.1 Hz, 2H), 3.54 (t, J = 6.1 Hz,2H), 3.22 (s, 5H), 2.95 - 2.87 (m, 1H), 2.62 (s, 1H), 2.39 - 2.33 (m, 1H), 2.01 - 1.77 (m, 6H), 1.63 - 1.41 (m, 4H), 0.75 (t, J = 7.4 Hz, 3H).LCMS $[M+H]^+$ =892.3 |
| **UB-180720 (220)** | 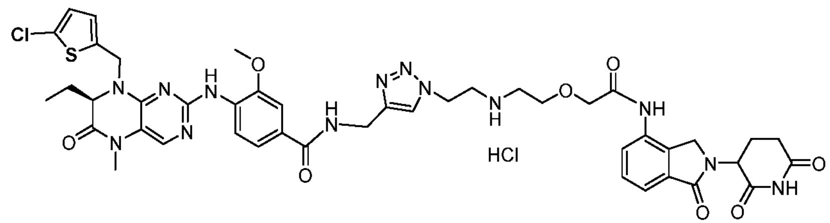 |
| | $^1$H NMR (400 MHz, DMSO-*d6*) δ 11.00 (s, 1H), 10.11 (s, 1H), 9.42 (s, 2H), 9.08 (t, J = 5.7 Hz, 1H), 8.09 (d, J = 7.3 Hz, 2H), 7.92 (s, 1H), 7.75 (dd, J = 7.9,1.2 Hz, 1H), 7.64 (d, J = 1.9 Hz, 1H), 7.60 - 7.49 (m, 3H), 7.11 (d, J = 3.8 Hz, 1H), 6.98 (d, J = 3.8 Hz, 1H), 5.23 - 5.07 (m, 2H), 4.80 (q, J = 7.9, 6.7 Hz, 3H), 4.53 - 4.34 (m, 5H), 4.20 (s, 2H), 3.94 (s, 3H), 3.81 (t, J = 4.8 Hz, 2H), 3.53 (t, J = 6.3 Hz, 2H), 3.22 (s, 5H), 2.91 (ddd, J = 18.2, 13.7, 5.5 Hz, 1H), 2.62 (d, J= 3.7 Hz, 1H), 2.36 (dd, J = 13.1, 4.4 Hz, 1H), 2.08 - 1.85 (m, 3H), 0.67 (t, J = 7.4 Hz, 3H).LCMS $[M+H]^+$ =954.2 |
| **UB-180723 (223)** | 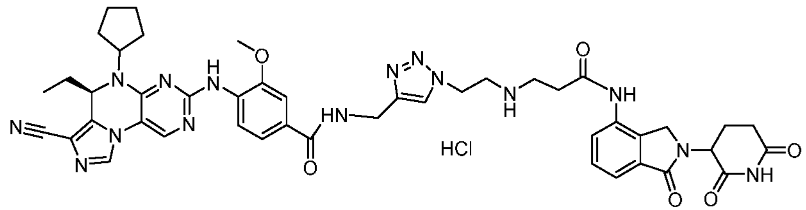 |
| | $^1$H NMR (400 MHz, DMSO-*d6*) δ 11.02 (s, 1H), 10.30 (s, 1H), 9.40 (s, 2H), 9.07 (t, J = 5.5 Hz, 1H), 8.76 (s, 1H), 8.67 (s, 1H), 8.13 (d, J = 10.4 Hz, 2H), 7.84 (dd, J = 7.1, 1.9 Hz, 1H), 7.66 - 7.45 (m, 5H), 5.25 (dd, J = 7.7, 4.0 Hz, 1H), 5.15 (dd, J = 13.3, 5.1 Hz, 1H), 4.76 (t, J = 6.5 Hz, 2H), 4.54 (d, J = 5.5 Hz, 2H), 4.50 - 4.31 (m, 4H), 3.92 (s, 3H), 3.25 (s, 3H), 2.91 (q, J = 8.6, 7.7 Hz, 3H), 2.64 (s, 1H), 2.29 (s, 2H), 2.08 - 1.50 (m, 14H), 0.74 (t, J = 7.3 Hz, 3H).LCMS $[M+H]^+$ =896.2 |

(continued)

| Examples | Structure and data analysis |
|---|---|
| **UB-180725 (225)** | 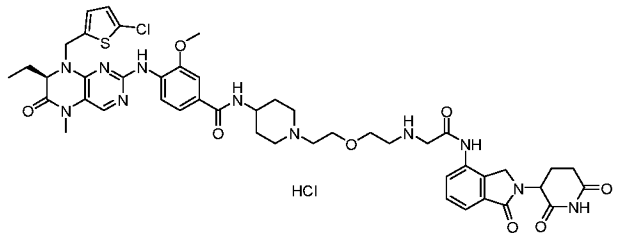 |
| | 1H NMR (400 MHz, DMSO-d6) δ 11.05 (s, 1H), 10.59 (s, 1H), 10.25 (s, 1H), 9.29 (s, 2H), 8.45 (d, J = 7.1 Hz, 1H), 8.27 (s, 1H), 7.87 (d, J = 5.5 Hz, 2H), 7.61 - 7.50 (m, 4H), 7.11 (d, J = 3.7 Hz, 1H), 6.99 (t, J = 3.6 Hz, 1H), 5.32 (t, J = 4.8 Hz, 1H), 5.23 - 5.09 (m, 3H), 4.75 (m, 1H), 4.51 - 4.34 (m, 4H), 4.07 (m, 3H), 3.96 (s, 3H), 3.82 (m, 2H), 3.75 (m, 2H), 3.59 (m, 2H), 3.24 (s, 3H), 2.10 - 1.95 (m, 8H), 1.86 (m, 2H), 1.46 (m, 2H), 0.85 (t, J = 6.6 Hz, 2H), 0.67 (t, J = 7.4 Hz, 3H). LCMS $[M+H]^+$ = 956 |
| **UB-180733 (233)** | 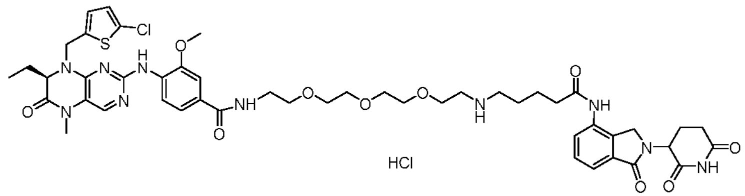 |
| | $^1$H NMR (400 MHz, DMSO-$d_6$) δ 11.02 (s, 1H), 10.02 (s, 1H), 9.79 (s, 1H), 8.89 (m, 2H), 8.64 (t, *J* = 5.6 Hz, 1H), 8.00 (d, *J* = 8.4 Hz, 1H), 7.94 (s, 1H), 7.83 (dd, *J* = 7.1, 2.0 Hz, 1H), 7.63 (d, *J* = 1.9 Hz, 1H), 7.53 - 7.46 (m, 3H), 7.11 (d, *J* = 3.8 Hz, 1H), 6.98 (d, *J* = 3.8 Hz, 1H), 5.20 - 5.10 (m, 2H), 4.83 (m, 1H), 4.54 (m, 1H), 4.48 - 4.33 (m, 2H), 3.94 (s, 3H), 3.69 (m, 3H), 3.54 (m, 10H), 3.42 (m, 3H), 3.21 (s, 3H), 3.07 (m, 2H), 2.93 (m, 3H), 2.42 (t, *J* = 6.8 Hz, 2H), 2.02 (m, 1H), 1.98 - 1.88 (m, 2H), 1.72 - 1.62 (m, 4H), 0.67 (t, *J* = 7.4 Hz, 3H). LCMS [M+H]+ = 1003 |
| **UB-180734 (234)** | 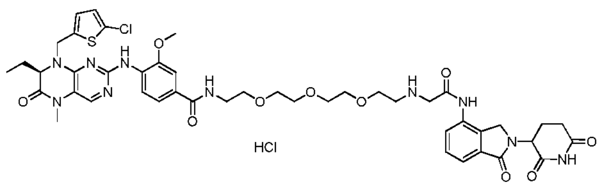 |
| | $^1$H NMR (400 MHz, DMSO-$d_6$) δ 11.05 (s, 1H), 10.81 (s, 1H), 9.89 (s, 1H), 9.21 (s, 2H), 8.65 (t, *J* = 5.6 Hz, 1H), 8.05 - 7.93 (m, 2H), 7.87 (dd, *J* = 7.3, 1.7 Hz, 1H), 7.64 (d, *J* = 1.9 Hz, 1H), 7.61 - 7.44 (m, 3H), 7.11 (d, *J* = 3.8 Hz, 1H), 6.98 (d, *J* = 3.8 Hz, 1H), 5.24 - 5.10 (m, 3H), 4.83 (d, *J* = 15.5 Hz, 1H), 4.61 - 4.24 (m, 4H), 4.06 (t, *J* = 5.5 Hz, 2H), 3.94 (s, 3H), 3.72 (t, *J* = 5.2 Hz, 2H), 3.56 (s, 3H), 3.53 (m, *J* = 4.4 Hz, 5H), 3.41 (q, *J* = 6.4, 6.0 Hz, 2H), 3.21 (m, 5H), 2.99 - 2.85 (m, 1H), 2.70 - 2.57 (m, 1H), 2.35 - 2.23 (m, 1H), 2.14 - 1.98 (m, 1H), 1.97 - 1.85 (m, 2H), 0.67 (t, *J* = 7.4 Hz, 3H). LCMS $[M+H]^+$ =961.1 |

(continued)

| Examples | Structure and data analysis |
|---|---|
| **UB-180745 (245)** | 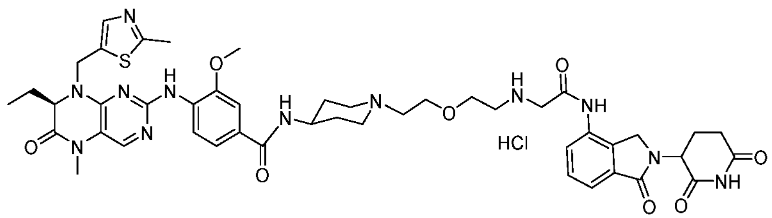 |
| | H NMR (400 MHz, DMSO-$d_6$) δ 11.04 (d, J = 3.2 Hz, 1H), 10.97 (d, J = 5.5 Hz, 1H), 10.63 (s, 1H), 9.83 (s, 1H), 9.48 (s, 2H), 8.60 (dd, J = 18.3, 7.0 Hz,1H), 8.05 (dd, J = 8.5, 4.9 Hz, 1H), 7.98 (s, 1H), 7.88 (dd, J = 7.4, 1.6 Hz, 1H), 7.77 - 7.70 (m, 1H), 7.63 (d, J = 1.8 Hz, 1H), 7.60 - 7.50 (m, 3H), 5.28 -5.12 (m, 3H), 4.85 (d, J = 15.5 Hz, 2H), 4.58 - 4.36 (m, 4H), 4.11 (d, J = 7.1 Hz, 2H), 3.96 (d, J = 8.1 Hz, 3H), 3.84 (s, 2H), 3.78 (t, J = 5.1 Hz, 2H), 3.61 (d,J = 11.5 Hz, 2H), 3.14 (d, J = 11.6 Hz, 2H), 3.01 - 2.85 (m, 1H), 2.64 (s, 1H), 2.36 - 2.27 (m, 1H), 2.18 - 1.92 (m, 7H), 0.69 (t, J = 7.4 Hz, 3H).LCMS [M+H]+ = 937.2 |
| **UB-180746 (246)** | 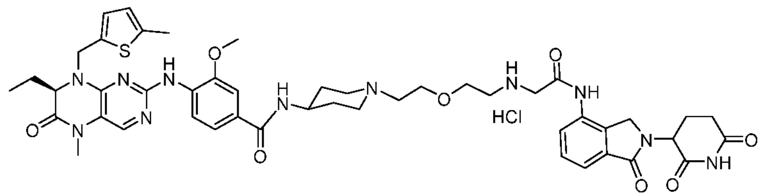 |
| | 1H NMR (400 MHz, DMSO-d6) δ 11.04 (s, 1H), 10.90 (s, 1H), 10.57 (s, 1H), 9.45 (s, 2H), 8.51 (d, J = 7.3 Hz, 1H), 8.23 (d, J = 8.4 Hz, 1H), 7.94 (s, 1H), 7.88(dt, J = 9.1, 4.6 Hz, 1H), 7.63 - 7.51 (m, 4H), 7.00 (d, J = 3.4 Hz, 1H), 6.66 (dd, J = 3.2, 1.3 Hz, 1H), 5.28 (d, J = 15.3 Hz, 1H), 5.16 (dd, J = 13.3, 5.1 Hz, 1H),4.70 (d, J = 15.3 Hz, 1H), 4.57-4.27 (m, 4H), 4.11 (s, 2H), 3.96 (d, J = 6.0 Hz, 3H), 3.84 (d, J = 5.7 Hz, 2H), 3.77 (t, J = 4.9 Hz, 2H), 3.61 (d, J = 11.7 Hz, 2H),3.23 (s, 7H), 3.14 (d, J = 11.5 Hz, 2H), 2.98 - 2.88 (m, 1H), 2.62 (d, J = 17.3 Hz, 1H), 2.35 (s, 3H), 2.30 - 2.24 (m, 1H), 2.15 - 1.86 (m, 7H), 0.70 (t, J = 7.4 Hz,3H).LCMS [M+H]+ = 936.2 |

(continued)

| Examples | Structure and data analysis |
|---|---|
| **UB-180747 (247)** | 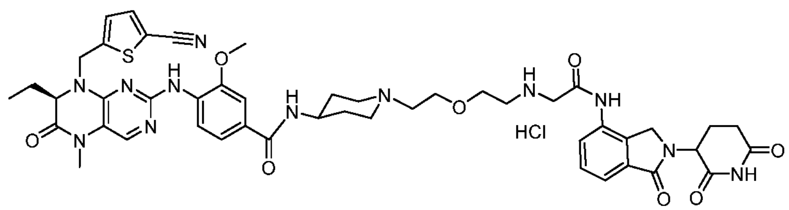 |
| | $^1$H NMR (400 MHz, DMSO-$d_6$) δ 11.04 (s, 1H), 10.85 (s, 1H), 10.55 (s, 1H), 9.44 (s, 2H), 8.44 (d, J = 7.1 Hz, 1H), 8.31 (d, J = 8.5 Hz, 1H), 8.11 (s, 1H),7.89 (d, J = 14.9 Hz, 2H), 7.84 (d, J = 3.8 Hz, 1H), 7.54 (ddt, J = 14.2, 9.3, 6.4 Hz, 4H), 7.38 (d, J = 3.9 Hz, 1H), 5.29 - 5.12 (m, 2H), 4.87 (d, J = 15.7 Hz,1H), 4.56 - 4.38 (m, 3H), 4.10 (s, 2H), 3.95 (s, 4H), 3.84 (s, 2H), 3.78 (d, J = 5.0 Hz, 2H), 3.64 - 3.59 (m, 2H), 3.42 (s, 2H), 3.31 (s, 1H), 3.12 (dd, J = 7.4,4.2 Hz, 3H), 3.00 - 2.87 (m, 2H), 2.62 (d, J = 16.8 Hz, 1H), 2.34 - 2.26 (m, 1H), 2.08 (dd, J = 26.0, 18.0 Hz, 5H), 1.83 (d, J = 7.7 Hz, 2H), 0.66 (t, J = 7.4Hz, 3H).LCMS $[M+H]^+$ = 947.2 |
| **UB-180763 (263)** | 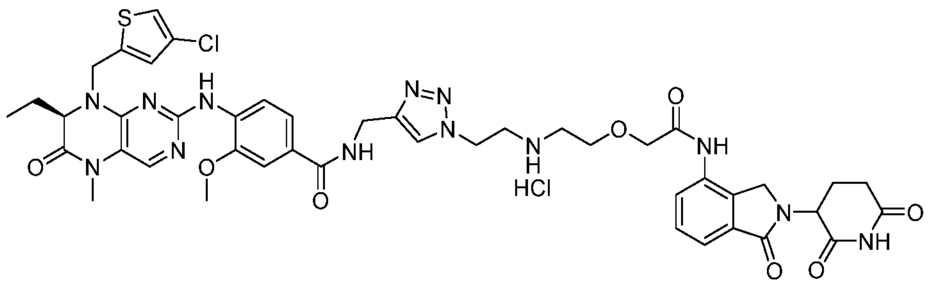 |
| | $^1$H NMR (400 MHz, DMSO-$d_6$) δ 11.00 (s, 1H), 10.08 (s, 1H), 9.37 (s, 2H), 9.05 (s, 1H), 8.10 (s, 2H), 7.92 (s, 1H), 7.75 (dd, J = 7.8, 1.2 Hz, 1H),7.66 - 7.45 (m, 5H), 7.22 (d, J = 1.6 Hz, 1H), 5.29 - 5.10 (m, 2H), 4.85 - 4.74 (m, 3H), 4.56 - 4.33 (m, 5H), 4.20 (s, 2H), 3.93 (s, 3H), 3.81 (t, J = 4.9Hz, 2H), 3.53 (s, 4H), 3.23 (s, 5H), 2.91 (ddd, J = 17.7, 13.5, 5.4 Hz, 1H), 2.62 (s, 1H), 2.41 - 2.30 (m, 1H), 2.07 - 1.97 (m, 1H), 1.90 (d, J = 7.9 Hz,2H), 0.70 (t, J = 7.4 Hz, 3H).LCMS $[M+H]^+$ = 954.2 |

(continued)

| Examples | Structure and data analysis |
|---|---|
| **UB-180764 (264)** | 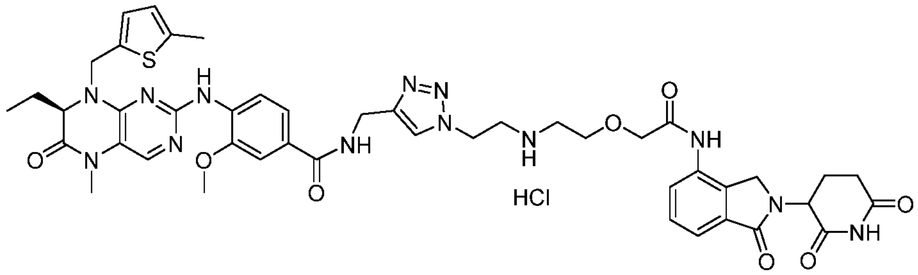<br>$^1$H NMR (400 MHz, DMSO-$d_6$) δ 11.00 (s, 1H), 10.09 (d, J = 3.0 Hz, 1H), 9.39 (s, 2H), 9.07 (s, 1H), 8.14 (s, 1H), 8.10 (s, 1H), 7.93 (s, 1H), 7.75 (dd, J =7.8, 1.2 Hz, 1H), 7.65 - 7.48 (m, 4H), 7.00 (d, J = 3.4 Hz, 1H), 6.66 (dt, J = 3.4, 1.8 Hz, 1H), 5.25 (d, J = 15.3 Hz, 1H), 5.14 (dd, J = 13.3, 5.1 Hz, 1H),4.82 - 4.69 (m, 3H), 4.55 - 4.33 (m, 5H), 4.20 (s, 2H), 3.95 (d, J = 7.2 Hz, 3H), 3.81 (t, J = 4.9 Hz, 2H), 3.53 (s, 4H), 3.22 (s, 5H), 2.91 (ddd, J = 17.6,13.4, 5.3 Hz, 1H), 2.62 (s, 1H), 2.35 (dd, J = 4.1, 1.0 Hz, 4H), 2.07 - 1.98 (m, 1H), 1.90 (d, J = 10.5 Hz, 2H), 0.74 - 0.67 (m, 3H).LCMS $[M+H]^+$ = 934.2 |
| **UB-180765 (265)** | 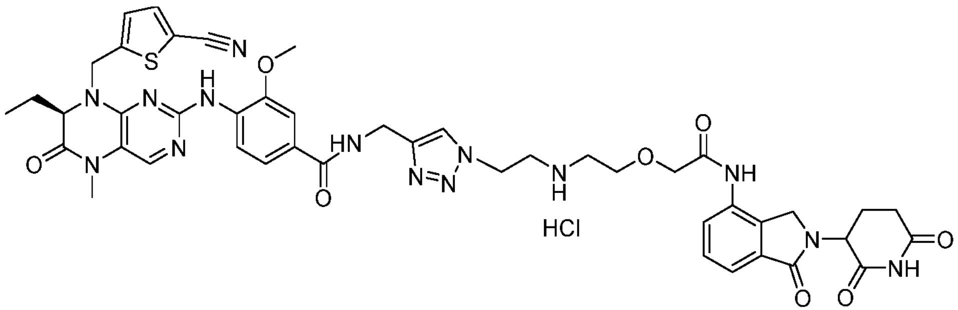<br>$^1$H NMR (400 MHz, DMSO-$d_6$) δ 11.00 (s, 1H), 10.11 (s, 1H), 9.43 (s, 2H), 9.07 (s, 1H), 8.10 (s, 1H), 8.00 (s, 1H), 7.93 (s, 1H), 7.84 (d, J = 3.8 Hz, 1H),7.75 (dd, J = 7.8, 1.2 Hz, 1H), 7.65 - 7.60 (m, 1H), 7.59 - 7.47 (m, 3H), 7.37 (d, J = 3.9 Hz, 1H), 5.24 (d, J = 15.8 Hz, 1H), 5.14 (dd, J = 13.2, 5.1 Hz, 1H),4.95 (d, J = 15.8 Hz, 1H), 4.79 (t, J = 6.6 Hz, 2H), 4.56 (t, J = 4.2 Hz, 1H), 4.54 - 4.49 (m, 2H), 4.49 - 4.34 (m, 2H), 4.20 (s, 2H), 3.93 (d, J = 2.9 Hz, 3H),3.82 (d, J = 4.9 Hz, 2H), 3.23 (s, 5H), 2.91 (ddd, J = 18.3, 13.7, 5.5 Hz, 1H), 2.60 (d, J = 17.5 Hz, 1H), 2.39 - 2.32 (m, 1H), 2.02 (dd, J = 11.0, 5.5 Hz, 1H),1.91 (d, J = 6.3 Hz, 2H), 0.67 (t, J = 7.3 Hz, 3H). LCMS $[M+H]^+$ = 945.2 |

(continued)

| Examples | Structure and data analysis |
| --- | --- |
| **UB-180766 (266)** | 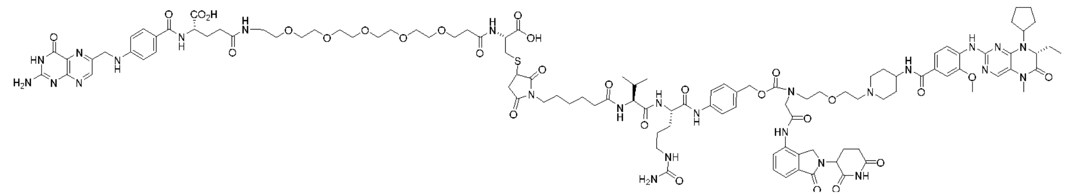 |
| | LCMS $[M/2+H]^+$ = 1165 |
| **UB-180767 (267)** | 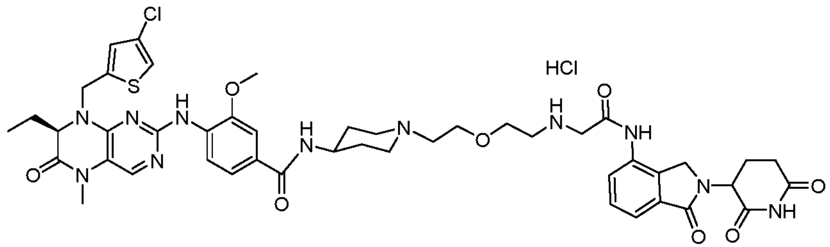 |
| | $^1$H NMR (400 MHz, DMSO-$d_6$) δ 11.03 (d, J = 3.2 Hz, 1H), 10.39 (d, J = 17.5 Hz, 1H), 10.17 (s, 1H), 9.30 (s, 2H), 8.73 (s, 1H), 8.43 (d, J = 7.1 Hz, 1H), 8.30 (d, J = 8.5 Hz, 1H), 7.96 - 7.84 (m, 8H), 7.55 - 7.44 (m, 3H), 7.34 (d, J = 2.2 Hz, 2H), 7.18 (d, J = 8.8 Hz, 2H), 5.15 (dt, J = 13.3, 4.8 Hz, 1H), 4.49 - 4.32 (m, 2H), 4.09 (d, J = 51.8 Hz, 2H), 3.61 (q, J = 10.7, 9.2 Hz, 2H), 3.38 (s, 2H), 3.28 (s, 5H), 3.15 (d, J = 39.6 Hz, 5H), 3.05 - 2.85 (m, 4H), 2.60 (d, J = 17.1 Hz, 1H), 2.37 - 2.15 (m, 3H), 2.01 (d, J = 11.9 Hz, 4H).LCMS [M+H]+ =956.4 |

**(2.9) Preparation procedure 9 of TED molecule:**

**Synthesis method of Compound UB-180811**

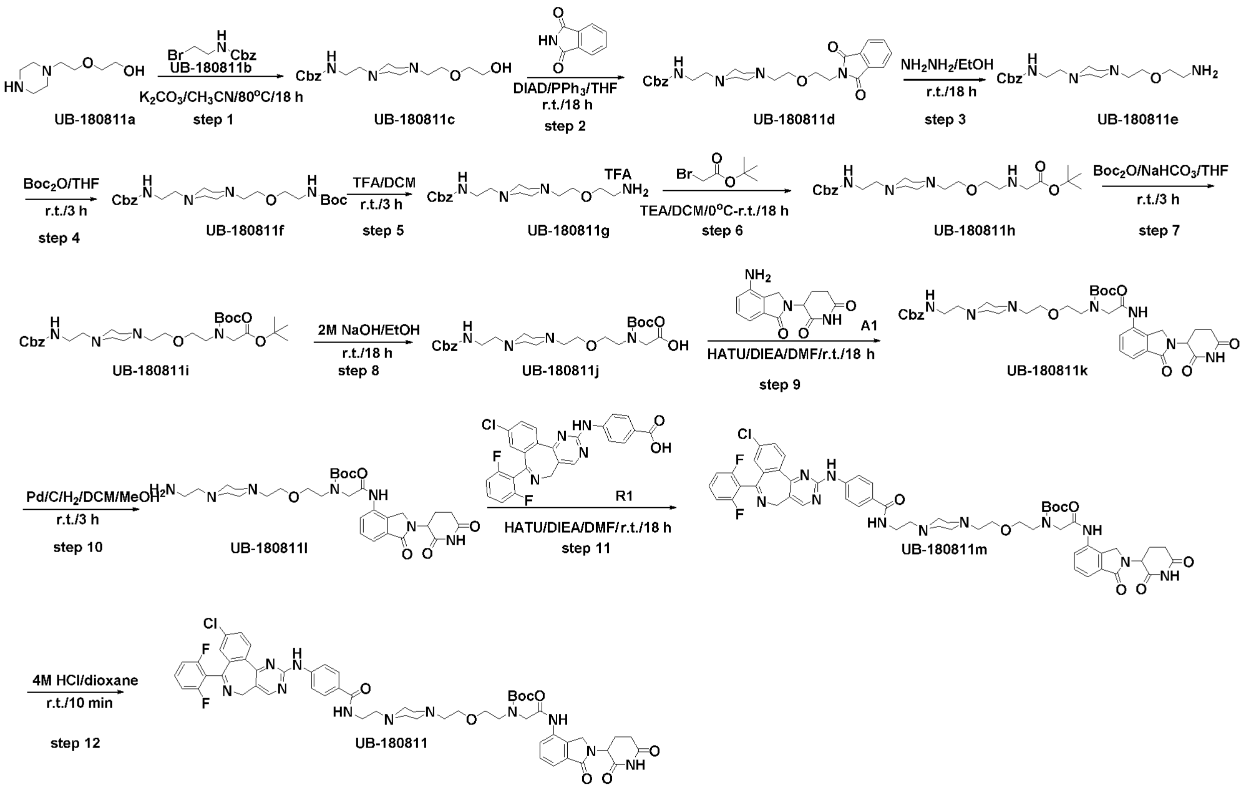


**Step 1: UB-180811c**

Compound **UB-180811a** (3.2 g, 18.2 mmol) was dissolved in acetonitrile (100 mL). Compound **UB-180811b** (4.7 g, 18.2 mmol) was added slowly dropwise, followed by the addition of potassium carbonate (5 g, 36.4 mmol), then reacted at 80 °C for 18 hours. After the completion of reaction, the reaction was filtrated. The filtrate was concentrated to obtain the crude product. The crude product was isolated by silica gel column chromatography (dichloromethane/methanol = 0% to 30%) to obtain target product **UB-180811c** (5.8 g, yield 90%) as colorless transparent oil. LCMS $[M+H]^+ = 352$

**Step 2: UB-180811d**

Compound **UB-180811c** (1.9 g, 5.4 mmol) was dissolved in tetrahydrofuran (40 mL) and di*iso*propyl azodicarboxylate (1.6 g, 8.1 mmol) and triphenylphosphine (2.1 g, 8.1 mmol) dropwise, reacted at room temperature for 2 hours, added the compound phthalimide (1.2 g, 8.1 mmol) and reacted at room temperature over the weekend. After completion of reaction, the reaction solution was concentrated to obtain a crude product, which was isolated by silica gel column chromatography (dichloromethane/methanol=0% to 10%) to obtain the target product **UB-180811d** (3.2 g) as a yellow transparent oil. LCMS $[M+H]^+ = 481$
$^1$H NMR (400 MHz, DMSO-$d_6$) δ 7.57 - 7.51 (m, 4H), 7.43 - 7.23 (m, 5H), 7.11 (m, 1H), 5.01 (d, *J* = 1.9 Hz, 2H), 3.75 (t, *J*= 5.7 Hz, 1H), 3.58 (q, *J* = 7.0, 6.4 Hz, 1H), 3.47 (d, *J*= 5.8 Hz, 4H), 3.14 - 2.97 (m, 2H), 2.47 - 1.92 (m, 12H).

**Step 3: UB-180811e**

Compound **UB-180811d** (1.5 g, 3.1 mmol) was dissolved in ethanol (20 mL) and added hydrazine (580 mg, 11.6 mmol), reacted at room temperature for 18 hours. When the reaction is cooled to room temperature, some solids will precipitate out. The reaction was filtered, the filtrate was dried by rotary evaporation. The crude product was washed repeatedly with ethanol, and the filtrate was dried by rotary dryer to obtain1.2 g crude product **UB-180758e.** LCMS [M+H]

$^{+}$ = 351

**Step 4: UB-180811f**

Compound **UB-180811e** (1 g, 2.8 mmol) was dissolved in tetrahydrofuran (20 mL). Di-*tert*-butyl dicarbonate (2 mL, 8.7 mmol) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-180811f** (580 mg, yield 42%) as a yellow transparent oil. LCMS $[M+H]^{+}$ = 451

**Step 5: UB-180811g**

Compound **UB-180811f** (580 mg, 1.28 mmol) was dissolved in dichloromethane (10 mL) and added trifluoroacetic acid (2 mL), reacted at room temperature for 3 hours. The target compound obtained by concentrating after completion of reaction was directly used in the next step. LCMS $[M+H]^{+}$ = 351

**Step 6: UB-180811h**

Triethylamine (84 mg, 0.84 mmol) was added dropwise to a solution of *tert*-butyl bromoacetate (82 mg, 0.42 mmol) in dichloromethane (10 mL) under ice bath, and then reacted for 10 minutes. Compound **UB -180811g** (150 mg, 0.42 mmol) was dissolved in dichloromethane (2 mL) and added to the above reaction, reacted at room temperature for 18 hours. The reaction was added saturated brine (10 mL) anded extract with dichloromethane (20 mL* 2). The organic phase was concentrated and purified by climbing large plate (dichloromethane/methanol = 10/1) to obtain the target product **UB-180811h** (40 mg, yield 20%) as a yellow transparent oil. LCMS $[M+H]^{+}$ = 465

**Step 7: UB-180811i**

Compound **UBI-180811h**(450 mg, 0.97 mmol) was dissolved in tetrahydrofuran (10 mL), added di-*tert*-butyl dicarbonate (632 mg, 2.9 mmol) and sodium bicarbonate (243 mg, 2.9 mmol), and reacted at room temperature for 3 hours. The reaction solution was concentrated to obtain a crude product, which was isolated by silica gel column chromatography (dichloromethane/methanol=0% to 5%) to obtain the target product **UB-180811i** (500 mg, yoeld 91%) as yellow transparent oil. LCMS $[M+H]^{+}$ = 565
$^{1}$H NMR (400 MHz, DMSO-$d_6$) $\delta$ 7.43 - 7.28 (m, 5H), 7.16 - 7.07 (m, 1H), 5.01 (s, 2H), 3.84 (d, *J* = 6.3 Hz, 2H), 3.45 (dt, *J* = 8.5, 5.8 Hz, 4H), 3.30 (d, *J* = 4.9 Hz, 2H), 3.09 (q, *J* = 6.5 Hz, 2H), 2.48 - 2.19 (m, 12H), 1.45 - 1.32 (m, 18H).

**Step 8: UB-180811j**

Compound **UBI-180811i** (500 mg, 0.88 mmol) was dissolved in ethanol (5 mL), added 2M NaOH (3 mL, 2 mmol), and reacted at room temperature for 18 hours. The reaction solution was concentrated, added water (3 mL), and then extracted with ether (10 mL *3) to remove organic impurities. The aqueous phase was neutralized to pH~6 using 1M HCl and lyophilized to obtain product **UB-180811j** (408 mg) as white solid. LCMS $[M+H]^{+}$ = 509

**Step 9: UB-180811k**

Compound **UBI-180811j** (405 mg, 0.8 mmol), **A1** (208 mg, 0.8 mmol), HATU (365 mg, 0.96 mmol), and DIPEA (0.4 mL) were dissolved in DMF (3 mL), reacted at room temperature for 18 hours. After completion of reaction, the reaction solution was concentrated to obtain a crude product, which was isolated by silica gel column chromatography (dichloromethane/methanol=0% to 20%) to obtain the target product **UB-180811k** (380 mg, yield 54%) as a yellow transparent oil. LCMS $[M+H]^{+}$ = 750

**Step 10: UB-180811l**

Compound **UB-180811k** (380 mg, 0.5mmol) was dissolved in dichloromethane (6 mL) methanol (3 mL), added 10% palladium on carbon (50 mg) and reacted at room temperature for 2 hours under hydrogen. After the completion of reaction, the palladium on carbon was filtered off, and the filtrate was concentrated to obtain light yellow crude product **UB-180811l** (300 mg). LCMS $[M+H]^{+}$ =616

**Step 11: UB-180811m**

Compound **UBI-180811l** (123 mg, 0.2 mmol), **R1** (95 mg, 0.2 mmol), HATU (152 mg, 0.4 mmol), and DIPEA (0.2 mL) were dissolved in DMF (3 mL), reacted at room temperature for 18 hours. The reaction was added saturated brine (10 mL), extracted with dichloromethane (20 mL*2). The organic phase was concentrated, and subjected to Prep-TLC (dichloromethane/methanol = 10/1) to obtain target product **UB-180811m** (100 mg, yield 46%) as yellow solid. LCMS $[M+H]^+$ = 1074

**Step 12: UB-180811**

Compound **UB-180811m** (100 mg) was dissolved in dichloromethane (1.5 mL) and added 4M HCl/dioxane (0.4 mL), reacted at room temperature for 5 minutes. The supernatant was discarded and the solid was washed with ether (10 mL*3), lyophilized to obtain product **UB-180811** (97 mg, yield 100%) as light yellow solid. LCMS $[M+H]^+$ = 974.4 $^1$H NMR (400 MHz, DMSO-$d_6$) $\delta$ 11.05 (s, 1H), 10.95 (s, 1H), 10.23 (s, 1H), 9.45 (s, 2H), 8.77 (m, 2H), 8.30 (d, *J* = 8.5 Hz, 1H), 8.04 - 7.83 (m, 6H), 7.55 (q, *J* = 7.3 Hz, 3H), 7.35 (d, *J* = 2.2 Hz, 1H), 7.18 (t, *J* = 8.8 Hz, 2H), 5.17 (dd, *J* = 13.2, 5.2 Hz, 1H), 4.54 (d, *J* = 17.8 Hz, 1H), 4.41 (d, *J* = 17.7 Hz, 1H), 4.15 (d, *J* = 4.8 Hz, 2H), 3.96 (m, 4H), 3.80 (m, 4H), 3.72 (m, 8H), 3.50 (m, 2H), 3.40 - 3.17 (m, 4H), 2.94 (m, 1H), 2.64 (m, 1H), 2.37 - 2.26 (m, 1H), 2.06 (m, 1H).

**Synthesis method of Compound UB-180849**

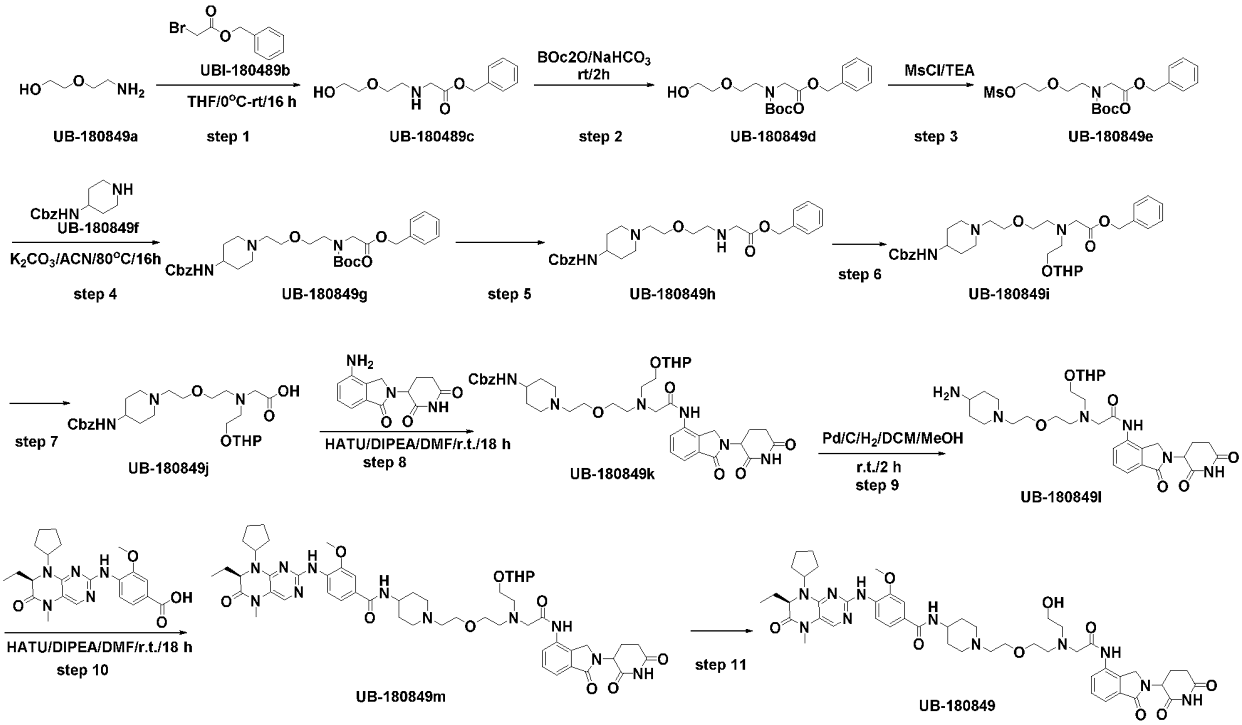


**Step 1: UB-180849c**

Compound **UB-180849b** (19.05g, 66.05mmol) was dissolved in tetrahydrofuran (250 mL), and added dropwise to a solution of Compound **UB-180849a** (22.95g, 218.7mmol) in tetrahydrofuran (550 mL) in ice bath. The reaction was carried out at room temperature for 16 hours. The reaction solution was concentrated and isolated by silica gel column chromatography (methanol/dichloromethane = 0% to 10%) to obtain product **UBI-180849c** (17.4g, yield 63%) as colorless transparent oil. $^1$H NMR (400 MHz, chloroform-d) $\delta$ 7.44 - 7.29 (m, 5H), 5.18 (s, 2H), 3.79 - 3.68 (m, 2H), 3.65 - 3.55 (m, 4H), 3.51 (s, 2H), 2.90 - 2.79 (m, 2H), 1.95 (s, 2H).

**Step 2: UB-180849d**

Compound **UB-180849c** (4.100g, 18.76mmol), (16.50 g, 65.14 mmol), $(Boc)_2O$ (28.43 g, 130.3 mmol) and

$NaHCO_3$ (10.94 g, 130.3 mmol) were dissolved in tetrahydrofuran (600 mL), reacted at room temperature for 2 hours. The reaction was filtered. The filtrate was concentrated and isolated by silica gel column chromatography (dichloromethane/methanol = 0% to 3%) to obtain product **UBI-180849d** (20.7g, yield 90%) as colorless transparent oil.
$^1$H NMR (400 MHz, chloroform-d) δ 7.43 - 7.29 (m, 5H), 5.17 (s, 2H), 4.06 (d, J = 33.2 Hz, 2H), 3.70 - 3.56 (m, 4H), 3.55 - 3.41 (m, 4H), 1.41 (d, J = 43.2 Hz, 9H).

**Step 3: UB-180849e**

Compound **UB-180849d** (119.78 g, 55.97 mmol) was dissolved in dichloromethane (500 mL), added TEA (11.33g, 111.9 mmol) and MsCl (7.692 g, 67.16 mmol) under ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtainproduct **UB-180849f** (22.94g, yield 95%) as yellow oil.
$^1$H NMR (400 MHz, chloroform-d) δ 7.43 - 7.29 (m, 5H), 5.21 - 5.13 (m, 2H), 4.32 - 4.21 (m, 2H), 4.06 (d, J = 33.2 Hz, 2H), 3.66 - 3.56 (m, 4H), 3.53 - 3.43 (m, 2H), 3.01 (s, 3H), 1.41 (d, J = 42.8 Hz, 9H).

**Step 4: UB-180849i**

Compound **UBI-180849h** (12.00g, 44.32mmol), **UBI-180849e** (22.95g, 53.18mmol), and $K_2CO_3$ (15.31g, 110.8mmol) were added to acetonitrile (300 mL) and reacted at 80 °C for 16 hours under stiring. The reaction was filtered. The filtrate was concentrated and then isolated by silica gel column chromatography (methanol/dichloromethane = 0% to 10%) to obtain product **UBI-180849c** (23.4g, yield 93%) as colorless transparent oil.
$^1$H NMR (400 MHz, chloroform-d) δ 7.38 - 7.30 (m, 10H), 5.17 - 5.14 (m, 2H), 5.09 (s, 2H), 4.62 (s, 1H), 4.13 - 4.02 (m, 2H), 3.63 - 3.42 (m, 8H), 2.97 - 2.66 (m, 2H), 2.51 (s, 2H), 2.14 (s, 2H), 2.01 - 1.85 (m, 2H), 1.39 (d, J = 42.4 Hz, 9H).

**Step 5: UB-180849h**

4 mol/L solution of hydrogen chloride in dioxane (2ml) was added dropwise to a solution of UB-180849g (500mg, 0.87mmol) in dichloromethane (20ml), stirred at room temperature for 1 hour. After completion of reaction, the reaction solution was concentrated to obtain compound UB-180849h (350 mg, 85%) as a yellow solid. The product was directly used in the next step without further purification. LCMS $[M+H]^+$ = 470.3

**Step 6: UB-180849i**

2-(2-Bromoethoxy)tetrahydro-2H-pyran (187mg, 0.89 mmol), **UB-180849h** (350mg, 0.75mmol), and potassium carbonate (206 mg, 1.49 mmlc) were dissolved in $CH_3CN$ (10 ml). The reaction solution was refluxed overnight. After completion of reaction, it was diluted with water and extracted with ethyl acetate. The organic layers were combined, dried, filtered, and concentrated. The crude product was isolated by silica gel column chromatography (methanol/dichloromethane=5%) to obtain target product **UB-180849I** (250mg, yield 56%) as yellow oil. LCMS $[M+H]^+$ = 598.4

**Step 7: UB-180849j**

Compound **UB-180849i** (250 mg, 19.66 mmol) was dissolved in tetrahydrofuran (20 mL), added NaOH (20 mmol, 5 mL), and reacted overnight at room temperature under stirring. The reaction solution was added water, concentrated and extracted with ethyl acetate.The pH of the aqueous phase was adjusted to 6 using IN HCl. After the aqueous phase was lyophilized, the solid was dissolved in dichloromethane (200 mL) and methanol (20 mL). It was filtered. The filtrate was concentrated to obtain product **UBI-180849j** (130g, yield 61%) as yellow oil. LCMS $[M+H]^+$ = 342.9; $^1$H NMR (400 MHz, DMSO-$d_6$) δ 7.43 - 7.26 (m, 5H), 5.02 (s, 2H), 4.03 (q, *J* = 7.1 Hz, 1H), 3.87 (d, *J* = 9.8 Hz, 2H), 3.74 - 3.68 (m, 2H), 3.51 (q, *J* = 5.8 Hz, 3H), 3.37 (t, *J* = 5.8 Hz, 10H), 3.13 (s, 2H), 2.99 (s, 2H), 1.99 (s, 2H), 1.91 (s, 2H), 1.71 (d, *J* = 11.7 Hz, 2H), 1.34 (m, 6H).

**Step 8: UB-180849k**

Compound **UBI-180849j** (130mg, 0.26mmol), A1 (66mg, 0.26mmol), and HATU (144mg, 0.39 mmol) were dissolved in DMF (3 mL) and DIPEA (0.3 mL), and reacted at room temperature for 16 hours. The reaction was quenched with water (10 mL), then extracted with ethyl acetate (8 mL*3). The organic phases were combined, then dried over anhydrous sodium sulfate and concentrated to obtain crude product. The crude product was purified by reversed-phase column (methanol/water = 5% to 95%, collected at 70%) to obtain **UBI-180849k** (80mg, yield 62%) as white solid. LCMS

[M+H] + = 749.5

**Step 9: UB-180849l**

Under hydrogen atmosphere, compound **UBI-180849k** (80mg, 0.10mmol), Pd/C (20mg) were dissolved in methanol (1 mL) and dichloromethane (10mL), and the reaction solution was reacted at room temperature for 1 hour. The reaction solution was filtered through Celite. The filtrate was concentrated and purified via reversed-phase column (methanol/water = 5% to 95%, collected at 15%) to obtain the product **UBI-1808491** (35mg, yield 53%) as yellow oil. LCMS $[M+H]^+$ = 615.4

**Step 10: UB-180849m**

Compound **UBI-1808491** (35 mg, 0.06 mmol), P1 (24.2 mg, 0.06 mmol), and HATU (32 mg, 85.4μmol) were dissolved in DMF (2 mL) and DIPEA (0.3 mL), and reacted at room temperature for 18 hours. The reaction was quenched with water (20 mL), then extracted with ethyl acetate (20 mL*3). The organic phases were combined, then dried over anhydrous sodium sulfate and concentrated to obtain crude product. The crude product was isolated by silica gel column chromatography (methanol/dichloromethane=10%) to obtain **UB-180849m** (20 mg, yield 34%) as yellow solid. LCMS $[M+H]^+$ = 1022.6

**Step 11: UB-180849**

A solution of **UB-180849m** (20 mg, 20 μmol) and *p*-toluenesulfonic acid monohydrate (5 mg) in methanol (2 ml) was stirred at room temperature for 6 hours, and added saturated aqueous sodium bicarbonate solution (1 ml), concentrated under reduced pressure to obtain crude product. The crude product was purified by reversed-phase column (methanol/water = 5% to 95%, collected at 65%) to obtain **UB-180849** (3mg, yield 16%) as yellow solid. $^1$H NMR (400 MHz, Methanol-d4) δ 8.48 (d, J = 8.4 Hz, 1H), 7.86 (dd, J = 16.4, 7.8 Hz, 1H), 7.78 (s, 1H), 7.64 (dd, J = 11.9, 7.5 Hz, 1H), 7.57 - 7.50 (m,1H), 7.47 (d, J = 7.8 Hz, 2H), 4.51 (d, J = 9.2 Hz, 2H), 4.28 (dd, J = 7.7, 3.6 Hz, 1H), 3.99 (s, 3H), 3.92 (ddd, J = 17.3, 11.7, 5.4 Hz, 2H), 3.65 (dt, J = 11.0,5.2 Hz, 5H), 3.59 (s, 3H), 3.52 (d, J = 10.9 Hz, 2H), 3.04 (d, J = 12.3 Hz, 2H), 2.88 (ddt, J = 16.5, 11.2, 5.2 Hz, 5H), 2.66 (dt, J = 11.7, 5.6 Hz, 2H), 2.49 (dt, J= 19.8, 6.8 Hz, 2H), 2.26 - 2.10 (m, 4H), 2.00 - 1.80 (m, 8H), 1.78 - 1.67 (m, 4H), 0.86 (t, J = 7.5 Hz, 3H)

**Synthesis method of Compound UB-180828**

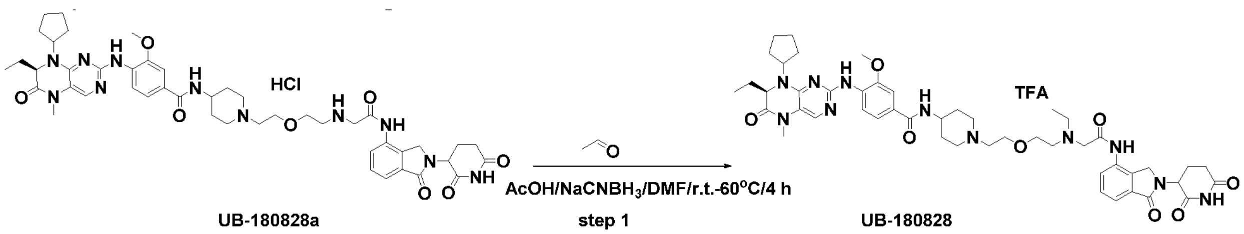


UB-180828a UB-180828

**Step 1: UB-180828**

Compound **UB-180828a** (34 mg, 0.036 mmol), acetaldehyde (0.02 mL, 0.1 mmol), glacial acetic acid (9 mg, 0.072 mmol) were dissolved in DMF (3 mL) and reacted for 1 hour at room temperature. Then $NaBH_3CN$ (6.3 mg, 0.1 mmol) was added and reacted at 60°C for 3 hours. The reaction solution was prepared and purified to obtain product **UB-180828** (15 mg, yield 40%) as white solid. LCMS $[M+H]^+$ = 992
$^1$H NMR (400 MHz, DMSO-$d_6$) δ 11.05 (s, 1H), 10.55 (s, 1H), 9.69 (s, 3H), 8.37 (s, 1H), 8.21 (s, 1H), 7.88 (d, J = 7.6 Hz, 1H), 7.81 (s, 1H), 7.60 (t, J = 5.8 Hz, 2H), 7.51 (d, J = 7.2 Hz, 2H), 5.32 (t, J = 4.8 Hz, 1H), 5.19 (dd, J = 13.2, 5.2 Hz, 1H), 4.47 - 4.32 (m, 4H), 4.27 (d, J = 8.8 Hz, 3H), 4.02 (s, 2H), 3.93 (s, 3H), 3.79 (s, 5H), 3.24 (s, 3H), 2.66 (dt, J = 7.5, 2.5 Hz, 2H), 2.61 (s, 1H), 2.00 (dq, J = 13.1, 7.8, 6.2 Hz, 7H), 1.90 - 1.78 (m, 6H), 1.69 (dd, J = 14.4, 7.1 Hz, 3H), 1.53 (t, J = 6.3 Hz, 2H), 1.46 (d, J = 7.1 Hz, 2H), 0.81 (t, J = 7.0 Hz, 3H).

**Synthesis method of Compound UB-180835**

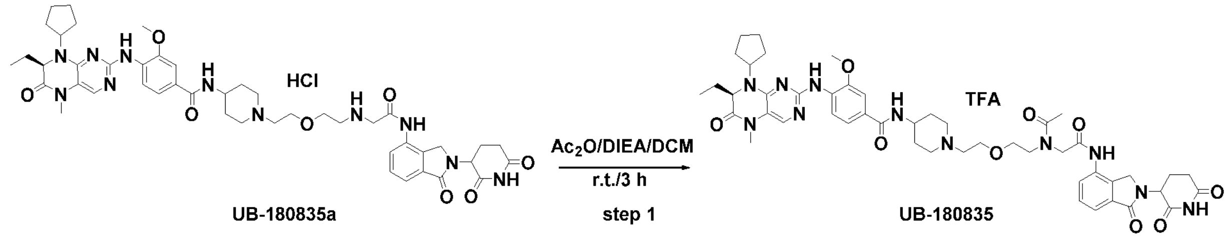


UB-180835a UB-180835

**Step 1: UB-180835**

Compound **UB-180835a** (26 mg, 0.028 mmol), acetic anhydride (1 drop), DIEA (1 drop) were dissolved in dichloromethane (3 mL) and reacted for 3 hours at room temperature. The reaction solution was prepared and purified to obtain product **UB-180835** (10 mg, yield 30%) as white solid. LCMS $[M+H]^+$ = 936
$^1$H NMR (400 MHz, DMSO-$d_6$) δ 11.03 (s, 1H), 10.06 (s, 1H), 9.86 (s, 1H), 9.30 (s, 1H), 8.39 (d, *J* = 7.2 Hz, 1H), 8.13 (s, 1H), 7.87 - 7.78 (m, 2H), 7.53 (m, 4H), 5.16 (m, 1H), 4.38 (m, 3H), 4.28 (m, 2H), 4.15 (s, 2H), 4.01 (m, 2H), 3.93 (s, 3H), 3.79 (m, 2H), 3.73 (m, 2H), 3.62 (m, 2H), 3.55 (m, 2H), 3.24 (s, 3H), 3.13 (m, 2H), 2.92 (m, 2H), 2.67 - 2.59 (m, 2H), 2.33 (m, 1H), 2.12 (s, 2H), 2.02 (m, 4H), 1.98 (m, 2H), 1.83 (m, 5H), 1.64 (m, 2H), 1.52 (m, 2H), 0.76 (t, *J* = 7.4 Hz, 3H).

**Synthesis method of Compound UB-180840**

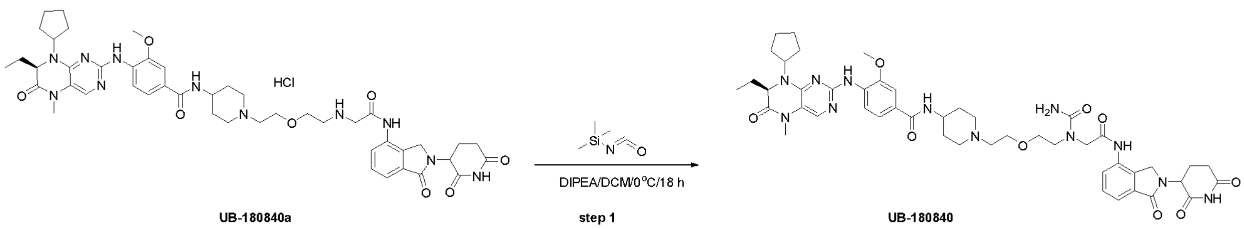


UB-180840a UB-180840

**Step 1: UB-180840**

Compound **UB-180840a** (30 mg, 0.03 mmol), trimethylsilyl isocyanate (7 mg, **0.06** mmol), DIPEA (7 mg) were dissolved in dichloromethane (10 mL) and reacted at room temperature for 18 hours. Reaction solution was prepared and purified to obtain product **UB-180840** (2 mg, yield 7%) as white solid. LCMS [M+H]+ = 937
$^1$H NMR (400 MHz, DMSO-$d_6$) δ 11.03 (s, 1H), 9.88 (s, 1H), 9.60 (s, 1H), 8.76 (s, 1H), 8.43 (d, *J* = 7.3 Hz, 1H), 8.07 (d, *J* = 8.5 Hz, 1H), 7.90 - 7.78 (m, 2H), 7.58 - 7.46 (m, 4H), 6.03 (s, 2H), 5.16 (dd, *J* = 13.3, 5.2 Hz, 1H), 4.46 - 4.34 (m, 3H), 4.32 - 4.19 (m, 2H), 4.11 (s, 2H), 4.07 - 3.96 (m, 2H), 3.93 (s, 3H), 3.78 (t, *J* = 5.0 Hz, 2H), 3.60 - 3.57 (m, 4H), 3.23 (s, 3H), 3.13 (s, 3H), 2.97 - 2.88 (m, 2H), 2.63 (d, *J* = 3.7 Hz, 1H), 2.33 (d, *J* = 3.8 Hz, 1H), 2.02 (ddd, *J* = 18.7, 11.1, 4.6 Hz, 7H), 1.84 (dq, *J* = 10.8, 3.8 Hz, 5H), 1.62 (s, 2H), 1.50 (t, *J* = 7.1 Hz, 2H), 0.76 (t, *J* = 7.4 Hz, 3H).

**Synthesis method of Compound UB-180834(699-CHO)**

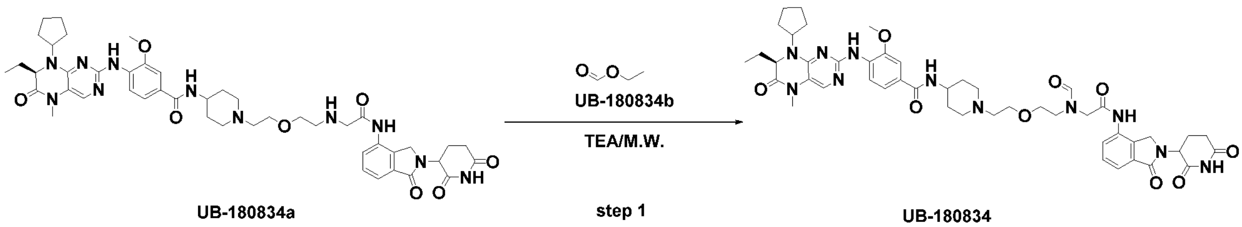


UB-180834a UB-180834

## Step 1: UB-180834

Compound **UB-180834a** (30 mg, 0.03 mmol) was dissolved in **UB-180834b** (5 mL), and then added triethylamine (5 mg, 0.05 mmol). The reaction was heated to 60 °C under microwave and reacted for 1 hour. The reaction solution was concentrated and prepared to obtain target product **UB-180834** (14.2 mg, yield 46%) as white solid.

**Table B11**

| Cmpd. No. | Structure and data analysis |
|---|---|
| **UB-180809 (809)** | 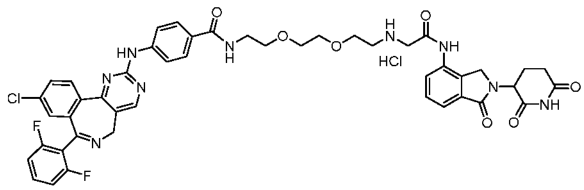 |
| | $^1$H NMR (400 MHz, DMSO-d6) δ 11.05 (s, 1H), 10.71 (s, 1H), 10.17 (s, 1H), 9.15 (s, 2H), 8.73 (s, 1H), 8.43 (t, J = 5.6 Hz, 1H), 8.30 (dd, J = 8.5, 2.7 Hz,1H), 7.94 - 7.81 (m, 7H), 7.61 - 7.49 (m, 3H), 7.34 (d, J = 2.2 Hz, 1H), 7.17 (t, J = 8.8 Hz, 2H), 5.17 (dd, J = 13.3, 5.1 Hz, 1H), 4.52 - 4.33 (m, 2H), 4.06(t, J = 5.6 Hz, 2H), 3.75 (d, J = 5.2 Hz, 2H), 3.56 (d, J = 6.0 Hz, 2H), 3.44 (q, J = 6.5, 6.1 Hz, 2H), 3.23 (q, J = 5.2 Hz, 2H), 3.01 - 2.86 (m, 2H), 2.67 -2.58 (m, 1H), 2.26 (tt, J = 13.2, 6.5 Hz, 1H), 2.10 - 1.99 (m, 1H). LCMS [M+H]$^+$ = 906.5 |
| **UB-180810 (810)** | 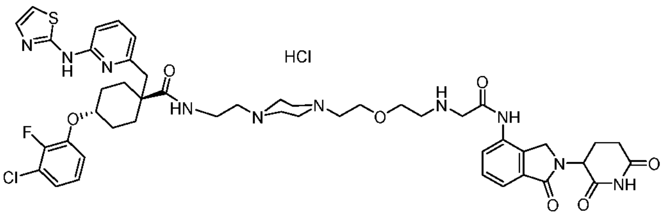 |
| | $^1$H NMR (400 MHz, DMSO-d6) δ 11.92 (s, 1H), 11.05 (s, 1H), 10.72 (s, 1H), 9.16 (s, 2H), 7.87 (dd, J = 7.5, 1.5 Hz, 1H), 7.74 (t, J = 5.5 Hz, 1H), 7.64 (t, J =7.8 Hz, 1H), 7.60 - 7.51 (m, 2H), 7.46 (d, J = 3.8 Hz, 1H), 7.21 - 7.07 (m, 4H), 7.00 (d, J = 8.2 Hz, 1H), 6.75 (d, J = 7.4 Hz, 1H), 5.17 (dd, J = 13.3, 5.2 Hz,1H), 4.54 (s, 1H), 4.50 - 4.33 (m, 2H), 4.06 (s, 2H), 3.59 - 3.52 (m, 5H), 3.37 (t, J = 6.3 Hz, 2H), 3.20 (dd, J = 12.0, 6.2 Hz, 4H), 2.64 (d, J = 3.6 Hz, 1H),2.35 - 2.20 (m, 1H), 2.10 - 2.00 (m, 1H), 1.96 - 1.58 (m, 9H). LCMS [M+H]$^+$ = 891.4 |

(continued)

| Cmpd. No. | Structure and data analysis |
|---|---|
| UB-180811 (811) | 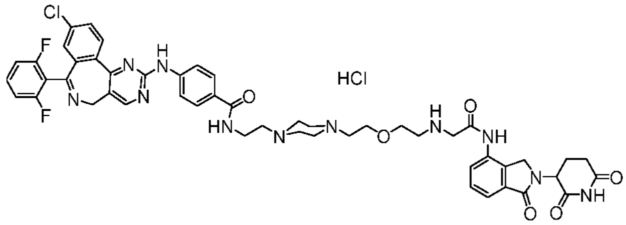 HCl |
| | $^1$H NMR (400 MHz, DMSO-d6) δ 11.05 (s, 1H), 10.95 (s, 1H), 10.23 (s, 1H), 9.45 (s, 2H), 8.77 (m, 2H), 8.30 (d, J = 8.5 Hz, 1H), 8.04 - 7.83 (m, 6H), 7.55 (q, J = 7.3 Hz, 3H), 7.35 (d, J = 2.2 Hz, 1H), 7.18 (t, J = 8.8 Hz, 2H), 5.17 (dd, J = 13.2, 5.2 Hz, 1H), 4.54 (d, J = 17.8 Hz, 1H), 4.41 (d, J = 17.7 Hz, 1H), 4.15 (d, J = 4.8 Hz, 2H), 3.96 (m, 4H), 3.80 (m, 4H), 3.72 (m, 8H), 3.50 (m, 2H), 3.40 - 3.17 (m, 4H), 2.94 (m, 1H), 2.64 (m, 1H), 2.37 - 2.26 (m, 1H), 2.06 (m, 1H). LCMS $[M+H]^+$ = 974.4 |
| UB-180812 (812) | 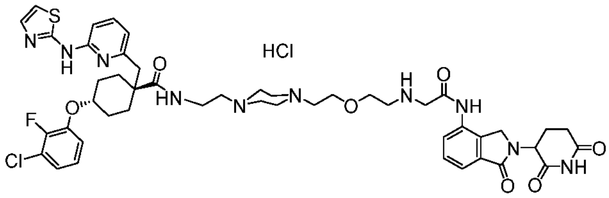 HCl |
| | $^1$H NMR (400 MHz, DMSO-$d_6$) δ 11.04 (s, 1H), 10.91 (s, 1H), 9.43 (s, 1H), 8.12 (s, 1H), 7.98 (d, *J* = 8.4 Hz, 0.5H), 7.88 (d, *J* = 7.3 Hz, 1H), 7.72 (m, 1.5H), 7.60 - 7.39 (m, 4H), 7.20 - 7.05 (m, 5H), 6.82 (d, *J* = 7.4 Hz, 1H), 5.16 (dd, *J* = 13.3, 5.2 Hz, 1H), 4.60 - 4.49 (m, 2H), 4.40 (d, *J* = 17.7 Hz, 1H), 4.13 (s, 2H), 3.82 (m, 12H), 3.24 (m, 8H), 3.00 (s, 2H), 2.96 - 2.89 (m, 1H), 2.68 - 2.60 (m, 1H), 2.36 - 2.26 (m, 1H), 2.10 - 1.96 (m, 3H), 1.86 - 1.59 (m, 6H). LCMS $[M+H]^+$ = 959.4 |
| UB-180813 (813) | 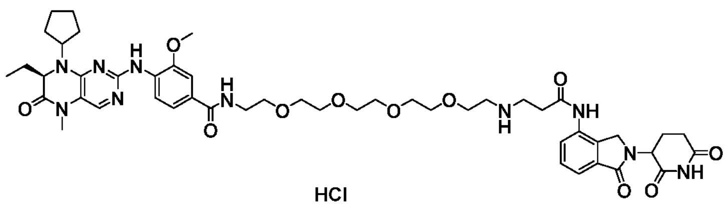 **HCl** |
| | $^1$H NMR (400 MHz, DMSO-$d_6$) δ 11.03 (s, 1H), 10.30 (s, 1H), 9.69 (s, 1H), 9.02 (s, 2H), 8.67 (t, *J* = 5.7 Hz, 1H), 7.85 (t, *J* = 4.1 Hz, 3H), 7.63 (s, 1H), 7.61 - 7.39 (m, 3H), 5.15 (dd, *J* = 13.3, 5.1 Hz, 1H), 4.59 - 4.26 (m, 3H), 4.16 (t, *J* = 8.8 Hz, 1H), 3.90 (s, 3H), 3.72 (t, *J* = 5.2 Hz, 2H), 3.59 - 3.49 (m, 13H), 3.29 - 3.19 (m, 5H), 3.14 (q, *J* = 5.7 Hz,3H), 2.92 (t, *J* = 7.3 Hz, 3H), 2.62 (d, *J* = 17.3 Hz, 1H), 2.33 (td, *J* = 13.3, 8.7 Hz, 1H), 2.10 - 1.76 (m, 7H), 1.60 - 1.37 (m, 4H), 1.29 (dd, J = 13.5, 6.7 Hz, 1H), 0.75 (t, *J* = 7.4 Hz, 3H). LCMS $[M+1]^+$=957.7 |
| UB-180814 (814) | 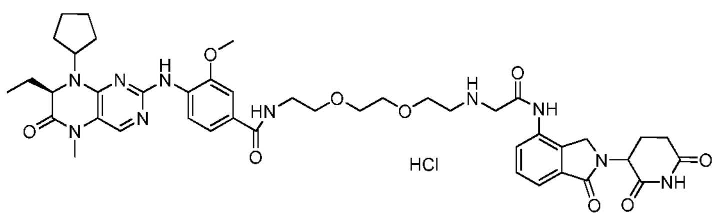 HCl |
| | $^1$H NMR (400 MHz, DMSO-$d_6$) δ 11.04 (s, 1H), 10.85 (s, 1H), 9.72 (s, 1H), 9.23 (d, *J* = 7.8 Hz, 2H), 8.74 (d, *J* = 5.8 Hz, 1H), 8.01 - 7.76 (m, 3H), 7.65 (d, *J* = 1.8 Hz, 1H), 7.62 - 7.41 (m, 3H), 5.16 (dd, *J* = 13.3, 5.1 Hz, 1H), 4.56 - 4.32 (m, 3H), 4.15 (t, *J* = 8.8 Hz, 1H), 4.07 (t, *J* = 5.1 Hz, 2H), 3.90 (s, 3H), 3.75 (t, *J* = 5.1 Hz, 2H), 3.59 (d, *J* = 11.7 Hz, 5H), 3.22 (m, 4H), 2.94 (ddd, *J* = 17.8, 13.4, 5.3 Hz, 1H), 2.67 - 2.58 (m, 1H), 2.28 (qd, *J* = 13.2, 4.5 Hz, 1H), 2.16 - 1.67 (m, 7H), 1.60 - 1.38 (m, 4H), 1.39 - 1.26 (m, 3H), 0.75 (t, *J* = 7.4 Hz, 3H). LCMS $[M+1]^+$ =855.6 |

(continued)

| Cmpd. No. | Structure and data analysis |
|---|---|
| UB-180815 (815) | 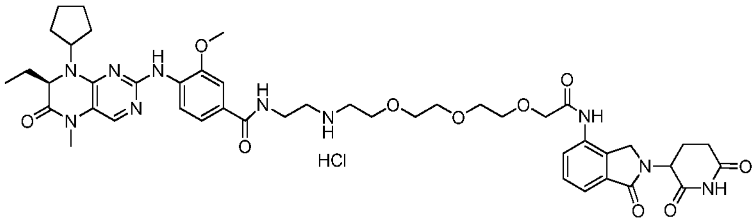 |
| | $^1$H NMR (400 MHz, DMSO-$d_6$) δ 11.01 (s, 1H), 9.78 (s, 1H), 8.94 (d, *J* = 28.3 Hz, 3H), 7.98 (d, *J* = 7.3 Hz, 1H), 7.84 (s, 1H), 7.75 (dd, *J* = 7.7, 1.3 Hz, 1H), 7.69 (d, *J* = 1.9 Hz, 1H), 7.63 - 7.47 (m, 3H), 5.15 (dd, *J* = 13.3, 5.2 Hz, 1H), 4.52 - 4.28 (m, 3H), 4.21 (t, *J* = 8.3 Hz, 1H), 4.15 (s, 2H), 3.93 (s, 3H), 3.69 (dd, *J* = 5.9, 3.5 Hz, 3H), 3.61 (d, *J* = 11.1 Hz, 8H), 3.22 (s, 3H), 3.16 (q, *J* = 7.1, 6.1 Hz, 4H), 2.62 (dd, *J* = 20.8, 16.9 Hz, 1H), 2.38 - 2.30 (m, 1H), 2.07 - 1.71 (m, 8H), 1.67 - 1.36 (m, 4H), 1.36 - 1.26 (m, 1H), 0.75 (t, *J* = 7.4 Hz, 3H). LCMS $[M+1]^+$ =899.7 |
| UB-180820 (820) | 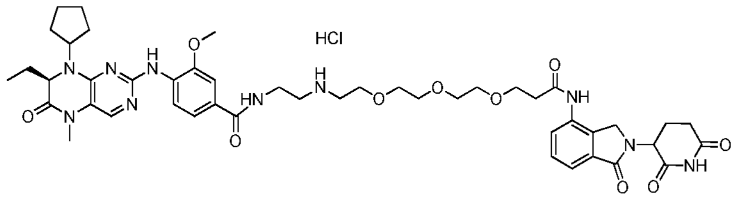 $^1$H NMR (400 MHz, DMSO-$d_6$) δ 11.03 (s, 1H), 10.04 (s, 1H), 9.78 (s, 1H), 9.18 (s, 2H), 9.05 (t, *J* = 5.6 Hz, 1H), 7.88 (t, *J* = 4.2 Hz, 2H), 7.83 (dd, *J* = 6.8, 2.2 Hz, 1H), 7.76 (s, 1H), 7.63 (d, *J* = 8.3 Hz, 1H), 7.50 (d, *J* = 7.0 Hz, 2H), 5.14 (dd, *J* = 13.2, 5.1 Hz, 1H), 4.50 (dd, *J* = 6.4, 3.2 Hz, 1H), 4.47 - 4.27 (m, 2H), 4.17 (p, *J* = 8.8 Hz, 1H), 3.93 (s, 3H), 3.71 (t, *J* = 6.4 Hz, 6H), 3.54 (d, *J* = 8.2 Hz, 10H), 3.22 (s, 3H), 3.15 (q, *J* = 5.4 Hz, 4H), 2.93 (ddd, *J* = 18.1, 13.5, 5.2 Hz, 1H), 2.74 - 2.56 (m, 3H), 2.33 (qd, *J* = 13.3, 4.7 Hz, 1H), 2.15 - 1.88 (m, 4H), 1.83 (tt, *J* = 14.2, 6.6 Hz, 3H), 1.48 (dtt, *J* = 19.3, 8.1, 4.2 Hz, 4H), 0.75 (t, *J* = 7.4 Hz, 3H). LCMS $[M/2+1]^+$ =457.3 |
| UB-180821 (821) | 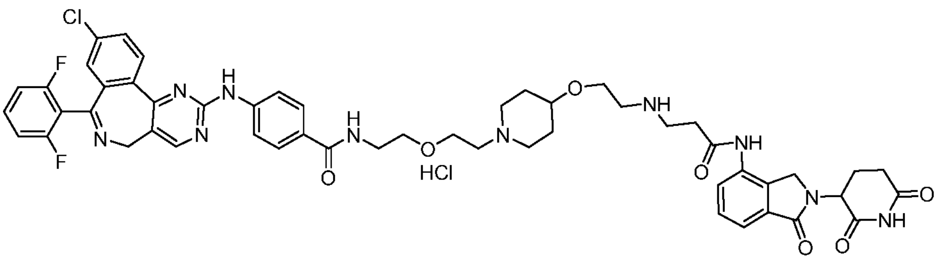 |
| | $^1$H NMR (400 MHz, DMSO-$d_6$)δ 11.03 (s, 1H), 10.33 - 10.12 (m, 2H), 9.14 (s, 1H), 8.73 (s, 1H), 8.41 (d, *J* = 8.9 Hz, 1H), 8.28 (s, 1H), 7.94 - 7.67 (m, 6H), 7.58 - 7.45(m, 3H),7.33 (s, 1H), 7.35 - 7.12 (m, 2H), 5.16 (dd, *J* = 13.1, 5.4 Hz, 1H), 4.45 (d, *J* = 17.4 Hz, 2H), 3.79 (s, 2H), 3.76 - 3.54 (m, 4H), 3.52 - 3.46 (m, 3H), 3.16 (s, 4H), 3.01 (s, 2H), 3.08 - 2.71 (m, 4H), 2.61-2.53 (m,, 2H), 2.32 (d, *J* = 11.7 Hz, 2H), 2.03 -1.96 (m, 2H). LCMS $[M+H]^+$ =1003.5 |

(continued)

| Cmpd. No. | Structure and data analysis |
|---|---|
| UB-180822 (822) | 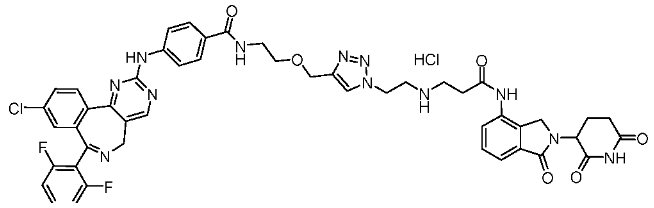 |
| | $^1$H NMR (400 MHz, DMSO-d6) δ 11.03 (s, 1H), 10.28 (s, 1H), 10.18 (s, 1H), 9.32 (s, 2H), 8.73 (s, 1H), 8.42 (t, J = 5.6 Hz, 1H), 8.30 (d, J = 8.5 Hz, 1H), 8.23(s, 1H), 7.93 - 7.81 (m, 6H), 7.61 - 7.45 (m, 3H), 7.35 (d, J = 2.1 Hz, 1H), 7.17 (t, J = 8.8 Hz, 2H), 5.16 (dd, J = 13.3, 5.1 Hz, 1H), 4.77 (t, J = 6.4 Hz, 2H), 4.59 (s, 2H), 4.50 - 4.33 (m, 2H), 3.60 (t, J = 6.1 Hz, 2H), 3.56 - 3.50 (m, 2H), 3.44 (q, J = 6.0 Hz, 3H), 3.29 - 3.20 (m, 2H), 2.93 (tt, J = 12.6, 5.6 Hz, 4H),2.62 (dd, J = 16.7, 3.7 Hz, 1H), 2.38 - 2.27 (m, 1H), 2.10 - 1.99 (m, 1H). LCMS $[M+H]^+$ = 957.5 |
| UB-180828 (828) | 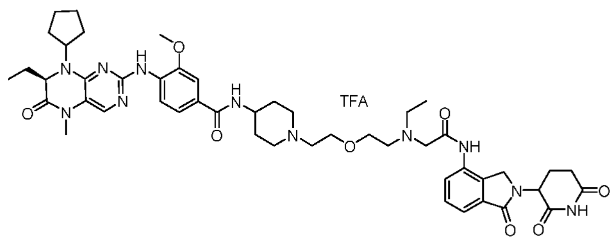 |
| | $^1$H NMR (400 MHz, DMSO-$d_6$) δ 11.05 (s, 1H), 10.55 (s, 1H), 9.69 (s, 3H), 8.37 (s, 1H), 8.21 (s, 1H), 7.88 (d, J = 7.6 Hz, 1H), 7.81 (s, 1H), 7.60 (t, J = 5.8 Hz, 2H), 7.51 (d, J = 7.2 Hz, 2H), 5.32 (t, J = 4.8 Hz, 1H), 5.19 (dd, J = 13.2, 5.2 Hz, 1H), 4.47 - 4.32 (m, 4H), 4.27 (d, J = 8.8 Hz, 3H), 4.02 (s, 2H), 3.93 (s, 3H), 3.79 (s, 5H), 3.24 (s, 3H), 2.66 (dt, J = 7.5, 2.5 Hz, 2H), 2.61 (s, 1H), 2.00 (dq, J = 13.1, 7.8, 6.2 Hz, 7H), 1.90 - 1.78 (m, 6H), 1.69 (dd, J = 14.4, 7.1 Hz, 3H), 1.53 (t, J = 6.3 Hz, 2H), 1.46 (d, J = 7.1 Hz, 2H), 0.81 (dt, J = 36.9, 7.0 Hz, 6H). LCMS $[M+H]^+$ = 992 |
| UB-180833 (833) | 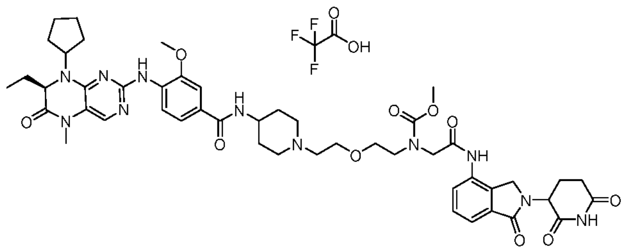<br>$^1$H NMR (400 MHz, DMSO-$d_6$) δ 11.03 (s, 1H), 9.95 (d, J = 9.4 Hz, 1H), 9.47 (d, J = 27.8 Hz, 1H), 9.12 (s, 1H), 8.44 (d, J = 7.2 Hz, 1H), 7.96 (d, J = 8.2 Hz, 1H), 7.84 (t, J = 6.3 Hz, 1H), 7.79 (s, 1H), 7.53 (dd, J = 8.8, 6.7 Hz, 4H), 5.16 (dd, J = 13.3, 5.1 Hz, 1H), 4.43 (m, 1H), 4.40 - 4.31 (m, 2H), 4.17 (m, 4H), 4.05 - 3.98 (m, 2H), 3.92 (s, 3H), 3.76 (m, 4H), 3.64 (m, 2H), 3.58 (m, 1H), 3.52 (m, 2H), 3.28 (m, 2H), 3.23 (s, 3H), 3.14 (m, 2H), 2.97 - 2.88 (m, 1H), 2.63 (m, 1H), 2.38 - 2.29 (m, 1H), 2.08 - 1.91 (m, 5H), 1.88 - 1.75 (m, 5H), 1.61 - 1.42 (m, 4H), 0.76 (t, J = 7.4 Hz, 3H). LCMS $[M+H]^+$ = 952.8 |

(continued)

| Cmpd. No. | Structure and data analysis |
|---|---|
| UB-180834 (834) | 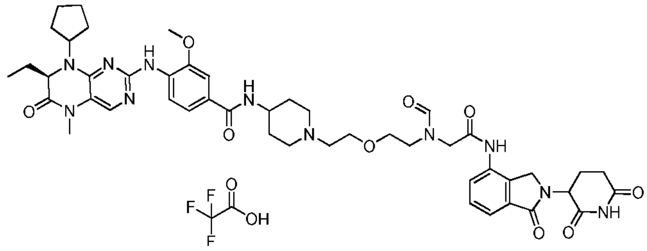 |
| | $^1$H NMR (400 MHz, DMSO-$d_6$) δ 11.03 (s, 1H), 10.12 (s, 1H), 9.99 (s, 1H), 9.35 (s, 1H), 8.40 (d, $J$ = 7.2 Hz, 1H), 8.18 - 8.03 (m, 2H), 7.90 - 7.73 (m, 2H), 7.53 (t, $J$ = 8.1 Hz, 4H), 5.16 (dd, $J$ = 13.2, 4.7 Hz, 1H), 4.40 - 4.35 (m, 2H), 4.28 (s, 1H), 4.18 (s, 1H), 4.00 (s, 1H), 3.93 (s, 3H), 3.76 (dt, $J$ = 12.0, 4.9 Hz, 3H), 3.64 - 3.52 (m, 4H), 3.24 (s, 3H), 3.14 (d, $J$ = 10.9 Hz, 2H), 2.93 (ddd, $J$ = 18.1, 13.5, 5.2 Hz, 2H), 2.63 (t, $J$ = 15.5 Hz, 2H), 2.33 (t, $J$ = 6.6 Hz, 2H), 2.01 (ddd, $J$ = 21.6, 13.0, 6.1 Hz, 5H), 1.92 - 1.76 (m, 4H), 1.64 (s, 2H), 1.52 (d, $J$ = 7.4 Hz, 2H), 0.76 (t, $J$ = 7.4 Hz, 3H). LCMS $[M+1]^+$=922.8 |
| UB-180835 (835) | 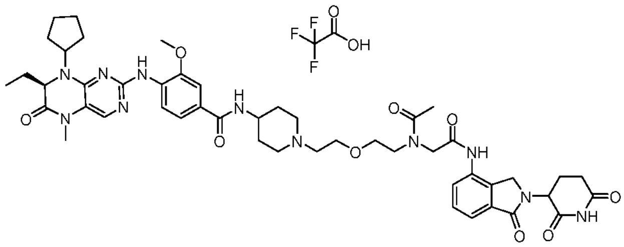 |
| | $^1$H NMR (400 MHz, DMSO-$d_6$) δ 11.03 (s, 1H), 10.06 (s, 1H), 9.86 (s, 1H), 9.30 (s, 1H), 8.39 (d, $J$ = 7.2 Hz, 1H), 8.13 (s, 1H), 7.87 - 7.78 (m, 2H), 7.53 (m, 4H), 5.16 (m, 1H), 4.38 (m, 3H), 4.28 (m, 2H), 4.15 (s, 2H), 4.01 (m, 2H), 3.93 (s, 3H), 3.79 (m, 2H), 3.73 (m, 2H), 3.62 (m, 2H), 3.55 (m, 2H), 3.24 (s, 3H), 3.13 (m, 2H), 2.92 (m, 2H), 2.67 - 2.59 (m, 2H), 2.33 (m, 1H), 2.12 (s, 2H), 2.02 (m, 4H), 1.98 (m, 2H), 1.83 (m, 5H), 1.64 (m, 2H), 1.52 (m, 2H), 0.76 (t, $J$ = 7.4 Hz, 3H). LCMS $[M+H]^+$ =936 |
| UB-180836 (836) | 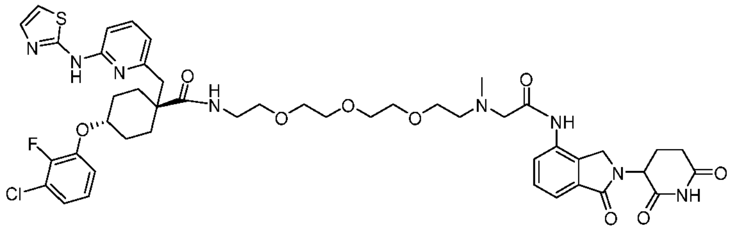 |
| | $^1$H NMR (400 MHz, Methanol-d4) δ 7.86 - 7.79 (m, 1H), 7.64 - 7.59 (m, 1H), 7.54 - 7.46 (m, 2H), 7.29 (dd, J = 3.7, 2.1 Hz, 1H), 7.03 (tt, J = 5.2, 2.2 Hz,3H), 6.88 (dd, J = 3.7, 1.2 Hz, 1H), 6.82 (dd, J = 8.1, 3.9 Hz, 1H), 6.70 - 6.63 (m, 1H), 4.73 - 4.65 (m, 2H), 4.63 - 4.47 (m, 4H), 3.72 (d, J = 0.8 Hz, 1H),3.63 - 3.55 (m, 8H), 3.52 - 3.46 (m, 4H), 3.41 - 3.37 (m, 2H), 3.28 (d, J = 5.6 Hz, 2H), 2.97 (s, 2H), 2.70 (t, J = 5.0 Hz, 2H), 2.37 (d, J = 3.4 Hz, 5H), 2.31 -2.12 (m, 2H), 2.04 - 1.87 (m, 6H), 1.75 - 1.59 (m, 2H). LCMS $[M+H]^+$ = 949.6 |
| UB-180840 (840) | 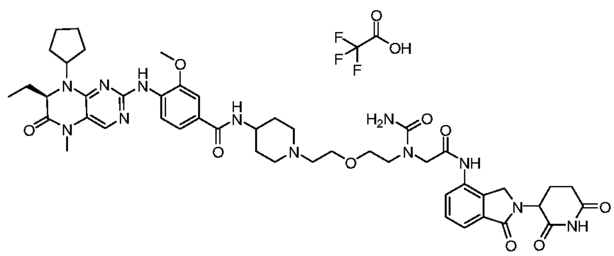 |

(continued)

| Cmpd. No. | Structure and data analysis |
|---|---|
| | $^1$H NMR (400 MHz, DMSO-$d_6$) δ 11.03 (s, 1H), 9.88 (s, 1H), 9.60 (s, 1H), 8.76 (s, 1H), 8.43 (d, *J* = 7.3 Hz, 1H), 8.07 (d, *J* = 8.5 Hz, 1H), 7.90 - 7.78 (m, 2H), 7.58 - 7.46 (m, 4H), 6.03 (s, 2H), 5.16 (dd, *J* = 13.3, 5.2 Hz, 1H), 4.46 - 4.34 (m, 3H), 4.32 - 4.19 (m, 2H), 4.11 (s, 2H), 4.07 - 3.96 (m, 2H), 3.93 (s, 3H), 3.78 (t, *J* = 5.0 Hz, 2H), 3.60 - 3.57 (m, 4H), 3.23 (s, 3H), 3.13 (s, 3H), 2.97 - 2.88 (m, 2H), 2.63 (d, *J* = 3.7 Hz, 1H), 2.33 (d, *J* = 3.8 Hz, 1H), 2.02 (ddd, *J* = 18.7, 11.1, 4.6 Hz, 7H), 1.84 (dq, *J* = 10.8, 3.8 Hz, 5H), 1.62 (s, 2H), 1.50 (t, *J* = 7.1 Hz, 2H), 0.76 (t, *J* = 7.4 Hz, 3H). LCMS $[M+H]^+$ =937 |
| **UB-180844 (844)** | 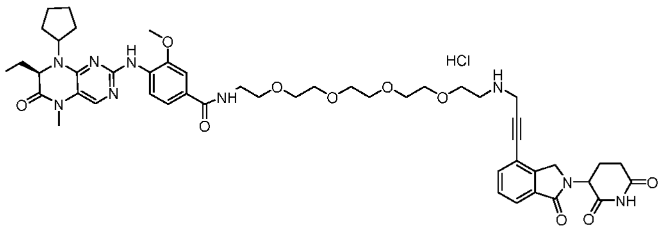 |
| | $^1$H NMR (400 MHz, DMSO-$d_6$) δ 11.05 (s, 1H), 9.59 (m, 3H), 8.63 (t, *J* = 5.5 Hz, 1H), 7.91 - 7.79 (m, 3H), 7.74 (d, *J* = 7.5 Hz, 1H), 7.63 - 7.57 (m, 2H), 7.54 (dd, *J* = 8.3, 1.8 Hz, 1H), 5.18 (dd, *J* = 13.3, 5.1 Hz, 1H), 4.57 (d, *J* = 18.0 Hz, 1H), 4.47 (m, 1H), 4.38 (d, *J* = 17.9 Hz, 1H), 4.19 (m, 3H), 3.90 (s, 3H), 3.75 (t, *J* = 5.1 Hz, 2H), 3.58 (m, 2H), 3.56 (m, 2H), 3.53 (m, 4H), 3.51 (s, 3H), 3.25 (m, 2H), 3.22 (s, 3H), 2.95 (m, 1H), 2.62 (m, 1H), 2.42 - 2.35 (m, 1H), 2.09 - 1.73 (m, 8H), 1.57 - 1.41 (m, 4H), 0.75 (t, *J* = 7.4 Hz, 3H). LCMS $[M+H]^+$ = 924 |
| **UB-180845 (845)** | 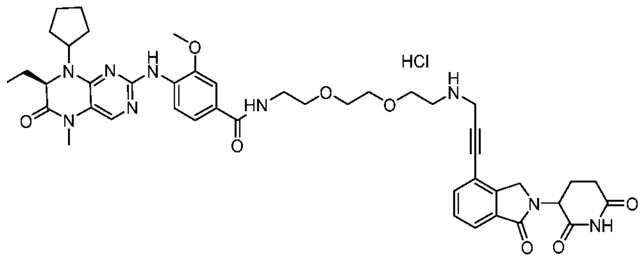 |
| | $^1$H NMR (400 MHz, DMSO-$d_6$) δ 11.05 (s, 1H), 9.37 (m, 3H), 8.59 (m, 1H), 7.97 (m, 1H), 7.85 - 7.71 (m, 3H), 7.64 - 7.49 (m, 3H), 5.18 (dd, *J* = 13.3, 5.1 Hz, 1H), 4.57 - 4.49 (m, 1H), 4.39 (t, *J* = 15.8 Hz, 2H), 4.22 (m, 3H), 3.91 (s, 2H), 3.73 (m, 2H), 3.61 (s, 3H), 3.56 (m, 2H), 3.47 - 3.43 (m, 5H), 3.23 (s, 3H), 2.94 (m, 1H), 2.63 (m, 1H), 2.39 (m, 1H), 2.07 - 1.79 (m, 8H), 1.62 - 1.45 (m, 4H), 0.75 (t, *J* = 7.4 Hz, 3H). LCMS $[M+H]^+$ = 836 |
| **UB-180846 (846)** | 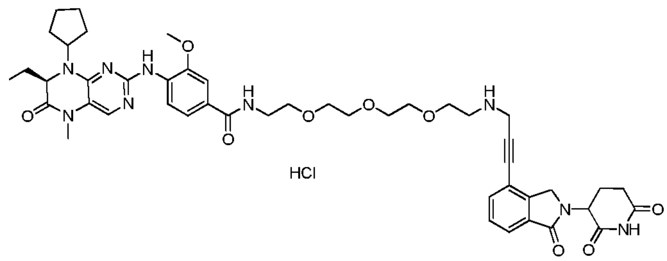 |
| | $^1$H NMR (400 MHz, DMSO-$d_6$) δ 11.04 (s, 1H), 9.79 (s, 2H), 9.51 (s, 1H), 8.68 (d, *J* = 5.6 Hz, 1H), 7.91 (d, *J* = 8.4 Hz, 1H), 7.87 (s, 1H), 7.81 (d, *J* = 7.6 Hz, 1H), 7.74 (d, *J* = 7.5 Hz, 1H), 7.65 - 7.42 (m, 3H), 5.17 (dd, *J* = 13.3, 5.1 Hz, 1H), 4.60 (d, *J* = 18.1 Hz, 1H), 4.46 (dd, *J* = 6.6, 3.3 Hz, 1H), 4.38 (d, *J* = 18.1 Hz, 1H), 4.19 (d, *J* = 10.0 Hz, 3H), 3.90 (s, 3H), 3.76 (t, *J* = 5.1 Hz, 2H), 3.56 (d, *J* = 9.0 Hz, 10H), 3.24 (s, 1H), 3.22 (s, 3H), 2.94 (ddd, *J* = 18.0, 13.6, 5.5 Hz, 1H), 2.63 (dd, *J* = 18.4, 14.6 Hz, 2H), 2.42 (dd, *J* = 13.3, 4.5 Hz, 1H), 2.07 - 1.77 (m, 8H), 1.55 - 1.45 (m, 4H), 1.35 - 1.25 (m, 1H), 0.75 (t, *J* = 7.4 Hz, 3H). LCMS $[M+1]^+$ =880.7 |

(continued)

| Cmpd. No. | Structure and data analysis |
|---|---|
| **UB-180847 (847)** | 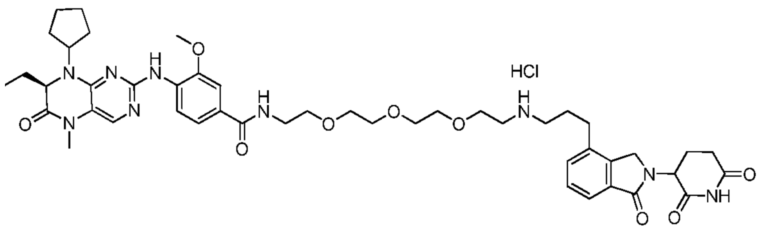<br>$^1H$ NMR (400 MHz, DMSO-$d_6$) δ 11.02 (s, 1H), 9.62 (s, 1H), 9.23 (s, 0H), 8.98 (s, 2H), 8.67 (t, *J* = 5.6 Hz, 1H), 7.92 - 7.81 (m, 2H), 7.67 - 7.57 (m, 2H), 7.55 (dd, *J* = 8.3, 1.8 Hz, 1H), 7.51 - 7.45 (m, 2H), 5.15 (dd, *J* = 13.3, 5.1 Hz, 1H), 4.58 - 4.41 (m, 2H), 4.32 (d, *J* = 17.2 Hz, 1H), 4.17 (t, *J* = 8.8 Hz, 1H), 3.90 (s, 3H), 3.82 - 3.64 (m, 3H), 3.65 - 3.58 (m, 3H), 3.46 - 3.37 (m, 5H), 3.22 (s, 3H), 3.11 (ddt, *J* = 12.3, 9.1, 4.9 Hz, 3H), 2.94 (td, *J* = 16.1, 14.4, 5.3 Hz, 4H), 2.73 (t, *J* = 7.7 Hz, 2H), 2.63 (dd, *J* = 17.4, 13.8 Hz, 2H), 2.45 - 2.26 (m, 1H), 2.11 - 1.70 (m, 9H), 1.56 - 1.43 (m, 4H), 0.75 (t, *J* = 7.4 Hz, 3H). LCMS $[M+1]^+$ =884.7 |
| **UB-180848 (848)** | 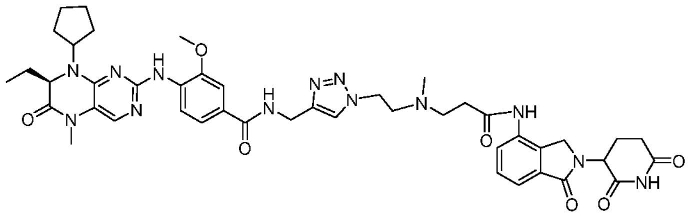<br>$^1H$ NMR (400 MHz, DMSO-d6) δ 11.01 (s, 1H), 9.83 (d, J = 19.2 Hz, 1H), 8.87 (s, 1H), 8.42 (d, J = 8.8 Hz, 1H), 7.95 (d, J = 5.3 Hz, 1H), 7.82 (d, J =13.2 Hz, 2H), 7.60 (s, 1H), 7.53 - 7.38 (m, 4H), 5.21 - 4.73 (m, 1H), 4.42 (s, 2H), 4.33 (d, J = 7.7 Hz, 2H), 4.24 (dd, J = 7.5, 3.5 Hz, 1H), 3.92 (d, J = 4.8Hz, 3H), 2.93 - 2.67 (m, 4H), 2.27 (d, J = 6.0 Hz, 3H), 2.09 - 1.84 (m, 5H), 1.78 (s, 4H), 1.67 - 1.49 (m, 4H), 0.76 (t, J = 7.4 Hz, 3H). LCMS $[M+H]^+$ = 876.9 |
| **UB-180849 (849)** | 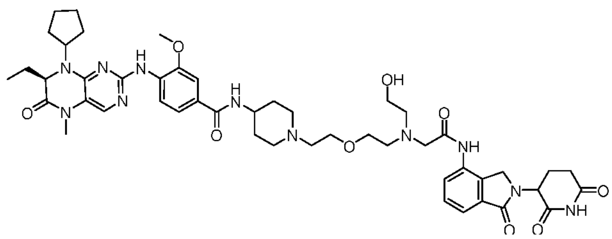<br>$^1H$ NMR (400 MHz, Methanol-d4) δ 8.48 (d, J = 8.4 Hz, 1H), 7.86 (dd, J = 16.4, 7.8 Hz, 1H), 7.78 (s, 1H), 7.64 (dd, J = 11.9, 7.5 Hz, 1H), 7.57 - 7.50 (m,1H), 7.47 (d, J = 7.8 Hz, 2H), 4.51 (d, J = 9.2 Hz, 2H), 4.28 (dd, J = 7.7, 3.6 Hz, 1H), 3.99 (s, 3H), 3.92 (ddd, J = 17.3, 11.7, 5.4 Hz, 2H), 3.65 (dt, J = 11.0,5.2 Hz, 5H), 3.59 (s, 3H), 3.52 (d, J = 10.9 Hz, 2H), 3.04 (d, J = 12.3 Hz, 2H), 2.88 (ddt, J = 16.5, 11.2, 5.2 Hz, 5H), 2.66 (dt, J = 11.7, 5.6 Hz, 2H), 2.49 (dt, J = 19.8, 6.8 Hz, 2H), 2.26 - 2.10 (m, 4H), 2.00 - 1.80 (m, 8H), 1.78 - 1.67 (m, 4H), 0.86 (t, J = 7.5 Hz, 3H). LCMS $[M+H]^+$ = 938.7 |
| | |

(continued)

| Cmpd. No. | Structure and data analysis |
|---|---|
| UB-180855 (855) | 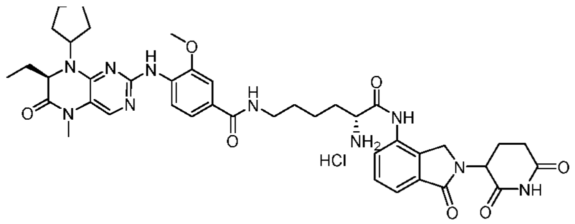 |
| | $^1$H NMR (400 MHz, DMSO-$d_6$) δ 11.03 (s, 1H), 9.49 (s, 1H), 8.62 (m, 1H), 8.45 (m, 2H), 7.90 - 7.71 (m, 3H), 7.63 - 7.46 (m, 4H), 5.16 (dd, *J* = 13.1, 5.2 Hz, 1H), 4.64 - 4.33 (m, 3H), 4.17 (t, *J* = 8.6 Hz, 2H), 3.89 (s, 3H), 3.22 (s, 3H), 2.99 - 2.83 (m, 1H), 2.62 (d, *J* = 17.0 Hz, 1H), 2.32 (dt, *J* = 12.9, 6.4 Hz, 1H), 2.08 - 1.71 (m, 11H), 1.63 - 1.38 (m, 8H), 0.76 (t, *J* = 7.3 Hz, 3H). LCMS $[M+H]^+$ =795.6 |
| UB-180856 (856) | 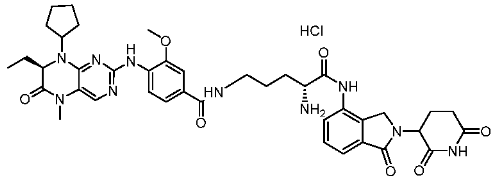 |
| | $^1$H NMR (400 MHz, DMSO-$d_6$) δ 11.02 (d, *J* = 3.3 Hz, 1H), 8.94 - 8.68 (m, 1H), 8.42 (s, 3H), 8.07 - 7.73 (m, 3H), 7.71 - 7.31 (m, 4H), 5.15 (dt, *J* = 12.0, 5.7 Hz, 1H), 4.55 - 4.37 (m, 2H), 4.18 (dd, *J* = 21.9, 12.6 Hz, 2H), 3.89 (d, *J* = 1.9 Hz, 3H), 3.49 (d, *J* = 2.4 Hz, 1H), 3.22 (s, 3H), 3.00 - 2.84 (m, 1H), 2.59 (m, 1H), 2.28 (p, *J* = 10.1, 9.4 Hz, 1H), 2.16 - 1.65 (m, 12H), 1.48 (d, *J* = 35.1 Hz, 5H), 1.24 (s, 2H), 0.75 (t, *J* = 7.4 Hz, 3H). LCMS $[M+H]^+$ =781.6 |

**Example 3 Synthesis of ACTED Compound**

**Example 3.1 Synthesis of TED with linker**

**Synthesis method of Compound 256**

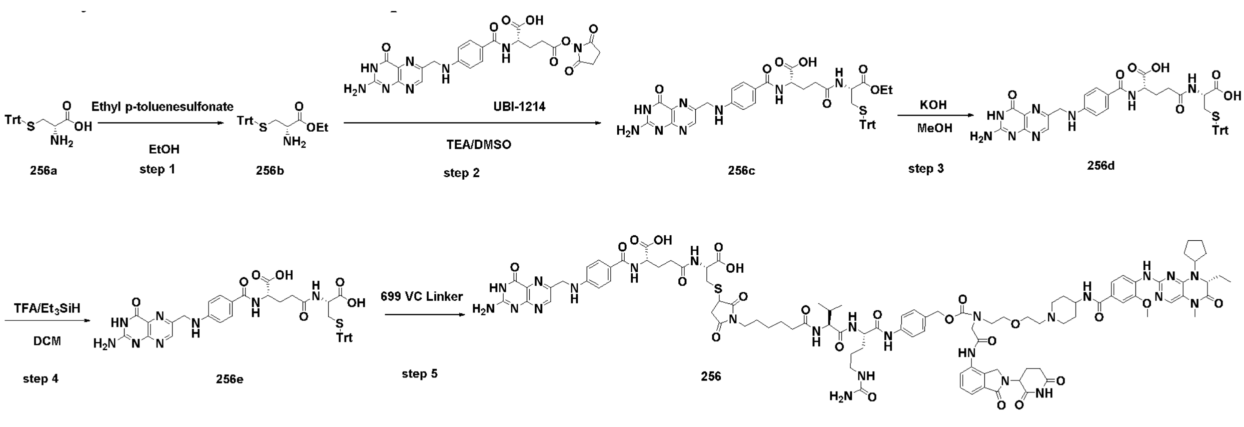


**Step 1: 256b (V1318-051)**

Compound **156a** (19 g, 52 mmol) and ethyl *p*-toluenesulfonate (21 g, 105 mmol) were dissolved in ethanol (150 mL) and reacted under reflux for 16 hours. The reaction solution was concentrated to obtain crude product. The crude product was isolated by silica gel column chromatography (ethyl acetate/petroleum ether = 1/3) to obtain target product **256b** (23 g, yield 100 %) as yellow oil. LCMS [M+H]+ =392.0

**Step 2: 256c (V1318-053)**

Compound **256b** (2.0 g, 3.7 mmol) was dissolved in DMSO (30 mL), then added **UBI-1214** (1.45 g, 3.7 mmol) and TEA (1.9 g, 18.6 mmol), and the reaction was reacted at 25°C for 16 hours. The reaction solution was slurried with ether (50 mL*3), then filtered to obtain target product **156c** (2.3 g, yield 77%) as brown solid. The solid was directly used in the next reaction.

**Step 3: 256d (V1318-062)**

Compound **156c** (3 g, 3.7 mmol) was dissolved in methanol (30 mL) and water (5 mL) and then added KOH (825 mg, 14.7 mmol). The reaction was carried out at 25°C for 18 hours. The crude product was purified by a reversed-phase column (methanol/water=0% to 95%, 45 minutes, collected at 3%) to obtain target product **256d** (750 mg, yield 26 %) as yellow solid. LCMS $[M+H]^+$ =787.2

**Step 4: 256e (V1318-064)**

Compound **256d** (100 mg, 0.13 mmol) was dissolved in TFA (3 mL) and dichloromethane (3 mL), followed by adding a catalytic amount of $Et_3SiH$, and then reacted at room temperature for 1 hour. The reaction solution was concentrated to obtain target crude product **256e** (100 mg, yield 100%) as a yellow solid. The solid was directly used in the next step. LCMS $[M+H]^+$ =545.0

**Step 5: 256 (V1318-065)**

Compound **156e** (5 mg, 0.11 mmol) was dissolved in N,N-dimethylformamide (1 mL) and DIEA (6 mg, 0.04 mmol) and then added **1699-Vc-linker** (10 mg, 0.01 mmol)), and then reacted at room temperature for 16 hours. The reaction solution was purified by Prep-HPLC to obtain target product **256** (7.6 mg, yield 40%) as yellow solid. LCMS $[M/2+H]^+$ =1018.8

**Synthesis method of Compound 258**

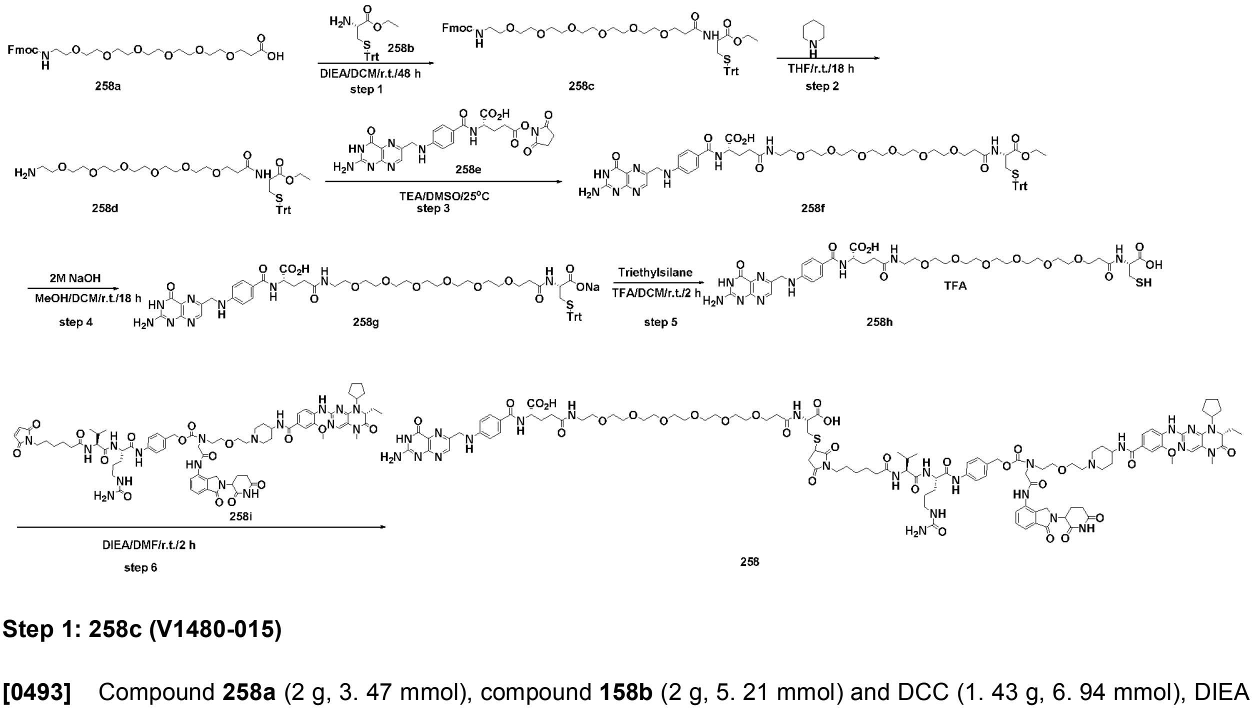


**Step 1: 258c (V1480-015)**

Compound **258a** (2 g, 3. 47 mmol), compound **158b** (2 g, 5. 21 mmol) and DCC (1. 43 g, 6. 94 mmol), DIEA (2 mL) were dissolved in dry dichloromethane (50 mL) and reacted at 25°C for 48 hours. After the completion of reaction, water (20mL) was added, extracted with dichloromethane (20mL*3). The organic phases were combined, then washed with saturated brine, dried over anhydrous $Na_2SO_4$, concentrated under reduced pressure and isolated by reversed-

phase column (MeOH/$H_2O$ = 2 % to 97%) to obtain target product **258c** as yellow transparent oil (1. 5 g, yield 45%). LCMS [M+H] + = 949

**Step 2: 258d (V1480-018)**

Compound **258c** was dissolved in dry tetrahydrofuran (15 mL), added piperidine (9 mL, 90 mmol) and reacted at room temperature for 18 hours. After the completion of reaction, water (20mL) was added, extracted with ethyl acetate (20mL *3). The organic phases were combined, then washed with saturated brine, dried over anhydrous $Na_2SO_4$, concentrated under reduced pressure and isolated by silica gel column chromatography (dichloromethane/MeOH=0% to 50%) to obtain target compound **258d** (900 mg, yield 78%) as yellow oil $^1$H NMR (400 MHz, DMSO-$d_6$) δ 8.36 (d, *J* = 7. 8 Hz, 1H), 7. 42 - 7. 20 (m, 15H), 4. 10 (td, *J* = 8. 1, 5. 4 Hz, 1H), 4. 02 - 3. 97 (m, 2H), 3. 58 (t, *J* = 6. 5 Hz, 2H), 3. 53 - 3. 45 (m, 19H), 3. 40 (d, *J* = 5. 6 Hz, 2H), 2. 73 (t, *J* = 5. 6 Hz, 2H), 2. 35 (tt, *J* = 7. 1, 2. 9 Hz, 3H), 1. 10 (t, *J* = 7. 1 Hz, 3H). LCMS [M+H] + = 727

**Step 3:258f (V1480-020)**

Compound **258e**(1. 65 g, 3. 0 mmol), compound **258d** (900 mg, 1. 23 mmol) and triethylamine (500 mg) were dissolved in dry DMSO (10 mL) and reacted at 25 °C for 24 hours. Ether (50 mL∗3) was added and stirring for 3 minutes. The ether layer was removed and centrifuging with acetonitrile (100 mL). The solid was collected to obtain the target product **258f** (2 g) as red transparent oil. LCMS [M+H] + =1150.

**Step 4: 258g (V1480-022)**

Compound **158f** (500 mg, 0.43 umol) were dissolved in dichloromethane (20 mL) and MeOH (10 mL) and then aded 2M NaOH (1 mL). The reaction was reacted at room temperature for 18 hours. The reaction solution was concentrated under reduced pressure and isolated by a reversed-phase column (MeOH/$H_2O$ = 2% to 30%) to obtaintarget product **258g** (30 mg, purity 70%) as yellow solid. LCMS [M+H] + =1122

**Step 5: 258h (V1480-027)**

Compound **258g** (40 mg, 0.035 mmol) was dissolved in dry dichloromethane (4 mL) and TFA (0. 5 mL) and triethylsilane (3 drops). The reaction was reacted at room temperature for 2 hours. The reaction was concentrated under reduced pressure, added ether (10 mL) and stirred. Solids were formed. The solids is filtered to obtain yellow target product **258h** (45 mg, purity 70%). $^1$H NMR (400 MHz, DMSO-$d_6$) δ 8. 65 (d, *J* = 1. 6 Hz, 1H), 8. 23 - 8. 15 (m, 2H), 7. 88 (q, *J* = 7. 6, 6. 6 Hz, 1H), 7. 69 - 7. 61 (m, 2H), 6. 64 (dd, *J* = 8. 9, 2. 3 Hz, 2H), 4. 49 (s, 2H), 4. 44 - 4. 23 (m, 4H), 3. 60 (td, *J* = 6. 5, 1. 6 Hz, 3H), 3. 52 - 3. 43 (m, 19H), 3. 41 - 3. 28 (m, 3H), 3. 18 (t, *J* = 5. 7 Hz, 2H), 2. 89 - 2. 79 (m, 1H), 2. 78 - 2. 65 (m, 2H), 2. 43 - 2. 35 (m, 3H), 2. 26 - 2. 13 (m, 2H), 2. 10 - 1. 80 (m, 3H). LCMS [M+H] + =880.

**Step 6: 258 (V1480-029)**

Compound **258h** (7 mg, 8∗$10^{-3}$ mmol), compound **258i** (12 mg, 8∗$10^{-3}$ mmol), DIEA (5 mg) were dissolved in dry DMF (0. 7 mL), reacted at room temperature for 2 hours. The reaction solution was filtered, prepared by Prep-HPLC to obtain target product **258** (10. 4 mg, 55% yield) as yellow solid. LCMS [M/2+H] + =1186

**Synthesis method of Compound 266**

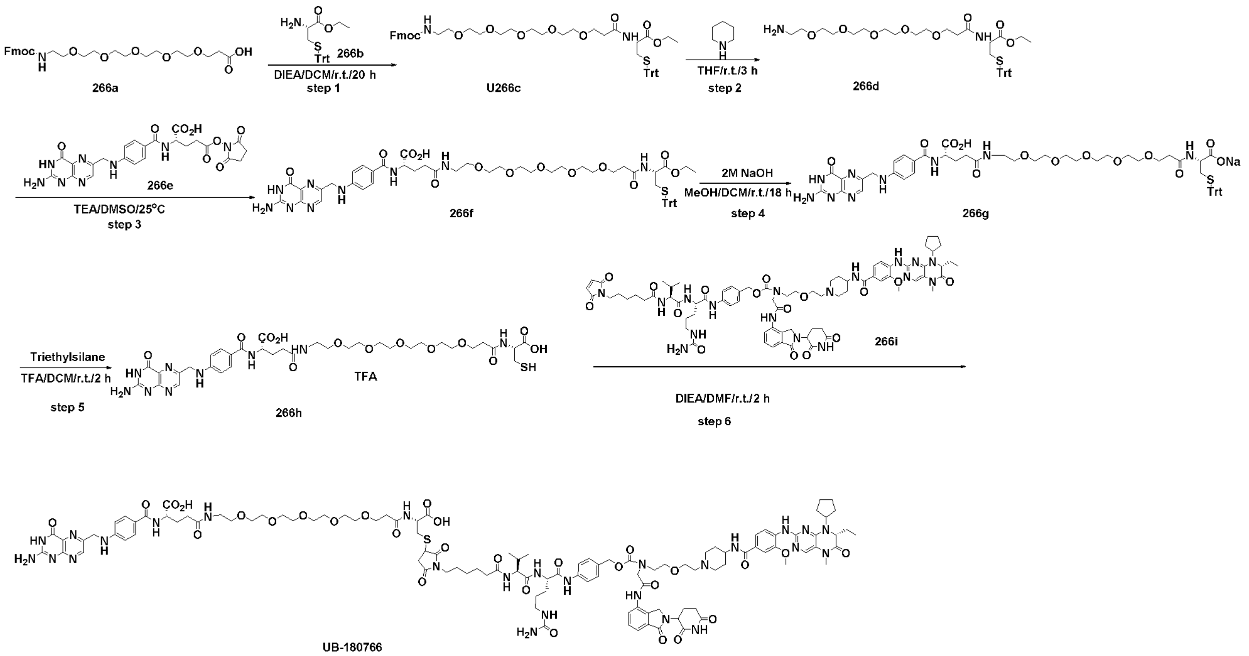


**Step 1: 266c (V1480-023)**

Compound **266a**(1. 5 g, 2. 82 mmol), compound **266b** (1. 65 g, 4. 23 mmol) and (1. 16 g, 5. 64 mmol), DIEA (1. 5 mL) were dissolved in dry dichloromethane (50 mL) and reacted at 25°C for 48 hours. After the completion of reaction, water (20mL) was added, extracted with dichloromethane (20mL ∗3). The organic phases were combined, then washed with saturated brine, dried over anhydrous $Na_2SO_4$, concentrated under reduced pressure and isolated by reversed-phase column ($MeOH/H_2O$ = 2 % to 97%) to obtain target product **266c** (900 mg, yield 35%) as yellow transparent oil. LCMS $[M+H]^+$ = 905

**Step 2: 266d (V1480-026)**

Compound **266c** (900 mg, 1 mmol) was dissolved in dry tetrahydrofuran (15 mL), added piperidine (5 mL, 68. 8 mmol) and reacted at room temperature for 3 hours. After the completion of reaction, water (20mL) was added, extracted with ethyl acetate (20mL∗3). The organic phases were combined, then washed with saturated brine, dried over anhydrous $Na_2SO_4$, concentrated under reduced pressure and isolated by reversed-phase column ($MeOH/H_2O$ = 2 % to 80%) to obtain target compound **266d** (460 mg, yield 67%) as yellow oil.
LCMS $[M+H]^+$ = 683.

**Step 3: 266f (V1480-028)**

Compound **266e** (725 mg, 1.34 mmol), compound **266d** (460 mg, 0.67 mmol) and triethylamine (270 mg) were dissolved in dry DMSO (4 mL) and reacted at 25 °C for 18 hours, added ether (50 mL∗3) and stirred for 3 minutes. The ether layer was removed and centrifuging with acetonitrile (100 mL). The solid was collected to obtain the target product **266f** (1 g) as red transparent oil. LCMS $[M+H]^+$ =1107

**Step 4: 266g (V1480-030)**

Compound **266f** (1 g, 0.9 umol) were dissolved in dichloromethane (10 mL) and MeOH (20 mL) and then aded 2M NaOH (2 mL). The reaction was reacted at room temperature for 3 hours. The reaction solution was concentrated under reduced pressure and isolated by a reversed-phase column ($MeOH/H_2O$ = 2% to 30%) to obtain target product **266g** (170 mg, yield 17%) as yellow solid. LCMS $[M+H]^+$ =1078

**Step 5: 266h (V1480-031)**

Compound **266g** (40 mg, 0.037 mmol) was dissolved in dry dichloromethane (4 mL) and TFA (0. 5 mL) and triethylsilane (3 drops). The reaction was reacted at room temperature for 2 hours. The reaction was concentrated under

reduced pressure, added ether (10 mL) and stirred. Solid was formed. The solid was filtered to obtain yellow target product **266h** (25 mg, yield 80%). LCMS $[M+H]^+$ =836

**Step 6: 266 (V1480-032)**

Compound **266h** (10 mg, $12*10^{-3}$ mmol), compound **266i** (12 mg, $8*10^{-3}$ mmol), DIEA (5 mg) were dissolved in dry DMF (0. 7 mL), reacted at room temperature for 2 hours. The reaction solution was filtered, then purified by Prep-HPLC to obtain target product **266** (6. 2 mg, 33% yield) as yellow solid. LCMS $[M/2+H]^+$ =1165

**Synthesis method of Compound 157**

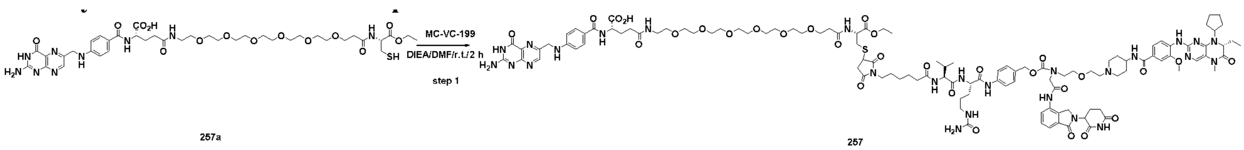


257a

257

**Step 1: 257 (V1162-111)**

The solution of Compound **257a** (5 mg, $8*10^{-3}$ mmol), (12 mg, $8*10^{-3}$ mmol), TEA (5 mg) in N,N-dimethylformamide (0.7 mL) was reacted at room temperature for 2 hours. The reaction solution was isolated and purified by Prep-HPLC to obtain target compound (4.0 mg, yield 30.7%) as yellow solid. LCMS $[M/2+H]^+$ =1200.5

**Synthesis method of Compound MC-VC-133 / 180/199:**

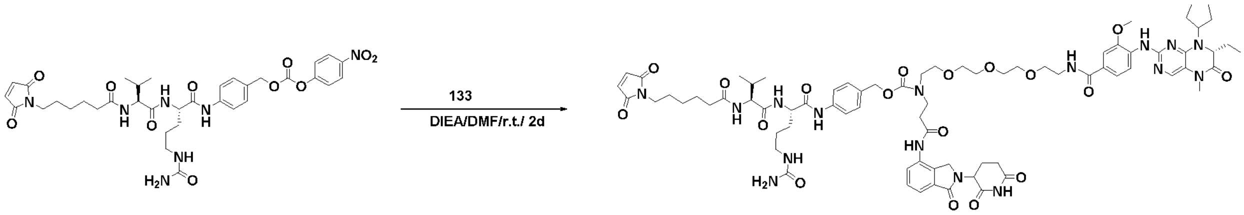


MC-VC-PAB-PNP

MC-VC-133

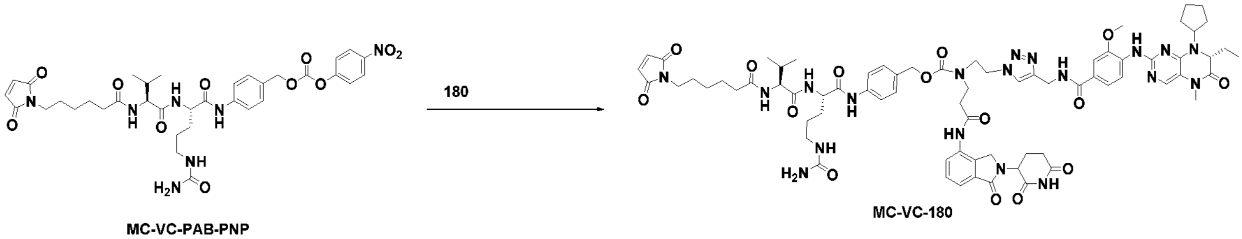


MC-VC-180

MC-VC-PAB-PNP

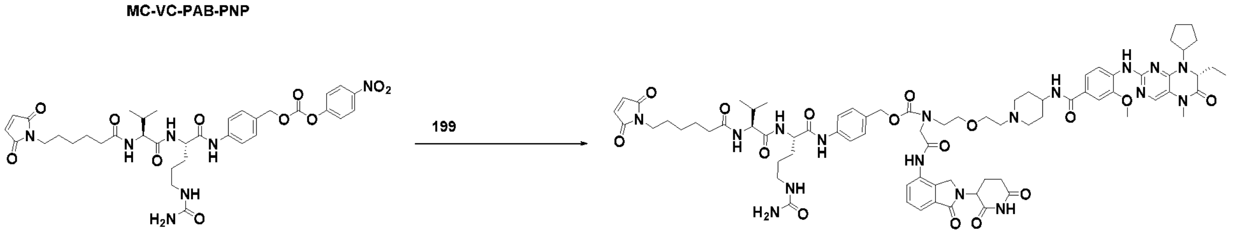


MC-VC-PAB-PNP

MC-VC-199

Compound **133/ 180/ 199** (40 mg, leq.), **MC-VC-PAB-PNP** (1 eq.), **HOBT** (2eq.) were dissolved in **DMF** (0.8 mL) and **DIEA** (2eq.), reacted at room temperature for 48 hours under nitrogen protection. The reaction solution was directly isolated by a reversed-phase preparative chromatography column to obtain the target compound **MC-Vc-133/180/199.**

**Synthesis method of Compound UBI-1212**

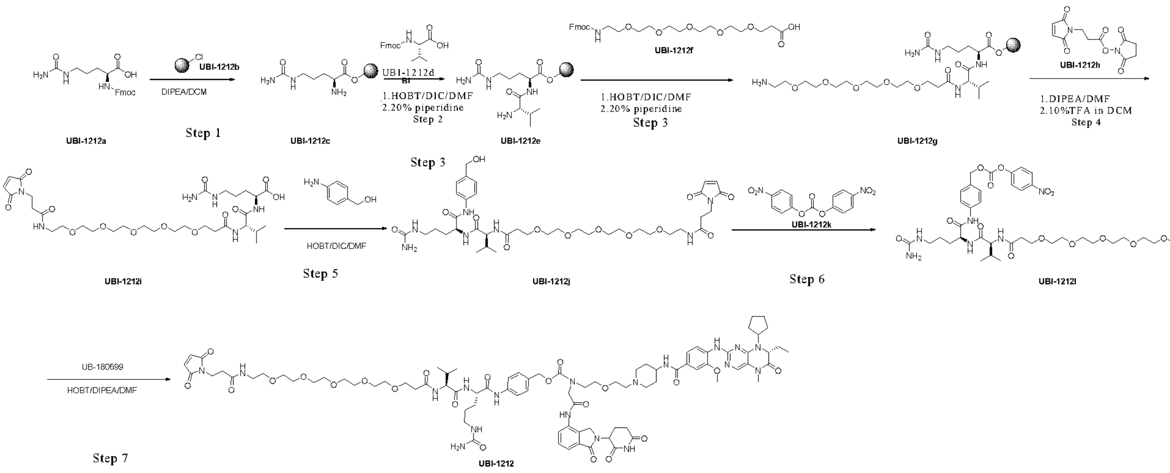


**Step 1: UBI-1212c (V1162-120)**

MC-Val-Cit-OHCl-TrtCl-resin (**UBI-1212b**) was placed in the filter, added 10mL of dichloromethane, stirred for one hour and then filtered. Then a solution of Fmoc-Cit-OH (**UBI-1212a**) (600mg, 1.5mmol) and DIPEA (0.66ml) in dichloromethane was added, reacted at room temperature for one hour. The reaction solution was added methanol and stirred for 15 minutes, and then filtered to obtain solids. The solids was washed with the following solvents: dichloromethane (5 x 50mL x 0.5 min), N,N-dimethylformamide (5 x 50 mL x 0.5 min), piperidine : N,N-dimethylformamide (1 :4, 1 x 1 min, 2 x 10 min), N,N-dimethylformamide (5 x 50 mL x 0.5 min), dichloromethane (5 x 50 mL x 0.5 min) to obtain the target compound resin (**UBI-1212c**).

**Step 2: UBI-1212e (V1162-121)**

**Compound UBI-1212c** washed with N,N-dimethylformamide (5 x 50 mL x 0.5 min) and then added Fmoc-Val-OH (**UBI-1212d**) (950mg, 2.8mmol), HOBt (567mg, 4.2mmol) in N,N-dimethylformamide (20 mL). After stirring for 10 minutes, DIC (538 mg, 4.2 mmol) was added and reacted at room temperature for 1.5 hours. The reaction solution was filtered to obtain solids, which was washed with the following solvent: N,N-dimethylformamide (5 x 50 mL x 0.5 min), piperidine : N,N-dimethylformamide (1 :4, 1 x 1 min, 2 x 10 min), N,N-dimethylformamide (5 x 50 mL x 0.5 min) to obtain target compound resin (**UBI-1212e**).

**Step 3: UBI-1212g (V1162-122)**

**Compound UBI-1212e** was washed with N,N-dimethylformamide (5 x 50 mL x 0.5 min) and then added **UBI-1212f** (740mg, 1.4mmol), HOBt (567mg, 4.2mmol) in N,N-dimethylformamide (20 mL). After stirring for 10 minutes, DIC (538 mg, 4.2 mmol) was added and reacted at room temperature for 1.5 hours. The reaction solution was filtered to obtain solids, which was washed with the following solvent: N,N-dimethylformamide (5 x 50 mL x 0.5 min), piperidine : N,N-dimethylformamide (1 :4, 1 x 1 min, 2 x 10 min), N,N-dimethylformamide (5 x 50 mL x 0.5 min) to obtain target compound resin (**UBI-1212g**).

**Step 4: UBI-1212i (V1162-123)**

**Compound UBI-1212g** was washed with N,N-dimethylformamide (5 x 50 mL x 0.5 min) and then added **UBI-1212h** (740mg, 2.8mmol) in N,N-dimethylformamide (20mL) and DIPEA (0.66mL) solution, reacted at room temperature for 1 hour. The reaction solution was washed with N,N-dimethylformamide (5 x 50 mL x 0.5 min), then added dichloromethane (10 mL) and TFA (1 mL) were added, and reacted at room temperature for 2 hours. The reaction solution was filtered, and the filtrate was concentrated to obtain crude product as oil. Then it was isolated and purified by reversed-phase column (MeOH/$H_2O$=5% to 95%, collected at 45%, detected by UV 210) to obtain target compound **UBI-1212i** (600mg) as colorless oil. $^1$H NMR (400 MHz, DMSO-$d_6$) δ 8.19 (d, *J* = 7.3 Hz, 1H), 8.02 (d, *J* = 5.7 Hz, 1H), 7.83 (d, *J* = 9.1 Hz, 1H), 7.35 - 7.11 (m, 2H), 7.00 (s, 2H), 5.95 (s, 1H), 4.25 (dd, *J* = 9.0, 6.7 Hz, 1H), 4.12 (t, *J* = 7.1 Hz, 1H), 3.81

(m, 8H), 3.59 (t, *J* = 7.3 Hz, 4H), 3.49 (t, *J* = 5.3 Hz, 8H), 3.36 (t, *J* = 5.9 Hz, 2H), 3.15 (q, *J* = 5.8 Hz, 2H), 2.95 (t, *J* = 6.7 Hz, 2H), 2.37 - 2.19 (m, 4H), 2.01 - 1.88 (m, 1H), 1.70 (d, *J* = 12.4 Hz, 1H), 1.60 - 1.52 (m, 1H), 1.43 - 1.30 (m, 2H), 0.84 (dd, *J* = 15.4, 6.8 Hz, 6H). LC-MS: $(M+H)^+$=717.5

**Step 5: UBI-1212j (V1162-129)**

A solution of **Compound UBI-1212i** (442 mg, 0.61 mmol), 4-aminobenzyl alcohol (152mg, 1.2 mmol) in N,N-dimethylformamide (4 mL) was added HOBT (166 mg, 1.2 mmol) and DIC (194ul, 1.2mmol). The reaction was reacted at room temperature for 2 hours. The reaction solution was purified and isolated by reversed-phase column (MeOH/$H_2O$ = 5% to 95%, collected at 40%) to obtain **UBI-1212j** (140mg, yield 28%) LC-MS: $(M+H)^+$=822.4

**Step 6: UBI-1212l (V1162-134)**

**Compound UBI-1212j** (380 mg, 0.46 mmol), **UBI-1212k** (281 mg, 0.92 mmol) were added to a solution of DIPEA (0.3 mL) and N,N-dimethylformamide (2 mL), and reacted at room temperature for 18 hours. The reaction solution was purified and isolated by a reversed-phase column (acetonitrile/water = 5% to 50%, collected at 40%) to obtain **UBI-1212l** (130 mg, yield 28.5%) as light yellow solid.

$^1$H NMR (400 MHz, DMSO-$d_6$) δ 10.06 (s, 1H), 8.38 - 8.23 (m, 2H), 8.14 (d, *J* = 7.4 Hz, 1H), 8.01 (t, *J* = 5.6 Hz, 1H), 7.88 (d, *J* = 8.6 Hz, 1H), 7.70 - 7.63 (m, 2H), 7.60 - 7.53 (m, 2H), 7.44 - 7.37 (m, 2H), 7.00 (s, 2H), 6.15 - 5.96 (m, 1H), 5.25 (s, 2H), 4.98 (s, 4H), 4.43 - 4.36 (m, 1H), 4.24 (dd, *J* = 8.6, 6.6 Hz, 1H), 3.64 - 3.56 (m, 4H), 3.52 - 3.45 (m, 16H), 3.36 (t, *J* = 5.9 Hz, 2H), 3.15 (q, *J* = 5.8 Hz, 2H), 3.06 - 2.91 (m, 2H), 2.33 (dd, *J* = 7.9, 6.7 Hz, 2H), 1.97 (h, *J* = 6.7 Hz, 1H), 1.78 - 1.32 (m, 4H), 0.85 (dd, *J* = 13.3, 6.7 Hz, 6H). LC-MS : $(M+H)^+$=987.4

**Step 7: UBI-1212 (V1162-136)**

**Compound UBI-1212l** (40 mg, 0.04 mmol) and compound **199** (37 mg, 0.04 mmol), and HOBT (5.4 mg, 0.04 mmol) were added to a solution of DIEA (15ul, 0.08 mmol) and N,N-dimethylformamide (500uL), reacted at room temperature for 36 hours. The reaction solution was purified by Prep-HPLC to obtain target compound **UBI-1212** (45 mg, yield 65.2 %) as white solid. $^1$H NMR (400 MHz, DMSO-$d_6$) δ 11.02 (d, *J* = 7.0 Hz, 1H), 10.04 - 9.87 (m, 2H), 9.38 (s, 1H), 8.99 (s, 1H), 8.43 (d, *J* = 7.3 Hz, 1H), 8.10 (d, *J* = 6.7 Hz,2H), 8.03 - 7.98 (m, 1H), 7.86 (d, *J* = 7.8 Hz, 2H), 7.79 (s, 1H), 7.73 (d, *J* = 7.7 Hz, 1H), 7.62 (d, *J* = 8.3 Hz, 1H), 7.56 - 7.50 (m, 6H), 7.34 (d, *J* = 8.4 Hz, 1H), 7.25 (d, *J* = 8.2 Hz, 1H), 7.00 (s, 2H), 6.00 (s, 2H), 5.14 (td, *J* = 12.6, 5.1 Hz, 1H), 5.04 (d, *J* = 14.2 Hz, 1H), 4.44 - 4.12 (m, 2H), 3.98 - 3.86 (m, 12H), 3.80 - 3.68 (m, 10H), 3.64 - 3.52 (m, 12H), 3.38 - 3.33 (m, 12 H), 3.26 - 3.19 (m, 3H), 3.14 (q, *J* = 5.8 Hz, 5H), 2.37 - 2.30 (m, 4H), 2.09 - 1.74 (m,4H), 1.62 - 1.40 (m,9H), 0.88 - 0.79 (m, 9H), 0.76 (t, *J* = 7.4 Hz, 3H). LC-MS: M+H=1742.9

**Synthesis method of Compound UBI-1211**

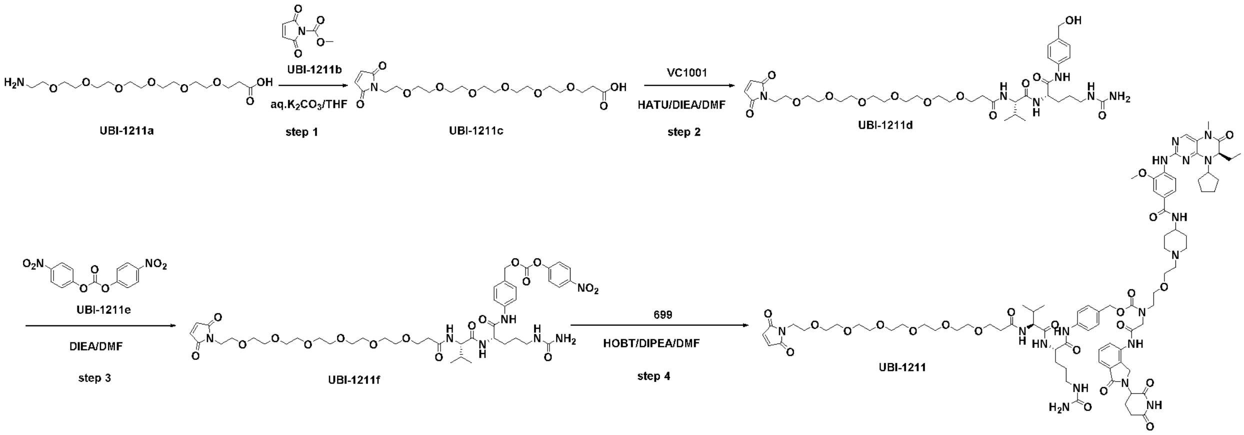


**Step 1: UBI-1211c (V1318-111)**

Compound **UBI-1211a** (2 g, 5.7 mmol) was dissolved in tetrahydrofuran (20 mL), then added **UBI-1211b** (1.1 g, 6.8 mmol) and $K_2CO_3$ aqueous solution (10 mL). The reaction was carried out at room temperature for 1 hours. After

tetrahydrofuran was removed, the reaction solution was purified by a reversed-phase column (methanol/water = 0% to 25%, 45 minutes, collected at 0%) to obtain crude product as white solid. The crude product was acidified with 3N HCl and then purified by reversed-phase column (methanol/water = 0% to 80%, 45 minutes, collected at 25%) to obtain target product **UBI-1211c** (lg, yield 41%) as yellow oil. LCMS $[M+H]^+$ =434.3

**Step 2: UBI-1211d (V1318-112)**

**UBI-1211c** (200 mg, 0.46 mmol) was dissolved in N,N-dimethylformamide (3 mL) and then added HATU (191 mg, 0.50 mmol) and DIEA(163 mg, 1.26 mmol. After reacting at room temperature for 20 minutes, VC1001 (UBI-1207) (159 mg, 0.42 mmol) was added, and then reacting at room temperature for 16 hours. The reaction solution was concentrated and purified by a reversed-phase column (methanol/water = 0% to 95%, 45 minutes, collected at 50%) to obtain 200 mg crude product as colorless oil. The crude product was subjected to Prep-TLC (methanol/dichloromethane = 1/10) to obtain target product **UBI-1211d** (100 mg, yield 27%) as colorless oil. $^1H$ NMR (400 MHz, Chloroform-*d*) $\delta$ 7.59 - 7.47 (m, 2H), 7.25 (d, *J*= 8.6 Hz, 2H), 6.71 (s, 2H), 4.57 (s, 2H), 4.23 (s, 1H), 3.91 - 3.37 (m, 26H), 3.19 - 2.98 (m, 2H), 2.68 (m, 12H), 1.49 - 1.36 (m, 2H), 1.12 - 0.70 (m, 6H).

**Step 3: UBI-1211f(V1318-114)**

Compound **UBI-1211d** (100 mg, 0.13 mmol), **UBI-1211e** (76 mg, 0.25 mmol) were dissolved in DIPEA (0.3 mL) and N,N-dimethylformamide (2 mL), and then reacted at room temperature for 18 hours. The reaction was purified by a reversed-phase column (acetonitrile/water = 5% to 50%, 40min, collected at 40%). After lyophilization, target product **UBI-1211f** (27 mg, yield 22%) as light yellow solid was obtained. LCMS $[M+H]^+$ =960.3

**Step 4: UBI-1211 (V1318-115)**

Compound **UB-1211f** (27 mg, 0.03 mmol) and **199** (26 mg, 0.03 mmol), HOBT (4 mg, 0.03 mmol), DIEA (7 mg, 0.06 mmol) were dissolved in N,N-dimethylformamide (0.3 mL), then reacted at room temperature for 36 hours. The reaction solution was purified by Prep-HPLC to obtain target product **UBI-1211** (10 mg, yield 21%) as white solid. LCMS $[M+H]^+$ =1715.6.

**Example 3.2 Synthesis of ACTED Compound**

Example 3.2.1

Compound TECP (Tris(2-chloroethyl) phosphate) (67 uL, 5mmol/L in PBS buffer, pH=7.4) was added to a solution of anti-CD33 antibody (2.5mg, 5mg/mL) in PBS buffer solution at 0°C, incubated for 2 hours at 37°C after well-mixed. The reaction solution was ultrafiltered for 5 times (500rpm, 5min) and replaced with PBS buffer solution. Then the reaction solution was cooled to 4 °C, and added TED with linker (53ul, 5mmol/L N,N-dimethylformamide solution), and reacted at 4 °C for 1 hour. L-cysteine was added and stirred for 10 minutes. After ultrafiltration for 5 times (14 krpm, 5min), clear ACTED solution was obtained by concentration.

Analysis Conditions:

The elution method of HIC column:

| Time (min) | % B |
|---|---|
| 0.0 | 0 |
| 12.0 | 100 |
| 12.1 | 0 |
| 18.0 | 0 |

Mobile Phase A: 1.5M $(NH_4)_2SO_4$ buffer solution: pH=6.9-7.1.
198.21g $(NH_4)_2SO_4$ was dissolved in 800 mL of water, and diluted to 1L. Adjusted to pH=7 using $Na_2HPO_4$ (25mM).
Mobile Phase B: 0.025M $K_2HPO_4$ and 0.025M $KH_2PO_4$ buffer solution: pH=6.9-7.1.
a: S1: 0.025M $K_2HPO_4$: 4.35g $K_2HPO_4$ was dissolved in 800mL of water, and diluted to 1L

b: S2: 0.025M $KH_2PO_4$: :3.4g $K_2HPO_4$ was dissolved in 800mL of water, and diluted to 1L
c: S1 (610ml) and S2 (390mL) were mixed and then added 0.025M $K_2HPO_4$ buffer solution, adjusted to pH=6.9-7.1.
d: Mobile phase B was prepared using c 75% + isopropanol (25%)
Notes: All mobile phases need to be filtered by a filter membrane.

Examples of ACTED compounds:

**Table C: ACTED Compound**

| Example | Structure | DAR (Drug Antibody ratio) |
|---|---|---|
| AC-721(UB-180721) | Anti-CD33-Ab-MC-VC-633 | 2.0 |
| AC-252(UB-180752) | Anti-CD33-Ab-MC-VC-680 | 4.0 |
| AC-222(UB-180722) | Anti-CD33-Ab-MC-VC-680 | 2.0 |
| AC-253(UB-180753) | Anti-CD33-Ab-MC-VC-699 | 3.6 |
| AC-305(UB-180805) | Anti-CD33-Ab-PEG5-VC-699 | 2.3 |
| AC-306 (UB-180806) | Anti-CD33-Ab-PEG5-VC-699 | 3.6 |
| AC-825(UB-180825) | Anti-CD33-Ab-PEG6-VC-699 | 6.5 |

MC and VC are abbreviations for amino acids, and their structure is as follows:

| **MC** | **VC (Val-Cit)** | VC-PAB (Val-Cit-PAB) |
|---|---|---|
| 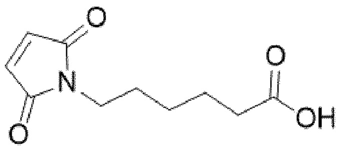 | 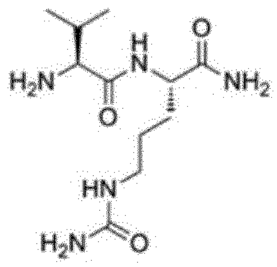 | 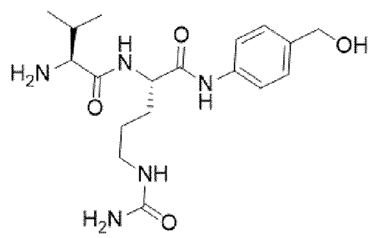 |

PEG5 and PEG6 refer to $(-CH_2-CH_2-O-)_5$ and $(-CH_2-CH_2-O-)_6$;

Anti-CD33-Ab refers to anti-CD33 antibody.

UB-180721:

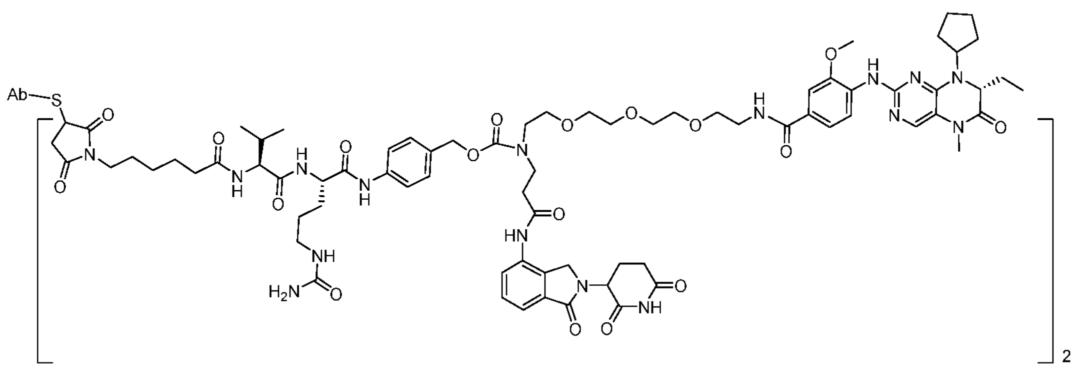

UB-180722:

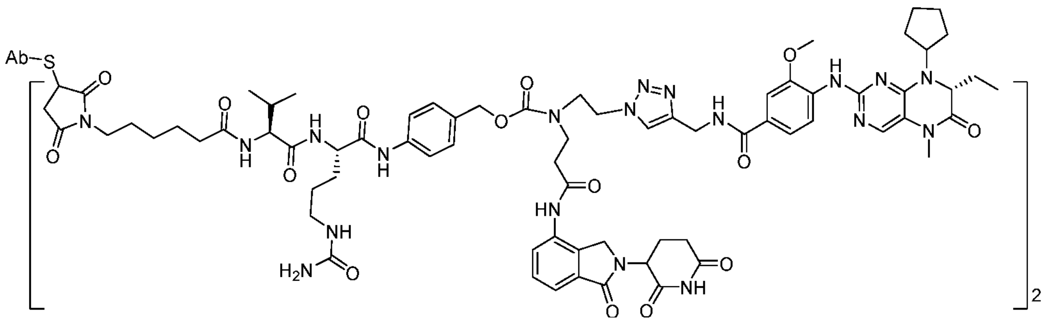

UB-180752:

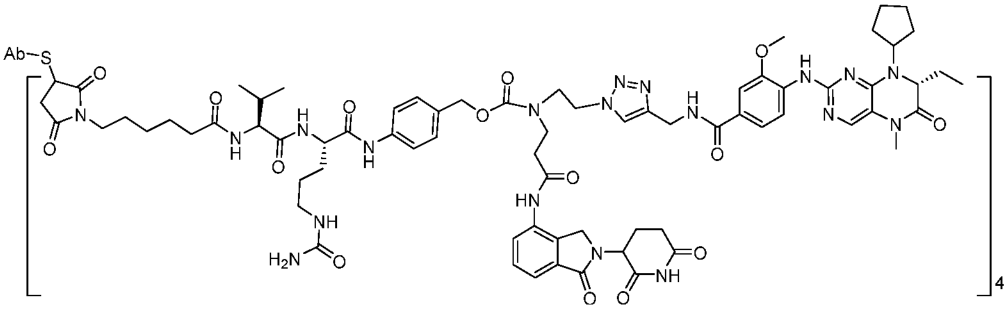

UB-180753:

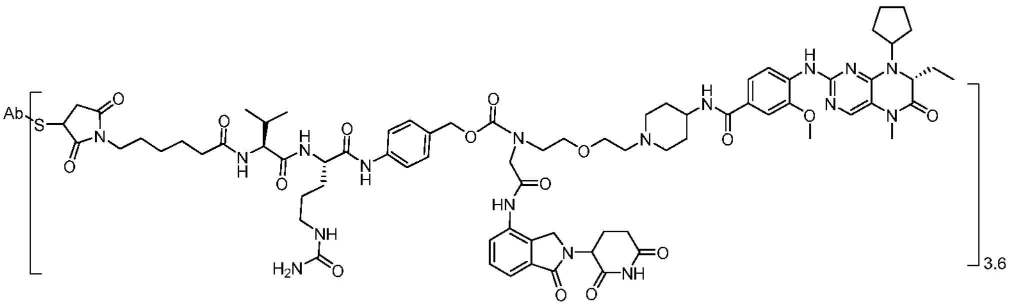

UB-180805:

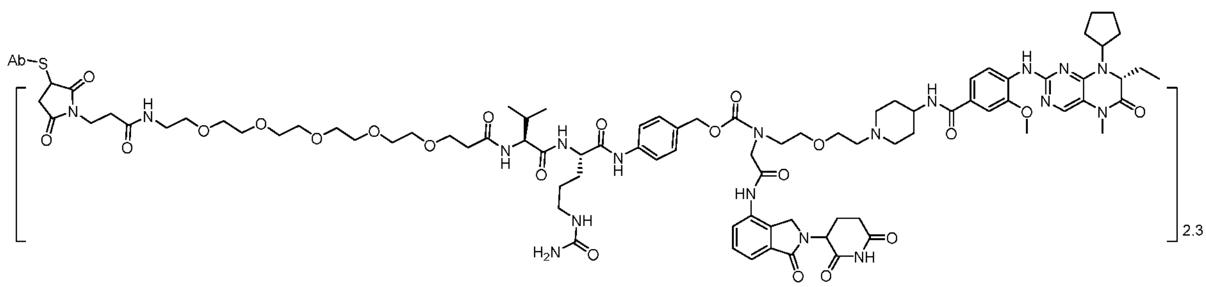

UB-180806:

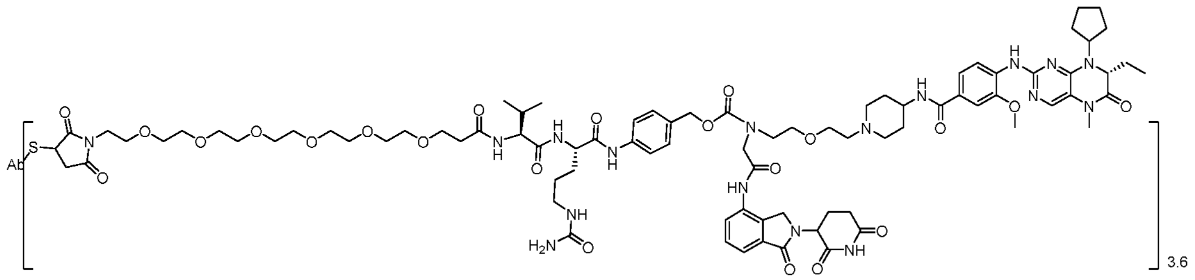

UB-180825:

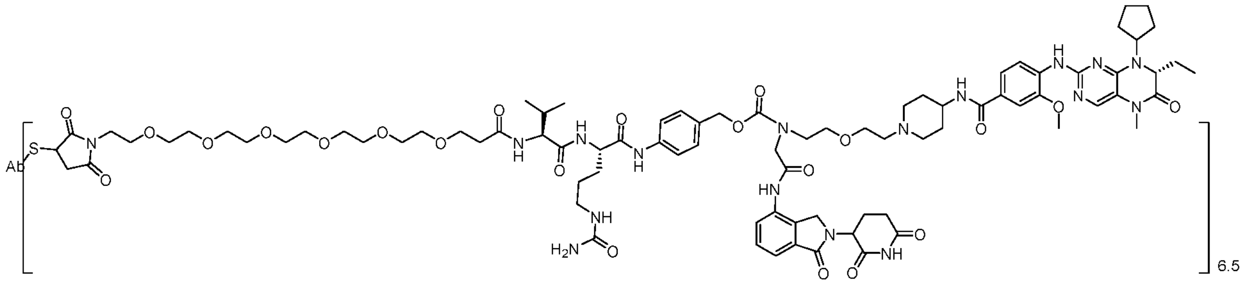

in each formula, Ab refers to antibody.

Similarly, a series of ACTED compounds with DAR ranging from 2 to 5 were synthesized using AC-001~AC-006 as TED with linker.

AC-001

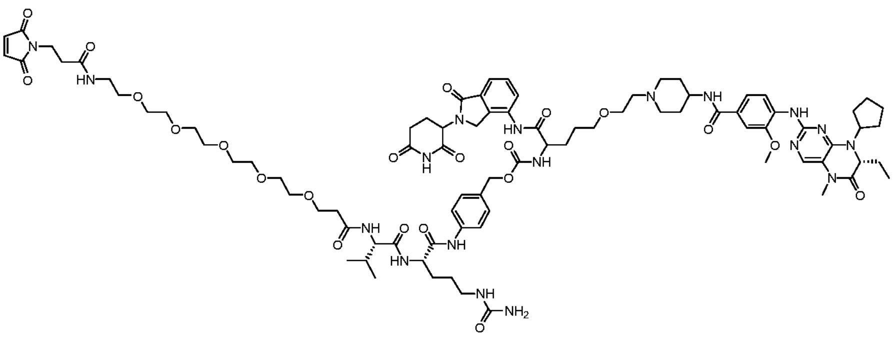

AC-002

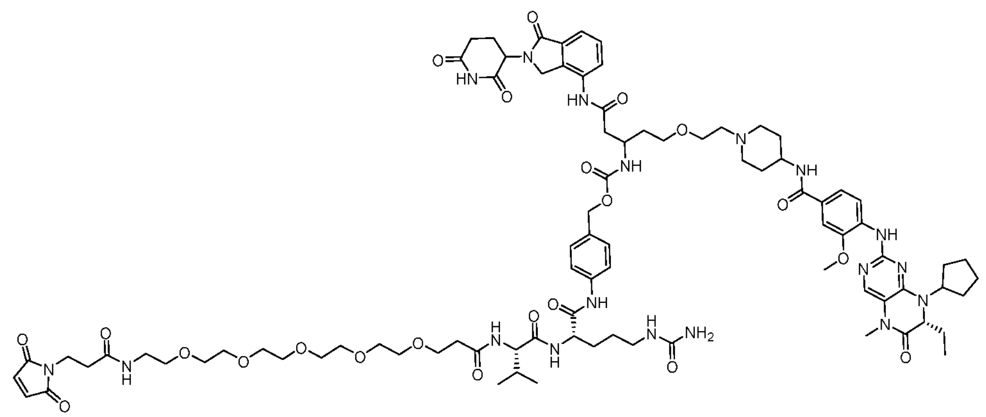

AC-003

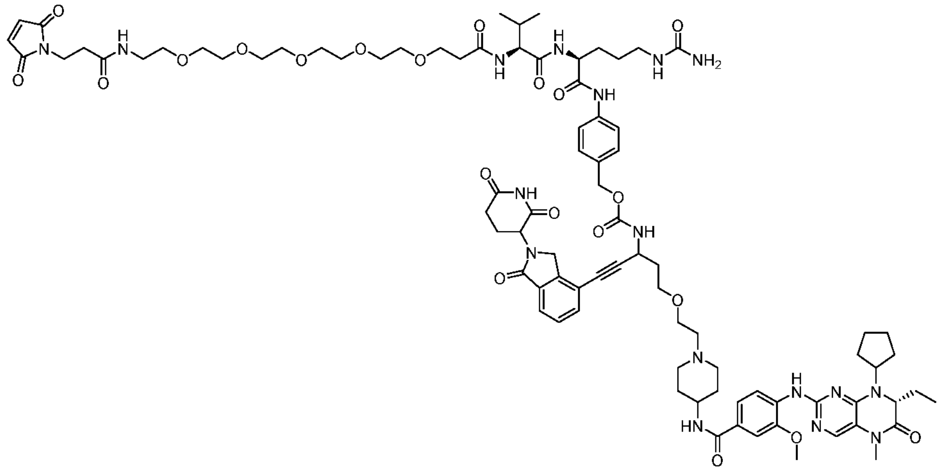

AC-004

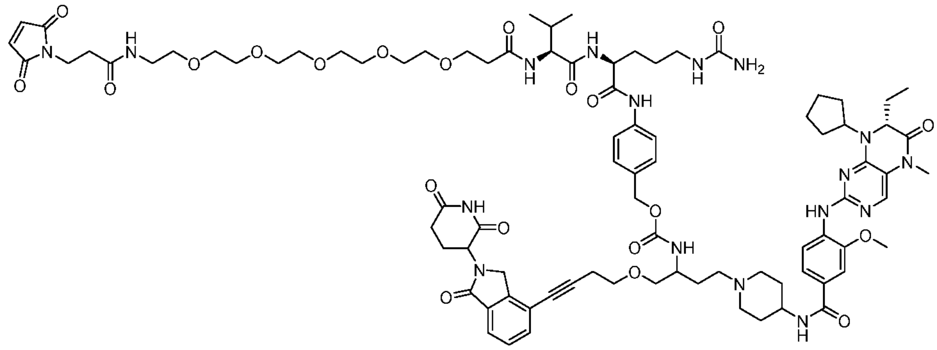

AC-005

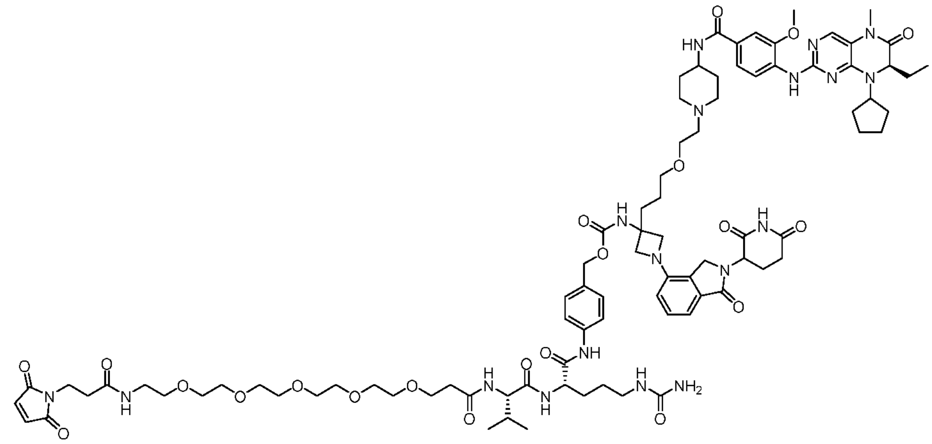

AC-006

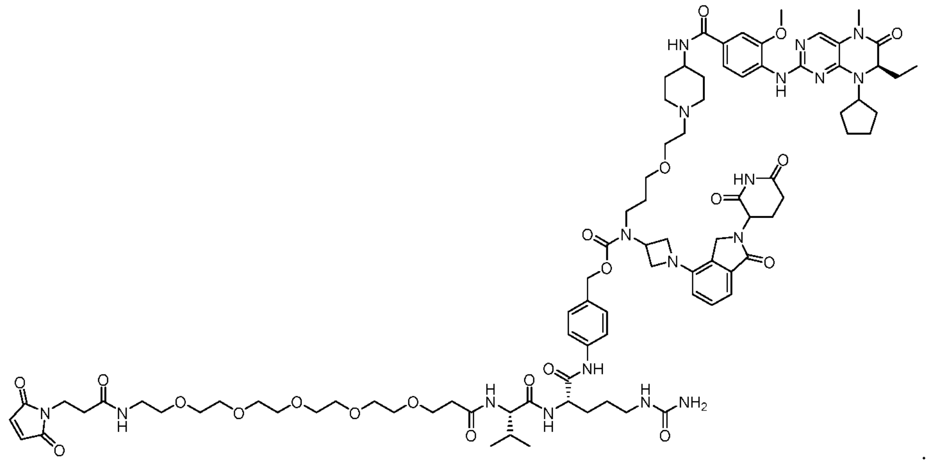

### Example 4: Preparation of T ED conjugated with Folic Acid

Synthesis method of Compound UB-180839 FR-Peptide-Linker S-S-730

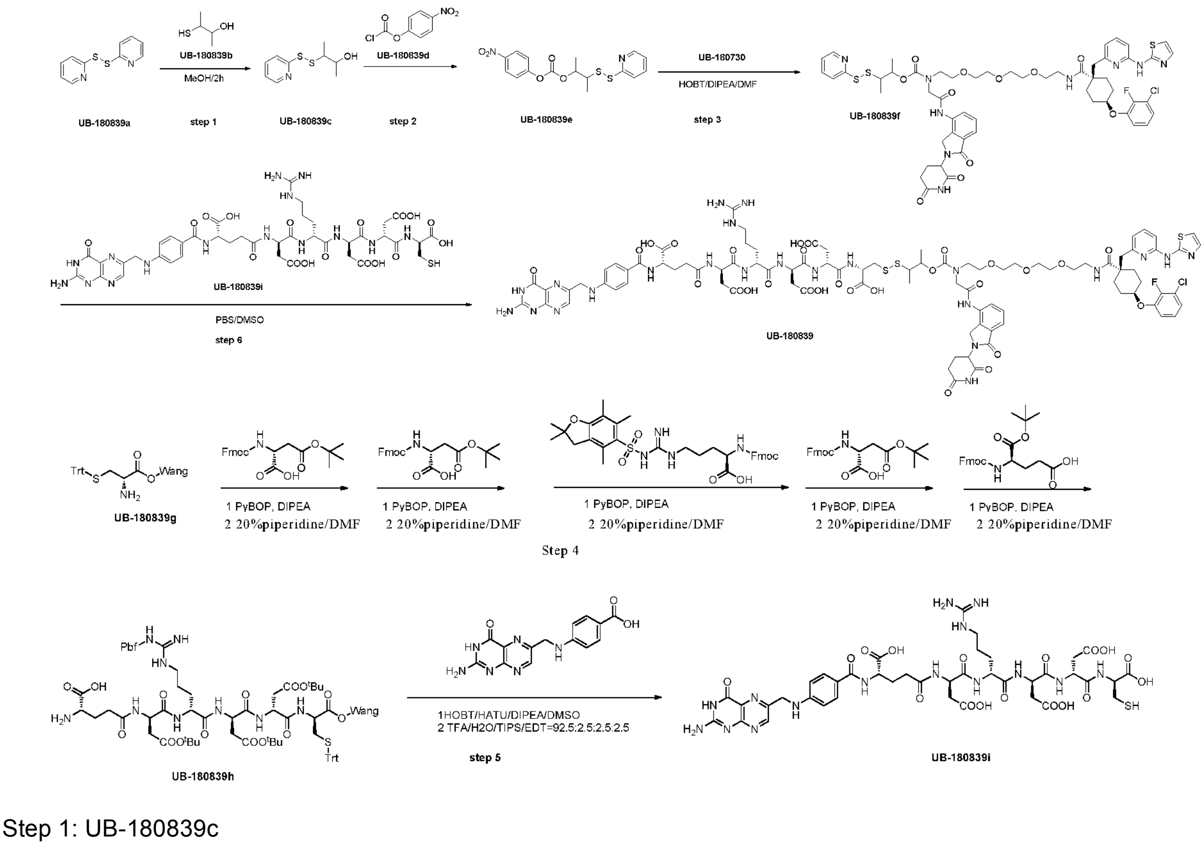


Step 1: UB-180839c

Compound UB-180839a (500 mg, 2.3 mmol) was dissolved in methanol (10 mL) and then added UB-180839b (80 mg, 0.76 mmol). The reaction was carried out at room temperature overnight under nitrogen protection. The reaction solution was concentrated to obtain crude product. The crude product was isolated by column chromatography (ethyl acetate/dichloromethane = 1/10) to obtain crude product UB-180839c (300 mg, yield 61%) as colorless oil.

Step 2: UB-180839e

Compound UB-180839c (300 mg, 1.4 mmol) was dissolved in dichloromethane (10 mL), and then added UB-180839d (309 mg, 1.5 mmol) and TEA (282 mg, 2.8 mmol). The reaction was carried out at room temperature overnight under nitrogen protection. The reaction solution was concentrated and purified via reversed-phase column (methanol/water = 5 % to 95%, collected at 70%) to obtain target product UBI-180839e (80 mg, yield 15%) as yellow oil.

Step 3: UB-180839f

Compound UB-180839e (20 mg, 0.05 mmol) and UB-180730 (49 mg, 0.05 mmol), HOBT (7 mg, 0.05 mmol), DIEA (14 mg, 0.11 mmol) were dissolved in N,N-dimethylformaldehyde amide (2 mL). The reaction was allowed to react at room temperature for 36 hours. The reaction solution was prepared to obtain UB-180839f (10 mg, yield 16%) as white solid product.

Step 4: UB-180839h

The resin of compound UB-180839g was placed in a reactor with a filter device, added 50 mL dichloromethane, and reacted for 1 hour on the shaker. The resin was obtained after filtration and was added to the piperidine/DMF (1:4) solution to react for 15 minutes, and then washed with anhydrous DMF (5 x 30 mL x 0.5 min) to obtain the resin.

N-Fluorenylmethoxycarbonyl-D-aspartate-4-tert-butyl ester (3.8g, 9.2 mmol), PyBOP (7.2g, 13.8 mmol), DIPEA (2.4mL, 6.9mL) were dissolved in DMF, and then added Resin. The mixture was reacted for 2h on a shaker. Then mixture was washed with DMF (5 x 30 mL x 0.5 min). The resulting resin was added to piperidine/DMF (1:4) solution and reacted for 15 minutes, then washed with anhydrous DMF (5 x 30 mL x 0.5 min) to obtain the resin.

N-Fluorenylmethoxycarbonyl-D-aspartate-4-tert-butyl ester (3.8g, 9.2 mmol), PyBOP (7.2g, 13.8 mmol), DIPEA (2.4mL, 6.9mL) were dissolved in DMF, and then added Resin. The mixture was reacted for 2h on a shaker. Then mixture was washed with DMF (5 x 30 mL x 0.5 min). The resulting resin was added to piperidine/DMF (1:4) solution and reacted for 15 minutes, then washed with anhydrous DMF (5 x 30 mL x 0.5 min) to obtain the resin.

Fmoc-D-Arg(Pbf)-OH(6g, 9.2 mmol), PyBOP (7.2g, 13.8 mmol), DIPEA (2.4mL, 6.9mL) were dissolved in DMF, and then added resin. The mixture was reacted for 2h on a shaker. Then mixture was washed with DMF (5 x 30 mL x 0.5 min). The resulting resin was added to piperidine/DMF (1:4) solution and reacted for 15 minutes, then washed with anhydrous DMF (5 x 30 mL x 0.5 min) to obtain the resin.

N-Fluorenylmethoxycarbonyl-D-aspartate-4-tert-butyl ester (3.8g, 9.2 mmol), PyBOP (7.2g, 13.8 mmol), DIPEA (2.4mL, 6.9mL) were dissolved in DMF, and then added Resin. The mixture was reacted for 2h on a shaker. Then mixture was washed with DMF (5 x 30 mL x 0.5 min). The resulting resin was added to piperidine/DMF (1:4) solution and reacted for 15 minutes, then washed with anhydrous DMF (5 x 30 mL x 0.5 min) to obtain the resin.

N-(9-Fluorenylmethoxycarbonyl)-D-glutamate-1-tert-butyl ester (3.8g, 9.2 mmol), PyBOP (7.2g, 13.8 mmol), DIPEA (2.4mL, 6.9mL) were dissolved in DMF, and then added resin. The mixture was reacted for 2h on a shaker. Then mixture was washed with DMF (5 x 30 mL x 0.5 min). The resulting resin was added to piperidine/DMF (1:4) solution and reacted for 15 minutes, then washed with anhydrous DMF (5 x 30 mL x 0.5 min) to obtain the resin.

Step 5: UB-180839i

Pteroic acid (1.75g, 4.6mmol) was added to DMSO (1L), then heated to 120 °C to dissolve, and slowly cooled to room temperature to obtain a clear solution. Then added UBI-180839h (20g) resin, HATU (5.2g, 13.8mmol), and DIPEA (7.2mL). The mixture was reacted for 16h on a shaker. After the reaction solution was filtered, the resin was washed with DMSO (50mLx3), DMF (50mLx3), DCM (50mlx3), respectively, to obtain crude product. The crude product was added to a solution of TFA/$H_2O$/TIPS/EDT=92.5:2.5:2.5:2.5(100 mL)and stirred for 1 h. The filtrate was obtained by filtration, concentrated and purified by reversed-phase column to obtain UB-180839i (80mg).

Step 6: UB-180839

Compound UB-180839g (10 mg, 0.01 mmol) was dissolved in DMSO (1 mL) and added UB-180839f (27 mg, 0.03 mmol) in PBS (0.5 mL) and DMSO (0.5 mL). The reaction solution was maintained at room temperature overnight. The reaction solution was prepared to obtain product UB-180839 (1.0 mg, yield 2%) as white solid.

Synthesis method of Compound UB-180838 (FR-Peptide-Linker S-S-699) was the same as UB-180839.

Synthesis method of Compound UB-180829 (FR-Peptide-Linker S-S-699) was the same as UB-180839.

Table D shows the Analytical data for TED conjugated with folic acid molecule 829, 838, and 839.

Table D

| | |
|---|---|
| **838** | 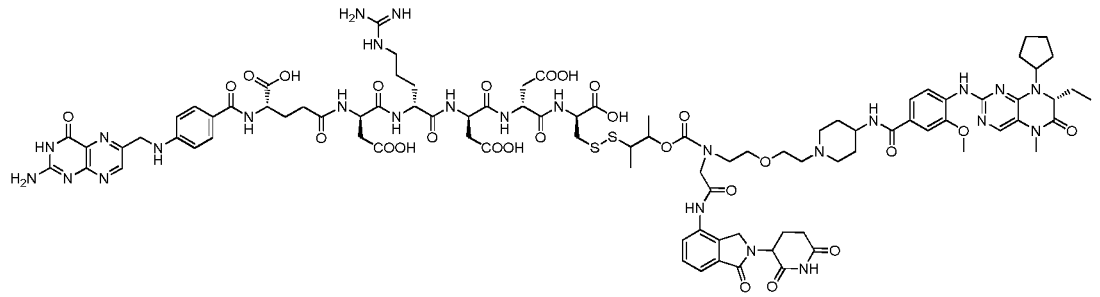 |
| | LCMS $[M/2+1]^+$ =1036.0 |
| **839** | 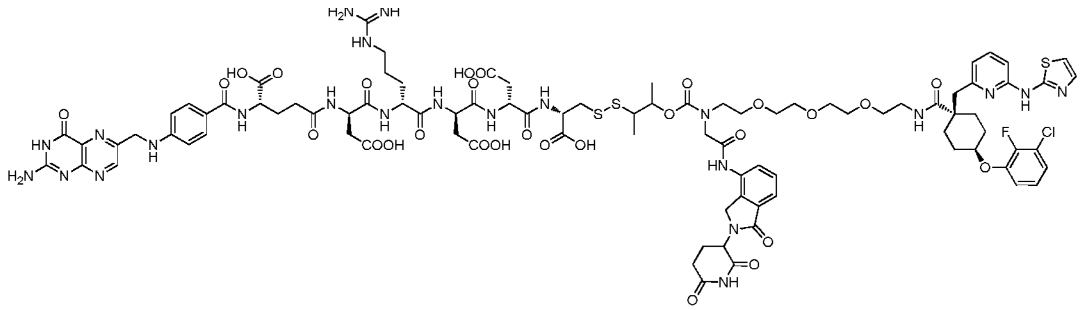 |
| | LCMS $[M/2+1]^+$ =1056.4 |
| **829** | 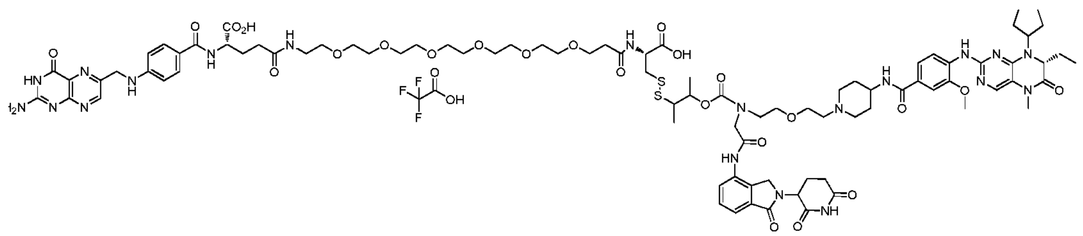 |
| | LCMS $[M/2+1]^+$ =953.0 |

**Example 5: Preparation of TED conjugated with polypeptide molecule**

Polypeptide CAADGCTWEVWGRECWY-$NH_2$ was synthesized using conventional polypeptide synthesis procedures.

Synthesis method of Peptide-TED conjugate UB-180759 is shown as follows:

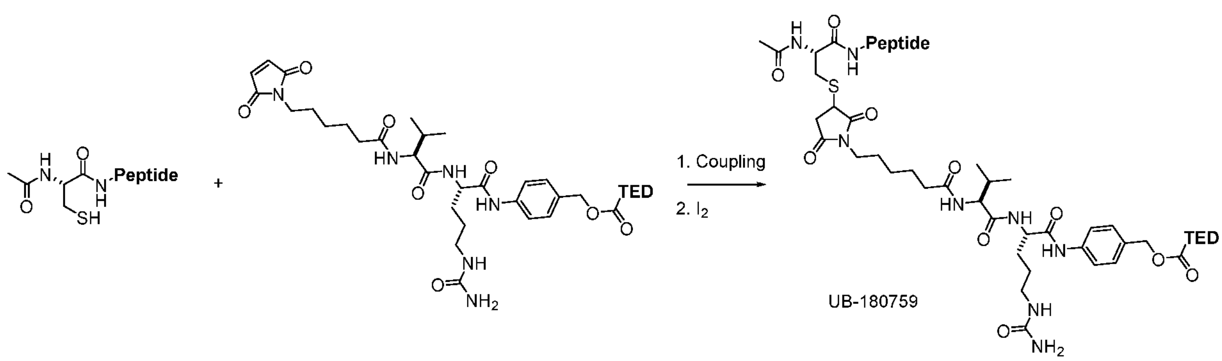


in the formula, Peptide (polypeptide) refers to any peptide drug (for example, a polypeptide with the sequence of CAADGCTWEVWGRECWY).

The coupling reaction operation of polypeptide and TED is the same as that of antibody and TED; the molecule after the peptide coupling with TED needs to be further oxidized. The oxidation operation is as follows: 40 mg of polypeptide (75% purity, i.e. 40*0.75/3710.7=0.008 mmol) was taken and added following solvents to make the concentration be about 1 mg/mL: 50% methanol in water 15 mL, 50% acetic acid 15 mL, 1 N HCl 1 mL. Iodine (253) 8eq was dissolved in a very small amount of methanol; finally added iodine solution under nitrogen protection. After reacting 30 minutes, ascorbic acid (176.12) was added: 18mg (added about ratio of 1:1 according to calculation of remaining molar amount of iodine) was directly added, dissolved in 5mL of water, which was added dropwise till the reaction become colorless. After the reaction was completed, the system was slightly turbid white. The pH was adjusted to weakly acidic condition with aqueous ammonia. After centrifugation, the supernatant was lyophilized and purified to obtain compound UB-180759. MS=3567 was detected.

## Test Example 1 Biological Test

### 1.1 Biological assay of the inhibitory effect of the compound of the present invention on MV4;11 cell proliferation

Experimental Materials:

| Reagent | Supplier | Article No. | Batch number |
|---|---|---|---|
| MV4;11 cell line | Viva | / | / |
| Iscove's modified D μL becco's medium | Gibco | 12200-036 | 1946620 |
| FBS | Gibco | 10091-148 | 1735161 |
| pen-strep | Gibco | 15140-122 | 1953108 |
| Trypsin-EDTA (0.25%), Phenol Red | Thermo | 25200056 | 1951208 |
| DPBS | Thermo | 14190144 | 1952086 |
| Cell Titer -Glo | Promega | G7573 | 0000310975 |

Experimental method:

1) Preparation of buffer

MV4;11 cell culture medium: Iscove's modified Dulbecco's medium was added 10% FBS and 1/100pen-strep.

2) Experimental Procedure:

(1) MV4;11 cells were subcultured with cell culture medium, and the well-growing cells are seeded in a 96-well plate, 100μL per well, the number of cells per well is 20,000, and the cells are cultured overnight in a cell incubator at 37°C and 5% $CO_2$.
(2) Drugs were prepared as 10mM stock solution with dimethyl sulfoxide (DMSO). The compounds were diluted (which ensure the concentration of DMSO in the culture system being 1%), each concentration in duplicate. Diluted compounds were added to cell culture wells (final concentration was 100μM, 33μM, 11μM...), shaking gently to mix. Two negative control wells were set only added cells (ensuring that the DMSO concentration in the culture system was 1%).

3) Measurement for Result:

(1) After incubating for 72 hours, 50μL Cell Titer-Glo was added to each well and incubating at room temperature for 10 minutes.
(2)The chemiluminescence signal values were read by using Envision.

(3) Data were analysed using Dose-response-inhibition equation in software GraphPad Prism to obtain the IC50 value.

**1.2 PLK1 protein expression biological assay of the compound of the present invention**

Experimental Materials

| Reagent | Supplier | Article No. | Batch number |
|---|---|---|---|
| MV4;11 cell line | Viva | / | / |
| RIPA Lysis and Extraction Buffer | Thermo | 89900 | TD265751 |
| Complete Tablets EDTA-free | Roche | 4693132001 | 27423500 |
| Novex™ 4 - 20% Triglycine Mini Gel, WedgeWell™ format, 12 wells | Invitrogen | XP04202BOX | 18052448 |
| PageRμLer™ added pre-stained protein ladder | Thermo | | |
| Novex™ Tris-Glycine SDS electrophoretic buffer (10X) | Thermo | LC2675 | 1952401 |
| Novex™ Tris-Glycine Transfer Buffer (25X) | Thermo | LC3675 | 1934476 |
| Pierce™ 20X TBS Tween™ 20 buffer | Thermo | 28360 | TD265259 |
| SuperSignal™ West Femto Maximum Sensitivity Substrate | Thermo | 34095 | TE265690 |
| Nitrocellulose membrane | Amersham | 10600001 | A14115264 |
| PLK1 (208G4) Rabbit mAb | Cell Signaling | 4513 | / |
| GAPDH (D16H11) XP® Rabbit mAb | Cell Signaling | 5174 | / |
| Anti -Rabbit IgG, HRP-linked antibody | Cell Signaling | 7074P2 | / |

Experimental method:

1) Preparation of buffer

| | |
|---|---|
| 1x electrophoretic buffer | Novex™ Tris-Glycine SDS electrophoretic buffer (10X) was diluted to 1X |
| 1x transfer buffer | Novex™ Tris-Glycine transfer buffer (25X) was diluted to 1X, added 20% methanol |
| 1x TBST | Pierce™ 20X TBS Tween™ 20 Buffer was diluted to 1X |
| Confining Liquid | 1x TBST with 5% skimmed milk |

2) Experimental procedure:

(1) MV4;11 cells were subcultured with cell culture medium, and the well-growing cells were seeded in a 6-well plate, 2ml per well, the number of cells per well is 1 million, and the cells were cultured overnight in a cell incubator at 37°C and 5% $CO_2$.
(2) Drugs were prepared as 10mM stock solution with dimethyl sulfoxide (DMSO). Diluted compounds were added to cell culture wells (ensuring that the concentration of DMSO in the culture system was 1%), shaking gently to mix. Negative control well was set in addition (by adding the same amount of DMSO).
(3) After incubating 6 hours, the cells were lysed using RIPA cells lysis buffer, proteins were extracted, the concentration of the proteins measured by BCA kits. 2X protein loading buffer was added, heated at 100°C for 10 minutes, and the samples were stored at -20°C.
(4) Samples were loaded, in amount of protein 25μg per well, onto polyacrylamide gel for electrophoresis.

(5) Proteins transfered from polyacrylamide gel to PVDF membrane, 5% skimmed milk was added to seal at room temperature for 1 hour.
(6) Primary antibodies (PLK1 (208G4) rabbit mAb and GAPDH (D16H11) XP® rabbit mAb) were incubated overnight at 4°C, the membranes were washed three times with TBST solution, 10 minutes for each.
(7) Secondary antibodies (Anti-rabbit IgG, HRP-linked antibody) were incubated at room temperature for 1 hour, and then the membranes were washed with TBST solution three times, 10 minutes for each.

3) Measurement for Result:

Finally, chromogenic solution (SuperSignal™ West Femto Maximum Sensitivity Substrate) was added for color development, photos were taken using automatic chemiluminescence instrument (ChemiDoc XRS+ (BIO-RAD)), pictures were collected and analyzed.

**1.3 Biological assay of inhibitory effect of the compound of the present invention on PLK1 enzyme activity**

Experimental Materials:

| Reagent | Supplier | Article No. | Batch number |
|---|---|---|---|
| ADP-Glo™ Kinase Assay | promega | V9101 | 0000312764 |
| ADP-Glo™ reagent | promega | V912A | 0000234146 |
| Kinase Dection Buffer | promega | V913A | 0000310185 |
| Kinase Dection Substrate | promega | V914A | 0000233450 |
| Ultra Pure ATP | promega | V915A | 0000301398 |
| ADP | promega | V916A | 0000307789 |
| PLK1 Kinase Enzyme System | promega | V2841 | 0000322411 |
| PLK1, active | promega | / | P1590-7 C306 |
| Casein, dephosphorylated | promega | / | C2090-7 D205 |
| 5x reaction buffer A | promega | / | M2814-6 C523 |
| DTT | promega | / | G1379-9 |

Experimental method:

1) Preparation of buffer

Enzyme reaction buffer: 5X buffer were diluted to 1X using dd$H_2O$, 50 uM DTT was added.

2) Experimental Procedure:

(1) Enzyme, substrate and ATP were diluted using 1X buffer.
(2) 2μL of 2.5X enzyme solution was added to each well.
(3) 1μL of diluted compound was added to each well, and incubated at room temperature for 15 minutes.
(4)2μL of 2.5X substrate/ATP mixture was added to each well, and incubated at room temperature for 1 hour.
(5) 5μLADP-glo was added to each well, and incubated at room temperature for 40 minutes.
(6)10μL detection reagent was added, incubated at room temperature for 30 minutes.

3) Measurement for Result:

(1)The chemiluminescence signal values were read using Envision.
(2) Data were analysed using Dose-response-inhibition equation in software GraphPad Prism to obtain the IC50 value.

**1.4 BRD4 (Bromodomain containing protein 4) protein expression biological assay of the compounds of the present invention**

The test method is similar to that in 1.2 PLK1 protein expression biological assays.

**1.5 PLK1 (Polo-like kinase 1) protein expression biological assay of the compounds of the present invention**

The test method was similar to that in 1.2 PLK1 protein expression biological assay.

**1.6 Results of Biological test**

Results for protein expression test of 1.2, 1.4 and 1.5 are shown in Figure 2.
The inhibitory activity $IC_{50}$ of following molecules on MV4;11 cells are between 0.1nM-10M: UB-180510,UB-180519, UB-180523, UB-180525, UB-180529, UB-180537, UB-180539, UB-180541, UB-180545, UB-180552, UB-180553, UB-180554, UB-180564, UB-180566, UB-180594, UB-180601, and UB-180602.
The inhibitory activity $IC_{50}$ of following molecules on Hela cells are between 0.1nM-10M: UB-180512, and UB-180525.
The above results show that the present invention provides a class of Targeted Enzyme Degradation (TED) molecules, which are consisted of a unit of small molecule compound targeting to target proteins, a uint binding E3 ubiquitin ligases and linker unit, which is able to bind with PLK and/or BRD4 proteins, promoting PLK1 and/or BRD4 protein to become more vulnerablly degradable by proteases, thereby inhibiting cell proliferation, and can be used as a protein degradation drug. The compound provided in the present invention has a good inhibitory effect on the proliferation of MV4-11 cells, indicating that the compound of the present invention can be prepared as antitumor drugs, especially those for the treatment of acute myeloid leukemia, AML).

**Test Example 2**

**(2.1) Cell Proliferation Experiment:**

Reagents: RPMI-1640 medium, McCoy's 5A medium, IMDM medium, MEM medium, L-15 medium, fetal bovine serum, Penicillin-Streptomycin double antibody, trypsin, etc., 2-mercaptoethanol, NEAA, pyruvate, etc.
The tumor cell lines used in this experiment are shown in Table 1.1:

**Table1 .1 List of Tumor Cell Lines**

| No. | Cell Name | Cell Source |
|---|---|---|
| 1 | Human Colon Cancer HT-29 | Shanghai Cell Bank of Chinese Academy of Sciences (CAS) |
| 2 | Human Colon Cancer HCT-116 | Shanghai Cell Bank of Chinese Academy of Sciences (CAS) |
| 3 | Human Small Cell Lung Cancer NCI-H82 | American Type Culture Collection (ATCC) |
| 4 | Human Monocytic Leukemia THP-1 | Shanghai Cell Bank of Chinese Academy of Sciences (CAS) |
| 5 | Human Acute Myelomonocytic Leukemia HL-60 | Shanghai Cell Bank of Chinese Academy of Sciences (CAS) |
| 6 | Human Glioma Cells U-87 MG | Shanghai Cell Bank of Chinese Academy of Sciences (CAS) |
| 7 | Human cervical adenocarcinoma cells Hela | Shanghai Cell Bank of Chinese Academy of Sciences (CAS) |
| 8 | Human Breast Cancer MDA-MB-231 | Shanghai Cell Bank of Chinese Academy of Sciences (CAS) |
| 9 | Human Acute Lymphoblastic Leukemia Cells MOLT-4 | Shanghai Cell Bank of Chinese Academy of Sciences (CAS) |

(continued)

| No. | Cell Name | Cell Source |
|---|---|---|
| 10 | Human Ovarian Cancer SK-OV-3 | Shanghai Cell Bank of Chinese Academy of Sciences (CAS) |
| 11 | Human Monocytic Leukemia HL-60 | Shanghai Cell Bank of Chinese Academy of Sciences (CAS) |
| 12 | Human Monocytic Leukemia MV4-11 | American Type Culture Collection (ATCC) |

The cells were cultured in conventional ways, and the cells should be passed at least for at least 2 generations before plating. Cells at the logarithmic growth phase are collected, prepared into single cell suspensions and counted. The concentration of cells was adjusted to the desired concentration, and cells were inoculated into a 96-well cell culture plate at 100 μl per well. 100 μL of complete medium of the test compound were added to each well, set up 2 duplicates for each concentration, and diluted with a 5-fold gradient, and continue to culture for 72 h. All cells were subjected to $EC_{50}$ test for corresponding samples.

The fluorescence intensity of each well was detected using the Alarm blue method, and $EC_{50}$ was calculated.

$EC_{50}$ was calculated according to the following formula:

$$Y=Max + (Min-Max)/[1+ (X/EC_{50}) \times Slope]$$

wherein Min, Max and Slope represent the minimum, maximum and slope respectively.

**(2.2) Flow Cytometry Experiment**

In order to test the binding ability of Ab. and ACTED to receptors (CD33, DLL3) on cell surface, cells were plated on 96-well plate, after that added primary antibody and incubated for 1 hour at 4 □, and then wash with PBS containing 1% BSA twice, then added secondary antibody and incubated at 4 □ for half an hour in the dark, washed three times and analyzed using a flow cytometer.

In order to test endocytosis of Ab. and ACTED, cells were first treated with antibody or antibody protein degradation agent conjugate for a period of time, then washed with PBS containg 1% BSA twice, and then added secondary antibody and incubated for half an hour in the dark at 4 □. In order test the level of target receptors on the cell surface, cells were incubated with FITC-anti-human CD33 or DLL3 antibody at 4 □ in the dark for half an hour, and finally washed three times and tested with a flow cytometer.

**(2.3) Western Blot**

Cells were treated with the compound for a period of time, after that the cells were collected by centrifugation. After washing with PBS, the cells were lysed by adding RIPA buffer; the cell lysate were added to the loading buffer and then appropriate volume were taken and slowly added to the corresponding wells of the gel plate, and run the SDS-PAGE gel (4%-12%). After running the gel, transfered to the PVDF membrane and sealed with 5% skimmed milk powder at room temperature for 1 hour. The membrane was placed in the primary antibody diluted with 5% skimmed milk powder and shaked slowly overnight at 4 □. After incubation with primary antibody, the membrane was washed 3 times using a TBST shaker; added secondary antibody diluted with 5% skimmed milk powder corresponding to the primary antibody, and shaked slowly at room temperature for 1 hour. After incubation with secondary antibody, the membrane was washed 3 times using the TBST shaker again. The PVDF membrane was flatted in the cassette, the strip was evenly infiltrated with ECL developer solution, and placed in ChemDoc XRS+ gel imager for taking pictures. The intensity of protein bands was analyzed quantitatively using Image**J** software.

DC50 was calculated.

**(2.4) *In vitro* kinase activity test**

The compound, enzyme, substrate and ATP were diluted using 1x reaction buffer to the desired concentration. 1 μL of compounds with different concentrations, 2 μL of enzyme, and 2 μL of substrate/ATP mixed solution were added to the 384-well plate, and incubated for 1 hour at room temperature. Then 5 μL of ADP-Glo™ reagent was added to each well and incubated at room temperature for 40 minutes. Finally, 10 μL of detection reagents were added, incubated at room temperature for 30 minutes, and chemiluminescence signals were detected using Envision.

IC50 was calculated.

The results of test are shown in Table 1.2

In the tablen A≤10 nM, 10 nM<B≤100 nM<C≤1000 nM, D > 1000 nM

| UB No. | MV4;11 EC50 nM | HCT116 EC50 nM | NCIH82 EC50 nM | HL-60 EC50 nM | HT-29 EC50 (nM) | U-87MG EC50 nM | MDA-MB-231 EC50 nM | THP-1 EC50 nM | Hela EC50 nM | MOL4-4 EC50(nM) | HUVEC EC50 nM | PBMC EC50 nM | Daudi EC50 (nM) | SKO3 EC50 (nM) | U937 EC50 (nM) | A549 EC50 (nM) | PLK1 DC50 (nM) (24h) | PLK1 DC50 (nM) (6h) | BRD4 DC50 (nM) (24h) | BRD4 DC50 (nM) (6h) | BRD4 IC50 nM |
|---|---|---|---|---|---|---|---|---|---|---|---|---|---|---|---|---|---|---|---|---|---|
| UB-180501 | D | C | - | - | - | - | - | - | - | - | - | - | - | - | - | - | - | - | - | - | - |
| UB-180502 | B | C | - | - | - | - | - | - | - | - | - | - | - | - | - | - | - | - | - | - | - |
| UB-180503 | C | C | - | - | - | - | - | - | - | - | - | - | - | - | - | - | - | - | - | - | - |
| UB-180504 | B | C | - | - | - | - | - | - | - | - | - | - | - | - | - | - | - | - | - | - | - |
| UB-180505 | C | - | - | - | - | - | - | - | - | - | - | - | - | - | - | - | - | - | - | - | - |
| UB-180506 | C | - | - | - | - | - | - | - | - | - | - | - | - | - | - | - | - | - | - | - | - |
| UB-180509 | D | D | - | - | - | - | - | - | - | - | - | - | - | - | - | - | - | - | - | - | - |
| UB-180510 | D | - | - | - | - | - | - | - | D | - | - | - | - | - | - | - | - | - | - | - | - |
| UB-180511 | C | - | - | - | - | - | - | - | D | - | - | - | - | - | - | - | - | - | - | - | - |
| UB-180512 | C | - | - | - | - | - | - | - | D | - | - | - | - | - | - | - | - | - | - | - | - |
| UB-180513 | B | B | A | B | A | B | C | B | A | B | C | C | B | A | A | B | - | - | - | - | B |
| UB-180514 | B | - | - | - | - | - | - | - | - | - | - | - | - | - | - | - | - | - | - | - | - |
| UB-180515 | D | - | - | - | - | - | - | - | D | - | - | - | - | - | - | - | - | - | - | - | - |
| UB-180516 | D | - | - | - | - | - | - | - | D | - | - | - | - | - | - | - | - | - | - | - | - |
| UB-180517 | B | - | - | - | - | - | - | - | D | - | - | - | - | - | - | - | - | - | - | - | - |
| UB-180518 | D | - | - | - | - | - | - | - | D | - | - | - | - | - | - | - | - | - | - | - | - |
| UB-180519 | B | - | - | - | - | - | - | - | D | - | - | - | - | - | - | - | - | - | - | - | - |
| UB-180520 | B | D | - | - | - | - | - | - | - | - | - | - | - | - | - | - | - | - | - | - | - |
| UB-180521 | D | - | - | - | - | - | - | - | D | - | - | - | - | - | - | - | - | - | - | - | - |
| UB-180522 | C | - | - | - | - | - | - | - | D | - | - | - | - | - | - | - | - | - | - | - | - |
| UB-180523 | B | - | - | - | - | - | - | - | D | - | - | - | - | - | - | - | - | D | - | D | - |
| UB-180524 | B | - | - | - | - | - | - | - | D | - | - | - | - | - | - | - | - | - | - | - | - |
| UB-180525 | B | - | - | - | - | - | - | - | D | - | - | - | - | - | - | - | - | D | - | D | - |
| UB-180526 | C | - | - | - | - | - | - | - | - | - | - | - | - | - | - | - | - | D | - | D | - |
| UB-180527 | D | - | - | - | - | - | - | - | - | - | - | - | - | - | - | - | - | - | - | - | - |
| UB-180528 | B | - | - | - | - | - | - | - | D | - | - | - | - | - | - | - | - | - | - | - | - |
| UB-180529 | B | - | - | - | - | - | - | - | D | - | - | - | - | - | - | - | - | D | - | D | - |
| UB-180530 | B | D | - | - | - | - | - | - | - | - | - | - | - | - | - | - | - | - | - | - | - |
| UB-180531 | B | - | - | - | - | - | - | - | - | - | - | - | - | - | - | - | - | - | - | - | - |
| UB-180532 | C | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180533 | C | - | - | - | - | - | - | - | - | - | - | - | - | - | - | - | - | - | - | - | - |
| UB-180534 | B | D | - | - | - | - | - | - | - | - | - | - | - | - | - | - | - | - | - | - | - |
| UB-180535 | B | D | - | D | - | - | - | - | - | - | D | D | - | - | - | - | - | - | - | - | - |
| UB-180536 | C | - | - | - | - | - | - | - | - | - | - | - | - | - | - | - | - | - | - | - | - |
| UB-180537 | C | - | - | - | - | - | - | - | D | - | - | - | - | - | - | - | - | - | - | - | - |
| UB-180538 | D | - | - | - | - | - | - | - | D | - | - | - | - | - | - | - | - | - | - | - | - |
| UB-180539 | B | - | - | - | - | - | - | - | D | - | - | - | - | - | - | - | - | - | - | - | - |
| UB-180540 | D | - | - | - | - | - | - | - | D | - | - | - | - | - | - | - | - | - | - | - | - |
| UB-180541 | C | - | - | - | - | - | - | - | D | - | - | - | - | - | - | - | - | - | - | - | - |
| UB-180542 | C | - | - | - | - | - | - | - | D | - | - | - | - | - | - | - | - | - | - | - | - |
| UB-180543 | B | - | - | - | - | - | - | - | D | - | - | - | - | - | - | - | - | - | - | - | - |
| UB-180544 | B | - | - | - | - | - | - | - | D | - | - | - | - | - | - | - | - | - | - | - | - |
| UB-180545 | C | - | - | - | - | - | - | - | D | - | - | - | - | - | - | - | - | - | - | - | - |
| UB-180546 | D | - | - | - | - | - | - | - | D | - | - | - | - | - | - | - | - | - | - | - | - |
| UB-180547 | D | - | - | - | - | - | - | - | D | - | - | - | - | - | - | - | - | - | - | - | - |
| UB-180548 | D | - | - | - | - | - | - | - | D | - | - | - | - | - | - | - | - | - | - | - | - |
| UB-180549 | D | - | - | - | - | - | - | - | D | - | - | - | - | - | - | - | - | - | - | - | - |
| UB-180550 | D | - | - | - | - | - | - | - | D | - | - | - | - | - | - | - | - | - | - | - | - |
| UB-180551 | D | - | - | - | - | - | - | - | D | - | - | - | - | - | - | - | - | - | - | - | - |
| UB-180552 | C | - | - | - | - | - | - | - | D | - | - | - | - | - | - | - | - | - | - | - | - |
| UB-180553 | C | - | - | - | - | - | - | - | D | - | - | - | - | - | - | - | - | - | - | - | - |
| UB-180554 | C | - | - | - | - | - | - | - | D | - | - | - | - | - | - | - | - | - | - | - | - |
| UB-180555 | B | - | - | - | - | - | - | - | D | - | - | - | - | - | - | - | - | - | - | - | - |
| UB-180556 | D | - | - | - | - | - | - | - | D | - | - | - | - | - | - | - | - | - | - | - | - |
| UB-180557 | C | - | - | - | - | - | - | - | D | - | - | - | - | - | - | - | - | - | - | - | - |
| UB-180558 | C | - | - | - | - | - | - | - | - | - | - | - | - | - | - | - | - | - | - | - | - |
| UB-180559 | C | D | - | - | - | - | - | - | - | - | - | - | - | - | - | - | - | - | - | - | - |
| UB-180560 | C | - | - | - | - | - | - | - | D | - | - | - | - | - | - | - | - | - | - | - | - |
| UB-180561 | D | - | - | - | - | - | - | - | D | - | - | - | - | - | - | - | - | - | - | - | - |
| UB-180562 | D | - | - | - | - | - | - | - | D | - | - | - | - | - | - | - | - | - | - | - | - |
| UB-180563 | C | - | - | - | - | - | - | - | D | - | - | - | - | - | - | - | - | - | - | - | - |
| UB-180564 | C | - | - | - | - | - | - | - | D | - | - | - | - | - | - | - | - | - | - | - | - |
| UB-180565 | B | - | - | - | - | - | - | - | D | - | - | - | - | - | - | - | - | - | - | - | B |
| UB-180566 | A | C | D | - | - | - | - | - | - | - | - | - | - | - | - | - | - | A | - | A | - |

| | | | | | | | | | | | | | | | | | | | | | |
|---|---|---|---|---|---|---|---|---|---|---|---|---|---|---|---|---|---|---|---|---|---|
| UB-180567 | B | - | - | - | - | - | - | - | - | - | - | - | - | - | - | - | - | D | - | D | - |
| UB-180568 | B | - | - | - | - | - | - | - | - | - | - | - | - | - | - | - | - | - | - | - | - |
| UB-180569 | B | - | - | - | - | - | - | - | - | - | - | - | - | - | - | - | - | - | - | - | - |
| UB-180570 | D | - | - | - | - | - | - | - | - | - | - | - | - | - | - | - | - | - | - | - | - |
| UB-180571 | C | - | - | - | - | - | - | - | - | - | - | - | - | - | - | - | - | - | - | - | - |
| UB-180572 | D | D | - | - | - | - | - | - | - | - | - | - | - | - | - | - | - | - | - | - | - |
| UB-180573 | D | D | - | - | - | - | - | - | - | - | - | - | - | - | - | - | - | - | - | - | - |
| UB-180574 | D | D | - | - | - | - | - | - | - | - | - | - | - | - | - | - | - | - | - | - | - |
| UB-180575 | B | D | - | - | - | - | - | - | - | - | - | - | - | - | - | - | - | - | - | - | - |
| UB-180576 | C | - | - | - | - | - | - | - | - | - | - | - | - | - | - | - | - | - | - | - | - |
| UB-180577 | D | D | - | - | - | - | - | - | - | - | - | - | - | - | - | - | - | - | - | - | - |
| UB-180578 | D | - | - | - | - | - | - | - | - | - | - | - | - | - | - | - | - | - | - | - | - |
| UB-180579 | B | - | - | - | - | - | - | - | - | - | - | - | - | - | - | - | - | C | - | C | - |
| UB-180580 | C | - | - | - | - | - | - | - | - | - | - | - | - | - | - | - | - | - | - | - | - |
| UB-180581 | B | D | - | C | - | - | - | - | - | - | D | D | - | - | - | - | - | A | - | - | - |
| UB-180582 | B | D | - | - | - | - | - | - | - | - | - | - | - | - | - | - | - | - | - | - | - |
| UB-180583 | C | - | - | - | - | - | - | - | - | - | - | - | - | - | - | - | - | - | - | - | - |
| UB-180584 | C | - | - | - | - | - | - | - | - | - | - | - | - | - | - | - | - | - | - | - | - |
| UB-180585 | D | - | - | - | - | - | - | - | - | - | - | - | - | - | - | - | - | - | - | - | - |
| UB-180586 | D | D | - | - | - | - | - | - | - | - | - | - | - | - | - | - | - | - | - | - | - |
| UB-180587 | D | - | - | - | - | - | - | - | - | - | - | - | - | - | - | - | - | - | - | - | - |
| UB-180588 | D | D | - | - | - | - | - | - | - | - | - | - | - | - | - | - | - | - | - | - | - |
| UB-180589 | D | - | - | - | - | - | - | - | - | - | - | - | - | - | - | - | - | - | - | - | - |
| UB-180590 | D | D | - | - | - | - | - | - | - | - | - | - | - | - | - | - | - | - | - | - | - |
| UB-180591 | C | - | - | - | - | - | - | - | - | - | - | - | - | - | - | - | - | - | - | - | - |
| UB-180592 | C | - | - | - | - | - | - | - | - | - | - | - | - | - | - | - | - | - | - | - | - |
| UB-180593 | C | - | - | - | - | - | - | - | - | - | - | - | - | - | - | - | - | - | - | - | - |
| UB-180594 | - | - | - | - | - | - | - | - | - | - | - | - | - | - | - | - | - | D | - | D | - |
| UB-180595 | D | - | - | - | - | - | - | - | - | - | - | - | - | - | - | - | - | - | - | - | - |
| UB-180596 | B | - | - | - | - | - | - | - | - | - | - | - | - | - | - | - | - | - | - | - | - |
| UB-180597 | D | - | - | - | - | - | - | - | - | - | - | - | - | - | - | - | - | - | - | - | - |
| UB-180598 | - | - | - | - | - | - | - | - | - | - | - | - | - | - | - | - | - | - | - | - | - |
| UB-180599 | D | - | - | - | - | - | - | - | - | - | - | - | - | - | - | - | - | - | - | - | - |
| UB-180600 | C | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180601 | - | - | - | - | - | - | - | - | - | - | - | - | - | - | - | - | - | D | - | D | - |
| UB-180602 | - | - | - | - | - | - | - | - | - | - | - | - | - | - | - | - | - | D | - | D | - |
| UB-180603 | C | - | - | - | - | - | - | - | - | - | - | - | - | - | - | - | - | - | - | - | - |
| UB-180604 | C | - | - | - | - | - | - | - | - | - | - | - | - | - | - | - | - | - | - | - | - |
| UB-180605 | B | - | C | - | C | C | - | - | - | - | - | - | C | - | - | - | - | - | - | - | - |
| UB-180606 | - | - | - | - | - | - | - | - | - | - | - | - | - | - | - | - | - | - | - | - | - |
| UB-180607 | B | - | - | - | - | - | - | - | - | - | - | - | - | - | - | - | - | - | - | - | - |
| UB-180608 | C | - | - | - | - | - | - | - | - | - | - | - | - | - | - | - | - | - | - | - | - |
| UB-180609 | B | - | - | - | - | - | - | - | - | - | - | - | - | - | - | - | - | - | - | - | - |
| UB-180610 | B | D | - | - | - | - | - | - | - | - | - | - | - | - | - | - | - | - | - | - | - |
| UB-180611 | B | D | - | - | - | - | - | - | - | - | - | - | - | - | - | - | - | - | - | - | - |
| UB-180612 | C | - | - | - | - | - | - | - | - | - | - | - | - | - | - | - | - | - | - | - | - |
| UB-180613 | C | - | - | - | - | - | - | - | - | - | - | - | - | - | - | - | - | - | - | - | - |
| UB-180614 | D | - | - | - | - | - | - | - | - | - | - | - | - | - | - | - | - | - | - | - | - |
| UB-180615 | B | - | - | - | - | - | - | - | - | - | - | - | - | - | - | - | - | - | - | - | - |
| UB-180616 | D | D | - | - | - | - | - | - | - | - | - | - | - | - | - | - | - | - | - | - | - |
| UB-180617 | D | D | - | - | - | - | - | - | - | - | - | - | - | - | - | - | - | - | - | - | - |
| UB-180618 | D | - | - | - | - | - | - | - | - | - | - | - | - | - | - | - | - | - | - | - | - |
| UB-180619 | D | - | - | - | - | - | - | - | - | - | - | - | - | - | - | - | - | - | - | - | - |
| UB-180621 | D | - | - | - | - | - | - | - | - | - | - | - | - | - | - | - | - | - | - | - | - |
| UB-180622 | B | - | - | - | - | - | - | - | - | - | - | - | - | - | - | - | - | - | - | - | - |
| UB-180623 | D | - | - | - | - | - | - | - | - | - | - | - | - | - | - | - | - | - | - | - | - |
| UB-180624 | B | - | - | - | - | - | - | - | - | - | - | - | - | - | - | - | - | - | A | - | - |
| UB-180625 | B | D | D | - | - | - | - | - | - | - | - | - | - | - | - | - | A | - | A | - | - |
| UB-180626 | B | D | D | - | - | - | - | - | - | - | - | - | - | - | - | - | - | - | - | - | - |
| UB-180627 | B | D | D | - | - | - | - | - | - | - | - | - | - | - | - | - | C | - | A | - | - |
| UB-180628 | B | D | D | - | - | - | - | - | - | - | - | - | - | - | - | - | - | - | - | - | - |
| UB-180629 | C | D | - | - | - | - | - | - | - | - | - | - | - | - | - | - | - | - | - | - | - |
| UB-180630 | C | D | - | - | - | - | - | - | - | - | - | - | - | - | - | - | - | - | - | - | - |
| UB-180631 | B | - | - | - | - | - | - | - | - | - | - | - | - | - | - | - | B | - | A | - | - |
| UB-180632 | B | - | - | - | - | - | - | - | - | - | - | - | - | - | - | - | - | A | - | A | - |
| UB-180633 | B | D | D | C | C | C | C | C | C | - | D | D | - | - | - | - | B | C | A | A | B |
| UB-180634 | A | D | D | C | C | C | C | C | C | - | - | - | - | - | - | - | - | - | - | - | - |
| UB-180635 | - | D | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180636 | B | - | - | - | - | - | - | - | - | - | - | - | - | - | - | - | - | B | - | B | B |
| UB-180637 | B | D | D | - | - | - | - | - | - | - | - | - | - | - | - | - | - | - | - | - | - |
| UB-180638 | B | D | D | - | - | - | - | - | - | - | - | - | - | - | - | - | - | - | - | - | - |
| UB-180639 | B | D | D | - | - | - | - | - | - | - | - | - | - | - | - | - | - | - | - | - | - |
| UB-180640 | C | D | - | - | - | - | - | - | - | - | - | - | - | - | - | - | - | - | - | - | - |
| UB-180641 | C | D | - | - | - | - | - | - | - | - | - | - | - | - | - | - | - | - | - | - | - |
| UB-180642 | C | D | - | - | - | - | - | - | - | - | - | - | - | - | - | - | - | - | - | - | - |
| UB-180643 | B | C | D | - | - | - | - | - | - | - | - | - | - | - | - | - | - | - | - | - | - |
| UB-180644 | B | D | D | - | - | - | - | - | - | - | - | - | - | - | - | - | - | - | - | - | - |
| UB-180645 | B | C | D | - | - | - | - | - | - | - | - | - | - | - | - | - | - | - | - | - | - |
| UB-180646 | B | D | - | - | - | - | - | - | - | - | - | - | - | - | - | - | - | - | - | - | - |
| UB-180647 | B | D | - | - | - | - | - | - | - | - | - | - | - | - | - | - | B | - | A | - | - |
| UB-180648 | - | D | - | - | - | - | - | - | - | - | - | - | - | - | - | - | - | - | - | - | - |
| UB-180649 | B | - | - | - | - | - | - | - | - | - | - | - | - | - | - | - | - | - | - | - | - |
| UB-180650 | - | - | - | - | - | - | - | - | - | - | - | - | - | - | - | - | - | - | - | - | - |
| UB-180651 | - | - | - | - | - | - | - | - | - | - | - | - | - | - | - | - | - | - | - | - | - |
| UB-180652 | B | - | - | - | - | - | - | - | - | - | - | - | - | - | - | - | - | - | - | - | - |
| UB-180653 | C | - | - | - | - | - | - | - | - | - | - | - | - | - | - | - | - | - | - | - | - |
| UB-180654 | - | - | - | - | - | - | - | - | - | - | - | - | - | - | - | - | - | - | - | - | - |
| UB-180655 | B | - | - | - | - | - | - | - | - | - | D | D | - | - | - | - | - | C | - | A | B |
| UB-180656 | C | - | - | - | - | - | - | - | - | - | - | - | - | - | - | - | - | - | - | - | - |
| UB-180657 | B | - | - | - | - | - | - | - | - | - | - | - | - | - | - | - | - | - | - | - | - |
| UB-180658 | B | C | D | C | C | C | C | C | C | - | - | - | - | - | - | - | - | B | - | B | - |
| UB-180659 | B | - | - | - | - | - | - | - | - | - | - | - | - | - | - | - | C | - | A | - | - |
| UB-180660 | B | C | D | C | C | C | C | C | C | - | D | D | - | - | - | - | - | A | - | B | B |
| UB-180661 | - | - | - | - | - | - | - | - | - | - | - | - | - | - | - | - | - | - | - | - | - |
| UB-180662 | B | - | - | - | - | - | - | - | - | - | - | - | - | - | - | - | - | - | - | - | - |
| UB-180663 | B | - | - | - | - | - | - | - | - | - | - | - | - | - | - | - | A | A | A | A | B |
| UB-180664 | B | - | - | - | - | - | - | - | - | - | - | - | - | - | - | - | - | - | - | - | - |
| UB-180665 | - | - | - | - | - | - | - | - | - | - | - | - | - | - | - | - | - | - | - | - | - |
| UB-180666 | B | - | - | - | - | - | - | - | - | - | - | - | - | - | - | - | B | - | A | - | - |
| UB-180667 | B | - | C | C | C | C | C | - | - | C | - | - | - | - | - | - | A | - | A | - | B |
| UB-180668 | - | - | - | - | - | - | - | - | - | - | - | - | - | - | - | - | - | - | - | - | - |
| UB-180669 | A | C | - | - | - | - | - | - | - | - | - | - | - | - | - | - | - | - | - | - | D |

| | | | | | | | | | | | | | | | | | | | | |
|---|---|---|---|---|---|---|---|---|---|---|---|---|---|---|---|---|---|---|---|---|
| UB-180670 | C | - | - | - | - | - | - | - | - | - | - | - | - | - | - | - | - | - | - | - | - |
| UB-180671 | C | - | - | - | - | - | - | - | - | - | - | - | - | - | - | - | - | - | - | - | - |
| UB-180672 | C | - | - | - | - | - | - | - | - | - | - | - | - | - | - | - | - | - | - | - | - |
| UB-180673 | B | - | - | - | - | - | - | - | - | - | - | - | - | - | - | - | - | B | - | B | - |
| UB-180674 | A | - | - | - | - | - | - | - | - | - | - | - | - | - | - | - | - | A | - | A | - |
| UB-180675 | B | - | - | - | - | - | - | - | - | - | D | D | - | - | - | - | - | A | - | A | B |
| UB-180676 | B | - | - | - | - | - | - | - | - | - | - | - | - | - | - | - | - | - | - | - | - |
| UB-180677 | C | - | - | - | - | - | - | - | - | - | - | - | - | - | - | - | - | - | - | - | - |
| UB-180678 | B | - | C | C | C | C | C | - | - | C | C | C | - | - | - | - | - | C | - | A | B |
| UB-180679 | B | - | - | - | - | - | - | - | - | - | - | - | - | - | - | - | - | - | - | - | - |
| UB-180680 | B | - | C | C | C | C | C | - | - | C | D | D | C | - | - | - | B | A | A | A | B |
| UB-180681 | A | - | C | C | C | C | C | - | - | C | - | - | - | - | - | - | - | C | - | A | B |
| UB-180682 | B | - | - | - | - | - | - | - | - | - | - | - | - | - | - | - | - | - | - | - | - |
| UB-180683 | D | - | - | - | - | - | - | - | - | - | - | - | - | - | - | - | - | - | - | - | - |
| UB-180684 | B | - | - | - | - | - | - | - | - | - | - | - | - | - | - | - | - | - | - | - | - |
| UB-180685 | B | - | - | - | - | - | - | - | - | - | - | - | - | - | - | - | - | - | - | - | - |
| UB-180686 | B | - | - | - | - | - | - | - | - | - | - | - | - | - | - | - | - | A | - | A | B |
| UB-180687 | B | - | - | - | - | - | - | - | - | - | - | - | - | - | - | - | - | - | - | - | - |
| UB-180688 | B | - | - | - | - | - | - | - | - | - | - | - | - | - | - | - | - | - | - | - | - |
| UB-180689 | B | - | - | - | - | - | - | - | - | - | - | - | - | - | - | - | - | - | - | - | - |
| UB-180690 | B | - | - | - | - | - | - | - | - | - | - | - | - | - | - | - | - | - | - | - | - |
| UB-180691 | B | - | - | - | C | - | - | - | - | - | - | - | - | - | - | - | - | - | - | - | - |
| UB-180692 | C | - | - | - | - | - | - | - | - | - | - | - | - | - | - | - | - | - | - | - | - |
| UB-180693 | B | - | - | - | - | - | - | - | - | - | - | - | - | - | - | - | - | - | - | - | - |
| UB-180694 | C | - | - | - | - | - | - | - | - | - | - | - | - | - | - | - | - | - | - | - | - |
| UB-180695 | B | - | - | - | - | - | - | - | - | - | - | - | - | - | - | - | - | - | - | - | - |
| UB-180696 | B | - | - | - | - | - | - | - | - | - | - | - | - | - | - | - | - | - | - | - | - |
| UB-180697 | C | - | - | - | - | - | - | - | - | - | - | - | - | - | - | - | - | - | - | - | - |
| UB-180698 | B | - | - | - | - | - | - | - | - | - | - | - | - | - | - | - | - | - | - | - | - |
| UB-180699 | B | - | C | C | C | C | D | C | C | C | - | - | - | - | - | - | A | - | A | - | B |
| UB-180700 | C | - | - | - | - | - | - | - | - | - | - | - | - | - | - | - | - | - | - | - | - |
| UB-180701 | A | C | C | C | C | C | C | C | C | C | D | D | C | C | C | C | A | B | A | A | B |
| UB-180702 | B | - | - | - | - | - | - | - | - | - | - | - | - | - | - | - | - | - | - | - | - |
| UB-180703 | B | - | - | - | - | - | - | - | - | - | - | - | - | - | - | - | B | - | A | - | - |

| | | | | | | | | | | | | | | | | | | | | | |
|---|---|---|---|---|---|---|---|---|---|---|---|---|---|---|---|---|---|---|---|---|---|
| UB-180704 | A | - | - | - | - | - | - | - | - | - | C | C | - | - | - | - | B | B | A | A | - |
| UB-180705 | B | - | - | - | - | - | - | - | - | - | - | - | - | - | - | - | - | - | - | - | - |
| UB-180706 | C | - | - | - | - | - | - | - | - | - | - | - | - | - | - | - | - | - | - | - | - |
| UB-180707 | B | - | - | - | C | - | - | - | - | - | - | - | - | - | - | - | - | - | - | - | - |
| UB-180708 | B | - | - | - | - | - | - | - | - | - | - | - | - | - | - | - | - | - | - | - | - |
| UB-180709 | C | - | - | - | - | - | - | - | - | - | - | - | - | - | - | - | - | - | - | - | - |
| UB-180710 | B | - | - | - | - | - | - | - | - | - | - | - | - | - | - | - | - | - | - | - | - |
| UB-180711 | A | C | C | C | C | C | C | C | C | C | C | C | C | C | C | C | A | B | A | A | B |
| UB-180712 | B | - | - | - | - | - | - | - | - | - | - | - | - | - | - | - | - | - | - | - | - |
| UB-180713 | B | - | - | - | - | - | - | - | - | - | - | - | - | - | - | - | - | - | - | - | - |
| UB-180714 | B | - | - | - | - | - | - | - | - | - | C | C | - | - | - | - | A | B | A | A | - |
| UB-180715 | B | - | - | - | - | - | - | - | - | - | - | - | - | - | - | - | - | - | - | - | - |
| UB-180716 | A | - | - | - | - | - | - | - | - | - | - | - | - | - | - | - | - | - | - | - | - |
| UB-180717 | C | - | - | - | - | - | - | - | - | - | - | - | - | - | - | - | B | - | B | - | - |
| UB-180718 | C | - | - | - | - | - | - | - | - | - | - | - | - | - | - | - | C | - | C | - | - |
| UB-180719 | B | - | - | - | C | - | - | - | - | - | - | - | - | - | - | - | A | B | A | A | - |
| UB-180720 | A | C | C | C | C | C | C | C | C | C | - | - | - | - | - | - | A | - | A | - | B |
| UB-180721 | B | - | - | - | - | - | - | - | - | - | - | - | - | - | - | - | - | - | - | - | - |
| UB-180722 | B | - | - | - | - | - | - | - | - | - | - | - | - | - | - | - | - | - | - | - | - |
| UB-180723 | C | - | - | - | - | - | - | - | - | - | - | - | - | - | - | - | - | - | - | - | - |
| UB-180724 | D | - | - | - | - | - | - | - | - | - | D | D | - | - | - | - | - | A | - | - | - |
| UB-180725 | B | C | C | C | C | C | C | C | C | - | - | - | C | C | C | C | A | A | A | A | - |
| UB-180726 | C | - | - | - | - | - | - | - | - | - | - | - | - | - | - | - | - | - | - | - | - |
| UB-180727 | C | - | - | D | - | - | - | - | - | - | - | - | - | - | - | - | - | - | - | - | - |
| UB-180728 | C | - | - | - | - | - | - | - | - | - | - | - | - | - | - | - | - | - | - | - | - |
| UB-180729 | C | - | - | - | - | - | - | - | - | - | - | - | - | - | - | - | - | - | - | - | - |
| UB-180730 | B | C | - | D | - | - | - | - | - | - | D | D | - | - | - | - | - | A | - | - | - |
| UB-180731 | C | - | - | - | - | - | - | - | - | - | - | - | - | - | - | - | - | - | - | - | - |
| UB-180732 | C | - | - | - | - | - | - | - | - | - | - | - | - | - | - | - | - | - | - | - | - |
| UB-180733 | - | - | - | - | - | - | - | - | - | - | - | - | - | - | - | - | - | - | - | - | B |
| UB-180734 | - | - | - | - | - | - | - | - | - | - | - | - | - | - | - | - | - | - | - | - | - |
| UB-180735 | C | - | - | - | - | - | - | - | - | - | - | - | - | - | - | - | - | - | - | - | - |
| UB-180736 | C | - | - | - | - | - | - | - | - | - | - | - | - | - | - | - | - | - | - | - | - |
| UB-180738 | C | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180739 | D | - | - | - | - | - | - | - | - | - | - | - | - | - | - | - | - | - | - | - | - |
| UB-180740 | C | - | - | - | - | - | - | - | - | - | - | - | - | - | - | - | - | - | - | - | - |
| UB-180741 | D | - | - | - | - | - | - | - | - | - | - | - | - | - | - | - | - | - | - | - | - |
| UB-180742 | C | - | - | - | - | - | - | - | - | - | - | - | - | - | - | - | - | - | - | - | - |
| UB-180743 | C | - | - | - | - | - | - | - | - | - | - | - | - | - | - | - | - | - | - | - | - |
| UB-180744 | C | - | - | - | - | - | - | - | - | - | - | - | - | - | - | - | - | - | - | - | - |
| UB-180745 | A | - | - | - | - | - | - | - | - | - | - | - | - | - | - | - | A | D | A | A | - |
| UB-180746 | B | - | - | - | - | - | - | - | - | - | - | - | - | - | - | - | - | - | - | - | - |
| UB-180747 | B | - | - | - | - | - | - | - | - | - | - | - | - | - | - | - | - | - | - | - | - |
| UB-180748 | D | - | - | - | - | - | - | - | - | - | - | - | - | - | - | - | - | - | - | - | - |
| UB-180749 | C | - | - | - | - | - | - | - | - | - | - | - | - | - | - | - | - | - | - | - | - |
| UB-180751 | - | - | C | - | - | - | - | - | - | - | - | - | - | - | - | - | - | - | - | - | - |
| UB-180752 | C | - | - | D | - | - | - | - | - | - | D | D | - | - | - | - | - | - | A | A | - |
| UB-180753 | C | - | - | D | - | - | - | - | - | - | D | D | - | - | - | - | A | - | A | C | - |
| UB-180754 | - | - | - | - | - | - | - | - | - | - | - | - | - | - | - | - | - | - | - | - | - |
| UB-180755 | - | - | - | - | - | - | - | - | - | - | - | - | - | - | - | - | - | - | - | - | - |
| UB-180756 | - | - | - | - | - | C | - | - | - | - | - | - | - | - | - | - | - | - | - | - | - |
| UB-180757 | - | - | - | - | - | C | - | - | - | - | - | - | - | - | - | - | - | - | - | - | - |
| UB-180758 | - | - | - | - | - | C | - | - | - | - | - | - | - | - | - | - | - | - | - | - | - |
| UB-180759 | - | D | D | - | D | D | D | - | D | - | - | - | - | - | - | D | - | - | - | - | - |
| UB-180760 | - | - | - | - | - | - | - | - | - | - | - | - | - | - | - | - | - | - | - | - | - |
| UB-180761 | D | - | - | - | - | - | - | - | - | - | - | - | - | - | - | - | - | - | - | - | - |
| UB-180762 | B | - | - | - | - | - | - | - | - | - | - | - | - | - | - | - | - | - | - | - | - |
| UB-180763 | B | - | - | - | - | - | - | - | - | - | - | - | - | - | - | - | - | - | - | - | - |
| UB-180764 | A | - | - | - | - | - | - | - | - | - | - | - | - | - | - | - | - | - | - | - | - |
| UB-180765 | B | - | - | - | - | - | - | - | - | - | - | - | - | - | - | - | - | - | - | - | - |
| UB-180766 | - | - | - | - | - | - | - | - | - | - | - | - | - | - | - | - | - | - | - | - | - |
| UB-180767 | B | - | - | - | - | - | - | - | - | - | - | - | - | - | - | - | - | - | - | - | - |
| UB-180768 | C | - | - | - | - | - | - | - | - | - | - | - | - | - | - | - | - | - | - | - | - |
| UB-180769 | C | - | - | - | - | - | - | - | - | - | - | - | - | - | - | - | - | - | - | - | - |
| UB-180770 | C | - | - | - | - | - | - | - | - | - | - | - | - | - | - | - | - | - | - | - | - |
| UB-180771 | C | - | - | - | - | - | - | - | - | - | - | - | - | - | - | - | - | - | - | - | - |
| UB-180772 | D | - | - | - | - | - | - | - | - | - | - | - | - | - | - | - | - | - | - | - | - |
| UB-180773 | C | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180774 | C | - | - | - | - | - | - | - | - | - | - | - | - | - | - | - | - | - | - | - | - |
| UB-180775 | C | - | - | - | - | - | - | - | - | - | - | - | - | - | - | - | - | - | - | - | - |
| UB-180776 | C | - | - | - | - | - | - | - | - | - | - | - | - | - | - | - | - | - | - | - | - |
| UB-180777 | C | - | - | - | - | - | - | - | - | - | - | - | - | - | - | - | - | - | - | - | - |
| UB-180778 | D | - | - | - | - | - | - | - | - | - | - | - | - | - | - | - | - | - | - | - | - |
| UB-180779 | D | - | - | - | - | - | - | - | - | - | - | - | - | - | - | - | - | - | - | - | - |
| UB-180780 | D | - | - | - | - | - | - | - | - | - | - | - | - | - | - | - | - | - | - | - | - |
| UB-180781 | C | - | - | - | - | - | - | - | - | - | - | - | - | - | - | - | - | - | - | - | - |
| UB-180782 | D | - | - | - | - | - | - | - | - | - | - | - | - | - | - | - | - | - | - | - | - |
| UB-180783 | D | - | - | - | - | - | - | - | - | - | - | - | - | - | - | - | - | - | - | - | - |
| UB-180784 | D | - | - | - | - | - | - | - | - | - | - | - | - | - | - | - | - | - | - | - | - |
| UB-180785 | D | - | - | - | - | - | - | - | - | - | - | - | - | - | - | - | - | - | - | - | - |
| UB-180786 | D | - | - | - | - | - | - | - | - | - | - | - | - | - | - | - | - | - | - | - | - |
| UB-180787 | D | - | - | - | - | - | - | - | - | - | - | - | - | - | - | - | - | - | - | - | - |
| UB-180788 | D | - | - | - | - | - | - | - | - | - | - | - | - | - | - | - | - | - | - | - | - |
| UB-180789 | D | - | - | - | - | - | - | - | - | - | - | - | - | - | - | - | - | - | - | - | - |
| UB-180790 | D | - | - | - | - | - | - | - | - | - | - | - | - | - | - | - | - | - | - | - | - |
| UB-180791 | D | - | - | - | - | - | - | - | - | - | - | - | - | - | - | - | - | - | - | - | - |
| UB-180792 | C | - | - | - | - | - | - | - | - | - | - | - | - | - | - | - | - | - | - | - | - |
| UB-180793 | C | - | - | - | - | - | - | - | - | - | - | - | - | - | - | - | - | - | - | - | - |
| UB-180794 | D | - | - | - | - | - | - | - | - | - | - | - | - | - | - | - | - | - | - | - | - |
| UB-180795 | C | - | - | - | - | - | - | - | - | - | - | - | - | - | - | - | - | - | - | - | - |
| UB-180796 | D | - | - | - | - | - | - | - | - | - | - | - | - | - | - | - | - | - | - | - | - |
| UB-180797 | D | - | - | - | - | - | - | - | - | - | - | - | - | - | - | - | - | - | - | - | - |
| UB-180798 | C | - | - | - | - | - | - | - | - | - | - | - | - | - | - | - | - | - | - | - | - |
| UB-180799 | C | - | - | - | - | - | - | - | - | - | - | - | - | - | - | - | - | - | - | - | - |
| UB-180800 | A | - | - | A | - | - | - | - | - | - | - | - | - | - | - | - | - | - | - | - | - |
| UB-180801 | C | - | - | D | - | - | - | - | - | - | - | - | - | - | - | - | - | - | - | - | - |
| UB-180802 | C | C | - | D | - | - | - | - | - | - | - | - | - | - | - | - | - | A | - | - | - |
| UB-180803 | C | - | - | - | - | - | - | - | - | - | - | - | - | - | - | - | - | - | - | - | - |
| UB-180804 | C | - | - | D | - | - | - | - | - | - | D | D | - | - | - | - | - | - | - | - | - |
| UB-180805 | B | - | - | D | - | - | - | - | - | - | - | - | - | - | - | - | - | - | - | - | - |
| UB-180806 | A | - | C | D | - | - | - | - | - | - | D | D | - | - | - | - | - | - | - | - | - |
| UB-180807 | C | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180808 | C | - | - | - | - | - | - | - | - | - | - | - | - | - | - | - | - | - | - | - | - |
| UB-180809 | C | - | - | - | - | - | - | - | - | - | - | - | - | - | - | - | - | - | - | - | - |
| UB-180810 | C | C | - | - | - | - | - | - | - | - | D | D | - | - | - | - | - | A | - | - | - |
| UB-180811 | C | - | - | - | - | - | - | - | - | - | - | - | - | - | - | - | - | - | - | - | - |
| UB-180812 | C | - | - | - | - | - | - | - | - | - | - | - | - | - | - | - | - | - | - | - | - |
| UB-180813 | B | - | - | - | - | - | - | - | - | - | - | - | - | - | - | - | - | - | - | - | - |
| UB-180814 | B | - | - | - | - | - | - | - | - | - | - | - | - | - | - | - | - | - | - | - | - |
| UB-180815 | B | - | - | - | - | - | - | - | - | - | - | - | - | - | - | - | - | - | - | - | - |
| UB-180816 | C | - | - | - | - | - | - | - | - | - | - | - | - | - | - | - | - | - | - | - | - |
| UB-180817 | C | - | - | - | - | - | - | - | - | - | - | - | - | - | - | - | - | - | - | - | - |
| UB-180818 | A | C | C | B | C | C | C | - | D | - | C | C | C | - | - | C | A | n.a. | A | A | - |
| UB-180819 | C | - | - | - | - | - | - | - | - | - | - | - | - | - | - | - | - | - | - | - | - |
| UB-180820 | B | - | - | - | - | - | - | - | - | - | - | - | - | - | - | - | - | - | - | - | - |
| UB-180821 | D | - | - | - | - | - | - | - | - | - | - | - | - | - | - | - | - | - | - | - | - |
| UB-180822 | D | - | - | - | - | - | - | - | - | - | - | - | - | - | - | - | - | - | - | - | - |
| UB-180823 | C | - | - | - | - | - | - | - | - | - | - | - | - | - | - | - | - | - | - | - | - |
| UB-180825 | A | - | - | - | - | - | - | - | - | - | - | - | - | - | - | - | - | - | - | - | - |
| UB-180826 | - | - | - | - | - | - | - | - | - | - | D | D | C | - | - | - | - | - | - | - | - |
| UB-180827 | - | - | - | - | - | - | - | - | - | - | - | - | C | - | - | - | - | - | - | - | - |
| UB-180828 | A | C | - | - | - | - | - | - | - | - | - | - | - | - | - | - | - | - | - | - | - |
| UB-180829 | - | C | - | - | C | C | C | - | C | - | D | D | - | C | - | - | - | - | - | - | - |
| UB-180830 | - | - | D | - | - | - | - | - | - | - | D | D | - | - | - | - | - | - | - | - | - |
| UB-180831 | - | - | B | - | - | - | - | - | - | - | B | B | - | - | - | - | - | - | - | - | - |
| UB-180832 | - | - | D | - | - | - | - | - | - | - | D | D | - | - | - | - | - | - | - | - | - |
| UB-180833 | B | - | - | - | - | - | - | - | - | - | - | - | - | - | - | - | - | - | - | - | - |
| UB-180834 | B | - | - | - | - | - | - | - | - | - | - | - | - | - | - | - | - | - | - | - | - |
| UB-180835 | B | - | - | - | - | - | - | - | - | - | - | - | - | - | - | - | - | - | - | - | - |
| UB-180836 | C | - | - | - | - | - | - | - | - | - | - | - | - | - | - | - | - | - | - | - | - |
| UB-180837 | C | - | - | A | - | - | - | - | - | - | - | - | - | - | - | - | - | - | - | - | - |
| UB-180838 | - | C | - | - | C | - | C | - | C | - | - | - | - | - | - | - | - | - | - | - | - |
| UB-180839 | - | C | - | - | C | - | C | - | C | - | - | - | - | - | - | - | - | - | - | - | - |
| UB-180840 | B | - | - | - | - | - | - | - | - | - | - | - | - | - | - | - | - | - | - | - | - |
| UB-180841 | - | - | - | - | - | - | - | - | - | - | - | - | - | - | - | - | - | - | - | - | - |
| UB-180842 | - | - | - | - | - | - | - | - | - | - | - | - | - | - | - | - | - | - | - | - | - |

| | | | | | | | | | | | | | | | | | | | | | |
|---|---|---|---|---|---|---|---|---|---|---|---|---|---|---|---|---|---|---|---|---|---|
| UB-180843 | A | - | A | - | A | C | - | - | - | - | - | - | A | - | - | - | - | - | - | - | - |
| UB-180844 | A | - | C | - | C | C | - | - | - | - | - | - | C | - | - | - | - | B | - | B | - |
| UB-180845 | A | - | C | - | C | C | - | - | - | - | - | - | C | - | - | - | - | - | - | A | - |
| UB-180846 | A | - | C | - | C | C | - | - | - | - | - | - | C | - | - | - | - | - | - | B | - |
| UB-180847 | C | - | C | - | C | C | - | - | - | - | - | - | C | - | - | - | - | - | - | - | - |
| UB-180848 | A | - | C | - | C | C | - | - | - | - | - | - | C | - | - | - | - | - | - | - | - |
| UB-180849 | C | - | C | - | C | C | - | - | - | - | - | - | C | - | - | - | - | - | - | - | - |
| UB-180850 | C | - | C | - | C | C | - | - | - | - | - | - | C | - | - | - | - | - | - | - | - |
| UB-180851 | C | - | C | - | C | C | - | - | - | - | - | - | C | - | - | - | - | - | - | - | - |
| UB-180852 | C | - | C | - | C | C | - | - | - | - | - | - | C | - | - | - | - | - | - | - | - |
| UB-180853 | C | - | C | - | C | C | - | - | - | - | - | - | C | - | - | - | - | - | - | - | - |
| UB-180854 | C | - | C | - | C | C | - | - | - | - | - | - | C | - | - | - | - | - | - | - | - |
| UB-180855 | C | - | C | - | C | C | - | - | - | - | - | - | C | - | - | - | - | - | - | - | - |
| UB-180856 | C | - | C | - | C | C | - | - | - | - | - | - | C | - | - | - | - | - | - | - | - |
| UB-180857 | B | - | C | - | C | C | - | - | - | - | - | - | C | - | - | - | - | - | - | - | - |

**Test Example 3 Animal Experiment**

Female BALB/c nude mice (number: 58; age: 5-6 weeks) were purchased from Beijing Weitong Lihua Laboratory Animal Technology Co., Ltd. and raised in the SPF animal room of Suzhou Shengsu New Pharmaceutical Development Co., Ltd., temperature was 20-25 °C, relative humidity was 40%-70%, light and dark light for 12 hours each; animals were free to drink and eat freely. After normal feeding for about 5 days, mice with good physical signs can be selected for this experiment after veterinary inspection. Before grouping, animals were identified at their base of tail using maker pens. After grouping, each animal was identified by ear clipping.
Human acute monocytic leukemia cells MV-4-11 were obtained from American Type Culture Collection (ATCC, cryopreserved in liquid nitrogen in our laboratory).
Under culture conditions of 5% $CO_2$ and 37 °C, MV-4-11 cells were subjected to routine cell culture in IMDM medium with 10% fetal bovine serum. Passaged with 0.25% trypsin digestion. The passage was performed according to cell growth, passaging ratio is 1:3 or 1:6. MV-4-11 cells at logarithmic growth phase were collected, the cells were resuspended in 50% PBS (pH 7.4, 0.01 M) and 50% Matrigel after counting, the concentration of cells was adjusted to $7.0 \times 10^7$ cells/mL; the cells were placed in ice box, the cell suspension was suck up using 1 mL syringe, and injected into the Right anterior axillary subcutaneous of nude mice. Each animal was inoculated with 200 μL ($14 \times 10^6$ cells/mouse) to establish MV-4-11 xenograft tumor model. The animal status was regularly observed, the tumor diameter was measured using the electronic vernier caliper, the data were entered into Excel, the tumor volume was calculated, and the tumor growth was monitored. When the tumor volume reached 100-300 $mm^3$, animals with tumors that are too large, too small, or of uncertain formation were eliminated, and tumor-bearing mice with good health and similar tumor volumes were selected, and divided into 4 groups(n = 6~8) using randomized block method. After the start of experiment, the tumor diameter was measured twice a week, the tumor volume was calculated, and the animal weight was weighed and recorded at the same time.
Calculating formula for tumor volume (TV) is as follows:

$$\text{TV (mm}^3) = l \times w^2/2$$

wherein, *l* means the long diameter of the tumor (mm); w means the short diameter (mm) of the tumor.
Before each administration the compounds were taken and prepared into dosage formulation with certain concentration.
Animal grouping and dosage regimen see **Table 2.**

**Table. 2 The dosage regimen of the pharmacodynamic experiment of the nude mouse xenograft tumor model**

| Group | Sample | Number of animals | Dose ($mg \cdot kg^{-1}$) | Dosing volume ($mL \cdot kg^{-1}$) | Route of administr ation | Dosing frequency |
|---|---|---|---|---|---|---|
| **A** | 0.01 M PBS (pH 7.4) | 6 | - | 10 | i.v. | dosing once every other 2 days |
| **B** | UB-180752(AC-252) | 6 | 10 | 10 | i.v. | dosing once every other 2 days |
| **C** | UB-180753(AC-253) | 6 | 10 | 10 | i.v. | dosing once every other 2 days |
| **D** | UB-180699(199) | 8 | 10 | 10 | i.v. | dosing once every other 1 day |
| **E** | UB-180699(199) | 8 | 10 | 10 | i.g. | dosing once every other 1 day |

During the experiment, experimenters and veterinarians need take continuous observation on the physical signs and health of the experimental animals, weigh their weight and measure the diameter of the tumor on at regular intervals.
Calculating formula for relative tumor volume (RTV) is:

$$RTV = TV_t/TV_{initial}$$

wherein $TV_{initial}$ is the tumor volume measured when grouped administration; $TV_t$ is the tumor volume at each measurement during the administration period.

Calculating formula for the relative increasing rate of tumor (%T/C) is:

$$\%T/C = 100\% \times (RTV_T/RTV_C)$$

wherein $RTV_T$ means the RTV of treatment group; $RTV_C$ means the RTV of solvent control group. Calculating formula for tumor growth inhibition rate TGI (%) is:

$$TGI = 100\% \times [1-(TV_{t(T)}-TV_{initial(T)})/( TV_{t(C)}-TV_{initial(C)})]$$

wherein $TV_{t(T)}$ means the tumor volume of the treatment group at each measurement; $TV_{initial(T)}$ means the tumor volume of the treatment group when grouped administration; $TV_{t(C)}$ means tumor volume of solvent control group at each measurment; $TV_{initial(C)}$ means the tumor volume of solvent control group when grouped administration.

Calculating formula for loss rate of animal weight is:

$$\text{Loss rate of animal weight} = 100\% \times (BW_{initial}-BW_t)/BW_{initial}$$

wherein, $BW_t$ means the animal weight at each measurement during administration; $BW_{intial}$ means the the animal weight when grouped administration.

Calculating formula for inhibit rate of tumor weight IR (%) is:

$$IR = 100\% \times (W_C-W_T)/W_C$$

wherein $W_C$ means the tumor weight of control group; $W_T$ means the tumor weight of treatment group.

Result Analysis

Referring to Figure 3, TED compound 199 was administered to MV4;11 tumor model mice at a dose of 30mpk, injected every other day, and showed tumor shrinking effect. The weight of the mice was stable, and no other abnormalities of mice were observed.

ACTED compounds AC-252 and AC-253 were administered via injection to MV4;11 tumor model mice at a dose of 10mpk every other 2 days, and all showed tumor growth inhibitory effects (64%). The weight of the mice was stable, and no other abnormalities of mice were observed.

The amount of corresponding TED molecules carried, calculated based on ACTED, is an Extremely small amount. Taking 253 as an example, the administration dose is 10 mpk, which is equivalent to 0.014 mpk after converting into dose of TED molecule 199. This dose is far below the dose of 199 itself of 30mpk. At such a small amount of administration dose, it is difficult to observe expected tumor growth inhibitory effect if TED molecule 199 is used alone. The ACTED compounds of the present invention (as shown in Table C) all show excellent anti-tumor activity and excellent pharmacokinetic properties similar to AC-252 and AC-253.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

**Claims**

1. A conjugate of formula I:

A-L1-B formula I

wherein
A is a monovalent moiety of a targeting molecule;
B is a monovalent moiety of an E3 ligase ligand;
L1 is a linker connecting A and B; and L1 has a formula II:

-X-L2-Y- formula II

wherein
when X or Y is NH or NR, X or Y and part of L2 chain to which X or Y is attached are combined together to form a 4 - 8 membered aromatic or non-aromatic heterocyclic ring; wherein R is substituted or unsubstituted C1-C10 alkyl, -(C=O)-R', (C=O)NH-R', -NH(C=O)-R', $-SO_2$-R', $-NHSO_2$-R', - $SO_2$NH-R',-SO-R', -NHSO-R', -SONH-R', $-PO_3$-R', -NHCOO-R', -COO-R', or -NH-CO-NH- R', -NH-CO-O-R', or -X'-L3-Z; wherein L3 is linker, and Z is a polypeptide element or target molecule T; wherein R' is selected from the group consisting of H, substituted or unsubstituted C1-C6 alkyl, substituted or unsubstituted C3-C8 cycloalkyl, amino protecting group, and -X'-L3-Z;
or
each X and Y is independently selected from the group consisting of-O-, -S-, -NH-, -NR-, -(C=O)NH-, -NH(C=O)-, $-SO_2$-, $-NHSO_2$-, - $SO_2$NH-, -SO-, -NHSO-, -SONH-, $-PO_3$- -NHCOO-, -COO-,$-NHCOCH_2O$-, and -NH-CO-NH-; wherein R is substituted or unsubstituted C1-C10 alkyl, -(C=O)-R',-(C=O)NH-R', -NH(C=O)-R', $-SO_2$-R', $-NHSO_2$-R', $-SO_2$NH-R', -SO-R', -NHSO-R', -SONH-R', $-PO_3$-R',-NHCOO-R', -COO-R' or -NH-CO-NH-R', or -X'-L3-Z; wherein L3 is linker, and Z is a peptide element or target molecule T; wherein R' is selected from the group consisting of H, substituted or unsubstituted C1-C6 alkyl, substituted or unsubstituted C3-C8 cycloalkyl, protecting group, and -X'-L3-Z;
X' is selected from the group consisting of -O-, -S-, -NH-, -(C=O)NH-, -NH(C=O)-, $-SO_2$-, $-NHSO_2$-,$-SO_2$NH-, -SO-, -NHSO-, -SONH-, $-PO_3$-, -NHCOO-, -COO-, $-COOCH_2$-, and -NH-CO-NH-;
each L2 and L3 is independently linker selected from the group consisted of

(a) substituted or unsubstituted 5-20 membered carbon chain;
(b) substituted or unsubstituted 5-20 membered heterocarbon chain containing 1 to 5 heteroatoms selected from N, O and S;
(c) substituted or unsubstituted 5-20 membered carbon chain; and 1 to 4 chain atoms in the chain are replaced with heterocyclic ring selected from the group consisting of 4-10 membered saturated heterocyclic ring containing 1- 4 heteroatoms selected from N, O and S, and 5-10 membered partially unsaturated or fully unsaturated heterocyclic ring containing 1-4 heteroatoms selected from N, O and S;
(d) substituted or unsubstituted 5-20 membered heterocarbon chain containing 1 to 5 heteroatoms selected from N, O and S; and 1 to 4 chain atoms in the chain are replaced with heterocyclic ring selected from the group consisting of 4-10 membered saturated heterocyclic ring containing 1-4 heteroatoms selected from N, O and S, and 5-10 membered partially unsaturated or fully unsaturated heterocyclic ring containing 1-4 heteroatoms selected from N, O and S;

wherein the "substituted" means one or more (preferably, 1, 2, 3 or 4) hydrogen atoms in the group is substituted with substituent selected from the group consisting of C1-C10 alkyl, C3-C8 cycloalkyl, -COOH, -OH, -SH, $-NH_2$, -NHR, $-N(R_1)R_2$, -Z, -X'-L3-Z,

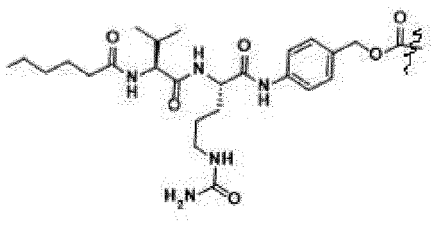
,

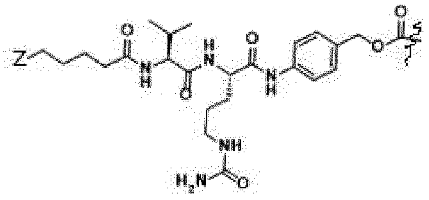,
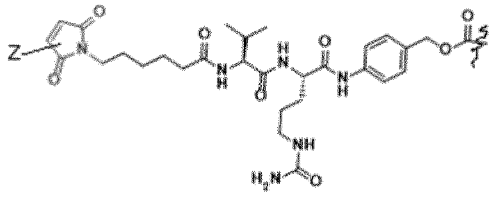,

and

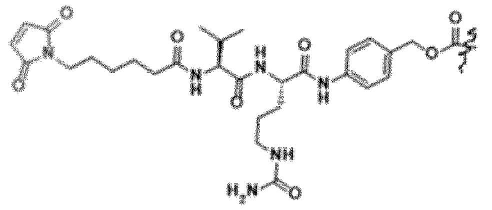;

wherein Z is a polypeptide element or target molecule T;
wherein when the substituent is -Z, -Z is located only on N atom; wherein each $R_1$ and $R_2$ is indenpedently H, C1-C6 alkyl, C3-C8 cycloalkyl, amino protecting group, -X'-L3-Z,

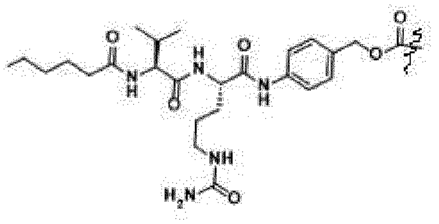,
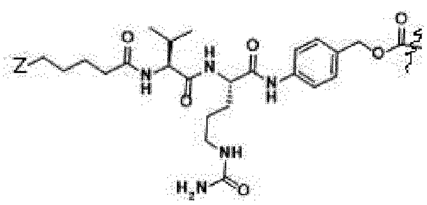,

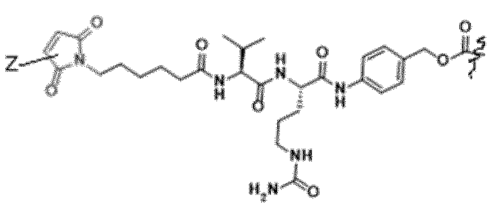, or
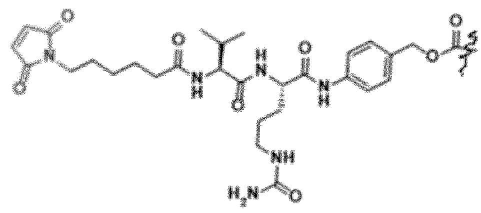;

wherein R is substituted or unsubstituted C1-C10 alkyl, -(C=O)-R', (C=O)NH-R', -NH(C=O)-R', $-SO_2$-R', $-NHSO_2$-R',$-SO_2$NH-R', -SO-R', -NHSO-R', -SONH-R', $-PO_3$-R', -NHCOO-R', -COO-R', or -NH-CO-NH-R', -NH-COO-R', or -X'-L3-Z; wherein L3 is linker, and Z is a polypeptide element or target molecule T; wherein R' is selected from the group consisting of H, substituted or unsubstituted C1-C6 alkyl, substituted or unsubstituted C3-C8 cycloalkyl, protecting group, and X'-L3-Z.

**2.** The conjugate of Claim 1, wherein L2 is linker selected from the group consisted of

(a) substituted 5-20 membered carbon chain;
(b) substituted 5-20 membered heterocarbon chain containing 1 to 5 heteroatoms selected from N, O and S;
(c) substituted 5-20 membered carbon chain; and 1 to 4 chain atoms in the chain are replaced with heterocyclic ring selected from the group consisting of 4-10 membered saturated heterocyclic ring containing 1-4 heteroatoms selected from N, O and S, and 5-10 membered partially unsaturated or fully unsaturated heterocyclic ring containing 1-4 heteroatoms selected from N, O and S;
(d) substituted 5-20 membered heterocarbon chain containing 1 to 5 heteroatoms selected from N, O and S; and 1 to 4 chain atoms in the chain are replaced with heterocyclic ring selected from the group consisting of 4-10 membered saturated heterocyclic ring containing 1-4 heteroatoms selected from N, O and S, and 5-10

membered partially unsaturated or fully unsaturated heterocyclic rings containing 1-4 heteroatoms selected from N, O and S;

wherein, the "substituted" means at least one hydrogen atom in the group is substituted with substituent selected from the group consisting of -NHR, $-N(R_1)R_2$, -Z, -X-L3-Z,

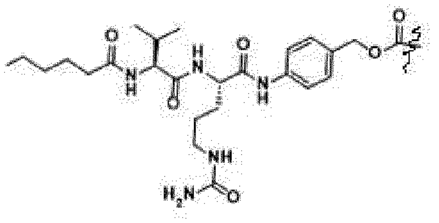
,
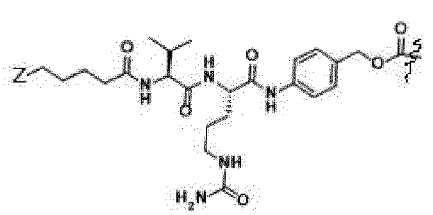
,

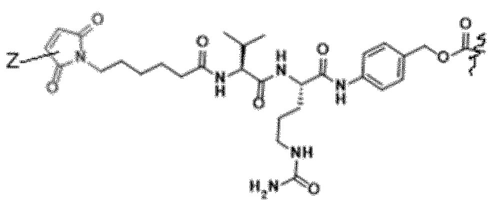
, and
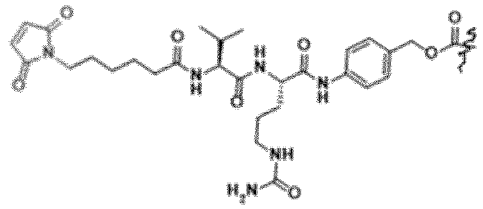
;

and when the substituent is -Z, -Z is located only on N atom;
and if the remaining hydrogen atoms are substituted, the definition of substituted is the same as that of claim 1.

**3.** The conjugate of Claim 1, wherein L2 and/or L3 are linkers selected from the group consisted of

(a) substituted or unsubstituted $-(CH_2)s-$;
(b) substituted or unsubstituted $-(CH_2)s-$, wherein 1-5 of $-CH_2-$ are replaced with O;
(c) substituted or unsubstituted $-(CH_2)s-$, wherein 1-2 of $-CH_2-$ are replaced with NR and optionally 1-2 of $-CH_2-$ are replaced with O; wherein R is H, amino protecting group or X'-L3-Z;
(d) substituted or unsubstituted $-(CH_2)s-$, wherein 1-4 of $-CH_2-$ are replaced with 5 membered unsaturated heterocyclic ring containing 1-4 heteroatoms selected from N, O, S and P;
(e) substituted or unsubstituted $-(CH_2)s-$, wherein 1-4 of $-CH_2-$ are replaced with 4-6 membered saturated heterocyclic ring containing 1-4 heteroatoms selected from N, O, S and P;
(f) substituted or unsubstituted $-(CH_2)s-$, wherein 1-4 of $-CH_2-$ are replaced with O and 4-6 membered saturated heterocyclic ring containing 1-4 heteroatoms selected from N, O, S and P;
(g) divalent 4-6 membered heterocyclic group containing 1-2 nitrogen atoms;

wherein s is an integer of 5-20.

**4.** The conjugate of Claim 1, wherein when X is NH or NR, X and part of L2 chain to which X is attached are combined together to form a 4-8 membered heterocyclic ring,

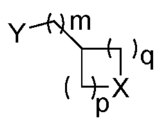
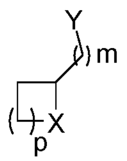

wherein Y and R are defined as above; m is an integer of 1-15; each p and q is indenpendently 0, 1, 2, or 3, and $p + q \geq 2$.

**5.** The conjugate of Claim 1, wherein L1 is selected from the group consisted of

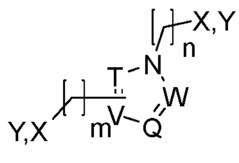 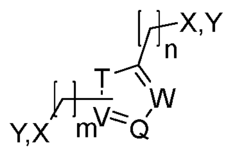 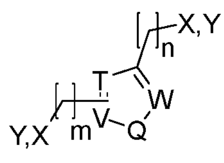 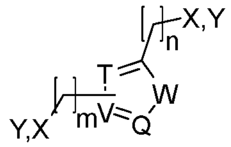

in the formula, "Y, X" means this terminal is X or Y; with the proviso that one end is X, and the other end is Y; X and Y are defined as above, each m and n is independently an integer of 1-15; each T, V, Q and W is independently CH, C=O, S, O, NH or NR; R is C1-C10 alkyl, -(C=O)R'-,-(C=O)NH-R', -NH(C=O)-R', $-SO_2$-R', $-NHSO_2$-R', - $SO_2NH$-R', -SO-R', -NHSO-R', -SONH- R', $-PO_3$-R',-NHCOO-R', -COO-R', -NH-CO-NH-R', -NH-CO-O-R', or N-X'-L3-Z ; wherein L3 is linker, and Z is a polypeptide element or target molecule T; R' and X' are defined as in Claim 1 or 2;
or, L1 is

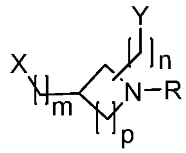

wherein X, Y and R are defined as above;
each m and n is independently an integer of 1-15; p is 1, 2 or 3.

**6.** The conjugate of Claim 1, wherein L1 is selected from the group consisted of

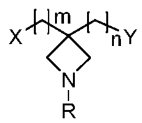 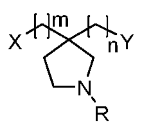 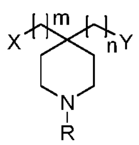 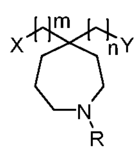 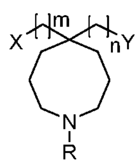

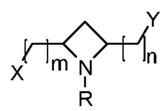 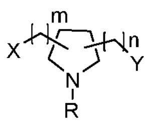 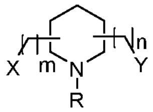 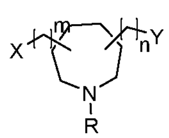 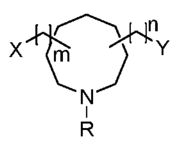

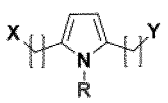 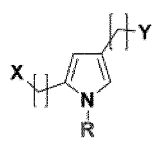 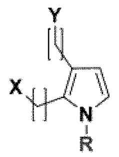 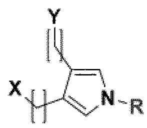

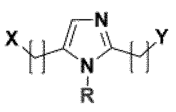 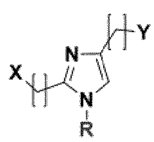 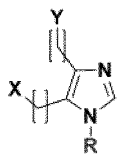

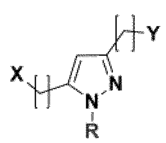 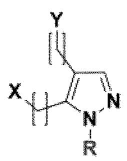 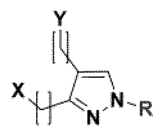

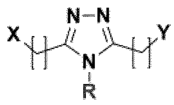 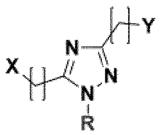 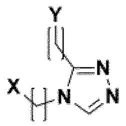 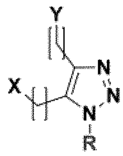

**7.** The conjugate of Claim 1, whereinL1 is selected from the group consisting of

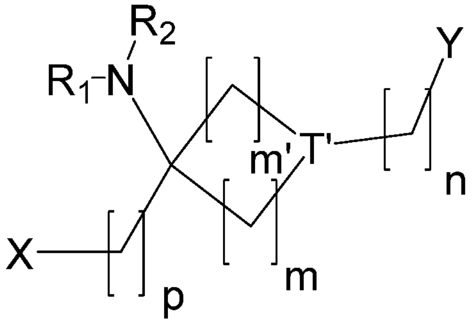 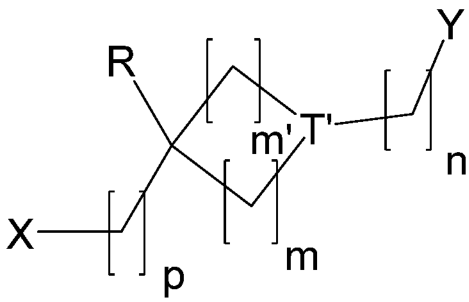 ;

wherein each m' and m is independently 0, 1, 2, 3, 4, or 5(preferably 0, 1, 2, or 3), and m' and m are not 0 at the same time;
each p and n is independently an integer of 1-15; T' is N or $CR_b$; wherein $R_b$ is a group selected from the group consisting of H, substituted or unsubstituted C1-C6 alkyl;
R, $R_1$ and $R_2$ are as defined in claim 1 or 2;
or, L1 is

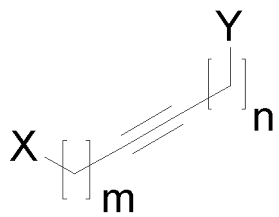

wherein each m and n is independently an integer of 1-15.

**8.** The conjugate of Claim 1 wherein L1 is selected from the group consisting of

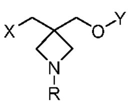 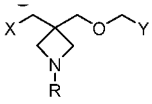 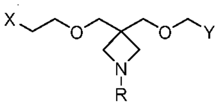 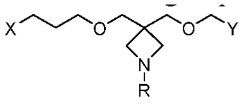 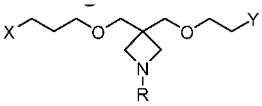

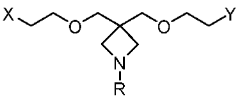 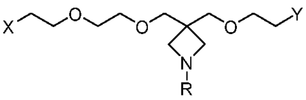 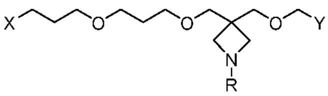 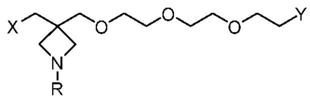

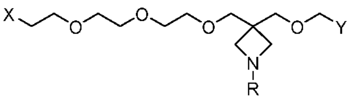 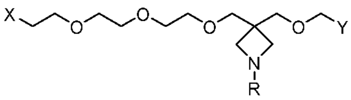 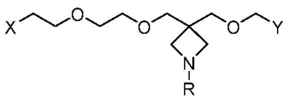

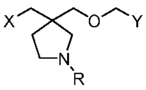 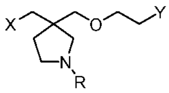 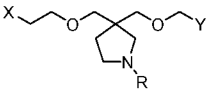 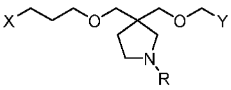 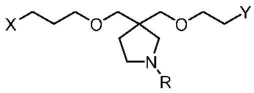

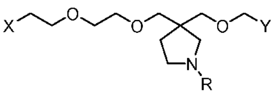 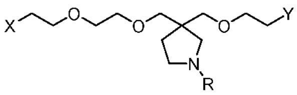 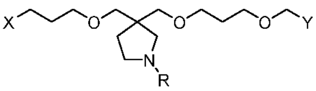 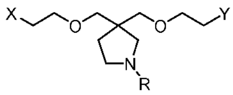

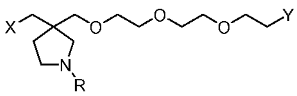 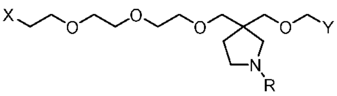 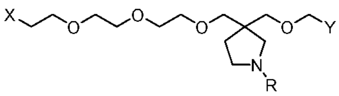

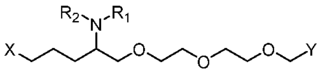
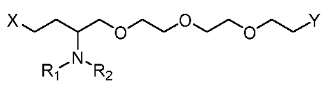
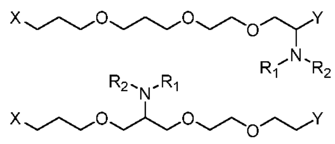
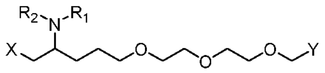
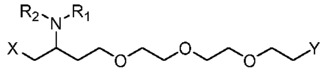
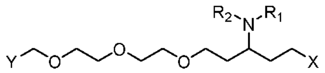
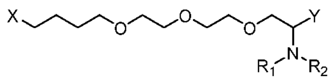
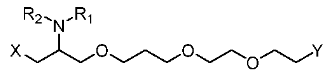
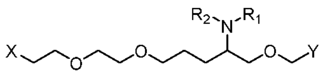
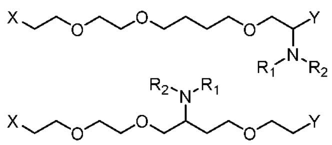
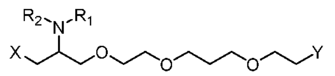
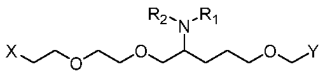
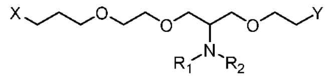
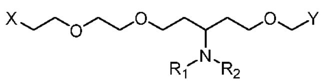
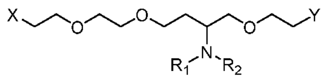
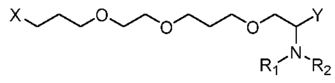
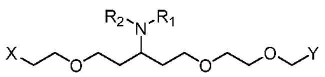
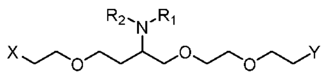
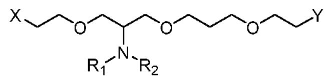
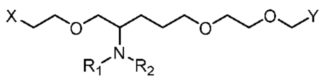
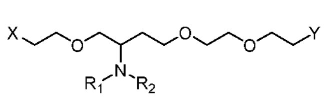
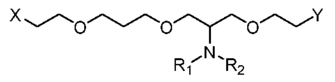
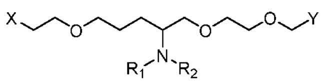
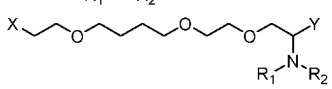
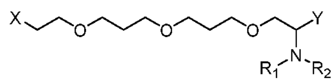
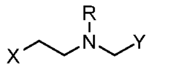
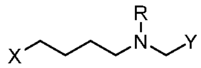
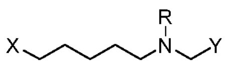
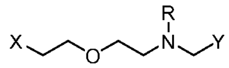
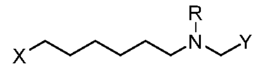
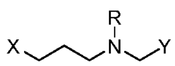
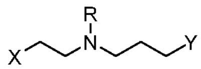
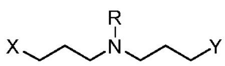
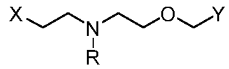
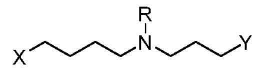
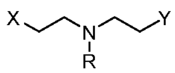
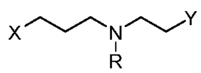
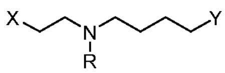
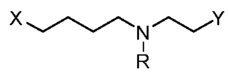
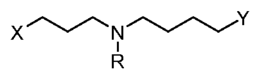

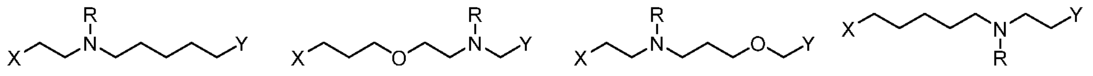

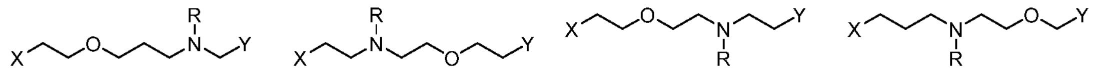

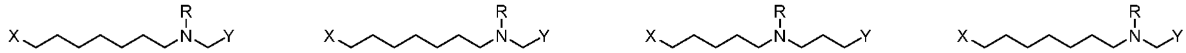

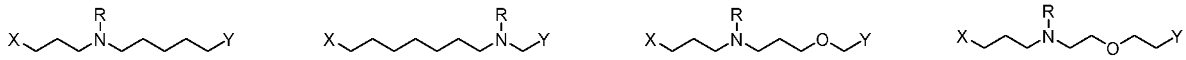

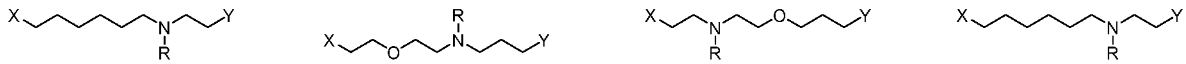

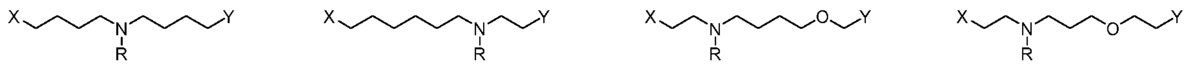

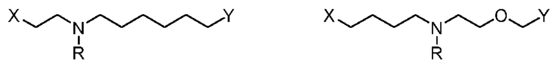

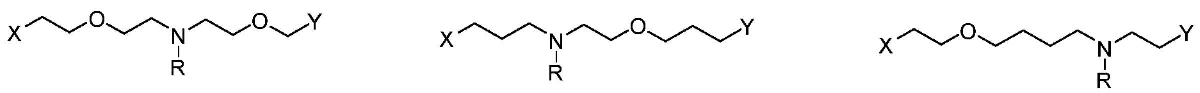

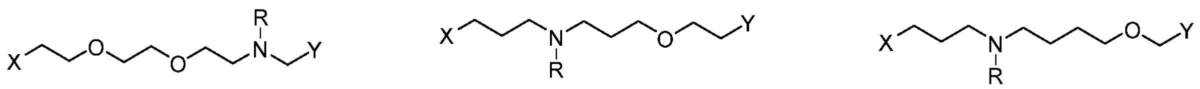

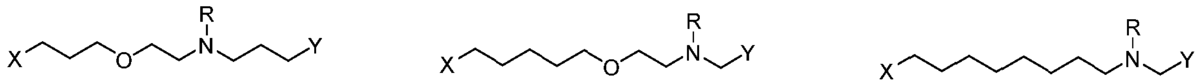

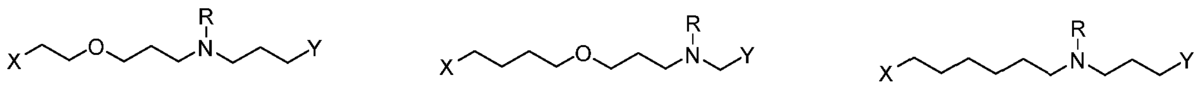

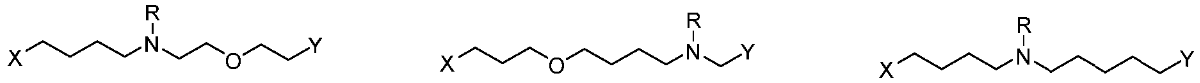

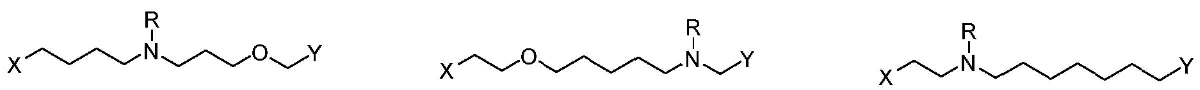

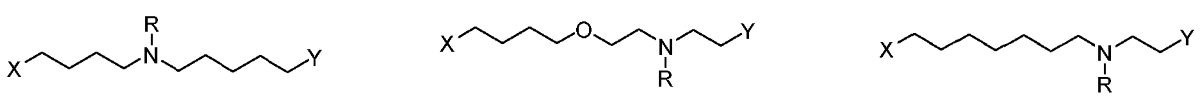

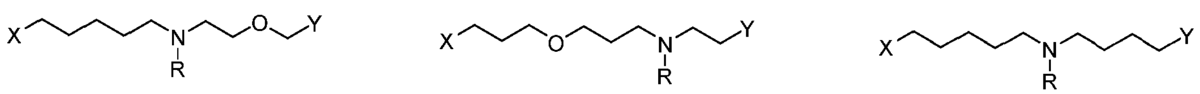

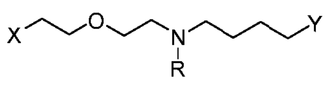

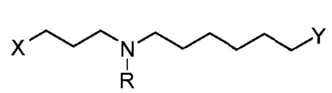

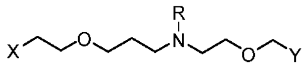
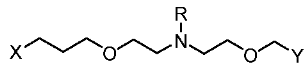
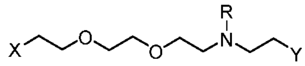
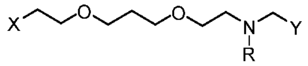
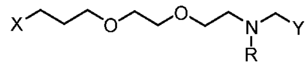
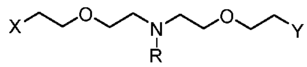
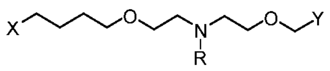
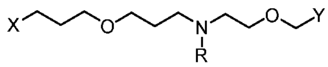
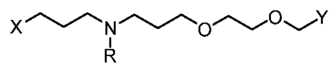
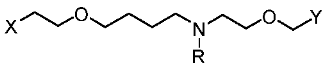
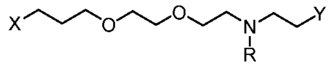
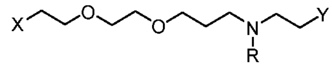
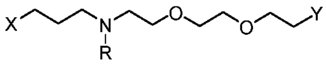
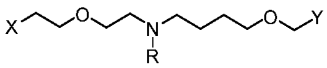
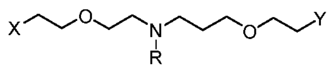
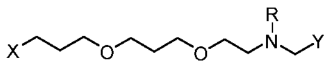
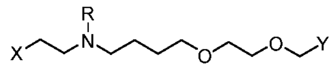
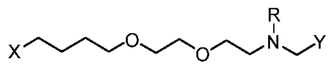
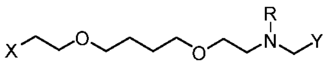
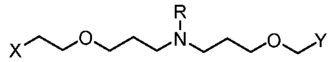
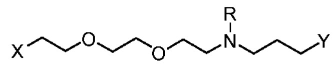
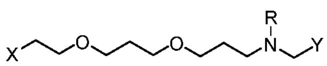
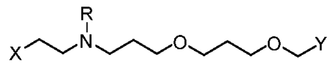
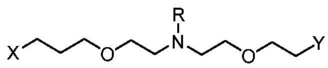
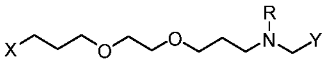
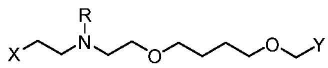
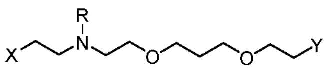
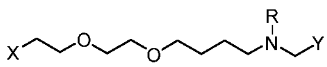
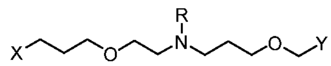
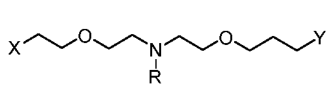
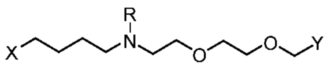
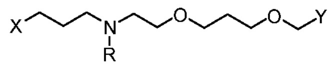
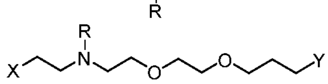
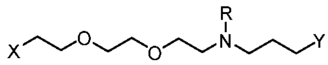
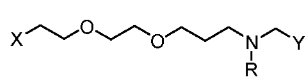
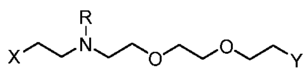

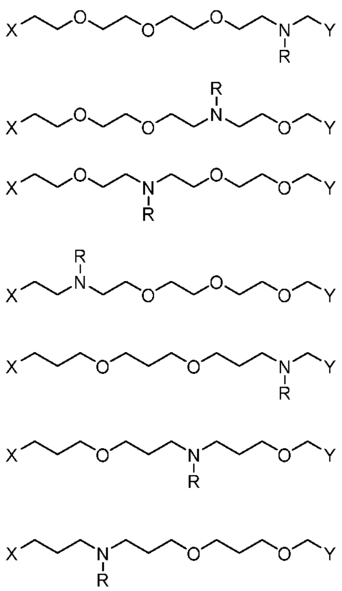

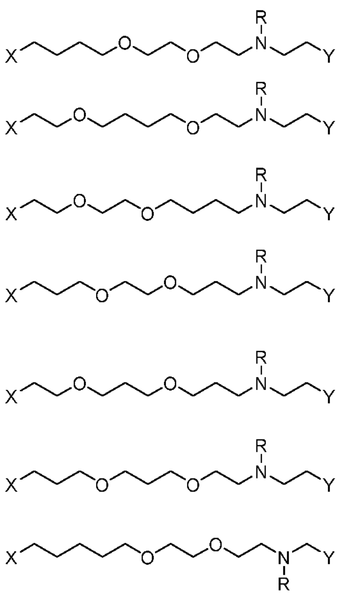

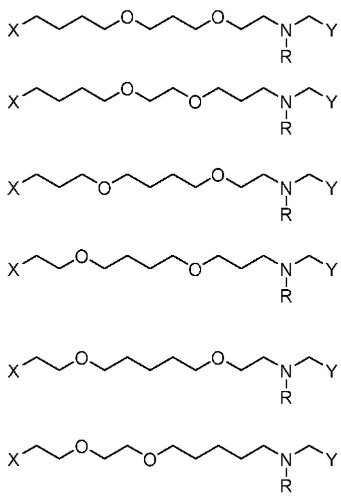

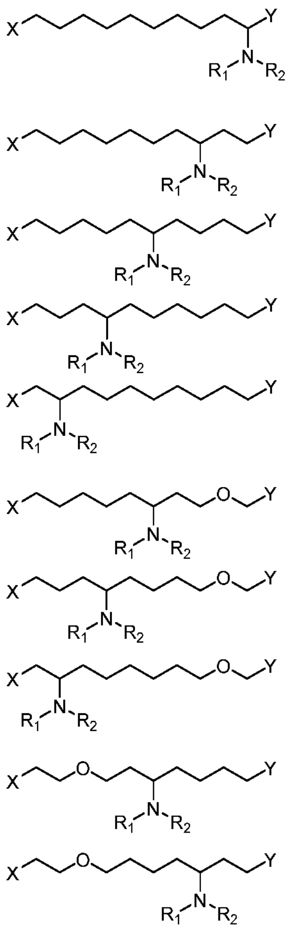

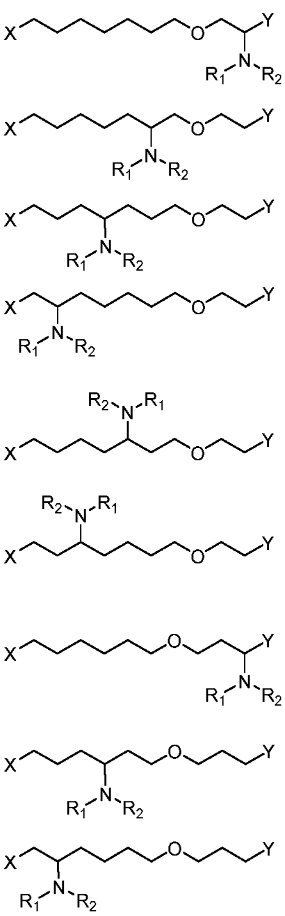

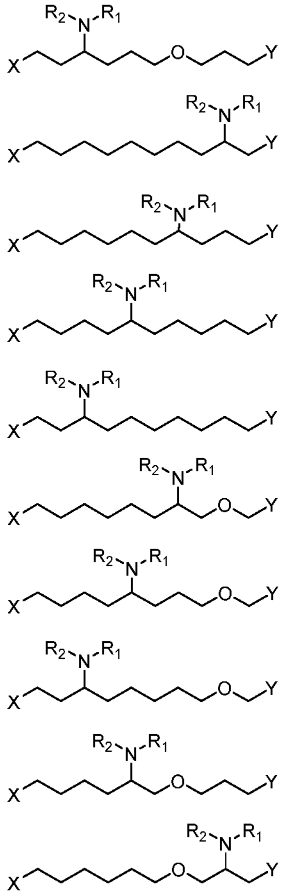

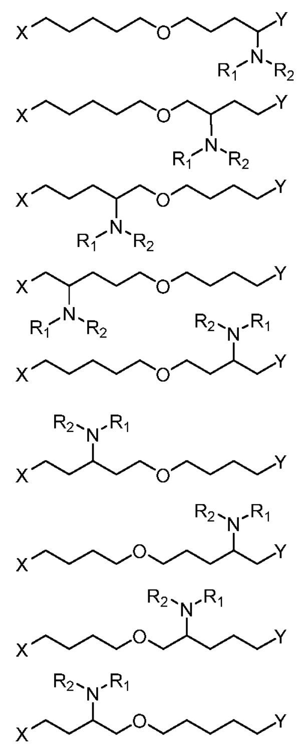

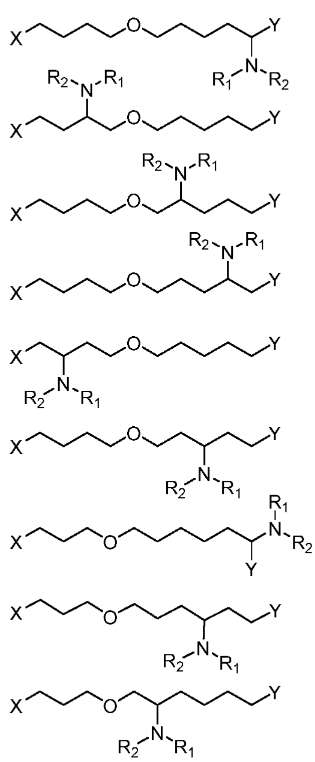

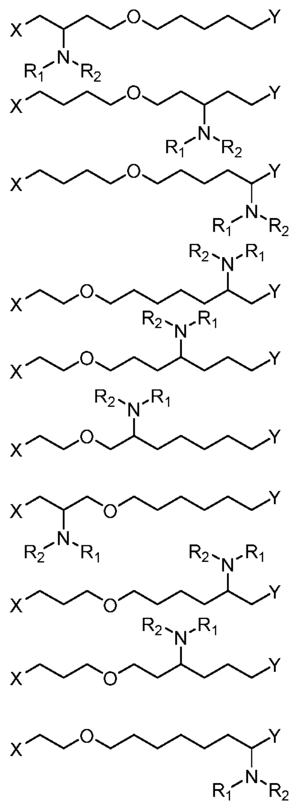

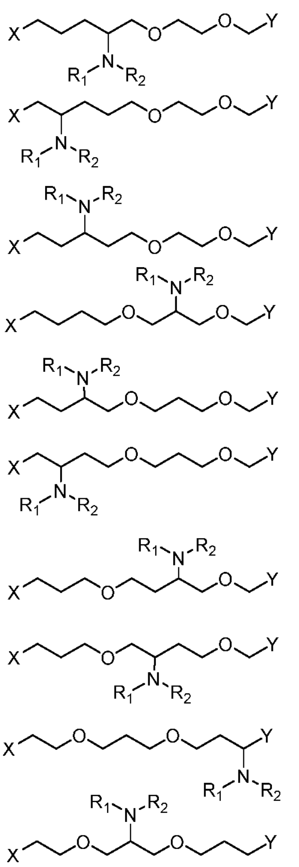

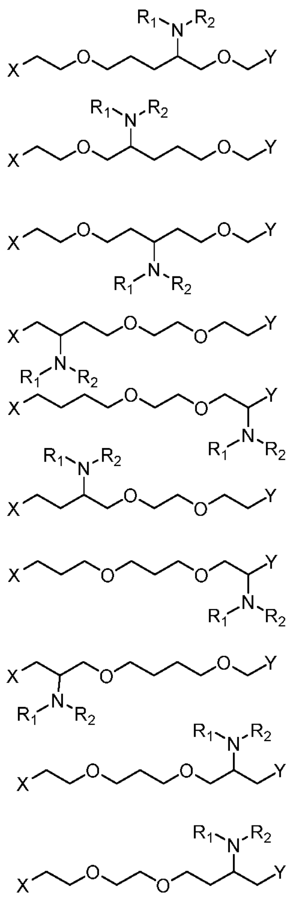

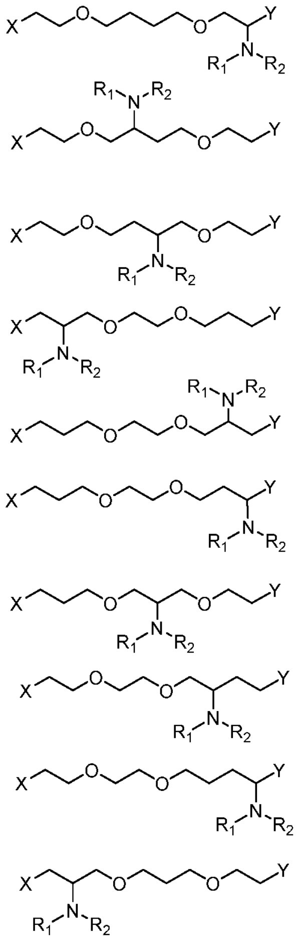

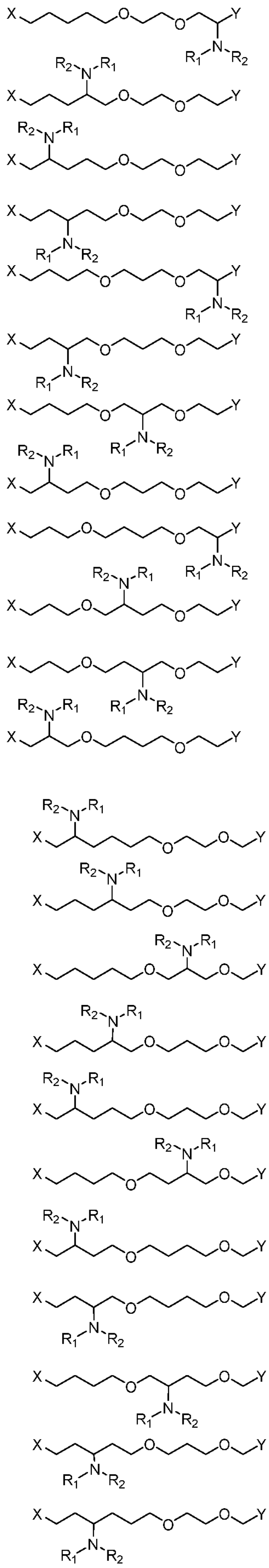

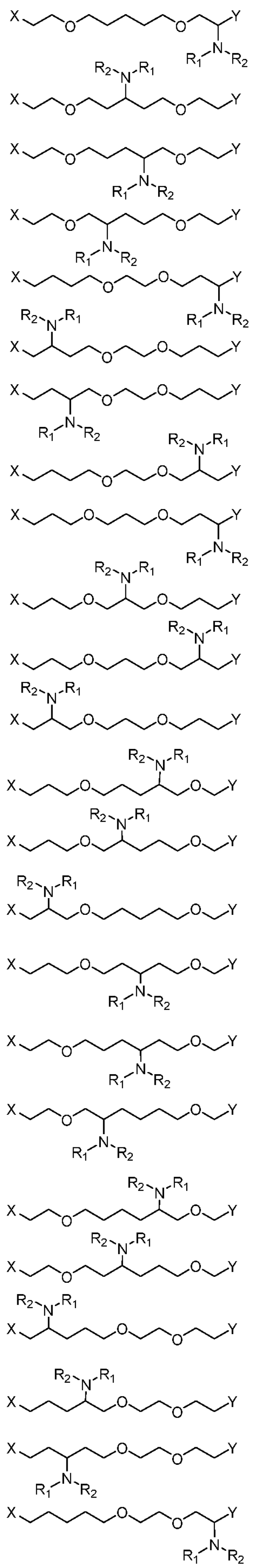

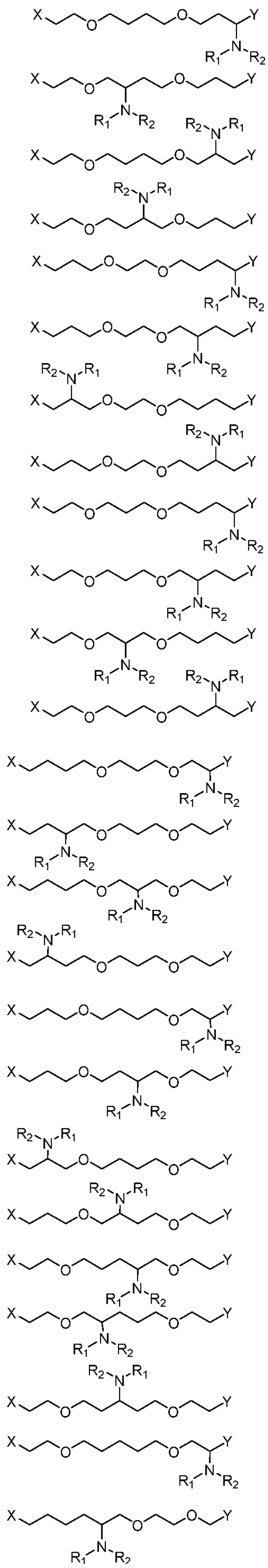

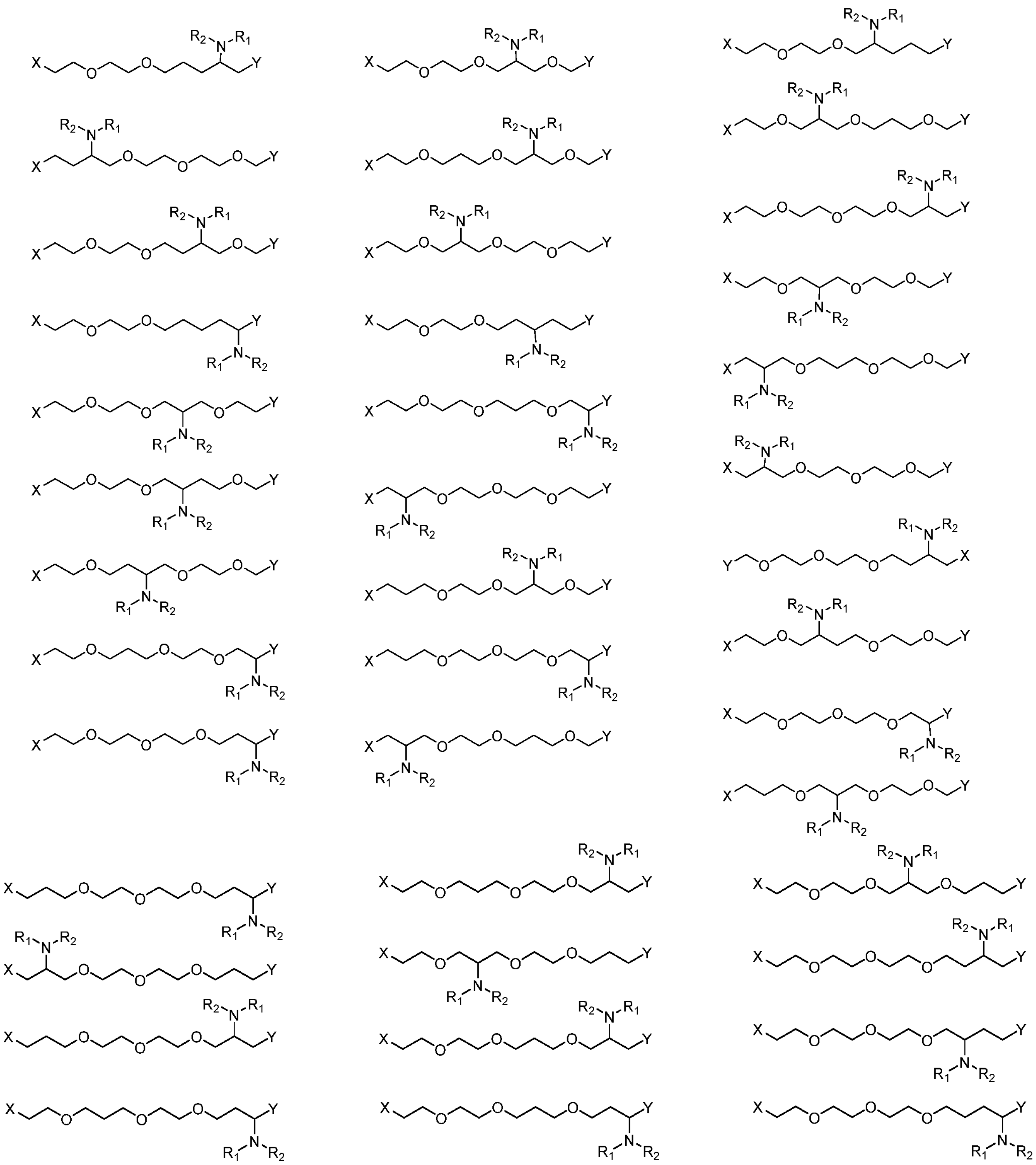

wherein, R or one of $R_1$ and $R_2$ is -X'-L3-Z;
the other one of $R_1$ and $R_2$, and X', X and Y are as defined in claim 1.

**9.** The conjugate of Claim 1, wherein L1 is selected from the group consisting of

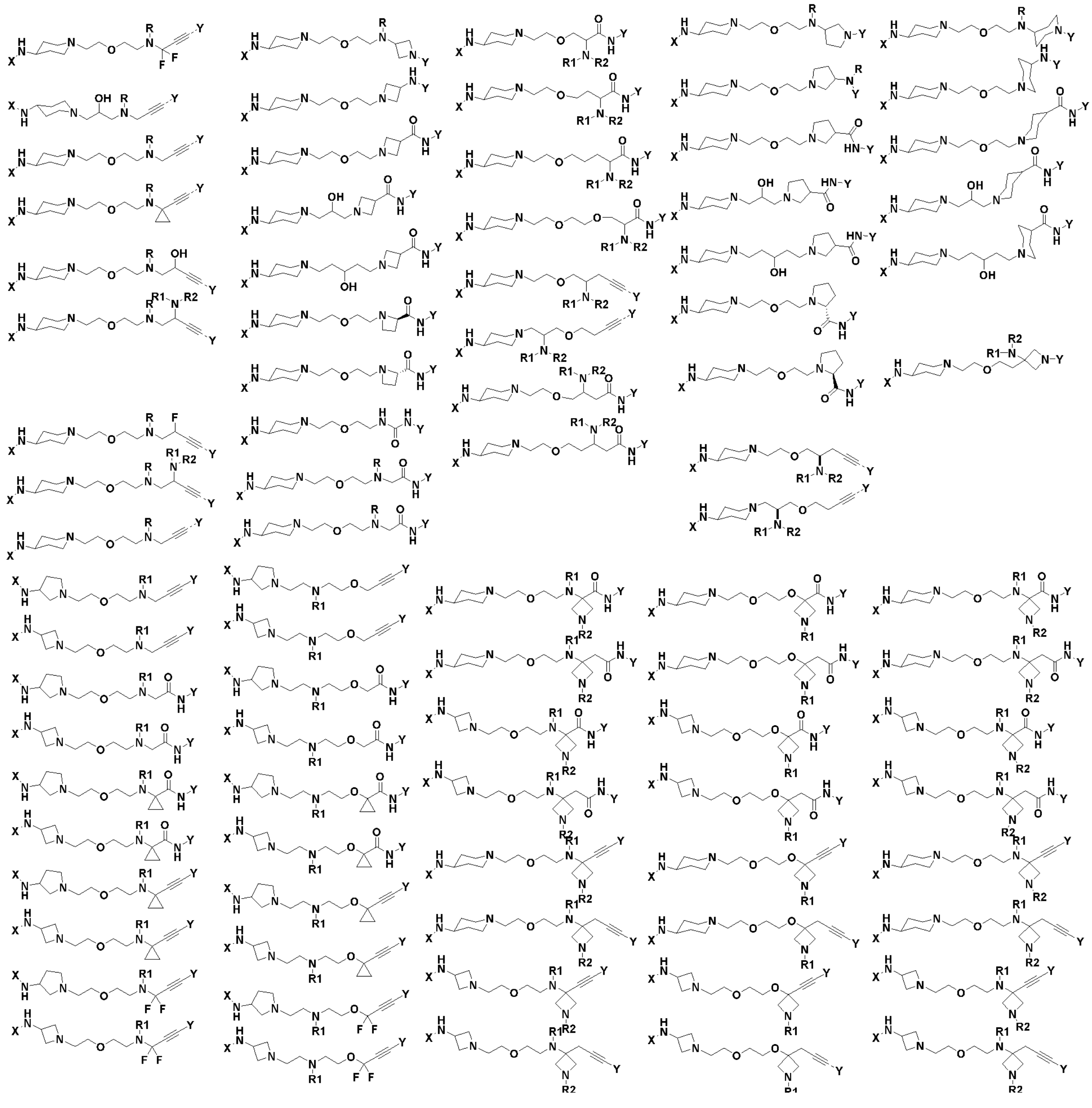

wherein, R or one of $R_1$ and $R_2$ is -X'-L3-Z;
the other one of $R_1$ and $R_2$, and X', X and Y are as defined in claim 1.

**10.** A pharmaceutical composition wherein the pharmaceutical composition comprises the conjugate of any one of Claims 1-8 and pharmaceutically acceptable carrier.

**11.** A preparation method of the conjugate of any one of Claims 1-8, wherein the reaction route of the method is shown as follows:

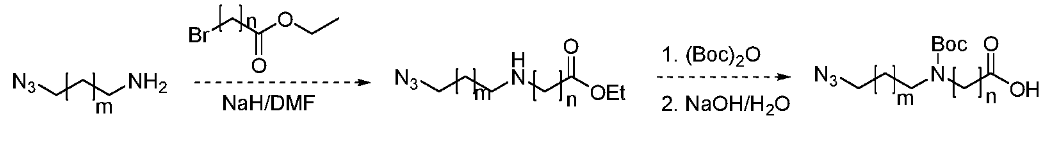


;

or

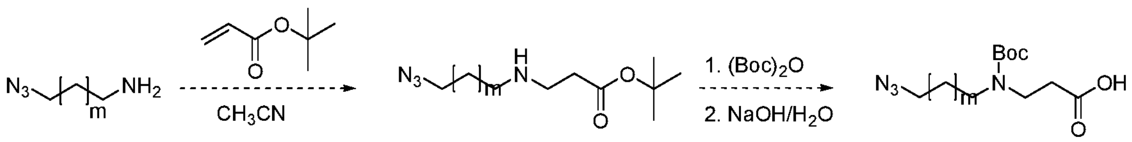


wherein m, and n are defined as above.

**12.** A use of the conjugate of any one of Claims 1-9 in the preparation of pharmaceutical compositions for the treatment of diseases associated with excess of the target protein.

**13.** A method for reducing the content of target proteins in a cell, wherein the cell is contacted with the conjugate of any one of Claims 1-9, thereby reducing the content of the target proteins in the cell.

**14.** A method for reducing the content of target proteins in a subject or treating diseases associated with excess of target proteins, wherein comprising step of administering the conjugate of any one of Claims 1-9 to a subject in need thereof.

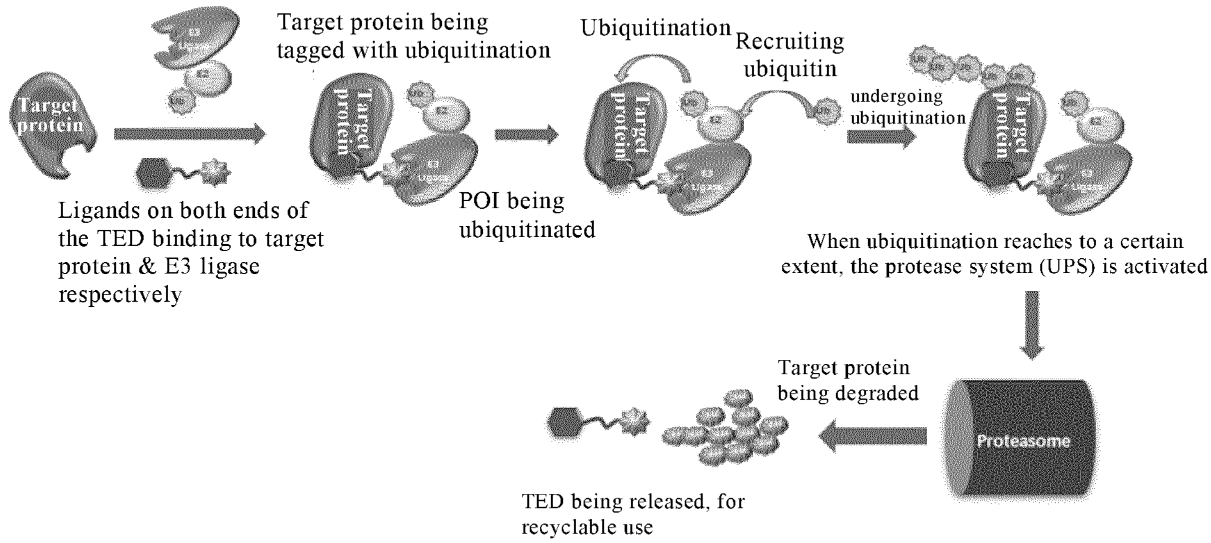


Figure 1

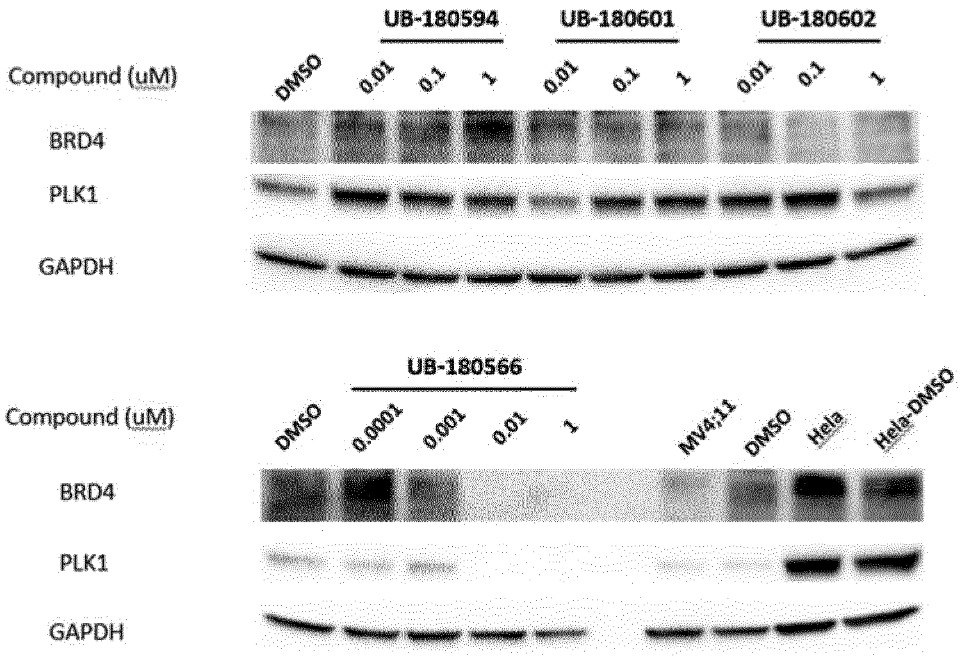


Figure 2

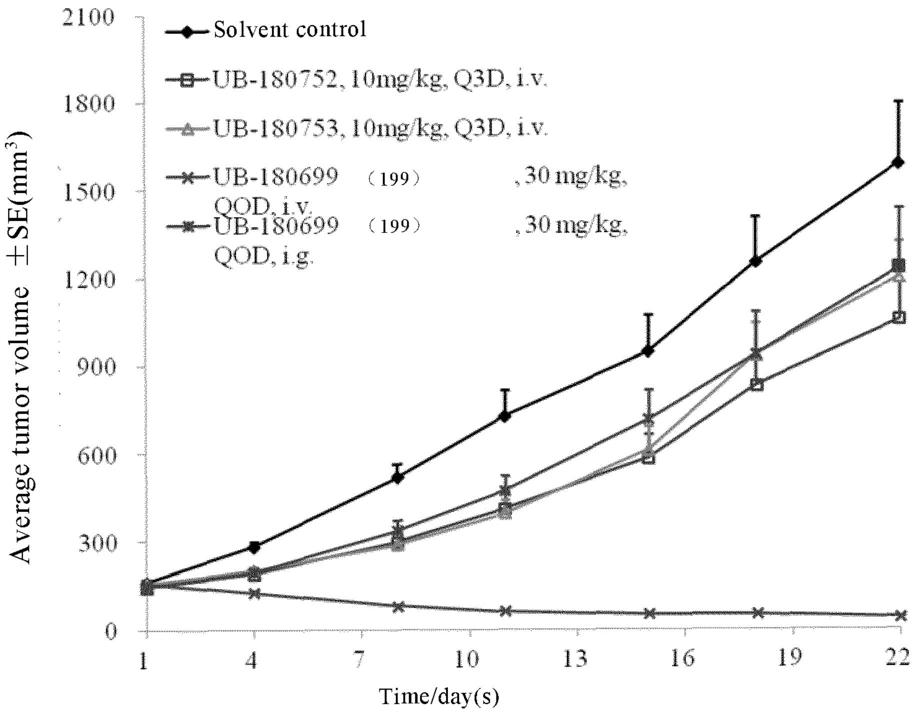


Figure 3

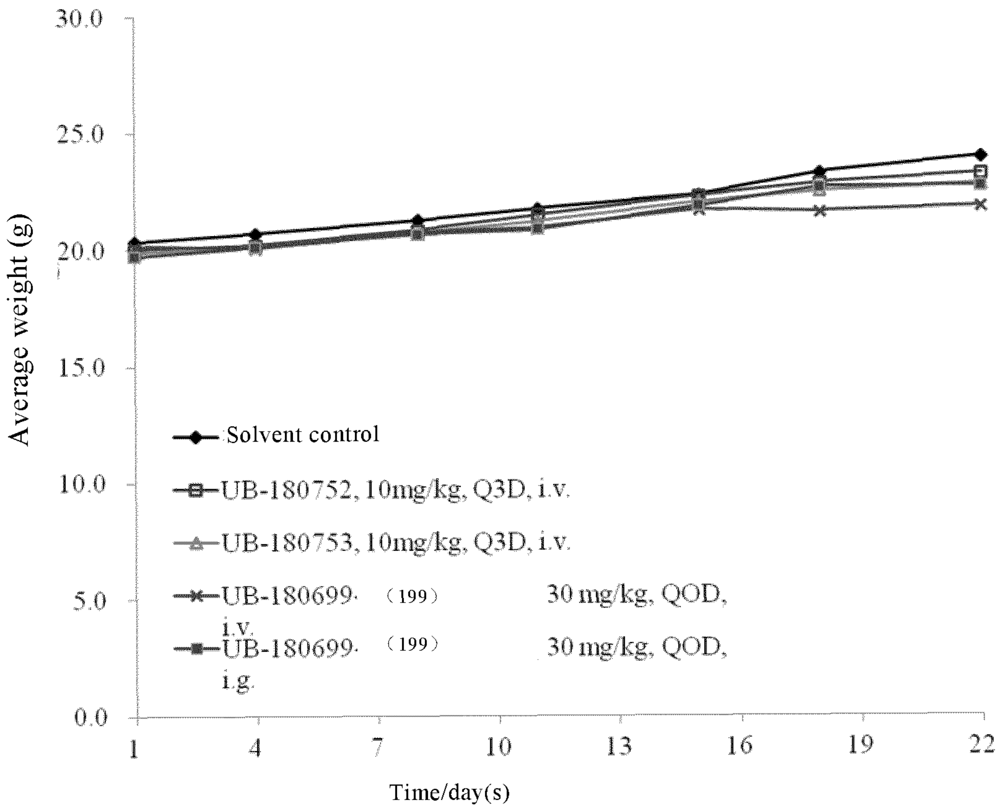


Figure 4

## INTERNATIONAL SEARCH REPORT

International application No.
**PCT/CN2019/110225**

**A. CLASSIFICATION OF SUBJECT MATTER**

A61K 47/66(2017.01)i; A61P 35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

Database: DWPI, SIPOABS, CPRSABS, CNABS, CNTXT, EPODOC, CNKI, CA, EMBASE, STN, NCBI, VEN; Keywords: E3连接酶, 泛素连接酶, 偶联, 靶向, E3 ligase, ubiquitin protein ligase,ligand, conjug+, target+

**C. DOCUMENTS CONSIDERED TO BE RELEVANT**

| Category* | Citation of document, with indication, where appropriate, of the relevant passages | Relevant to claim No. |
|---|---|---|
| X | WO 2018119441 A1 (ARVINAS, INC.; YALE UNIVERSITY) 28 June 2018 (2018-06-28) the abstract, and claims 1, 2 and 33-41 | 1-4, 10-12 |
| A | WO 2018119441 A1 (ARVINAS, INC.; YALE UNIVERSITY) 28 June 2018 (2018-06-28) the abstract, and claims 1, 2 and 33-41 | 5-9 |
| X | CN 108136044 A (ARVINAS, INC.) 08 June 2018 (2018-06-08) the abstract, and claims 1-11 | 1-3, 10-12 |
| A | CN 108136044 A (ARVINAS, INC.) 08 June 2018 (2018-06-08) the abstract, and claims 1-11 | 4-9 |
| A | WO 2017201449 A1 (GENENTECH, INC. et al.) 23 November 2017 (2017-11-23) claims 1 and 32, and description, paragraph [0172], lines 14-16 | 1-12 |
| X | WO 2018102067 A2 (ARVINAS, INC.) 07 June 2018 (2018-06-07) claims 1-36 | 1-4, 10-12 |
| A | WO 2018102067 A2 (ARVINAS, INC.) 07 June 2018 (2018-06-07) claims 1-36 | 5-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 December 2019** | **15 January 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/110225**

**C. DOCUMENTS CONSIDERED TO BE RELEVANT**

| Category* | Citation of document, with indication, where appropriate, of the relevant passages | Relevant to claim No. |
|---|---|---|
| PX | WO 2018226542 A1 (ARVINAS, INC.; YALE UNIVERSITY) 13 December 2018 (2018-12-13)<br>claims 1-37 | 1-4, 10-12 |
| PA | WO 2018226542 A1 (ARVINAS, INC.; YALE UNIVERSITY) 13 December 2018 (2018-12-13)<br>claims 1-37 | 5-9 |
| X | CN 106458993 A (ARVINAS, INC.) 22 February 2017 (2017-02-22)<br>the abstract, claim 1, and description, paragraphs [0144]-[0146] | 1-3, 10-12 |
| A | WO 2017185034 A1 (DANA-FARBER CANCER INSTITUTE, INC.) 26 October 2017 (2017-10-26)<br>claims 1-38 | 4-9 |
| X | WO 2017185034 A1 (DANA-FARBER CANCER INSTITUTE, INC.) 26 October 2017 (2017-10-26)<br>claims 1-38 | 1-3, 10-12 |
| A | CN 106458993 A (ARVINAS, INC.) 22 February 2017 (2017-02-22)<br>the abstract, claim 1, and description, paragraphs [0144]-[0146] | 4-9 |

Form PCT/ISA/210 (second sheet) (January 2015)

## INTERNATIONAL SEARCH REPORT

International application No.
**PCT/CN2019/110225**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-14**
because they relate to subject matter not required to be searched by this Authority, namely:

[1] The method of claim 13 is a method for reducing the target protein content in a cell, which is a method that cannot exclude the treatment methods of a disease; the method of claim 14 is a method for reducing the target protein content in a subject or treating a disease related to an excessive amount of target protein, which is the treatment method of the disease. Therefore the methods do not warrant a search according to the criteria set out in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/110225**

| Patent document cited in search report | Publication date (day/month/year) | Patent family member(s) | Publication date (day/month/year) |
|---|---|---|---|
| WO 2018119441 A1 | 28 June 2018 | WO 2018119441 A8 | 23 May 2019 |
| | | WO 2018119441 A9 | 10 October 2019 |
| | | KR 20190101406 A | 30 August 2019 |
| | | CA 3047586 A1 | 28 June 2018 |
| | | AU 2017382406 A1 | 18 April 2019 |
| | | EP 3559002 A1 | 30 October 2019 |
| | | CO 2019007892 A2 | 09 October 2019 |
| | | IL 267398 D0 | 29 August 2019 |
| | | US 2018193470 A1 | 12 July 2018 |
| CN 108136044 A | 08 June 2018 | EP 3302572 A1 | 11 April 2018 |
| | | BR 112017025975 A2 | 14 August 2018 |
| | | RU 2704807 C2 | 31 October 2019 |
| | | AU 2016270442 A1 | 07 December 2017 |
| | | MX 2017015605 A | 06 June 2018 |
| | | WO 2016197032 A1 | 08 December 2016 |
| | | JP 2018526429 A | 13 September 2018 |
| | | RU 2017145267 A | 16 July 2019 |
| | | CA 2988414 A1 | 08 December 2016 |
| | | RU 2017145267 A3 | 17 July 2019 |
| | | EP 3302572 A4 | 31 October 2018 |
| | | KR 20180035779 A | 06 April 2018 |
| WO 2017201449 A1 | 23 November 2017 | US 2019175612 A1 | 13 June 2019 |
| | | CN 109152843 A | 04 January 2019 |
| | | JP 2019522633 A | 15 August 2019 |
| | | EP 3458101 A1 | 27 March 2019 |
| WO 2018102067 A2 | 07 June 2018 | IL 266236 D0 | 30 June 2019 |
| | | MX 2019005007 A | 18 July 2019 |
| | | EP 3535265 A2 | 11 September 2019 |
| | | JP 2019532995 A | 14 November 2019 |
| | | CO 2019005712 A2 | 11 June 2019 |
| | | US 2018125821 A1 | 10 May 2018 |
| | | CA 3042260 A1 | 07 June 2018 |
| | | WO 2018102067 A3 | 19 July 2018 |
| | | CN 110234646 A | 13 September 2019 |
| | | AU 2017367872 A1 | 18 April 2019 |
| | | KR 20190067923 A | 17 June 2019 |
| WO 2018226542 A1 | 13 December 2018 | US 2018353501 A1 | 13 December 2018 |
| CN 106458993 A | 22 February 2017 | CA 2945975 A1 | 22 October 2015 |
| | | AU 2015247817 A1 | 27 October 2016 |
| | | EP 3131588 A4 | 10 January 2018 |
| | | EP 3131588 A2 | 22 February 2017 |
| | | AU 2015247817 B2 | 31 October 2019 |
| | | AU 2019240589 A1 | 24 October 2019 |
| | | RU 2016144289 A | 18 May 2018 |
| | | BR 112016024016 A2 | 23 January 2018 |
| | | KR 20170002446 A | 06 January 2017 |
| | | MX 2016013563 A | 09 May 2017 |
| | | US 2015291562 A1 | 15 October 2015 |
| | | JP 2017513862 A | 01 June 2017 |

Form PCT/ISA/210 (patent family annex) (January 2015)

**INTERNATIONAL SEARCH REPORT**
**Information on patent family members**

International application No.
**PCT/CN2019/110225**

| Patent document cited in search report | | | Publication date (day/month/year) | Patent family member(s) | | | Publication date (day/month/year) |
|---|---|---|---|---|---|---|---|
| | | | | RU | 2016144289 | A3 | 18 May 2018 |
| | | | | WO | 2015160845 | A3 | 10 December 2015 |
| | | | | WO | 2015160845 | A2 | 22 October 2015 |
| WO | 2017185034 | A1 | 26 October 2017 | EP | 3445764 | A1 | 27 February 2019 |
| | | | | JP | 2019514876 | A | 06 June 2019 |
| | | | | US | 2019112307 | A1 | 18 April 2019 |
| | | | | CA | 3020543 | A1 | 26 October 2017 |
| | | | | AU | 2017254711 | A1 | 11 October 2018 |
| | | | | CN | 109311869 | A | 05 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017176957 A1
- WO 2017176957 A
- WO 2017176958 A1


### Non-patent literature cited in the description


- **BERGE, S.M. et al.** Pharmaceutical Salts. *Journal of Pharmaceutical Science,* 1977, vol. 66, 1-19